(19) Europäisches Patentamt
European Patent Office
Office européen des brevets

(11) Publication number: 0 187 489 B1

(12) # EUROPEAN PATENT SPECIFICATION

(45) Date of publication of patent specification: 12.12.90

(21) Application number: 85308951.4

(22) Date of filing: 10.12.85

(51) Int. Cl.$^5$: C 07 D 251/16, C 07 D 251/46, C 07 D 409/12, C 07 D 403/12, C 07 D 405/14, C 07 D 239/46, C 07 D 239/52, C 07 D 401/12, C 07 D 405/12, C 07 D 491/044, A 01 N 47/36

(54) ## Herbicidal sulfonamides.

(30) Priority: 11.12.84 US 680549
29.08.85 US 769691
26.08.85 US 768158

(43) Date of publication of application:
16.07.86 Bulletin 86/29

(45) Publication of the grant of the patent:
12.12.90 Bulletin 90/50

(84) Designated Contracting States:
BE CH DE FR GB IT LI LU NL SE

(56) References cited:
EP-A-0 030 142
EP-A-0 084 224
EP-A-0 087 780
EP-A-0 095 925
EP-A-0 096 593
EP-A-0 101 670
EP-A-0 116 518
EP-A-0 132 230
EP-A-0 141 777
EP-A-0 155 767
EP-A-0 156 521
US-A- 368 069

The file contains technical information submitted after the application was filed and not included in this specification

(73) Proprietor: E.I. DU PONT DE NEMOURS AND COMPANY
1007 Market Street
Wilmington Delaware 19898 (US)

(72) Inventor: Hay, James Volney
36 Stature Drive
Newark Delaware 19713 (US)
Inventor: Zimmerman, Donna Frieze
Box 200A, R.D. No. 1
Landenberg Pennsylvania 19350 (US)
Inventor: Wexler, Barry Arthur
2205 Patwynn Road
Wilmington Delaware 19810 (US)

(74) Representative: Hildyard, Edward Martin et al
Frank B. Dehn & Co. European Patent Attorneys
Imperial House 15-19 Kingsway
London WC2B 6UZ (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 187 489 B1

Courier Press, Leamington Spa, England.

## Description

Background of the Invention

The invention relates to herbicidally active sulfonylurea compounds, agriculturally suitable compositions thereof and method of their use as herbicides or plant growth regulants.

In the most common situation, the control of undesired vegetation is desired to permit the growth of useful crops such as cotton, rice, corn, wheat and soybeans, to name a few. Unchecked weed growth in such useful crops can cause significant losses reducing profit to the farmer and increasing costs to the consumer. In other situations, herbicides are desired which will control all plant growth. Examples of areas in which complete control of all vegetation is desired are areas around fuel storage tanks, ammunition depots and industrial storage areas. There are many products commercially available for these purposes, but the search continues for products which are more effective, less costly and environmentally safe.

U.S. 4,368,069, issued January 11, 1983, discloses herbicidal benzenesulfonamides of formula

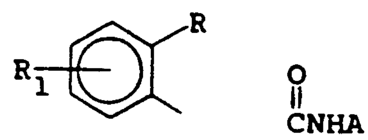

wherein

R is $-(CR_5R_6)_n-R_2$;

n is 0 or 1; and

$R_2$ may be $C_5-C_6$ cycloalkenyl or $C_2-C_5$ alkenyl.

South African Patent Application 83/3779. Swiss priority 5/26/82, discloses herbicidal sulfonamides of formula

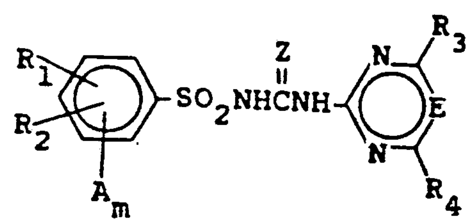

wherein

A is a radical of the formula C≡CR; and

R is, among others, a variety of alkyl or substituted alkyl groups.

Herbicidal thiophene sulfonamides are disclosed in European Patent Application (EP—A) No. 30,142, published June 10, 1981.

Herbicidal pyridinesulfonamides are disclosed in European Patent Application (EP—A) No. 13,480, published July 23, 1980).

U.S. 4,420,325 discloses herbicidal sulfonamides of the following formula

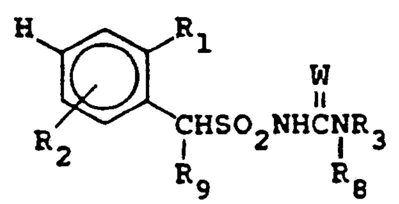

South African Patent Application 84/2722, published 10/13/84. Swiss priority 4/13/83, discloses herbicidal sulfonamides of formula

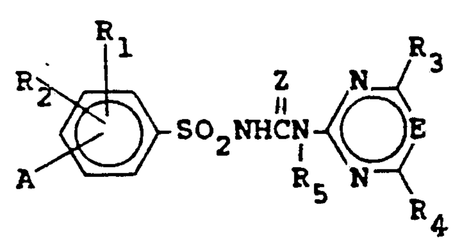

wherein

A is a radical of formula $-CR_6R_7XR_8$, $CR_9R_{10}R_{11}$ or $CHR_7SCQR_{21}$;

$R_6$ is H, $C_1-C_4$ alkyl or fluorine;

$R_7$ is H or $CH_3$:

X is O, S, SO or $SO_2$;

$R_8$ may be $C_2$—$C_5$ haloalkenyl, $C_3$—$C_5$ alkynyl, etc.

South African Patent Application 84/6610, German priority 8/25/83, discloses herbicidal pyrazolesulfonylureas of formula

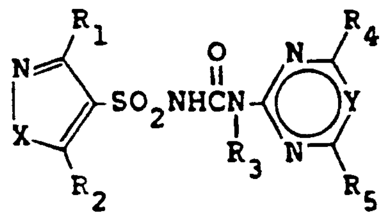

wherein

X is O or $NR_6$;

$R_1$ and $R_2$ are independently H, halogen, $C_1$—$C_6$ alkyl, $C_2$—$C_6$ alkenyl or $C_2$—$C_6$ alkynyl, which are optionally substituted by 1—3 halogen atoms. $C_1$—$C_4$ alkoxy, $C_1$—$C_4$ alkylsulfenyl, $C_1$—$C_4$ alkylsulfinyl, $C_1$—$C_4$ alkylsulfonyl, OH, etc.

South African Patent Application 83/6449, published 3/1/84, Swiss priority 8/1/82, discloses herbicidal sulfonylureas of the formula

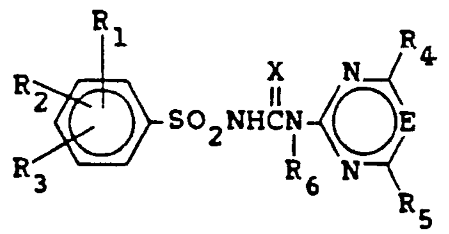

wherein

$R_3$ is $C_2$—$C_{10}$ alkenyl which is substituted by, *inter alia,* one or more fluorine or bromine atoms or by one or more hydroxyl, cyano or nitro groups.

European Publication No. 132,230, published 1/23/85, Swiss priority 7/18/83, discloses a process for preparing sulfonylureas of the formula

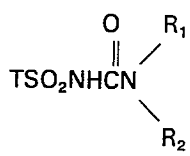

wherein

T denotes a substituted phenyl radical of formula

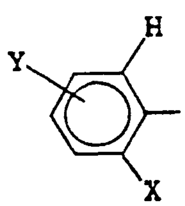

or

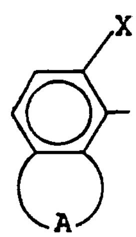

;

X is, *inter alia,* H, halogen, $C_2$—$C_4$ alkynyl;

$R_1$ is H or $C_1$—$C_4$ alkyl; and

$R_2$ is 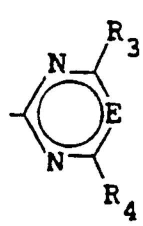 or 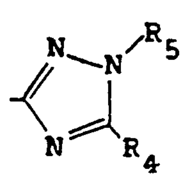 .

European Patent Application (EP—A) No. 83,975, published July 20, 1983, discloses herbicidal benzenesulfonamides of formula

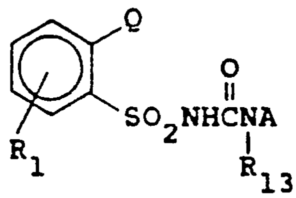

wherein

Q is selected from various five or six-membered aromatic or partially unsaturated heterocyclic rings containing 2 or 3 heteroatoms selected from O, S or NR.

European Patent Application (EP—A) No. 85,476, published August 10, 1983, discloses herbicidal benzenesulfonamides of formulae

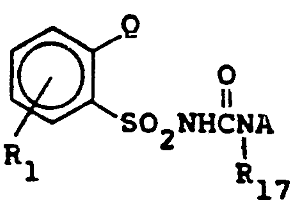

and

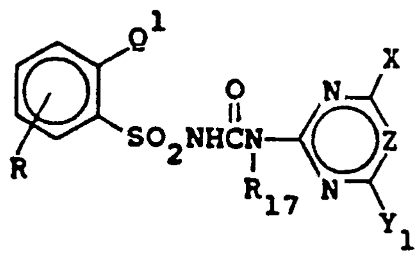

wherein

Q is selected from various 5-membered aromatic heterocycles, and their dihydro and tetrahydro analogs, which contain one heteroatom selected from O, S or NR, or Q is a saturated or partially unsaturated 6-membered ring containing one heteroatom selected from O or S; and

$Q^1$ is a 6-membered aromatic heterocycle containing one to three N atoms.

South African Patent Application 83/8416, published May, 1984, discloses herbicidal benzenesulfonamides of formula

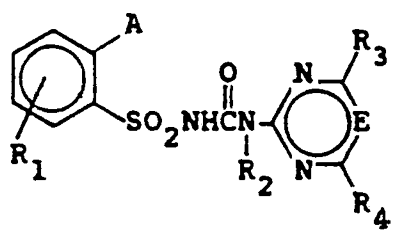

wherein

A is an unsaturated or only partially saturated 5- or 6-membered heterocyclic ring system which is bonded through a carbon atom and contains 1, 2 or 3 heteroatoms.

EP—A—95,925, published December 7, 1983, discloses herbicidal pyrazolesulfonamides in which the group adjacent to the sulfonamide moiety may be selected from H, $C_1$—$C_3$ alkyl, F, Cl, Br, $NO_2$, $OR_{16}$, $CO_2R_{23}$, $S(O)_nR_{24}$ or $SO_2NR_{19}R_{20}$.

EP—A—87,780, published September 7, 1983, filed by Nissan Chemical Industries discloses pyrazole sulfonamides of formula

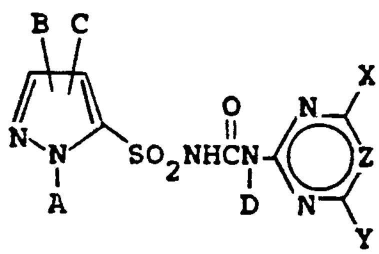

wherein

A is H, $C_1$—$C_8$ alkyl or optionally substituted phenyl; and

B and C are independently H, halogen, $NO_2$, $C_1$—$C_8$ alkyl, $CO_2R$, etc.;

EP—A—96,003, Ciba-Geigy, published November 28, 1983, discloses herbicidal compounds of general formula

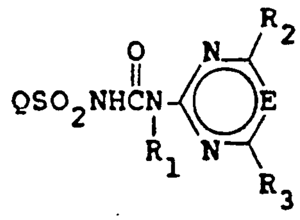

wherein

$R_1$ is H or $C_1$—$C_3$ alkyl; and

Q is an unsubstituted or substituted 5-membered heterocycle radical which is bound by way of a carbon atom and which contains 2 or 3 identical or different heteroatoms.

Unexamined Japanese Patent Application 9,013,778, priority July 15, 1982, filed by Nihon Noyaku describes herbicidal pyrazolesulfonamides with halo, alkyl and alkoxycarbonyl substituents.

Summary of the Invention

This invention is the discovery of novel compounds of Formula I with herbicidal activity, agriculturally suitable compositions containing them and a method-of-using said compounds as general and/or selective preemergent and/or postemergent herbicides or plant growth regulants.

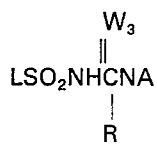

wherein /

L is 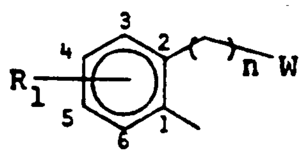 , 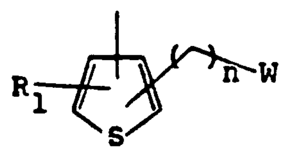 ,

L-1 L-2

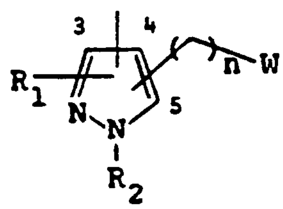 , 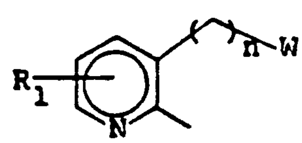 ,

L-3 L-4

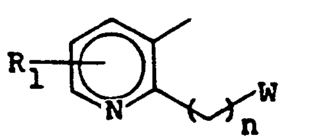 , 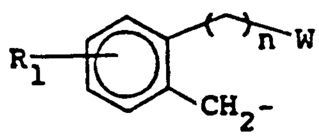 ,

L-5 L-6

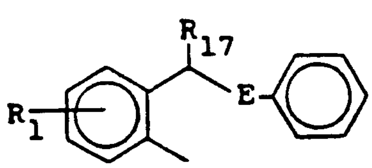 ,

L-7

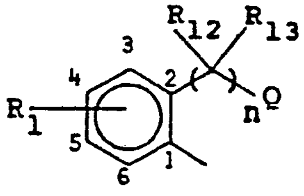 , 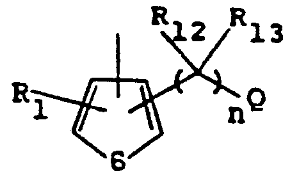 ,

L-8 L-9

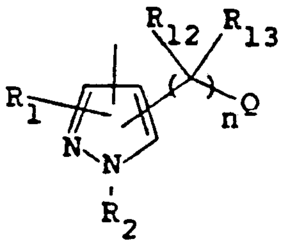 , 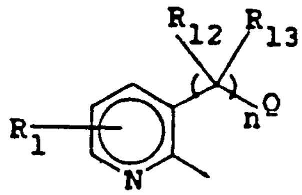 ,

L-10 L-11

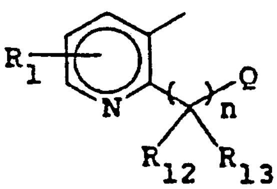 or 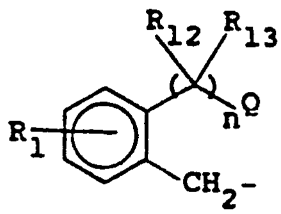 ;

L-12 L-13

R is H or $CH_3$;
$W_3$ is O or S;
n is 0 or 1;
E is $S(O)_p$ or Se;
p is 0, 1 or 2;
$R_1$ is H, $C_1$—$C_3$ alkyl, $C_1$—$C_3$ haloalkyl, halogen, nitro, $CH_2CN$, $CH_2OCH_3$, $CH_2SCH_3$, CN, $C_1$—$C_3$ alkoxy, $SO_2NR^IR^{II}$, $C_1$—$C_3$ alkylthio, $C_1$—$C_3$ alkylsulfinyl, $C_1$—$C_3$ alkylsulfonyl or $CO_2R^{III}$;
$R^I$ is H, $C_1$—$C_4$ alkyl, $C_2$—$C_3$ cyanoalkyl, methoxy or ethoxy;
$R^{II}$ is H, $C_1$—$C_4$ alkyl or $C_3$—$C_4$ alkenyl; or $R^I$ and $R^{II}$ may be taken together as $-(CH_2)_3-$, $-(CH_2)_4-$, $-(CH_2)_5-$ or $-CH_2CH_2OCH_2CH_2-$;
$R^{III}$ is $C_1$—$C_4$ alkyl, $C_3$—$C_4$ alkenyl, $C_3$—$C_4$ alkynyl, $C_2$—$C_4$ haloalkyl, $C_2$—$C_3$ cyanoalkyl, $C_5$—$C_6$ cycloalkyl, $C_4$—$C_7$ cycloalkylalkyl or $C_2$—$C_4$ alkoxyalkyl;
$R_2$ is $C_1$—$C_3$ alkyl;
W is $W_1$ or $W_2$;
$W_1$ is $C_2$—$C_8$ alkenyl or $C_4$—$C_7$ cycloalkenyl either of which may be optionally substituted with 1—3 atoms of F, Cl or Br, OH, CN, $NO_2$, $C_1$—$C_3$ alkoxy, $C_1$—$C_3$ alkylthio, $C_1$—$C_3$ alkylsulfinyl, $C_1$—$C_3$ alkylsulfonyl, amino, $C_2$—$C_4$ alkenyl, $C_1$—$C_3$ alkylamino, di($C_1$—$C_3$ alkyl)amino, ($C_1$—$C_3$ alkylamino)sulfamoyl, di($C_1$—$C_3$ alkylamino)sulfamoyl, $OC(O)C_1$—$C_3$ alkyl, $OC(O)C_1$—$C_3$ alkoxy, $OSO_2C_1$—$C_3$ alkyl, $OSO_2C_6H_5$, $OSO_2$—4—$CH_3$—$C_6H_4$, phenyl or phenyl substituted with halogen, $CH_3$, $OCH_3$, $SCH_3$ or $NO_2$;
$W_2$ is $C_2$ alkynyl which may be substituted with phenyl or phenyl substituted with halogen, $CH_3$, $OCH_3$, $SCH_3$ or $NO_2$ or $W_2$ is $C_3$—$C_8$ alkynyl which may be optionally substituted with 1—3 atoms of F, Cl or Br, OH, CN, $NO_2$, $C_1$—$C_3$ alkoxy, $C_1$—$C_3$ alkylthio, $C_1$—$C_3$ alkylsulfinyl, $C_1$—$C_3$ alkylsulfonyl, amino, $C_1$—$C_3$ alkylamino, di($C_1$—$C_3$ alkyl)amino, ($C_1$—$C_3$ alkylamino)sulfamoyl, di($C_1$—$C_3$ alkylamino)sulfamoyl, $OC(O)C_1$—$C_3$ alkyl, $OC(O)C_1$—$C_3$ alkoxy, $OSO_2C_1$—$C_3$ alkyl, $OSO_2C_6H_5$, $OSO_2$—4—$CH_3$—$C_6H_4$, phenyl or phenyl substituted with halogen, $CH_3$, $OCH_3$, $SCH_3$ or $NO_2$;
Q is a 3- or 4-membered heterocyclic ring which contains one heteroatom selected from O, S and $NR_3$, or a 3- or 4-membered carbocyclic ring in which one carbon atom may optionally be in the form of a carbonyl group, or a fully-saturated 5- or 6-membered carbocyclic ring, and Q may be optionally substituted with 1—4 substituents selected from halogen, CN, OH, $C_1$—$C_3$ alkyl, $C_1$—$C_3$ alkoxy, $C_1$—$C_3$ alkylthio, $C_2$—$C_3$ alkoxycarbonyl, $C_1$—$C_4$ alkylsulfonyl, ($C_1$—$C_4$ alkyl)aminosulfamoyl and di($C_1$—$C_4$ alkyl)-aminosulfamoyl;

$R_3$ is $C_1$—$C_4$ alkyl;
$R_{12}$ is H or $OR_{14}$;
$R_{13}$ is H or $C_1$—$C_4$ alkyl;
$R_{14}$ is H, $C_1$—$C_4$ alkyl, $C_3$—$C_4$ alkenyl, $C_3$—$C_4$ haloalkenyl, $C_3$—$C_4$ alkynyl, $C_3$—$C_4$ haloalkynyl, $C_2$—$C_4$ alkylcarbonyl, $C(O)NR_{15}R_{16}$, $C_1$—$C_4$ alkylsulfonyl, $C_2$—$C_4$ alkoxyalkyl or $C_2$—$C_4$ alkylthioalkyl;
$R_{15}$ and $R_{16}$ are independently H or $C_1$—$C_2$ alkyl;
$R_{17}$ is H or $C_1$—$C_4$ alkyl;

A is 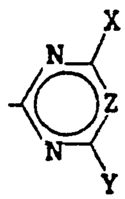 , 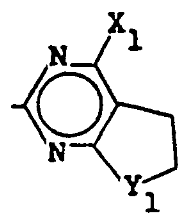 , 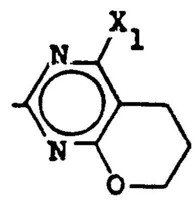 ,

A-1 A-2 A-3

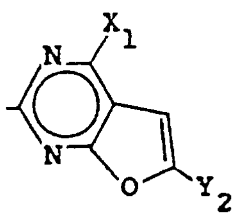 , 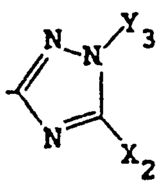 , 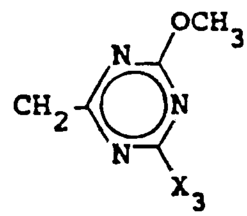 or

A-4 A-5 A-6

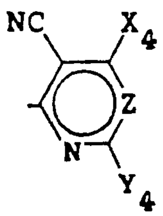 ;

A-7

X is H, $C_1$—$C_4$ alkyl, $C_1$—$C_4$ alkoxy, $C_1$—$C_4$ haloalkoxy, $C_1$—$C_4$ haloalkyl, $C_1$—$C_4$ haloalkylthio, $C_1$—$C_4$ alkylthio, halogen, $C_2$—$C_5$ alkoxyalkyl, $C_2$—$C_5$ alkoxyalkoxy, amino, $C_1$—$C_3$ alkylamino or di($C_1$—$C_3$ alkyl)-amino;
Y is H, $C_1$—$C_4$ alkyl, $C_1$—$C_4$ alkoxy, $C_1$—$C_4$ haloalkoxy, $C_1$—$C_4$ haloalkylthio, $C_1$—$C_4$ alkylthio, $C_2$—$C_5$ alkoxyalkyl, $C_2$—$C_5$ alkoxyalkoxy, amino, $C_1$—$C_3$ alkylamino, di($C_1$—$C_3$ alkyl)amino, $C_3$—$C_4$ alkenyloxy, $C_3$—$C_4$ alkynyloxy, $C_2$—$C_5$ alkylthioalkyl, $C_1$—$C_4$ haloalkyl, $C_3$—$C_5$ cycloalkyl,

$C_2$-$C_4$ alkynyl, 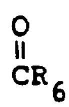 , 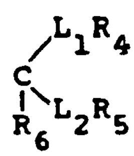 , 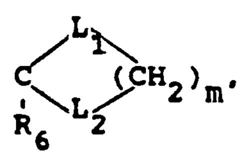 , 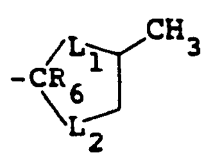

or $N(OCH_3)CH_3$;

m is 2 or 3;
$L_1$ and $L_2$ are independently O or S;
$R_4$ and $R_5$ are independently $C_1$—$C_2$ alkyl;
$R_6$ is H or $CH_3$;
Z is CH or N;
$Y_1$ is O or $CH_2$;
$X_1$ is $CH_3$, $OCH_3$, $OC_2H_5$ or $OCF_2H$;
$Y_2$ is H or $CH_3$;
$X_2$ is $CH_3$, $OCH_3$ or $SCH_3$;
$Y_3$ is $CH_3$, $CH_2CH_3$ or $CH_2CF_3$;
$X_3$ is $CH_3$ or $OCH_3$;
$Y_4$ is $CH_3$, $OCH_3$, $OC_2H_5$ or Cl; and

$X_4$ is $CH_3$, $OCH_3$, $OC_2H_5$ or $CH_2OCH_3$;
and their agriculturally suitable salts;
provided that

a) when X is Cl, F, Br or I, then Z is CH and Y is $OCH_3$, $OC_2F_5$, $N(OCH_3)CH_3$, $NHCH_3$, $N(CH_3)_2$ or $OCF_2H$;

b) when X or Y is $OCF_2H$ then Z is CH;

c) when L is L—2 or L—3, then the W substituent and the sulfonylurea bridge are on adjacent carbon atoms, and when L is L—9 or L—10, then Q and the sulfonylurea bridge are on adjacent carbon atoms;

d) when $W_1$ is $C_3$ alkenyl, then L is L—3, L—4, L—5 or L—6;

e) when L is L—1, then n is 1 and W is $W_2$;

f) when L is L—1 and $W_2$ is $C_2$ or $C_3$ alkynyl, said group must be substituted;

g) when $W_1$ is $C_3$—$C_8$ alkenyl or $C_4$—$C_7$ cycloalkenyl substituted with OH, then the OH substituent is not bonded directly to the olefinic group;

h) when L is L—3, then the sulfonylurea bridge is bonded at the 3- or 5-position;

i) when $W_3$ is S, then R is H, A is A—1, and Y is $CH_3$, $OCH_3$, $OC_2H_5$, $CH_2OCH_3$, $C_2H_5$, $CF_3$, $SCH_3$, $OCH_2CH{=}CH_2$, $OCH_2C{\equiv}CH$, $OCH_2CH_2OCH_3$ or $CH(OCH_3)_2$;

j) when the total number of carbon atoms of X and Y is greater than four, then the number of carbons of $R_1$ must be less than or equal to two and either the number of carbons of W must be less than or equal to four, or the number of carbons of Q must be less than or equal to six; and

k) the total number of carbon atoms of $R_{12}$ and $R_{13}$ must be less than or equal to four.

Provisos a), b), c), g), i), j) and k) are incorporated in order to exclude matter of no practical importance. The remaining provisos are incorporated to exclude matter disclosed in EP—A—41,404; US—A—4,368,069; EP—A—132,230; and ZA—86/6610.

In the above definitions, the term "alkyl" used either alone or in compound words such as "alkylthio" or "haloalkyl", denotes straight chain or branched alkyl, e.g., methyl, ethyl, *n*-propyl, isopropyl or the different butyl isomers.

Alkoxy denotes methoxy, ethoxy, *n*-propoxy, isopropoxy and the different butoxy isomers.

Alkenyl denotes straight chain or branched alkenes, e.g., vinyl, 1-propenyl, 2-propenyl, 3-propenyl and the different butenyl, pentenyl, hexenyl, heptenyl and octenyl isomers.

Alkynyl denotes straight chain or branched alkynes, e.g., ethynyl, 1-propynyl, 2-propynyl and the different butynyl, pentynyl, hexynyl, heptynyl and octynyl isomers.

Cycloalkenyl means cyclobutenyl, cyclopentenyl, cyclohexenyl and cycloheptenyl.

The term "halogen", either alone or in compound words such as "haloalkyl", denotes fluorine, chlorine, bromine or iodine.

In terms such as $C_2$—$C_3$ alkylthioalkyl, the specified number of carbon atoms is meant to define the *total* number of carbon atoms in that substituent group. For example, $C_2$—$C_3$ alkylthioalkyl would designate $CH_2SCH_3$, $CH_2SC_2H_5$, $CH_2CH_2SCH_3$ or $CH(CH_3)SCH_3$, and $C_2$—$C_5$ alkoxyalkoxy would represent $OCH_2OCH_3$ through $O(CH_2)_4OCH_3$ or $OCH_2O(CH_2)_3CH_3$ and the various structural isomers embraced therein.

Alkylsulfonyl denotes methylsulfonyl, ethylsulfonyl and the different propylsulfonyl isomers.

Alkylthio, alkylsulfinyl, alkylamino, alkylsulfamoyl, etc. are defined in an analogous manner.

Compounds of the invention preferred for reasons of increased ease of synthesis and/or greater herbicidal efficacy are:

1) Compounds of Formula I where
R is H;
$W_3$ is O; and
Q is

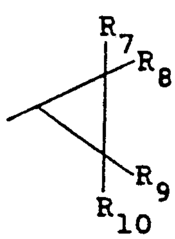
Q-1
,
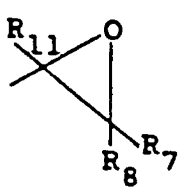
Q-2
,
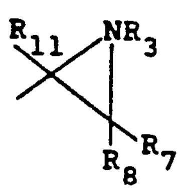
Q-3
,
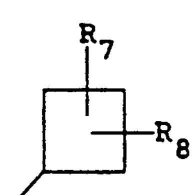
Q-4
,
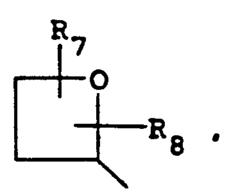
Q-5
,
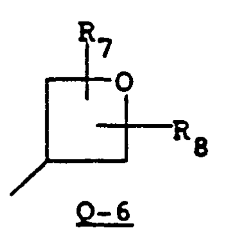
Q-6
,

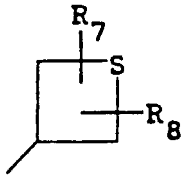
Q-7
,
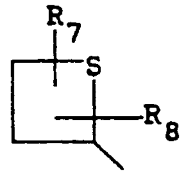
Q-8
,
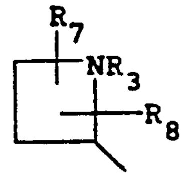
Q-9

or
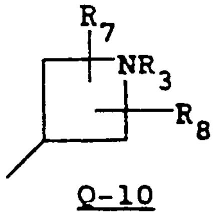
Q-10
;

wherein

$R_3$ is $C_1$—$C_3$ alkyl;

$R_7$ is H, F, Cl, Br, CN, $CH_3$ or $OCH_3$;

$R_8$ is H, F, Cl, Br, I, CN, OH, $C_1$—$C_3$ alkyl, $C_1$—$C_3$ alkoxy, $C_2$—$C_3$ alkoxycarbonyl, di($C_1$—$C_3$ alkyl)aminosulfamoyl, $C_1$—$C_3$ alkylthio or $C_1$—$C_3$ alkylsulfonyl;

$R_9$ and $R_{10}$ are independently H, $CH_3$, F, Cl or may be taken together to form C=O; and

$R_{11}$ is H or $C_1$—$C_3$ alkyl;

provided that

when $R_7$ and $R_8$ are attached to the same carbon atom as in Q—1, Q—2 or Q—3, or in Q—4 through Q—10, and $R_7$ is $OCH_3$, then $R_8$ is other than F, Cl, Br, I or OH.

2) Compounds of Preferred 1 where

$R_1$ is H, F, Cl, $NO_2$, $C_1$—$C_2$ alkyl, $C_1$—$C_2$ haloalkyl, $C_1$—$C_2$ alkoxy, $C_1$—$C_2$ alkylthio, $CH_2OCH_3$ or $CH_2SCH_3$, and when L is L—1, L—4, L—5, L—6, L—7, L—8, L—11, L—12 or L—13, $R_1$ is not *para* to the sulfonylurea bridge.

3) Compounds of Preferred 2 where

$R_{13}$ is H or $CH_3$;

$R_{14}$ is H, $C_1$—$C_2$ alkyl, $CH_2CH{=}CH_2$ or $CH_2C{\equiv}CH$;

X is $CH_3$, $OCH_3$, $OC_2H_5$, Cl, F, Br, $OCF_2H$, $CH_2F$, $OCH_2CH_2F$, $OCH_2CHF_2$, $OCH_2CF_3$, $CF_3$, $CH_2Cl$ or $CH_2Br$; and

Y is H, $C_1$—$C_3$ alkyl, $OCH_3$, $OC_2H_5$, $CH_2OCH_3$, $NHCH_3$, $N(OCH_3)CH_3$, $N(CH_3)_2$, $CF_3$, $SCH_3$, $OCH_2CH{=}CH_2$, $OCH_2C{\equiv}CH$, $CH_2OC_2H_5$, $OCH_2CH_2OCH_3$, $CH_2SCH_3$, $OCF_2H$, $SCF_2H$, cyclopropyl, $C{\equiv}CH$ or $C{\equiv}CCH_3$.

4) Compounds of Preferred 3 where

$W_1$ is $C_2$—$C_5$ alkenyl which may be optionally substituted by 1—3 atoms of F, Cl or Br, OH, $OCH_3$, $OC(O)CH_3$, $OSO_2CH_3$, CN or $SCH_3$, or $W_1$ is $C_5$—$C_6$ cycloalkenyl; and

$W_2$ is $C{\equiv}CC_6H_5$ or $C_3$—$C_5$ alkynyl which may be optionally substituted by 1—3 atoms of F, Cl or Br, OH, $OCH_3$, $OC(O)CH_3$, CN or $SCH_3$.

5) Compounds of Preferred 4 where

A is A—1;

X is $CH_3$, $OCH_3$, $OC_2H_5$, Cl or $OCF_2H$;

Y is $CH_3$, $C_2H_5$, $OCH_3$, $CH_2OCH_3$, $CH(OCH_3)_2$, $OCF_2H$, $NHCH_3$, $N(CH_3)_2$ or cyclopropyl; and

$R_1$ is H, $CH_3$, $OCH_3$, $SCH_3$ or Cl.

6) Compounds of Preferred 5 where

L is L—1.

7) Compounds of Preferred 5 where

L is L—2; and

W is $W_1$.

8) Compounds of Preferred 5 where

L is L—2; and

W is $W_2$.

9) Compounds of Preferred 5 where

L is L—3; and

W is $W_1$.

10) Compounds of Preferred 5 where

L is L—3; and

W is $W_2$.

11) Compounds of Preferred 5 where

L is L—4; and

W is $W_1$.

12) Compounds of Preferred 5 where
L is L—4; and
W is $W_2$.
13) Compounds of Preferred 5 where
L is L—5; and
W is $W_1$.
14) Compounds of Preferred 5 where
L is L—5; and
W is $W_2$.
15) Compounds of Preferred 5 where
L is L—6; and
W is $W_1$.
16) Compounds of Preferred 5 where
L is L—6; and
W is $W_2$.
17) Compounds of Preferred 5 where
L is L—7.
18) Compounds of Preferred 5 where
L is L—8; and
Q is Q—1, Q—2 or Q—4.
19) Compounds of Preferred 18 where
Q is Q—1.
20) Compounds of Preferred 19 where
$R_7$ is H, F, Cl, $CH_3$ or $OCH_3$;
$R_8$ is H, F, Cl, $CH_3$ or $OCH_3$; and
$R_9$ and $R_{10}$ are independently H, $CH_3$, F or Cl.
21) Compounds of Preferred 5 where
L is L—9 or L—10; and
Q is Q—1, Q—2 or Q—4.
22) Compounds of Preferred 21 where
$R_1$ is H;
$R_7$ is H, F, Cl, $CH_3$ or $OCH_3$;
$R_8$ is H, F, Cl, $CH_3$ or $OCH_3$;
$R_9$ and $R_{10}$ are independently H, $CH_3$, F or Cl; and
$R_{11}$ is H or $CH_3$.
23) Compounds of Preferred 5 where
L is L—11 or L—12; and
Q is Q—1, Q—2 or Q—4.
24) Compounds of Preferred 23 where
$R_1$ is H;
$R_7$ is H, F, Cl, $CH_3$ or $OCH_3$;
$R_8$ is H, F, Cl, $CH_3$ or $OCH_3$;
$R_9$ and $R_{10}$ are independently H, $CH_3$, F or Cl; and
$R_{11}$ is H or $CH_3$.
25) Compounds of Preferred 5 where
L is L—13; and
Q is Q—1, Q—2 or Q—4.
26) Compounds of Preferred 25 where
$R_1$ is H;
$R_7$ is H, F, Cl, $CH_3$ or $OCH_3$;
$R_8$ is H, F, Cl, $CH_3$ or $OCH_3$;
$R_9$ and $R_{10}$ are independently H, $CH_3$, F or Cl; and
$R_{11}$ is H or $CH_3$.

Compounds of the invention specifically preferred for reasons of their expected greatest ease of synthesis and/or greatest herbicidal efficacy are:

2-(1-cyclohexen-2-yl)-N-[4-methoxy-6-methylpyrimidin-2-yl)aminocarbonyl]-3-thiophenesulfonamide, m.p. 190°—192°C;

2-(1-cyclohexen-2-yl)-N-[4-methoxy-6-methyl-1,3,5-triazin-2-yl)aminocarbonyl]-3-thiophenesulfonamide, m.p. 169°—171°C;

N-[(4-methoxy-6-methylpyrimidin-2-yl)aminocarbonyl]-1-methyl-4-(1-propenyl)1H-pyrazole-5-sulfonamide, m.p. 155°—158°C.

2-(2,2-dichlorocyclopropyl)-N-[(4,6-dimethoxypyrimidin-2-yl)aminocarbonyl]benzenesulfonamide, m.p. 193°—195°C;

2-(2,2-dichlorocyclopropyl)-N-[(4-methoxy-6-methyl-1,3,5-(triazin-2-yl)-aminocarbonyl]benzenesulfonamide, m.p. 168°—170°C); and

N-[(4,6-dimethoxypyrimidin-2-yl)aminocarbonyl]-2-(2-oxiranylmethyl)benzenesulfonamide, m.p. 157°—159°C(d).

Other compounds of interest within the scope of Formula I include those disclosed in our U.S. Patent Applications Serial Nos. 680,549; 769,691 and 768,158. Copies of these U.S. Applications are available for inspection on the file of the present Application.

## Detailed Description of the Invention

### Synthesis

The compounds of Formula I can be prepared by reacting an appropriate sulfonyl isocyanate or sulfonyl isothiocyanate, II, with an appropriately substituted aminoheterocycle, III, as shown in Equation 1, where R, $W_3$, A and L are as previously defined.

Equation 1

$$\underset{\underline{III}}{RNHA} + \underset{\underline{II}}{LSO_2NCW} \xrightarrow[\text{24 hours}]{25\text{—}80°C} \underline{I}$$

The reaction is best performed in an inert solvent such as methylene chloride or toluene at 25 to 100°C. for 1 to 24 hours. Isolation of the product can be achieved by concentrating the solution and trituration with an appropriate solvent such as butyl chloride.

Alternatively, compounds of Formula I, where $W_3$ is O, can be prepared by reacting the sulfonamides of Formula IV with the carbamates of Formula V ($R'=CH_3$) in the presence of an excess of trimethylaluminum, as shown in Equation 2, where L, R and A are as previously defined, provided $R_1$ is other than $CO_2R'''$.

Equation 2

$$\underset{\underline{IV}}{LSO_2NH_2} + \underset{\underline{V}}{R'O\overset{\overset{\large O}{\|}}{C}\text{—}NRA} \xrightarrow[CH_2Cl_2]{Me_3Al} \underline{I}$$

The reactions are best performed in an inert solvent such as methylene chloride at the reflux point of the solution (40°C) for 10 to 24 hours. Isolation of the product is best achieved by exposing the reaction mixture to acetic acid, separation of the layers and concentrating the organic layer to a solid.

Alternatively, compounds of Formula I can be prepared by exposing a phenyl carbamate V ($R'=Ph$) to the sulfonamide IV in an appropriate solvent such as dioxane at 25 to 100°C in the presence of a strong base such as 1,8-diazabicyclo[5.4.0]undec-7-ene; acid workup affords the desired product, as disclosed in European Patent Application (EP—A) 44,807. Compounds of Formula I can also be prepared from a phenyl carbamate V and the *tert*-butyldimethylsilyl-protected sulfonamide IV ($LSO_2NHSi(CH_3)_2$-*t*-Bu). Addition of tetrabutylammonium fluoride to a mixture of the carbamate and sulfonamide affords after workup the desired product. The required carbamates can be prepared from the corresponding amines, III, and dimethyl or diphenylcarbonate or methyl or phenylchloroformate and a base such as sodium hydride.

The sulfonyl isocyanates, II, used in the preparation of I are known in the art and can be prepared by known methods. For example, isocyanates can be prepared by exposing an appropriate benzene or heterocyclic sulfonamide to phosgene in the presence of an alkyl isocyanate and an amine catalyst such as 1,4-diazabicyclo[2.2.2]octane at the reflux point of the solvent; see H. Ulrich and A. A. Y. Sayigh, *Newer Methods of Preparative Organic Chemistry,* Vol. VI, p. 223—241, Academic Press, New York and London, W. Forest Ed.

The sulfonyl isothiocyanates, II, used in the preparation of I, where W is S, can be prepared according to the procedure of K. Hartke, *Arch. Pharm., 299,* 174 (1966).

Sulfonamides of Formula L—8 (compound 3) where Q is Q—1, n=O, $R_1$, and $R_7$ through $R_{10}$ are as previously defined can be prepared in one or more of the ways outlined in Equations 3 through 7.

Equation 3

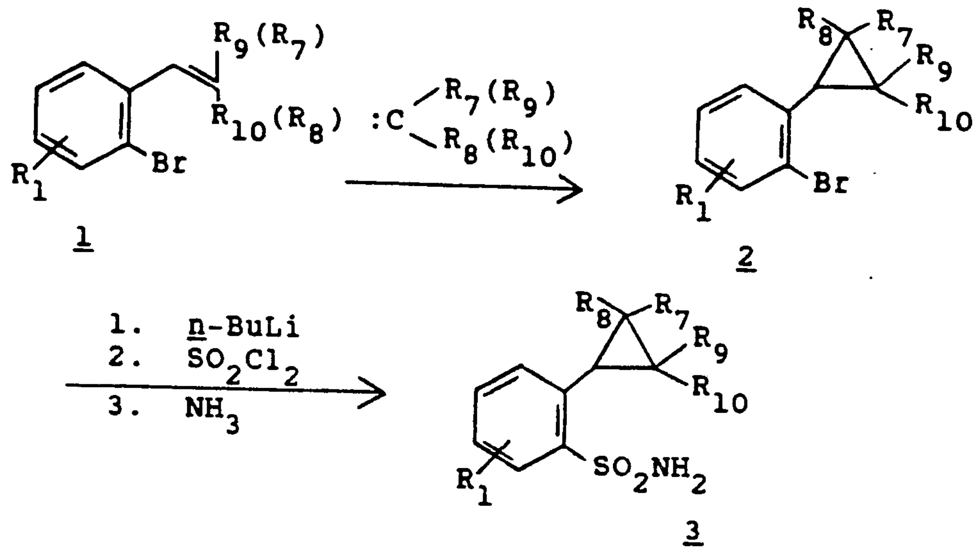


1 2 3

The addition of carbenes to styrene is well known in the art. For further details of such transformations see Keller, W. E., *"Compendium of Phase-Transfer Reactions and Related Synthetic Methods"*, Fluka A. G., Switzerland, p 39—64, 1979.

Equation 4

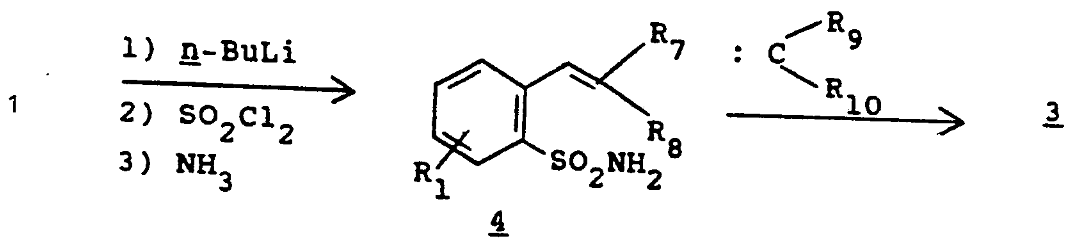


1 4 3

An alternate sequence, outlined below in Equation 5, can be employed in selected cases where lithiation of 2 may be a problem due to functional group incompatibility with *n*-BuLi, (i.e., $R_7=CO_2CH_3$).

Equation 5

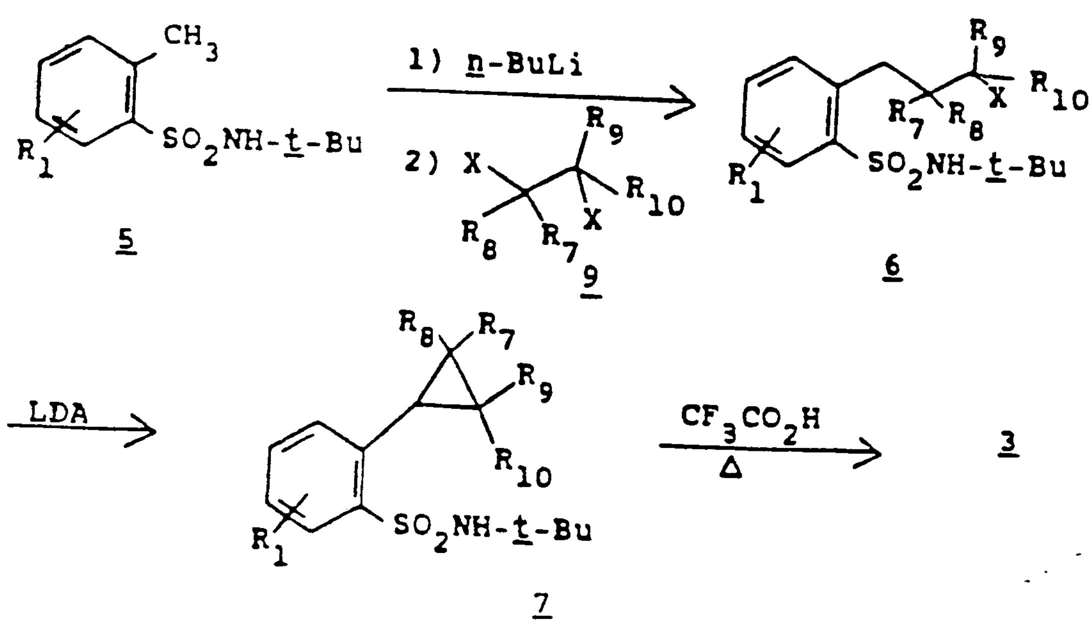


5 9 6 7 3

The sulfonamide 3 can also be prepared by direct alkylation with the preformed cyclopropyl methyl halide *8* as outlined in Equation 6.

Equation 6

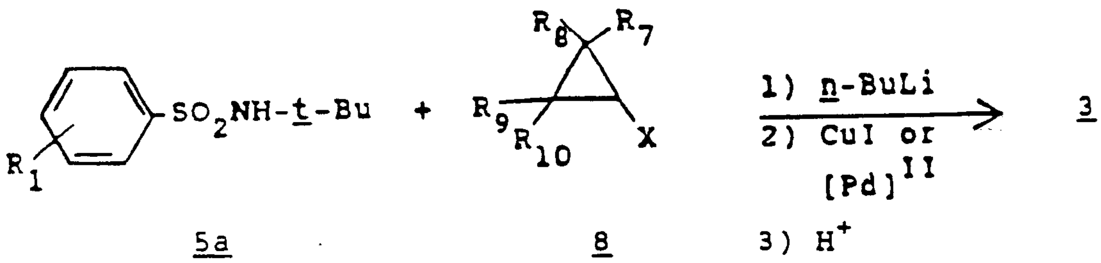


5a 8 3

For further details pertaining to this modification of the Ullmann reaction see, S. Gronwitz, and S. Lilgefors, *Chemica Scripta, 13,* 157—161 (1978—79) and A. Minato, K. Tamo, T. Hayasi, K. Suzuki and M. Kumada; *Tetrahedron Letters, 22,* 5319 (1981).

In Equation 5 the dihaloethane 9 may be replaced with other electrophiles such as propylene oxide and its derivatives, as shown in Equation 7.

Equation 7

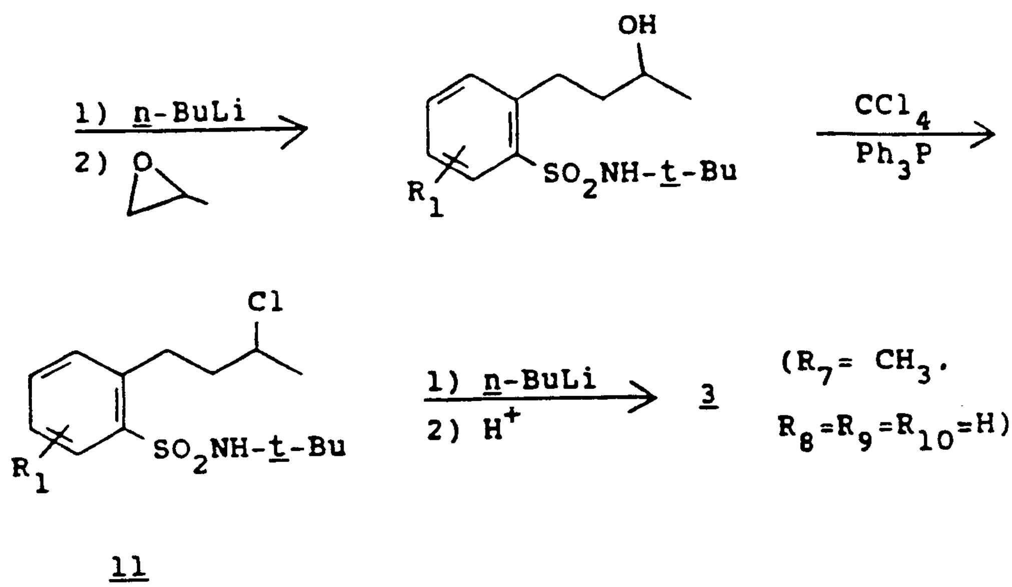


3 ($R_7$= $CH_3$, $R_8$=$R_9$=$R_{10}$=H)

11

The ortho-lithiation of compounds such as 1, 2, 5 and 5a is well known to those skilled in the art. For further details pertaining to this type of transformation see Meyers, A. I., Michlich, B. D., and Nolan, R. L., *J. Org. Chem., 39,* 2783, 1974.

Sulfonamides of Formula L—8 (Compound 12) where n is 1, Q is Q—1 through Q—3 and $R_1$, $R_7$ through $R_{10}$ and $R_{12}$ and $R_{13}$ are as previously defined can be prepared in a similar manner to that already described in Equations 3, 4 and 6, with slight modifications of the starting material. For example cyclopropanation of

the styrene homolog 13, followed by ortho-lithiation chemistry would afford the target Compound 12 as outlined in Equation 8.

Equation 8

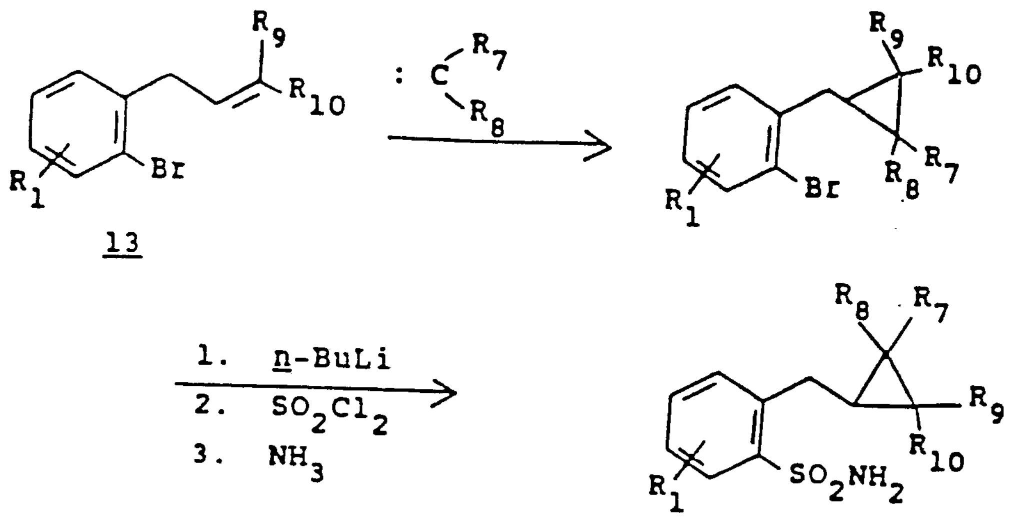


13

12

In an analogous manner to Equation 6, cyclopropane 12 can be prepared as outlined in Equation 9.

Equation 9

5a

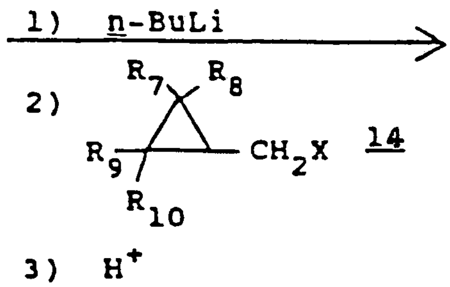


14

12

Alternatively, sulfonamide 12 can be prepared by a modified Ullmann reaction utilizing sulfonamide 5 as the starting material as outlined in Equation 10.

Equation 10

5 → 12

1) *n*-BuLi/CuI

2) 8

3) $H^+$

The epoxy sulfonamides of Formula L—8 (Compound 15) where Q is Q—2 and n, $R_1$, $R_7$, $R_8$ and $R_{11}$ are as previously defined can be prepared directly from olefins 1, 4 and 13 via epoxidation using *m*-chloroperbenzoic acid as described in Equation 11.

Equation 11

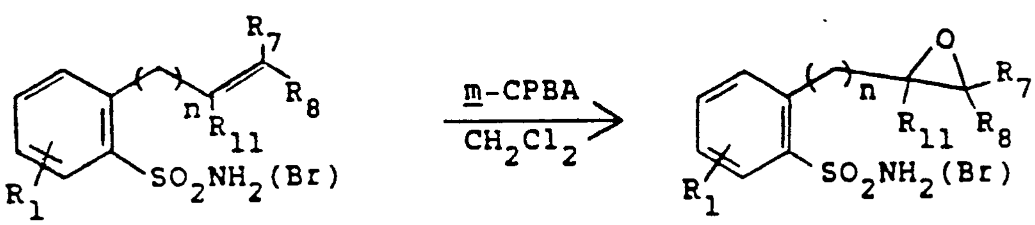


15

Further details pertaining to olefin epoxidations may be found in *Org. Syn.,* Coll. Vol. *1,* 494 (1944).

The aziridine sulfonamides where Q is Q—3 and n, $R_1$, $R_3$, $R_7$, $R_8$ and $R_{11}$ are as previously defined such as compound 16 can also be prepared from the corresponding olefins using the procedure outlined in Equation 12.

Equation 12

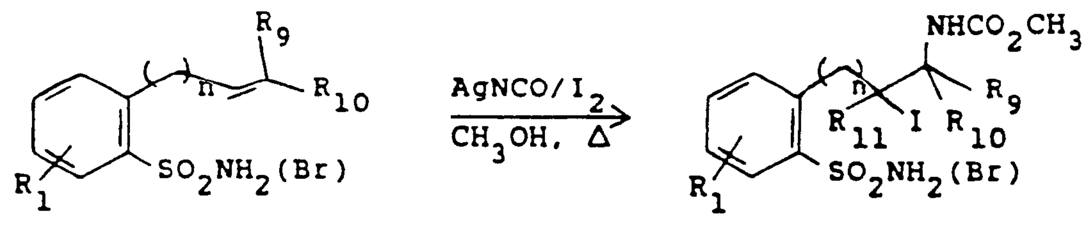


17

17

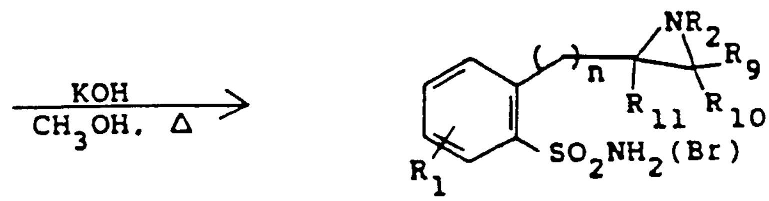


16

For further details see Gebelein, C. O., Swift, G., Swern, D., *J. Org. Chem., 32,* 3314 (1967).

Sulfonamides of Formula L—9, L—10, L—11, L—12 and L—13, where Q is Q—1 through Q—3 and n, $R_1$, $R_2$, $R_3$ and $R_7$ through $R_{13}$ are as previously defined can be prepared using one or more of the routes previously outlined in Equations 3 through 12.

For example, in the case of L—10, sulfonamide 18 can be prepared using the same route described in Equation 3 or 4. This is outlined in Equation 13.

Equation 13

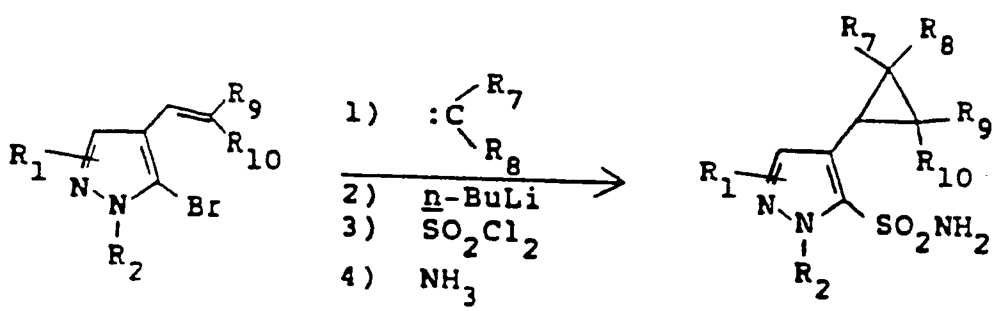


19

18

Sulfonamides (such as 20a—20d) containing a four-membered ring where $R_1$, $R_7$ and $R_8$ are as previously defined may be prepared in one or more of the ways outlined in Equations 14 through 17b.

Equation 14

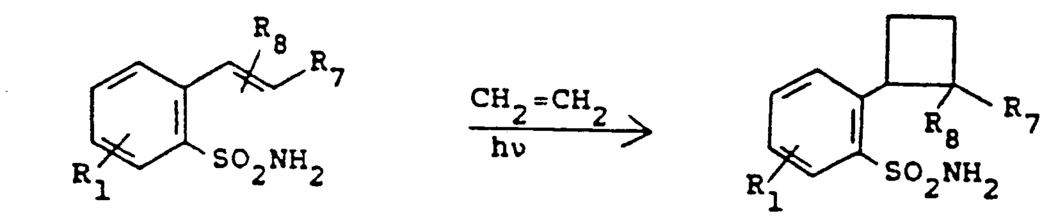


21 20a

or

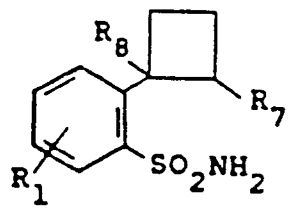

20b

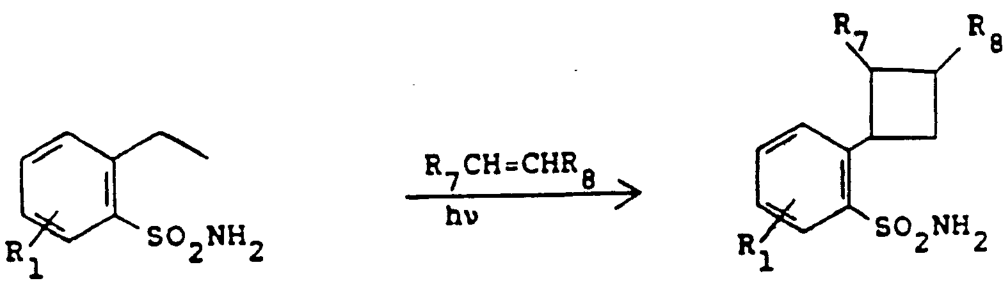


22 20c

or

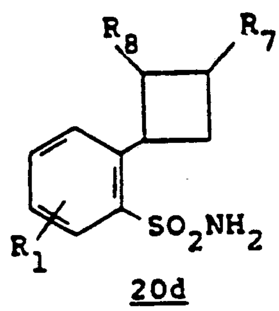

20d

Equation 15

22

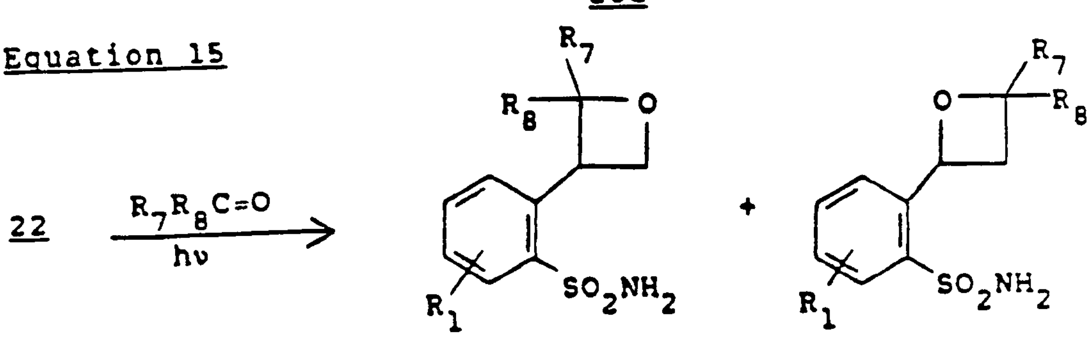


23 24

For a discussion of photochemical 2+2 cycloadditions of styrene with olefins, aldehydes and ketones (the Paterno-Buchi reaction) see Silversmith, Kitahara, Caserio and Roberts, *J. Am. Chem. Soc., 80,* 5840(1958).

An alternate synthesis of compounds such as 20a—20d is via the anionic ring closure of chloride 25 as outlined in Equation 16.

Equation 16

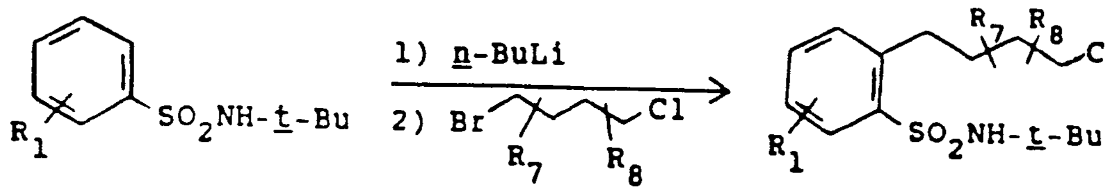


25

25 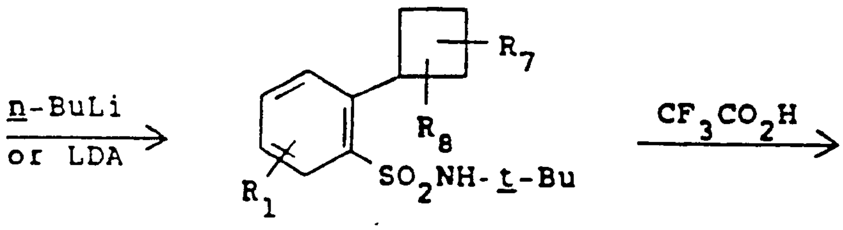

20

In a similar manner the oxetane ring systems can be formed as shown in Equation 17a and 17b.

Equation 17a

5a 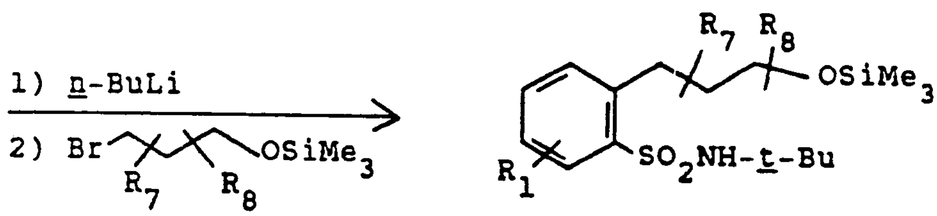


26

26 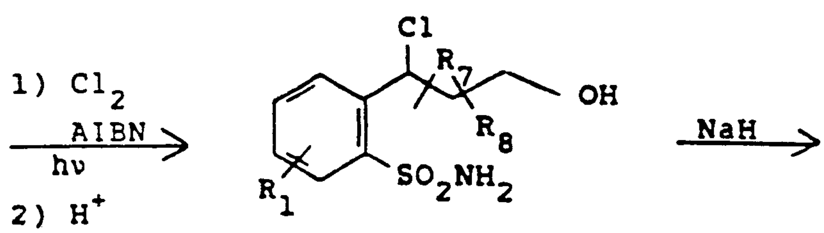

24

27

Equation 17b

5a 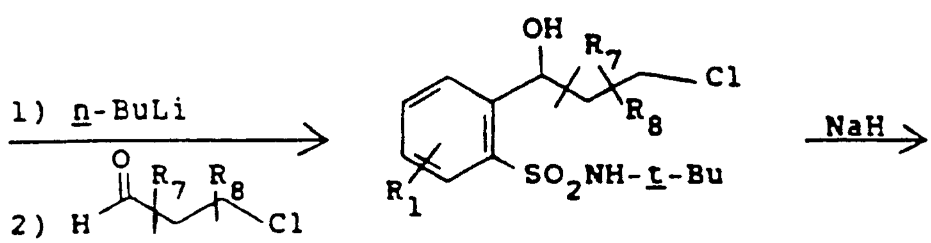

24

28

The methodology described in Equations 15, 17a and 17b can also be used to prepare the thio and amine analogs, that is sulfonamides of Formula L—8 where Q is Q—7 through Q—10 and $R_1$, $R_3$, $R_7$ and $R_8$ are as previously defined, and n=0 (i.e., 29 and 30).

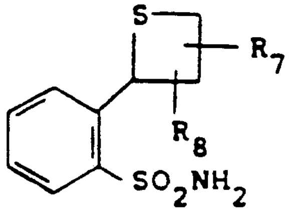

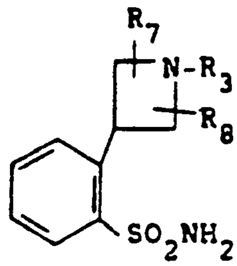

29 30

Examples of anionic four membered ring closures may be found in Corey, E. J. and Seebach, D., *Org. Synth., 50,* 72 (1970).

Sulfonamides of Formula L—9, L—10, L—11, L—12 and L—13 where Q is Q—4 through Q—10 and R, $R_1$, $R_2$, $R_3$, $R_7$ and $R_8$ are as previously defined can be prepared in a similar manner to that already taught for L—8 in Equations 14 through 17b utilizing the appropriate starting sulfonamides (e.g. 19) as described previously.

In the case where n is 1 the corresponding sulfonamides such as 31 and 32 can be prepared by the procedures outlined in Equation 18a and 18b.

Equation 18a

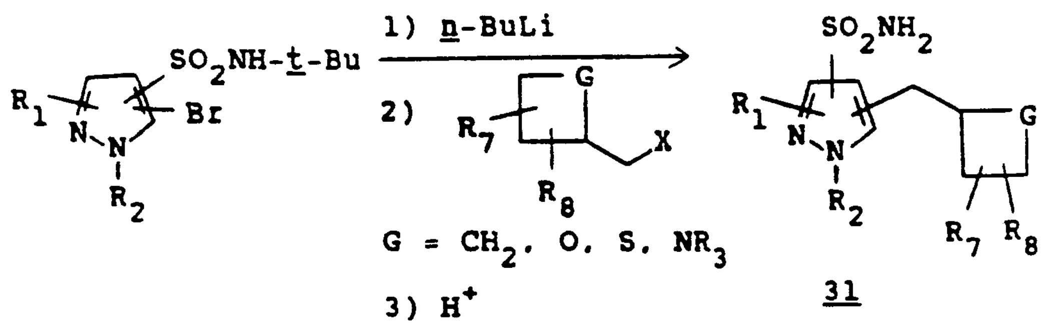


Equation 18b

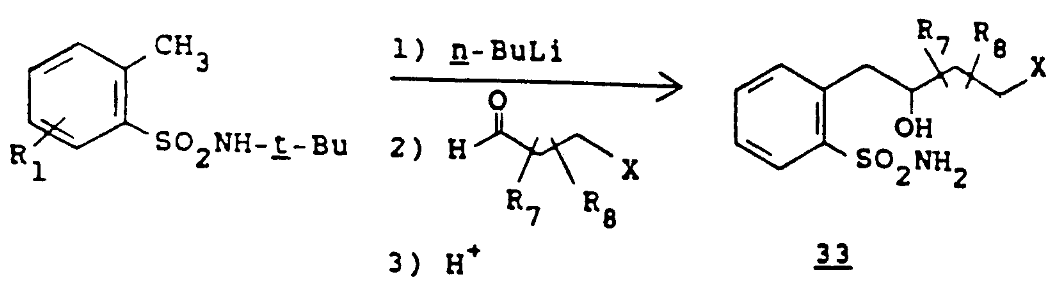


33

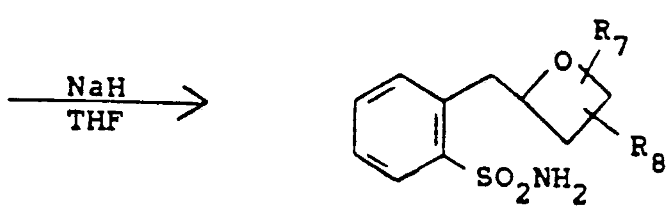


32

Sulfonamide 34 where $R_{12}$ is OH, $R_{13}$ is H (n=1) can be prepared as outlined in Equation 19.

Equation 19

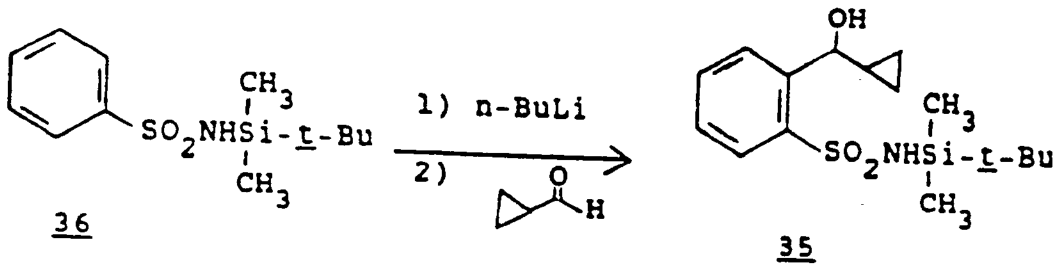


36

35

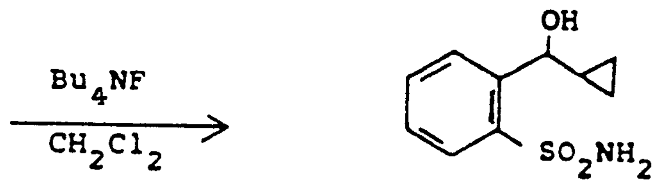


35

34

The use of the tert-butyldimethylsilyl group allows for low temperature generation of the anion and mild removal of the protecting group. In the same manner alkylation of 36 with ketones such as cyclobutanone allows for the preparation of sulfonamide 37 as outlined in Equation 20.

Equation 20

36

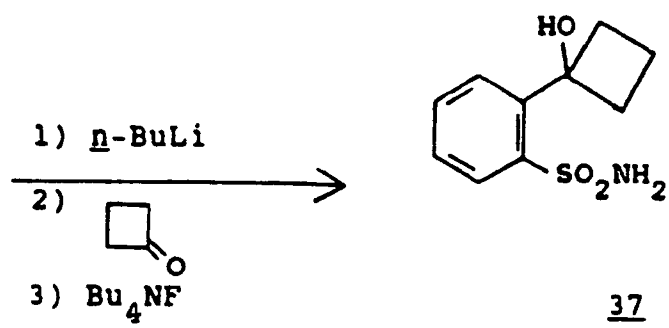


37

Preparation of a heterocyclic sulfonamide wherein $R_{12}$ is H and $R_{13}$ is OH (n=1) is accomplished in the same way as already taught for an aromatic system in Equations 19 and 20. For example, sulfonamide 38 can be prepared from protected pyrazole sulfonamide 39 as outlined in Equation 21.

Equation 21

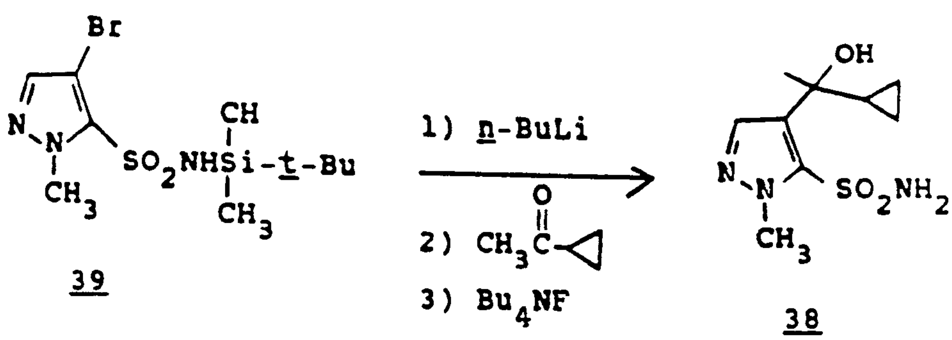


39

38

Sulfonamides such as 34 or 38 can be used to prepare sulfonamides such as 40. Generation of the alkoxide with sodium hydride followed by the addition of the appropriate alkylating group affords protected alcohols such as 40.

Equation 22

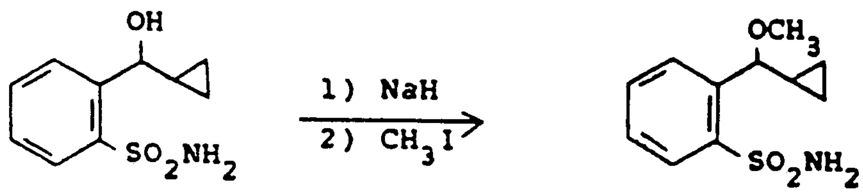


34 40

An alternate preparation of protected alcohols, for example 41, is to trap directly the incipient lithium alkoxide (42) resulting from the condensation of the anion of 36 with aldehydes and ketones. This is depicted in Equation 23.

Equation 23

36

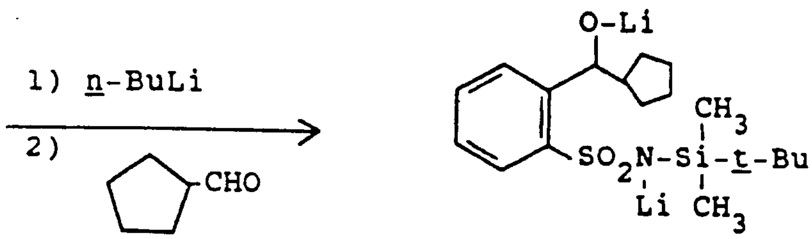


42

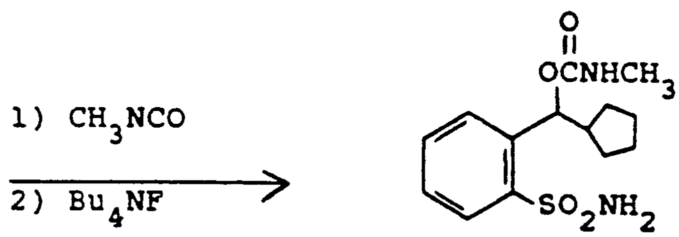


41

The intermediate sulfonamides IV where L is L—1 and W is as previously defined, for example 43, may be prepared from 5a via the lithiation, condensation protocol outlined in Equation 24.

Equation 24

5a

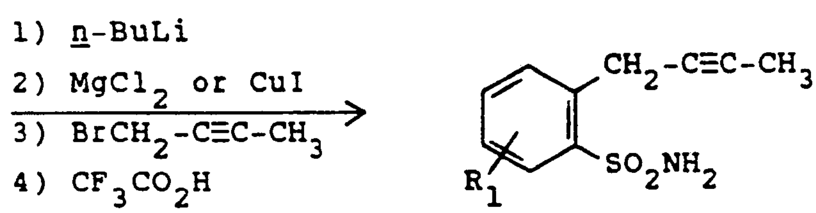


43

This same methodology can also be applied to other sulfonamides such as thiophene 44 and pyrazole 45 as outlined in Equation 25. The examples depicted are meant to be exemplary and the methodology is applicable to a wide range of values for W and the various aromatic thiophene, pyrazole, pyridine and benzyl isomers.

Equation 25

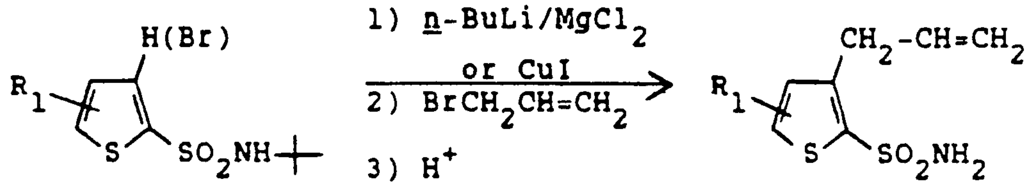


44a 44

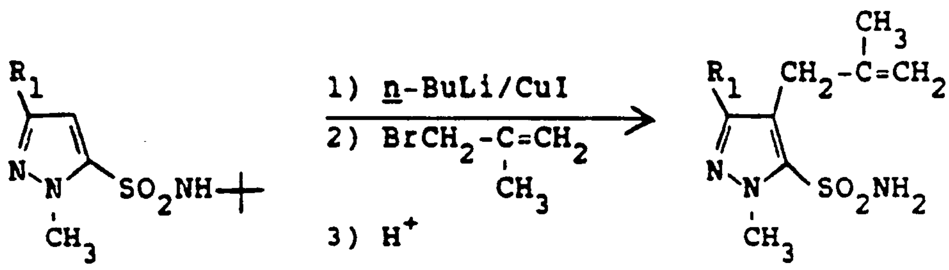


44b 45

Sulfonamides such as 43, 44 and 45 can also be prepared from the corresponding olefin or alkyne as outlined in Equation 26, where W is as previously defined.

Equation 26

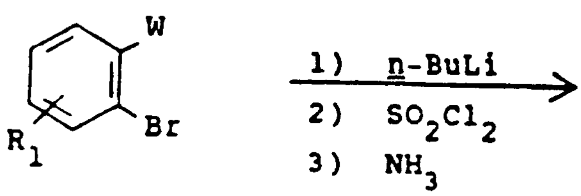


43

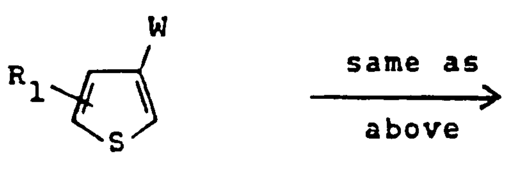


44

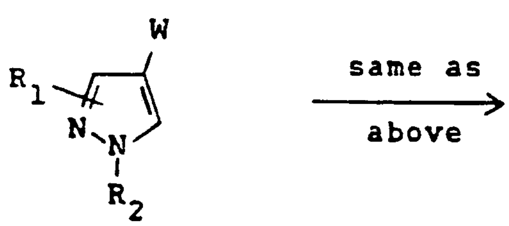


45

Olefinic sulfonamides such as 46 can be prepared from the methyl-substituted *t*-butylsulfonamide 47, as outlined in Equation 27. Here again, the example is intended to be exemplary and would be applicable to a wide variety of values for $R_1$ and $W_1$.

Equation 27

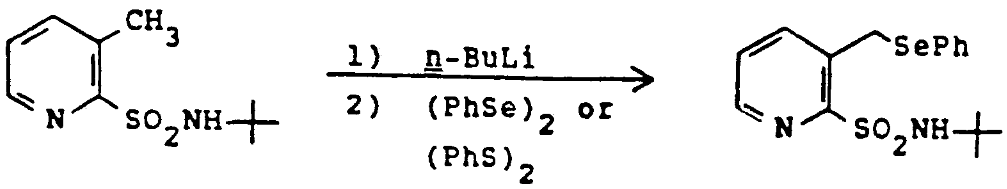


47 48

48

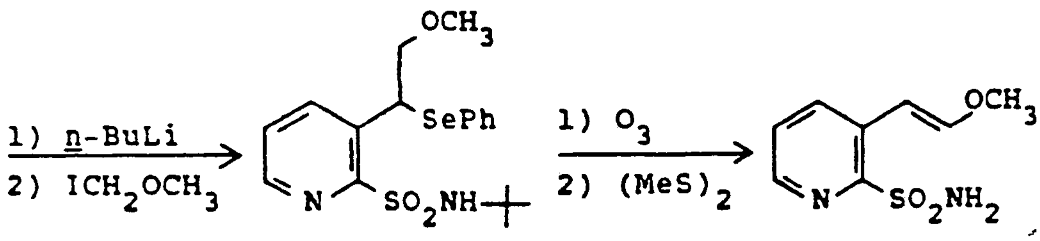


46

The sulfonamides IV of the invention such as 49 or 50 can also be prepared via a modification of the Ullman reaction as outlined in Equation 28.

Equation 28

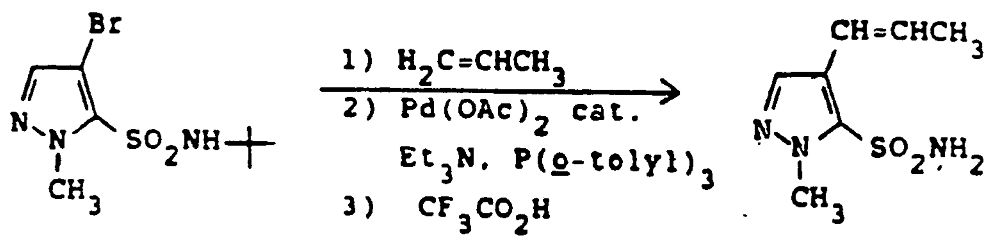


51 49

51

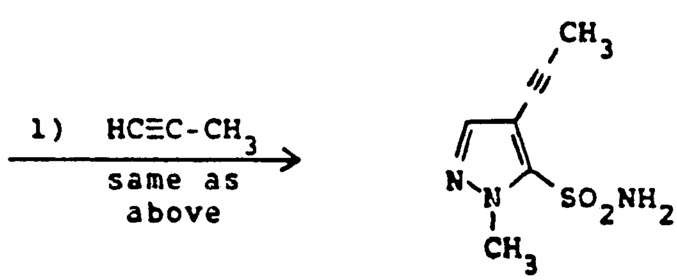


50

Intermediates such as 50 can also be used to prepare the corresponding olefins such as 49 via catalytic hydrogenation, employing a catalyst such as Lindlar catalyst.

Condensation of protected sulfonamides, for example 44c, with ketones and aldehydes followed by elimination also affords the desired sulfonamides. This is outlined in Equation 29.

Equation 29

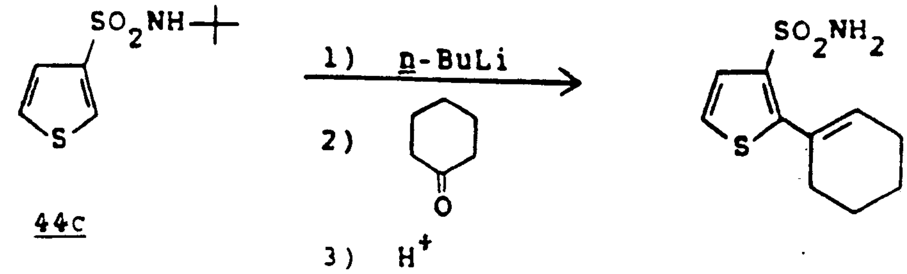


44c

Sulfonamides such as 44d can be prepared via the aldehyde 44e by a Knoevenagel type condensation with active methylene compounds as shown in Equation 30. Appropriate active methylene compounds include malonic esters, malononitrile, α-cyanoacetates, α-nitroacetates to name a few.

Equation 30

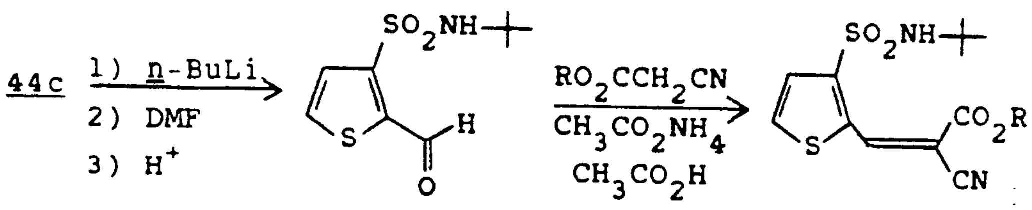


44e

44d

Similar condensation with pyrazole, pyridine or benzene aldehydes provides access to the corresponding difunctionalized alkenyl sulfonamides.

One skilled in the art will recognize that many of the methods described above can result in the formation of two possible geometrical isomers about the carbon-carbon double bond. This invention is meant to encompass all isomers. In cases where a mixture is obtained, the isomers can be separated by standard methods, such as fractional recrystallization or chromatography. Alternatively, the compounds can be used as a mixture of double bond isomers.

The lithiation of both aromatic and heterocyclic compounds is well known to one skilled in the art. For further information pertaining to this methodology see Stowell, J. C. "Carbanions in Organic Synthesis", John Wiley & Sons: New York, 1979; Snieckus, V. *Tetrahedron Lett.* 26, 1145 (1985) and *ibid,* 1149 (1985).

For further information pertaining to the synthesis of pyrazole sulfonamides see: EP—A—95,925; for thiophene sulfonamides, EP—A—30,142; for pyridine sulfonamides, EP—A—13,480, and benzyl sulfonamides, EP—A—51,466 and U.S. 4,420,325.

The heterocyclic amines of Formula III can be prepared by methods known in the literature or simple modifications thereof, by those skilled in the art. For instance, EP—A—84,224 (published July 27, 1983) and W. Braker et al, *J. Am. Chem. Soc.* 1947, *69,* 3072 describe methods for preparing aminopyrimidines and triazines substituted by acetal groups. Also, South African Patent Application Nos. 825,045 and 825,671 describe methods for preparing aminopyrimidines and triazines substituted by haloalkyl or haloalkylthio groups such as $OCH_2CH_2F$, $OCH_2CF_3$, $SCF_2H$, and $OCF_2H$ among other groups. South African Patent Application 837,434 (published October 5, 1983) describes methods for the synthesis of cyclopropylpyrimidines and triazines substituted by such groups as alkyl, haloalkyl, alkoxy, haloalkoxy, alkylamino, dialkylamino, and alkoxyalkyl.

The 5,6-dihydrofuro[2.3-d]pyrimidin-2-amines, and the cyclopenta[d]pyrimidin-2-amines, of Formula III, where A is A—2 and the 6,7-dihydro-5H-pyrano-[2.3-d]pyrimidin-2-amines, of Formula III, where A is A—3, can be prepared as described in EP—A No. 15,863. The furo[2.3-d]pyrimidin-2-amines, of Formula III, where A is A—4, are described in EP—A No. 46,677. Heterocycles of Formula III, where A is A—5, can be prepared as described in EP—A No. 73,562. Heterocycles of Formula III, where A is A—6, can be prepared by methods taught in EP—A—94,260. Heterocycles of Formula III, where A is A—7, can be prepared by methods taught in EP—A—125,864.

In addition, general methods for preparing aminopyrimidines and triazines have been reviewed in the following publications.

"The Chemistry of Heterocyclic Compounds", a series published by Interscience Publishers, Inc., New York and London;

"Pyrimidines", Vol. 16 of the same series by D. J. Brown;

"*s*-Triazines and Derivatives", Vol. 13 of the same series by E. M. Smolin and L. Rapaport; and

F. C. Schaefer, U.S. Patent 3,154,547 and K. R. Huffman and F. C. Schaefer, *J. Org. Chem., 28,* 1812 (1963), which describe the synthesis of triazines.

Agriculturally suitable salts of compounds of Formula I are also useful herbicides and can be prepared in a number of ways known to the art. For example, metal salts can be made by contacting compounds of Formula I with a solution of an alkali or alkaline earth metal salt having a sufficiently basic anion (e.g., hydroxide, alkoxide or carbonate). Quaternary amine salts can be made by similar techniques.

Salts of compounds of Formula I can also be prepared by exchange of one cation for another. Cationic exchange can be effected by direct contacting of an aqueous solution of a salt of a compound of Formula I (e.g., alkali metal or quaternary amine salt) with a solution containing the cation to be exchanged. This method is most effective when the desired salt containing the exchanged cation is insoluble in water, e.g., a copper salt, and can be separated by filtration.

Exchange may also be effected by passing an aqueous solution of a salt of a compound of Formula I (e.g., an alkali metal or quaternary amine salt) through a column packed with a cation exchange resin containing the cation to be exchanged. In this method, the cation of the resin is exchanged for that of the original salt and the desired product is eluted from the column. This method is particularly useful when the desired salt is water-soluble, e.g., a potassium, sodium or calcium salt.

Acid addition salts, useful in this invention, can be obtained by reacting a compound of Formula I with a suitable acid, e.g., *p*-toluenesulfonic acid, trichloroacetic acid or the like.

The preparation of the compounds of this invention is illustrated by the following examples.

Example 1

Preparation of 2-Ethenylbenzenesulfonamide

2-Bromostyrene (10.0 g, 54.9 mmol) was added dropwise to *n*-BuLi in 250 ml of tetrahydrofuran at −78°C. After stirring for 15 minutes the solution was added via a cannula to a solution of sulfurylchloride fluoride (9.7 g, 82.4 mmol) in 50 ml of tetrahydrofuran cooled to −78°C. The reaction mixture was quenched with brine and separated. The organic layer was dried and concentrated to approximately half-volume. This solution was added to anhydrous ammonia (100 ml) cooled to −78°C. The reaction was warmed to room temperature, excess ammonia was removed and the solids were filtered off. The filtrate was concentrated and the resulting oil was triturated with butyl chloride and ethyl acetate to yield the desired sulfonamide, m.p. 103—105°C.

Example 2

Preparation of 2-(2-Oxiranyl)benzenesulfonamide

A solution consisting of 2-ethenylbenzenesulfonamide (1.5 g, 8.1 mmol) *m*-chloroperbenzoic acid (1.4 g, 8.1 mmol) and potassium carbonate (1.1 g, 8.1 mmol) in 150 ml of chloroform was stirred for 4 days at room temperature. The reaction was filtered and the filtrate was washed with saturated sodium bicarbonate. The organic layer was separated, dried and concentrated. The resulting oil was flash chromatographed (methylene chloride) to yield the subject compound as a white solid, m.p. 136—139°C.

Example 3

Preparation of 1-Bromo-2-(2,2-Dichlorocyclopropyl)-benzene

A 20 ml solution of 50% sodium hydroxide was added dropwise to 2-bromostyrene (10.0 g, 54.9 mmol) in 100 ml of chloroform. The reaction mixture was warmed to 45°C for 1 hour. After cooling the solution was separated and the organic layer was dried over magnesium sulfate and concentrated. The resulting liquid was flash chromatographed, affording 11.6 g of the desired compound.

Example 4

Preparation of N-[(4,6-Dimethoxypyrimidin-2-yl)aminocarbonyl]-2-(2-oxiranyl)benzenesulfonamide

To a stirring solution of the 2-(2-oxiranyl)benzenesulfonamide (75 mg, 0.376 mmol) and 4,6-dimethoxy-2-aminophenoxycarbonyl pyrimidine (103 mg, 0.37 mmol) in 5 ml of acetonitrile was added 1,8-diazabicyclo-[5.4.0]undec-7-ene (57 mg, 0.37 mmol). The reaction was stirred for 3 hours followed by quenching with 3 ml of 5% hydrochloric acid. The solution was diluted with ethyl acetate, separated and dried. The organics were concentrated to yield 140 mg of the desired product, m.p. 177—179°C; NMR (200 MHz, $CDCl_3$)δ: 2.3 (dd, 1H); 3.1 (dd, 1H); 3.95 (s, 6H); 4.7 (dd, 1H); 5.88 (s, 1H); 7.0 (br, s, 1H); 7.55 (m, 3H) and 8.2 (dd, 1H).

Example 5

Preparation of 2-(2,2-Dichlorocyclopropyl)-N-[(4-methoxy-6-methylpyrimidin-2-yl)aminocarbonyl]-benzenesulfonamide

To a stirring solution of 2-(2,2-dichlorocyclopropyl)benzenesulfonamide (200 mg, 0.75 mmol) and 4-methyl-6-methoxy-2-aminophenoxycarbonyl pyrimidine (195 mg, 0.75 mmol) was added 1,8-diazabicyclo[5.4.0]-undec-7-ene (115 mg, 0.75 mmol). Work-up as described in Example 4 afforded 180 mg of the desired compound, m.p. 187—191°C; NMR (200 MHz, $CDCl_3$)δ: 2.05 (dd, 2H); 2.41 (s, 3H); 3.79 (dd, 1H); 3.94 (s, 3H); 6.27 (s, 1H); 7.2 (d, 1H); 7.57 (m, 3H) and 8.4 (dd, 1H).

Example 6

2-[(Cyclopropyl)(hydroxy)methyl]-N-[(dimethyl((1,1-dimethylethyl)silyl]benzenesulfonamide

To a stirring solution of *n*-BuLi (5.2 g, 80.8 mmol) in 300 ml of THF cooled to −10°C was added dropwise *tert*-butyldimethylsilylbenzenesulfonamide (10.0 g, 36.7 mmol) in 25 ml of THF. The solution was stirred for one hour. After cooling to −78°C, cyclopropyl aldehyde (2.8 g, 40.0 mmol) was added. The reaction mixture was stirred for 30 minutes, then quenched with brine. The organic layer was separated, dried over magnesium sulfate and concentrated *in vacuo.* Flash chromatography (20% EtOAc:hexane, v/v) afforded 2.7 g of the desired product as a viscous oil; NMR (200 MHz, $CDCl_3$): δ 0.24 (d, 6H), 0.4 (m, 1H), 0.45—0.8 (m, 3H), 0.88 (s, 9H)), 1.6 (m, 1H), 2.85 (d, 1H), 4.9 (dd, 1H), 5.30 (s, 1H), 5.67 (brs, 1H), 7.41 (t, 1H), 7.54 (t, 1H), 7.76 (d, 1H), 7.98 (d, 1H).

Example 7

N-[(4,6-Dimethylpyrimidin-2-yl)aminocarbonyl]-2-(1-hydroxycyclobutyl)benzenesulfonamide

To a mixture of the phenylcarbamate of 4,6-dimethyl-2-aminopyrimidine (178 mg, 0.73 mmol) and tert-butyldimethylsilane-protected 2-(1-hydroxycyclobutane)benzenesulfonamide in 5 ml of acetonitrile was added tetrabutylammonium fluoride (231 mg, 0.73 mmol). The reaction mixture was stirred for 5 minutes followed by the addition of 5% HCl. The resulting solids were collected and dried to give 250 mg of the desired product; m.p. 148—150°C; NMR (200 MHz, $CDCl_3$); δ 2.45 (s, 7H), 2.58 (m, 5H), 6.76 (s, 1H), 7.47—7.75 (m, 4H), 8.32 (d, 1H).

Example 8

Preparation of 2-(1-Cyclohexenyl)-3-thiophenesulfonamide

A solution of 3-bromo-2-(1-cyclohexenyl)-thiophene (prepared by the method of N. Gjos and S. Gronowitz, *Acta Chem. Scan., 26,* 1851—59, (1972)) (8.0 g, 32.9 mmol) in ether (30 ml) was added dropwise to a stirring solution of *n*-butyllithium (1.37 M, 25.2 ml, 34.6 mmol) in ether (60 ml) precooled to −78°C. The solution was stirred another 30 minutes at −78°C and was then treated with sulfuryl chloride (3.2 ml, 40 mmol) in hexane (10 ml). After an additional 30 minutes at −78°C, anhydrous ammonia (9 ml, 300 mmol) was added, the suspension warmed to 25°C and poured into ether, washed with water and brine, dried over magnesium sulfate and reduced in volume. The product was crystallized by trituration with butyl chloride and hexane affording 2.51 g white solid (31%) m.p. 115—121°C.

Example 9

Preparation of 2-(1-Cyclohexen-2-yl)-N-[(4-methoxy-6-methylpyrimidin-2-yl)aminocarbonyl]-3 -thiophenesulfonamide

To a stirring solution of 2-(1-cyclohexenyl)-3-thiophenesulfonamide (160 mg, 0.657 mmol), and 4-methyl-6-methoxy-2-aminophenoxycarbonyl pyrimidine (180 mg, 0.69 mmol) in 15 ml of acetonitrile was added 1,8-diazabicyclo[5.4.0]undec-7-ene (105 mg, 0.69 mmol). The reaction was stirred overnight, poured into water, acidified with acetic acid and extracted with methylene chloride, dried over magnesium sulfate and concentrated to yield the desired product, 170 mg, m.p. 190—192°C. NMR(δ) ($d_6$—DMSO) (200 MHz): 1.3—2.4 (m, with s at 2.10, 11H); 3.79 (s, 3H); 5.8—6.0 (m, 1H); 6.42 (s, 1H), 7.30 and 7.44 (AB q, J=5.4 Hz, 2H).

Example 10

Preparation of 4-(1-Hydroxypropyl)-1-methyl-5-*tert*-butylpyrazolesulfonamide

To a cooled solution of *n*-butyl lithium (1.8 g, 27.9 mmol) in 150 ml of tetrahydrofuran was added 4-bromo-5-*tert*-butylsulfonamide-N-methylpyrazole. After stirring for 30 minutes at −78°C, propionaldehyde (1.5 g, 27.9 mmole) was added dropwise. After standard workup, a red oil was isolated. Crystallization from ethyl acetate/hexane afforded 3.1 g of the desired compound, m.p. 113—115°C.

Example 11

Preparation of 4-(2-Propenyl)-1-methyl-5-pyrazole sulfonamide

The alcohol (3.0 g) from Example 10 was stirred in 25 ml of trifluoroacetic acid at 68°C for 30 minutes. The solution was allowed to cool to room temperature, and the volatile components were removed *in vacuo* to afford 510 mg of the desired sulfonamide, m.p. 157—161°C.

Example 12

Preparation of N-[(4,6-Dimethoxypyrimidin-2-yl)-aminocarbonyl]-1-methyl-4-(1-propenyl)-1H-pyrazole-5-sulfonamide

The sulfonamide from Example 11 (150 mg, 0.7 mmol) and the phenylcarbamate of 4,6-dimethoxy-2-aminopyrimidine (202 mg, 0.7 mmol) were dissolved in 5 ml of acetonitrile. 1,8-Diazabicyclo[5.4.0]undec-7-

ene (112 mg, 0.7 mmol) was added and the reaction was stirred for 30 minutes. Addition of 5 ml of 5% hydrochloric acid resulted in a solid, which was collected and dried to afford 280 mg of the desired product, m.p. 154—156°C. NMR (200 MHz, $CDCl_3$): δ 1.84 (d, 3H); 3.56 (s, 6H); 4.21 (s, 3H); 5.80 (s, 1H); 6.2 (d, q, 1H); 6.57 (d, d, 1H); 7.43 (br, s, 1H); 7.61 (s, 1H). The NMR was indicative of a *cis:trans* mixture of 1:7.

General Structure I

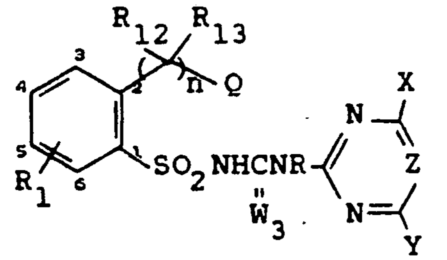

where $W_3$ is O unless otherwise indicated by *, where $W_3$ is S.

For ring systems Q—4 through Q—10, the substituents $R_7$ and $R_8$ are attached as follows

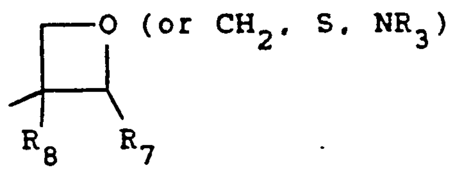

General Structure II

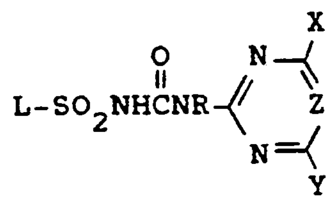

L-9 is

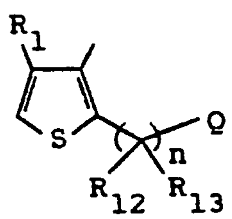

L-10 is

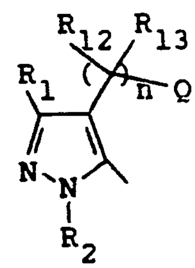

For ring systems Q—4 through Q—10, the substituents $R_7$ and $R_8$ are attached as follows

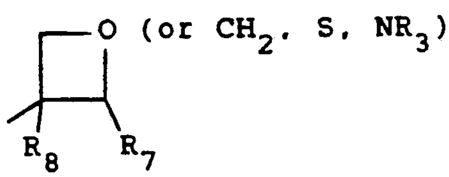

TABLE OF STRUCTURES (CONTINUED)

General Structure IIa

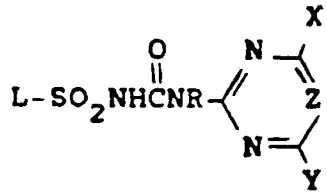

L-9 is

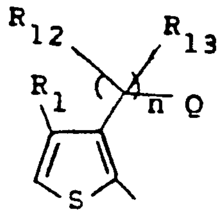

L-10 is

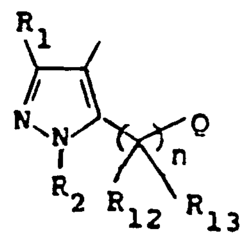

General Structure IIb

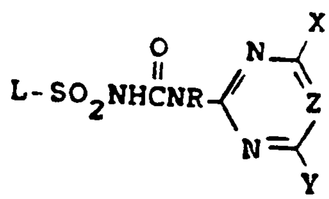

L-9 is

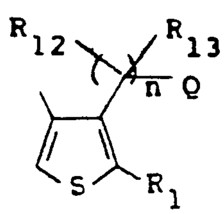

L-10 is

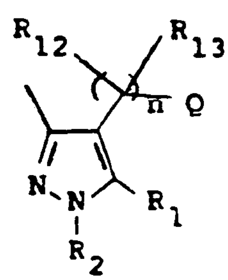

General Structure III

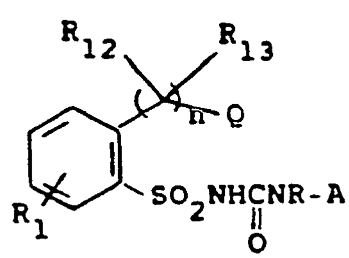

TABLE OF STRUCTURES (CONTINUED)

General Structure IV

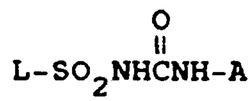

L-9 and L-10 are as in General Structure II.
n=O

General Structure V

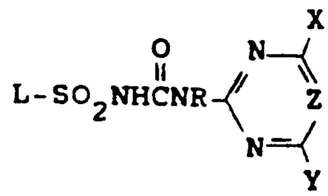

L-9 and L-10 are as in General Structure II.

$R_9$ and $R_{10}$ are C=O
n=O

General Structure VI

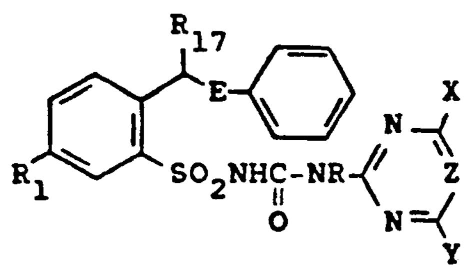

General Structure VII

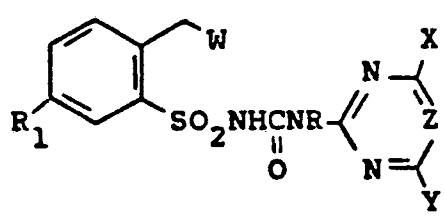

TABLE OF STRUCTURES (CONTINUED)

General Structure VIII

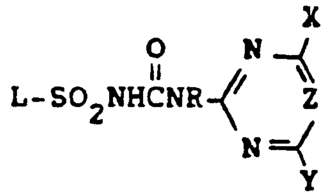

L-2 is

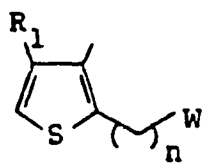

L-3 is

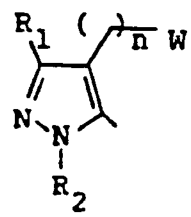

General Structure VIIIa

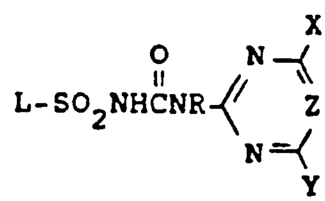

L-2 is

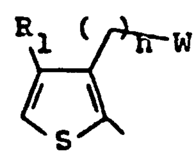

General Structure VIIIb

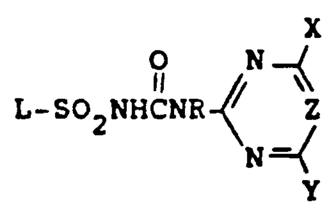

L-2 is

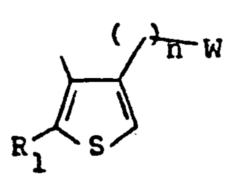

L-3 is

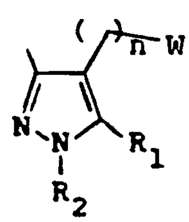

TABLE OF STRUCTURES (CONTINUED)

General Structure IX

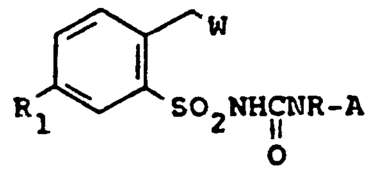

General Structure X

O

L-$SO_2$NHCNH-A

L-2 and L-3 are as in General Structure III.

General Structure Xa

O

L-$SO_2$NHCNH-A

L-2 and L-3 are as in General Structure IIIa.

Table I

General Structure I (wherein $R_{12}$ and $R_{13}$ are H)

| Q | n | R | $R_1$ | $R_7$ | $R_8$ | $R_9$ | $R_{10}$ | $R_{11}$ | X | Y | Z | m.p.(°C) |
|---|---|---|---|---|---|---|---|---|---|---|---|---|
| Q-1 | 0 | H | H | H | H | H | H | - | $CH_3$ | $CH_3$ | CH | 197-198.5 |
| Q-1 | 0 | H | H | H | H | H | H | - | $CH_3$ | $OCH_3$ | CH | 209-210 |
| Q-1 | 0 | H | H | H | H | H | H | - | $OCH_3$ | $OCH_3$ | CH | 202-204 |
| Q-1 | 0 | $CH_3$ | H | H | H | H | H | - | $CH_3$ | $OCH_3$ | N | 144-146 |
| Q-1 | 0 | H | H | H | H | H | H | - | $CH_3$ | $OCH_3$ | N | 190-191 |
| Q-1 | 0 | H | H | H | H | H | H | - | $OCH_3$ | $OCH_3$ | N | 174-176 |
| Q-1 | 0 | H | H | H | H | H | H | - | Cl | $OCH_3$ | CH | 192-194 |
| Q-1 | 0 | H | H | H | F | H | H | - | $CH_3$ | $CH_3$ | CH | |
| Q-1 | 0 | H | H | H | F | H | H | - | $CH_3$ | $CH_3$ | N | |
| Q-1 | 0 | H | H | H | F | H | H | - | $CH_3$ | $OCH_3$ | CH | |
| Q-1 | 0 | H | H | H | Cl | H | H | - | $OCH_3$ | $OCH_3$ | CH | 174-177 |
| Q-1 | 0 | H | H | H | Cl | H | H | - | $CH_3$ | $CH_3$ | N | 172-175 |
| Q-1 | 0 | H | H | Cl | H | H | H | - | $CH_3$ | $OCH_3$ | CH | 186-188 |
| Q-1 | 0 | H | H | Cl | H | H | H | - | $CH_3$ | $OCH_3$ | N | 156-160 |
| Q-1 | 0 | H | H | H | Br | H | H | - | $CH_3$ | $CH_3$ | CH | |
| Q-1 | 0 | H | H | Br | H | H | H | - | $CH_3$ | $OCH_3$ | CH | |
| Q-1 | 0 | H | H | Br | H | H | H | - | $OCH_3$ | $OCH_3$ | CH | |
| Q-1 | 0 | H | H | H | I | H | H | - | $CH_3$ | $CH_3$ | N | |
| Q-1 | 0 | H | H | H | H | H | H | - | $NHCH_3$ | $OCH_2CH_3$ | N | 203-206 |
| Q-1 | 0 | H | H | H | CN | H | H | - | $OCH_3$ | $OCH_3$ | N | |
| Q-1 | 0 | H | H | CN | H | H | H | - | $OCH_3$ | $OCH_3$ | CH | |
| Q-1 | 0 | H | H | H | $CH_3$ | H | H | - | $CH_3$ | $CH_3$ | CH | 155-159 |
| Q-1 | 0 | H | H | H | $CH_3$ | H | H | - | $CH_3$ | $OCH_3$ | CH | 180-182 |
| Q-1 | 0 | H | H | H | $CH_3$ | H | H | - | $OCH_3$ | $OCH_3$ | CH | 215-216 |
| Q-1 | 0 | H | H | $CH_3$ | H | H | H | - | Cl | $OCH_3$ | CH | 175-176 |
| Q-1 | 0 | H | H | $CH_3$ | H | H | H | - | $CH_3$ | $OCH_3$ | N | 154-157 |
| Q-1 | 0 | H | H | H | $CH_2CH_3$ | H | H | - | $OCH_3$ | $OCH_3$ | N | |
| Q-1 | 0 | H | H | $CH_2CH_3$ | H | H | H | - | $CH_3$ | $OCH_3$ | CH | |
| Q-1 | 0 | H | H | H | $CH_2CH_3$ | H | H | - | $OCH_3$ | $OCH_3$ | CH | |
| Q-1 | 0 | H | H | H | $CH_2CH_2CH_3$ | H | H | - | $CH_3$ | $OCH_3$ | N | |
| Q-1 | 0 | H | H | H | Cl | H | H | - | $CH_3$ | $CH_3$ | CH | 182-185 |

Table I (continued)

| Q | n | R | $R_1$ | $R_7$ | $R_8$ | $R_9$ | $R_{10}$ | $R_{11}$ | X | Y | Z | m.p.(°C) |
|---|---|---|---|---|---|---|---|---|---|---|---|---|
| Q-1 | 0 | H | H | H | Cl | H | H | - | $OCH_3$ | $OCH_3$ | N | 139-142 |
| Q-1 | 0 | H | H | H | Cl | H | H | - | Cl | $OCH_3$ | CH | 185-187 |
| Q-1 | 0 | H | H | H | Cl | H | H | - | $OCH_2CH_3$ | $NHCH_3$ | CH | 154-158 |
| Q-1 | 0 | H | H | H | Br | | $CH_3$ | - | $OCH_3$ | $CH_3$ | CH | |
| Q-1 | 0 | H | H | H | Br | H | Cl | - | $OCH_3$ | $CH_3$ | CH | |
| Q-1 | 0 | H | H | H | Br | H | F | - | $CH_3$ | $CH_3$ | N | |
| Q-1 | 0 | H | H | H | Br | H | F | - | $CH_3$ | $OCH_3$ | N | |
| Q-1 | 0 | H | H | H | Br | H | F | - | $OCH_3$ | $OCH_3$ | N | |
| Q-1 | 0 | H | H | H | Br | H | Br | - | $CH_3$ | $OCH_3$ | CH | |
| Q-1 | 0 | H | H | H | I | H | $CH_3$ | - | $OCH_3$ | $OCH_3$ | CH | |
| Q-1 | 0 | H | H | H | CN | H | $CH_3$ | - | $CH_3$ | $OCH_3$ | N | |
| Q-1 | 0 | H | H | H | CN | H | $CH_3$ | - | $OCH_3$ | $OCH_3$ | N | |
| Q-1 | 0 | H | H | H | CN | H | Cl | - | $CH_3$ | $CH_3$ | CH | |
| Q-1 | 0 | H | H | H | CN | H | F | - | $CH_3$ | $OCH_3$ | CH | |
| Q-1 | 0 | H | H | H | CN | H | Br | - | $OCH_3$ | $OCH_3$ | CH | |
| Q-1 | 0 | H | H | H | CN | $CH_3$ | H | - | $CH_3$ | $CH_3$ | N | |
| Q-1 | 0 | H | H | H | $OCH_3$ | H | $CH_3$ | - | $CH_3$ | $OCH_3$ | N | |
| Q-1 | 0 | H | H | H | $OCH_3$ | H | $CH_3$ | - | $OCH_3$ | $OCH_3$ | N | |
| Q-1 | 0 | H | H | H | $OCH_3$ | H | Cl | - | Cl | $OCH_3$ | CH | |
| Q-1 | 0 | H | H | H | $OCH_3$ | Cl | H | - | $CH_3$ | $OCH_3$ | CH | |
| Q-1 | 0 | H | H | H | $OCH_3$ | F | H | - | $OCH_3$ | $OCH_3$ | CH | |
| Q-1 | 0 | H | H | H | $OCH_3$ | H | Br | - | $CH_3$ | $OCH_3$ | N | |
| Q-1 | 0 | H | H | H | $CO_2CH_3$ | H | $CH_3$ | - | $OCH_3$ | $OCH_3$ | N | |
| Q-1 | 0 | H | H | H | $CO_2CH_3$ | H | $CH_3$ | - | $CH_3$ | $CH_3$ | CH | |
| Q-1 | 0 | H | H | H | $CO_2CH_3$ | H | $CH_3$ | - | $CH_3$ | $CH_3$ | CH | |
| Q-1 | 0 | H | H | H | $CO_2CH_3$ | H | F | - | $CH_3$ | $OCH_3$ | CH | |
| Q-1 | 0 | H | H | H | $CO_2CH_3$ | H | Cl | - | $OCH_3$ | $OCH_3$ | CH | |
| Q-1 | 0 | H | H | H | $CO_2CH_3$ | H | Cl | - | $CH_3$ | $OCH_3$ | N | |
| Q-1 | 0 | H | H | H | $CO_2CH_3$ | H | Br | - | $OCH_3$ | $OCH_3$ | N | |
| Q-1 | 0 | H | H | H | $SO_2N(CH_3)_2$ | H | $CH_3$ | - | $CH_3$ | $OCH_3$ | CH | |
| Q-1 | 0 | H | H | H | $SO_2N(CH_3)_2$ | H | $CH_3$ | - | $OCH_3$ | $OCH_3$ | CH | |
| Q-1 | 0 | H | H | H | $SO_2N(CH_3)_2$ | H | F | - | $CH_3$ | $OCH_3$ | CH | |

Table I (continued)

| Q | n | R | $R_1$ | $R_7$ | $R_8$ | $R_9$ | $R_{10}$ | $R_{11}$ | X | Y | Z | m.p. (°C) |
|---|---|---|---|---|---|---|---|---|---|---|---|---|
| Q-1 | 0 | H | H | H | $SO_2N(CH_3)_2$ | H | Cl | - | $CH_3$ | $OCH_3$ | N | |
| Q-1 | 0 | H | H | H | $SO_2N(CH_3)_2$ | H | Br | - | $OCH_3$ | $OCH_3$ | CH | |
| Q-1 | 0 | H | H | H | $SCH_3$ | H | $CH_3$ | - | Cl | $OCH_3$ | CH | |
| Q-1 | 0 | H | H | H | $SCH_3$ | H | F | - | $OCH_3$ | $OCH_3$ | N | |
| Q-1 | 0 | H | H | H | $SCH_3$ | H | Cl | - | $CH_3$ | $OCH_3$ | CH | |
| Q-1 | 0 | H | H | H | $OCH_3$ | H | Cl | - | $CH_3$ | $OCH_3$ | CH | |
| Q-1 | 0 | H | H | H | $OCH_3$ | H | H | - | $CH_3$ | $CH_3$ | CH | |
| Q-1 | 0 | H | H | H | $OCH_3$ | H | H | - | $OCH_3$ | $CH_3$ | CH | |
| Q-1 | 0 | H | H | $CH_3O$ | H | H | H | - | $OCH_3$ | $OCH_3$ | CH | |
| Q-1 | 0 | H | H | H | $OCH_2CH_3$ | H | H | - | $CH_3$ | $OCH_3$ | N | |
| Q-1 | 0 | H | H | H | $CO_2CH_3$ | H | H | - | $CH_3$ | $OCH_3$ | N | |
| Q-1 | 0 | H | H | H | $CO_2CH_3$ | H | H | - | $OCH_3$ | $OCH_3$ | N | |
| Q-1 | 0 | H | H | H | $CO_2CH_3$ | H | H | - | $CH_3$ | $OCH_3$ | CH | |
| Q-1 | 0 | H | H | H | $CO_2CH_3$ | H | H | - | $OCH_3$ | $OCH_3$ | CH | |
| Q-1 | 0 | H | H | H | $CO_2CH_2CH_3$ | H | H | - | $CH_3$ | $OCH_3$ | N | |
| Q-1 | 0 | H | H | H | $SO_2N(CH_3)_2$ | H | H | - | $OCH_3$ | $OCH_3$ | N | |
| Q-1 | 0 | H | H | H | $SO_2N(CH_3)_2$ | H | H | - | $CH_3$ | $CH_3$ | CH | |
| Q-1 | 0 | H | H | H | $SCH_3$ | H | H | - | $OCH_3$ | $OCH_3$ | CH | |
| Q-1 | 0 | H | H | H | $SCH_3$ | H | H | - | $CH_3$ | $CH_3$ | N | |
| Q-1 | 0 | H | H | H | $SO_2CH_3$ | H | H | - | $CH_3$ | $OCH_3$ | N | |
| Q-1 | 0 | H | H | H | $SO_2CH_3$ | H | H | - | Cl | $OCH_3$ | CH | |
| Q-1 | 0 | H | H | H | $SO_2CH_3$ | H | H | - | $CH_3$ | $OCH_3$ | CH | |
| Q-1 | 0 | H | H | H | $SO_2CH_2CH_3$ | H | H | - | $OCH_3$ | $OCH_3$ | CH | |
| Q-1 | 0 | H | H | H | $CH_3$ | H | $CH_3$ | - | $CH_3$ | $OCH_3$ | N | |
| Q-1 | 0 | H | H | H | $CH_3$ | H | $CH_3$ | - | $OCH_3$ | $OCH_3$ | N | |
| Q-1 | 0 | H | H | H | $CH_3$ | $CH_3$ | H | - | $CH_3$ | $CH_3$ | CH | |
| Q-1 | 0 | H | H | H | $CH_3$ | H | F | - | $CH_3$ | $OCH_3$ | CH | |
| Q-1 | 0 | H | H | H | $CH_3$ | H | F | - | $OCH_3$ | $OCH_3$ | CH | |
| Q-1 | 0 | H | H | H | $CH_3$ | H | F | - | $CH_3$ | $OCH_3$ | N | |
| Q-1 | 0 | H | H | H | $CH_3$ | H | Cl | - | $OCH_3$ | $OCH_3$ | N | |
| Q-1 | 0 | H | H | H | $CH_3$ | Br | H | - | $CH_3$ | $OCH_3$ | CH | |
| Q-1 | 0 | H | H | H | F | H | $CH_3$ | - | $OCH_3$ | $OCH_3$ | CH | |

Table I (continued)

| Q | n | R | $R_1$ | $R_7$ | $R_8$ | $R_9$ | $R_{10}$ | $R_{11}$ | X | Y | Z | m.p.(°C) |
|---|---|---|---|---|---|---|---|---|---|---|---|---|
| Q-1 | 0 | H | H | H | F | H | $CH_3$ | - | $CH_3$ | $OCH_3$ | CH | |
| Q-1 | 0 | H | H | H | Cl | H | $CH_3$ | - | $CH_3$ | $OCH_3$ | N | |
| Q-1 | 0 | H | H | H | Cl | H | F | - | $OCH_3$ | $OCH_3$ | CH | |
| Q-1 | 0 | H | H | H | Cl | H | Cl | - | Cl | $OCH_3$ | CH | |
| Q-1 | 0 | H | H | H | Cl | H | Cl | - | $OCH_3$ | $OCH_3$ | N | |
| Q-1 | 0 | H | H | H | Cl | H | Br | - | $CH_3$ | $OCH_3$ | CH | |
| Q-1 | 0 | H | H | H | $SO_2CH_3$ | H | $CH_3$ | - | $CH_3$ | $CH_3$ | CH | |
| Q-1 | 0 | H | H | H | $SO_2CH_3$ | H | $CH_3$ | - | $OCH_3$ | $CH_3$ | CH | |
| Q-1 | 0 | H | H | H | $SO_2CH_3$ | H | $CH_3$ | - | $OCH_3$ | $OCH_3$ | CH | |
| Q-1 | 0 | H | H | H | $SO_2CH_3$ | H | $CH_3$ | - | $CH_3$ | $CH_3$ | N | |
| Q-1 | 0 | H | H | H | $SO_2CH_3$ | H | $CH_3$ | - | $CH_3$ | $OCH_3$ | N | |
| Q-1 | 0 | H | H | H | $SO_2CH_3$ | H | $CH_3$ | - | $OCH_3$ | $OCH_3$ | N | |
| Q-1 | 0 | H | H | H | $SO_2CH_3$ | H | $CH_3$ | - | Cl | $OCH_3$ | CH | |
| Q-1 | 0 | H | H | H | $SO_2CH_3$ | H | Cl | - | $OCH_3$ | $OCH_3$ | CH | |
| Q-1 | 0 | H | H | H | $SO_2CH_3$ | H | Cl | - | $CH_3$ | $OCH_3$ | N | |
| Q-1 | 0 | H | H | H | $SO_2CH_3$ | H | F | - | $OCH_3$ | $OCH_3$ | N | |
| Q-1 | 0 | H | H | H | $SO_2CH_3$ | H | Br | - | $CH_3$ | $CH_3$ | CH | |
| Q-1 | 0 | H | H | F | F | H | H | - | $CH_3$ | $OCH_3$ | CH | |
| Q-1 | 0 | H | H | F | F | H | H | - | $OCH_3$ | $OCH_3$ | CH | |
| Q-1 | 0 | H | H | F | F | H | H | - | $CH_3$ | $CH_3$ | N | |
| Q-1 | 0 | H | H | F | F | H | H | - | $CH_3$ | $OCH_3$ | N | |
| Q-1 | 0 | H | H | F | F | H | H | - | $OCH_3$ | $OCH_3$ | N | |
| Q-1 | 0 | H | H | F | F | H | H | - | Cl | $OCH_3$ | CH | |
| Q-1 | 0 | H | H | Cl | Cl | H | H | - | $CH_3$ | $OCH_3$ | CH | 187-191 |
| Q-1 | 0 | H | H | Cl | Cl | H | H | - | $OCH_3$ | $OCH_3$ | CH | 193-195 |
| Q-1 | 0 | H | H | Cl | Cl | H | H | - | $CH_3$ | $OCH_3$ | N | 168-170 |
| Q-1 | 0 | H | H | Cl | Cl | H | H | - | $OCH_3$ | $OCH_3$ | N | 190-193 |
| Q-1 | 0 | H | H | Cl | Cl | H | H | - | $CH_3$ | $CH_3$ | CH | 233-235 |
| Q-1 | 0 | H | H | Cl | Cl | H | H | - | $CH_3$ | $CH_3$ | N | 195-199 |
| Q-1 | 0 | H | H | Cl | Cl | H | H | - | Cl | $OCH_3$ | CH | 199-202 |
| Q-1 | 0 | H | H | Br | Br | H | H | - | $CH_3$ | $OCH_3$ | N | |
| Q-1 | 0 | H | H | Br | Br | H | H | - | $OCH_3$ | $OCH_3$ | N | |

Table I (continued)

| Q | n | R | $R_1$ | $R_7$ | $R_8$ | $R_9$ | $R_{10}$ | $R_{11}$ | X | Y | Z | m.p.(°C) |
|---|---|---|---|---|---|---|---|---|---|---|---|---|
| Q-1 | 0 | H | H | Br | Br | H | H | - | $CH_3$ | $OCH_3$ | CH | |
| Q-1 | 0 | H | H | Br | Br | H | H | - | $OCH_3$ | $OCH_3$ | CH | |
| Q-1 | 0 | H | H | Br | Br | H | H | - | $CH_3$ | $CH_3$ | CH | |
| Q-1 | 0 | H | H | Br | Br | H | H | - | $CH_3$ | $CH_3$ | N | |
| Q-1 | 0 | H | H | I | I | H | H | - | $OCH_3$ | $OCH_3$ | CH | |
| Q-1 | 0 | H | H | CN | CN | H | H | - | Cl | $OCH_3$ | CH | |
| Q-1 | 0 | H | H | CN | CN | H | H | - | $OCH_3$ | $OCH_3$ | N | |
| Q-1 | 0 | H | H | CN | CN | H | H | - | $OCH_3$ | $OCH_3$ | CH | |
| Q-1 | 0 | H | H | CN | CN | H | H | - | $CH_3$ | $OCH_3$ | CH | |
| Q-1 | 0 | H | H | CN | CN | H | H | - | $CH_3$ | $CH_3$ | CH | |
| Q-1 | 0 | H | H | CN | CN | H | H | - | $OCH_3$ | $CH_3$ | N | |
| Q-1 | 0 | H | H | $CH_3$ | $CH_3$ | H | H | - | $CH_3$ | $CH_3$ | CH | |
| Q-1 | 0 | H | H | $CH_3$ | $CH_3$ | H | H | - | $CH_3$ | $CH_3$ | N | |
| Q-1 | 0 | H | H | $CH_3$ | $CH_3$ | H | H | - | $CH_3$ | $OCH_3$ | N | |
| Q-1 | 0 | H | H | $CH_3$ | $CH_3$ | H | H | - | $OCH_3$ | $OCH_3$ | N | |
| Q-1 | 0 | H | H | $CH_3$ | $CH_3$ | H | H | - | $CH_3$ | $OCH_3$ | CH | |
| Q-1 | 0 | H | H | $CH_3$ | $CH_3$ | H | H | - | $OCH_3$ | $OCH_3$ | CH | |
| Q-1 | 0 | H | H | $OCH_3$ | $OCH_3$ | H | H | - | Cl | $OCH_3$ | CH | 154-156 |
| Q-1 | 0 | H | H | $CH_3$ | $CH_2CH_3$ | H | H | - | $OCH_3$ | $OCH_3$ | N | |
| Q-1 | 0 | H | H | $CH_3$ | $CH_2CH_3$ | H | H | - | $CH_3$ | $CH_3$ | CH | |
| Q-1 | 0 | H | H | $OCH_3$ | $OCH_3$ | H | H | - | $CH_3$ | $OCH_3$ | N | 152-155 |
| Q-1 | 0 | H | H | $OCH_3$ | $OCH_3$ | H | H | - | $OCH_3$ | $OCH_3$ | CH | 179-180 |
| Q-1 | 0 | H | H | $OCH_3$ | $OCH_3$ | H | H | - | $CH_3$ | $CH_3$ | N | |
| Q-1 | 0 | H | H | F | F | H | $CH_3$ | - | $CH_3$ | $CH_3$ | CH | |
| Q-1 | 0 | H | H | F | F | H | F | - | $OCH_3$ | $OCH_3$ | CH | |
| Q-1 | 0 | H | H | F | F | H | Cl | - | $CH_3$ | $CH_3$ | N | |
| Q-1 | 0 | H | H | F | F | H | Br | - | $CH_3$ | $OCH_3$ | N | |
| Q-1 | 0 | H | H | Cl | Cl | H | $CH_3$ | - | $OCH_3$ | $OCH_3$ | N | |
| Q-1 | 0 | H | H | Cl | Cl | H | Cl | - | $CH_3$ | $OCH_3$ | CH | |
| Q-1 | 0 | H | H | Cl | Cl | H | Cl | - | $OCH_3$ | $OCH_3$ | CH | |
| Q-1 | 0 | H | H | Cl | Cl | H | F | - | $CH_3$ | $OCH_3$ | N | |
| Q-1 | 0 | H | H | Cl | Cl | H | Br | - | $OCH_3$ | $OCH_3$ | N | |

Table I (continued)

| Q | n | R | $R_1$ | $R_7$ | $R_8$ | $R_9$ | $R_{10}$ | $R_{11}$ | X | Y | Z | m.p.(°C) |
|---|---|---|---|---|---|---|---|---|---|---|---|---|
| Q-1 | 0 | H | H | Br | Br | H | $CH_3$ | - | $CH_3$ | $CH_3$ | CH | |
| Q-1 | 0 | H | H | Br | Br | H | $CH_3$ | - | $CH_3$ | $OCH_3$ | CH | |
| Q-1 | 0 | H | H | Br | Br | H | Cl | - | $OCH_3$ | $OCH_3$ | CH | |
| Q-1 | 0 | H | H | Br | Br | H | Cl | - | $CH_3$ | $CH_3$ | N | |
| Q-1 | 0 | H | H | Br | Br | Cl | H | - | $CH_3$ | $OCH_3$ | N | |
| Q-1 | 0 | H | H | Br | Br | H | F | - | $OCH_3$ | $OCH_3$ | N | |
| Q-1 | 0 | H | H | Br | Br | H | Br | - | Cl | $OCH_3$ | CH | |
| Q-1 | 0 | H | H | CN | CN | H | $CH_3$ | - | $CH_3$ | $OCH_3$ | CH | |
| Q-1 | 0 | H | H | CN | CN | H | $CH_3$ | - | $OCH_3$ | $OCH_3$ | CH | |
| Q-1 | 0 | H | H | CN | CN | H | F | - | $CH_3$ | $OCH_3$ | N | |
| Q-1 | 0 | H | H | $CH_3$ | $CH_3$ | H | $CH_3$ | - | $OCH_3$ | $OCH_3$ | N | |
| Q-1 | 0 | H | H | $CH_3$ | $CH_3$ | H | $CH_3$ | - | $CH_3$ | $CH_3$ | CH | |
| Q-1 | 0 | H | H | $CH_3$ | $CH_3$ | H | $CH_3$ | - | $CH_3$ | $OCH_3$ | CH | |
| Q-1 | 0 | H | H | $CH_3$ | $CH_3$ | $CH_3$ | H | - | $OCH_3$ | $OCH_3$ | CH | |
| Q-1 | 0 | H | H | $CH_3$ | $CH_3$ | H | F | - | $CH_3$ | $OCH_3$ | N | |
| Q-1 | 0 | H | H | $CH_3$ | $CH_3$ | H | F | - | $OCH_3$ | $OCH_3$ | N | |
| Q-1 | 0 | H | H | $CH_3$ | $CH_3$ | H | Cl | - | $CH_3$ | $OCH_3$ | CH | |
| Q-1 | 0 | H | H | $CH_3$ | $CH_3$ | H | Cl | - | $OCH_3$ | $OCH_3$ | CH | |
| Q-1 | 0 | H | H | $CH_3$ | $CH_3$ | H | Br | - | $CH_3$ | $OCH_3$ | CH | |
| Q-1 | 0 | H | H | $OCH_3$ | $OCH_3$ | H | $CH_3$ | - | $OCH_3$ | $OCH_3$ | CH | |
| Q-1 | 0 | H | H | $OCH_3$ | $OCH_3$ | H | $CH_3$ | - | Cl | $OCH_3$ | CH | |
| Q-1 | 0 | H | H | $OCH_3$ | $OCH_3$ | H | F | - | $OCH_3$ | $OCH_3$ | CH | |
| Q-1 | 0 | H | H | $CO_2CH_3$ | $CO_2CH_3$ | H | $CH_3$ | - | $CH_3$ | $OCH_3$ | CH | |
| Q-1 | 0 | H | H | F | F | $CH_3$ | $CH_3$ | - | $CH_3$ | $OCH_3$ | CH | |
| Q-1 | 0 | H | H | F | F | $CH_3$ | $CH_3$ | - | $OCH_3$ | $OCH_3$ | CH | |
| Q-1 | 0 | H | H | F | F | F | F | - | $CH_3$ | $CH_3$ | N | |
| Q-1 | 0 | H | H | F | F | F | F | - | $CH_3$ | $OCH_3$ | N | |
| Q-1 | 0 | H | H | F | F | F | F | - | $OCH_3$ | $OCH_3$ | N | |
| Q-1 | 0 | H | H | F | F | Cl | Cl | - | Cl | $OCH_3$ | CH | |
| Q-1 | 0 | H | H | F | F | Cl | Cl | - | $CH_3$ | $OCH_3$ | CH | |
| Q-1 | 0 | H | H | F | F | Br | Br | - | $OCH_3$ | $OCH_3$ | CH | |
| Q-1 | 0 | H | H | F | F | Br | Br | - | $CH_3$ | $OCH_3$ | N | |

Table I (continued)

| Q | n | R | $R_1$ | $R_7$ | $R_8$ | $R_9$ | $R_{10}$ | $R_{11}$ | X | Y | Z | m.p.(°C) |
|---|---|---|---|---|---|---|---|---|---|---|---|---|
| Q-1 | 0 | H | H | Cl | Cl | $CH_3$ | $CH_3$ | - | $OCH_3$ | $OCH_3$ | N | |
| Q-1 | 0 | H | H | Cl | Cl | $CH_3$ | $CH_3$ | - | $CH_3$ | $CH_3$ | CH | |
| Q-1 | 0 | H | H | Cl | Cl | $CH_3$ | $CH_3$ | - | $CH_3$ | $OCH_3$ | CH | |
| Q-1 | 0 | H | H | Cl | Cl | F | F | - | $OCH_3$ | $OCH_3$ | CH | |
| Q-1 | 0 | H | H | Cl | Cl | F | F | - | $CH_3$ | $OCH_3$ | N | |
| Q-1 | 0 | H | H | Cl | Cl | Cl | Cl | - | $OCH_3$ | $OCH_3$ | N | |
| Q-1 | 0 | H | H | Cl | Cl | Cl | Cl | - | $CH_3$ | $OCH_3$ | CH | |
| Q-1 | 0 | H | H | Cl | Cl | Br | Br | - | $OCH_3$ | $OCH_3$ | CH | |
| Q-1 | 0 | H | H | Cl | Cl | Br | Br | - | $CH_3$ | $OCH_3$ | CH | |
| Q-1 | 0 | H | H | I | I | $CH_3$ | $CH_3$ | - | $CH_3$ | $OCH_3$ | N | |
| Q-1 | 0 | H | H | CN | CN | $CH_3$ | $CH_3$ | - | $OCH_3$ | $OCH_3$ | CH | |
| Q-1 | 0 | H | H | CN | CN | $CH_3$ | $CH_3$ | - | Cl | $OCH_3$ | CH | |
| Q-1 | 0 | H | H | CN | CN | F | F | - | $CH_3$ | $OCH_3$ | CH | |
| Q-1 | 0 | H | H | CN | CN | $CH_3$ | $CH_3$ | - | $CH_3$ | $CH_3$ | CH | |
| Q-1 | 0 | H | H | CN | CN | F | F | - | $OCH_3$ | $CH_3$ | CH | |
| Q-1 | 0 | H | H | CN | CN | F | F | - | $OCH_3$ | $OCH_3$ | CH | |
| Q-1 | 0 | H | H | CN | CN | Cl | Cl | - | $CH_3$ | $CH_3$ | N | |
| Q-1 | 0 | H | H | CN | CN | Br | Br | - | $CH_3$ | $OCH_3$ | N | |
| Q-1 | 0 | H | H | CN | CN | Br | Br | - | $OCH_3$ | $OCH_3$ | N | |
| Q-1 | 0 | H | H | $CH_3$ | $CH_3$ | $CH_3$ | $CH_3$ | - | $CH_3$ | $OCH_3$ | CH | |
| Q-1 | 0 | H | H | $CH_3$ | $CH_3$ | $CH_3$ | $CH_3$ | - | $OCH_3$ | $OCH_3$ | CH | |
| Q-1 | 0 | H | H | $CH_3$ | $CH_3$ | $CH_3$ | $CH_3$ | - | $CH_3$ | $OCH_3$ | N | |
| Q-1 | 0 | H | H | $CH_3$ | $CH_3$ | F | F | - | $OCH_3$ | $OCH_3$ | N | |
| Q-1 | 0 | H | H | $CH_3$ | $CH_3$ | Cl | Cl | - | $CH_3$ | $CH_3$ | CH | |
| Q-1 | 0 | H | H | $CH_3$ | $CH_3$ | Cl | Cl | - | $CH_3$ | $OCH_3$ | CH | |
| Q-1 | 0 | H | H | $CH_3$ | $CH_3$ | Cl | Cl | - | $OCH_3$ | $OCH_3$ | CH | |
| Q-1 | 0 | H | H | $CH_3$ | $CH_3$ | Br | Br | - | $CH_3$ | $CH_3$ | N | |
| Q-1 | 0 | H | H | $CH_3$ | $CH_3$ | Br | Br | - | $CH_3$ | $OCH_3$ | N | |
| Q-1 | 0 | H | H | $OCH_3$ | $OCH_3$ | $CH_3$ | $CH_3$ | - | Cl | $OCH_3$ | CH | |
| Q-1 | 0 | H | H | $OCH_3$ | $OCH_3$ | $CH_3$ | $CH_3$ | - | $CH_3$ | $OCH_3$ | CH | |
| Q-1 | 0 | H | H | $OCH_3$ | $OCH_3$ | Cl | Cl | - | $OCH_3$ | $OCH_3$ | CH | |
| Q-1 | 0 | H | H | $OCH_3$ | $OCH_3$ | F | F | - | $CH_3$ | $OCH_3$ | N | |

Table I (continued)

| Q | n | R | $R_1$ | $R_7$ | $R_8$ | $R_9$ | $R_{10}$ | $R_{11}$ | X | Y | Z | m.p. (°C) |
|---|---|---|---|---|---|---|---|---|---|---|---|---|
| Q-1 | 0 | H | H | $OCH_3$ | $OCH_3$ | F | F | – | $OCH_3$ | $OCH_3$ | N | |
| Q-1 | 0 | H | H | $OCH_3$ | $OCH_3$ | F | F | – | $CH_3$ | $CH_3$ | CH | |
| Q-1 | 0 | H | H | $OCH_3$ | $OCH_3$ | Br | Br | – | $CH_3$ | $OCH_3$ | CH | |
| Q-1 | 0 | H | H | $OCH_3$ | $OCH_3$ | Br | Br | – | $OCH_3$ | $OCH_3$ | CH | |
| Q-1 | 0 | H | H | F | Cl | H | H | – | Cl | $OCH_3$ | CH | |
| Q-1 | 0 | H | H | F | Br | H | H | – | $CH_3$ | $OCH_3$ | CH | |
| Q-1 | 0 | H | H | F | $CH_3$ | H | H | – | $OCH_3$ | $OCH_3$ | CH | |
| Q-1 | 0 | H | H | F | CN | H | H | – | $CH_3$ | $CH_3$ | N | |
| Q-1 | 0 | H | H | F | $CH_2CH_3$ | H | H | – | $OCH_3$ | $OCH_3$ | N | |
| Q-1 | 0 | H | H | F | $OCH_3$ | H | H | – | $CH_3$ | $CH_3$ | CH | |
| Q-1 | 0 | H | H | F | $CO_2CH_3$ | H | H | – | $CH_3$ | $OCH_3$ | CH | |
| Q-1 | 0 | H | H | F | $SO_2N(CH_3)_2$ | H | H | – | $OCH_3$ | $OCH_3$ | CH | |
| Q-1 | 0 | H | H | F | $SCH_3$ | H | H | – | $CH_3$ | $OCH_3$ | N | |
| Q-1 | 0 | H | H | F | $SO_2CH_3$ | H | H | – | $OCH_3$ | $OCH_3$ | N | |
| Q-1 | 0 | H | H | Cl | F | H | H | – | $CH_3$ | $CH_3$ | CH | |
| Q-1 | 0 | H | H | Cl | F | H | H | – | $OCH_3$ | $OCH_3$ | CH | |
| Q-1 | 0 | H | H | Cl | Br | H | H | – | $CH_3$ | $OCH_3$ | CH | |
| Q-1 | 0 | H | H | Cl | F | H | H | – | $CH_3$ | $OCH_3$ | N | |
| Q-1 | 0 | H | H | Cl | CN | H | H | – | $OCH_3$ | $OCH_3$ | CH | |
| Q-1 | 0 | H | H | Cl | $CH_3$ | H | H | – | Cl | $OCH_3$ | CH | |
| Q-1 | 0 | H | H | Cl | $CH_3$ | H | H | – | $CH_3$ | $OCH_3$ | CH | |
| Q-1 | 0 | H | H | Cl | $OCH_3$ | H | H | – | $CH_3$ | $CH_3$ | CH | |
| Q-1 | 0 | H | H | Cl | $OCH_3$ | H | H | – | $OCH_3$ | $CH_3$ | CH | |
| Q-1 | 0 | H | H | Cl | $CO_2CH_3$ | H | H | – | $OCH_3$ | $OCH_3$ | CH | |
| Q-1 | 0 | H | H | Cl | $SO_2N(CH_3)_2$ | H | H | – | $CH_3$ | $CH_3$ | N | |
| Q-1 | 0 | H | H | Cl | $SCH_3$ | H | H | – | $CH_3$ | $OCH_3$ | N | |
| Q-1 | 0 | H | H | Cl | $SO_2CH_3$ | H | H | – | $OCH_3$ | $OCH_3$ | N | |
| Q-1 | 0 | H | H | Br | F | H | H | – | $OCH_3$ | $OCH_3$ | N | |
| Q-1 | 0 | H | H | Br | F | H | H | – | $OCH_3$ | $OCH_3$ | CH | |
| Q-1 | 0 | H | H | Br | F | H | H | – | $CH_3$ | $OCH_3$ | N | |
| Q-1 | 0 | H | H | Br | Cl | H | H | – | $OCH_3$ | $OCH_3$ | N | |
| Q-1 | 0 | H | H | Br | Cl | H | H | – | $CH_3$ | $CH_3$ | CH | |
| Q-1 | 0 | H | H | Br | Cl | H | H | – | $CH_3$ | $OCH_3$ | CH | |

Table I (continued)

| Q | n | R | $R_1$ | $R_7$ | $R_8$ | $R_9$ | $R_{10}$ | $R_{11}$ | X | Y | Z | m.p.(°C) |
|---|---|---|---|---|---|---|---|---|---|---|---|---|
| Q-1 | 0 | H | H | Br | Br | H | H | - | $OCH_3$ | $OCH_3$ | CH | |
| Q-1 | 0 | H | H | Br | CN | H | H | - | $CH_3$ | $CH_3$ | N | |
| Q-1 | 0 | H | H | Br | $CH_3$ | H | H | - | $CH_3$ | $OCH_3$ | N | |
| Q-1 | 0 | H | H | Br | $CH_3$ | H | H | - | $OCH_3$ | $OCH_3$ | N | |
| Q-1 | 0 | H | H | Br | $CH_3$ | H | H | - | Cl | $OCH_3$ | CH | |
| Q-1 | 0 | H | H | Br | $CH_2CH_3$ | H | H | - | $CH_3$ | $OCH_3$ | CH | |
| Q-1 | 0 | H | H | Br | $OCH_3$ | H | H | - | $OCH_3$ | $OCH_3$ | CH | |
| Q-1 | 0 | H | H | Br | $OCH_3$ | H | H | - | $CH_3$ | $CH_3$ | N | |
| Q-1 | 0 | H | H | Br | $CO_2CH_3$ | H | H | - | $CH_3$ | $CH_3$ | CH | |
| Q-1 | 0 | H | H | Br | $SO_2N(CH_3)_2$ | H | H | - | $CH_3$ | $OCH_3$ | CH | |
| Q-1 | 0 | H | H | Br | $SCH_3$ | H | H | - | $OCH_3$ | $OCH_3$ | CH | |
| Q-1 | 0 | H | H | Br | $SO_2CH_3$ | H | H | - | $CH_3$ | $OCH_3$ | N | |
| Q-1 | 0 | H | H | I | F | H | H | - | $OCH_3$ | $OCH_3$ | N | |
| Q-1 | 0 | H | H | I | Cl | H | H | - | $CH_3$ | $OCH_3$ | CH | |
| Q-1 | 0 | H | H | I | Br | H | H | - | $OCH_3$ | $OCH_3$ | CH | |
| Q-1 | 0 | H | H | I | $CH_3$ | H | H | - | $CH_3$ | $OCH_3$ | CH | |
| Q-1 | 0 | H | H | CN | F | H | H | - | $CH_3$ | $OCH_3$ | N | |
| Q-1 | 0 | H | H | CN | Cl | H | H | - | $OCH_3$ | $OCH_3$ | N | |
| Q-1 | 0 | H | H | CN | Br | H | H | - | Cl | $OCH_3$ | CH | |
| Q-1 | 0 | H | H | CN | $CH_3$ | H | H | - | $OCH_3$ | $OCH_3$ | N | |
| Q-1 | 0 | H | H | CN | $CH_2CH_3$ | H | H | - | $CH_3$ | $OCH_3$ | CH | |
| Q-1 | 0 | H | H | CN | $OCH_3$ | H | H | - | $CH_3$ | $CH_3$ | CH | |
| Q-1 | 0 | H | H | CN | $CO_2CH_3$ | H | H | - | $OCH_3$ | $CH_3$ | CH | |
| Q-1 | 0 | H | H | CN | $SCH_3$ | H | H | - | $CH_3$ | $OCH_3$ | CH | |
| Q-1 | 0 | H | H | CN | $SO_2CH_3$ | H | H | - | $CH_3$ | $CH_3$ | N | |
| Q-1 | 0 | H | H | H | F | F | Cl | - | $CH_3$ | $OCH_3$ | N | |
| Q-1 | 0 | H | H | H | F | F | Cl | - | $OCH_3$ | $OCH_3$ | N | |
| Q-1 | 0 | H | H | H | F | Cl | Br | - | $CH_3$ | $OCH_3$ | CH | |
| Q-1 | 0 | H | H | H | F | Br | F | - | $OCH_3$ | $OCH_3$ | CH | |
| Q-1 | 0 | H | H | H | $CH_3$ | F | Cl | - | $CH_3$ | $OCH_3$ | N | |
| Q-1 | 0 | H | H | H | $CH_3$ | F | Br | - | $OCH_3$ | $OCH_3$ | N | |
| Q-1 | 0 | H | H | H | $CH_3$ | F | $CH_3$ | - | $CH_3$ | $CH_3$ | CH | |

Table I (continued)

| Q | n | R | $R_1$ | $R_7$ | $R_8$ | $R_9$ | $R_{10}$ | $R_{11}$ | X | Y | Z | m.p.(°C) |
|---|---|---|---|---|---|---|---|---|---|---|---|---|
| Q-1 | 0 | H | H | H | $CH_3$ | Br | $CH_3$ | - | $CH_3$ | $OCH_3$ | CH | |
| Q-1 | 0 | H | H | H | $CH_3$ | Cl | $CH_3$ | - | $OCH_3$ | $OCH_3$ | CH | |
| Q-1 | 0 | H | H | H | Cl | F | Cl | - | $CH_3$ | $CH_3$ | N | |
| Q-1 | 0 | H | H | H | Cl | F | Br | - | $CH_3$ | $OCH_3$ | N | |
| Q-1 | 0 | H | H | H | Cl | Br | Cl | - | $OCH_3$ | $OCH_3$ | N | |
| Q-1 | 0 | H | H | H | Cl | F | $CH_3$ | - | Cl | $OCH_3$ | CH | |
| Q-1 | 0 | H | H | H | CN | F | Cl | - | $CH_3$ | $OCH_3$ | CH | |
| Q-1 | 0 | H | H | H | CN | F | Br | - | $OCH_3$ | $OCH_3$ | CH | |
| Q-1 | 0 | H | H | H | CN | F | $CH_3$ | - | $CH_3$ | $OCH_3$ | N | |
| Q-1 | 0 | H | H | H | CN | Cl | $CH_3$ | - | $OCH_3$ | $OCH_3$ | N | |
| Q-1 | 0 | H | H | H | CN | Cl | Br | - | $CH_3$ | $CH_3$ | CH | |
| Q-1 | 0 | H | H | H | $CH_3$ | F | Cl | - | $CH_3$ | $OCH_3$ | CH | |
| Q-1 | 0 | H | H | H | $CH_3$ | F | Br | - | $OCH_3$ | $OCH_3$ | CH | |
| Q-1 | 0 | H | H | H | $CH_3$ | F | $CH_3$ | - | $CH_3$ | $OCH_3$ | N | |
| Q-1 | 0 | H | H | H | $CH_3$ | Cl | Br | - | $OCH_3$ | $OCH_3$ | N | |
| Q-1 | 0 | H | H | H | $CH_3$ | Cl | Br | - | $CH_3$ | $OCH_3$ | CH | |
| Q-1 | 0 | H | H | H | $CH_3$ | Br | $CH_3$ | - | $OCH_3$ | $OCH_3$ | CH | |
| Q-1 | 0 | H | H | H | $CH_2CH_3$ | F | $Cl_3$ | - | $CH_3$ | $OCH_3$ | CH | |
| Q-1 | 0 | H | H | H | $CH_2CH_2CH_3$ | F | Br | - | $CH_3$ | $OCH_3$ | N | |
| Q-1 | 0 | H | H | H | $OCH_3$ | F | Cl | - | $OCH_3$ | $OCH_3$ | CH | |
| Q-1 | 0 | H | H | H | $OCH_3$ | F | Br | - | Cl | $OCH_3$ | CH | |
| Q-1 | 0 | H | H | H | $OCH_3$ | F | $CH_3$ | - | $OCH_3$ | $OCH_3$ | N | |
| Q-1 | 0 | H | H | H | $OCH_3$ | Cl | Br | - | $CH_3$ | $OCH_3$ | CH | |
| Q-1 | 0 | H | H | F | Cl | F | Cl | - | $OCH_3$ | $OCH_3$ | CH | |
| Q-1 | 0 | H | H | F | Cl | F | $CH_3$ | - | $CH_3$ | $OCH_3$ | N | |
| Q-1 | 0 | H | H | F | Br | Cl | $CH_3$ | - | $OCH_3$ | $OCH_3$ | N | |
| Q-1 | 0 | H | H | Cl | $CH_3$ | $CH_3$ | F | - | $CH_3$ | $CH_3$ | CH | |
| Q-1 | 0 | H | H | Br | $OCH_3$ | $CH_3$ | F | - | $CH_3$ | $OCH_3$ | CH | |
| Q-1 | 0 | H | H | $SCH_3$ | $CO_2CH_3$ | F | Cl | - | $OCH_3$ | $OCH_3$ | CH | |
| Q-1 | 0 | H | H | Br | Cl | F | Cl | - | $CH_3$ | $OCH_3$ | N | |
| Q-1 | 0 | H | H | Br | I | F | Cl | - | $OCH_3$ | $OCH_3$ | N | |
| Q-1 | 0 | H | H | $CH_3$ | $CO_2CH_3$ | $CH_3$ | Br | - | $CH_3$ | $OCH_3$ | CH | |

Table I (continued)

| Q | n | R | $R_1$ | $R_7$ | $R_8$ | $R_9$ | $R_{10}$ | $R_{11}$ | X | Y | Z | m.p. (°C) |
|---|---|---|---|---|---|---|---|---|---|---|---|---|
| Q-1 | 0 | H | H | Cl | Br | Cl | Br | - | $OCH_3$ | $OCH_3$ | CH | |
| Q-1 | 0 | H | H | Cl | Br | Cl | $CH_3$ | - | $CH_3$ | $OCH_3$ | CH | |
| Q-1 | 0 | H | H | I | F | F | Br | - | $OCH_3$ | $OCH_3$ | CH | |
| Q-1 | 0 | H | H | CN | F | F | Cl | - | Cl | $OCH_3$ | CH | |
| Q-1 | 0 | H | H | CN | Br | Cl | $CH_3$ | - | $OCH_3$ | $OCH_3$ | N | |
| Q-1 | 1 | H | H | H | H | H | H | - | $CH_3$ | $CH_3$ | CH | |
| Q-1 | 1 | H | H | H | H | H | H | - | $CH_3$ | $OCH_3$ | CH | |
| Q-1 | 1 | H | H | H | H | H | H | - | $OCH_3$ | $OCH_3$ | CH | |
| Q-1 | 1 | H | H | H | H | H | H | - | $CH_3$ | $CH_3$ | N | |
| Q-1 | 1 | H | H | H | H | H | H | - | $CH_3$ | $OCH_3$ | N | |
| Q-1 | 1 | H | H | H | H | H | H | - | $OCH_3$ | $OCH_3$ | N | |
| Q-1 | 1 | H | H | H | H | H | H | - | Cl | $OCH_3$ | CH | |
| Q-1 | 1 | H | H | H | F | H | H | - | $CH_3$ | $CH_3$ | CH | |
| Q-1 | 1 | H | H | H | F | H | H | - | $CH_3$ | $CH_3$ | N | |
| Q-1 | 1 | H | H | H | F | H | H | - | $CH_3$ | $OCH_3$ | CH | |
| Q-1 | 1 | H | H | H | Cl | H | H | - | $OCH_3$ | $OCH_3$ | CH | |
| Q-1 | 1 | H | H | H | Cl | H | H | - | $CH_3$ | $CH_3$ | N | |
| Q-1 | 1 | H | H | Cl | H | H | H | - | $CH_3$ | $OCH_3$ | CH | |
| Q-1 | 1 | H | H | Cl | H | H | H | - | $CH_3$ | $OCH_3$ | N | |
| Q-1 | 1 | H | H | H | Br | H | H | - | $CH_3$ | $CH_3$ | CH | |
| Q-1 | 1 | H | H | Br | H | H | H | - | $CH_3$ | $OCH_3$ | CH | |
| Q-1 | 1 | H | H | Br | H | H | H | - | $OCH_3$ | $OCH_3$ | CH | |
| Q-1 | 1 | H | H | H | I | H | H | - | $CH_3$ | $CH_3$ | N | |
| Q-1 | 1 | H | H | I | H | H | H | - | $CH_3$ | $OCH_3$ | N | |
| Q-1 | 1 | H | H | H | CN | H | H | - | $OCH_3$ | $OCH_3$ | N | |
| Q-1 | 1 | H | H | CN | H | H | H | - | $OCH_3$ | $OCH_3$ | CH | |
| Q-1 | 1 | H | H | H | $CH_3$ | H | H | - | $CH_3$ | $CH_3$ | CH | |
| Q-1 | 1 | H | H | H | $CH_3$ | H | H | - | $CH_3$ | $OCH_3$ | CH | |
| Q-1 | 1 | H | H | H | $CH_3$ | H | H | - | $OCH_3$ | $OCH_3$ | CH | |
| Q-1 | 1 | H | H | $CH_3$ | H | H | H | - | $CH_3$ | $CH_3$ | N | |
| Q-1 | 1 | H | H | $CH_3$ | H | H | H | - | $CH_3$ | $OCH_3$ | N | |
| Q-1 | 1 | H | H | H | $CH_2CH_3$ | H | H | - | $OCH_3$ | $OCH_3$ | N | |

Table I (continued)

| Q | n | R | $R_1$ | $R_7$ | $R_8$ | $R_9$ | $R_{10}$ | $R_{11}$ | X | Y | Z | m.p. (°C) |
|---|---|---|---|---|---|---|---|---|---|---|---|---|
| Q-1 | 1 | H | H | H | $CH_2CH_3$ | H | H | - | $OCH_3$ | $OCH_3$ | CH | |
| Q-1 | 1 | H | H | H | $CH_2CH_2CH_3$ | H | H | - | $CH_3$ | $OCH_3$ | N | |
| Q-1 | 1 | H | H | H | $OCH_3$ | H | H | - | $CH_3$ | $CH_3$ | CH | |
| Q-1 | 1 | H | H | H | $OCH_3$ | H | H | - | $OCH_3$ | $CH_3$ | CH | |
| Q-1 | 1 | H | H | $CH_3O$ | H | H | H | - | $OCH_3$ | $OCH_3$ | CH | |
| Q-1 | 1 | H | H | H | $OCH_2CH_3$ | H | H | - | $CH_3$ | $CH_3$ | N | |
| Q-1 | 1 | H | H | H | $CO_2CH_3$ | H | H | - | $CH_3$ | $OCH_3$ | N | |
| Q-1 | 1 | H | H | H | $CO_2CH_3$ | H | H | - | $OCH_3$ | $OCH_3$ | N | |
| Q-1 | 1 | H | H | H | $CO_2CH_3$ | H | H | - | $CH_3$ | $OCH_3$ | CH | |
| Q-1 | 1 | H | H | H | $CO_2CH_3$ | H | H | - | $OCH_3$ | $OCH_3$ | CH | |
| Q-1 | 1 | H | H | H | $CO_2CH_2CH_3$ | H | H | - | $CH_3$ | $OCH_3$ | N | |
| Q-1 | 1 | H | H | H | $SO_2N(CH_3)_2$ | H | H | - | $OCH_3$ | $OCH_3$ | N | |
| Q-1 | 1 | H | H | H | $SO_2N(CH_3)_2$ | H | H | - | $CH_3$ | $CH_3$ | CH | |
| Q-1 | 1 | H | H | H | $SCH_3$ | H | H | - | $OCH_3$ | $OCH_3$ | CH | |
| Q-1 | 1 | H | H | H | $SCH_3$ | H | H | - | $CH_3$ | $CH_3$ | N | |
| Q-1 | 1 | H | H | H | $SO_2CH_3$ | H | H | - | $CH_3$ | $OCH_3$ | N | |
| Q-1 | 1 | H | H | $SO_2CH_3$ | H | H | H | - | $OCH_3$ | $OCH_3$ | N | |
| Q-1 | 1 | H | H | H | $SO_2CH_3$ | H | H | - | Cl | $OCH_3$ | CH | |
| Q-1 | 1 | H | H | H | $SO_2CH_3$ | H | H | - | $CH_3$ | $OCH_3$ | CH | |
| Q-1 | 1 | H | H | H | $SO_2CH_2CH_3$ | H | H | - | $OCH_3$ | $OCH_3$ | CH | |
| Q-1 | 1 | H | H | H | $CH_3$ | H | $CH_3$ | - | $CH_3$ | $OCH_3$ | N | |
| Q-1 | 1 | H | H | H | $CH_3$ | H | $CH_3$ | - | $OCH_3$ | $OCH_3$ | N | |
| Q-1 | 1 | H | H | H | $CH_3$ | $CH_3$ | H | - | $CH_3$ | $CH_3$ | CH | |
| Q-1 | 1 | H | H | H | $CH_3$ | H | F | - | $CH_3$ | $OCH_3$ | CH | |
| Q-1 | 1 | H | H | H | $CH_3$ | H | F | - | $OCH_3$ | $OCH_3$ | CH | |
| Q-1 | 1 | H | H | H | $CH_3$ | H | F | - | $CH_3$ | $OCH_3$ | N | |
| Q-1 | 1 | H | H | H | $CH_3$ | H | Cl | - | $OCH_3$ | $OCH_3$ | N | |
| Q-1 | 1 | H | H | H | $CH_3$ | Br | H | - | $CH_3$ | $OCH_3$ | CH | |
| Q-1 | 1 | H | H | H | F | H | $CH_3$ | - | $OCH_3$ | $OCH_3$ | CH | |
| Q-1 | 1 | H | H | H | F | H | $CH_3$ | - | $CH_3$ | $OCH_3$ | CH | |
| Q-1 | 1 | H | H | H | Cl | H | $CH_3$ | - | $CH_3$ | $OCH_3$ | N | |
| Q-1 | 1 | H | H | H | Cl | H | F | - | $OCH_3$ | $OCH_3$ | CH | |

Table I (continued)

| Q | n | R | $R_1$ | $R_7$ | $R_8$ | $R_9$ | $R_{10}$ | $R_{11}$ | X | Y | Z | m.p.(°C) |
|---|---|---|---|---|---|---|---|---|---|---|---|---|
| Q-1 | 1 | H | H | H | Cl | H | Cl | - | Cl | $OCH_3$ | CH | |
| Q-1 | 1 | H | H | H | Cl | H | Cl | - | $OCH_3$ | $OCH_3$ | N | |
| Q-1 | 1 | H | H | H | Cl | H | Br | - | $CH_3$ | $OCH_3$ | CH | |
| Q-1 | 1 | H | H | H | $SO_2CH_3$ | H | $CH_3$ | - | $CH_3$ | $CH_3$ | CH | |
| Q-1 | 1 | H | H | H | $SO_2CH_3$ | H | $CH_3$ | - | $OCH_3$ | $CH_3$ | CH | |
| Q-1 | 1 | H | H | H | $SO_2CH_3$ | H | $CH_3$ | - | $OCH_3$ | $OCH_3$ | CH | |
| Q-1 | 1 | H | H | H | $SO_2CH_3$ | H | $CH_3$ | - | $CH_3$ | $CH_3$ | N | |
| Q-1 | 1 | H | H | H | $SO_2CH_3$ | H | $CH_3$ | - | $CH_3$ | $OCH_3$ | N | |
| Q-1 | 1 | H | H | H | $SO_2CH_3$ | H | $CH_3$ | - | $OCH_3$ | $OCH_3$ | N | |
| Q-1 | 1 | H | H | H | $SO_2CH_3$ | H | $CH_3$ | - | Cl | $OCH_3$ | CH | |
| Q-1 | 1 | H | H | H | $SO_2CH_3$ | H | Cl | - | $OCH_3$ | $OCH_3$ | CH | |
| Q-1 | 1 | H | H | H | $SO_2CH_3$ | H | Cl | - | $CH_3$ | $OCH_3$ | N | |
| Q-1 | 1 | H | H | H | $SO_2CH_3$ | H | F | - | $OCH_3$ | $OCH_3$ | N | |
| Q-1 | 1 | H | H | H | $SO_2CH_3$ | H | Br | - | $CH_3$ | $CH_3$ | CH | |
| Q-1 | 1 | H | H | F | F | H | H | - | $CH_3$ | $OCH_3$ | CH | |
| Q-1 | 1 | H | H | F | F | H | H | - | $OCH_3$ | $OCH_3$ | CH | |
| Q-1 | 1 | H | H | F | F | H | H | - | $CH_3$ | $CH_3$ | N | |
| Q-1 | 1 | H | H | F | F | H | H | - | $CH_3$ | $OCH_3$ | N | |
| Q-1 | 1 | H | H | F | F | H | H | - | $OCH_3$ | $OCH_3$ | N | |
| Q-1 | 1 | H | H | F | F | H | H | - | Cl | $OCH_3$ | CH | |
| Q-1 | 1 | H | H | Cl | Cl | H | H | - | $CH_3$ | $OCH_3$ | CH | |
| Q-1 | 1 | H | H | Cl | Cl | H | H | - | $OCH_3$ | $OCH_3$ | CH | |
| Q-1 | 1 | H | H | Cl | Cl | H | H | - | $CH_3$ | $OCH_3$ | N | |
| Q-1 | 1 | H | H | Cl | Cl | H | H | - | $OCH_3$ | $OCH_3$ | N | |
| Q-1 | 1 | H | H | Cl | Cl | H | H | - | $CH_3$ | $CH_3$ | CH | |
| Q-1 | 1 | H | H | Cl | Cl | H | H | - | $CH_3$ | $CH_3$ | N | |
| Q-1 | 1 | H | H | Cl | Cl | H | H | - | Cl | $OCH_3$ | CH | |
| Q-1 | 1 | H | H | Br | Br | H | H | - | $CH_3$ | $OCH_3$ | N | |
| Q-1 | 1 | H | H | Br | Br | H | H | - | $OCH_3$ | $OCH_3$ | N | |
| Q-1 | 1 | H | H | Br | Br | H | H | - | $CH_3$ | $OCH_3$ | CH | |
| Q-1 | 1 | H | H | Br | Br | H | H | - | $OCH_3$ | $OCH_3$ | CH | |
| Q-1 | 1 | H | H | Br | Br | H | H | - | $CH_3$ | $CH_3$ | N | |

Table I (continued)

| Q | n | R | $R_1$ | $R_7$ | $R_8$ | $R_9$ | $R_{10}$ | $R_{11}$ | X | Y | Z | m.p.(°C) |
|---|---|---|---|---|---|---|---|---|---|---|---|---|
| Q-1 | 1 | H | H | I | I | H | H | - | $OCH_3$ | $OCH_3$ | CH | |
| Q-1 | 1 | H | H | CN | CN | H | H | - | Cl | $OCH_3$ | CH | |
| Q-1 | 1 | H | H | CN | CN | H | H | - | $OCH_3$ | $OCH_3$ | N | |
| Q-1 | 1 | H | H | CN | CN | H | H | - | $CH_3$ | $OCH_3$ | CH | |
| Q-1 | 1 | H | H | F | F | H | $CH_3$ | - | $CH_3$ | $CH_3$ | CH | |
| Q-1 | 1 | H | H | F | F | H | $CH_3$ | - | $OCH_3$ | $CH_3$ | CH | |
| Q-1 | 1 | H | H | F | F | H | F | - | $OCH_3$ | $OCH_3$ | CH | |
| Q-1 | 1 | H | H | F | F | H | Cl | - | $CH_3$ | $CH_3$ | N | |
| Q-1 | 1 | H | H | F | F | H | Br | - | $CH_3$ | $OCH_3$ | N | |
| Q-1 | 1 | H | H | Cl | Cl | H | $CH_3$ | - | $OCH_3$ | $OCH_3$ | N | |
| Q-1 | 1 | H | H | Cl | Cl | H | $CH_3$ | - | $CH_3$ | $OCH_3$ | CH | |
| Q-1 | 1 | H | H | Cl | Cl | H | Cl | - | $OCH_3$ | $OCH_3$ | CH | |
| Q-1 | 1 | H | H | Cl | Cl | H | F | - | $CH_3$ | $OCH_3$ | N | |
| Q-1 | 1 | H | H | Cl | Cl | H | Br | - | $OCH_3$ | $OCH_3$ | N | |
| Q-1 | 1 | H | H | Br | Br | H | $CH_3$ | - | $CH_3$ | $CH_3$ | CH | |
| Q-1 | 1 | H | H | Br | Br | H | $CH_3$ | - | $CH_3$ | $OCH_3$ | CH | |
| Q-1 | 1 | H | H | Br | Br | H | Cl | - | $OCH_3$ | $OCH_3$ | CH | |
| Q-1 | 1 | H | H | Br | Br | H | Cl | - | $CH_3$ | $CH_3$ | N | |
| Q-1 | 1 | H | H | Br | Br | Cl | H | - | $CH_3$ | $OCH_3$ | N | |
| Q-1 | 1 | H | H | Br | Br | H | F | - | $OCH_3$ | $OCH_3$ | N | |
| Q-1 | 1 | H | H | Br | Br | H | Br | - | Cl | $OCH_3$ | CH | |
| Q-1 | 1 | H | H | CN | CN | H | $CH_3$ | - | $CH_3$ | $OCH_3$ | CH | |
| Q-1 | 1 | H | H | CN | CN | H | $CH_3$ | - | $OCH_3$ | $OCH_3$ | CH | |
| Q-1 | 1 | H | H | CN | CN | H | F | - | $CH_3$ | $OCH_3$ | N | |
| Q-1 | 1 | H | H | $CH_3$ | $CH_3$ | H | $CH_3$ | - | $OCH_3$ | $OCH_3$ | N | |
| Q-1 | 1 | H | H | $CH_3$ | $CH_3$ | H | $CH_3$ | - | $CH_3$ | $CH_3$ | CH | |
| Q-1 | 1 | H | H | $CH_3$ | $CH_3$ | H | $CH_3$ | - | $CH_3$ | $OCH_3$ | CH | |
| Q-1 | 1 | H | H | $CH_3$ | $CH_3$ | $CH_3$ | H | - | $OCH_3$ | $OCH_3$ | CH | |
| Q-1 | 1 | H | H | $CH_3$ | $CH_3$ | H | F | - | $CH_3$ | $OCH_3$ | N | |
| Q-1 | 1 | H | H | $CH_3$ | $CH_3$ | H | F | - | $OCH_3$ | $OCH_3$ | N | |
| Q-1 | 1 | H | H | $CH_3$ | $CH_3$ | H | Cl | - | $CH_3$ | $OCH_3$ | CH | |
| Q-1 | 1 | H | H | $CH_3$ | $CH_3$ | H | Cl | - | $OCH_3$ | $OCH_3$ | CH | |

Table I (continued)

| Q | n | R | $R_1$ | $R_7$ | $R_8$ | $R_9$ | $R_{10}$ | $R_{11}$ | X | Y | Z | m.p. (°C) |
|---|---|---|---|---|---|---|---|---|---|---|---|---|
| Q-1 | 1 | H | H | $CH_3$ | $CH_3$ | H | Br | - | $CH_3$ | $OCH_3$ | CH | |
| Q-1 | 1 | H | H | $OCH_3$ | $OCH_3$ | H | $CH_3$ | - | $OCH_3$ | $OCH_3$ | CH | |
| Q-1 | 1 | H | H | $OCH_3$ | $OCH_3$ | H | $CH_3$ | - | Cl | $OCH_3$ | CH | |
| Q-1 | 1 | H | H | $OCH_3$ | $OCH_3$ | H | F | - | $OCH_3$ | $OCH_3$ | N | |
| Q-1 | 1 | H | H | CN | CN | $CH_3$ | $CH_3$ | - | $CH_3$ | $CH_3$ | CH | |
| Q-1 | 1 | H | H | CN | CN | F | F | - | $OCH_3$ | $CH_3$ | CH | |
| Q-1 | 1 | H | H | CN | CN | F | F | - | $OCH_3$ | $OCH_3$ | CH | |
| Q-1 | 1 | H | H | CN | CN | Cl | Cl | - | $CH_3$ | $CH_3$ | N | |
| Q-1 | 1 | H | H | CN | CN | Br | Br | - | $CH_3$ | $OCH_3$ | N | |
| Q-1 | 1 | H | H | CN | CN | Br | Br | - | $OCH_3$ | $OCH_3$ | N | |
| Q-1 | 1 | H | H | $CH_3$ | $CH_3$ | $CH_3$ | $CH_3$ | - | $CH_3$ | $OCH_3$ | CH | |
| Q-1 | 1 | H | H | $CH_3$ | $CH_3$ | $CH_3$ | $CH_3$ | - | $OCH_3$ | $OCH_3$ | CH | |
| Q-1 | 1 | H | H | $CH_3$ | $CH_3$ | $CH_3$ | $CH_3$ | - | $CH_3$ | $OCH_3$ | N | |
| Q-1 | 1 | H | H | $CH_3$ | $CH_3$ | F | F | - | $OCH_3$ | $OCH_3$ | N | |
| Q-1 | 1 | H | H | $CH_3$ | $CH_3$ | Cl | Cl | - | $CH_3$ | $CH_3$ | CH | |
| Q-1 | 1 | H | H | $CH_3$ | $CH_3$ | Cl | Cl | - | $CH_3$ | $OCH_3$ | CH | |
| Q-1 | 1 | H | H | $CH_3$ | $CH_3$ | Cl | Cl | - | $OCH_3$ | $OCH_3$ | CH | |
| Q-1 | 1 | H | H | $CH_3$ | $CH_3$ | Br | Br | - | $CH_3$ | $CH_3$ | N | |
| Q-1 | 1 | H | H | $CH_3$ | $CH_3$ | Br | Br | - | $CH_3$ | $OCH_3$ | N | |
| Q-1 | 1 | H | H | $OCH_3$ | $OCH_3$ | $CH_3$ | $CH_3$ | - | Cl | $OCH_3$ | CH | |
| Q-1 | 1 | H | H | $OCH_3$ | $OCH_3$ | $CH_3$ | $CH_3$ | - | $CH_3$ | $OCH_3$ | CH | |
| Q-1 | 1 | H | H | $OCH_3$ | $OCH_3$ | Cl | Cl | - | $OCH_3$ | $OCH_3$ | CH | |
| Q-1 | 1 | H | H | $OCH_3$ | $OCH_3$ | F | F | - | $CH_3$ | $OCH_3$ | N | |
| Q-1 | 1 | H | H | $OCH_3$ | $OCH_3$ | F | F | - | $OCH_3$ | $OCH_3$ | N | |
| Q-1 | 1 | H | H | $OCH_3$ | $OCH_3$ | F | F | - | $CH_3$ | $CH_3$ | CH | |
| Q-1 | 1 | H | H | $OCH_3$ | $OCH_3$ | Br | Br | - | $CH_3$ | $OCH_3$ | CH | |
| Q-1 | 1 | H | H | Cl | $OCH_3$ | H | H | - | $CH_3$ | $CH_3$ | CH | |
| Q-1 | 1 | H | H | Cl | $OCH_3$ | H | H | - | $OCH_3$ | $CH_3$ | CH | |
| Q-1 | 1 | H | H | Cl | $CO_2CH_3$ | H | H | - | $OCH_3$ | $OCH_3$ | CH | |
| Q-1 | 1 | H | H | Cl | $SO_2N(CH_3)_2$ | H | H | - | $CH_3$ | $CH_3$ | N | |
| Q-1 | 1 | H | H | Cl | $SCH_3$ | H | H | - | $CH_3$ | $OCH_3$ | N | |
| Q-1 | 1 | H | H | Cl | $SO_2CH_3$ | H | H | - | $OCH_3$ | $OCH_3$ | N | |

Table I (continued)

| Q | n | R | $R_1$ | $R_7$ | $R_8$ | $R_9$ | $R_{10}$ | $R_{11}$ | X | Y | Z | m.p. (°C) |
|---|---|---|---|---|---|---|---|---|---|---|---|---|
| Q-1 | 1 | H | H | Br | F | H | H | - | $CH_3$ | $OCH_3$ | CH | |
| Q-1 | 1 | H | H | Br | F | H | H | - | $OCH_3$ | $OCH_3$ | CH | |
| Q-1 | 1 | H | H | Br | F | H | H | - | $CH_3$ | $OCH_3$ | N | |
| Q-1 | 1 | H | H | Br | Cl | H | H | - | $OCH_3$ | $OCH_3$ | N | |
| Q-1 | 1 | H | H | Br | Cl | H | H | - | $CH_3$ | $CH_3$ | CH | |
| Q-1 | 1 | H | H | Br | Cl | H | H | - | $CH_3$ | $OCH_3$ | CH | |
| Q-1 | 1 | H | H | Br | Br | H | H | - | Cl | $OCH_3$ | CH | |
| Q-1 | 1 | H | H | Br | CN | H | H | - | $CH_3$ | $CH_3$ | N | |
| Q-1 | 1 | H | H | Br | $CH_3$ | H | H | - | $CH_3$ | $OCH_3$ | N | |
| Q-1 | 1 | H | H | Br | $CH_3$ | H | H | - | $OCH_3$ | $OCH_3$ | N | |
| Q-1 | 1 | H | H | Br | $CH_3$ | H | H | - | Cl | $OCH_3$ | CH | |
| Q-1 | 1 | H | H | Br | $CH_2CH_3$ | H | H | - | $CH_3$ | $OCH_3$ | CH | |
| Q-1 | 1 | H | H | Br | $OCH_3$ | H | H | - | $OCH_3$ | $OCH_3$ | CH | |
| Q-1 | 1 | H | H | Br | $OCH_3$ | H | H | - | $CH_3$ | $OCH_3$ | N | |
| Q-1 | 1 | H | H | Br | $OCH_2CH_3$ | H | H | - | $OCH_3$ | $OCH_3$ | N | |
| Q-1 | 1 | H | H | Br | $CO_2CH_3$ | H | H | - | $CH_3$ | $CH_3$ | CH | |
| Q-1 | 1 | H | H | Br | $SO_2N(CH_3)_2$ | H | H | - | $CH_3$ | $OCH_3$ | CH | |
| Q-1 | 1 | H | H | Br | $SCH_3$ | H | H | - | $OCH_3$ | $OCH_3$ | CH | |
| Q-1 | 1 | H | H | Br | $SO_2CH_3$ | H | H | - | $CH_3$ | $OCH_3$ | N | |
| Q-1 | 1 | H | H | I | F | H | H | - | $OCH_3$ | $OCH_3$ | N | |
| Q-1 | 1 | H | H | I | Cl | H | H | - | $CH_3$ | $OCH_3$ | CH | |
| Q-1 | 1 | H | H | I | Br | H | H | - | $OCH_3$ | $OCH_3$ | CH | |
| Q-1 | 1 | H | H | I | $CH_3$ | H | H | - | $CH_3$ | $OCH_3$ | CH | |
| Q-1 | 1 | H | H | CN | F | H | H | - | $CH_3$ | $OCH_3$ | N | |
| Q-1 | 1 | H | H | CN | Cl | H | H | - | $OCH_3$ | $OCH_3$ | CH | |
| Q-1 | 1 | H | H | CN | Br | H | H | - | Cl | $OCH_3$ | CH | |
| Q-1 | 1 | H | H | CN | $CH_3$ | H | H | - | $OCH_3$ | $OCH_3$ | N | |
| Q-1 | 1 | H | H | CN | $CH_2CH_3$ | H | H | - | $CH_3$ | $OCH_3$ | CH | |
| Q-1 | 1 | H | H | F | Cl | F | Cl | - | $OCH_3$ | $OCH_3$ | CH | |
| Q-1 | 1 | H | H | F | Cl | F | $CH_3$ | - | $CH_3$ | $OCH_3$ | N | |
| Q-1 | 1 | H | H | F | Br | Cl | $CH_3$ | - | $OCH_3$ | $OCH_3$ | N | |
| Q-1 | 1 | H | H | Cl | $CH_3$ | $CH_3$ | F | - | $CH_3$ | $CH_3$ | CH | |

Table I (continued)

| Q | n | R | $R_1$ | $R_7$ | $R_8$ | $R_9$ | $R_{10}$ | $R_{11}$ | X | Y | Z | m.p. (°C) |
|---|---|---|---|---|---|---|---|---|---|---|---|---|
| Q-1 | 1 | H | H | Br | $OCH_3$ | $CH_3$ | F | - | $CH_3$ | $OCH_3$ | CH | |
| Q-1 | 1 | H | H | Br | Cl | F | Cl | - | $CH_3$ | $OCH_3$ | N | |
| Q-1 | 1 | H | H | Br | I | F | Cl | - | $OCH_3$ | $OCH_3$ | N | |
| Q-1 | 1 | H | H | $CH_3$ | $CH_2CH_3$ | $CH_3$ | Br | - | $CH_3$ | $OCH_3$ | CH | |
| Q-1 | 1 | H | H | Cl | Br | Cl | Br | - | $OCH_3$ | $OCH_3$ | CH | |
| Q-1 | 1 | H | H | Cl | Br | Cl | $CH_3$ | - | $CH_3$ | $OCH_3$ | CH | |
| Q-1 | 1 | H | H | $CO_2CH_3$ | $SCH_3$ | F | Cl | - | $CH_3$ | $OCH_3$ | N | |
| Q-1 | 1 | H | H | I | F | F | Br | - | $OCH_3$ | $OCH_3$ | CH | |
| Q-1 | 1 | H | H | CN | F | F | Cl | - | Cl | $OCH_3$ | CH | |
| Q-1 | 1 | H | H | CN | Br | Cl | $CH_3$ | - | $OCH_3$ | $OCH_3$ | N | |

| Q | n | R | $R_1$ | $R_7$ | $R_8$ | $R_{11}$ | X | Y | Z | m.p.(°C) |
|---|---|---|---|---|---|---|---|---|---|---|
| Q-2 | 0 | H | H | H | H | H | $CH_3$ | $CH_3$ | CH | 158-160 |
| Q-2 | 0 | H | H | H | H | H | $CH_3$ | $OCH_3$ | CH | |
| Q-2 | 0 | H | H | H | H | H | $OCH_3$ | $OCH_3$ | CH | 177-179 |
| Q-2 | 0 | H | H | H | H | H | $CH_3$ | $CH_3$ | N | |
| Q-2 | 0 | H | H | H | H | H | $CH_3$ | $OCH_3$ | N | |
| Q-2 | 0 | H | H | H | H | H | $OCH_3$ | $OCH_3$ | N | |
| Q-2 | 0 | H | H | H | H | H | Cl | $OCH_3$ | CH | |
| Q-2 | 0 | H | H | H | $CH_3$ | H | $CH_3$ | $OCH_3$ | CH | |
| Q-2 | 0 | H | H | H | $CH_3$ | H | $OCH_3$ | $OCH_3$ | CH | |
| Q-2 | 0 | H | H | H | $CH_3$ | H | $OCH_3$ | $OCH_3$ | N | |
| Q-2 | 0 | H | H | H | $CH_3$ | H | $CH_3$ | $OCH_3$ | CH | |
| Q-2 | 0 | H | H | H | Cl | H | $CH_3$ | $OCH_3$ | CH | |
| Q-2 | 0 | H | H | H | Cl | H | $OCH_3$ | $CH_3$ | N | |
| Q-2 | 0 | H | H | H | Cl | H | $OCH_3$ | $OCH_3$ | CH | |
| Q-2 | 0 | H | H | H | Cl | H | Cl | $OCH_3$ | CH | |
| Q-2 | 0 | H | H | H | F | H | $CH_3$ | $CH_3$ | N | |
| Q-2 | 0 | H | H | H | F | H | $CH_3$ | $CH_3$ | CH | |

Table I (continued)

| Q | n | R | $R_1$ | $R_7$ | $R_8$ | $R_{11}$ | X | Y | Z | m.p.(°C) |
|---|---|---|---|---|---|---|---|---|---|---|
| Q-2 | 0 | H | H | H | F | H | $OCH_3$ | $CH_3$ | CH | |
| Q-2 | 0 | H | H | H | F | H | $OCH_3$ | $OCH_3$ | CH | |
| Q-2 | 0 | H | H | H | F | H | $CH_3$ | $OCH_3$ | N | |
| Q-2 | 0 | H | H | H | F | H | Cl | $OCH_3$ | CH | |
| Q-2 | 0 | H | H | H | CN | H | $CH_3$ | $OCH_3$ | N | |
| Q-2 | 0 | H | H | H | CN | H | $OCH_3$ | $OCH_3$ | CH | |
| Q-2 | 0 | H | H | H | $OCH_3$ | H | $CH_3$ | $OCH_3$ | N | |
| Q-2 | 0 | H | H | H | $OCH_3$ | H | $OCH_3$ | $OCH_3$ | CH | |
| Q-2 | 0 | H | H | H | $OCH_3$ | H | $OCH_3$ | $OCH_3$ | N | |
| Q-2 | 0 | H | H | H | $CO_2CH_3$ | H | $CH_3$ | $CH_3$ | N | |
| Q-2 | 0 | H | H | H | $CO_2CH_3$ | H | $OCH_3$ | $CH_3$ | CH | |
| Q-2 | 0 | H | H | H | $CO_2CH_3$ | H | $OCH_3$ | $OCH_3$ | CH | |
| Q-2 | 0 | H | H | H | $CO_2CH_3$ | H | $CH_3$ | $OCH_3$ | N | |
| Q-2 | 0 | H | H | H | $CO_2CH_3$ | H | $CH_3$ | $CH_3$ | CH | |
| Q-2 | 0 | H | H | H | $CO_2CH_3$ | H | $OCH_3$ | $OCH_3$ | N | |
| Q-2 | 0 | H | H | H | $CO_2CH_3$ | H | Cl | $OCH_3$ | CH | |
| Q-2 | 0 | H | H | H | $SO_2N(CH_3)_2$ | H | $CH_3$ | $CH_3$ | N | |
| Q-2 | 0 | H | H | H | $SO_2N(CH_3)_2$ | H | $OCH_3$ | $OCH_3$ | CH | |
| Q-2 | 0 | H | H | H | $SO_2N(CH_3)_2$ | H | $OCH_3$ | $OCH_3$ | N | |
| Q-2 | 0 | H | H | H | $SO_2N(CH_3)_2$ | H | $CH_3$ | $OCH_3$ | CH | |
| Q-2 | 0 | H | H | H | $SCH_3$ | H | $OCH_3$ | $OCH_3$ | CH | |
| Q-2 | 0 | H | H | H | $SCH_3$ | H | $CH_3$ | $CH_3$ | CH | |
| Q-2 | 0 | H | H | H | $SCH_3$ | H | $OCH_3$ | $CH_3$ | CH | |
| Q-2 | 0 | H | H | H | $SCH_3$ | H | Cl | $OCH_3$ | CH | |
| Q-2 | 0 | H | H | H | $SO_2CH_3$ | H | $CH_3$ | $CH_3$ | CH | |
| Q-2 | 0 | H | H | H | $SO_2CH_3$ | H | $CH_3$ | $OCH_3$ | CH | |
| Q-2 | 0 | H | H | H | $SO_2CH_3$ | H | $CH_3$ | $OCH_3$ | N | |
| Q-2 | 0 | H | H | F | F | H | $CH_3$ | $CH_3$ | CH | |
| Q-2 | 0 | H | H | F | F | H | $OCH_3$ | $CH_3$ | CH | |
| Q-2 | 0 | H | H | Cl | Cl | H | $OCH_3$ | $OCH_3$ | CH | |
| Q-2 | 0 | H | H | Cl | Cl | H | $CH_3$ | $CH_3$ | N | |
| Q-2 | 0 | H | H | F | Cl | H | $OCH_3$ | $CH_3$ | N | |

Table I (continued)

| Q | n | R | $R_1$ | $R_7$ | $R_8$ | $R_{11}$ | X | Y | Z | m.p. (°C) |
|---|---|---|---|---|---|---|---|---|---|---|
| Q-2 | 0 | H | H | F | $CH_3$ | H | $CH_3$ | $CH_3$ | CH | |
| Q-2 | 0 | H | H | $CH_3$ | $CH_3$ | H | $OCH_3$ | $OCH_3$ | CH | |
| Q-2 | 0 | H | H | $CH_3$ | $CH_2CH_3$ | H | $OCH_3$ | $OCH_3$ | N | |
| Q-2 | 0 | H | H | F | Br | H | Cl | $OCH_3$ | CH | |
| Q-2 | 0 | H | H | Br | Br | H | Cl | $OCH_3$ | CH | |
| Q-2 | 0 | H | H | Br | Br | H | $OCH_3$ | $OCH_3$ | CH | |
| Q-2 | 0 | H | H | $CO_2CH_3$ | $CO_2CH_3$ | H | $CH_3$ | $CH_3$ | N | |
| Q-2 | 0 | H | H | $CO_2CH_3$ | $CO_2CH_3$ | H | $CH_3$ | $OCH_3$ | CH | |
| Q-2 | 0 | H | H | $CO_2CH_3$ | $CO_2CH_3$ | H | $OCH_3$ | $OCH_3$ | CH | |
| Q-2 | 0 | H | H | CN | CN | H | $CH_3$ | $OCH_3$ | CH | |
| Q-2 | 0 | H | H | CN | CN | H | $OCH_3$ | $OCH_3$ | CH | |
| Q-2 | 0 | H | H | CN | CN | H | $OCH_3$ | $OCH_3$ | N | |
| Q-2 | 0 | H | H | $OCH_3$ | $OCH_3$ | H | $CH_3$ | $OCH_3$ | CH | |
| Q-2 | 0 | H | H | $OCH_2CH_3$ | $OCH_2CH_3$ | H | $OCH_3$ | $CH_3$ | CH | |
| Q-2 | 0 | H | H | $SCH_3$ | $SCH_3$ | H | $OCH_3$ | $OCH_3$ | CH | |
| Q-2 | 0 | H | H | $SCH_3$ | $SCH_3$ | H | $CH_3$ | $CH_3$ | N | |
| Q-2 | 0 | H | H | $SO_2CH_3$ | $SO_2CH_3$ | H | $OCH_3$ | $OCH_3$ | CH | |
| Q-2 | 0 | H | H | $SO_2CH_3$ | $SO_2CH_3$ | H | $OCH_3$ | $OCH_3$ | N | |
| Q-2 | 0 | H | H | H | H | $CH_3$ | $CH_3$ | $CH_3$ | CH | |
| Q-2 | 0 | H | H | H | H | $CH_3$ | $CH_3$ | $OCH_3$ | CH | |
| Q-2 | 0 | H | H | H | H | $CH_3$ | $OCH_3$ | $OCH_3$ | CH | |
| Q-2 | 0 | H | H | H | H | $CH_2CH_3$ | $CH_3$ | $CH_3$ | N | |
| Q-2 | 0 | H | H | F | H | $CH_3$ | $CH_3$ | $OCH_3$ | N | |
| Q-2 | 0 | H | H | Cl | H | $CH_3$ | $OCH_3$ | $OCH_3$ | N | |
| Q-2 | 0 | H | H | F | F | $CH_3$ | $CH_3$ | $OCH_3$ | CH | |
| Q-2 | 0 | H | H | Br | H | $CH_3$ | $OCH_3$ | $OCH_3$ | CH | |
| Q-2 | 0 | H | H | Cl | Cl | $CH_3$ | $CH_3$ | $OCH_3$ | N | |
| Q-2 | 0 | H | 5-$CH_3$ | H | H | - | $OCH_3$ | $OCH_3$ | N | |
| Q-2 | 0 | H | 5-$OCH_3$ | H | H | - | $CH_3$ | $CH_3$ | CH | |
| Q-2 | 0 | H | 5-$SCH_3$ | H | H | - | $CH_3$ | $OCH_3$ | CH | |
| Q-2 | 0 | H | 5-Cl | Cl | H | - | $OCH_3$ | $OCH_3$ | CH | |
| Q-2 | 0 | H | 6-Cl | F | F | $CH_3$ | $CH_3$ | $CH_3$ | N | |

Table I (continued)

| Q | n | R | $R_1$ | $R_7$ | $R_8$ | $R_{11}$ | X | Y | Z | m.p.(°C) |
|---|---|---|---|---|---|---|---|---|---|---|
| Q-2 | 0 | $CH_3$ | H | H | H | H | $OCH_3$ | $OCH_3$ | N | |
| Q-2 | 1 | H | H | H | H | H | $OCH_3$ | $OCH_3$ | CH | |
| Q-2 | 1 | H | H | F | F | H | $CH_3$ | $OCH_3$ | CH | |
| Q-2 | 1 | H | H | F | Cl | H | $CH_3$ | $CH_3$ | N | |
| Q-2 | 1 | H | H | $CH_3$ | Cl | H | $OCH_3$ | $CH_3$ | CH | |
| Q-2 | 1 | H | H | F | H | H | $CH_3$ | $OCH_3$ | CH | |
| Q-2 | 1 | H | H | Cl | H | H | $OCH_3$ | $OCH_3$ | CH | |
| Q-2 | 1 | H | H | Br | H | H | $CH_3$ | $CH_3$ | N | |
| Q-2 | 1 | H | H | Cl | Cl | H | $CH_3$ | $OCH_3$ | CH | |
| Q-2 | 1 | H | H | $CH_3$ | H | H | $CH_3$ | $OCH_3$ | CH | |
| Q-2 | 1 | H | H | CH | H | H | $OCH_3$ | $OCH_3$ | CH | |
| Q-2 | 1 | H | H | CH | CH | H | $CH_3$ | $OCH_3$ | N | |
| Q-2 | 1 | H | H | $CH_3$ | Br | H | $CH_3$ | $CH_3$ | CH | |
| Q-2 | 1 | H | H | F | Br | H | Cl | $OCH_3$ | CH | |
| Q-2 | 1 | H | 5-$CH_3$ | F | H | H | $OCH_3$ | $OCH_3$ | CH | |
| Q-2 | 1 | H | 5-$OCH_3$ | Cl | H | $CH_3$ | $CH_3$ | $CH_3$ | N | |
| Q-2 | 1 | H | 5-Cl | Cl | Cl | $CH_2CH_3$ | $CH_3$ | $OCH_3$ | N | |

| Q | n | R | $R_1$ | $R_3$ | $R_7$ | $R_8$ | $R_{11}$ | X | Y | Z | m.p.(°C) |
|---|---|---|---|---|---|---|---|---|---|---|---|
| Q-3 | 0 | H | H | $CH_3$ | H | H | H | $CH_3$ | $CH_3$ | CH | |
| Q-3 | 0 | H | H | $CH_3$ | H | H | H | $CH_3$ | $OCH_3$ | CH | |
| Q-3 | 0 | H | H | $CH_3$ | H | H | H | $OCH_3$ | $OCH_3$ | CH | |
| Q-3 | 0 | H | H | $CH_3$ | H | H | H | $CH_3$ | $CH_3$ | N | |
| Q-3 | 0 | H | H | $CH_3$ | H | H | H | $CH_3$ | $OCH_3$ | N | |
| Q-3 | 0 | H | H | $CH_3$ | H | H | H | Cl | $OCH_3$ | CH | |
| Q-3 | 0 | H | H | $CH_3$ | H | $CH_3$ | H | $CH_3$ | $OCH_3$ | CH | |
| Q-3 | 0 | H | H | $CH_3$ | H | $CH_3$ | H | $OCH_3$ | $OCH_3$ | CH | |
| Q-3 | 0 | H | H | $CH_3$ | H | $CH_3$ | H | $OCH_3$ | $OCH_3$ | N | |
| Q-3 | 0 | H | H | $CH_3$ | H | $CH_3$ | H | $CH_3$ | $OCH_3$ | CH | |
| Q-3 | 0 | H | H | $CH_3$ | H | Cl | H | $CH_3$ | $OCH_3$ | CH | |

Table I (continued)

| Q | n | R | $R_1$ | $R_3$ | $R_7$ | $R_8$ | $R_{11}$ | X | Y | Z | m.p.(°C) |
|---|---|---|---|---|---|---|---|---|---|---|---|
| Q-3 | 0 | H | H | $CH_3$ | H | Cl | H | $OCH_3$ | $OCH_3$ | N | |
| Q-3 | 0 | H | H | $CH_3$ | H | Cl | H | $OCH_3$ | $OCH_3$ | CH | |
| Q-3 | 0 | H | H | $CH_3$ | H | Cl | H | Cl | $OCH_3$ | CH | |
| Q-3 | 0 | H | H | $CH_3$ | H | F | H | $CH_3$ | $CH_3$ | N | |
| Q-3 | 0 | H | H | $CH_3$ | H | F | H | $CH_3$ | $CH_3$ | CH | |
| Q-3 | 0 | H | H | $CH_3$ | H | F | H | $OCH_3$ | $CH_3$ | CH | |
| Q-3 | 0 | H | H | $CH_3$ | H | F | H | $OCH_3$ | $OCH_3$ | CH | |
| Q-3 | 0 | H | H | $CH_3$ | H | F | H | $CH_3$ | $OCH_3$ | N | |
| Q-3 | 0 | H | H | $CH_3$ | H | F | H | Cl | $OCH_3$ | CH | |
| Q-3 | 0 | H | H | $CH_3$ | H | CN | H | $CH_3$ | $OCH_3$ | N | |
| Q-3 | 0 | H | H | $CH_3$ | H | CN | H | $OCH_3$ | $OCH_3$ | CH | |
| Q-3 | 0 | H | H | $CH_3$ | H | $OCH_3$ | H | $CH_3$ | $OCH_3$ | N | |
| Q-3 | 0 | H | H | $CH_3$ | H | $OCH_3$ | H | $OCH_3$ | $OCH_3$ | CH | |
| Q-3 | 0 | H | H | $CH_3$ | H | $OCH_3$ | H | $OCH_3$ | $OCH_3$ | N | |
| Q-3 | 0 | H | H | $CH_3$ | H | $CO_2CH_3$ | H | $CH_3$ | $CH_3$ | N | |
| Q-3 | 0 | H | H | $CH_3$ | H | $CO_2CH_3$ | H | $OCH_3$ | $CH_3$ | CH | |
| Q-3 | 0 | H | H | $CH_3$ | H | $CO_2CH_3$ | H | $OCH_3$ | $OCH_3$ | CH | |
| Q-3 | 0 | H | H | $CH_3$ | H | $CO_2CH_3$ | H | $CH_3$ | $OCH_3$ | CH | |
| Q-3 | 0 | H | H | $CH_3$ | H | $CO_2CH_3$ | H | $CH_3$ | $CH_3$ | CH | |
| Q-3 | 0 | H | H | $CH_3$ | H | $CO_2CH_3$ | H | $OCH_3$ | $OCH_3$ | N | |
| Q-3 | 0 | H | H | $CH_3$ | H | $CO_2CH_3$ | H | Cl | $OCH_3$ | CH | |
| Q-3 | 0 | H | H | $CH_3$ | H | $SO_2N(CH_3)_2$ | H | $CH_3$ | $CH_3$ | N | |
| Q-3 | 0 | H | H | $CH_3$ | H | $SO_2N(CH_3)_2$ | H | $OCH_3$ | $OCH_3$ | CH | |
| Q-3 | 0 | H | H | $CH_3$ | H | $SO_2N(CH_3)_2$ | H | $OCH_3$ | $OCH_3$ | N | |
| Q-3 | 0 | H | H | $CH_3$ | H | $SO_2N(CH_3)_2$ | H | $CH_3$ | $OCH_3$ | CH | |
| Q-3 | 0 | H | H | $CH_3$ | H | $SCH_3$ | H | $OCH_3$ | $OCH_3$ | CH | |
| Q-3 | 0 | H | H | $CH_3$ | H | $SCH_3$ | H | $CH_3$ | $CH_3$ | Cl | |
| Q-3 | 0 | H | H | $CH_3$ | H | $SCH_3$ | H | $OCH_3$ | $CH_3$ | CH | |
| Q-3 | 0 | H | H | $CH_3$ | H | $SCH_3$ | H | Cl | $OCH_3$ | CH | |
| Q-3 | 0 | H | H | $CH_3$ | H | $SO_2CH_3$ | H | $CH_3$ | $CH_3$ | CH | |
| Q-3 | 0 | H | H | $CH_3$ | H | $SO_2CH_3$ | H | $CH_3$ | $OCH_3$ | CH | |
| Q-3 | 0 | H | H | $CH_3$ | H | $SO_2CH_3$ | H | $CH_3$ | $OCH_3$ | N | |

Table I (continued)

| Q | n | R | $R_1$ | $R_3$ | $R_7$ | $R_8$ | $R_{11}$ | X | Y | Z | m.p. (°C) |
|---|---|---|---|---|---|---|---|---|---|---|---|
| Q-3 | 0 | H | H | $CH_3$ | F | F | H | $CH_3$ | $CH_3$ | CH | |
| Q-3 | 0 | H | H | $CH_3$ | F | F | H | $OCH_3$ | $CH_3$ | Cl | |
| Q-3 | 0 | H | H | $CH_3$ | Cl | Cl | H | $OCH_3$ | $OCH_3$ | CH | |
| Q-3 | 0 | H | H | $CH_3$ | Cl | Cl | H | $CH_3$ | $CH_3$ | N | |
| Q-3 | 0 | H | H | $CH_3$ | F | Cl | H | $OCH_3$ | $CH_3$ | N | |
| Q-3 | 0 | H | H | $CH_3$ | F | $CH_3$ | H | $CH_3$ | $CH_3$ | CH | |
| Q-3 | 0 | H | H | $CH_3$ | $CH_3$ | $CH_3$ | H | $OCH_3$ | $OCH_3$ | CH | |
| Q-3 | 0 | H | H | $CH_3$ | $CH_3$ | $CH_2CH_3$ | H | $OCH_3$ | $OCH_3$ | N | |
| Q-3 | 0 | H | H | $CH_3$ | F | Br | H | Cl | $OCH_3$ | CH | |
| Q-3 | 0 | H | H | $CH_3$ | Br | Br | H | $OCH_3$ | $OCH_3$ | CH | |
| Q-3 | 0 | H | H | $CH_3$ | CN | CN | H | $CH_3$ | $OCH_3$ | CH | |
| Q-3 | 0 | H | H | $CH_3$ | CN | CN | H | $OCH_3$ | $OCH_3$ | CH | |
| Q-3 | 0 | H | H | $CH_3$ | CN | CN | H | $OCH_3$ | $OCH_3$ | N | |
| Q-3 | 0 | H | H | $CH_3$ | $OCH_3$ | $OCH_3$ | H | $CH_3$ | $OCH_3$ | CH | |
| Q-3 | 0 | H | H | $CH_3$ | H | H | $CH_3$ | $CH_3$ | $CH_3$ | CH | |
| Q-3 | 0 | H | H | $CH_3$ | H | H | $CH_3$ | $CH_3$ | $OCH_3$ | CH | |
| Q-3 | 0 | H | H | $CH_3$ | H | H | $OCH_3$ | $OCH_3$ | $CH_3$ | CH | |
| Q-3 | 0 | H | H | $CH_3$ | H | H | $CH_2CH_3$ | $OCH_3$ | $CH_3$ | N | |
| Q-3 | 0 | H | H | $CH_3$ | F | H | $CH_3$ | $CH_3$ | $OCH_3$ | N | |
| Q-3 | 0 | H | H | $CH_3$ | Cl | H | $CH_3$ | $OCH_3$ | $OCH_3$ | N | |
| Q-3 | 0 | H | H | $CH_3$ | F | F | $CH_3$ | $CH_3$ | $OCH_3$ | CH | |
| Q-3 | 0 | H | H | $CH_3$ | Br | H | $CH_3$ | $OCH_3$ | $OCH_3$ | CH | |
| Q-3 | 0 | H | H | $CH_3$ | Cl | Cl | $CH_3$ | $CH_3$ | $OCH_3$ | N | |
| Q-3 | 0 | H | 5-$CH_3$ | $CH_3$ | H | H | – | $OCH_3$ | $OCH_3$ | N | |
| Q-3 | 0 | H | 6-$OCH_3$ | $CH_3$ | H | H | – | $CH_3$ | $CH_3$ | CH | |
| Q-3 | 0 | H | 3-$SCH_3$ | $CH_3$ | H | H | – | $CH_3$ | $OCH_3$ | CH | |
| Q-3 | 0 | H | 5-Cl | $CH_3$ | Cl | H | – | $OCH_3$ | $OCH_3$ | CH | |
| Q-3 | 0 | H | 6-Cl | $CH_2CH_3$ | F | F | $CH_3$ | $CH_3$ | $CH_3$ | N | |
| Q-3 | 0 | H | 5-$OCH_2F$ | $CH_2CH_3$ | Cl | H | H | $OCH_3$ | $OCH_3$ | N | |
| Q-3 | 0 | $CH_3$ | H | $CH_2CH_3$ | H | H | H | $OCH_3$ | $OCH_3$ | N | |
| Q-3 | 1 | H | H | $CH_2CH_3$ | H | H | H | $OCH_3$ | $OCH_3$ | CH | |
| Q-3 | 1 | H | H | $CH_3$ | F | F | H | $CH_3$ | $OCH_3$ | CH | |

Table I (continued)

| Q | n | R | $R_1$ | $R_3$ | $R_7$ | $R_8$ | $R_{11}$ | X | Y | Z | m.p. (°C) |
|---|---|---|---|---|---|---|---|---|---|---|---|
| Q-3 | 1 | H | H | $CH_3$ | F | Cl | H | $CH_3$ | $OCH_3$ | N | |
| Q-3 | 1 | H | H | $CH_2CH_3CH_3$ | $CH_3$ | Cl | H | $OCH_3$ | $CH_3$ | CH | |
| Q-3 | 1 | H | H | $CH_3$ | F | H | H | $CH_3$ | $OCH_3$ | CH | |
| Q-3 | 1 | H | H | $CH_3$ | Cl | H | H | $OCH_3$ | $OCH_3$ | CH | |
| Q-3 | 1 | H | H | $CH_3$ | Br | H | H | $CH_3$ | $OCH_3$ | N | |
| Q-3 | 1 | H | H | $CH_2CH_3$ | Cl | Cl | H | $CH_3$ | $OCH_3$ | CH | |
| Q-3 | 1 | H | H | $CH_3$ | $CH_3$ | H | H | $CH_3$ | $OCH_3$ | CH | |
| Q-3 | 1 | H | H | $CH_3$ | CN | H | H | $OCH_3$ | $OCH_3$ | CH | |
| Q-3 | 1 | H | H | $CH_3$ | CN | CN | H | $CH_3$ | $OCH_3$ | N | |
| Q-3 | 1 | H | H | $CH_3$ | $CH_3$ | Br | H | $CH_3$ | $CH_3$ | CH | |
| Q-3 | 1 | H | H | $CH_3$ | F | Br | H | Cl | $OCH_3$ | CH | |
| Q-3 | 1 | H | 3-$CH_3$ | $CH_3$ | F | H | H | $OCH_3$ | $OCH_3$ | CH | |
| Q-3 | 1 | H | 5-$OCH_3$ | $CH_3$ | Cl | H | $CH_3$ | $CH_3$ | $CH_3$ | N | |
| Q-3 | 1 | H | 5-Cl | $CH_3$ | Cl | Cl | $CH_2CH_3$ | $CH_3$ | $OCH_3$ | N | |

| Q | n | R | $R_1$ | $R_7$ | $R_8$ | X | Y | Z | m.p.(°C) |
|---|---|---|---|---|---|---|---|---|---|
| Q-4 | 0 | H | H | H | H | $CH_3$ | $CH_3$ | CH | 197-199 |
| Q-4 | 0 | H | H | H | H | $CH_3$ | $OCH_3$ | CH | 192-194 |
| Q-4 | 0 | H | H | H | H | $OCH_3$ | $OCH_3$ | CH | 194-196 |
| Q-4 | 0 | H | H | H | H | $CH_3$ | $CH_3$ | N | |
| Q-4 | 0 | H | H | H | H | $CH_3$ | $OCH_3$ | N | 155-158 |
| Q-4 | 0 | H | H | H | H | $OCH_3$ | $OCH_3$ | N | 150-152 |
| Q-4 | 0 | H | H | H | H | Cl | $OCH_3$ | CH | 182-184 |
| Q-4 | 0 | H | H | H | $CH_3$ | $CH_3$ | $OCH_3$ | CH | |
| Q-4 | 0 | H | H | H | $CH_3$ | $OCH_3$ | $OCH_3$ | CH | |
| Q-4 | 0 | H | H | H | $CH_3$ | $OCH_3$ | $OCH_3$ | N | |
| Q-4 | 0 | H | H | H | $CH_3$ | $CH_3$ | $OCH_3$ | CH | |
| Q-4 | 0 | H | H | H | Cl | $CH_3$ | $OCH_3$ | CH | |
| Q-4 | 0 | H | H | H | Cl | $OCH_3$ | $CH_3$ | N | |
| Q-4 | 0 | H | H | H | Cl | $OCH_3$ | $OCH_3$ | CH | |

Table I (continued)

| Q | n | R | $R_1$ | $R_7$ | $R_8$ | X | Y | Z | m.p.(°C) |
|---|---|---|---|---|---|---|---|---|---|
| Q-4 | 0 | H | H | H | Cl | Cl | $OCH_3$ | CH | |
| Q-4 | 0 | H | H | H | F | $CH_3$ | $CH_3$ | N | |
| Q-4 | 0 | H | H | H | F | $CH_3$ | $CH_3$ | CH | |
| Q-4 | 0 | H | H | H | F | $OCH_3$ | $CH_3$ | CH | |
| Q-4 | 0 | H | H | H | F | $OCH_3$ | $OCH_3$ | CH | |
| Q-4 | 0 | H | H | H | F | $CH_3$ | $OCH_3$ | N | |
| Q-4 | 0 | H | H | H | F | Cl | $OCH_3$ | CH | |
| Q-4 | 0 | H | H | H | CN | $CH_3$ | $OCH_3$ | N | |
| Q-4 | 0 | H | H | H | CN | $OCH_3$ | $OCH_3$ | CH | |
| Q-4 | 0 | H | H | H | $OCH_3$ | $CH_3$ | $OCH_3$ | N | |
| Q-4 | 0 | H | H | H | $OCH_3$ | $OCH_3$ | $OCH_3$ | CH | |
| Q-4 | 0 | H | H | H | $OCH_3$ | $OCH_3$ | $OCH_3$ | N | |
| Q-4 | 0 | H | H | H | $CO_2CH_3$ | $CH_3$ | $CH_3$ | N | |
| Q-4 | 0 | H | H | H | $CO_2CH_3$ | $OCH_3$ | $CH_3$ | CH | |
| Q-4 | 0 | H | H | H | $CO_2CH_3$ | $OCH_3$ | $OCH_3$ | CH | |
| Q-4 | 0 | H | H | H | $CO_2CH_3$ | $CH_3$ | $OCH_3$ | N | |
| Q-4 | 0 | H | H | H | $CO_2CH_3$ | $CH_3$ | $CH_3$ | CH | |
| Q-4 | 0 | H | H | H | $CO_2CH_3$ | $OCH_3$ | $OCH_3$ | N | |
| Q-4 | 0 | H | H | H | $CO_2CH_3$ | Cl | $OCH_3$ | CH | |
| Q-4 | 0 | H | H | H | OH | $CH_3$ | $CH_3$ | CH | 148-150 |
| Q-4 | 0 | H | H | H | OH | $CH_3$ | $OCH_3$ | CH | 163-164 |
| Q-4 | 0 | H | H | H | OH | $OCH_3$ | $OCH_3$ | CH | 133-136 |
| Q-4 | 0 | H | H | H | OH | $CH_3$ | $OCH_3$ | N | 165-167 |
| Q-4 | 0 | H | H | H | OH | $OCH_3$ | $OCH_3$ | N | 160-162 |
| Q-4 | 0 | H | H | H | OH | Cl | $OCH_3$ | CH | 138-140 |
| Q-4 | 0 | H | H | H | $SO_2N(CH_3)_2$ | $CH_3$ | $OCH_3$ | N | |
| Q-4 | 0 | H | H | H | $SO_2N(CH_3)_2$ | $OCH_3$ | $OCH_3$ | CH | |
| Q-4 | 0 | H | H | H | $SO_2N(CH_3)_2$ | $OCH_3$ | $OCH_3$ | N | |
| Q-4 | 0 | H | H | H | $SO_2N(CH_3)_2$ | $CH_3$ | $OCH_3$ | CH | |
| Q-4 | 0 | H | H | H | $SCH_3$ | $OCH_3$ | $OCH_3$ | CH | |
| Q-4 | 0 | H | H | H | $SCH_3$ | $CH_3$ | $CH_3$ | CH | |
| Q-4 | 0 | H | H | H | $SCH_3$ | $OCH_3$ | $CH_3$ | CH | |

Table I (continued)

| Q | n | R | $R_1$ | $R_7$ | $R_8$ | X | Y | Z | m.p.(°C) |
|---|---|---|---|---|---|---|---|---|---|
| Q-4 | 0 | H | H | H | $SCH_3$ | Cl | $OCH_3$ | CH | |
| Q-4 | 0 | H | H | H | $SO_2CH_3$ | $CH_3$ | $CH_3$ | CH | |
| Q-4 | 0 | H | H | H | $SO_2CH_3$ | $CH_3$ | $OCH_3$ | CH | |
| Q-4 | 0 | H | H | H | $SO_2CH_3$ | $CH_3$ | $OCH_3$ | N | |
| Q-4 | 0 | H | H | F | F | $CH_3$ | $CH_3$ | CH | |
| Q-4 | 0 | H | H | F | F | $OCH_3$ | $CH_3$ | CH | |
| Q-4 | 0 | H | H | Cl | Cl | $OCH_3$ | $OCH_3$ | CH | |
| Q-4 | 0 | H | H | Cl | Cl | $CH_3$ | $CH_3$ | N | |
| Q-4 | 0 | H | H | F | Cl | $OCH_3$ | $CH_3$ | N | |
| Q-4 | 0 | H | H | F | $CH_3$ | $CH_3$ | $CH_3$ | CH | |
| Q-4 | 0 | H | H | $CH_3$ | $CH_3$ | $OCH_3$ | $OCH_3$ | CH | |
| Q-4 | 0 | H | H | $CH_3$ | $CH_2CH_3$ | $OCH_3$ | $OCH_3$ | N | |
| Q-4 | 0 | H | H | F | Br | Cl | $OCH_3$ | CH | |
| Q-4 | 0 | H | H | Br | Br | $OCH_3$ | $OCH_3$ | CH | |
| Q-4 | 0 | H | H | CN | CN | $CH_3$ | $OCH_3$ | CH | |
| Q-4 | 0 | H | H | CN | CN | $OCH_3$ | $OCH_3$ | CH | |
| Q-4 | 0 | H | H | CN | CN | $OCH_3$ | $OCH_3$ | N | |
| Q-4 | 0 | H | H | $OCH_3$ | $OCH_3$ | $CH_3$ | $OCH_3$ | CH | |
| Q-4 | 0 | H | 5-$CH_3$ | F | H | $CH_3$ | $OCH_3$ | N | |
| Q-4 | 0 | H | 6-$CH_3$ | Cl | H | $OCH_3$ | $OCH_3$ | N | |
| Q-4 | 0 | H | H | F | F | $CH_3$ | $OCH_3$ | CH | |
| Q-4 | 0 | H | 3-$CH_3$ | Br | H | $OCH_3$ | $OCH_3$ | CH | |
| Q-4 | 0 | H | H | Cl | Cl | $CH_3$ | $OCH_3$ | N | |
| Q-4 | 0 | H | 5-$CH_3$ | H | H | $OCH_3$ | $OCH_3$ | N | |
| Q-4 | 0 | H | 5-$OCH_3$ | H | H | $CH_3$ | $CH_3$ | CH | |
| Q-4 | 0 | H | 6-$SCH_3$ | H | H | $CH_3$ | $OCH_3$ | CH | |
| Q-4 | 0 | H | 5-Cl | Cl | H | $OCH_3$ | $OCH_3$ | CH | |
| Q-4 | 0 | H | 3-Cl | F | F | $CH_3$ | $CH_3$ | N | |
| Q-4 | 0 | H | 5-$OCH_2F$ | Cl | H | $OCH_3$ | $OCH_3$ | N | |
| Q-4 | 0 | $CH_3$ | H | H | H | $OCH_3$ | $OCH_3$ | N | |
| Q-4 | 1 | H | H | H | H | $OCH_3$ | $OCH_3$ | CH | |
| Q-4 | 1 | H | H | F | F | $CH_3$ | $OCH_3$ | CH | |

Table I (continued)

| Q | n | R | $R_1$ | $R_7$ | $R_8$ | X | Y | Z | m.p.(°C) |
|---|---|---|---|---|---|---|---|---|---|
| Q-4 | 1 | H | H | F | Cl | $CH_3$ | $CH_3$ | N | |
| Q-4 | 1 | H | H | $CH_3$ | Cl | $OCH_3$ | $CH_3$ | CH | |
| Q-4 | 1 | H | H | F | H | $CH_3$ | $OCH_3$ | CH | |
| Q-4 | 1 | H | H | Cl | H | $OCH_3$ | $OCH_3$ | CH | |
| Q-4 | 1 | H | H | Br | H | $CH_3$ | $OCH_3$ | N | |
| Q-4 | 1 | H | H | Cl | Cl | $CH_3$ | $OCH_3$ | CH | |
| Q-4 | 1 | H | H | $CH_3$ | H | $CH_3$ | $OCH_3$ | CH | |
| Q-4 | 1 | H | H | CN | H | $OCH_3$ | $OCH_3$ | CH | |
| Q-4 | 1 | H | H | CN | CN | $CH_3$ | $OCH_3$ | N | |
| Q-4 | 1 | H | H | $CH_3$ | Br | $CH_3$ | $CH_3$ | CH | |
| Q-4 | 1 | H | H | F | Br | Cl | $OCH_3$ | CH | |
| Q-4 | 1 | H | 5-$CH_3$ | F | H | $OCH_3$ | $OCH_3$ | CH | |
| Q-4 | 1 | H | 5-$OCH_3$ | Cl | H | $CH_3$ | $CH_3$ | N | |
| Q-4 | 1 | H | 5-Cl | Cl | Cl | $CH_3$ | $OCH_3$ | N | |
| Q-5 | 0 | H | H | H | H | $CH_3$ | $CH_3$ | CH | |
| Q-5 | 0 | H | H | H | H | $CH_3$ | $OCH_3$ | CH | |
| Q-5 | 0 | H | H | H | H | $OCH_3$ | $OCH_3$ | CH | |
| Q-5 | 0 | H | H | H | H | $CH_3$ | $CH_3$ | N | |
| Q-5 | 0 | H | H | H | H | $CH_3$ | $OCH_3$ | N | |
| Q-5 | 0 | H | H | H | H | $OCH_3$ | $OCH_3$ | N | |
| Q-5 | 0 | H | H | H | H | Cl | $OCH_3$ | CH | |
| Q-5 | 0 | H | H | H | $CH_3$ | $CH_3$ | $OCH_3$ | CH | |
| Q-5 | 0 | H | H | H | $CH_3$ | $OCH_3$ | $OCH_3$ | CH | |
| Q-5 | 0 | H | H | H | $CH_3$ | $OCH_3$ | $OCH_3$ | N | |
| Q-5 | 0 | H | H | H | $CH_3$ | $CH_3$ | $OCH_3$ | CH | |
| Q-5 | 0 | H | H | H | Cl | $CH_3$ | $OCH_3$ | CH | |
| Q-5 | 0 | H | H | H | Cl | $OCH_3$ | $CH_3$ | N | |
| Q-5 | 0 | H | H | H | Cl | $OCH_3$ | $OCH_3$ | CH | |
| Q-5 | 0 | H | H | H | Cl | Cl | $OCH_3$ | CH | |
| Q-5 | 0 | H | H | H | F | $CH_3$ | $CH_3$ | N | |
| Q-5 | 0 | H | H | H | F | $CH_3$ | $CH_3$ | CH | |
| Q-5 | 0 | H | H | H | F | $OCH_3$ | $CH_3$ | CH | |

Table I (continued)

| Q | n | R | $R_1$ | $R_7$ | $R_8$ | X | Y | Z | m.p.(°C) |
|---|---|---|---|---|---|---|---|---|---|
| Q-5 | 0 | H | H | H | F | $OCH_3$ | $OCH_3$ | CH | |
| Q-5 | 0 | H | H | H | F | $OCH_3$ | $OCH_3$ | CH | |
| Q-5 | 0 | H | H | H | F | $CH_3$ | $OCH_3$ | N | |
| Q-5 | 0 | H | H | H | F | Cl | $OCH_3$ | CH | |
| Q-5 | 0 | H | H | H | CN | $CH_3$ | $OCH_3$ | N | |
| Q-5 | 0 | H | H | H | CN | $OCH_3$ | $OCH_3$ | CH | |
| Q-5 | 0 | H | H | H | $OCH_3$ | $CH_3$ | $OCH_3$ | N | |
| Q-5 | 0 | H | H | H | $OCH_3$ | $OCH_3$ | $OCH_3$ | CH | |
| Q-5 | 0 | H | H | H | $OCH_3$ | $OCH_3$ | $OCH_3$ | N | |
| Q-5 | 0 | H | H | H | $CO_2CH_3$ | $CH_3$ | $CH_3$ | N | |
| Q-5 | 0 | H | H | H | $CO_2CH_3$ | $OCH_3$ | $CH_3$ | CH | |
| Q-5 | 0 | H | H | H | $CO_2CH_3$ | $OCH_3$ | $OCH_3$ | CH | |
| Q-5 | 0 | H | H | H | $CO_2CH_3$ | $CH_3$ | $OCH_3$ | N | |
| Q-5 | 0 | H | H | H | $CO_2CH_3$ | $CH_3$ | $CH_3$ | CH | |
| Q-5 | 0 | H | H | H | $CO_2CH_3$ | $OCH_3$ | $OCH_3$ | N | |
| Q-5 | 0 | H | H | H | $CO_2CH_3$ | Cl | $OCH_3$ | CH | |
| Q-5 | 0 | H | H | H | $SO_2N(CH_3)_2$ | $CH_3$ | $CH_3$ | N | |
| Q-5 | 0 | H | H | H | $SO_2N(CH_3)_2$ | $OCH_3$ | $OCH_3$ | CH | |
| Q-5 | 0 | H | H | H | $SO_2N(CH_3)_2$ | $OCH_3$ | $OCH_3$ | N | |
| Q-5 | 0 | H | H | H | $SO_2N(CH_3)_2$ | $CH_3$ | $OCH_3$ | CH | |
| Q-5 | 0 | H | H | H | $SCH_3$ | $OCH_3$ | $OCH_3$ | CH | |
| Q-5 | 0 | H | H | H | $SCH_3$ | $CH_3$ | $CH_3$ | Cl | |
| Q-5 | 0 | H | H | H | $SCH_3$ | $OCH_3$ | $CH_3$ | CH | |
| Q-5 | 0 | H | H | H | $SCH_3$ | Cl | $OCH_3$ | CH | |
| Q-5 | 0 | H | H | H | $SO_3CH_3$ | $CH_3$ | $CH_3$ | CH | |
| Q-5 | 0 | H | H | H | $SO_3CH_3$ | $CH_3$ | $OCH_3$ | CH | |
| Q-5 | 0 | H | H | H | $SO_3CH_3$ | $CH_3$ | $OCH_3$ | N | |
| Q-5 | 0 | H | H | F | F | $CH_3$ | $CH_3$ | CH | |
| Q-5 | 0 | H | H | F | F | $OCH_3$ | $CH_3$ | N | |
| Q-5 | 0 | H | H | Cl | Cl | $OCH_3$ | $OCH_3$ | CH | |
| Q-5 | 0 | H | H | Cl | Cl | $CH_3$ | $CH_3$ | N | |
| Q-5 | 0 | H | H | F | Cl | $OCH_3$ | $CH_3$ | N | |

Table I (continued)

| Q | n | R | $R_1$ | $R_7$ | $R_8$ | X | Y | Z | m.p.(°C) |
|---|---|---|---|---|---|---|---|---|---|
| Q-5 | 0 | H | H | F | $CH_3$ | $CH_3$ | $CH_3$ | CH | |
| Q-5 | 0 | H | H | $CH_3$ | $CH_3$ | $OCH_3$ | $OCH_3$ | CH | |
| Q-5 | 0 | H | H | $CH_3$ | $CH_2CH_3$ | $OCH_3$ | $OCH_3$ | N | |
| Q-5 | 0 | H | H | F | Br | Cl | $OCH_3$ | CH | |
| Q-5 | 0 | H | H | Br | Br | $OCH_3$ | $OCH_3$ | CH | |
| Q-5 | 0 | H | H | $CO_2CH_3$ | $CO_2CH_3$ | $OCH_3$ | $OCH_3$ | CH | |
| Q-5 | 0 | H | H | CN | CN | $CH_3$ | $OCH_3$ | CH | |
| Q-5 | 0 | H | H | CN | CN | $OCH_3$ | $OCH_3$ | CH | |
| Q-5 | 0 | H | H | CN | CN | $OCH_3$ | $OCH_3$ | N | |
| Q-5 | 0 | H | H | $OCH_3$ | $OCH_3$ | $CH_3$ | $OCH_3$ | CH | |
| Q-5 | 0 | H | 6-$CH_3$ | $CH_3$ | $CH_3$ | $OCH_3$ | $OCH_3$ | CH | |
| Q-5 | 0 | H | 3-$CH_3$ | $CH_3$ | H | $CH_3$ | $CH_3$ | N | |
| Q-5 | 0 | H | 5-$CH_3$ | F | H | $CH_3$ | $OCH_3$ | N | |
| Q-5 | 0 | H | 5-$CH_3$ | Cl | H | $OCH_3$ | $OCH_3$ | N | |
| Q-5 | 0 | H | H | F | F | $CH_3$ | $OCH_3$ | CH | |
| Q-5 | 0 | H | 6-$CH_3$ | Br | H | $OCH_3$ | $OCH_3$ | CH | |
| Q-5 | 0 | H | H | Cl | Cl | $CH_3$ | $OCH_3$ | N | |
| Q-5 | 0 | H | 3-$CH_3$ | H | H | $OCH_3$ | $OCH_3$ | N | |
| Q-5 | 0 | H | 5-$OCH_3$ | H | H | $CH_3$ | $CH_3$ | CH | |
| Q-5 | 0 | H | 5-$SCH_3$ | H | H | $CH_3$ | $OCH_3$ | CH | |
| Q-5 | 0 | H | 5-Cl | Cl | H | $OCH_3$ | $OCH_3$ | CH | |
| Q-5 | 0 | H | 6-Cl | F | F | $CH_3$ | $CH_3$ | N | |
| Q-5 | 0 | H | 5-$OCH_2F$ | Cl | F | $OCH_3$ | $OCH_3$ | N | |
| Q-5 | 0 | $CH_3$ | H | H | H | $OCH_3$ | $OCH_3$ | N | |
| Q-5 | 1 | H | H | H | H | $OCH_3$ | $OCH_3$ | CH | |
| Q-5 | 1 | H | H | F | F | $CH_3$ | $OCH_3$ | CH | |
| Q-5 | 1 | H | H | F | Cl | $CH_3$ | $CH_3$ | N | |
| Q-5 | 1 | H | H | $CH_3$ | Cl | $OCH_3$ | $CH_3$ | CH | |
| Q-5 | 1 | H | H | F | H | $CH_3$ | $OCH_3$ | CH | |
| Q-5 | 1 | H | H | Cl | H | $OCH_3$ | $OCH_3$ | CH | |
| Q-5 | 1 | H | H | Br | H | $CH_3$ | $OCH_3$ | N | |
| Q-5 | 1 | H | H | Cl | Cl | $CH_3$ | $OCH_3$ | CH | |

Table I (continued)

| Q | n | R | $R_1$ | $R_7$ | $R_8$ | X | Y | Z | m.p.(°C) |
|---|---|---|---|---|---|---|---|---|---|
| Q-5 | 1 | H | H | $CH_3$ | H | $CH_3$ | $OCH_3$ | CH | |
| Q-5 | 1 | H | H | CN | H | $OCH_3$ | $OCH_3$ | CH | |
| Q-5 | 1 | H | H | CN | CN | $CH_3$ | $OCH_3$ | N | |
| Q-5 | 1 | H | H | $CH_3$ | Br | $CH_3$ | $CH_3$ | CH | |
| Q-5 | 1 | H | H | F | Br | Cl | $OCH_3$ | CH | |
| Q-5 | 1 | H | 3-$CH_3$ | F | H | $OCH_3$ | $OCH_3$ | CH | |
| Q-5 | 1 | H | 5-$OCH_3$ | Cl | H | $CH_3$ | $CH_3$ | N | |
| Q-5 | 1 | H | 6-Cl | Cl | H | $CH_3$ | $OCH_3$ | N | |
| Q-6 | 0 | H | H | H | H | $CH_3$ | $CH_3$ | CH | |
| Q-6 | 0 | H | H | H | H | $CH_3$ | $OCH_3$ | CH | |
| Q-6 | 0 | H | H | H | H | $OCH_3$ | $OCH_3$ | CH | |
| Q-6 | 0 | H | H | H | H | $CH_3$ | $CH_3$ | N | |
| Q-6 | 0 | H | H | H | H | $CH_3$ | $OCH_3$ | N | |
| Q-6 | 0 | H | H | H | H | $OCH_3$ | $OCH_3$ | N | |
| Q-6 | 0 | H | H | H | H | Cl | $OCH_3$ | CH | |
| Q-6 | 0 | H | H | H | $CH_3$ | $CH_3$ | $OCH_3$ | CH | |
| Q-6 | 0 | H | H | H | $CH_3$ | $OCH_3$ | $OCH_3$ | CH | |
| Q-6 | 0 | H | H | H | $CH_3$ | $OCH_3$ | $OCH_3$ | N | |
| Q-6 | 0 | H | H | H | $CH_3$ | $CH_3$ | $OCH_3$ | CH | |
| Q-6 | 0 | H | H | H | Cl | $CH_3$ | $OCH_3$ | CH | |
| Q-6 | 0 | H | H | H | Cl | $OCH_3$ | $CH_3$ | N | |
| Q-6 | 0 | H | H | H | Cl | $OCH_3$ | $OCH_3$ | CH | |
| Q-6 | 0 | H | H | H | Cl | Cl | $OCH_3$ | CH | |
| Q-6 | 0 | H | H | H | F | $CH_3$ | $CH_3$ | N | |
| Q-6 | 0 | H | H | H | F | $CH_3$ | $CH_3$ | CH | |
| Q-6 | 0 | H | H | H | F | $OCH_3$ | $CH_3$ | CH | |
| Q-6 | 0 | H | H | H | F | $OCH_3$ | $OCH_3$ | CH | |
| Q-6 | 0 | H | H | H | F | $CH_3$ | $OCH_3$ | N | |
| Q-6 | 0 | H | H | H | F | Cl | $OCH_3$ | CH | |
| Q-6 | 0 | H | H | H | CN | $CH_3$ | $OCH_3$ | N | |
| Q-6 | 0 | H | H | H | CN | $OCH_3$ | $OCH_3$ | CH | |
| Q-6 | 0 | H | H | H | $OCH_3$ | $CH_3$ | $OCH_3$ | N | |

Table I (continued)

| Q | n | R | $R_1$ | $R_7$ | $R_8$ | X | Y | Z | m.p.(°C) |
|---|---|---|---|---|---|---|---|---|---|
| Q-6 | 0 | H | H | H | $OCH_3$ | $OCH_3$ | $OCH_3$ | CH | |
| Q-6 | 0 | H | H | H | $OCH_3$ | $OCH_3$ | $OCH_3$ | N | |
| Q-6 | 0 | H | H | H | $CO_2CH_3$ | $CH_3$ | $CH_3$ | N | |
| Q-6 | 0 | H | H | H | $CO_2CH_3$ | $OCH_3$ | $CH_3$ | CH | |
| Q-6 | 0 | H | H | H | $CO_2CH_3$ | $OCH_3$ | $OCH_3$ | CH | |
| Q-6 | 0 | H | H | H | $CO_2CH_3$ | $CH_3$ | $OCH_3$ | N | |
| Q-6 | 0 | H | H | H | $CO_2CH_3$ | $CH_3$ | $CH_3$ | CH | |
| Q-6 | 0 | H | H | H | $CO_2CH_3$ | $OCH_3$ | $OCH_3$ | N | |
| Q-6 | 0 | H | H | H | $CO_2CH_3$ | Cl | $OCH_3$ | CH | |
| Q-6 | 0 | H | H | H | $SO_2N(CH_3)_2$ | $CH_3$ | $CH_3$ | N | |
| Q-6 | 0 | H | H | H | $SO_2N(CH_3)_2$ | $OCH_3$ | $OCH_3$ | CH | |
| Q-6 | 0 | H | H | H | $SO_2N(CH_3)_2$ | $OCH_3$ | $OCH_3$ | N | |
| Q-6 | 0 | H | H | H | $SO_2N(CH_3)_2$ | $CH_3$ | $OCH_3$ | CH | |
| Q-6 | 0 | H | H | H | $SCH_3$ | $OCH_3$ | $OCH_3$ | CH | |
| Q-6 | 0 | H | H | H | $SCH_3$ | $CH_3$ | $CH_3$ | CH | |
| Q-6 | 0 | H | H | H | $SCH_3$ | $OCH_3$ | $CH_3$ | CH | |
| Q-6 | 0 | H | H | H | $SCH_3$ | Cl | $OCH_3$ | CH | |
| Q-6 | 0 | H | H | H | $SO_2CH_3$ | $CH_3$ | $CH_3$ | CH | |
| Q-6 | 0 | H | H | H | $SO_2CH_3$ | $CH_3$ | $OCH_3$ | CH | |
| Q-6 | 0 | H | H | H | $SO_2CH_3$ | $CH_3$ | $OCH_3$ | N | |
| Q-6 | 0 | H | H | F | F | $CH_3$ | $CH_3$ | CH | |
| Q-6 | 0 | H | H | F | F | $OCH_3$ | $CH_3$ | Cl | |
| Q-6 | 0 | H | H | Cl | Cl | $OCH_3$ | $OCH_3$ | CH | |
| Q-6 | 0 | H | H | Cl | Cl | $CH_3$ | $CH_3$ | N | |
| Q-6 | 0 | H | H | F | Cl | $OCH_3$ | $CH_3$ | N | |
| Q-6 | 0 | H | H | F | $CH_3$ | $CH_3$ | $CH_3$ | CH | |
| Q-6 | 0 | H | H | $CH_3$ | $CH_3$ | $OCH_3$ | $OCH_3$ | CH | |
| Q-6 | 0 | H | H | $CH_3$ | $CH_2CH_3$ | $OCH_3$ | $OCH_3$ | N | |
| Q-6 | 0 | H | H | F | Br | Cl | $OCH_3$ | CH | |
| Q-6 | 0 | H | H | Br | Br | $OCH_3$ | $OCH_3$ | CH | |
| Q-6 | 0 | H | H | CN | CN | $CH_3$ | $OCH_3$ | CH | |
| Q-6 | 0 | H | H | CN | CN | $OCH_3$ | $OCH_3$ | CH | |

Table I (continued)

| Q | n | R | $R_1$ | $R_7$ | $R_8$ | X | Y | Z | m.p.(°C) |
|---|---|---|---|---|---|---|---|---|---|
| Q-6 | 0 | H | H | CN | CN | $OCH_3$ | $OCH_3$ | N | |
| Q-6 | 0 | H | H | $OCH_3$ | $OCH_3$ | $CH_3$ | $OCH_3$ | CH | |
| Q-6 | 0 | H | 5-$CH_3$ | $CH_3$ | H | $CH_3$ | $CH_3$ | N | |
| Q-6 | 0 | H | 6-$CH_3$ | F | H | $CH_3$ | $OCH_3$ | N | |
| Q-6 | 0 | H | 3-$CH_3$ | Cl | H | $OCH_3$ | $OCH_3$ | N | |
| Q-6 | 0 | H | H | F | F | $CH_3$ | $OCH_3$ | CH | |
| Q-6 | 0 | H | 5-$CH_3$ | Br | H | $OCH_3$ | $OCH_3$ | CH | |
| Q-6 | 0 | H | H | Cl | Cl | $CH_3$ | $OCH_3$ | N | |
| Q-6 | 0 | H | 6-$CH_3$ | H | H | $OCH_3$ | $OCH_3$ | N | |
| Q-6 | 0 | H | 5-$OCH_3$ | H | H | $CH_3$ | $CH_3$ | CH | |
| Q-6 | 0 | H | 5-$SCH_3$ | H | H | $CH_3$ | $OCH_3$ | CH | |
| Q-6 | 0 | H | 5-Cl | Cl | H | $OCH_3$ | $OCH_3$ | CH | |
| Q-6 | 0 | H | 3-Cl | F | F | $CH_3$ | $CH_3$ | N | |
| Q-6 | 0 | H | 5-$OCH_2F$ | Cl | H | $OCH_3$ | $OCH_3$ | N | |
| Q-6 | 0 | $CH_3$ | H | H | H | $OCH_3$ | $OCH_3$ | N | |
| Q-6 | 1 | H | H | H | H | $OCH_3$ | $OCH_3$ | CH | |
| Q-6 | 1 | H | H | F | F | $CH_3$ | $OCH_3$ | CH | |
| Q-6 | 1 | H | H | F | Cl | $CH_3$ | $CH_3$ | N | |
| Q-6 | 1 | H | H | $CH_3$ | Cl | $OCH_3$ | $CH_3$ | CH | |
| Q-6 | 1 | H | H | F | H | $CH_3$ | $OCH_3$ | CH | |
| Q-6 | 1 | H | H | Cl | H | $OCH_3$ | $OCH_3$ | CH | |
| Q-6 | 1 | H | H | Br | H | $CH_3$ | $OCH_3$ | N | |
| Q-6 | 1 | H | H | Cl | Cl | $CH_3$ | $OCH_3$ | CH | |
| Q-6 | 1 | H | H | $CH_3$ | H | $CH_3$ | $OCH_3$ | CH | |
| Q-6 | 1 | H | H | CN | H | $OCH_3$ | $OCH_3$ | CH | |
| Q-6 | 1 | H | H | CN | CN | $CH_3$ | $OCH_3$ | N | |
| Q-6 | 1 | H | H | $CH_3$ | Br | $CH_3$ | $CH_3$ | CH | |
| Q-6 | 1 | H | H | F | Br | Cl | $OCH_3$ | CH | |
| Q-6 | 1 | H | 3-$CH_3$ | F | H | $OCH_3$ | $OCH_3$ | CH | |
| Q-6 | 1 | H | 3-$OCH_3$ | Cl | H | $CH_3$ | $CH_3$ | N | |
| Q-6 | 1 | H | 5-Cl | Cl | Cl | $CH_3$ | $OCH_3$ | N | |
| Q-7 | 0 | H | H | H | H | $CH_3$ | $CH_3$ | CH | |
| Q-7 | 0 | H | H | H | H | $CH_3$ | $OCH_3$ | CH | |

Table I (continued)

| Q | n | R | $R_1$ | $R_7$ | $R_8$ | X | Y | Z | m.p.(°C) |
|---|---|---|---|---|---|---|---|---|---|
| Q-7 | 0 | H | H | H | H | $OCH_3$ | $OCH_3$ | CH | |
| Q-7 | 0 | H | H | H | H | $CH_3$ | $CH_3$ | N | |
| Q-7 | 0 | H | H | H | H | $CH_3$ | $OCH_3$ | N | |
| Q-7 | 0 | H | H | H | H | $OCH_3$ | $OCH_3$ | N | |
| Q-7 | 0 | H | H | H | H | Cl | $OCH_3$ | CH | |
| Q-7 | 0 | H | H | H | $CH_3$ | $CH_3$ | $CH_3$ | CH | |
| Q-7 | 0 | H | H | H | $CH_3$ | $OCH_3$ | $OCH_3$ | CH | |
| Q-7 | 0 | H | H | H | $CH_3$ | $OCH_3$ | $OCH_3$ | N | |
| Q-7 | 0 | H | H | H | $CH_3$ | $CH_3$ | $OCH_3$ | CH | |
| Q-7 | 0 | H | H | H | Cl | $CH_3$ | $OCH_3$ | CH | |
| Q-7 | 0 | H | H | H | Cl | $OCH_3$ | $CH_3$ | N | |
| Q-7 | 0 | H | H | H | Cl | $OCH_3$ | $OCH_3$ | CH | |
| Q-7 | 0 | H | H | H | Cl | Cl | $OCH_3$ | CH | |
| Q-7 | 0 | H | H | H | F | $CH_3$ | $CH_3$ | N | |
| Q-7 | 0 | H | H | H | F | $CH_3$ | $CH_3$ | CH | |
| Q-7 | 0 | H | H | H | F | $OCH_3$ | $CH_3$ | CH | |
| Q-7 | 0 | H | H | H | F | $OCH_3$ | $OCH_3$ | CH | |
| Q-7 | 0 | H | H | H | F | $CH_3$ | $OCH_3$ | N | |
| Q-7 | 0 | H | H | H | F | Cl | $OCH_3$ | CH | |
| Q-7 | 0 | H | H | H | CN | $CH_3$ | $OCH_3$ | N | |
| Q-7 | 0 | H | H | H | CN | $OCH_3$ | $OCH_3$ | CH | |
| Q-7 | 0 | H | H | H | $OCH_3$ | $CH_3$ | $OCH_3$ | N | |
| Q-7 | 0 | H | H | H | $OCH_3$ | $OCH_3$ | $OCH_3$ | CH | |
| Q-7 | 0 | H | H | H | $OCH_3$ | $OCH_3$ | $OCH_3$ | N | |
| Q-7 | 0 | H | H | H | $CO_2CH_3$ | $CH_3$ | $CH_3$ | N | |
| Q-7 | 0 | H | H | H | $CO_2CH_3$ | $OCH_3$ | $CH_3$ | CH | |
| Q-7 | 0 | H | H | H | $CO_2CH_3$ | $OCH_3$ | $OCH_3$ | CH | |
| Q-7 | 0 | H | H | H | $CO_2CH_3$ | $CH_3$ | $OCH_3$ | N | |
| Q-7 | 0 | H | H | H | $CO_2CH_3$ | $CH_3$ | $CH_3$ | CH | |
| Q-7 | 0 | H | H | H | $CO_2CH_3$ | $OCH_3$ | $OCH_3$ | N | |
| Q-7 | 0 | H | H | H | $CO_2CH_3$ | Cl | $OCH_3$ | CH | |
| Q-7 | 0 | H | H | H | $SO_2N(CH_3)_2$ | $CH_3$ | $CH_3$ | N | |

Table I (continued)

| Q | n | R | $R_1$ | $R_7$ | $R_8$ | X | Y | Z | m.p.(°C) |
|---|---|---|---|---|---|---|---|---|---|
| Q-7 | 0 | H | H | H | $SO_2N(CH_3)_2$ | $OCH_3$ | $OCH_3$ | CH | |
| Q-7 | 0 | H | H | H | $SO_2N(CH_3)_2$ | $OCH_3$ | $OCH_3$ | N | |
| Q-7 | 0 | H | H | H | $SO_2N(CH_3)_2$ | $CH_3$ | $OCH_3$ | CH | |
| Q-7 | 0 | H | H | H | $SCH_3$ | $OCH_3$ | $OCH_3$ | CH | |
| Q-7 | 0 | H | H | H | $SCH_3$ | $CH_3$ | $CH_3$ | Cl | |
| Q-7 | 0 | H | H | H | $SCH_3$ | $OCH_3$ | $CH_3$ | CH | |
| Q-7 | 0 | H | H | H | $SCH_3$ | Cl | $OCH_3$ | CH | |
| Q-7 | 0 | H | H | H | $SO_2CH_3$ | $CH_3$ | $CH_3$ | CH | |
| Q-7 | 0 | H | H | H | $SO_2CH_3$ | $CH_3$ | $OCH_3$ | CH | |
| Q-7 | 0 | H | H | H | $SO_2CH_3$ | $CH_3$ | $OCH_3$ | N | |
| Q-7 | 0 | H | H | F | F | $CH_3$ | $CH_3$ | CH | |
| Q-7 | 0 | H | H | F | F | $OCH_3$ | $CH_3$ | Cl | |
| Q-7 | 0 | H | H | Cl | Cl | $OCH_3$ | $OCH_3$ | CH | |
| Q-7 | 0 | H | H | Cl | Cl | $CH_3$ | $CH_3$ | N | |
| Q-7 | 0 | H | H | F | Cl | $OCH_3$ | $CH_3$ | N | |
| Q-7 | 0 | H | H | F | $CH_3$ | $CH_3$ | $CH_3$ | CH | |
| Q-7 | 0 | H | H | $CH_3$ | $CH_3$ | $OCH_3$ | $OCH_3$ | CH | |
| Q-7 | 0 | H | H | $CH_3$ | $CH_2CH_3$ | $OCH_3$ | $OCH_3$ | N | |
| Q-7 | 0 | H | H | F | Br | Cl | $OCH_3$ | CH | |
| Q-7 | 0 | H | H | Br | Br | $OCH_3$ | $OCH_3$ | CH | |
| Q-7 | 0 | H | H | CN | CN | $CH_3$ | $OCH_3$ | CH | |
| Q-7 | 0 | H | H | CN | CN | $OCH_3$ | $OCH_3$ | CH | |
| Q-7 | 0 | H | H | CN | CN | $OCH_3$ | $OCH_3$ | N | |
| Q-7 | 0 | H | H | $OCH_3$ | $OCH_3$ | $CH_3$ | $OCH_3$ | CH | |
| Q-7 | 0 | H | 3-$CH_3$ | $CH_3$ | H | $CH_3$ | $OCH_3$ | CH | |
| Q-7 | 0 | H | 5-$CH_3$ | $CH_3$ | H | $OCH_3$ | $OCH_3$ | CH | |
| Q-7 | 0 | $CH_3$ | H | $CH_3$ | H | $CH_3$ | $CH_3$ | N | |
| Q-7 | 0 | $CH_3$ | H | F | H | $CH_3$ | $OCH_3$ | N | |
| Q-7 | 0 | H | 5-$CH_3$ | Cl | H | $OCH_3$ | $OCH_3$ | N | |
| Q-7 | 0 | H | H | F | F | $CH_3$ | $OCH_3$ | CH | |
| Q-7 | 0 | H | H | Br | H | $OCH_3$ | $OCH_3$ | CH | |
| Q-7 | 0 | H | H | Cl | Cl | $CH_3$ | $OCH_3$ | N | |

Table I (continued)

| Q | n | R | $R_1$ | $R_7$ | $R_8$ | X | Y | Z | m.p.(°C) |
|---|---|---|---|---|---|---|---|---|---|
| Q-7 | 0 | H | 5-$CH_3$ | H | H | $OCH_3$ | $OCH_3$ | N | |
| Q-7 | 0 | H | 6-$OCH_3$ | H | H | $CH_3$ | $CH_3$ | CH | |
| Q-7 | 0 | H | 5-$SCH_3$ | H | H | $CH_3$ | $OCH_3$ | CH | |
| Q-7 | 0 | H | 3-Cl | Cl | H | $OCH_3$ | $OCH_3$ | CH | |
| Q-7 | 0 | H | 5-Cl | F | F | $CH_3$ | $CH_3$ | N | |
| Q-7 | 0 | H | 3-$OCH_2F$ | Cl | H | $OCH_3$ | $OCH_3$ | N | |
| Q-7 | 0 | $CH_3$ | H | H | H | $OCH_3$ | $OCH_3$ | N | |
| Q-7 | 1 | H | H | H | H | $OCH_3$ | $OCH_3$ | CH | |
| Q-7 | 1 | H | H | F | F | $CH_3$ | $OCH_3$ | CH | |
| Q-7 | 1 | H | H | F | Cl | $CH_3$ | $CH_3$ | N | |
| Q-7 | 1 | H | H | $CH_3$ | Cl | $OCH_3$ | $CH_3$ | CH | |
| Q-7 | 1 | H | H | F | H | $CH_3$ | $OCH_3$ | CH | |
| Q-7 | 1 | H | H | Cl | H | $OCH_3$ | $OCH_3$ | CH | |
| Q-7 | 1 | H | H | Br | H | $CH_3$ | $OCH_3$ | N | |
| Q-7 | 1 | H | H | Cl | Cl | $CH_3$ | $OCH_3$ | CH | |
| Q-7 | 1 | H | H | $CH_3$ | H | $CH_3$ | $OCH_3$ | CH | |
| Q-7 | 1 | H | H | CN | H | $OCH_3$ | $OCH_3$ | CH | |
| Q-7 | 1 | H | H | CN | CN | $CH_3$ | $OCH_3$ | N | |
| Q-7 | 1 | H | H | $CH_3$ | Br | $CH_3$ | $CH_3$ | CH | |
| Q-7 | 1 | H | H | F | Br | Cl | $OCH_3$ | CH | |
| Q-7 | 1 | H | 5-$CH_3$ | F | H | $OCH_3$ | $OCH_3$ | CH | |
| Q-7 | 1 | H | 5-$OCH_3$ | Cl | H | $CH_3$ | $CH_3$ | N | |
| Q-7 | 1 | H | 5-Cl | Cl | H | $CH_3$ | $OCH_3$ | N | |
| Q-8 | 0 | H | H | H | H | $CH_3$ | $CH_3$ | CH | |
| Q-8 | 0 | H | H | H | H | $CH_3$ | $OCH_3$ | CH | |
| Q-8 | 0 | H | H | H | H | $OCH_3$ | $OCH_3$ | CH | |
| Q-8 | 0 | H | H | H | H | $CH_3$ | $CH_3$ | N | |
| Q-8 | 0 | H | H | H | H | $CH_3$ | $OCH_3$ | N | |
| Q-8 | 0 | H | H | H | H | $OCH_3$ | $OCH_3$ | N | |
| Q-8 | 0 | H | H | H | H | Cl | $OCH_3$ | CH | |
| Q-8 | 0 | H | H | H | $CH_3$ | $CH_3$ | $OCH_3$ | CH | |
| Q-8 | 0 | H | H | H | $CH_3$ | $OCH_3$ | $OCH_3$ | CH | |

Table I (continued)

| Q | n | R | $R_1$ | $R_7$ | $R_8$ | X | Y | Z | m.p.(°C) |
|---|---|---|---|---|---|---|---|---|---|
| Q-8 | 0 | H | H | H | $CH_3$ | $OCH_3$ | $OCH_3$ | N | |
| Q-8 | 0 | H | H | H | $CH_3$ | $CH_3$ | $CH_3$ | CH | |
| Q-8 | 0 | H | H | H | Cl | $CH_3$ | $OCH_3$ | CH | |
| Q-8 | 0 | H | H | H | Cl | $OCH_3$ | $CH_3$ | N | |
| Q-8 | 0 | H | H | H | Cl | $OCH_3$ | $OCH_3$ | CH | |
| Q-8 | 0 | H | H | H | Cl | Cl | $OCH_3$ | CH | |
| Q-8 | 0 | H | H | H | F | $CH_3$ | $CH_3$ | N | |
| Q-8 | 0 | H | H | H | F | $CH_3$ | $CH_3$ | CH | |
| Q-8 | 0 | H | H | H | F | $OCH_3$ | $CH_3$ | CH | |
| Q-8 | 0 | H | H | H | F | $OCH_3$ | $OCH_3$ | CH | |
| Q-8 | 0 | H | H | H | F | $CH_3$ | $OCH_3$ | N | |
| Q-8 | 0 | H | H | H | F | Cl | $OCH_3$ | CH | |
| Q-8 | 0 | H | H | H | CN | $CH_3$ | $OCH_3$ | N | |
| Q-8 | 0 | H | H | H | CN | $OCH_3$ | $OCH_3$ | CH | |
| Q-8 | 0 | H | H | H | $OCH_3$ | $CH_3$ | $OCH_3$ | N | |
| Q-8 | 0 | H | H | H | $OCH_3$ | $OCH_3$ | $OCH_3$ | CH | |
| Q-8 | 0 | H | H | H | $OCH_3$ | $OCH_3$ | $OCH_3$ | N | |
| Q-8 | 0 | H | H | H | $CO_2CH_3$ | $CH_3$ | $CH_3$ | N | |
| Q-8 | 0 | H | H | H | $CO_2CH_3$ | $OCH_3$ | $CH_3$ | CH | |
| Q-8 | 0 | H | H | H | $CO_2CH_3$ | $OCH_3$ | $OCH_3$ | CH | |
| Q-8 | 0 | H | H | H | $CO_2CH_3$ | $CH_3$ | $OCH_3$ | N | |
| Q-8 | 0 | H | H | H | $CO_2CH_3$ | $CH_3$ | $CH_3$ | CH | |
| Q-8 | 0 | H | H | H | $CO_2CH_3$ | $OCH_3$ | $OCH_3$ | N | |
| Q-8 | 0 | H | H | H | $CO_2CH_3$ | Cl | $OCH_3$ | CH | |
| Q-8 | 0 | H | H | H | $SO_2N(CH_3)_2$ | $CH_3$ | $CH_3$ | N | |
| Q-8 | 0 | H | H | H | $SO_2N(CH_3)_2$ | $OCH_3$ | $OCH_3$ | CH | |
| Q-8 | 0 | H | H | H | $SO_2N(CH_3)_2$ | $OCH_3$ | $OCH_3$ | N | |
| Q-8 | 0 | H | H | H | $SO_2N(CH_3)_2$ | $CH_3$ | $OCH_3$ | CH | |
| Q-8 | 0 | H | H | H | $SCH_3$ | $OCH_3$ | $OCH_3$ | CH | |
| Q-8 | 0 | H | H | H | $SCH_3$ | $CH_3$ | $CH_3$ | CH | |
| Q-8 | 0 | H | H | H | $SCH_3$ | $OCH_3$ | $CH_3$ | CH | |
| Q-8 | 0 | H | H | H | $SCH_3$ | Cl | $OCH_3$ | CH | |

Table I (continued)

| Q | n | R | $R_1$ | $R_7$ | $R_8$ | X | Y | Z | m.p.(°C) |
|---|---|---|---|---|---|---|---|---|---|
| Q-8 | 0 | H | H | H | $SO_2CH_3$ | $CH_3$ | $CH_3$ | CH | |
| Q-8 | 0 | H | H | H | $SO_2CH_3$ | $CH_3$ | $OCH_3$ | CH | |
| Q-8 | 0 | H | H | H | $SO_2CH_3$ | $CH_3$ | $OCH_3$ | N | |
| Q-8 | 0 | H | H | F | F | $CH_3$ | $CH_3$ | CH | |
| Q-8 | 0 | H | H | F | F | $OCH_3$ | $CH_3$ | Cl | |
| Q-8 | 0 | H | H | Cl | Cl | $OCH_3$ | $OCH_3$ | CH | |
| Q-8 | 0 | H | H | Cl | Cl | $CH_3$ | $CH_3$ | N | |
| Q-8 | 0 | H | H | F | Cl | $OCH_3$ | $CH_3$ | N | |
| Q-8 | 0 | H | H | F | $CH_3$ | $CH_3$ | $CH_3$ | CH | |
| Q-8 | 0 | H | H | $CH_3$ | $CH_3$ | $OCH_3$ | $OCH_3$ | CH | |
| Q-8 | 0 | H | H | $CH_3$ | $CH_2CH_3$ | $OCH_3$ | $OCH_3$ | N | |
| Q-8 | 0 | H | H | F | Br | Cl | $OCH_3$ | CH | |
| Q-8 | 0 | H | H | Br | Br | $OCH_3$ | $OCH_3$ | CH | |
| Q-8 | 0 | H | H | CN | CN | $CH_3$ | $OCH_3$ | CH | |
| Q-8 | 0 | H | H | CN | CN | $OCH_3$ | $OCH_3$ | CH | |
| Q-8 | 0 | H | H | CN | CN | $OCH_3$ | $OCH_3$ | N | |
| Q-8 | 0 | H | H | $OCH_3$ | $OCH_3$ | $CH_3$ | $OCH_3$ | CH | |
| Q-8 | 0 | H | H | H | Br | $CH_3$ | $CH_3$ | CH | |
| Q-8 | 0 | H | H | H | Br | $CH_3$ | $OCH_3$ | CH | |
| Q-8 | 0 | H | H | H | Br | $OCH_3$ | $OCH_3$ | CH | |
| Q-8 | 0 | H | 3-$CH_3$ | $CH_3$ | H | $CH_3$ | $CH_3$ | N | |
| Q-8 | 0 | H | 5-$CH_3$ | F | H | $CH_3$ | $OCH_3$ | N | |
| Q-8 | 0 | H | H | Cl | H | $OCH_3$ | $OCH_3$ | N | |
| Q-8 | 0 | H | H | F | F | $CH_3$ | $OCH_3$ | CH | |
| Q-8 | 0 | H | H | Br | H | $OCH_3$ | $OCH_3$ | N | |
| Q-8 | 0 | H | H | Cl | Cl | $CH_3$ | $OCH_3$ | N | |
| Q-8 | 0 | H | 5-$CH_3$ | H | H | $OCH_3$ | $OCH_3$ | N | |
| Q-8 | 0 | H | 5-$OCH_3$ | H | H | $CH_3$ | $CH_3$ | CH | |
| Q-8 | 0 | H | 5-$SCH_3$ | H | H | $CH_3$ | $OCH_3$ | CH | |
| Q-8 | 0 | H | 5-Cl | Cl | H | $OCH_3$ | $OCH_3$ | CH | |
| Q-8 | 0 | H | 6-Cl | F | F | $CH_3$ | $CH_3$ | N | |
| Q-8 | 0 | H | 5-$OCH_2F$ | Cl | H | $OCH_3$ | $OCH_3$ | N | |

Table I (continued)

| Q | n | R | $R_1$ | $R_7$ | $R_8$ | X | Y | Z | m.p.(°C) |
|---|---|---|---|---|---|---|---|---|---|
| Q-8 | 0 | $CH_3$ | H | H | H | $OCH_3$ | $OCH_3$ | N | |
| Q-8 | 1 | H | H | H | H | $OCH_3$ | $OCH_3$ | CH | |
| Q-8 | 1 | H | H | F | F | $CH_3$ | $OCH_3$ | CH | |
| Q-8 | 1 | H | H | F | Cl | $CH_3$ | $CH_3$ | N | |
| Q-8 | 1 | H | H | $CH_3$ | Cl | $OCH_3$ | $CH_3$ | CH | |
| Q-8 | 1 | H | H | F | H | $CH_3$ | $OCH_3$ | CH | |
| Q-8 | 1 | H | H | Cl | H | $OCH_3$ | $OCH_3$ | CH | |
| Q-8 | 1 | H | H | Br | H | $CH_3$ | $OCH_3$ | N | |
| Q-8 | 1 | H | H | Cl | Cl | $CH_3$ | $OCH_3$ | CH | |
| Q-8 | 1 | H | H | $CH_3$ | H | $CH_3$ | $OCH_3$ | CH | |
| Q-8 | 1 | H | H | CN | H | $OCH_3$ | $OCH_3$ | CH | |
| Q-8 | 1 | H | H | CN | CN | $CH_3$ | $OCH_3$ | N | |
| Q-8 | 1 | H | H | $CH_3$ | Br | $CH_3$ | $CH_3$ | CH | |
| Q-8 | 1 | H | H | F | Br | Cl | $OCH_3$ | CH | |
| Q-8 | 1 | H | 5-$CH_3$ | F | H | $OCH_3$ | $OCH_3$ | CH | |
| Q-8 | 1 | H | 3-$OCH_3$ | Cl | H | $CH_3$ | $CH_3$ | N | |
| Q-8 | 1 | H | 5-Cl | Cl | Cl | $CH_3$ | $OCH_3$ | N | |

| Q | n | R | $R_1$ | $R_3$ | $R_7$ | $R_8$ | X | Y | Z | m.p.(°C) |
|---|---|---|---|---|---|---|---|---|---|---|
| Q-9 | 0 | H | H | $CH_3$ | H | H | $CH_3$ | $CH_3$ | CH | |
| Q-9 | 0 | H | H | $CH_3$ | H | H | $CH_3$ | $OCH_3$ | CH | |
| Q-9 | 0 | H | H | $CH_3$ | H | H | $OCH_3$ | $OCH_3$ | CH | |
| Q-9 | 0 | H | H | $CH_3$ | H | H | $CH_3$ | $CH_3$ | N | |
| Q-9 | 0 | H | H | $CH_3$ | H | H | $CH_3$ | $OCH_3$ | N | |
| Q-9 | 0 | H | H | $CH_3$ | H | H | $OCH_3$ | $OCH_3$ | N | |
| Q-9 | 0 | H | H | $CH_3$ | H | H | Cl | $OCH_3$ | CH | |
| Q-9 | 0 | H | H | $CH_3$ | H | $CH_3$ | $CH_3$ | $OCH_3$ | CH | |
| Q-9 | 0 | H | H | $CH_3$ | H | $CH_3$ | $CH_3$ | $OCH_3$ | CH | |
| Q-9 | 0 | H | H | $CH_3$ | H | $CH_3$ | $OCH_3$ | $OCH_3$ | CH | |
| Q-9 | 0 | H | H | $CH_3$ | H | $CH_3$ | $OCH_3$ | $OCH_3$ | N | |

Table I (continued)

| Q | n | R | $R_1$ | $R_3$ | $R_7$ | $R_8$ | X | Y | Z | m.p.(°C) |
|---|---|---|---|---|---|---|---|---|---|---|
| Q-9 | 0 | H | H | $CH_3$ | H | $CH_3$ | $CH_3$ | $CH_3$ | CH | |
| Q-9 | 0 | H | H | $CH_3$ | H | Cl | $CH_3$ | $OCH_3$ | CH | |
| Q-9 | 0 | H | H | $CH_3$ | H | Cl | $OCH_3$ | $CH_3$ | N | |
| Q-9 | 0 | H | H | $CH_3$ | H | Cl | $OCH_3$ | $OCH_3$ | CH | |
| Q-9 | 0 | H | H | $CH_3$ | H | Cl | Cl | $OCH_3$ | CH | |
| Q-9 | 0 | H | H | $CH_3$ | H | F | $CH_3$ | $CH_3$ | N | |
| Q-9 | 0 | H | H | $CH_3$ | H | F | $CH_3$ | $CH_3$ | CH | |
| Q-9 | 0 | H | H | $CH_3$ | H | F | $OCH_3$ | $CH_3$ | CH | |
| Q-9 | 0 | H | H | $CH_3$ | H | F | $OCH_3$ | $OCH_3$ | CH | |
| Q-9 | 0 | H | H | $CH_3$ | H | F | $CH_3$ | $OCH_3$ | N | |
| Q-9 | 0 | H | H | $CH_3$ | H | F | Cl | $OCH_3$ | CH | |
| Q-9 | 0 | H | H | $CH_3$ | H | CN | $CH_3$ | $OCH_3$ | N | |
| Q-9 | 0 | H | H | $CH_3$ | H | CN | $OCH_3$ | $OCH_3$ | CH | |
| Q-9 | 0 | H | H | $CH_3$ | H | $OCH_3$ | $CH_3$ | $OCH_3$ | N | |
| Q-9 | 0 | H | H | $CH_3$ | H | $OCH_3$ | $OCH_3$ | $OCH_3$ | CH | |
| Q-9 | 0 | H | H | $CH_3$ | H | $OCH_3$ | $OCH_3$ | $OCH_3$ | N | |
| Q-9 | 0 | H | H | $CH_3$ | H | $CO_2CH_3$ | $CH_3$ | $CH_3$ | N | |
| Q-9 | 0 | H | H | $CH_3$ | H | $CO_2CH_3$ | $OCH_3$ | $CH_3$ | CH | |
| Q-9 | 0 | H | H | $CH_3$ | H | $CO_2CH_3$ | $OCH_3$ | $OCH_3$ | CH | |
| Q-9 | 0 | H | H | $CH_3$ | H | $CO_2CH_3$ | $CH_3$ | $OCH_3$ | N | |
| Q-9 | 0 | H | H | $CH_3$ | H | $CO_2CH_3$ | $CH_3$ | $CH_3$ | CH | |
| Q-9 | 0 | H | H | $CH_3$ | H | $CO_2CH_3$ | $OCH_3$ | $OCH_3$ | N | |
| Q-9 | 0 | H | H | $CH_3$ | H | $CO_2CH_3$ | Cl | $OCH_3$ | CH | |
| Q-9 | 0 | H | H | $CH_3$ | H | $SO_2N(CH_3)_2$ | $CH_3$ | $CH_3$ | N | |
| Q-9 | 0 | H | H | $CH_3$ | H | $SO_2N(CH_3)_2$ | $OCH_3$ | $OCH_3$ | CH | |
| Q-9 | 0 | H | H | $CH_3$ | H | $SO_2N(CH_3)_2$ | $OCH_3$ | $OCH_3$ | N | |
| Q-9 | 0 | H | H | $CH_3$ | H | $SO_2N(CH_3)_2$ | $CH_3$ | $OCH_3$ | CH | |
| Q-9 | 0 | H | H | $CH_3$ | H | $SCH_3$ | $OCH_3$ | $OCH_3$ | CH | |
| Q-9 | 0 | H | H | $CH_3$ | H | $SCH_3$ | $CH_3$ | $CH_3$ | CH | |
| Q-9 | 0 | H | H | $CH_3$ | H | $SCH_3$ | $OCH_3$ | $CH_3$ | CH | |
| Q-9 | 0 | H | H | $CH_3$ | H | $SCH_3$ | Cl | $OCH_3$ | CH | |
| Q-9 | 0 | H | H | $CH_3$ | H | $SO_2CH_3$ | $CH_3$ | $CH_3$ | CH | |

Table I (continued)

| Q | n | R | $R_1$ | $R_3$ | $R_7$ | $R_8$ | X | Y | Z | m.p.(°C) |
|---|---|---|---|---|---|---|---|---|---|---|
| Q-9 | 0 | H | H | $CH_3$ | H | $SO_2CH_3$ | $CH_3$ | $OCH_3$ | CH | |
| Q-9 | 0 | H | H | $CH_3$ | H | $SO_2CH_3$ | $CH_3$ | $OCH_3$ | N | |
| Q-9 | 0 | H | H | $CH_3$ | F | F | $CH_3$ | $CH_3$ | CH | |
| Q-9 | 0 | H | H | $CH_3$ | F | F | $OCH_3$ | $CH_3$ | Cl | |
| Q-9 | 0 | H | H | $CH_3$ | Cl | Cl | $OCH_3$ | $OCH_3$ | CH | |
| Q-9 | 0 | H | H | $CH_3$ | Cl | Cl | $CH_3$ | $CH_3$ | N | |
| Q-9 | 0 | H | H | $CH_3$ | F | Cl | $OCH_3$ | $CH_3$ | N | |
| Q-9 | 0 | H | H | $CH_3$ | F | $CH_3$ | $CH_3$ | $CH_3$ | CH | |
| Q-9 | 0 | H | H | $CH_3$ | $CH_3$ | $CH_3$ | $OCH_3$ | $OCH_3$ | CH | |
| Q-9 | 0 | H | H | $CH_3$ | $CH_3$ | $CH_2CH_3$ | $OCH_3$ | $OCH_3$ | N | |
| Q-9 | 0 | H | H | $CH_3$ | F | Br | Cl | $OCH_3$ | CH | |
| Q-9 | 0 | H | H | $CH_3$ | Br | Br | $OCH_3$ | $OCH_3$ | CH | |
| Q-9 | 0 | H | H | $CH_3$ | CN | CN | $CH_3$ | $OCH_3$ | CH | |
| Q-9 | 0 | H | H | $CH_3$ | CN | CN | $OCH_3$ | $OCH_3$ | CH | |
| Q-9 | 0 | H | H | $CH_3$ | CN | CN | $OCH_3$ | $OCH_3$ | N | |
| Q-9 | 0 | H | H | $CH_3$ | $OCH_3$ | $OCH_3$ | $CH_3$ | $OCH_3$ | CH | |
| Q-9 | 0 | H | H | $CH_3$ | H | Br | $CH_3$ | $CH_3$ | CH | |
| Q-9 | 0 | H | H | $CH_3$ | H | Br | $CH_3$ | $OCH_3$ | CH | |
| Q-9 | 0 | H | H | $CH_3$ | H | Br | $OCH_3$ | $OCH_3$ | CH | |
| Q-9 | 0 | H | H | $CH_2CH_3$ | H | H | $CH_3$ | $CH_3$ | N | |
| Q-9 | 0 | H | H | $CH_3$ | F | H | $CH_3$ | $OCH_3$ | N | |
| Q-9 | 0 | H | H | $CH_3$ | Cl | H | $OCH_3$ | $OCH_3$ | N | |
| Q-9 | 0 | H | H | $CH_3$ | F | F | $CH_3$ | $OCH_3$ | CH | |
| Q-9 | 0 | H | H | $CH_3$ | Br | H | $OCH_3$ | $OCH_3$ | N | |
| Q-9 | 0 | H | H | $CH_3$ | Cl | Cl | $CH_3$ | $OCH_3$ | N | |
| Q-9 | 0 | H | 5-$SCH_3$ | $CH_3$ | H | H | $CH_3$ | $OCH_3$ | CH | |
| Q-9 | 0 | H | 5-Cl | $CH_3$ | Cl | H | $OCH_3$ | $OCH_3$ | CH | |
| Q-9 | 0 | H | 5-Cl | $CH_3$ | F | F | $CH_3$ | $CH_3$ | N | |
| Q-9 | 0 | H | 5-$OCH_2F$ | $CH_3$ | Cl | H | $OCH_3$ | $OCH_3$ | N | |
| Q-9 | 0 | $CH_3$ | H | $CH_3$ | H | H | $OCH_3$ | $OCH_3$ | N | |
| Q-9 | 1 | H | H | $CH_3$ | H | H | $OCH_3$ | $OCH_3$ | CH | |
| Q-9 | 1 | H | H | $CH_3$ | F | F | $CH_3$ | $OCH_3$ | CH | |

Table I (continued)

| Q | n | R | $R_1$ | $R_3$ | $R_7$ | $R_8$ | X | Y | Z | m.p. (°C) |
|---|---|---|---|---|---|---|---|---|---|---|
| Q-9 | 1 | H | H | $CH_3$ | F | Cl | $CH_3$ | $CH_3$ | N | |
| Q-9 | 1 | H | H | $CH_3$ | $CH_3$ | Cl | $OCH_3$ | $CH_3$ | CH | |
| Q-9 | 1 | H | H | $CH_3$ | F | H | $CH_3$ | $OCH_3$ | CH | |
| Q-9 | 1 | H | H | $CH_3$ | Cl | H | $OCH_3$ | $OCH_3$ | CH | |
| Q-9 | 1 | H | H | H | Br | H | $CH_3$ | $OCH_3$ | N | |
| Q-9 | 1 | H | H | H | Cl | Cl | $CH_3$ | $OCH_3$ | CH | |
| Q-9 | 1 | H | H | H | $CH_3$ | H | $CH_3$ | $OCH_3$ | CH | |
| Q-9 | 1 | H | H | H | CN | H | $OCH_3$ | $OCH_3$ | CH | |
| Q-9 | 1 | H | H | H | CN | CN | $CH_3$ | $OCH_3$ | N | |
| Q-9 | 1 | H | H | H | $CH_3$ | Br | $CH_3$ | $CH_3$ | CH | |
| Q-9 | 1 | H | H | H | F | Br | Cl | $OCH_3$ | CH | |
| Q-9 | 1 | H | 3-$CH_3$ | H | F | H | $OCH_3$ | $OCH_3$ | CH | |
| Q-9 | 1 | H | 6-$OCH_3$ | $CH_3$ | Cl | H | $CH_3$ | $CH_3$ | N | |
| Q-9 | 1 | H | 5-Cl | $CH_2CH_3$ | Cl | Cl | $CH_3$ | $OCH_3$ | N | |
| Q-10 | 0 | H | H | $CH_3$ | H | H | $CH_3$ | $CH_3$ | CH | |
| Q-10 | 0 | H | H | $CH_3$ | H | H | $CH_3$ | $OCH_3$ | CH | |
| Q-10 | 0 | H | H | $CH_3$ | H | H | $OCH_3$ | $OCH_3$ | CH | |
| Q-10 | 0 | H | H | $CH_3$ | H | H | $CH_3$ | $CH_3$ | N | |
| Q-10 | 0 | H | H | $CH_3$ | H | H | $CH_3$ | $OCH_3$ | N | |
| Q-10 | 0 | H | H | $CH_3$ | H | H | $OCH_3$ | $OCH_3$ | N | |
| Q-10 | 0 | H | H | $CH_3$ | H | H | Cl | $OCH_3$ | CH | |
| Q-10 | 0 | H | H | $CH_3$ | H | $CH_3$ | $CH_3$ | $OCH_3$ | CH | |
| Q-10 | 0 | H | H | $CH_3$ | H | $CH_3$ | $OCH_3$ | $OCH_3$ | CH | |
| Q-10 | 0 | H | H | $CH_3$ | H | $CH_3$ | $OCH_3$ | $OCH_3$ | N | |
| Q-10 | 0 | H | H | $CH_3$ | H | $CH_3$ | $CH_3$ | $OCH_3$ | N | |
| Q-10 | 0 | H | H | $CH_3$ | H | Cl | $OCH_3$ | $OCH_3$ | CH | |
| Q-10 | 0 | H | H | $CH_3$ | H | Cl | $OCH_3$ | $CH_3$ | N | |
| Q-10 | 0 | H | H | $CH_3$ | H | Cl | $OCH_3$ | $OCH_3$ | CH | |
| Q-10 | 0 | H | H | $CH_3$ | H | Cl | Cl | $OCH_3$ | CH | |
| Q-10 | 0 | H | H | $CH_3$ | H | F | $CH_3$ | $CH_3$ | N | |
| Q-10 | 0 | H | H | $CH_3$ | H | F | $CH_3$ | $CH_3$ | CH | |
| Q-10 | 0 | H | H | $CH_3$ | H | F | $OCH_3$ | $CH_3$ | CH | |

Table I (continued)

| Q | n | R | $R_1$ | $R_3$ | $R_7$ | $R_8$ | X | Y | Z | m.p.(°C) |
|---|---|---|---|---|---|---|---|---|---|---|
| Q-10 | 0 | H | H | $CH_3$ | H | F | $OCH_3$ | $OCH_3$ | CH | |
| Q-10 | 0 | H | H | $CH_3$ | H | F | $CH_3$ | $OCH_3$ | N | |
| Q-10 | 0 | H | H | $CH_3$ | H | F | Cl | $OCH_3$ | CH | |
| Q-10 | 0 | H | H | $CH_3$ | H | CN | $CH_3$ | $OCH_3$ | N | |
| Q-10 | 0 | H | H | $CH_3$ | H | CN | $OCH_3$ | $OCH_3$ | CH | |
| Q-10 | 0 | H | H | $CH_3$ | H | $OCH_3$ | $CH_3$ | $OCH_3$ | N | |
| Q-10 | 0 | H | H | $CH_3$ | H | $OCH_3$ | $OCH_3$ | $OCH_3$ | CH | |
| Q-10 | 0 | H | H | $CH_3$ | H | $OCH_3$ | $OCH_3$ | $OCH_3$ | N | |
| Q-10 | 0 | H | H | $CH_3$ | H | $CO_2CH_3$ | $CH_3$ | $CH_3$ | N | |
| Q-10 | 0 | H | H | $CH_3$ | H | $CO_2CH_3$ | $OCH_3$ | $CH_3$ | CH | |
| Q-10 | 0 | H | H | $CH_3$ | H | $CO_2CH_3$ | $OCH_3$ | $OCH_3$ | CH | |
| Q-10 | 0 | H | H | $CH_3$ | H | $CO_2CH_3$ | $CH_3$ | $OCH_3$ | N | |
| Q-10 | 0 | H | H | $CH_3$ | H | $CO_2CH_3$ | $CH_3$ | $CH_3$ | CH | |
| Q-10 | 0 | H | H | $CH_3$ | H | $CO_2CH_3$ | $OCH_3$ | $OCH_3$ | N | |
| Q-10 | 0 | H | H | $CH_3$ | H | $CO_2CH_3$ | Cl | $OCH_3$ | CH | |
| Q-10 | 0 | H | H | $CH_3$ | H | $SO_2N(CH_3)_2$ | $CH_3$ | $CH_3$ | N | |
| Q-10 | 0 | H | H | $CH_3$ | H | $SO_2N(CH_3)_2$ | $OCH_3$ | $OCH_3$ | CH | |
| Q-10 | 0 | H | H | $CH_3$ | H | $SO_2N(CH_3)_2$ | $OCH_3$ | $OCH_3$ | N | |
| Q-10 | 0 | H | H | $CH_3$ | H | $SO_2N(CH_3)_2$ | $CH_3$ | $OCH_3$ | CH | |
| Q-10 | 0 | H | H | $CH_3$ | H | $SCH_3$ | $OCH_3$ | $OCH_3$ | CH | |
| Q-10 | 0 | H | H | $CH_3$ | H | $SCH_3$ | $CH_3$ | $CH_3$ | CH | |
| Q-10 | 0 | H | H | $CH_3$ | H | $SCH_3$ | $OCH_3$ | $CH_3$ | CH | |
| Q-10 | 0 | H | H | $CH_3$ | H | $SCH_3$ | Cl | $OCH_3$ | CH | |
| Q-10 | 0 | H | H | $CH_3$ | H | $SO_2CH_3$ | $CH_3$ | $CH_3$ | CH | |
| Q-10 | 0 | H | H | $CH_3$ | H | $SO_2CH_3$ | $CH_3$ | $OCH_3$ | CH | |
| Q-10 | 0 | H | H | $CH_3$ | H | $SO_2CH_3$ | $CH_3$ | $OCH_3$ | N | |
| Q-10 | 0 | H | H | $CH_3$ | F | F | $CH_3$ | $CH_3$ | CH | |
| Q-10 | 0 | H | H | $CH_3$ | F | F | $OCH_3$ | $CH_3$ | Cl | |
| Q-10 | 0 | H | H | $CH_3$ | Cl | Cl | $OCH_3$ | $OCH_3$ | CH | |
| Q-10 | 0 | H | H | $CH_3$ | Cl | Cl | $CH_3$ | $CH_3$ | N | |
| Q-10 | 0 | H | H | $CH_3$ | F | Cl | $OCH_3$ | $CH_3$ | N | |
| Q-10 | 0 | H | H | $CH_3$ | F | $CH_3$ | $CH_3$ | $CH_3$ | CH | |

Table I (continued)

| Q | n | R | $R_1$ | $R_3$ | $R_7$ | $R_8$ | X | Y | Z | m.p.(°C) |
|---|---|---|---|---|---|---|---|---|---|---|
| Q-10 | 0 | H | H | $CH_3$ | $CH_3$ | $CH_3$ | $OCH_3$ | $OCH_3$ | CH | |
| Q-10 | 0 | H | H | $CH_3$ | $CH_3$ | $CH_2CH_3$ | $OCH_3$ | $OCH_3$ | H | |
| Q-10 | 0 | H | H | $CH_3$ | F | Br | Cl | $OCH_3$ | CH | |
| Q-10 | 0 | H | H | $CH_3$ | Br | Br | $OCH_3$ | $OCH_3$ | CH | |
| Q-10 | 0 | H | H | $CH_3$ | CN | CN | $CH_3$ | $OCH_3$ | CH | |
| Q-10 | 0 | H | H | $CH_3$ | CN | CN | $OCH_3$ | $OCH_3$ | CH | |
| Q-10 | 0 | H | H | $CH_3$ | CN | CN | $OCH_3$ | $OCH_3$ | N | |
| Q-10 | 0 | H | H | $CH_3$ | $OCH_3$ | $OCH_3$ | $CH_3$ | $OCH_3$ | CH | |

| Q | n | R | $R_1$ | $R_7$ | $R_8$ | $R_3$ | X | Y | Z | m.p. (°C) |
|---|---|---|---|---|---|---|---|---|---|---|
| Q-10 | 0 | H | H | H | Br | $CH_3$ | $CH_3$ | $CH_3$ | CH | |
| Q-10 | 0 | H | H | H | Br | $CH_3$ | $CH_3$ | $OCH_3$ | CH | |
| Q-10 | 0 | H | H | H | Br | $CH_3$ | $OCH_3$ | $OCH_3$ | CH | |
| Q-10 | 0 | H | H | H | H | $CH_2CH_2CH_2CH_3$ | $OCH_3$ | $CH_3$ | N | |
| Q-10 | 0 | H | 5-$CH_3$ | F | H | $CH_3$ | $CH_3$ | $OCH_3$ | N | |
| Q-10 | 0 | H | H | Cl | H | $CH_3$ | $OCH_3$ | $OCH_3$ | N | |
| Q-10 | 0 | H | H | F | F | $CH_3$ | $CH_3$ | $OCH_3$ | CH | |
| Q-10 | 0 | H | H | Br | H | $CH_3$ | $OCH_3$ | $OCH_3$ | N | |
| Q-10 | 0 | H | H | Cl | Cl | $CH_3$ | $CH_3$ | $OCH_3$ | N | |
| Q-10 | 0 | H | 6-$CH_3$ | H | H | $CH_2CH_2CH_3$ | $OCH_3$ | $OCH_3$ | N | |
| Q-10 | 0 | H | 5-$OCH_3$ | H | H | $CH_2CH_3$ | $CH_3$ | $CH_3$ | CH | |
| Q-10 | 0 | H | 3-$SCH_3$ | H | H | $CH_2CH_2CH_3$ | $CH_3$ | $OCH_3$ | CH | |
| Q-10 | 0 | H | 6-Cl | Cl | H | $CH_2CH_3$ | $OCH_3$ | $OCH_3$ | CH | |
| Q-10 | 0 | H | 5-Cl | F | F | $CH_3$ | $CH_3$ | $CH_3$ | N | |
| Q-10 | 0 | H | 5-$OCH_2F$ | Cl | H | $CH_3$ | $OCH_3$ | $OCH_3$ | N | |
| Q-10 | 0 | $CH_3$ | H | H | H | H | $OCH_3$ | $OCH_3$ | N | |
| Q-10 | 1 | H | H | H | H | H | $OCH_3$ | $OCH_3$ | CH | |
| Q-10 | 1 | H | H | F | F | H | $CH_3$ | $OCH_3$ | CH | |
| Q-10 | 1 | H | H | F | Cl | $CH_3$ | $CH_3$ | $CH_3$ | N | |
| Q-10 | 1 | H | H | $CH_3$ | Cl | H | $OCH_3$ | $CH_3$ | CH | |

Table I (continued)

| Q | n | R | $R_1$ | $R_7$ | $R_8$ | $R_3$ | X | Y | Z | m.p. (°C) |
|---|---|---|---|---|---|---|---|---|---|---|
| Q-10 | 1 | H | H | F | H | H | $CH_3$ | $OCH_3$ | CH | |
| Q-10 | 1 | H | H | Cl | H | $CH_3$ | $OCH_3$ | $OCH_3$ | CH | |
| Q-10 | 1 | H | H | Br | H | H | $CH_3$ | $OCH_3$ | N | |
| Q-10 | 1 | H | H | Cl | Cl | $CH_3$ | $CH_3$ | $OCH_3$ | CH | |
| Q-10 | 1 | H | H | $CH_3$ | H | $CH_3$ | $CH_3$ | $OCH_3$ | CH | |
| Q-10 | 1 | H | H | CN | H | $CH_3$ | $OCH_3$ | $OCH_3$ | CH | |
| Q-10 | 1 | H | H | CN | CN | H | $CH_3$ | $OCH_3$ | N | |
| Q-10 | 1 | H | H | $CH_3$ | Br | H | $CH_3$ | $CH_3$ | CH | |
| Q-10 | 1 | H | H | F | Br | $CH_3$ | Cl | $OCH_3$ | CH | |
| Q-10 | 1 | H | 3-$CH_3$ | F | H | H | $OCH_3$ | $OCH_3$ | CH | |
| Q-10 | 1 | H | 5-$OCH_3$ | Cl | H | $CH_3$ | $CH_3$ | $CH_3$ | N | |
| Q-10 | 1 | H | 6-Cl | Cl | Cl | $CH_2CH_3$ | $CH_3$ | $OCH_3$ | N | |

| Q | n | R | $R_1$ | $R_3$ | $R_7$ | $R_8$ | $R_9$ | $R_{10}$ | $R_{11}$ | X | Y | Z | m.p.(°C) |
|---|---|---|---|---|---|---|---|---|---|---|---|---|---|
| Q-1 | 0 | H | H | - | H | H | H | H | - | $NHCH_3$ | $OCH_2CH_3$ | N | 203-206 |
| Q-1 | 0 | H | H | - | H | H | H | H | - | $CH_3$ | $SCH_3$ | CH | |
| Q-1 | 0 | H | H | - | H | H | Cl | H | - | $N(CH_3)H$ | $OCH_2CH_3$ | N | 154-158 |
| Q-1 | 0 | H | H | - | Cl | Cl | H | H | - | $OCH_3$ | $CH(OCH_3)_2$ | CH | |
| Q-1 | 0 | H | H | - | $CH_3$ | H | H | H | - | $OCH_3$ | $OCH_2CH=CH_2$ | CH | |
| Q-1 | 0 | H | H | - | $CH_3$ | H | H | Cl | - | $OCH_3$ | $OCH_2CH_3$ | CH | |
| Q-1 | 0 | H | H | - | F | F | H | H | - | $OCH_2CH_3$ | $OCH_2F$ | N | |
| Q-1 | 0 | H | H | - | H | CN | H | H | - | $OCH_3$ | $SCH_3$ | CH | |
| Q-1 | 0 | H | Cl | - | Br | H | H | $CH_3$ | - | $OCH_3$ | Br | CH | |
| Q-1 | 0 | H | H | - | H | Br | H | H | - | $OCH_3$ | $NH_2$ | CH | |
| Q-1 | 0 | H | H | - | H | Br | Cl | H | - | $OCH_3$ | $NHCH_3$ | N | |
| Q-1 | 0 | H | H | - | F | F | Cl | F | - | $CH_3$ | $OCH_2C\equiv CH$ | CH | |
| Q-1 | 0 | H | H | - | F | CN | H | H | - | $OCH_3$ | $CH_2CF_3$ | CH | |
| Q-1 | 0 | H | H | - | H | H | H | H | - | $OCF_2CH_3$ | $CH_3$ | N | |
| Q-1 | 0 | H | H | - | H | H | H | H | - | $OCH_3$ | $C\equiv CH$ | CH | |

Table I (continued)

| Q | n | R | $R_1$ | $R_3$ | $R_7$ | $R_8$ | $R_9$ | $R_{10}$ | $R_{11}$ | X | Y | Z | m.p. (°C) |
|---|---|---|---|---|---|---|---|---|---|---|---|---|---|
| Q-1 | 0 | H | H | - | Cl | Cl | H | H | - | $OCH_3$ | $\overset{O}{\overset{\parallel}{C}}CH_3$ | CH | |
| Q-2 | 0 | H | H | - | H | H | H | H | H | $OCH_3$ | $OCH_2CH_3$ | N | |
| Q-2 | 0 | H | H | - | Cl | H | H | H | H | $CH_3OCH_3$ | $CH_2CF_3$ | N | |
| Q-2 | 0 | H | H | - | Cl | H | $CH_3$ | H | H | $OCH_3$ | $N(CH_3)_2$ | N | |
| Q-4 | 0 | H | H | - | H | H | H | H | - | $CH_3$ | $CH_2CH_3$ | CH | |
| Q-4 | 0 | H | H | - | H | H | H | H | - | $OCH_3$ | $OCH_2OCH_3$ | CH | |
| Q-5 | 0 | H | H | - | Cl | H | H | H | - | $OCH_3$ | $SCH_2CH_3$ | CH | |
| Q-5 | 0 | H | H | - | Cl | Cl | H | H | - | $OCF_2H$ | $CH_2CH_3$ | CH | |
| Q-5 | 0 | H | H | - | $CH_3$ | H | H | $CH_3$ | - | $OCH_3$ | F | CH | |
| Q-9 | 0 | H | H | $CH_3$ | H | H | H | H | - | $SCH_2CH_2F$ | $HNCH_3$ | CH | |
| Q-9 | 0 | H | H | H | H | H | H | F | - | $OCH_3$ | $OCH_2CH{=}CH_2$ | CH | |
| Q-3 | 0 | H | H | $CH_3$ | H | H | H | H | H | $OCH_2CH_3$ | $SCH_2CH_3$ | N | |
| Q-3 | 0 | H | H | $CH_3$ | F | H | H | Br | $CH_3$ | $OCH_3$ | I | CH | |
| Q-3 | 0 | H | H | H | H | H | H | H | H | $OCH_2OCH_2CH_3$ | $CH_3$ | CH | |
| Q-7 | 0 | H | H | - | H | H | H | H | - | $OCH_2OCH_2CH_3$ | $OCH_3$ | N | |
| Q-7 | 0 | H | H | - | H | H | H | H | - | $OCHF_2$ | $N(CH_3)_2$ | CH | |
| Q-7 | 0 | H | H | - | Cl | H | H | H | - | $OCH_3$ | $N(OCH_3)CH_3$ | N | |

General Structure I (wherein $R_{12}$ and $R_{13}$ are H) (W = S)

| Q | n | R | $R_1$ | $R_7$ | $R_8$ | $R_9$ | $R_{10}$ | $R_{11}$ | X | Y | Z | m.p. (°C) |
|---|---|---|---|---|---|---|---|---|---|---|---|---|
| Q-1 | 0 | H | H | H | H | H | H | - | $CH_3$ | $CH_3$ | CH | |
| Q-1 | 0 | H | H | H | H | H | H | - | $CH_3$ | $OCH_3$ | CH | |
| Q-1 | 0 | H | H | H | H | H | H | - | $OCH_3$ | $OCH_3$ | CH | |
| Q-1 | 0 | H | 5-$CH_3$ | H | H | H | H | - | $CH_3$ | $OCH_3$ | N | |
| Q-1 | 0 | H | H | H | H | H | H | - | $CH_3$ | $OCH_3$ | N | |
| Q-1 | 0 | H | H | H | H | H | H | - | $OCH_3$ | $OCH_3$ | N | |
| Q-1 | 0 | H | H | H | H | H | H | - | Cl | $OCH_3$ | CH | |
| Q-1 | 0 | H | H | H | F | H | H | - | $CH_3$ | $CH_3$ | CH | |

Table I (continued)

| Q | n | R | $R_1$ | $R_7$ | $R_8$ | $R_9$ | $R_{10}$ | $R_{11}$ | X | Y | Z | m.p. (°C) |
|---|---|---|---|---|---|---|---|---|---|---|---|---|
| Q-1 | 0 | H | H | H | F | H | H | - | $CH_3$ | $CH_3$ | N | |
| Q-1 | 0 | H | H | H | F | H | H | - | $CH_3$ | $OCH_3$ | CH | |
| Q-1 | 0 | H | H | H | Cl | H | H | - | $OCH_3$ | $OCH_3$ | CH | |
| Q-1 | 0 | H | H | H | Cl | H | H | - | $CH_3$ | $CH_3$ | N | |
| Q-1 | 0 | H | H | Cl | H | H | H | - | $CH_3$ | $OCH_3$ | CH | |
| Q-1 | 0 | H | H | Cl | H | H | H | - | $CH_3$ | $OCH_3$ | N | |
| Q-1 | 0 | H | H | H | Br | H | H | - | $CH_3$ | $CH_3$ | CH | |
| Q-1 | 0 | H | H | Br | H | H | H | - | $CH_3$ | $OCH_3$ | CH | |
| Q-1 | 0 | H | H | Br | H | H | H | - | $OCH_3$ | $OCH_3$ | CH | |
| Q-1 | 0 | H | H | H | I | H | H | - | $CH_3$ | $CH_3$ | N | |
| Q-1 | 0 | H | H | I | H | H | H | - | $CH_3$ | $OCH_3$ | N | |
| Q-1 | 0 | H | H | H | CN | H | H | - | $OCH_3$ | $OCH_3$ | N | |
| Q-1 | 0 | H | H | CN | H | H | H | - | $OCH_3$ | $OCH_3$ | CH | |
| Q-1 | 0 | H | H | H | $CH_3$ | H | H | - | $CH_3$ | $CH_3$ | CH | |
| Q-1 | 0 | H | H | H | $CH_3$ | H | H | - | $CH_3$ | $OCH_3$ | CH | |
| Q-1 | 0 | H | H | H | $CH_3$ | H | H | - | $OCH_3$ | $OCH_3$ | CH | |
| Q-1 | 0 | H | H | $CH_3$ | H | H | H | - | Cl | $OCH_3$ | CH | |
| Q-1 | 0 | H | H | $CH_3$ | H | H | H | - | $CH_3$ | $OCH_3$ | N | |
| Q-1 | 0 | H | H | H | $CH_2CH_3$ | H | H | - | $OCH_3$ | $OCH_3$ | N | |
| Q-1 | 0 | H | H | $CH_2CH_3$ | H | H | H | - | $CH_3$ | $OCH_3$ | CH | |
| Q-1 | 0 | H | H | H | $CH_2CH_3$ | H | H | - | $OCH_3$ | $OCH_3$ | CH | |
| Q-1 | 0 | H | H | H | $CH_2CH_2CH_3$ | H | H | - | $CH_3$ | $OCH_3$ | N | |
| Q-1 | 0 | H | H | H | Cl | H | H | - | $CH_3$ | $CH_3$ | CH | |
| Q-1 | 0 | H | H | H | Cl | H | H | - | $OCH_3$ | $OCH_3$ | N | |
| Q-1 | 0 | H | H | H | Cl | H | H | - | Cl | $OCH_3$ | CH | |

Table IA

General Structure I (wherein $R_{12}$ is OH; $R_{13}$ is H)

| Q | n | R | $R_1$ | $R_7$ | $R_8$ | $R_9$ | $R_{10}$ | $R_{11}$ | X | Y | Z | m.p. (°C) |
|---|---|---|---|---|---|---|---|---|---|---|---|---|
| Q-1 | 1 | H | H | H | H | H | H | - | $CH_3$ | $CH_3$ | CH | 148-150 |
| Q-1 | 1 | H | H | H | H | H | H | - | $CH_3$ | $OCH_3$ | CH | 140-142 |
| Q-1 | 1 | H | H | H | H | H | H | - | $OCH_3$ | $OCH_3$ | CH | 148-150 |
| Q-1 | 1 | H | H | H | H | H | H | - | $CH_3$ | $CH_3$ | N | |
| Q-1 | 1 | H | H | H | H | H | H | - | $CH_3$ | $OCH_3$ | N | 109-111 |
| Q-1 | 1 | H | H | H | H | H | H | - | $OCH_3$ | $OCH_3$ | N | 147-149 |
| Q-1 | 1 | H | H | H | H | H | H | - | Cl | $OCH_3$ | CH | 124-126 |
| Q-1 | 1 | H | H | H | F | H | H | - | $CH_3$ | $CH_3$ | CH | |
| Q-1 | 1 | H | H | H | F | H | H | - | $CH_3$ | $CH_3$ | N | |
| Q-1 | 1 | H | H | H | F | H | H | - | $CH_3$ | $OCH_3$ | CH | |
| Q-1 | 1 | H | H | H | Cl | H | H | - | $OCH_3$ | $OCH_3$ | CH | |
| Q-1 | 1 | H | H | H | Cl | H | H | - | $CH_3$ | $CH_3$ | N | |
| Q-1 | 1 | H | H | Cl | H | H | H | - | $CH_3$ | $OCH_3$ | CH | |
| Q-1 | 1 | H | H | Cl | H | H | H | - | $CH_3$ | $OCH_3$ | N | |
| Q-1 | 1 | H | H | H | Br | H | H | - | $CH_3$ | $CH_3$ | CH | |
| Q-1 | 1 | H | H | Br | H | H | H | - | $CH_3$ | $OCH_3$ | CH | |
| Q-1 | 1 | H | H | Br | H | H | H | - | $OCH_3$ | $OCH_3$ | CH | |
| Q-1 | 1 | H | H | H | I | H | H | - | $CH_3$ | $CH_3$ | N | |
| Q-1 | 1 | H | H | I | H | H | H | - | $CH_3$ | $OCH_3$ | N | |
| Q-1 | 1 | H | H | H | CN | H | H | - | $OCH_3$ | $OCH_3$ | N | |
| Q-1 | 1 | H | H | CN | H | H | H | - | $OCH_3$ | $OCH_3$ | CH | |
| Q-1 | 1 | H | H | H | $CH_3$ | H | H | - | $CH_3$ | $CH_3$ | CH | |

Table IA (continued)

| Q | n | R | $R_1$ | $R_7$ | $R_8$ | $R_9$ | $R_{10}$ | $R_{11}$ | X | Y | Z | m.p. (°C) |
|---|---|---|---|---|---|---|---|---|---|---|---|---|
| Q-1 | 1 | H | H | H | $CH_3$ | H | H | - | $CH_3$ | $OCH_3$ | CH | |
| Q-1 | 1 | H | H | H | $CH_3$ | H | H | - | $OCH_3$ | $OCH_3$ | CH | |
| Q-1 | 1 | H | H | $CH_3$ | H | H | H | - | $CH_3$ | $CH_3$ | N | |
| Q-1 | 1 | H | H | $CH_3$ | H | H | H | - | $CH_3$ | $OCH_3$ | N | |
| Q-1 | 1 | H | H | H | $CH_2CH_3$ | H | H | - | $OCH_3$ | $OCH_3$ | N | |
| Q-1 | 1 | H | H | H | $CH_2CH_3$ | H | H | - | $OCH_3$ | $OCH_3$ | CH | |
| Q-1 | 1 | H | H | H | $CH_2CH_2CH_3$ | H | H | - | $CH_3$ | $OCH_3$ | N | |
| Q-1 | 1 | H | H | H | $OCH_3$ | H | H | - | $CH_3$ | $CH_3$ | CH | |
| Q-1 | 1 | H | H | H | $OCH_3$ | H | H | - | $OCH_3$ | $CH_3$ | CH | |
| Q-1 | 1 | H | H | $CH_3O$ | H | H | H | - | $OCH_3$ | $OCH_3$ | CH | |
| Q-1 | 1 | H | H | H | $OCH_2CH_3$ | H | H | - | $CH_3$ | $CH_3$ | N | |
| Q-1 | 1 | H | H | H | $CO_2CH_3$ | H | H | - | $CH_3$ | $OCH_3$ | N | |
| Q-1 | 1 | H | H | H | $CO_2CH_3$ | H | H | - | $OCH_3$ | $OCH_3$ | N | |
| Q-1 | 1 | H | H | H | $CO_2CH_3$ | H | H | - | $CH_3$ | $OCH_3$ | CH | |
| Q-1 | 1 | H | H | H | $CO_2CH_3$ | H | H | - | $OCH_3$ | $OCH_3$ | CH | |
| Q-1 | 1 | H | H | H | $CO_2CH_2CH_3$ | H | H | - | $CH_3$ | $OCH_3$ | N | |
| Q-1 | 1 | H | H | H | $SO_2N(CH_3)_2$ | H | H | - | $OCH_3$ | $OCH_3$ | N | |
| Q-1 | 1 | H | H | H | $SO_2N(CH_3)_2$ | H | H | - | $CH_3$ | $CH_3$ | CH | |
| Q-1 | 1 | H | H | H | $SCH_3$ | H | H | - | $OCH_3$ | $OCH_3$ | CH | |
| Q-1 | 1 | H | H | H | $SCH_3$ | H | H | - | $CH_3$ | $CH_3$ | N | |
| Q-1 | 1 | H | H | H | $SO_2CH_3$ | H | H | - | $CH_3$ | $OCH_3$ | N | |
| Q-1 | 1 | H | H | H | $SO_2CH_3$ | H | H | - | Cl | $OCH_3$ | CH | |
| Q-1 | 1 | H | H | H | $SO_2CH_3$ | H | H | - | $CH_3$ | $OCH_3$ | CH | |
| Q-1 | 1 | H | H | H | $SO_2CH_2CH_3$ | H | H | - | $OCH_3$ | $OCH_3$ | CH | |
| Q-1 | 1 | H | H | H | $CH_3$ | H | $CH_3$ | - | $CH_3$ | $OCH_3$ | N | |
| Q-1 | 1 | H | H | H | $CH_3$ | H | $CH_3$ | - | $OCH_3$ | $OCH_3$ | N | |
| Q-1 | 1 | H | H | H | $CH_3$ | $CH_3$ | H | - | $CH_3$ | $CH_3$ | CH | |
| Q-1 | 1 | H | H | H | $CH_3$ | H | F | - | $CH_3$ | $OCH_3$ | CH | |
| Q-1 | 1 | H | H | H | $CH_3$ | H | F | - | $OCH_3$ | $OCH_3$ | CH | |
| Q-1 | 1 | H | H | H | $CH_3$ | H | F | - | $CH_3$ | $OCH_3$ | N | |

Table IA (continued)

| Q | n | R | $R_1$ | $R_7$ | $R_8$ | $R_9$ | $R_{10}$ | $R_{11}$ | X | Y | Z | m.p.(°C) |
|---|---|---|---|---|---|---|---|---|---|---|---|---|
| Q-1 | 1 | H | H | H | $CH_3$ | H | Cl | - | $OCH_3$ | $OCH_3$ | N | |
| Q-1 | 1 | H | H | H | $CH_3$ | Br | H | - | $CH_3$ | $OCH_3$ | CH | |
| Q-1 | 1 | H | H | H | F | H | $CH_3$ | - | $OCH_3$ | $OCH_3$ | CH | |
| Q-1 | 1 | H | H | H | F | H | $CH_3$ | - | $CH_3$ | $OCH_3$ | CH | |
| Q-1 | 1 | H | H | H | Cl | H | $CH_3$ | - | $CH_3$ | $OCH_3$ | N | |
| Q-1 | 1 | H | H | H | Cl | H | F | - | $OCH_3$ | $OCH_3$ | CH | |
| Q-1 | 1 | H | H | H | Cl | H | Cl | - | Cl | $OCH_3$ | CH | |
| Q-1 | 1 | H | H | H | Cl | H | Cl | - | $OCH_3$ | $OCH_3$ | N | |
| Q-1 | 1 | H | H | H | Cl | H | Br | - | $CH_3$ | $OCH_3$ | CH | |
| Q-1 | 1 | H | H | H | $SO_2CH_3$ | H | $CH_3$ | - | $CH_3$ | $CH_3$ | CH | |
| Q-1 | 1 | H | H | H | $SO_2CH_3$ | H | $CH_3$ | - | $OCH_3$ | $CH_3$ | CH | |
| Q-1 | 1 | H | H | H | $SO_2CH_3$ | H | $CH_3$ | - | $OCH_3$ | $OCH_3$ | CH | |
| Q-1 | 1 | H | H | H | $SO_2CH_3$ | H | $CH_3$ | - | $CH_3$ | $CH_3$ | N | |
| Q-1 | 1 | H | H | H | $SO_2CH_3$ | H | $CH_3$ | - | $CH_3$ | $OCH_3$ | N | |
| Q-1 | 1 | H | H | H | $SO_2CH_3$ | H | $CH_3$ | - | $OCH_3$ | $OCH_3$ | N | |
| Q-1 | 1 | H | H | H | $SO_2CH_3$ | H | $CH_3$ | - | Cl | $OCH_3$ | CH | |
| Q-1 | 1 | H | H | H | $SO_2CH_3$ | H | Cl | - | $OCH_3$ | $OCH_3$ | CH | |
| Q-1 | 1 | H | H | H | $SO_2CH_3$ | H | Cl | - | $CH_3$ | $OCH_3$ | N | |
| Q-1 | 1 | H | H | H | $SO_2CH_3$ | H | F | - | $OCH_3$ | $OCH_3$ | N | |
| Q-1 | 1 | H | H | H | $SO_2CH_3$ | H | Br | - | $CH_3$ | $CH_3$ | CH | |
| Q-1 | 1 | H | H | F | F | H | H | - | $CH_3$ | $OCH_3$ | CH | |
| Q-1 | 1 | H | H | F | F | H | H | - | $OCH_3$ | $OCH_3$ | CH | |
| Q-1 | 1 | H | H | F | F | H | H | - | $CH_3$ | $CH_3$ | N | |
| Q-1 | 1 | H | H | F | F | H | H | - | $CH_3$ | $OCH_3$ | N | |
| Q-1 | 1 | H | H | F | F | H | H | - | $OCH_3$ | $OCH_3$ | N | |
| Q-1 | 1 | H | H | F | F | H | H | - | Cl | $OCH_3$ | CH | |
| Q-1 | 1 | H | H | Cl | Cl | H | H | - | $CH_3$ | $OCH_3$ | CH | |
| Q-1 | 1 | H | H | Cl | Cl | H | H | - | $OCH_3$ | $OCH_3$ | CH | |
| Q-1 | 1 | H | H | Cl | Cl | H | H | - | $CH_3$ | $OCH_3$ | N | |
| Q-1 | 1 | H | H | Cl | Cl | H | H | - | $OCH_3$ | $OCH_3$ | N | |
| Q-1 | 1 | H | H | Cl | Cl | H | H | - | $CH_3$ | $CH_3$ | CH | |
| Q-1 | 1 | H | H | Cl | Cl | H | H | - | $CH_3$ | $CH_3$ | N | |
| Q-1 | 1 | H | H | Cl | Cl | H | H | - | Cl | $OCH_3$ | CH | |

Table IA (continued)

| Q | n | R | $R_1$ | $R_7$ | $R_8$ | $R_9$ | $R_{10}$ | $R_{11}$ | X | Y | Z | m.p.(°C) |
|---|---|---|---|---|---|---|---|---|---|---|---|---|
| Q-1 | 1 | H | H | Br | Br | H | H | - | $CH_3$ | $OCH_3$ | N | |
| Q-1 | 1 | H | H | Br | Br | H | H | - | $OCH_3$ | $OCH_3$ | N | |
| Q-1 | 1 | H | H | Br | Br | H | H | - | $CH_3$ | $OCH_3$ | CH | |
| Q-1 | 1 | H | H | Br | Br | H | H | - | $OCH_3$ | $OCH_3$ | CH | |
| Q-1 | 1 | H | H | Br | Br | H | H | - | $CH_3$ | $CH_3$ | N | |
| Q-1 | 1 | H | H | I | I | H | H | - | $OCH_3$ | $OCH_3$ | CH | |
| Q-1 | 1 | H | H | CN | CN | H | H | - | Cl | $OCH_3$ | CH | |
| Q-1 | 1 | H | H | CN | CN | H | H | - | $OCH_3$ | $OCH_3$ | N | |
| Q-1 | 1 | H | H | CN | CN | H | H | - | $CH_3$ | $OCH_3$ | CH | |
| Q-1 | 1 | H | H | F | F | H | $CH_3$ | - | $CH_3$ | $CH_3$ | CH | |
| Q-1 | 1 | H | H | F | F | H | $CH_3$ | - | $OCH_3$ | $CH_3$ | CH | |
| Q-1 | 1 | H | H | F | F | H | F | - | $OCH_3$ | $OCH_3$ | CH | |
| Q-1 | 1 | H | H | F | F | H | Cl | - | $CH_3$ | $CH_3$ | N | |
| Q-1 | 1 | H | H | F | F | H | Br | - | $CH_3$ | $OCH_3$ | N | |
| Q-1 | 1 | H | H | Cl | Cl | H | $CH_3$ | - | $OCH_3$ | $OCH_3$ | N | |
| Q-1 | 1 | H | H | Cl | Cl | H | $CH_3$ | - | $CH_3$ | $OCH_3$ | CH | |
| Q-1 | 1 | H | H | Cl | Cl | H | Cl | - | $OCH_3$ | $OCH_3$ | CH | |
| Q-1 | 1 | H | H | Cl | Cl | H | F | - | $CH_3$ | $OCH_3$ | N | |
| Q-1 | 1 | H | H | Cl | Cl | H | Br | - | $OCH_3$ | $OCH_3$ | N | |
| Q-1 | 1 | H | H | Br | Br | H | $CH_3$ | - | $CH_3$ | $CH_3$ | CH | |
| Q-1 | 1 | H | H | Br | Br | H | $CH_3$ | - | $CH_3$ | $OCH_3$ | CH | |
| Q-1 | 1 | H | H | Br | Br | H | Cl | - | $OCH_3$ | $OCH_3$ | CH | |
| Q-1 | 1 | H | H | Br | Br | H | Cl | - | $CH_3$ | $CH_3$ | N | |
| Q-1 | 1 | H | H | Br | Br | Cl | H | - | $CH_3$ | $OCH_3$ | N | |
| Q-1 | 1 | H | H | Br | Br | H | F | - | $OCH_3$ | $OCH_3$ | N | |
| Q-1 | 1 | H | H | Br | Br | H | Br | - | Cl | $OCH_3$ | CH | |
| Q-1 | 1 | H | H | CN | CN | H | $CH_3$ | - | $CH_3$ | $OCH_3$ | CH | |
| Q-1 | 1 | H | H | CN | CN | H | $CH_3$ | - | $OCH_3$ | $OCH_3$ | CH | |
| Q-1 | 1 | H | H | CN | CN | H | F | - | $CH_3$ | $OCH_3$ | N | |
| Q-1 | 1 | H | H | $CH_3$ | $CH_3$ | H | $CH_3$ | - | $OCH_3$ | $OCH_3$ | N | |
| Q-1 | 1 | H | H | $CH_3$ | $CH_3$ | H | $CH_3$ | - | $CH_3$ | $CH_3$ | CH | |
| Q-1 | 1 | H | H | $CH_3$ | $CH_3$ | H | $CH_3$ | - | $CH_3$ | $OCH_3$ | CH | |
| Q-1 | 1 | H | H | $CH_3$ | $CH_3$ | $CH_3$ | H | - | $OCH_3$ | $OCH_3$ | CH | |

Table IB

General Structure I (wherein $R_{12}$ is OH; $R_{13}$ is $CH_3$)

| Q | n | R | $R_1$ | $R_7$ | $R_8$ | $R_9$ | $R_{10}$ | $R_{11}$ | X | Y | Z | m.p. (°C) |
|---|---|---|---|---|---|---|---|---|---|---|---|---|
| Q-1 | 1 | H | H | H | H | H | H | - | $CH_3$ | $CH_3$ | CH | |
| Q-1 | 1 | H | H | H | H | H | H | - | $CH_3$ | $OCH_3$ | CH | |
| Q-1 | 1 | H | H | H | H | H | H | - | $OCH_3$ | $OCH_3$ | CH | |
| Q-1 | 1 | H | H | H | H | H | H | - | $CH_3$ | $CH_3$ | N | |
| Q-1 | 1 | H | H | H | H | H | H | - | $CH_3$ | $OCH_3$ | N | |
| Q-1 | 1 | H | H | H | H | H | H | - | $OCH_3$ | $OCH_3$ | N | |
| Q-1 | 1 | H | H | H | H | H | H | - | Cl | $OCH_3$ | CH | |
| Q-1 | 1 | H | H | H | F | H | H | - | $CH_3$ | $CH_3$ | CH | |
| Q-1 | 1 | H | H | H | F | H | H | - | $CH_3$ | $CH_3$ | N | |
| Q-1 | 1 | H | H | H | F | H | H | - | $CH_3$ | $OCH_3$ | CH | |
| Q-1 | 1 | H | H | H | Cl | H | H | - | $OCH_3$ | $OCH_3$ | CH | |
| Q-1 | 1 | H | H | H | Cl | H | H | - | $CH_3$ | $CH_3$ | N | |
| Q-1 | 1 | H | H | Cl | H | H | H | - | $CH_3$ | $OCH_3$ | CH | |
| Q-1 | 1 | H | H | Cl | H | H | H | - | $CH_3$ | $OCH_3$ | N | |
| Q-1 | 1 | H | H | H | Br | H | H | - | $CH_3$ | $CH_3$ | CH | |
| Q-1 | 1 | H | H | Br | H | H | H | - | $CH_3$ | $OCH_3$ | CH | |
| Q-1 | 1 | H | H | Br | H | H | H | - | $OCH_3$ | $OCH_3$ | CH | |
| Q-1 | 1 | H | H | H | I | H | H | - | $CH_3$ | $CH_3$ | N | |
| Q-1 | 1 | H | H | I | H | H | H | - | $CH_3$ | $OCH_3$ | N | |
| Q-1 | 1 | H | H | H | CN | H | H | - | $OCH_3$ | $OCH_3$ | N | |
| Q-1 | 1 | H | H | CN | H | H | H | - | $OCH_3$ | $OCH_3$ | CH | |
| Q-1 | 1 | H | H | H | $CH_3$ | H | H | - | $CH_3$ | $CH_3$ | CH | |

Table IC

General Structure I (wherein $R_{12}$ is $OCH_3$; $R_{13}$ is H)

| Q | n | R | $R_1$ | $R_7$ | $R_8$ | $R_9$ | $R_{10}$ | $R_{11}$ | X | Y | Z | m.p. (°C) |
|---|---|---|---|---|---|---|---|---|---|---|---|---|
| Q-1 | 1 | H | H | H | H | H | H | - | $CH_3$ | $CH_3$ | CH | |
| Q-1 | 1 | H | H | H | H | H | H | - | $CH_3$ | $OCH_3$ | CH | |
| Q-1 | 1 | H | H | H | H | H | H | - | $OCH_3$ | $OCH_3$ | CH | |
| Q-1 | 1 | H | H | H | H | H | H | - | $CH_3$ | $CH_3$ | N | |
| Q-1 | 1 | H | H | H | H | H | H | - | $CH_3$ | $OCH_3$ | N | |
| Q-1 | 1 | H | H | H | H | H | H | - | $OCH_3$ | $OCH_3$ | N | |
| Q-1 | 1 | H | H | H | H | H | H | - | Cl | $OCH_3$ | CH | |
| Q-1 | 1 | H | H | H | F | H | H | - | $CH_3$ | $CH_3$ | CH | |
| Q-1 | 1 | H | H | H | F | H | H | - | $CH_3$ | $CH_3$ | N | |
| Q-1 | 1 | H | H | H | F | H | H | - | $CH_3$ | $OCH_3$ | CH | |
| Q-1 | 1 | H | H | H | Cl | H | H | - | $OCH_3$ | $OCH_3$ | CH | |
| Q-1 | 1 | H | H | H | Cl | H | H | - | $CH_3$ | $CH_3$ | N | |
| Q-1 | 1 | H | H | Cl | H | H | H | - | $CH_3$ | $OCH_3$ | CH | |
| Q-1 | 1 | H | H | Cl | H | H | H | - | $CH_3$ | $OCH_3$ | N | |
| Q-1 | 1 | H | H | H | Br | H | H | - | $CH_3$ | $CH_3$ | CH | |
| Q-1 | 1 | H | H | Br | H | H | H | - | $CH_3$ | $OCH_3$ | CH | |
| Q-1 | 1 | H | H | Br | H | H | H | - | $OCH_3$ | $OCH_3$ | CH | |
| Q-1 | 1 | H | H | H | I | H | H | - | $CH_3$ | $CH_3$ | N | |
| Q-1 | 1 | H | H | I | H | H | H | - | $CH_3$ | $OCH_3$ | N | |
| Q-1 | 1 | H | H | H | CN | H | H | - | $OCH_3$ | $OCH_3$ | N | |
| Q-1 | 1 | H | H | CN | H | H | H | - | $OCH_3$ | $OCH_3$ | CH | |
| Q-1 | 1 | H | H | H | $CH_3$ | H | H | - | $CH_3$ | $CH_3$ | CH | |

**Table ID**

**General Structure I** (wherein $R_{12}$ is OH; $R_{13}$ is $CH_3$)

| Q | n | R | $R_1$ | $R_7$ | $R_8$ | X | Y | Z | m.p.(°C) |
|---|---|---|---|---|---|---|---|---|---|
| Q-4 | 1 | H | H | H | H | $CH_3$ | $CH_3$ | CH | |
| Q-4 | 1 | H | H | H | H | $CH_3$ | $OCH_3$ | CH | |
| Q-4 | 1 | H | H | H | H | $OCH_3$ | $OCH_3$ | CH | |
| Q-4 | 1 | H | H | H | H | $CH_3$ | $CH_3$ | N | |
| Q-4 | 1 | H | H | H | H | $CH_3$ | $OCH_3$ | N | |
| Q-4 | 1 | H | H | H | H | $OCH_3$ | $OCH_3$ | N | |
| Q-4 | 1 | H | H | H | H | Cl | $OCH_3$ | CH | |
| Q-4 | 1 | H | H | H | $CH_3$ | $CH_3$ | $OCH_3$ | CH | |
| Q-4 | 1 | H | H | H | $CH_3$ | $OCH_3$ | $OCH_3$ | CH | |
| Q-4 | 1 | H | H | H | $CH_3$ | $OCH_3$ | $OCH_3$ | N | |
| Q-4 | 1 | H | H | H | $CH_3$ | $CH_3$ | $OCH_3$ | CH | |
| Q-4 | 1 | H | H | H | Cl | $CH_3$ | $OCH_3$ | CH | |
| Q-4 | 1 | H | H | H | Cl | $OCH_3$ | $CH_3$ | N | |
| Q-4 | 1 | H | H | H | Cl | $OCH_3$ | $OCH_3$ | CH | |
| Q-4 | 1 | H | H | H | Cl | Cl | $OCH_3$ | CH | |
| Q-4 | 1 | H | H | H | F | $CH_3$ | $CH_3$ | N | |
| Q-4 | 1 | H | H | H | F | $CH_3$ | $CH_3$ | CH | |
| Q-4 | 1 | H | H | H | F | $OCH_3$ | $CH_3$ | CH | |
| Q-4 | 1 | H | H | H | F | $OCH_3$ | $OCH_3$ | CH | |
| Q-4 | 1 | H | H | H | F | $CH_3$ | $OCH_3$ | N | |
| Q-4 | 1 | H | H | H | F | Cl | $OCH_3$ | CH | |
| Q-4 | 1 | H | H | H | CN | $CH_3$ | $OCH_3$ | N | |
| Q-4 | 1 | H | H | H | CN | $OCH_3$ | $OCH_3$ | CH | |
| Q-4 | 1 | H | H | H | $OCH_3$ | $CH_3$ | $OCH_3$ | N | |
| Q-4 | 1 | H | H | H | $OCH_3$ | $OCH_3$ | $OCH_3$ | CH | |
| Q-4 | 1 | H | H | H | $OCH_3$ | $OCH_3$ | $OCH_3$ | N | |
| Q-4 | 1 | H | H | H | $CO_2CH_3$ | $CH_3$ | $CH_3$ | N | |
| Q-4 | 1 | H | H | H | $CO_2CH_3$ | $OCH_3$ | $CH_3$ | CH | |
| Q-4 | 1 | H | H | H | $CO_2CH_3$ | $OCH_3$ | $OCH_3$ | CH | |
| Q-4 | 1 | H | H | H | $CO_2CH_3$ | $CH_3$ | $OCH_3$ | N | |
| Q-4 | 1 | H | H | H | $CO_2CH_3$ | $CH_3$ | $CH_3$ | CH | |
| Q-4 | 1 | H | H | H | $CO_2CH_3$ | $OCH_3$ | $OCH_3$ | N | |
| Q-4 | 1 | H | H | H | $CO_2CH_3$ | Cl | $OCH_3$ | CH | |

Table IE

General Structure I (wherein $R_{12}$ is OH; $R_{13}$ is $CH_3$)

| Q | n | R | $R_1$ | $R_7$ | $R_8$ | X | Y | Z | m.p.(°C) |
|---|---|---|---|---|---|---|---|---|---|
| Q-4 | 1 | H | H | H | H | $CH_3$ | $CH_3$ | CH | |
| Q-4 | 1 | H | H | H | H | $CH_3$ | $OCH_3$ | CH | |
| Q-4 | 1 | H | H | H | H | $OCH_3$ | $OCH_3$ | CH | |
| Q-4 | 1 | H | H | H | H | $CH_3$ | $CH_3$ | N | |
| Q-4 | 1 | H | H | H | H | $CH_3$ | $OCH_3$ | N | |
| Q-4 | 1 | H | H | H | H | $OCH_3$ | $OCH_3$ | N | |
| Q-4 | 1 | H | H | H | H | Cl | $OCH_3$ | CH | |
| Q-4 | 1 | H | H | H | $CH_3$ | $CH_3$ | $OCH_3$ | CH | |
| Q-4 | 1 | H | H | H | $CH_3$ | $OCH_3$ | $OCH_3$ | CH | |
| Q-4 | 1 | H | H | H | $CH_3$ | $OCH_3$ | $OCH_3$ | N | |
| Q-4 | 1 | H | H | H | $CH_3$ | $CH_3$ | $OCH_3$ | CH | |
| Q-4 | 1 | H | H | H | Cl | $CH_3$ | $OCH_3$ | CH | |
| Q-4 | 1 | H | H | H | Cl | $OCH_3$ | $CH_3$ | N | |
| Q-4 | 1 | H | H | H | Cl | $OCH_3$ | $OCH_3$ | CH | |
| Q-4 | 1 | H | H | H | Cl | Cl | $OCH_3$ | CH | |
| Q-4 | 1 | H | H | H | F | $CH_3$ | $CH_3$ | N | |
| Q-4 | 1 | H | H | H | F | $CH_3$ | $CH_3$ | CH | |
| Q-4 | 1 | H | H | H | F | $OCH_3$ | $CH_3$ | CH | |
| Q-4 | 1 | H | H | H | F | $OCH_3$ | $OCH_3$ | CH | |
| Q-4 | 1 | H | H | H | F | $CH_3$ | $OCH_3$ | N | |
| Q-4 | 1 | H | H | H | F | Cl | $OCH_3$ | CH | |
| Q-4 | 1 | H | H | H | CN | $CH_3$ | $OCH_3$ | N | |
| Q-4 | 1 | H | H | H | CN | $OCH_3$ | $OCH_3$ | CH | |
| Q-4 | 1 | H | H | H | $OCH_3$ | $CH_3$ | $OCH_3$ | N | |
| Q-4 | 1 | H | H | H | $OCH_3$ | $OCH_3$ | $OCH_3$ | CH | |
| Q-4 | 1 | H | H | H | $OCH_3$ | $OCH_3$ | $OCH_3$ | N | |
| Q-4 | 1 | H | H | H | $CO_2CH_3$ | $CH_3$ | $CH_3$ | N | |
| Q-4 | 1 | H | H | H | $CO_2CH_3$ | $OCH_3$ | $CH_3$ | CH | |
| Q-4 | 1 | H | H | H | $CO_2CH_3$ | $OCH_3$ | $OCH_3$ | CH | |
| Q-4 | 1 | H | H | H | $CO_2CH_3$ | $CH_3$ | $OCH_3$ | N | |
| Q-4 | 1 | H | H | H | $CO_2CH_3$ | $CH_3$ | $CH_3$ | CH | |
| Q-4 | 1 | H | H | H | $CO_2CH_3$ | $OCH_3$ | $OCH_3$ | N | |
| Q-4 | 1 | H | H | H | $CO_2CH_3$ | Cl | $OCH_3$ | CH | |

Table IF

General Structure I (wherein $R_{12}$ is $OCH_3$; $R_{13}$ is H)

| Q | n | R | $R_1$ | $R_7$ | $R_8$ | X | Y | Z | m.p.(°C) |
|---|---|---|---|---|---|---|---|---|---|
| Q-4 | 1 | H | H | H | H | $CH_3$ | $CH_3$ | CH | |
| Q-4 | 1 | H | H | H | H | $CH_3$ | $OCH_3$ | CH | |
| Q-4 | 1 | H | H | H | H | $OCH_3$ | $OCH_3$ | CH | |
| Q-4 | 1 | H | H | H | H | $CH_3$ | $CH_3$ | N | |
| Q-4 | 1 | H | H | H | H | $CH_3$ | $OCH_3$ | N | |
| Q-4 | 1 | H | H | H | H | $OCH_3$ | $OCH_3$ | N | |
| Q-4 | 1 | H | H | H | H | Cl | $OCH_3$ | CH | |
| Q-4 | 1 | H | H | H | $CH_3$ | $CH_3$ | $OCH_3$ | CH | |
| Q-4 | 1 | H | H | H | $CH_3$ | $OCH_3$ | $OCH_3$ | CH | |
| Q-4 | 1 | H | H | H | $CH_3$ | $OCH_3$ | $OCH_3$ | N | |
| Q-4 | 1 | H | H | H | $CH_3$ | $CH_3$ | $OCH_3$ | CH | |
| Q-4 | 1 | H | H | H | Cl | $CH_3$ | $OCH_3$ | CH | |
| Q-4 | 1 | H | H | H | Cl | $OCH_3$ | $CH_3$ | N | |
| Q-4 | 1 | H | H | H | Cl | $OCH_3$ | $OCH_3$ | CH | |
| Q-4 | 1 | H | H | H | Cl | Cl | $OCH_3$ | CH | |
| Q-4 | 1 | H | H | H | F | $CH_3$ | $CH_3$ | N | |
| Q-4 | 1 | H | H | H | F | $CH_3$ | $CH_3$ | CH | |
| Q-4 | 1 | H | H | H | F | $OCH_3$ | $CH_3$ | CH | |
| Q-4 | 1 | H | H | H | F | $OCH_3$ | $OCH_3$ | CH | |
| Q-4 | 1 | H | H | H | F | $CH_3$ | $OCH_3$ | N | |
| Q-4 | 1 | H | H | H | F | Cl | $OCH_3$ | CH | |
| Q-4 | 1 | H | H | H | CN | $CH_3$ | $OCH_3$ | N | |
| Q-4 | 1 | H | H | H | CN | $OCH_3$ | $OCH_3$ | CH | |
| Q-4 | 1 | H | H | H | $OCH_3$ | $CH_3$ | $OCH_3$ | N | |
| Q-4 | 1 | H | H | H | $OCH_3$ | $OCH_3$ | $OCH_3$ | CH | |
| Q-4 | 1 | H | H | H | $OCH_3$ | $OCH_3$ | $OCH_3$ | N | |
| Q-4 | 1 | H | H | H | $CO_2CH_3$ | $CH_3$ | $CH_3$ | N | |
| Q-4 | 1 | H | H | H | $CO_2CH_3$ | $OCH_3$ | $CH_3$ | CH | |
| Q-4 | 1 | H | H | H | $CO_2CH_3$ | $OCH_3$ | $OCH_3$ | CH | |
| Q-4 | 1 | H | H | H | $CO_2CH_3$ | $CH_3$ | $OCH_3$ | N | |
| Q-4 | 1 | H | H | H | $CO_2CH_3$ | $CH_3$ | $CH_3$ | CH | |
| Q-4 | 1 | H | H | H | $CO_2CH_3$ | $OCH_3$ | $OCH_3$ | N | |
| Q-4 | 1 | H | H | H | $CO_2CH_3$ | Cl | $OCH_3$ | CH | |

Table IG

General Structure I (wherein $R_{12}$ is $OCH_3$; $R_{13}$ is $CH_3$)

| Q | n | R | $R_1$ | $R_7$ | $R_8$ | X | Y | Z | m.p.(°C) |
|---|---|---|---|---|---|---|---|---|---|
| Q-4 | 1 | H | H | H | H | $CH_3$ | $CH_3$ | CH | |
| Q-4 | 1 | H | H | H | H | $CH_3$ | $OCH_3$ | CH | |
| Q-4 | 1 | H | H | H | H | $OCH_3$ | $OCH_3$ | CH | |
| Q-4 | 1 | H | H | H | H | $CH_3$ | $CH_3$ | N | |
| Q-4 | 1 | H | H | H | H | $CH_3$ | $OCH_3$ | N | |
| Q-4 | 1 | H | H | H | H | $OCH_3$ | $OCH_3$ | N | |
| Q-4 | 1 | H | H | H | H | Cl | $OCH_3$ | CH | |
| Q-4 | 1 | H | H | H | $CH_3$ | $CH_3$ | $OCH_3$ | CH | |
| Q-4 | 1 | H | H | H | $CH_3$ | $OCH_3$ | $OCH_3$ | CH | |
| Q-4 | 1 | H | H | H | $CH_3$ | $OCH_3$ | $OCH_3$ | N | |
| Q-4 | 1 | H | H | H | $CH_3$ | $CH_3$ | $OCH_3$ | CH | |
| Q-4 | 1 | H | H | H | Cl | $CH_3$ | $OCH_3$ | CH | |
| Q-4 | 1 | H | H | H | Cl | $OCH_3$ | $CH_3$ | N | |
| Q-4 | 1 | H | H | H | Cl | $OCH_3$ | $OCH_3$ | CH | |
| Q-4 | 1 | H | H | H | Cl | Cl | $OCH_3$ | CH | |
| Q-4 | 1 | H | H | H | F | $CH_3$ | $CH_3$ | N | |
| Q-4 | 1 | H | H | H | F | $CH_3$ | $CH_3$ | CH | |
| Q-4 | 1 | H | H | H | F | $OCH_3$ | $CH_3$ | CH | |
| Q-4 | 1 | H | H | H | F | $OCH_3$ | $OCH_3$ | CH | |
| Q-4 | 1 | H | H | H | F | $CH_3$ | $OCH_3$ | N | |
| Q-4 | 1 | H | H | H | F | Cl | $OCH_3$ | CH | |
| Q-4 | 1 | H | H | H | CN | $CH_3$ | $OCH_3$ | N | |
| Q-4 | 1 | H | H | H | CN | $OCH_3$ | $OCH_3$ | CH | |
| Q-4 | 1 | H | H | H | $OCH_3$ | $CH_3$ | $OCH_3$ | N | |
| Q-4 | 1 | H | H | H | $OCH_3$ | $OCH_3$ | $OCH_3$ | CH | |
| Q-4 | 1 | H | H | H | $OCH_3$ | $OCH_3$ | $OCH_3$ | N | |
| Q-4 | 1 | H | H | H | $CO_2CH_3$ | $CH_3$ | $CH_3$ | N | |
| Q-4 | 1 | H | H | H | $CO_2CH_3$ | $OCH_3$ | $CH_3$ | CH | |
| Q-4 | 1 | H | H | H | $CO_2CH_3$ | $OCH_3$ | $OCH_3$ | CH | |
| Q-4 | 1 | H | H | H | $CO_2CH_3$ | $CH_3$ | $OCH_3$ | N | |
| Q-4 | 1 | H | H | H | $CO_2CH_3$ | $CH_3$ | $CH_3$ | CH | |
| Q-4 | 1 | H | H | H | $CO_2CH_3$ | $OCH_3$ | $OCH_3$ | N | |
| Q-4 | 1 | H | H | H | $CO_2CH_3$ | Cl | $OCH_3$ | CH | |

Table II

General Structure II (wherein $R_{12}$ and $R_{13}$ are H)

| L | $Q_1$ | n | R | $R_1$ | $R_2$ | $R_7$ | $R_8$ | $R_9$ | $R_{10}$ | $R_{11}$ | X | Y | Z | m.p. (°C) |
|---|---|---|---|---|---|---|---|---|---|---|---|---|---|---|
| L-9 | Q-1 | 0 | H | H | - | H | H | H | H | - | $CH_3$ | $CH_3$ | CH | |
| L-9 | Q-1 | 0 | H | H | - | H | Cl | H | H | - | $CH_3$ | $OCH_3$ | CH | |
| L-9 | Q-1 | 0 | H | H | - | Cl | Cl | H | H | - | $OCH_3$ | $OCH_3$ | CH | |
| L-9 | Q-1 | 0 | H | H | - | F | F | H | H | - | $CH_3$ | $CH_3$ | N | |
| L-9 | Q-1 | 0 | H | H | - | H | Br | H | H | - | $CH_3$ | $OCH_3$ | N | |
| L-9 | Q-1 | 0 | H | H | - | $CH_3$ | H | H | H | - | $OCH_3$ | $OCH_3$ | N | |
| L-9 | Q-1 | 0 | H | H | - | H | $OCH_3$ | H | H | - | Cl | $OCH_3$ | CH | |
| L-9 | Q-1 | 0 | H | H | - | H | F | H | H | - | $CH_3$ | $OCH_3$ | CH | |
| L-9 | Q-1 | 0 | H | H | - | Cl | Br | H | H | - | $OCH_3$ | $OCH_3$ | CH | |
| L-9 | Q-1 | 0 | H | H | - | Cl | $CH_3$ | H | H | - | $CH_3$ | $OCH_3$ | N | |
| L-9 | Q-1 | 0 | H | H | - | F | $CH_3$ | H | H | - | $OCH_3$ | $OCH_3$ | CH | |
| L-9 | Q-1 | 0 | H | H | - | H | Cl | Cl | H | - | $CH_3$ | $CH_3$ | N | |
| L-9 | Q-1 | 0 | H | H | - | H | $CH_3$ | F | F | - | $OCH_3$ | $OCH_3$ | CH | |
| L-9 | Q-1 | 0 | H | $CH_3$ | - | H | H | H | H | - | Cl | $OCH_3$ | CH | |
| L-9 | Q-1 | 0 | $CH_3$ | H | - | H | H | H | H | - | $CH_3$ | $OCH_3$ | N | |
| L-9 | Q-1 | 1 | H | H | - | F | F | H | H | - | $OCH_3$ | $OCH_3$ | CH | |
| L-9 | Q-1 | 1 | H | H | - | H | H | H | H | - | $OCH_3$ | $OCH_3$ | CH | |
| L-9 | Q-2 | 0 | H | H | - | H | H | - | - | H | $CH_3$ | $CH_3$ | CH | |
| L-9 | Q-2 | 0 | H | H | - | H | H | - | - | H | $OCH_3$ | $CH_3$ | CH | |
| L-9 | Q-2 | 0 | H | H | - | H | H | - | - | H | $OCH_3$ | $OCH_3$ | CH | |
| L-9 | Q-2 | 0 | H | H | - | Cl | H | - | - | H | $CH_3$ | $OCH_3$ | N | |
| L-9 | Q-2 | 0 | H | H | - | F | H | - | - | H | Cl | $OCH_3$ | CH | |
| L-9 | Q-2 | 0 | H | H | - | H | Br | - | - | H | $OCH_3$ | $OCH_3$ | CH | |
| L-9 | Q-2 | 0 | H | H | - | F | F | - | - | H | $OCH_3$ | $OCH_3$ | N | |
| L-9 | Q-2 | 0 | H | H | - | Cl | Cl | - | - | H | $CH_3$ | $OCH_3$ | CH | |
| L-9 | Q-2 | 0 | H | H | - | H | $CH_3$ | - | - | H | $CH_3$ | $OCH_3$ | CH | |
| L-9 | Q-2 | 0 | H | H | - | $CH_3$ | $CH_3$ | - | - | H | $OCH_3$ | $OCH_3$ | CH | |
| L-9 | Q-2 | 0 | H | H | - | H | $CO_2CH_3$ | - | - | H | $CH_3$ | $CH_3$ | CH | |
| L-9 | Q-2 | 0 | H | H | - | H | F | - | - | $CH_3$ | $OCH_3$ | $OCH_3$ | CH | |
| L-9 | Q-2 | 0 | H | $OCH_3$ | - | H | Cl | - | - | H | $OCH_3$ | $CH_3$ | N | |
| L-9 | Q-2 | 0 | $CH_3$ | Cl | - | F | $CH_3$ | - | - | $CH_3$ | $CH_3$ | $OCH_3$ | CH | |

Table II (continued)

| L | $Q_1$ | n | R | $R_1$ | $R_3$ | $R_7$ | $R_8$ | $R_9$ | $R_{10}$ | $R_{11}$ | X | Y | Z | m.p. (°C) |
|---|---|---|---|---|---|---|---|---|---|---|---|---|---|---|
| L-9 | Q-2 | 1 | H | H | - | H | Cl | - | - | H | $CH_3$ | $OCH_3$ | CH | |
| L-9 | Q-2 | 1 | H | H | - | Cl | Cl | - | - | H | $OCH_3$ | $OCH_3$ | N | |
| L-9 | Q-2 | 1 | H | H | - | F | $CH_3$ | - | - | H | Cl | $OCH_3$ | CH | |
| L-9 | Q-3 | 0 | H | H | $CH_3$ | H | H | - | - | H | $CH_3$ | $CH_3$ | CH | |
| L-9 | Q-3 | 0 | H | H | $CH_3$ | H | Cl | - | - | H | $CH_3$ | $OCH_3$ | CH | |
| L-9 | Q-3 | 0 | H | H | $CH_3$ | Cl | Cl | - | - | H | $OCH_3$ | $OCH_3$ | CH | |
| L-9 | Q-3 | 0 | H | H | $CH_3$ | F | F | - | - | H | $CH_3$ | $CH_3$ | N | |
| L-9 | Q-3 | 0 | H | H | $CH_3$ | H | Br | - | - | H | $CH_3$ | $OCH_3$ | N | |
| L-9 | Q-3 | 0 | H | H | $CH_3$ | $CH_3$ | H | - | - | H | $OCH_3$ | $OCH_3$ | N | |
| L-9 | Q-3 | 0 | H | H | $CH_3$ | H | $OCH_3$ | - | - | H | Cl | $OCH_3$ | CH | |
| L-9 | Q-3 | 0 | H | H | $CH_3$ | H | F | - | - | H | $CH_3$ | $OCH_3$ | CH | |
| L-9 | Q-3 | 0 | H | H | $CH_3$ | Cl | Br | - | - | H | $OCH_3$ | $OCH_3$ | CH | |
| L-9 | Q-3 | 0 | H | H | $CH_3$ | Cl | $CH_3$ | - | - | H | $CH_3$ | $OCH_3$ | N | |
| L-9 | Q-3 | 0 | H | H | $CH_3$ | F | $CH_3$ | - | - | $CH_3$ | $OCH_3$ | $OCH_3$ | CH | |
| L-9 | Q-3 | 0 | H | H | $CH_3$ | H | Cl | - | - | H | $CH_3$ | $CH_3$ | N | |
| L-9 | Q-3 | 0 | H | H | $CH_3$ | H | $CH_3$ | - | - | H | $OCH_3$ | $OCH_3$ | CH | |
| L-9 | Q-3 | 0 | H | $CH_3$ | $CH_3$ | H | H | - | - | H | Cl | $OCH_3$ | CH | |
| L-9 | Q-3 | 0 | $CH_3$ | H | $CH_3$ | H | H | - | - | H | $CH_3$ | $OCH_3$ | N | |
| L-9 | Q-3 | 1 | H | H | $CH_3$ | F | F | - | - | H | $OCH_3$ | $OCH_3$ | CH | |
| L-9 | Q-3 | 1 | H | H | $CH_3$ | H | H | - | - | H | $OCH_3$ | $OCH_3$ | CH | |
| L-9 | Q-4 | 0 | H | H | - | H | H | - | - | - | $CH_3$ | $CH_3$ | CH | |
| L-9 | Q-4 | 0 | H | H | - | H | H | - | - | - | $OCH_3$ | $CH_3$ | CH | |
| L-9 | Q-4 | 0 | H | H | - | H | H | - | - | - | $OCH_3$ | $OCH_3$ | CH | |
| L-9 | Q-4 | 0 | H | H | - | Cl | H | - | - | - | $CH_3$ | $OCH_3$ | N | |
| L-9 | Q-4 | 0 | H | H | - | F | H | - | - | - | Cl | $OCH_3$ | CH | |
| L-9 | Q-4 | 0 | H | H | - | H | Br | - | - | - | $OCH_3$ | $OCH_3$ | CH | |
| L-9 | Q-4 | 0 | H | H | - | F | F | - | - | - | $OCH_3$ | $OCH_3$ | N | |
| L-9 | Q-4 | 0 | H | H | - | Cl | Cl | - | - | - | $CH_3$ | $OCH_3$ | CH | |
| L-9 | Q-4 | 0 | H | H | - | H | $CH_3$ | - | - | - | $CH_3$ | $OCH_3$ | CH | |
| L-9 | Q-4 | 0 | H | H | - | $CH_3$ | $CH_3$ | - | - | - | $OCH_3$ | $OCH_3$ | CH | |
| L-9 | Q-4 | 0 | H | H | - | H | $CO_2CH_3$ | - | - | - | $CH_3$ | $CH_3$ | CH | |
| L-9 | Q-4 | 0 | H | H | - | H | F | - | - | - | $OCH_3$ | $OCH_3$ | CH | |
| L-9 | Q-4 | 0 | H | $OCH_3$ | - | H | Cl | - | - | - | $OCH_3$ | $CH_3$ | N | |

Table II (continued)

| L | $Q_1$ | n | R | $R_1$ | $R_2$ | $R_7$ | $R_8$ | $R_9$ | $R_{10}$ | $R_{11}$ | X | Y | Z | m.p. (°C) |
|---|---|---|---|---|---|---|---|---|---|---|---|---|---|---|
| L-9 | Q-4 | 0 | $CH_3$ | Cl | - | F | $CH_3$ | - | - | - | $CH_3$ | $OCH_3$ | CH | |
| L-9 | Q-4 | 1 | H | H | - | H | Cl | - | - | - | $CH_3$ | $OCH_3$ | CH | |
| L-9 | Q-4 | 1 | H | H | - | Cl | Cl | - | - | - | $OCH_3$ | $OCH_3$ | N | |
| L-9 | Q-4 | 1 | H | H | - | F | $CH_3$ | - | - | - | Cl | $OCH_3$ | CH | |
| L-9 | Q-5 | 0 | H | H | - | H | H | - | - | - | $CH_3$ | $CH_3$ | CH | |
| L-9 | Q-5 | 0 | H | H | - | H | Cl | - | - | - | $CH_3$ | $OCH_3$ | CH | |
| L-9 | Q-5 | 0 | H | H | - | Cl | Cl | - | - | - | $OCH_3$ | $OCH_3$ | CH | |
| L-9 | Q-5 | 0 | H | H | - | F | F | - | - | - | $CH_3$ | $CH_3$ | N | |
| L-9 | Q-5 | 0 | H | H | - | H | Br | - | - | - | $CH_3$ | $OCH_3$ | N | |
| L-9 | Q-5 | 0 | H | H | - | $CH_3$ | H | - | - | - | $OCH_3$ | $OCH_3$ | N | |
| L-9 | Q-5 | 0 | H | H | - | H | $OCH_3$ | - | - | - | Cl | $OCH_3$ | CH | |
| L-9 | Q-5 | 0 | H | H | - | H | F | - | - | - | $CH_3$ | $OCH_3$ | CH | |
| L-9 | Q-5 | 0 | H | H | - | Cl | Br | - | - | - | $OCH_3$ | $OCH_3$ | CH | |
| L-9 | Q-5 | 0 | H | H | - | Cl | $CH_3$ | - | - | - | $CH_3$ | $OCH_3$ | N | |
| L-9 | Q-5 | 0 | H | H | - | F | $CH_3$ | - | - | - | $OCH_3$ | $OCH_3$ | CH | |
| L-9 | Q-5 | 0 | H | H | - | H | Cl | - | - | - | $CH_3$ | $CH_3$ | N | |
| L-9 | Q-5 | 0 | H | H | - | H | $CH_3$ | - | - | - | $OCH_3$ | $OCH_3$ | CH | |
| L-9 | Q-5 | 0 | H | $CH_3$ | - | H | H | - | - | - | Cl | $OCH_3$ | CH | |
| L-9 | Q-5 | 0 | $CH_3$ | H | - | H | H | - | - | - | $CH_3$ | $OCH_3$ | N | |
| L-9 | Q-5 | 1 | H | H | - | F | F | - | - | - | $OCH_3$ | $OCH_3$ | CH | |
| L-9 | Q-5 | 1 | H | H | - | H | H | - | - | - | $OCH_3$ | $OCH_3$ | CH | |
| L-9 | Q-6 | 0 | H | H | - | H | H | - | - | - | $CH_3$ | $CH_3$ | CH | |
| L-9 | Q-6 | 0 | H | H | - | H | H | - | - | - | $OCH_3$ | $CH_3$ | CH | |
| L-9 | Q-6 | 0 | H | H | - | H | H | - | - | - | $OCH_3$ | $OCH_3$ | CH | |
| L-9 | Q-6 | 0 | H | H | - | Cl | H | - | - | - | $CH_3$ | $OCH_3$ | N | |
| L-9 | Q-6 | 0 | H | H | - | F | H | - | - | - | Cl | $OCH_3$ | CH | |
| L-9 | Q-6 | 0 | H | H | - | H | Br | - | - | - | $OCH_3$ | $OCH_3$ | CH | |
| L-9 | Q-6 | 0 | H | H | - | F | F | - | - | - | $OCH_3$ | $OCH_3$ | N | |
| L-9 | Q-6 | 0 | H | H | - | Cl | Cl | - | - | - | $CH_3$ | $OCH_3$ | CH | |
| L-9 | Q-6 | 0 | H | H | - | H | $CH_3$ | - | - | - | $CH_3$ | $OCH_3$ | CH | |
| L-9 | Q-6 | 0 | H | H | - | $CH_3$ | $CH_3$ | - | - | - | $OCH_3$ | $OCH_3$ | CH | |
| L-9 | Q-6 | 0 | H | H | - | H | $CO_2CH_3$ | - | - | - | $CH_3$ | $CH_3$ | CH | |
| L-9 | Q-6 | 0 | H | H | - | H | F | - | - | - | $OCH_3$ | $OCH_3$ | CH | |

Table II (continued)

| L | $Q_1$ | n | R | $R_1$ | $R_2$ | $R_7$ | $R_8$ | $R_9$ | $R_{10}$ | $R_{11}$ | X | Y | Z | m.p. (°C) |
|---|---|---|---|---|---|---|---|---|---|---|---|---|---|---|
| L-9 | Q-6 | 0 | H | $OCH_3$ | - | H | Cl | - | - | - | $OCH_3$ | $CH_3$ | N | |
| L-9 | Q-6 | 0 | $CH_3$ | Cl | - | F | $CH_3$ | - | - | - | $CH_3$ | $OCH_3$ | CH | |
| L-9 | Q-6 | 1 | H | H | - | H | Cl | - | - | - | $CH_3$ | $OCH_3$ | CH | |
| L-9 | Q-6 | 1 | H | H | - | Cl | Cl | - | - | - | $OCH_3$ | $OCH_3$ | N | |
| L-9 | Q-6 | 1 | H | H | - | F | $CH_3$ | - | - | - | Cl | $OCH_3$ | CH | |
| L-9 | Q-7 | 0 | H | H | - | H | H | - | - | - | $CH_3$ | $CH_3$ | CH | |
| L-9 | Q-7 | 0 | H | H | - | H | Cl | - | - | - | $CH_3$ | $OCH_3$ | CH | |
| L-9 | Q-7 | 0 | H | H | - | Cl | Cl | - | - | - | $OCH_3$ | $OCH_3$ | CH | |
| L-9 | Q-7 | 0 | H | H | - | F | F | - | - | - | $CH_3$ | $CH_3$ | N | |
| L-9 | Q-7 | 0 | H | H | - | H | Br | - | - | - | $CH_3$ | $OCH_3$ | N | |
| L-9 | Q-7 | 0 | H | H | - | $CH_3$ | H | - | - | - | $OCH_3$ | $OCH_3$ | N | |
| L-9 | Q-7 | 0 | H | H | - | H | $OCH_3$ | - | - | - | Cl | $OCH_3$ | CH | |
| L-9 | Q-7 | 0 | H | H | - | H | F | - | - | - | $CH_3$ | $OCH_3$ | CH | |
| L-9 | Q-7 | 0 | H | H | - | Cl | Br | - | - | - | $OCH_3$ | $OCH_3$ | CH | |
| L-9 | Q-7 | 0 | H | H | - | Cl | $CH_3$ | - | - | - | $CH_3$ | $OCH_3$ | N | |
| L-9 | Q-7 | 0 | H | H | - | F | $CH_3$ | - | - | - | $OCH_3$ | $OCH_3$ | CH | |
| L-9 | Q-7 | 0 | H | H | - | H | Cl | - | - | - | $CH_3$ | $CH_3$ | N | |
| L-9 | Q-7 | 0 | H | H | - | H | $CH_3$ | - | - | - | $OCH_3$ | $OCH_3$ | CH | |
| L-9 | Q-7 | 0 | H | $CH_3$ | - | H | H | - | - | - | Cl | $OCH_3$ | CH | |
| L-9 | Q-7 | 0 | $CH_3$ | H | - | H | H | - | - | - | $CH_3$ | $OCH_3$ | N | |
| L-9 | Q-7 | 1 | H | H | - | F | F | - | - | - | $OCH_3$ | $OCH_3$ | CH | |
| L-9 | Q-7 | 1 | H | H | - | H | H | - | - | - | $OCH_3$ | $OCH_3$ | CH | |
| L-9 | Q-8 | 0 | H | H | - | H | H | - | - | - | $CH_3$ | $CH_3$ | CH | |
| L-9 | Q-8 | 0 | H | H | - | H | H | - | - | - | $OCH_3$ | $CH_3$ | CH | |
| L-9 | Q-8 | 0 | H | H | - | H | H | - | - | - | $OCH_3$ | $OCH_3$ | CH | |
| L-9 | Q-8 | 0 | H | H | - | Cl | H | - | - | - | $CH_3$ | $OCH_3$ | N | |
| L-9 | Q-8 | 0 | H | H | - | F | H | - | - | - | Cl | $OCH_3$ | CH | |
| L-9 | Q-8 | 0 | H | H | - | H | Br | - | - | - | $OCH_3$ | $OCH_3$ | CH | |
| L-9 | Q-8 | 0 | H | H | - | F | F | - | - | - | $OCH_3$ | $OCH_3$ | N | |
| L-9 | Q-8 | 0 | H | H | - | Cl | Cl | - | - | - | $CH_3$ | $OCH_3$ | CH | |
| L-9 | Q-8 | 0 | H | H | - | H | $CH_3$ | - | - | - | $CH_3$ | $OCH_3$ | CH | |
| L-9 | Q-8 | 0 | H | H | - | $CH_3$ | $CH_3$ | - | - | - | $OCH_3$ | $OCH_3$ | CH | |

Table II (continued)

| L | $Q_1$ | n | R | $R_1$ | $R_3$ | $R_7$ | $R_8$ | $R_9$ | $R_{10}$ | $R_{11}$ | X | Y | Z | m.p. (°C) |
|---|---|---|---|---|---|---|---|---|---|---|---|---|---|---|
| L-9 | Q-8 | 0 | H | H | - | H | $CO_2CH_3$ | - | - | - | $CH_3$ | $CH_3$ | CH | |
| L-9 | Q-8 | 0 | H | H | - | H | F | - | - | - | $OCH_3$ | $OCH_3$ | CH | |
| L-9 | Q-8 | 0 | H | $OCH_3$ | - | H | Cl | - | - | - | $OCH_3$ | $CH_3$ | N | |
| L-9 | Q-8 | 0 | $CH_3$ | Cl | - | F | $CH_3$ | - | - | - | $CH_3$ | $OCH_3$ | CH | |
| L-9 | Q-8 | 1 | H | H | - | H | Cl | - | - | - | $CH_3$ | $OCH_3$ | CH | |
| L-9 | Q-8 | 1 | H | H | - | Cl | Cl | - | - | - | $OCH_3$ | $OCH_3$ | N | |
| L-9 | Q-8 | 1 | H | H | - | F | $CH_3$ | - | - | - | Cl | $OCH_3$ | CH | |
| L-9 | Q-9 | 0 | H | H | $CH_3$ | H | H | - | - | - | $CH_3$ | $CH_3$ | CH | |
| L-9 | Q-9 | 0 | H | H | $CH_3$ | H | Cl | - | - | - | $CH_3$ | $OCH_3$ | CH | |
| L-9 | Q-9 | 0 | H | H | $CH_3$ | Cl | Cl | - | - | - | $OCH_3$ | $OCH_3$ | CH | |
| L-9 | Q-9 | 0 | H | H | $CH_3$ | F | F | - | - | - | $CH_3$ | $CH_3$ | N | |
| L-9 | Q-9 | 0 | H | H | $CH_3$ | H | Br | - | - | - | $CH_3$ | $OCH_3$ | N | |
| L-9 | Q-9 | 0 | H | H | $CH_3$ | $CH_3$ | H | - | - | - | $OCH_3$ | $OCH_3$ | N | |
| L-9 | Q-9 | 0 | H | H | $CH_3$ | H | $OCH_3$ | - | - | - | Cl | $OCH_3$ | CH | |
| L-9 | Q-9 | 0 | H | H | $CH_3$ | H | F | - | - | - | $CH_3$ | $OCH_3$ | CH | |
| L-9 | Q-9 | 0 | H | H | $CH_3$ | Cl | Br | - | - | - | $OCH_3$ | $OCH_3$ | CH | |
| L-9 | Q-9 | 0 | H | H | $CH_3$ | Cl | $CH_3$ | - | - | - | $CH_3$ | $OCH_3$ | N | |
| L-9 | Q-9 | 0 | H | H | $CH_3$ | F | $CH_3$ | - | - | - | $OCH_3$ | $OCH_3$ | CH | |
| L-9 | Q-9 | 0 | H | H | $CH_3$ | H | Cl | - | - | - | $CH_3$ | $CH_3$ | N | |
| L-9 | Q-9 | 0 | H | H | $CH_3$ | H | $CH_3$ | - | - | - | $OCH_3$ | $OCH_3$ | CH | |
| L-9 | Q-9 | 0 | H | $CH_3$ | $CH_3$ | H | H | - | - | - | Cl | $OCH_3$ | CH | |
| L-9 | Q-9 | 0 | $CH_3$ | H | $CH_3$ | H | H | - | - | - | $CH_3$ | $OCH_3$ | N | |
| L-9 | Q-9 | 1 | H | H | $CH_3$ | F | F | - | - | - | $OCH_3$ | $OCH_3$ | CH | |
| L-9 | Q-9 | 1 | H | H | $CH_3$ | H | H | - | - | - | $OCH_3$ | $OCH_3$ | CH | |
| L-9 | Q-10 | 0 | H | H | $CH_3$ | H | H | - | - | - | $CH_3$ | $CH_3$ | CH | |
| L-9 | Q-10 | 0 | H | H | $CH_3$ | H | H | - | - | - | $OCH_3$ | $CH_3$ | CH | |
| L-9 | Q-10 | 0 | H | H | $CH_3$ | H | H | - | - | - | $OCH_3$ | $OCH_3$ | CH | |
| L-9 | Q-10 | 0 | H | H | $CH_3$ | Cl | H | - | - | - | $CH_3$ | $OCH_3$ | N | |
| L-9 | Q-10 | 0 | H | H | $CH_3$ | F | H | - | - | - | Cl | $OCH_3$ | CH | |
| L-9 | Q-10 | 0 | H | H | $CH_3$ | H | Br | - | - | - | $OCH_3$ | $OCH_3$ | CH | |
| L-9 | Q-10 | 0 | H | H | $CH_3$ | F | F | - | - | - | $OCH_3$ | $OCH_3$ | N | |
| L-9 | Q-10 | 0 | H | H | $CH_3$ | Cl | Cl | - | - | - | $CH_3$ | $OCH_3$ | CH | |

Table II (continued)

| L | $Q_1$ | n | R | $R_1$ | $R_2/R_3$ | $R_7$ | $R_8$ | $R_9$ | $R_{10}$ | $R_{11}$ | X | Y | Z | m.p. (°C) |
|---|---|---|---|---|---|---|---|---|---|---|---|---|---|---|
| L-9 | Q-10 | 0 | H | H | $CH_3$ | H | $CH_3$ | - | - | - | $CH_3$ | $OCH_3$ | CH | |
| L-9 | Q-10 | 0 | H | H | $CH_3$ | $CH_3$ | $CH_3$ | - | - | - | $OCH_3$ | $OCH_3$ | CH | |
| L-9 | Q-10 | 0 | H | H | $CH_3$ | H | $CO_2CH_3$ | - | - | - | $CH_3$ | $CH_3$ | CH | |
| L-9 | Q-10 | 0 | H | H | $CH_3$ | H | F | - | - | - | $OCH_3$ | $OCH_3$ | CH | |
| L-9 | Q-10 | 0 | H | $OCH_3$ | $CH_3$ | H | Cl | - | - | - | $OCH_3$ | $CH_3$ | N | |
| L-9 | Q-10 | 0 | $CH_3$ | Cl | $CH_3$ | F | $CH_3$ | - | - | - | $CH_3$ | $OCH_3$ | CH | |
| L-9 | Q-10 | 1 | H | H | $CH_3$ | H | Cl | - | - | - | $CH_3$ | $OCH_3$ | CH | |
| L-9 | Q-10 | 1 | H | H | $CH_3$ | Cl | Cl | - | - | - | $OCH_3$ | $OCH_3$ | N | |
| L-9 | Q-10 | 1 | H | H | $CH_3$ | F | $CH_3$ | - | - | - | Cl | $OCH_3$ | CH | |
| L-10 | Q-1 | 0 | H | H | $CH_3$ | H | H | H | H | - | $CH_3$ | $CH_3$ | CH | |
| L-10 | Q-1 | 0 | H | H | $CH_3$ | H | Cl | H | H | - | $CH_3$ | $OCH_3$ | CH | |
| L-10 | Q-1 | 0 | H | H | $CH_3$ | Cl | Cl | H | H | - | $OCH_3$ | $OCH_3$ | CH | |
| L-10 | Q-1 | 0 | H | H | $CH_3$ | F | F | H | H | - | $CH_3$ | $CH_3$ | N | |
| L-10 | Q-1 | 0 | H | H | $CH_3$ | H | Br | H | H | - | $CH_3$ | $OCH_3$ | N | |
| L-10 | Q-1 | 0 | H | H | $CH_3$ | $CH_3$ | H | H | H | - | $OCH_3$ | $OCH_3$ | N | |
| L-10 | Q-1 | 0 | H | H | $CH_3$ | H | $OCH_3$ | H | H | - | Cl | $OCH_3$ | CH | |
| L-10 | Q-1 | 0 | H | H | $CH_3$ | H | F | H | H | - | $CH_3$ | $OCH_3$ | CH | |
| L-10 | Q-1 | 0 | H | H | $CH_3$ | Cl | Br | H | H | - | $OCH_3$ | $OCH_3$ | CH | |
| L-10 | Q-1 | 0 | H | H | $CH_3$ | Cl | $CH_3$ | H | H | - | $CH_3$ | $CH_3$ | N | |
| L-10 | Q-1 | 0 | H | H | $CH_3$ | F | $CH_3$ | H | H | - | $OCH_3$ | $OCH_3$ | CH | |
| L-10 | Q-1 | 0 | H | H | $CH_3$ | H | Cl | Cl | H | - | $CH_3$ | $CH_3$ | N | |
| L-10 | Q-1 | 0 | H | H | $CH_3$ | H | $CH_3$ | F | F | - | $OCH_3$ | $OCH_3$ | CH | |
| L-10 | Q-1 | 0 | H | $CH_3$ | $CH_3$ | H | H | H | H | - | Cl | $OCH_3$ | CH | |
| L-10 | Q-1 | 0 | $CH_3$ | H | $CH_3$ | H | H | H | H | - | $CH_3$ | $OCH_3$ | N | |
| L-10 | Q-1 | 1 | H | H | $CH_3$ | F | F | H | H | - | $OCH_3$ | $OCH_3$ | CH | |
| L-10 | Q-1 | 1 | H | H | $CH_3$ | H | H | H | H | - | $OCH_3$ | $OCH_3$ | CH | |
| L-10 | Q-2 | 0 | H | H | $CH_3$ | H | H | - | - | H | $CH_3$ | $CH_3$ | CH | |
| L-10 | Q-2 | 0 | H | H | $CH_3$ | H | H | - | - | H | $OCH_3$ | $CH_3$ | CH | |
| L-10 | Q-2 | 0 | H | H | $CH_3$ | H | H | - | - | H | $OCH_3$ | $OCH_3$ | CH | |
| L-10 | Q-2 | 0 | H | H | $CH_3$ | Cl | H | - | - | H | $CH_3$ | $OCH_3$ | N | |
| L-10 | Q-2 | 0 | H | H | $CH_3$ | F | H | - | - | H | Cl | $OCH_3$ | CH | |
| L-10 | Q-2 | 0 | H | H | $CH_3$ | H | Br | - | - | H | $OCH_3$ | $OCH_3$ | CH | |

Table II (continued)

| L | $Q_1$ | n | R | $R_1$ | $R_2$ | $R_7$ | $R_8$ | $R_3$ | $R_{10}$ | $R_{11}$ | X | Y | Z | m.p. (°C) |
|---|---|---|---|---|---|---|---|---|---|---|---|---|---|---|
| L-10 | Q-2 | 0 | H | H | $CH_3$ | F | F | - | - | H | $OCH_3$ | $OCH_3$ | N | |
| L-10 | Q-2 | 0 | H | H | $CH_3$ | Cl | Cl | - | - | H | $CH_3$ | $OCH_3$ | CH | |
| L-10 | Q-2 | 0 | H | H | $CH_3$ | H | $CH_3$ | - | - | H | $CH_3$ | $OCH_3$ | CH | |
| L-10 | Q-2 | 0 | H | H | $CH_3$ | $CH_3$ | $CH_3$ | - | - | H | $OCH_3$ | $OCH_3$ | CH | |
| L-10 | Q-2 | 0 | H | H | $CH_3$ | H | $CO_2CH_3$ | - | - | H | $CH_3$ | $CH_3$ | CH | |
| L-10 | Q-2 | 0 | H | H | $CH_3$ | H | F | - | - | $CH_3$ | $OCH_3$ | $OCH_3$ | CH | |
| L-10 | Q-2 | 0 | H | $OCH_3$ | $CH_3$ | H | Cl | - | - | H | $OCH_3$ | $CH_3$ | N | |
| L-10 | Q-2 | 0 | $CH_3$ | Cl | $CH_3$ | F | $CH_3$ | - | - | $CH_3$ | $CH_3$ | $OCH_3$ | CH | |
| L-10 | Q-2 | 1 | H | H | $CH_3$ | H | Cl | - | - | H | $CH_3$ | $OCH_3$ | CH | |
| L-10 | Q-2 | 1 | H | H | $CH_3$ | Cl | Cl | - | - | H | $OCH_3$ | $OCH_3$ | N | |
| L-10 | Q-2 | 1 | H | H | $CH_3$ | F | $CH_3$ | - | - | H | Cl | $OCH_3$ | CH | |
| L-10 | Q-3 | 0 | H | H | $CH_3$ | H | H | $CH_3$ | - | H | $CH_3$ | $CH_3$ | CH | |
| L-10 | Q-3 | 0 | H | H | $CH_3$ | H | Cl | $CH_3$ | - | H | $CH_3$ | $OCH_3$ | CH | |
| L-10 | Q-3 | 0 | H | H | $CH_3$ | Cl | Cl | $CH_3$ | - | H | $OCH_3$ | $OCH_3$ | CH | |
| L-10 | Q-3 | 0 | H | H | $CH_3$ | F | F | $CH_3$ | - | H | $CH_3$ | $CH_3$ | N | |
| L-10 | Q-3 | 0 | H | H | $CH_3$ | H | Br | $CH_3$ | - | H | $CH_3$ | $OCH_3$ | N | |
| L-10 | Q-3 | 0 | H | H | $CH_3$ | $CH_3$ | H | $CH_3$ | - | H | $OCH_3$ | $OCH_3$ | N | |
| L-10 | Q-3 | 0 | H | H | $CH_3$ | H | $OCH_3$ | $CH_3$ | - | H | Cl | $OCH_3$ | CH | |
| L-10 | Q-3 | 0 | H | H | $CH_3$ | H | F | $CH_3$ | - | H | $CH_3$ | $OCH_3$ | CH | |
| L-10 | Q-3 | 0 | H | H | $CH_3$ | Cl | Br | $CH_3$ | - | H | $OCH_3$ | $OCH_3$ | CH | |
| L-10 | Q-3 | 0 | H | H | $CH_3$ | Cl | $CH_3$ | $CH_3$ | - | H | $CH_3$ | $OCH_3$ | N | |
| L-10 | Q-3 | 0 | H | H | $CH_3$ | F | $CH_3$ | $CH_3$ | - | $CH_3$ | $OCH_3$ | $OCH_3$ | CH | |
| L-10 | Q-3 | 0 | H | H | $CH_3$ | H | Cl | $CH_2CH_3$ | - | H | $CH_3$ | $CH_3$ | N | |
| L-10 | Q-3 | 0 | H | H | $CH_3$ | H | $CH_3$ | $CH_3$ | - | H | $OCH_3$ | $OCH_3$ | CH | |
| L-10 | Q-3 | 0 | H | $CH_3$ | $CH_3$ | H | H | $CH_3$ | - | H | Cl | $OCH_3$ | CH | |
| L-10 | Q-3 | 0 | $CH_3$ | H | $CH_3$ | H | H | $CH_3$ | - | H | $CH_3$ | $OCH_3$ | N | |
| L-10 | Q-3 | 1 | H | H | $CH_3$ | F | F | $CH_3$ | - | H | $OCH_3$ | $OCH_3$ | CH | |
| L-10 | Q-3 | 1 | H | H | $CH_3$ | H | H | $CH_3$ | - | H | $OCH_3$ | $OCH_3$ | CH | |
| L-10 | Q-4 | 0 | H | H | $CH_3$ | H | H | - | - | - | $CH_3$ | $CH_3$ | CH | |
| L-10 | Q-4 | 0 | H | H | $CH_3$ | H | H | - | - | - | $OCH_3$ | $CH_3$ | CH | |
| L-10 | Q-4 | 0 | H | H | $CH_3$ | H | H | - | - | - | $OCH_3$ | $OCH_3$ | CH | |
| L-10 | Q-4 | 0 | H | H | $CH_3$ | Cl | H | - | - | - | $CH_3$ | $OCH_3$ | N | |

Table II (continued)

| L | $Q_1$ | n | R | $R_1$ | $R_2$ | $R_7$ | $R_8$ | $R_9$ | $R_{10}$ | $R_{11}$ | X | Y | Z | m.p. (°C) |
|---|---|---|---|---|---|---|---|---|---|---|---|---|---|---|
| L-10 | Q-4 | 0 | H | H | $CH_3$ | F | H | - | - | - | Cl | $OCH_3$ | CH | |
| L-10 | Q-4 | 0 | H | H | $CH_3$ | H | Br | - | - | - | $OCH_3$ | $OCH_3$ | CH | |
| L-10 | Q-4 | 0 | H | H | $CH_3$ | F | F | - | - | - | $OCH_3$ | $OCH_3$ | N | |
| L-10 | Q-4 | 0 | H | H | $CH_3$ | Cl | Cl | - | - | - | $CH_3$ | $OCH_3$ | CH | |
| L-10 | Q-4 | 0 | H | H | $CH_3$ | H | $CH_3$ | - | - | - | $CH_3$ | $OCH_3$ | CH | |
| L-10 | Q-4 | 0 | H | H | $CH_3$ | $CH_3$ | $CH_3$ | - | - | - | $OCH_3$ | $OCH_3$ | CH | |
| L-10 | Q-4 | 0 | H | H | $CH_3$ | H | $CO_2CH_3$ | - | - | - | $CH_3$ | $CH_3$ | CH | |
| L-10 | Q-4 | 0 | H | H | $CH_3$ | H | F | - | - | - | $OCH_3$ | $OCH_3$ | CH | |
| L-10 | Q-4 | 0 | H | $OCH_3$ | $CH_3$ | H | Cl | - | - | - | $OCH_3$ | $CH_3$ | N | |
| L-10 | Q-4 | 0 | $CH_3$ | Cl | $CH_3$ | F | $CH_3$ | - | - | - | $CH_3$ | $OCH_3$ | CH | |
| L-10 | Q-4 | 1 | H | H | $CH_3$ | H | Cl | - | - | - | $CH_3$ | $OCH_3$ | CH | |
| L-10 | Q-4 | 1 | H | H | $CH_3$ | Cl | Cl | - | - | - | $OCH_3$ | $OCH_3$ | N | |
| L-10 | Q-4 | 1 | H | H | $CH_3$ | F | $CH_3$ | - | - | - | Cl | $OCH_3$ | CH | |
| L-10 | Q-5 | 0 | H | H | $CH_3$ | H | H | - | - | - | $CH_3$ | $CH_3$ | CH | |
| L-10 | Q-5 | 0 | H | H | $CH_3$ | H | Cl | - | - | - | $CH_3$ | $OCH_3$ | CH | |
| L-10 | Q-5 | 0 | H | H | $CH_3$ | Cl | Cl | - | - | - | $OCH_3$ | $OCH_3$ | CH | |
| L-10 | Q-5 | 0 | H | H | $CH_3$ | F | F | - | - | - | $CH_3$ | $CH_3$ | N | |
| L-10 | Q-5 | 0 | H | H | $CH_3$ | H | Br | - | - | - | $CH_3$ | $OCH_3$ | N | |
| L-10 | Q-5 | 0 | H | H | $CH_3$ | $CH_3$ | H | - | - | - | $OCH_3$ | $OCH_3$ | N | |
| L-10 | Q-5 | 0 | H | H | $CH_3$ | H | $OCH_3$ | - | - | - | Cl | $OCH_3$ | CH | |
| L-10 | Q-5 | 0 | H | H | $CH_3$ | H | F | - | - | - | $CH_3$ | $OCH_3$ | CH | |
| L-10 | Q-5 | 0 | H | H | $CH_3$ | Cl | Br | - | - | - | $OCH_3$ | $OCH_3$ | CH | |
| L-10 | Q-5 | 0 | H | H | $CH_3$ | Cl | $CH_3$ | - | - | - | $CH_3$ | $OCH_3$ | N | |
| L-10 | Q-5 | 0 | H | H | $CH_3$ | F | $CH_3$ | - | - | - | $OCH_3$ | $OCH_3$ | CH | |
| L-10 | Q-5 | 0 | H | H | $CH_3$ | H | Cl | - | - | - | $CH_3$ | $CH_3$ | N | |
| L-10 | Q-5 | 0 | H | H | $CH_3$ | H | $CH_3$ | - | - | - | $OCH_3$ | $OCH_3$ | CH | |
| L-10 | Q-5 | 0 | H | $CH_3$ | $CH_3$ | H | H | - | - | - | Cl | $OCH_3$ | CH | |
| L-10 | Q-5 | 0 | $CH_3$ | H | $CH_3$ | H | H | - | - | - | $CH_3$ | $OCH_3$ | N | |
| L-10 | Q-5 | 1 | H | H | $CH_3$ | F | F | - | - | - | $OCH_3$ | $OCH_3$ | CH | |
| L-10 | Q-5 | 1 | H | H | $CH_3$ | H | H | - | - | - | $OCH_3$ | $OCH_3$ | CH | |
| L-10 | Q-6 | 0 | H | H | $CH_3$ | H | H | - | - | - | $CH_3$ | $CH_3$ | CH | |
| L-10 | Q-6 | 0 | H | H | $CH_3$ | H | H | - | - | - | $OCH_3$ | $CH_3$ | CH | |

Table II (continued)

| L | $Q_1$ | n | R | $R_1$ | $R_2$ | $R_7$ | $R_8$ | $R_9$ | $R_{10}$ | $R_{11}$ | X | Y | Z | m.p. (°C) |
|---|---|---|---|---|---|---|---|---|---|---|---|---|---|---|
| L-10 | Q-6 | 0 | H | H | $CH_3$ | H | H | - | - | - | $OCH_3$ | $OCH_3$ | CH | |
| L-10 | Q-6 | 0 | H | H | $CH_3$ | Cl | H | - | - | - | $CH_3$ | $OCH_3$ | N | |
| L-10 | Q-6 | 0 | H | H | $CH_3$ | F | H | - | - | - | Cl | $OCH_3$ | CH | |
| L-10 | Q-6 | 0 | H | H | $CH_3$ | H | Br | - | - | - | $OCH_3$ | $OCH_3$ | CH | |
| L-10 | Q-6 | 0 | H | H | $CH_3$ | F | F | - | - | - | $OCH_3$ | $OCH_3$ | N | |
| L-10 | Q-6 | 0 | H | H | $CH_3$ | Cl | Cl | - | - | - | $CH_3$ | $OCH_3$ | CH | |
| L-10 | Q-6 | 0 | H | H | $CH_3$ | H | $CH_3$ | - | - | - | $CH_3$ | $OCH_3$ | CH | |
| L-10 | Q-6 | 0 | H | H | $CH_3$ | $CH_3$ | $CH_3$ | - | - | - | $OCH_3$ | $OCH_3$ | CH | |
| L-10 | Q-6 | 0 | H | H | $CH_3$ | H | $CO_2CH_3$ | - | - | - | $CH_3$ | $CH_3$ | CH | |
| L-10 | Q-6 | 0 | H | H | $CH_3$ | H | F | - | - | - | $OCH_3$ | $OCH_3$ | CH | |
| L-10 | Q-6 | 0 | H | $OCH_3$ | $CH_3$ | H | Cl | - | - | - | $OCH_3$ | $CH_3$ | N | |
| L-10 | Q-6 | 0 | $CH_3$ | Cl | $CH_3$ | F | $CH_3$ | - | - | - | $CH_3$ | $OCH_3$ | CH | |
| L-10 | Q-6 | 1 | H | H | $CH_3$ | H | Cl | - | - | - | $CH_3$ | $OCH_3$ | CH | |
| L-10 | Q-6 | 1 | H | H | $CH_3$ | Cl | Cl | - | - | - | $OCH_3$ | $OCH_3$ | N | |
| L-10 | Q-6 | 1 | H | H | $CH_3$ | F | $CH_3$ | - | - | - | Cl | $OCH_3$ | CH | |
| L-10 | Q-7 | 0 | H | H | $CH_3$ | H | H | - | - | - | $CH_3$ | $CH_3$ | CH | |
| L-10 | Q-7 | 0 | H | H | $CH_3$ | H | Cl | - | - | - | $CH_3$ | $OCH_3$ | CH | |
| L-10 | Q-7 | 0 | H | H | $CH_3$ | Cl | Cl | - | - | - | $OCH_3$ | $OCH_3$ | CH | |
| L-10 | Q-7 | 0 | H | H | $CH_3$ | F | F | - | - | - | $CH_3$ | $CH_3$ | N | |
| L-10 | Q-7 | 0 | H | H | $CH_3$ | H | Br | - | - | - | $CH_3$ | $OCH_3$ | N | |
| L-10 | Q-7 | 0 | H | H | $CH_3$ | $CH_3$ | H | - | - | - | $OCH_3$ | $OCH_3$ | N | |
| L-10 | Q-7 | 0 | H | H | $CH_3$ | H | $OCH_3$ | - | - | - | Cl | $OCH_3$ | CH | |
| L-10 | Q-7 | 0 | H | H | $CH_3$ | H | F | - | - | - | $CH_3$ | $OCH_3$ | CH | |
| L-10 | Q-7 | 0 | H | H | $CH_3$ | Cl | Br | - | - | - | $OCH_3$ | $OCH_3$ | CH | |
| L-10 | Q-7 | 0 | H | H | $CH_3$ | Cl | $CH_3$ | - | - | - | $CH_3$ | $OCH_3$ | N | |
| L-10 | Q-7 | 0 | H | H | $CH_3$ | F | $CH_3$ | - | - | - | $OCH_3$ | $OCH_3$ | CH | |
| L-10 | Q-7 | 0 | H | H | $CH_3$ | H | Cl | - | - | - | $CH_3$ | $CH_3$ | N | |
| L-10 | Q-7 | 0 | H | H | $CH_3$ | H | $CH_3$ | - | - | - | $OCH_3$ | $OCH_3$ | CH | |
| L-10 | Q-7 | 0 | H | $CH_3$ | $CH_3$ | H | H | - | - | - | Cl | $OCH_3$ | CH | |
| L-10 | Q-7 | 0 | $CH_3$ | H | $CH_3$ | H | H | - | - | - | $CH_3$ | $OCH_3$ | N | |
| L-10 | Q-7 | 1 | H | H | $CH_3$ | F | F | - | - | - | $OCH_3$ | $OCH_3$ | CH | |
| L-10 | Q-7 | 1 | H | H | $CH_3$ | H | H | - | - | - | $OCH_3$ | $OCH_3$ | CH | |

Table II (continued)

| L | $Q_1$ | n | R | $R_1$ | $R_2$ | $R_7$ | $R_8$ | $R_9$ | $R_3$ | $R_{11}$ | X | Y | Z | m.p. (°C) |
|---|---|---|---|---|---|---|---|---|---|---|---|---|---|---|
| L-10 | Q-8 | 0 | H | H | $CH_3$ | H | H | - | - | - | $CH_3$ | $CH_3$ | CH | |
| L-10 | Q-8 | 0 | H | H | $CH_3$ | H | H | - | - | - | $OCH_3$ | $CH_3$ | CH | |
| L-10 | Q-8 | 0 | H | H | $CH_3$ | H | H | - | - | - | $OCH_3$ | $OCH_3$ | CH | |
| L-10 | Q-8 | 0 | H | H | $CH_3$ | Cl | H | - | - | - | $CH_3$ | $OCH_3$ | N | |
| L-10 | Q-8 | 0 | H | H | $CH_3$ | F | H | - | - | - | Cl | $OCH_3$ | CH | |
| L-10 | Q-8 | 0 | H | H | $CH_3$ | H | Br | - | - | - | $OCH_3$ | $OCH_3$ | CH | |
| L-10 | Q-8 | 0 | H | H | $CH_3$ | F | F | - | - | - | $OCH_3$ | $OCH_3$ | N | |
| L-10 | Q-8 | 0 | H | H | $CH_3$ | Cl | Cl | - | - | - | $CH_3$ | $OCH_3$ | CH | |
| L-10 | Q-8 | 0 | H | H | $CH_3$ | H | $CH_3$ | - | - | - | $CH_3$ | $OCH_3$ | CH | |
| L-10 | Q-8 | 0 | H | H | $CH_3$ | $CH_3$ | $CH_3$ | - | - | - | $OCH_3$ | $OCH_3$ | CH | |
| L-10 | Q-8 | 0 | H | H | $CH_3$ | $CH_3$ | $CO_2CH_3$ | - | - | - | $CH_3$ | $CH_3$ | CH | |
| L-10 | Q-8 | 0 | H | H | $CH_3$ | H | F | - | - | - | $OCH_3$ | $OCH_3$ | CH | |
| L-10 | Q-8 | 0 | H | $OCH_3$ | $CH_3$ | H | Cl | - | - | - | $OCH_3$ | $CH_3$ | N | |
| L-10 | Q-8 | 0 | $CH_3$ | Cl | $CH_3$ | F | $CH_3$ | - | - | - | $CH_3$ | $OCH_3$ | CH | |
| L-10 | Q-8 | 1 | H | H | $CH_3$ | H | Cl | - | - | - | $CH_3$ | $OCH_3$ | CH | |
| L-10 | Q-8 | 1 | H | H | $CH_3$ | Cl | Cl | - | - | - | $OCH_3$ | $OCH_3$ | N | |
| L-10 | Q-8 | 1 | H | H | $CH_3$ | F | $CH_3$ | - | - | - | Cl | $OCH_3$ | CH | |
| L-10 | Q-9 | 0 | H | H | $CH_3$ | H | H | - | $CH_3$ | - | $CH_3$ | $CH_3$ | CH | |
| L-10 | Q-9 | 0 | H | H | $CH_3$ | H | Cl | - | $CH_3$ | - | $CH_3$ | $OCH_3$ | CH | |
| L-10 | Q-9 | 0 | H | H | $CH_3$ | Cl | Cl | - | $CH_3$ | - | $OCH_3$ | $OCH_3$ | CH | |
| L-10 | Q-9 | 0 | H | H | $CH_3$ | F | F | - | $CH_3$ | - | $CH_3$ | $CH_3$ | N | |
| L-10 | Q-9 | 0 | H | H | $CH_3$ | H | Br | - | $CH_3$ | - | $CH_3$ | $OCH_3$ | N | |
| L-10 | Q-9 | 0 | H | H | $CH_3$ | $CH_3$ | H | - | $CH_3$ | - | $OCH_3$ | $OCH_3$ | N | |
| L-10 | Q-9 | 0 | H | H | $CH_3$ | H | $OCH_3$ | - | $CH_3$ | - | Cl | $OCH_3$ | CH | |
| L-10 | Q-9 | 0 | H | H | $CH_3$ | H | F | - | $CH_3$ | - | $CH_3$ | $OCH_3$ | CH | |
| L-10 | Q-9 | 0 | H | H | $CH_3$ | Cl | Br | - | $CH_3$ | - | $OCH_3$ | $OCH_3$ | CH | |
| L-10 | Q-9 | 0 | H | H | $CH_3$ | Cl | $CH_3$ | - | $CH_3$ | - | $CH_3$ | $OCH_3$ | N | |
| L-10 | Q-9 | 0 | H | H | $CH_3$ | F | $CH_3$ | - | $CH_3$ | - | $OCH_3$ | $OCH_3$ | CH | |
| L-10 | Q-9 | 0 | H | H | $CH_3$ | H | Cl | - | $CH_3$ | - | $CH_3$ | $CH_3$ | N | |
| L-10 | Q-9 | 0 | H | H | $CH_3$ | H | $CH_3$ | - | $CH_2CH_3$ | - | $OCH_3$ | $OCH_3$ | CH | |
| L-10 | Q-9 | 0 | H | $CH_3$ | $CH_3$ | H | H | - | $CH_2CH_3$ | - | Cl | $OCH_3$ | CH | |
| L-10 | Q-9 | 0 | $CH_3$ | H | $CH_3$ | H | H | - | $CH_2CH_2CH_3$ | - | $CH_3$ | $OCH_3$ | N | |

Table II (continued)

| L | $Q_1$ | n | R | $R_1$ | $R_2$ | $R_7$ | $R_8$ | $R_9$ | $R_3$ | $R_{10}$ | X | Y | Z | m.p. (°C) |
|---|---|---|---|---|---|---|---|---|---|---|---|---|---|---|
| L-10 | Q-9 | 1 | H | H | $CH_3$ | F | F | - | $CH_3$ | - | $OCH_3$ | $OCH_3$ | CH | |
| L-10 | Q-9 | 1 | H | H | $CH_3$ | H | H | - | $CH_2CH_3$ | - | $OCH_3$ | $OCH_3$ | CH | |
| L-10 | Q-10 | 0 | H | H | $CH_3$ | H | H | - | $CH_3$ | - | $CH_3$ | $CH_3$ | CH | |
| L-10 | Q-10 | 0 | H | H | $CH_3$ | H | H | - | $CH_3$ | - | $OCH_3$ | $CH_3$ | CH | |
| L-10 | Q-10 | 0 | H | H | $CH_3$ | H | H | - | $CH_3$ | - | $OCH_3$ | $OCH_3$ | CH | |
| L-10 | Q-10 | 0 | H | H | $CH_3$ | Cl | H | - | $CH_3$ | - | $CH_3$ | $OCH_3$ | N | |
| L-10 | Q-10 | 0 | H | H | $CH_3$ | F | H | - | $CH_3$ | - | Cl | $OCH_3$ | CH | |
| L-10 | Q-10 | 0 | H | H | $CH_3$ | H | Br | - | $CH_3$ | - | $OCH_3$ | $OCH_3$ | CH | |
| L-10 | Q-10 | 0 | H | H | $CH_3$ | F | F | - | $CH_3$ | - | $OCH_3$ | $OCH_3$ | N | |
| L-10 | Q-10 | 0 | H | H | $CH_3$ | Cl | Cl | - | $CH_3$ | - | $CH_3$ | $OCH_3$ | CH | |
| L-10 | Q-10 | 0 | H | H | $CH_3$ | H | $CH_3$ | - | $CH_3$ | - | $CH_3$ | $OCH_3$ | CH | |
| L-10 | Q-10 | 0 | H | H | $CH_3$ | $CH_3$ | $CH_3$ | - | $CH_3$ | - | $OCH_3$ | $OCH_3$ | CH | |
| L-10 | Q-10 | 0 | H | H | $CH_3$ | H | $CO_2CH_3$ | - | $CH_3$ | - | $CH_3$ | $CH_3$ | CH | |
| L-10 | Q-10 | 0 | H | H | $CH_3$ | H | F | - | $CH_3$ | - | $OCH_3$ | $OCH_3$ | CH | |
| L-10 | Q-10 | 0 | H | $OCH_3$ | $CH_3$ | H | Cl | - | $CH_3$ | - | $OCH_3$ | $CH_3$ | N | |
| L-10 | Q-10 | 0 | $CH_3$ | Cl | $CH_3$ | F | $CH_3$ | - | $CH_3$ | - | $CH_3$ | $OCH_3$ | CH | |
| L-10 | Q-10 | 1 | H | H | $CH_3$ | H | Cl | - | $CH_2CH_3$ | - | $CH_3$ | $OCH_3$ | CH | |
| L-10 | Q-10 | 1 | H | H | $CH_3$ | Cl | Cl | - | $CH_3$ | - | $OCH_3$ | $OCH_3$ | N | |
| L-10 | Q-10 | 1 | H | H | $CH_3$ | F | $CH_3$ | - | $CH_2CH_3$ | - | Cl | $OCH_3$ | CH | |
| L-11 | Q-1 | 0 | H | H | - | H | H | H | - | H | $CH_3$ | $CH_3$ | CH | |
| L-11 | Q-1 | 0 | H | H | - | Cl | Cl | H | - | H | $OCH_3$ | $OCH_3$ | CH | |
| L-11 | Q-1 | 0 | H | H | - | F | F | H | - | H | $CH_3$ | $CH_3$ | N | |
| L-11 | Q-1 | 0 | H | H | - | H | Br | H | - | H | $CH_3$ | $OCH_3$ | N | |
| L-11 | Q-1 | 0 | H | H | - | $CH_3$ | Br | H | - | H | $OCH_3$ | $OCH_3$ | N | |
| L-11 | Q-1 | 0 | H | H | - | H | $OCH_3$ | H | - | H | Cl | $OCH_3$ | CH | |
| L-11 | Q-1 | 0 | H | H | - | H | F | H | - | H | $CH_3$ | $OCH_3$ | CH | |
| L-11 | Q-1 | 0 | H | H | - | Cl | Br | H | - | H | $OCH_3$ | $OCH_3$ | CH | |
| L-11 | Q-1 | 0 | H | H | - | Cl | $CH_3$ | H | - | H | $CH_3$ | $OCH_3$ | N | |
| L-11 | Q-1 | 0 | H | H | - | F | $CH_3$ | H | - | H | $OCH_3$ | $OCH_3$ | CH | |
| L-11 | Q-1 | 0 | H | H | - | H | Cl | Cl | - | H | $CH_3$ | $CH_3$ | N | |
| L-11 | Q-1 | 0 | H | H | - | H | $CH_3$ | F | - | H | $OCH_3$ | $OCH_3$ | CH | |
| L-11 | Q-1 | 0 | H | $CH_3$ | - | H | H | H | - | H | Cl | $OCH_3$ | CH | |

Table II (continued)

| L | $Q_1$ | n | R | $R_1$ | $R_3$ | $R_7$ | $R_8$ | $R_9$ | $R_{10}$ | $R_{11}$ | X | Y | Z | m.p. (°C) |
|---|---|---|---|---|---|---|---|---|---|---|---|---|---|---|
| L-11 | Q-1 | 0 | $CH_3$ | H | - | H | H | H | H | - | $CH_3$ | $OCH_3$ | N | |
| L-11 | Q-1 | 1 | H | H | - | F | F | H | H | - | $OCH_3$ | $OCH_3$ | CH | |
| L-11 | Q-1 | 1 | H | H | - | H | H | H | H | - | $OCH_3$ | $OCH_3$ | CH | |
| L-11 | Q-2 | 0 | H | H | - | H | H | - | - | H | $CH_3$ | $CH_3$ | CH | |
| L-11 | Q-2 | 0 | H | H | - | H | H | - | - | H | $OCH_3$ | $CH_3$ | CH | |
| L-11 | Q-2 | 0 | H | H | - | H | H | - | - | H | $OCH_3$ | $OCH_3$ | CH | |
| L-11 | Q-2 | 0 | H | H | - | Cl | H | - | - | H | $CH_3$ | $OCH_3$ | N | |
| L-11 | Q-2 | 0 | H | H | - | F | H | - | - | H | Cl | $OCH_3$ | CH | |
| L-11 | Q-2 | 0 | H | H | - | H | Br | - | - | H | $OCH_3$ | $OCH_3$ | CH | |
| L-11 | Q-2 | 0 | H | H | - | F | F | - | - | H | $OCH_3$ | $OCH_3$ | N | |
| L-11 | Q-2 | 0 | H | H | - | Cl | Cl | - | - | H | $CH_3$ | $OCH_3$ | CH | |
| L-11 | Q-2 | 0 | H | H | - | H | $CH_3$ | - | - | H | $CH_3$ | $OCH_3$ | CH | |
| L-11 | Q-2 | 0 | H | H | - | $CH_3$ | $CH_3$ | - | - | H | $OCH_3$ | $OCH_3$ | CH | |
| L-11 | Q-2 | 0 | H | H | - | H | $CO_2CH_3$ | - | - | H | $CH_3$ | $CH_3$ | CH | |
| L-11 | Q-2 | 0 | H | H | - | H | F | - | - | $CH_3$ | $OCH_3$ | $OCH_3$ | CH | |
| L-11 | Q-2 | 0 | H | $OCH_3$ | - | H | Cl | - | - | H | $OCH_3$ | $CH_3$ | N | |
| L-11 | Q-2 | 0 | $CH_3$ | Cl | - | F | $CH_3$ | - | - | $CH_3$ | $CH_3$ | $OCH_3$ | CH | |
| L-11 | Q-2 | 1 | H | H | - | H | Cl | - | - | H | $CH_3$ | $OCH_3$ | CH | |
| L-11 | Q-2 | 1 | H | H | - | Cl | Cl | - | - | H | $OCH_3$ | $OCH_3$ | N | |
| L-11 | Q-2 | 1 | H | H | - | F | $CH_3$ | - | - | H | Cl | $OCH_3$ | CH | |
| L-11 | Q-3 | 0 | H | H | $CH_3$ | H | H | - | - | H | $CH_3$ | $CH_3$ | CH | |
| L-11 | Q-3 | 0 | H | H | $CH_3$ | H | Cl | - | - | H | $CH_3$ | $OCH_3$ | CH | |
| L-11 | Q-3 | 0 | H | H | $CH_3$ | Cl | Cl | - | - | H | $OCH_3$ | $OCH_3$ | CH | |
| L-11 | Q-3 | 0 | H | H | $CH_3$ | F | F | - | - | H | $CH_3$ | $CH_3$ | N | |
| L-11 | Q-3 | 0 | H | H | $CH_3$ | H | Br | - | - | H | $CH_3$ | $OCH_3$ | N | |
| L-11 | Q-3 | 0 | H | H | $CH_3$ | $CH_3$ | H | - | - | H | $OCH_3$ | $OCH_3$ | N | |
| L-11 | Q-3 | 0 | H | H | $CH_3$ | H | $OCH_3$ | - | - | H | Cl | $OCH_3$ | CH | |
| L-11 | Q-3 | 0 | H | H | $CH_3$ | H | F | - | - | H | $CH_3$ | $OCH_3$ | CH | |
| L-11 | Q-3 | 0 | H | H | $CH_3$ | Cl | Br | - | - | H | $OCH_3$ | $OCH_3$ | CH | |
| L-11 | Q-3 | 0 | H | H | $CH_3$ | Cl | $CH_3$ | - | - | H | $CH_3$ | $OCH_3$ | N | |
| L-11 | Q-3 | 0 | H | H | $CH_3$ | F | $CH_3$ | - | - | H | $OCH_3$ | $OCH_3$ | CH | |
| L-11 | Q-3 | 0 | H | H | $CH_3$ | Cl | Cl | - | - | H | $CH_3$ | $CH_3$ | N | |

Table II (continued)

| L | $Q_1$ | n | R | $R_1$ | $R_3$ | $R_7$ | $R_8$ | $R_9$ | $R_{10}$ | $R_{11}$ | X | Y | Z | m.p. (°C) |
|---|---|---|---|---|---|---|---|---|---|---|---|---|---|---|
| L-11 | Q-3 | 0 | H | H | $CH_3$ | H | $CH_3$ | - | - | H | $OCH_3$ | $OCH_3$ | CH | |
| L-11 | Q-3 | 0 | H | $CH_3$ | $CH_3$ | H | H | - | - | H | Cl | $OCH_3$ | CH | |
| L-11 | Q-3 | 0 | $CH_3$ | H | $CH_3$ | H | H | - | - | H | $CH_3$ | $OCH_3$ | N | |
| L-11 | Q-3 | 1 | H | H | $CH_3$ | F | F | - | - | H | $OCH_3$ | $OCH_3$ | CH | |
| L-11 | Q-3 | 1 | H | H | $CH_3$ | H | H | - | - | H | $OCH_3$ | $OCH_3$ | CH | |
| L-11 | Q-4 | 0 | H | H | - | H | H | - | - | - | $CH_3$ | $CH_3$ | CH | |
| L-11 | Q-4 | 0 | H | H | - | H | H | - | - | - | $OCH_3$ | $CH_3$ | CH | |
| L-11 | Q-4 | 0 | H | H | - | H | H | - | - | - | $OCH_3$ | $OCH_3$ | CH | |
| L-11 | Q-4 | 0 | H | H | - | Cl | H | - | - | - | $CH_3$ | $OCH_3$ | N | |
| L-11 | Q-4 | 0 | H | H | - | F | H | - | - | - | Cl | $OCH_3$ | CH | |
| L-11 | Q-4 | 0 | H | H | - | H | Br | - | - | - | $OCH_3$ | $OCH_3$ | CH | |
| L-11 | Q-4 | 0 | H | H | - | F | F | - | - | - | $OCH_3$ | $OCH_3$ | N | |
| L-11 | Q-4 | 0 | H | H | - | Cl | Cl | - | - | - | $CH_3$ | $OCH_3$ | CH | |
| L-11 | Q-4 | 0 | H | H | - | H | $CH_3$ | - | - | - | $CH_3$ | $OCH_3$ | CH | |
| L-11 | Q-4 | 0 | H | H | - | $CH_3$ | $CH_3$ | - | - | - | $OCH_3$ | $OCH_3$ | CH | |
| L-11 | Q-4 | 0 | H | H | - | H | $CO_2CH_3$ | - | - | - | $CH_3$ | $CH_3$ | CH | |
| L-11 | Q-4 | 0 | H | H | - | H | F | - | - | - | $OCH_3$ | $OCH_3$ | CH | |
| L-11 | Q-4 | 0 | H | $OCH_3$ | - | H | Cl | - | - | - | $OCH_3$ | $CH_3$ | N | |
| L-11 | Q-4 | 0 | $CH_3$ | Cl | - | F | $CH_3$ | - | - | - | $CH_3$ | $OCH_3$ | CH | |
| L-11 | Q-4 | 1 | H | H | - | H | Cl | - | - | - | $CH_3$ | $OCH_3$ | CH | |
| L-11 | Q-4 | 1 | H | H | - | Cl | Cl | - | - | - | $OCH_3$ | $OCH_3$ | N | |
| L-11 | Q-4 | 1 | H | H | - | F | $CH_3$ | - | - | - | Cl | $OCH_3$ | CH | |
| L-11 | Q-5 | 0 | H | H | - | H | H | - | - | - | $CH_3$ | $CH_3$ | CH | |
| L-11 | Q-5 | 0 | H | H | - | H | Cl | - | - | - | $CH_3$ | $OCH_3$ | CH | |
| L-11 | Q-5 | 0 | H | H | - | Cl | Cl | - | - | - | $OCH_3$ | $OCH_3$ | CH | |
| L-11 | Q-5 | 0 | H | H | - | F | F | - | - | - | $CH_3$ | $CH_3$ | N | |
| L-11 | Q-5 | 0 | H | H | - | H | Br | - | - | - | $CH_3$ | $OCH_3$ | N | |
| L-11 | Q-5 | 0 | H | H | - | $CH_3$ | H | - | - | - | $OCH_3$ | $OCH_3$ | N | |
| L-11 | Q-5 | 0 | H | H | - | H | $OCH_3$ | - | - | - | Cl | $OCH_3$ | CH | |
| L-11 | Q-5 | 0 | H | H | - | H | F | - | - | - | $CH_3$ | $OCH_3$ | CH | |
| L-11 | Q-5 | 0 | H | H | - | Cl | Br | - | - | - | $OCH_3$ | $OCH_3$ | CH | |
| L-11 | Q-5 | 0 | H | H | - | Cl | $CH_3$ | - | - | - | $CH_3$ | $OCH_3$ | N | |

Table II (continued)

| L | $Q_1$ | n | R | $R_1$ | $R_3$ | $R_7$ | $R_8$ | $R_9$ | $R_{10}$ | $R_{11}$ | X | Y | Z | m.p. (°C) |
|---|---|---|---|---|---|---|---|---|---|---|---|---|---|---|
| L-11 | Q-5 | 0 | H | H | - | F | $CH_3$ | - | - | - | $OCH_3$ | $OCH_3$ | CH | |
| L-11 | Q-5 | 0 | H | H | - | H | Cl | - | - | - | $CH_3$ | $CH_3$ | N | |
| L-11 | Q-5 | 0 | H | H | - | H | $CH_3$ | - | - | - | $OCH_3$ | $OCH_3$ | CH | |
| L-11 | Q-5 | 0 | H | $CH_3$ | - | H | $CH_3$ | - | - | - | Cl | $OCH_3$ | CH | |
| L-11 | Q-5 | 0 | $CH_3$ | H | - | H | H | - | - | - | $CH_3$ | $OCH_3$ | N | |
| L-11 | Q-5 | 1 | H | H | - | F | F | - | - | - | $OCH_3$ | $OCH_3$ | CH | |
| L-11 | Q-5 | 1 | H | H | - | H | H | - | - | - | $OCH_3$ | $OCH_3$ | CH | |
| L-11 | Q-6 | 0 | H | H | - | H | H | - | - | - | $CH_3$ | $CH_3$ | CH | |
| L-11 | Q-6 | 0 | H | H | - | H | H | - | - | - | $OCH_3$ | $CH_3$ | CH | |
| L-11 | Q-6 | 0 | H | H | - | H | H | - | - | - | $OCH_3$ | $OCH_3$ | CH | |
| L-11 | Q-6 | 0 | H | H | - | Cl | H | - | - | - | $CH_3$ | $OCH_3$ | N | |
| L-11 | Q-6 | 0 | H | H | - | F | H | - | - | - | Cl | $OCH_3$ | CH | |
| L-11 | Q-6 | 0 | H | H | - | H | Br | - | - | - | $OCH_3$ | $OCH_3$ | CH | |
| L-11 | Q-6 | 0 | H | H | - | F | F | - | - | - | $OCH_3$ | $OCH_3$ | N | |
| L-11 | Q-6 | 0 | H | H | - | Cl | Cl | - | - | - | $CH_3$ | $OCH_3$ | CH | |
| L-11 | Q-6 | 0 | H | H | - | H | $CH_3$ | - | - | - | $CH_3$ | $OCH_3$ | CH | |
| L-11 | Q-6 | 0 | H | H | - | $CH_3$ | $CH_3$ | - | - | - | $OCH_3$ | $OCH_3$ | CH | |
| L-11 | Q-6 | 0 | H | H | - | H | $CH_2CH_3$ | - | - | - | $CH_3$ | $CH_3$ | CH | |
| L-11 | Q-6 | 0 | H | H | - | H | F | - | - | - | $OCH_3$ | $OCH_3$ | CH | |
| L-11 | Q-6 | 0 | H | $OCH_3$ | - | H | Cl | - | - | - | $OCH_3$ | $CH_3$ | N | |
| L-11 | Q-6 | 0 | $CH_3$ | Cl | - | F | $CH_3$ | - | - | - | $CH_3$ | $OCH_3$ | CH | |
| L-11 | Q-6 | 1 | H | H | - | H | Cl | - | - | - | $CH_3$ | $OCH_3$ | CH | |
| L-11 | Q-6 | 1 | H | H | - | Cl | Cl | - | - | - | $OCH_3$ | $OCH_3$ | N | |
| L-11 | Q-6 | 1 | H | H | - | F | $CH_3$ | - | - | - | Cl | $OCH_3$ | CH | |
| L-11 | Q-7 | 0 | H | H | - | H | H | - | - | - | $CH_3$ | $CH_3$ | CH | |
| L-11 | Q-7 | 0 | H | H | - | H | Cl | - | - | - | $CH_3$ | $OCH_3$ | CH | |
| L-11 | Q-7 | 0 | H | H | - | Cl | Cl | - | - | - | $OCH_3$ | $OCH_3$ | CH | |
| L-11 | Q-7 | 0 | H | H | - | F | F | - | - | - | $CH_3$ | $CH_3$ | N | |
| L-11 | Q-7 | 0 | H | H | - | H | Br | - | - | - | $CH_3$ | $OCH_3$ | N | |
| L-11 | Q-7 | 0 | H | H | - | $CH_3$ | H | - | - | - | $OCH_3$ | $OCH_3$ | N | |
| L-11 | Q-7 | 0 | H | H | - | H | $OCH_3$ | - | - | - | Cl | $OCH_3$ | CH | |
| L-11 | Q-7 | 0 | H | H | - | H | F | - | - | - | $CH_3$ | $OCH_3$ | CH | |

Table II (continued)

| L | $Q_1$ | n | R | $R_1$ | $R_3$ | $R_7$ | $R_8$ | $R_9$ | $R_{10}$ | $R_{11}$ | X | Y | Z | m.p. (°C) |
|---|---|---|---|---|---|---|---|---|---|---|---|---|---|---|
| L-11 | Q-7 | 0 | H | H | - | Cl | Br | - | - | - | $OCH_3$ | $OCH_3$ | CH | |
| L-11 | Q-7 | 0 | H | H | - | Cl | $CH_3$ | - | - | - | $CH_3$ | $OCH_3$ | N | |
| L-11 | Q-7 | 0 | H | H | - | F | $CH_3$ | - | - | - | $OCH_3$ | $OCH_3$ | CH | |
| L-11 | Q-7 | 0 | H | H | - | H | Cl | - | - | - | $CH_3$ | $CH_3$ | N | |
| L-11 | Q-7 | 0 | H | H | - | H | $CH_3$ | - | - | - | $OCH_3$ | $OCH_3$ | CH | |
| L-11 | Q-7 | 0 | H | $CH_3$ | - | H | H | - | - | - | Cl | $OCH_3$ | CH | |
| L-11 | Q-7 | 0 | $CH_3$ | H | - | H | H | - | - | - | $CH_3$ | $OCH_3$ | N | |
| L-11 | Q-7 | 1 | H | H | - | F | F | - | - | - | $OCH_3$ | $OCH_3$ | CH | |
| L-11 | Q-7 | 1 | H | H | - | H | H | - | - | - | $OCH_3$ | $OCH_3$ | CH | |
| L-11 | Q-8 | 0 | H | H | - | H | H | - | - | - | $CH_3$ | $CH_3$ | CH | |
| L-11 | Q-8 | 0 | H | H | - | H | H | - | - | - | $OCH_3$ | $CH_3$ | CH | |
| L-11 | Q-8 | 0 | H | H | - | H | H | - | - | - | $OCH_3$ | $OCH_3$ | CH | |
| L-11 | Q-8 | 0 | H | H | - | Cl | H | - | - | - | $CH_3$ | $OCH_3$ | N | |
| L-11 | Q-8 | 0 | H | H | - | F | H | - | - | - | Cl | $OCH_3$ | CH | |
| L-11 | Q-8 | 0 | H | H | - | H | Br | - | - | - | $OCH_3$ | $OCH_3$ | CH | |
| L-11 | Q-8 | 0 | H | H | - | F | F | - | - | - | $OCH_3$ | $OCH_3$ | N | |
| L-11 | Q-8 | 0 | H | H | - | Cl | Cl | - | - | - | $CH_3$ | $OCH_3$ | CH | |
| L-11 | Q-8 | 0 | H | H | - | H | $CH_3$ | - | - | - | $CH_3$ | $OCH_3$ | CH | |
| L-11 | Q-8 | 0 | H | H | - | $CH_3$ | $CH_3$ | - | - | - | $OCH_3$ | $OCH_3$ | CH | |
| L-11 | Q-8 | 0 | H | H | - | H | $CH_2CH_3$ | - | - | - | $CH_3$ | $CH_3$ | CH | |
| L-11 | Q-8 | 0 | H | H | - | H | F | - | - | - | $OCH_3$ | $OCH_3$ | CH | |
| L-11 | Q-8 | 0 | H | $OCH_3$ | - | H | Cl | - | - | - | $OCH_3$ | $CH_3$ | N | |
| L-11 | Q-8 | 0 | $CH_3$ | Cl | - | F | $CH_3$ | - | - | - | $CH_3$ | $OCH_3$ | CH | |
| L-11 | Q-8 | 1 | H | H | - | H | Cl | - | - | - | $CH_3$ | $OCH_3$ | CH | |
| L-11 | Q-8 | 1 | H | H | - | Cl | Cl | - | - | - | $OCH_3$ | $OCH_3$ | N | |
| L-11 | Q-8 | 1 | H | H | - | F | $CH_3$ | - | - | - | Cl | $OCH_3$ | CH | |

Table II (continued)

| L | $Q_1$ | n | R | $R_1$ | $R_3$ | $R_7$ | $R_8$ | $R_9$ | $R_{10}$ | $R_{11}$ | X | Y | Z | m.p. (°C) |
|---|---|---|---|---|---|---|---|---|---|---|---|---|---|---|
| L-11 | Q-9 | 0 | H | H | $CH_3$ | H | H | - | - | - | $CH_3$ | $CH_3$ | CH | |
| L-11 | Q-9 | 0 | H | H | $CH_3$ | H | Cl | - | - | - | $CH_3$ | $OCH_3$ | CH | |
| L-11 | Q-9 | 0 | H | H | $CH_3$ | Cl | Cl | - | - | - | $OCH_3$ | $OCH_3$ | CH | |
| L-11 | Q-9 | 0 | H | H | $CH_3$ | F | F | - | - | - | $CH_3$ | $CH_3$ | N | |
| L-11 | Q-9 | 0 | H | H | $CH_3$ | H | Br | - | - | - | $CH_3$ | $OCH_3$ | N | |
| L-11 | Q-9 | 0 | H | H | $CH_3$ | $CH_3$ | H | - | - | - | $OCH_3$ | $OCH_3$ | N | |
| L-11 | Q-9 | 0 | H | H | $CH_3$ | H | $OCH_3$ | - | - | - | Cl | $OCH_3$ | CH | |
| L-11 | Q-9 | 0 | H | H | $CH_3$ | H | F | - | - | - | $CH_3$ | $OCH_3$ | CH | |
| L-11 | Q-9 | 0 | H | H | $CH_3$ | Cl | Br | - | - | - | $OCH_3$ | $OCH_3$ | CH | |
| L-11 | Q-9 | 0 | H | H | $CH_3$ | Cl | $CH_3$ | - | - | - | $CH_3$ | $OCH_3$ | N | |
| L-11 | Q-9 | 0 | H | H | $CH_3$ | F | $CH_3$ | - | - | - | $OCH_3$ | $OCH_3$ | CH | |
| L-11 | Q-9 | 0 | H | H | $CH_3$ | H | Cl | - | - | - | $CH_3$ | $CH_3$ | N | |
| L-11 | Q-9 | 0 | H | H | $CH_3$ | H | $CH_3$ | - | - | - | $OCH_3$ | $OCH_3$ | CH | |
| L-11 | Q-9 | 0 | H | $CH_3$ | $CH_3$ | H | H | - | - | - | Cl | $OCH_3$ | CH | |
| L-11 | Q-9 | 0 | $CH_3$ | H | $CH_3$ | H | H | - | - | - | $CH_3$ | $OCH_3$ | N | |
| L-11 | Q-9 | 1 | H | H | $CH_3$ | F | F | - | - | - | $OCH_3$ | $OCH_3$ | CH | |
| L-11 | Q-9 | 1 | H | H | $CH_3$ | H | H | - | - | - | $OCH_3$ | $OCH_3$ | CH | |
| L-11 | Q-10 | 0 | H | H | $CH_3$ | H | H | - | - | - | $CH_3$ | $CH_3$ | CH | |
| L-11 | Q-10 | 0 | H | H | $CH_3$ | H | H | - | - | - | $OCH_3$ | $CH_3$ | CH | |
| L-11 | Q-10 | 0 | H | H | $CH_3$ | H | H | - | - | - | $OCH_3$ | $OCH_3$ | CH | |
| L-11 | Q-10 | 0 | H | H | $CH_3$ | Cl | H | - | - | - | $CH_3$ | $OCH_3$ | N | |
| L-11 | Q-10 | 0 | H | H | $CH_3$ | F | H | - | - | - | Cl | $OCH_3$ | CH | |
| L-11 | Q-10 | 0 | H | H | $CH_3$ | H | Br | - | - | - | $OCH_3$ | $OCH_3$ | CH | |
| L-11 | Q-10 | 0 | H | H | $CH_3$ | F | F | - | - | - | $OCH_3$ | $OCH_3$ | N | |
| L-11 | Q-10 | 0 | H | H | $CH_3$ | Cl | Cl | - | - | - | $CH_3$ | $OCH_3$ | CH | |
| L-11 | Q-10 | 0 | H | H | $CH_3$ | H | $CH_3$ | - | - | - | $CH_3$ | $OCH_3$ | CH | |
| L-11 | Q-10 | 0 | H | H | $CH_3$ | $CH_3$ | $CH_3$ | - | - | - | $OCH_3$ | $OCH_3$ | CH | |
| L-11 | Q-10 | 0 | H | H | $CH_3$ | H | $CO_2CH_3$ | - | - | - | $CH_3$ | $CH_3$ | CH | |
| L-11 | Q-10 | 0 | H | H | $CH_3$ | H | F | - | - | - | $OCH_3$ | $OCH_3$ | CH | |
| L-11 | Q-10 | 0 | H | $OCH_3$ | $CH_3$ | H | Cl | - | - | - | $OCH_3$ | $CH_3$ | N | |
| L-11 | Q-10 | 0 | $CH_3$ | Cl | $CH_3$ | F | $CH_3$ | - | - | - | $CH_3$ | $OCH_3$ | CH | |
| L-11 | Q-10 | 1 | H | H | $CH_3$ | H | Cl | - | - | - | $CH_3$ | $OCH_3$ | CH | |

Table II (continued)

| L | $Q_1$ | n | R | $R_1$ | $R_3$ | $R_7$ | $R_8$ | $R_9$ | $R_{10}$ | $R_{11}$ | X | Y | Z | m.p. (°C) |
|---|---|---|---|---|---|---|---|---|---|---|---|---|---|---|
| L-11 | Q-10 | 1 | H | H | $CH_3$ | Cl | Cl | - | - | - | $OCH_3$ | $OCH_3$ | N | |
| L-11 | Q-10 | 1 | H | H | $CH_3$ | F | $CH_3$ | - | - | - | Cl | $OCH_3$ | CH | |
| L-12 | Q-1 | 0 | H | H | - | H | H | H | H | - | $CH_3$ | $CH_3$ | CH | |
| L-12 | Q-1 | 0 | H | H | - | H | Cl | H | H | - | $CH_3$ | $OCH_3$ | CH | |
| L-12 | Q-1 | 0 | H | H | - | Cl | Cl | H | H | - | $OCH_3$ | $OCH_3$ | CH | |
| L-12 | Q-1 | 0 | H | H | - | F | F | H | H | - | $CH_3$ | $CH_3$ | N | |
| L-12 | Q-1 | 0 | H | H | - | H | Br | H | H | - | $CH_3$ | $OCH_3$ | N | |
| L-12 | Q-1 | 0 | H | H | - | $CH_3$ | H | H | H | - | $OCH_3$ | $OCH_3$ | N | |
| L-12 | Q-1 | 0 | H | H | - | H | $OCH_3$ | H | H | - | Cl | $OCH_3$ | CH | |
| L-12 | Q-1 | 0 | H | H | - | H | F | H | H | - | $CH_3$ | $OCH_3$ | CH | |
| L-12 | Q-1 | 0 | H | H | - | Cl | Br | H | H | - | $OCH_3$ | $OCH_3$ | CH | |
| L-12 | Q-1 | 0 | H | H | - | Cl | $CH_3$ | H | H | - | $CH_3$ | $OCH_3$ | N | |
| L-12 | Q-1 | 0 | H | H | - | F | $CH_3$ | H | H | - | $OCH_3$ | $OCH_3$ | CH | |
| L-12 | Q-1 | 0 | H | H | - | H | Cl | Cl | H | - | $CH_3$ | $CH_3$ | N | |
| L-12 | Q-1 | 0 | H | H | - | H | $CH_3$ | F | F | - | $OCH_3$ | $OCH_3$ | CH | |
| L-12 | Q-1 | 0 | H | $CH_3$ | - | H | H | H | H | - | Cl | $OCH_3$ | CH | |
| L-12 | Q-1 | 0 | $CH_3$ | H | - | H | H | H | H | - | $CH_3$ | $OCH_3$ | N | |
| L-12 | Q-1 | 1 | H | H | - | F | F | H | H | - | $OCH_3$ | $OCH_3$ | CH | |
| L-12 | Q-1 | 1 | H | H | - | H | H | H | H | - | $OCH_3$ | $OCH_3$ | CH | |
| L-12 | Q-2 | 0 | H | H | - | H | H | - | - | H | $CH_3$ | $CH_3$ | CH | |
| L-12 | Q-2 | 0 | H | H | - | H | H | - | - | H | $OCH_3$ | $CH_3$ | CH | |
| L-12 | Q-2 | 0 | H | H | - | H | H | - | - | H | $OCH_3$ | $OCH_3$ | CH | |
| L-12 | Q-2 | 0 | H | H | - | Cl | H | - | - | H | $CH_3$ | $OCH_3$ | N | |
| L-12 | Q-2 | 0 | H | H | - | F | H | - | - | H | Cl | $OCH_3$ | CH | |
| L-12 | Q-2 | 0 | H | H | - | H | Br | - | - | H | $OCH_3$ | $OCH_3$ | CH | |
| L-12 | Q-2 | 0 | H | H | - | F | F | - | - | H | $OCH_3$ | $OCH_3$ | N | |
| L-12 | Q-2 | 0 | H | H | - | Cl | Cl | - | - | H | $CH_3$ | $OCH_3$ | CH | |
| L-12 | Q-2 | 0 | H | H | - | H | $CH_3$ | - | - | H | $CH_3$ | $OCH_3$ | CH | |
| L-12 | Q-2 | 0 | H | H | - | $CH_3$ | $CH_3$ | - | - | H | $OCH_3$ | $OCH_3$ | CH | |
| L-12 | Q-2 | 0 | H | H | - | H | $CO_2CH_3$ | - | - | H | $CH_3$ | $CH_3$ | CH | |
| L-12 | Q-2 | 0 | H | H | - | H | F | - | - | $CH_3$ | $OCH_3$ | $OCH_3$ | CH | |
| L-12 | Q-2 | 0 | H | $OCH_3$ | - | H | Cl | - | - | H | $OCH_3$ | $CH_3$ | N | |

Table II (continued)

| L | $Q_1$ | n | R | $R_1$ | $R_3$ | $R_7$ | $R_8$ | $R_9$ | $R_{10}$ | $R_{11}$ | X | Y | Z | m.p. (°C) |
|---|---|---|---|---|---|---|---|---|---|---|---|---|---|---|
| L-12 | Q-2 | 0 | $CH_3$ | Cl | - | F | $CH_3$ | - | - | $CH_3$ | $CH_3$ | $OCH_3$ | CH | |
| L-12 | Q-2 | 1 | H | H | - | H | Cl | - | - | H | $CH_3$ | $OCH_3$ | CH | |
| L-12 | Q-2 | 1 | H | H | - | Cl | Cl | - | - | H | $OCH_3$ | $OCH_3$ | N | |
| L-12 | Q-2 | 1 | H | H | - | F | $CH_3$ | - | - | H | Cl | $OCH_3$ | CH | |
| L-12 | Q-3 | 0 | H | H | $CH_3$ | H | H | - | - | H | $CH_3$ | $CH_3$ | CH | |
| L-12 | Q-3 | 0 | H | H | $CH_3$ | H | Cl | - | - | H | $CH_3$ | $OCH_3$ | CH | |
| L-12 | Q-3 | 0 | H | H | $CH_3$ | Cl | Cl | - | - | H | $OCH_3$ | $OCH_3$ | CH | |
| L-12 | Q-3 | 0 | H | H | $CH_3$ | F | F | - | - | H | $CH_3$ | $CH_3$ | N | |
| L-12 | Q-3 | 0 | H | H | $CH_3$ | H | Br | - | - | H | $CH_3$ | $OCH_3$ | N | |
| L-12 | Q-3 | 0 | H | H | $CH_3$ | $CH_3$ | H | - | - | H | $OCH_3$ | $OCH_3$ | N | |
| L-12 | Q-3 | 0 | H | H | $CH_3$ | H | $OCH_3$ | - | - | H | Cl | $OCH_3$ | CH | |
| L-12 | Q-3 | 0 | H | H | $CH_3$ | H | F | - | - | H | $CH_3$ | $OCH_3$ | CH | |
| L-12 | Q-3 | 0 | H | H | $CH_3$ | Cl | Br | - | - | H | $OCH_3$ | $OCH_3$ | CH | |
| L-12 | Q-3 | 0 | H | H | $CH_3$ | Cl | $CH_3$ | - | - | H | $CH_3$ | $OCH_3$ | N | |
| L-12 | Q-3 | 0 | H | H | $CH_3$ | F | $CH_3$ | - | - | H | $OCH_3$ | $OCH_3$ | CH | |
| L-12 | Q-3 | 0 | H | H | $CH_3$ | H | Cl | - | - | H | $CH_3$ | $CH_3$ | N | |
| L-12 | Q-3 | 0 | H | H | $CH_3$ | H | $CH_3$ | - | - | $CH_3$ | $OCH_3$ | $OCH_3$ | CH | |
| L-12 | Q-3 | 0 | H | $CH_3$ | $CH_3$ | H | H | - | - | H | Cl | $OCH_3$ | CH | |
| L-12 | Q-3 | 0 | $CH_3$ | H | $CH_3$ | H | H | - | - | H | $CH_3$ | $OCH_3$ | N | |
| L-12 | Q-3 | 1 | H | H | $CH_3$ | F | F | - | - | H | $OCH_3$ | $OCH_3$ | CH | |
| L-12 | Q-3 | 1 | H | H | $CH_3$ | H | H | - | - | H | $OCH_3$ | $OCH_3$ | CH | |
| L-12 | Q-4 | 0 | H | H | - | H | H | - | - | - | $CH_3$ | $CH_3$ | CH | |
| L-12 | Q-4 | 0 | H | H | - | H | H | - | - | - | $OCH_3$ | $CH_3$ | CH | |
| L-12 | Q-4 | 0 | H | H | - | H | H | - | - | - | $OCH_3$ | $OCH_3$ | CH | |
| L-12 | Q-4 | 0 | H | H | - | Cl | H | - | - | - | $CH_3$ | $OCH_3$ | N | |
| L-12 | Q-4 | 0 | H | H | - | F | H | - | - | - | Cl | $OCH_3$ | CH | |
| L-12 | Q-4 | 0 | H | H | - | H | Br | - | - | - | $OCH_3$ | $OCH_3$ | CH | |
| L-12 | Q-4 | 0 | H | H | - | F | F | - | - | - | $OCH_3$ | $OCH_3$ | N | |
| L-12 | Q-4 | 0 | H | H | - | Cl | Cl | - | - | - | $CH_3$ | $OCH_3$ | CH | |
| L-12 | Q-4 | 0 | H | H | - | H | $CH_3$ | - | - | - | $CH_3$ | $OCH_3$ | CH | |
| L-12 | Q-4 | 0 | H | H | - | $CH_3$ | $CH_3$ | - | - | - | $OCH_3$ | $OCH_3$ | CH | |
| L-12 | Q-4 | 0 | H | H | - | H | $CO_2CH_3$ | - | - | - | $CH_3$ | $CH_3$ | CH | |

Table II (continued)

| L | $Q_1$ | n | R | $R_1$ | $R_3$ | $R_7$ | $R_8$ | $R_9$ | $R_{10}$ | $R_{11}$ | X | Y | Z | m.p. (°C) |
|---|---|---|---|---|---|---|---|---|---|---|---|---|---|---|
| L-12 | Q-4 | 0 | H | H | - | H | F | - | - | - | $OCH_3$ | $OCH_3$ | CH | |
| L-12 | Q-4 | 0 | H | $OCH_3$ | - | H | Cl | - | - | - | $OCH_3$ | $CH_3$ | N | |
| L-12 | Q-4 | 0 | $CH_3$ | Cl | - | F | $CH_3$ | - | - | - | $CH_3$ | $OCH_3$ | CH | |
| L-12 | Q-4 | 1 | H | H | - | H | Cl | - | - | - | $CH_3$ | $OCH_3$ | CH | |
| L-12 | Q-4 | 1 | H | H | - | Cl | Cl | - | - | - | $OCH_3$ | $OCH_3$ | N | |
| L-12 | Q-4 | 1 | H | H | - | F | $CH_3$ | - | - | - | Cl | $OCH_3$ | CH | |
| L-12 | Q-5 | 0 | H | H | - | H | H | - | - | - | $CH_3$ | $CH_3$ | CH | |
| L-12 | Q-5 | 0 | H | H | - | H | Cl | - | - | - | $CH_3$ | $OCH_3$ | CH | |
| L-12 | Q-5 | 0 | H | H | - | Cl | Cl | - | - | - | $OCH_3$ | $OCH_3$ | CH | |
| L-12 | Q-5 | 0 | H | H | - | F | F | - | - | - | $CH_3$ | $CH_3$ | N | |
| L-12 | Q-5 | 0 | H | H | - | H | Br | - | - | - | $CH_3$ | $OCH_3$ | N | |
| L-12 | Q-5 | 0 | H | H | - | $CH_3$ | H | - | - | - | $OCH_3$ | $OCH_3$ | N | |
| L-12 | Q-5 | 0 | H | H | - | H | $OCH_3$ | - | - | - | Cl | $OCH_3$ | CH | |
| L-12 | Q-5 | 0 | H | H | - | Cl | Br | - | - | - | $OCH_3$ | $OCH_3$ | CH | |
| L-12 | Q-5 | 0 | H | H | - | Cl | $CH_3$ | - | - | - | $CH_3$ | $OCH_3$ | N | |
| L-12 | Q-5 | 0 | H | H | - | F | $CH_3$ | - | - | - | $OCH_3$ | $OCH_3$ | CH | |
| L-12 | Q-5 | 0 | H | H | - | H | Cl | - | - | - | $CH_3$ | $CH_3$ | N | |
| L-12 | Q-5 | 0 | H | H | - | H | $CH_3$ | - | - | - | $OCH_3$ | $OCH_3$ | CH | |
| L-12 | Q-5 | 0 | H | $CH_3$ | - | H | H | - | - | - | Cl | $OCH_3$ | CH | |
| L-12 | Q-5 | 0 | $CH_3$ | H | - | H | H | - | - | - | $CH_3$ | $OCH_3$ | N | |
| L-12 | Q-5 | 1 | H | H | - | F | F | - | - | - | $OCH_3$ | $OCH_3$ | CH | |
| L-12 | Q-5 | 1 | H | H | - | H | H | - | - | - | $OCH_3$ | $OCH_3$ | CH | |
| L-12 | Q-6 | 0 | H | H | - | H | H | - | - | - | $CH_3$ | $CH_3$ | CH | |
| L-12 | Q-6 | 0 | H | H | - | H | H | - | - | - | $OCH_3$ | $CH_3$ | CH | |
| L-12 | Q-6 | 0 | H | H | - | H | H | - | - | - | $OCH_3$ | $OCH_3$ | CH | |
| L-12 | Q-6 | 0 | H | H | - | Cl | H | - | - | - | $CH_3$ | $OCH_3$ | N | |
| L-12 | Q-6 | 0 | H | H | - | F | H | - | - | - | Cl | $OCH_3$ | CH | |
| L-12 | Q-6 | 0 | H | H | - | H | Br | - | - | - | $OCH_3$ | $OCH_3$ | CH | |
| L-12 | Q-6 | 0 | H | H | - | F | F | - | - | - | $OCH_3$ | $OCH_3$ | N | |
| L-12 | Q-6 | 0 | H | H | - | Cl | Cl | - | - | - | $CH_3$ | $OCH_3$ | CH | |
| L-12 | Q-6 | 0 | H | H | - | H | $CH_3$ | - | - | - | $CH_3$ | $OCH_3$ | CH | |
| L-12 | Q-6 | 0 | H | H | - | $CH_3$ | $CH_3$ | - | - | - | $OCH_3$ | $OCH_3$ | CH | |

Table II (continued)

| L | $Q_1$ | n | R | $R_1$ | $R_3$ | $R_7$ | $R_8$ | $R_9$ | $R_{10}$ | $R_{11}$ | X | Y | Z | m.p. (°C) |
|---|---|---|---|---|---|---|---|---|---|---|---|---|---|---|
| L-12 | Q-6 | 0 | H | H | - | H | $CO_2CH_3$ | - | - | - | $CH_3$ | $CH_3$ | CH | |
| L-12 | Q-6 | 0 | H | H | - | H | F | - | - | - | $OCH_3$ | $OCH_3$ | CH | |
| L-12 | Q-6 | 0 | H | $OCH_3$ | - | H | Cl | - | - | - | $OCH_3$ | $CH_3$ | N | |
| L-12 | Q-6 | 0 | $CH_3$ | Cl | - | F | $CH_3$ | - | - | - | $CH_3$ | $OCH_3$ | CH | |
| L-12 | Q-6 | 1 | H | H | - | H | Cl | - | - | - | $CH_3$ | $OCH_3$ | CH | |
| L-12 | Q-6 | 1 | H | H | - | Cl | Cl | - | - | - | $OCH_3$ | $OCH_3$ | N | |
| L-12 | Q-6 | 1 | H | H | - | F | $CH_3$ | - | - | - | Cl | $OCH_3$ | CH | |
| L-12 | Q-7 | 0 | H | H | - | H | H | - | - | - | $CH_3$ | $CH_3$ | CH | |
| L-12 | Q-7 | 0 | H | H | - | H | Cl | - | - | - | $CH_3$ | $OCH_3$ | CH | |
| L-12 | Q-7 | 0 | H | H | - | Cl | Cl | - | - | - | $OCH_3$ | $OCH_3$ | CH | |
| L-12 | Q-7 | 0 | H | H | - | F | F | - | - | - | $CH_3$ | $CH_3$ | N | |
| L-12 | Q-7 | 0 | H | H | - | H | Br | - | - | - | $CH_3$ | $OCH_3$ | N | |
| L-12 | Q-7 | 0 | H | H | - | $CH_3$ | H | - | - | - | $OCH_3$ | $OCH_3$ | N | |
| L-12 | Q-7 | 0 | H | H | - | H | $OCH_3$ | - | - | - | Cl | $OCH_3$ | CH | |
| L-12 | Q-7 | 0 | H | H | - | H | F | - | - | - | $CH_3$ | $OCH_3$ | CH | |
| L-12 | Q-7 | 0 | H | H | - | Cl | Br | - | - | - | $OCH_3$ | $OCH_3$ | CH | |
| L-12 | Q-7 | 0 | H | H | - | Cl | $CH_3$ | - | - | - | $CH_3$ | $OCH_3$ | N | |
| L-12 | Q-7 | 0 | H | H | - | F | $CH_3$ | - | - | - | $OCH_3$ | $OCH_3$ | CH | |
| L-12 | Q-7 | 0 | H | H | - | H | Cl | - | - | - | $CH_3$ | $CH_3$ | N | |
| L-12 | Q-7 | 0 | H | H | - | H | $CH_3$ | - | - | - | $OCH_3$ | $OCH_3$ | CH | |
| L-12 | Q-7 | 0 | H | $CH_3$ | - | H | H | - | - | - | Cl | $OCH_3$ | CH | |
| L-12 | Q-7 | 0 | $CH_3$ | H | - | H | H | - | - | - | $CH_3$ | $OCH_3$ | N | |
| L-12 | Q-7 | 1 | H | H | - | F | F | - | - | - | $OCH_3$ | $OCH_3$ | CH | |
| L-12 | Q-7 | 1 | H | H | - | H | H | - | - | - | $OCH_3$ | $OCH_3$ | CH | |
| L-12 | Q-8 | 0 | H | H | - | H | H | - | - | - | $CH_3$ | $CH_3$ | CH | |
| L-12 | Q-8 | 0 | H | H | - | H | H | - | - | - | $OCH_3$ | $CH_3$ | CH | |
| L-12 | Q-8 | 0 | H | H | - | H | H | - | - | - | $OCH_3$ | $OCH_3$ | CH | |
| L-12 | Q-8 | 0 | H | H | - | Cl | H | - | - | - | $CH_3$ | $OCH_3$ | N | |
| L-12 | Q-8 | 0 | H | H | - | F | H | - | - | - | Cl | $OCH_3$ | CH | |
| L-12 | Q-8 | 0 | H | H | - | H | Br | - | - | - | $OCH_3$ | $OCH_3$ | CH | |
| L-12 | Q-8 | 0 | H | H | - | F | F | - | - | - | $OCH_3$ | $OCH_3$ | N | |
| L-12 | Q-8 | 0 | H | H | - | Cl | Cl | - | - | - | $CH_3$ | $OCH_3$ | CH | |

Table II (continued)

| L | $Q_1$ | n | R | $R_1$ | $R_3$ | $R_7$ | $R_8$ | $R_9$ | $R_{10}$ | $R_{11}$ | X | Y | Z | m.p. (°C) |
|---|---|---|---|---|---|---|---|---|---|---|---|---|---|---|
| L-12 | Q-8 | 0 | H | H | - | H | $CH_3$ | - | - | - | $CH_3$ | $OCH_3$ | CH | |
| L-12 | Q-8 | 0 | H | H | - | H | $CH_3$ | - | - | - | $CH_3$ | $OCH_3$ | CH | |
| L-12 | Q-8 | 0 | H | H | - | $CH_3$ | $CH_3$ | - | - | - | $OCH_3$ | $OCH_3$ | CH | |
| L-12 | Q-8 | 0 | H | H | - | H | $CO_2CH_3$ | - | - | - | $CH_3$ | $CH_3$ | CH | |
| L-12 | Q-8 | 0 | H | H | - | H | F | - | - | - | $OCH_3$ | $OCH_3$ | CH | |
| L-12 | Q-8 | 0 | H | $OCH_3$ | - | H | Cl | - | - | - | $OCH_3$ | $CH_3$ | N | |
| L-12 | Q-8 | 0 | H | $OCH_3$ | - | H | Cl | - | - | - | $OCH_3$ | $CH_3$ | N | |
| L-12 | Q-8 | 0 | $CH_3$ | Cl | - | F | $CH_3$ | - | - | - | $CH_3$ | $OCH_3$ | CH | |
| L-12 | Q-8 | 1 | H | H | - | H | Cl | - | - | - | $CH_3$ | $OCH_3$ | CH | |
| L-12 | Q-8 | 1 | H | H | - | Cl | Cl | - | - | - | $OCH_3$ | $OCH_3$ | N | |
| L-12 | Q-8 | 1 | H | H | - | F | $CH_3$ | - | - | - | Cl | $OCH_3$ | CH | |
| L-12 | Q-9 | 0 | H | H | $CH_3$ | H | H | - | - | - | $CH_3$ | $CH_3$ | CH | |
| L-12 | Q-9 | 0 | H | H | $CH_3$ | H | Cl | - | - | - | $CH_3$ | $OCH_3$ | CH | |
| L-12 | Q-9 | 0 | H | H | $CH_3$ | Cl | Cl | - | - | - | $OCH_3$ | $OCH_3$ | CH | |
| L-12 | Q-9 | 0 | H | H | $CH_3$ | F | F | - | - | - | $CH_3$ | $CH_3$ | N | |
| L-12 | Q-9 | 0 | H | H | $CH_3$ | H | Br | - | - | - | $CH_3$ | $OCH_3$ | N | |
| L-12 | Q-9 | 0 | H | H | $CH_3$ | $CH_3$ | H | - | - | - | $OCH_3$ | $OCH_3$ | N | |
| L-12 | Q-9 | 0 | H | H | $CH_3$ | H | $OCH_3$ | - | - | - | Cl | $OCH_3$ | CH | |
| L-12 | Q-9 | 0 | H | H | $CH_3$ | H | H | - | - | - | $CH_3$ | $OCH_3$ | CH | |
| L-12 | Q-9 | 0 | H | H | $CH_3$ | Cl | Br | - | - | - | $OCH_3$ | $OCH_3$ | CH | |
| L-12 | Q-9 | 0 | H | H | $CH_3$ | Cl | $CH_3$ | - | - | - | $CH_3$ | $OCH_3$ | N | |
| L-12 | Q-9 | 0 | H | H | $CH_3$ | F | $CH_3$ | - | - | - | $OCH_3$ | $OCH_3$ | CH | |
| L-12 | Q-9 | 0 | H | H | $CH_3$ | H | Cl | - | - | - | $CH_3$ | $CH_3$ | N | |
| L-12 | Q-9 | 0 | H | H | $CH_3$ | H | $CH_3$ | - | - | - | $OCH_3$ | $OCH_3$ | CH | |
| L-12 | Q-9 | 0 | H | $CH_3$ | $CH_3$ | H | H | - | - | - | Cl | $OCH_3$ | CH | |
| L-12 | Q-9 | 0 | $CH_3$ | H | $CH_3$ | H | H | - | - | - | $CH_3$ | $OCH_3$ | N | |
| L-12 | Q-9 | 1 | H | H | $CH_3$ | F | F | - | - | - | $OCH_3$ | $OCH_3$ | CH | |
| L-12 | Q-9 | 1 | H | H | $CH_3$ | H | H | - | - | - | $OCH_3$ | $OCH_3$ | CH | |
| L-12 | Q-10 | 0 | H | H | $CH_3$ | H | H | - | - | - | $CH_3$ | $CH_3$ | CH | |
| L-12 | Q-10 | 0 | H | H | $CH_3$ | H | H | - | - | - | $OCH_3$ | $CH_3$ | CH | |
| L-12 | Q-10 | 0 | H | H | $CH_3$ | H | H | - | - | - | $OCH_3$ | $OCH_3$ | CH | |
| L-12 | Q-10 | 0 | H | H | $CH_3$ | H | H | - | - | - | $OCH_3$ | $OCH_3$ | CH | |
| L-12 | Q-10 | 0 | H | H | $CH_3$ | Cl | H | - | - | - | $CH_3$ | $OCH_3$ | N | |

Table II (continued)

| L | $Q_1$ | n | R | $R_1$ | $R_3$ | $R_7$ | $R_8$ | $R_9$ | $R_{10}$ | $R_{11}$ | X | Y | Z | m.p. (°C) |
|---|---|---|---|---|---|---|---|---|---|---|---|---|---|---|
| L-12 | Q-10 | 0 | H | H | $CH_3$ | F | H | - | - | - | Cl | $OCH_3$ | CH | |
| L-12 | Q-10 | 0 | H | H | $CH_3$ | H | Br | - | - | - | $OCH_3$ | $OCH_3$ | CH | |
| L-12 | Q-10 | 0 | H | H | $CH_3$ | F | F | - | - | - | $OCH_3$ | $OCH_3$ | N | |
| L-12 | Q-10 | 0 | H | H | $CH_2CH_3$ | Cl | Cl | - | - | - | $CH_3$ | $OCH_3$ | CH | |
| L-12 | Q-10 | 0 | H | H | $CH_3$ | H | $CH_3$ | - | - | - | $CH_3$ | $OCH_3$ | CH | |
| L-12 | Q-10 | 0 | H | H | $CH_3$ | $CH_3$ | $CH_3$ | - | - | - | $OCH_3$ | $OCH_3$ | CH | |
| L-12 | Q-10 | 0 | H | H | $CH_3$ | H | $CO_2CH_3$ | - | - | - | $CH_3$ | $CH_3$ | CH | |
| L-12 | Q-10 | 0 | H | H | $CH_3$ | H | F | - | - | - | $OCH_3$ | $OCH_3$ | CH | |
| L-12 | Q-10 | 0 | H | $OCH_3$ | $CH_3$ | H | Cl | - | - | - | $OCH_3$ | $CH_3$ | N | |
| L-12 | Q-10 | 0 | $CH_3$ | Cl | $CH_3$ | F | $CH_3$ | - | - | - | $CH_3$ | $OCH_3$ | CH | |
| L-12 | Q-10 | 1 | H | H | $CH_3$ | H | Cl | - | - | - | $CH_3$ | $OCH_3$ | CH | |
| L-12 | Q-10 | 1 | H | H | $CH_3$ | Cl | Cl | - | - | - | $OCH_3$ | $OCH_3$ | N | |
| L-12 | Q-10 | 1 | H | H | $CH_3$ | F | $CH_3$ | - | - | - | Cl | $OCH_3$ | CH | |
| L-13 | Q-1 | 0 | H | H | - | H | H | H | H | - | $CH_3$ | $CH_3$ | CH | |
| L-13 | Q-1 | 0 | H | H | - | H | Cl | H | H | - | $CH_3$ | $OCH_3$ | CH | |
| L-13 | Q-1 | 0 | H | H | - | Cl | Cl | H | H | - | $OCH_3$ | $OCH_3$ | CH | |
| L-13 | Q-1 | 0 | H | H | - | F | F | H | H | - | $CH_3$ | $CH_3$ | N | |
| L-13 | Q-1 | 0 | H | H | - | H | Br | H | H | - | $CH_3$ | $OCH_3$ | N | |
| L-13 | Q-1 | 0 | H | H | - | $CH_3$ | H | H | H | - | $OCH_3$ | $OCH_3$ | N | |
| L-13 | Q-1 | 0 | H | H | - | H | $OCH_3$ | H | H | - | Cl | $OCH_3$ | CH | |
| L-13 | Q-1 | 0 | H | H | - | H | F | H | H | - | Cl | $OCH_3$ | CH | |
| L-13 | Q-1 | 0 | H | H | - | Cl | Br | H | H | - | $OCH_3$ | $OCH_3$ | CH | |
| L-13 | Q-1 | 0 | H | H | - | Cl | $CH_3$ | H | H | - | $CH_3$ | $OCH_3$ | N | |
| L-13 | Q-1 | 0 | H | H | - | F | $CH_3$ | H | H | - | $OCH_3$ | $OCH_3$ | CH | |
| L-13 | Q-1 | 0 | H | H | - | H | Cl | Cl | H | - | $CH_3$ | $CH_3$ | N | |
| L-13 | Q-1 | 0 | H | H | - | H | $CH_3$ | F | F | - | $OCH_3$ | $OCH_3$ | CH | |
| L-13 | Q-1 | 0 | H | $CH_3$ | - | H | H | H | H | - | Cl | $OCH_3$ | CH | |
| L-13 | Q-1 | 0 | $CH_3$ | H | - | H | H | H | H | - | $CH_3$ | $OCH_3$ | N | |
| L-13 | Q-1 | 1 | H | H | - | F | F | H | H | - | $OCH_3$ | $OCH_3$ | CH | |
| L-13 | Q-1 | 1 | H | H | - | H | H | H | H | - | $OCH_3$ | $OCH_3$ | CH | |
| L-13 | Q-2 | 0 | H | H | - | H | H | - | - | H | $CH_3$ | $CH_3$ | CH | |
| L-13 | Q-2 | 0 | H | H | - | H | H | - | - | H | $OCH_3$ | $CH_3$ | CH | |

Table II (continued)

| L | $Q_1$ | n | R | $R_1$ | $R_3$ | $R_7$ | $R_8$ | $R_9$ | $R_{10}$ | $R_{11}$ | X | Y | Z | m.p. (°C) |
|---|---|---|---|---|---|---|---|---|---|---|---|---|---|---|
| L-13 | Q-2 | 0 | H | H | - | H | H | - | - | H | $OCH_3$ | $OCH_3$ | CH | |
| L-13 | Q-2 | 0 | H | H | - | Cl | H | - | - | H | $CH_3$ | $OCH_3$ | N | |
| L-13 | Q-2 | 0 | H | H | - | F | H | - | - | H | Cl | $OCH_3$ | CH | |
| L-13 | Q-2 | 0 | H | H | - | H | Br | - | - | H | $OCH_3$ | $OCH_3$ | CH | |
| L-13 | Q-2 | 0 | H | H | - | F | F | - | - | H | $OCH_3$ | $OCH_3$ | N | |
| L-13 | Q-2 | 0 | H | H | - | Cl | Cl | - | - | H | $CH_3$ | $OCH_3$ | CH | |
| L-13 | Q-2 | 0 | H | H | - | H | $CH_3$ | - | - | H | $CH_3$ | $OCH_3$ | CH | |
| L-13 | Q-2 | 0 | H | H | - | $CH_3$ | $CH_3$ | - | - | H | $OCH_3$ | $OCH_3$ | CH | |
| L-13 | Q-2 | 0 | H | H | - | H | $CO_2CH_3$ | - | - | H | $CH_3$ | $CH_3$ | CH | |
| L-13 | Q-2 | 0 | H | H | - | H | F | - | - | $CH_3$ | $OCH_3$ | $OCH_3$ | CH | |
| L-13 | Q-2 | 0 | H | $OCH_3$ | - | H | Cl | - | - | H | $OCH_3$ | $CH_3$ | N | |
| L-13 | Q-2 | 0 | $CH_3$ | Cl | - | F | $CH_3$ | - | - | $CH_3$ | $CH_3$ | $OCH_3$ | CH | |
| L-13 | Q-2 | 1 | H | H | - | H | Cl | - | - | H | $CH_3$ | $OCH_3$ | CH | |
| L-13 | Q-2 | 1 | H | H | - | Cl | Cl | - | - | H | $OCH_3$ | $OCH_3$ | N | |
| L-13 | Q-2 | 1 | H | H | - | F | $CH_3$ | - | - | H | Cl | $OCH_3$ | CH | |
| L-13 | Q-3 | 0 | H | H | $CH_3$ | H | H | - | - | H | $CH_3$ | $CH_3$ | CH | |
| L-13 | Q-3 | 0 | H | H | $CH_3$ | H | Cl | - | - | H | $CH_3$ | $OCH_3$ | CH | |
| L-13 | Q-3 | 0 | H | H | $CH_3$ | Cl | Cl | - | - | H | $OCH_3$ | $OCH_3$ | CH | |
| L-13 | Q-3 | 0 | H | H | $CH_3$ | F | F | - | - | H | $CH_3$ | $CH_3$ | N | |
| L-13 | Q-3 | 0 | H | H | $CH_3$ | H | Br | - | - | H | $CH_3$ | $OCH_3$ | N | |
| L-13 | Q-3 | 0 | H | H | $CH_3$ | $CH_3$ | H | - | - | H | $OCH_3$ | $OCH_3$ | N | |
| L-13 | Q-3 | 0 | H | H | $CH_3$ | H | $OCH_3$ | - | - | H | Cl | $OCH_3$ | CH | |
| L-13 | Q-3 | 0 | H | H | $CH_3$ | H | F | - | - | H | $CH_3$ | $OCH_3$ | CH | |
| L-13 | Q-3 | 0 | H | H | $CH_3$ | Cl | Br | - | - | $CH_3$ | $OCH_3$ | $OCH_3$ | CH | |
| L-13 | Q-3 | 0 | H | H | $CH_3$ | Cl | $CH_3$ | - | - | $CH_3$ | $CH_3$ | $OCH_3$ | N | |
| L-13 | Q-3 | 0 | H | H | $CH_3$ | F | $CH_3$ | - | - | H | $OCH_3$ | $OCH_3$ | CH | |
| L-13 | Q-3 | 0 | H | H | $CH_3$ | H | Cl | - | - | H | $CH_3$ | $CH_3$ | N | |
| L-13 | Q-3 | 0 | H | H | $CH_3$ | H | $CH_3$ | - | - | H | $OCH_3$ | $OCH_3$ | CH | |
| L-13 | Q-3 | 0 | H | $CH_3$ | $CH_3$ | H | H | - | - | H | Cl | $OCH_3$ | CH | |
| L-13 | Q-3 | 0 | $CH_3$ | H | $CH_3$ | H | H | - | - | H | $CH_3$ | $OCH_3$ | N | |
| L-13 | Q-3 | 1 | H | H | $CH_3$ | F | F | - | - | H | $OCH_3$ | $OCH_3$ | CH | |
| L-13 | Q-3 | 1 | H | H | $CH_3$ | H | H | - | - | H | $OCH_3$ | $OCH_3$ | CH | |

Table II (continued)

| L | $Q_1$ | n | R | $R_1$ | $R_3$ | $R_7$ | $R_8$ | $R_9$ | $R_{10}$ | $R_{11}$ | X | Y | Z | m.p. (°C) |
|---|---|---|---|---|---|---|---|---|---|---|---|---|---|---|
| L-13 | Q-4 | 0 | H | H | - | H | H | - | - | - | $CH_3$ | $CH_3$ | CH | |
| L-13 | Q-4 | 0 | H | H | - | H | H | - | - | - | $OCH_3$ | $CH_3$ | CH | |
| L-13 | Q-4 | 0 | H | H | - | H | H | - | - | - | $OCH_3$ | $OCH_3$ | CH | |
| L-13 | Q-4 | 0 | H | H | - | Cl | H | - | - | - | $CH_3$ | $OCH_3$ | N | |
| L-13 | Q-4 | 0 | H | H | - | F | H | - | - | - | Cl | $OCH_3$ | CH | |
| L-13 | Q-4 | 0 | H | H | - | H | Br | - | - | - | $OCH_3$ | $OCH_3$ | CH | |
| L-13 | Q-4 | 0 | H | H | - | F | F | - | - | - | $OCH_3$ | $OCH_3$ | N | |
| L-13 | Q-4 | 0 | H | H | - | Cl | Cl | - | - | - | $CH_3$ | $OCH_3$ | CH | |
| L-13 | Q-4 | 0 | H | H | - | H | $CH_3$ | - | - | - | $CH_3$ | $OCH_3$ | CH | |
| L-13 | Q-4 | 0 | H | H | - | $CH_3$ | $CH_3$ | - | - | - | $OCH_3$ | $OCH_3$ | CH | |
| L-13 | Q-4 | 0 | H | H | - | H | $CO_2CH_3$ | - | - | - | $CH_3$ | $CH_3$ | CH | |
| L-13 | Q-4 | 0 | H | H | - | H | F | - | - | - | $CH_3$ | $OCH_3$ | CH | |
| L-13 | Q-4 | 0 | H | $OCH_3$ | - | H | Cl | - | - | - | $OCH_3$ | $CH_3$ | N | |
| L-13 | Q-4 | 0 | $CH_3$ | Cl | - | F | $CH_3$ | - | - | - | $CH_3$ | $OCH_3$ | CH | |
| L-13 | Q-4 | 1 | H | H | - | H | Cl | - | - | - | $CH_3$ | $OCH_3$ | CH | |
| L-13 | Q-4 | 1 | H | H | - | Cl | Cl | - | - | - | $OCH_3$ | $OCH_3$ | N | |
| L-13 | Q-4 | 1 | H | H | - | F | $CH_3$ | - | - | - | Cl | $OCH_3$ | CH | |
| L-13 | Q-5 | 0 | H | H | - | H | H | - | - | - | $CH_3$ | $CH_3$ | CH | |
| L-13 | Q-5 | 0 | H | H | - | H | Cl | - | - | - | $CH_3$ | $OCH_3$ | CH | |
| L-13 | Q-5 | 0 | H | H | - | Cl | Cl | - | - | - | $OCH_3$ | $OCH_3$ | CH | |
| L-13 | Q-5 | 0 | H | H | - | F | F | - | - | - | $CH_3$ | $CH_3$ | N | |
| L-13 | Q-5 | 0 | H | H | - | H | Br | - | - | - | $CH_3$ | $OCH_3$ | N | |
| L-13 | Q-5 | 0 | H | H | - | $CH_3$ | H | - | - | - | $OCH_3$ | $OCH_3$ | N | |
| L-13 | Q-5 | 0 | H | H | - | H | $OCH_3$ | - | - | - | Cl | $OCH_3$ | CH | |
| L-13 | Q-5 | 0 | H | H | - | H | F | - | - | - | $CH_3$ | $OCH_3$ | CH | |
| L-13 | Q-5 | 0 | H | H | - | Cl | Br | - | - | - | $OCH_3$ | $OCH_3$ | CH | |
| L-13 | Q-5 | 0 | H | H | - | Cl | $CH_3$ | - | - | - | $CH_3$ | $OCH_3$ | N | |
| L-13 | Q-5 | 0 | H | H | - | F | $CH_3$ | - | - | - | $OCH_3$ | $OCH_3$ | CH | |
| L-13 | Q-5 | 0 | H | H | - | H | Cl | - | - | - | $CH_3$ | $CH_3$ | N | |
| L-13 | Q-5 | 0 | H | H | - | H | $CH_3$ | - | - | - | $OCH_3$ | $OCH_3$ | CH | |
| L-13 | Q-5 | 0 | H | $CH_3$ | - | H | H | - | - | - | Cl | $OCH_3$ | CH | |
| L-13 | Q-5 | 0 | $CH_3$ | H | - | H | H | - | - | - | $CH_3$ | $OCH_3$ | N | |

Table II (continued)

| L | $Q_1$ | n | R | $R_1$ | $R_3$ | $R_7$ | $R_8$ | $R_9$ | $R_{10}$ | $R_{11}$ | X | Y | Z | m.p. (°C) |
|---|---|---|---|---|---|---|---|---|---|---|---|---|---|---|
| L-13 | Q-5 | 1 | H | H | - | F | F | - | - | - | $OCH_3$ | $OCH_3$ | CH | |
| L-13 | Q-5 | 1 | H | H | - | H | H | - | - | - | $OCH_3$ | $OCH_3$ | CH | |
| L-13 | Q-6 | 0 | H | H | - | H | H | - | - | - | $CH_3$ | $CH_3$ | CH | |
| L-13 | Q-6 | 0 | H | H | - | H | H | - | - | - | $OCH_3$ | $CH_3$ | CH | |
| L-13 | Q-6 | 0 | H | H | - | H | H | - | - | - | $OCH_3$ | $OCH_3$ | CH | |
| L-13 | Q-6 | 0 | H | H | - | Cl | H | - | - | - | $CH_3$ | $OCH_3$ | N | |
| L-13 | Q-6 | 0 | H | H | - | F | H | - | - | - | Cl | $OCH_3$ | CH | |
| L-13 | Q-6 | 0 | H | H | - | H | Br | - | - | - | $OCH_3$ | $OCH_3$ | CH | |
| L-13 | Q-6 | 0 | H | H | - | F | F | - | - | - | $OCH_3$ | $OCH_3$ | N | |
| L-13 | Q-6 | 0 | H | H | - | Cl | Cl | - | - | - | $CH_3$ | $OCH_3$ | CH | |
| L-13 | Q-6 | 0 | H | H | - | H | $CH_3$ | - | - | - | $CH_3$ | $OCH_3$ | CH | |
| L-13 | Q-6 | 0 | H | H | - | $CH_3$ | $CH_3$ | - | - | - | $OCH_3$ | $OCH_3$ | CH | |
| L-13 | Q-6 | 0 | H | H | - | H | $CO_2CH_3$ | - | - | - | $CH_3$ | $CH_3$ | CH | |
| L-13 | Q-6 | 0 | H | H | - | H | F | - | - | - | $OCH_3$ | $OCH_3$ | CH | |
| L-13 | Q-6 | 0 | H | $OCH_3$ | - | H | Cl | - | - | - | $OCH_3$ | $CH_3$ | N | |
| L-13 | Q-6 | 0 | $CH_3$ | Cl | - | F | $CH_3$ | - | - | - | $CH_3$ | $OCH_3$ | CH | |
| L-13 | Q-6 | 1 | H | H | - | H | Cl | - | - | - | $CH_3$ | $OCH_3$ | CH | |
| L-13 | Q-6 | 1 | H | H | - | Cl | Cl | - | - | - | $OCH_3$ | $OCH_3$ | N | |
| L-13 | Q-6 | 1 | H | H | - | F | $CH_3$ | - | - | - | Cl | $OCH_3$ | CH | |
| L-13 | Q-7 | 0 | H | H | - | H | H | - | - | - | $CH_3$ | $CH_3$ | CH | |
| L-13 | Q-7 | 0 | H | H | - | H | Cl | - | - | - | $CH_3$ | $OCH_3$ | CH | |
| L-13 | Q-7 | 0 | H | H | - | Cl | Cl | - | - | - | $OCH_3$ | $OCH_3$ | CH | |
| L-13 | Q-7 | 0 | H | H | - | F | F | - | - | - | $CH_3$ | $CH_3$ | N | |
| L-13 | Q-7 | 0 | H | H | - | H | Br | - | - | - | $CH_3$ | $OCH_3$ | N | |
| L-13 | Q-7 | 0 | H | H | - | $CH_3$ | H | - | - | - | $OCH_3$ | $OCH_3$ | N | |
| L-13 | Q-7 | 0 | H | H | - | H | $OCH_3$ | - | - | - | Cl | $OCH_3$ | CH | |
| L-13 | Q-7 | 0 | H | H | - | H | F | - | - | - | $CH_3$ | $OCH_3$ | CH | |
| L-13 | Q-7 | 0 | H | H | - | Cl | Br | - | - | - | $OCH_3$ | $OCH_3$ | CH | |
| L-13 | Q-7 | 0 | H | H | - | Cl | $CH_3$ | - | - | - | $CH_3$ | $OCH_3$ | N | |
| L-13 | Q-7 | 0 | H | H | - | F | $CH_3$ | - | - | - | $OCH_3$ | $OCH_3$ | CH | |
| L-13 | Q-7 | 0 | H | H | - | H | Cl | - | - | - | $CH_3$ | $CH_3$ | N | |
| L-13 | Q-7 | 0 | H | H | - | H | $CH_3$ | - | - | - | $OCH_3$ | $OCH_3$ | CH | |

Table II (continued)

| L | $Q_1$ | n | R | $R_1$ | $R_3$ | $R_7$ | $R_8$ | $R_9$ | $R_{10}$ | $R_{11}$ | X | Y | Z | m.p. (°C) |
|---|---|---|---|---|---|---|---|---|---|---|---|---|---|---|
| L-13 | Q-7 | 0 | H | $CH_3$ | - | H | H | - | - | - | Cl | $OCH_3$ | CH | |
| L-13 | Q-7 | 0 | $CH_3$ | H | - | H | H | - | - | - | $CH_3$ | $OCH_3$ | N | |
| L-13 | Q-7 | 1 | H | H | - | F | F | - | - | - | $OCH_3$ | $OCH_3$ | CH | |
| L-13 | Q-7 | 1 | H | H | - | H | H | - | - | - | $OCH_3$ | $OCH_3$ | CH | |
| L-13 | Q-8 | 0 | H | H | - | H | H | - | - | - | $CH_3$ | $CH_3$ | CH | |
| L-13 | Q-8 | 0 | H | H | - | H | H | - | - | - | $OCH_3$ | $CH_3$ | CH | |
| L-13 | Q-8 | 0 | H | H | - | H | H | - | - | - | $OCH_3$ | $OCH_3$ | CH | |
| L-13 | Q-8 | 0 | H | H | - | Cl | H | - | - | - | $CH_3$ | $OCH_3$ | N | |
| L-13 | Q-8 | 0 | H | H | - | F | H | - | - | - | Cl | $OCH_3$ | CH | |
| L-13 | Q-8 | 0 | H | H | - | H | Br | - | - | - | $OCH_3$ | $OCH_3$ | CH | |
| L-13 | Q-8 | 0 | H | H | - | F | F | - | - | - | $OCH_3$ | $OCH_3$ | N | |
| L-13 | Q-8 | 0 | H | H | - | Cl | Cl | - | - | - | $CH_3$ | $OCH_3$ | CH | |
| L-13 | Q-8 | 0 | H | H | - | H | $CH_3$ | - | - | - | $CH_3$ | $OCH_3$ | CH | |
| L-13 | Q-8 | 0 | H | H | - | $CH_3$ | $CH_3$ | - | - | - | $OCH_3$ | $OCH_3$ | CH | |
| L-13 | Q-8 | 0 | H | H | - | H | $CO_2CH_3$ | - | - | - | $CH_3$ | $CH_3$ | CH | |
| L-13 | Q-8 | 0 | H | H | - | H | F | - | - | - | $OCH_3$ | $OCH_3$ | CH | |
| L-13 | Q-8 | 0 | H | $OCH_3$ | - | H | Cl | - | - | - | $OCH_3$ | $CH_3$ | N | |
| L-13 | Q-8 | 0 | $CH_3$ | Cl | - | F | $CH_3$ | - | - | - | $CH_3$ | $OCH_3$ | CH | |
| L-13 | Q-8 | 1 | H | H | - | H | Cl | - | - | - | $CH_3$ | $OCH_3$ | CH | |
| L-13 | Q-8 | 1 | H | H | - | Cl | Cl | - | - | - | $OCH_3$ | $OCH_3$ | N | |
| L-13 | Q-8 | 1 | H | H | - | F | $CH_3$ | - | - | - | Cl | $OCH_3$ | CH | |
| L-13 | Q-9 | 0 | H | H | $CH_3$ | H | H | - | - | - | $CH_3$ | $CH_3$ | CH | |
| L-13 | Q-9 | 0 | H | H | $CH_3$ | H | Cl | - | - | - | $CH_3$ | $OCH_3$ | CH | |
| L-13 | Q-9 | 0 | H | H | $CH_3$ | Cl | Cl | - | - | - | $OCH_3$ | $OCH_3$ | CH | |
| L-13 | Q-9 | 0 | H | H | $CH_3$ | F | F | - | - | - | $CH_3$ | $CH_3$ | N | |
| L-13 | Q-9 | 0 | H | H | $CH_3$ | H | Br | - | - | - | $CH_3$ | $OCH_3$ | N | |
| L-13 | Q-9 | 0 | H | H | $CH_3$ | $CH_3$ | H | - | - | - | $OCH_3$ | $OCH_3$ | N | |
| L-13 | Q-9 | 0 | H | H | $CH_3$ | H | $OCH_3$ | - | - | - | Cl | $OCH_3$ | CH | |
| L-13 | Q-9 | 0 | H | H | $CH_3$ | H | F | - | - | - | $CH_3$ | $OCH_3$ | CH | |
| L-13 | Q-9 | 0 | H | H | $CH_2CH_3$ | Cl | Br | - | - | - | $OCH_3$ | $OCH_3$ | CH | |
| L-13 | Q-9 | 0 | H | H | $CH_2CH_3$ | Cl | $CH_3$ | - | - | - | $CH_3$ | $OCH_3$ | N | |
| L-13 | Q-9 | 0 | H | H | $CH_3$ | F | $CH_3$ | - | - | - | $OCH_3$ | $OCH_3$ | CH | |
| L-13 | Q-9 | 0 | H | H | $CH_3$ | H | Cl | - | - | - | $CH_3$ | $CH_3$ | N | |

Table II (continued)

| L | $Q_1$ | n | R | $R_1$ | $R_3$ | $R_7$ | $R_8$ | $R_9$ | $R_{10}$ | $R_{11}$ | X | Y | Z | m.p. (°C) |
|---|---|---|---|---|---|---|---|---|---|---|---|---|---|---|
| L-13 | Q-9 | 0 | H | H | $CH_3$ | H | $CH_3$ | - | - | - | $OCH_3$ | $OCH_3$ | CH | |
| L-13 | Q-9 | 0 | H | $CH_3$ | $CH_3$ | H | H | - | - | - | Cl | $OCH_3$ | CH | |
| L-13 | Q-9 | 0 | $CH_3$ | $OCH_3$ | $CH_3$ | H | H | - | - | - | $CH_3$ | $OCH_3$ | N | |
| L-13 | Q-9 | 1 | H | H | $CH_3$ | F | F | - | - | - | $OCH_3$ | $OCH_3$ | CH | |
| L-13 | Q-9 | 1 | H | H | $CH_3$ | H | H | - | - | - | $OCH_3$ | $OCH_3$ | CH | |
| L-13 | Q-10 | 0 | H | H | $CH_3$ | H | H | - | - | - | $CH_3$ | $CH_3$ | CH | |
| L-13 | Q-10 | 0 | H | H | $CH_3$ | H | H | - | - | - | $OCH_3$ | $OCH_3$ | CH | |
| L-13 | Q-10 | 0 | H | H | $CH_3$ | Cl | H | - | - | - | $CH_3$ | $OCH_3$ | N | |
| L-13 | Q-10 | 0 | H | H | $CH_3$ | F | H | - | - | - | Cl | $OCH_3$ | CH | |
| L-13 | Q-10 | 0 | H | H | $CH_3$ | F | Br | - | - | - | $OCH_3$ | $OCH_3$ | CH | |
| L-13 | Q-10 | 0 | H | H | $CH_3$ | F | F | - | - | - | $OCH_3$ | $OCH_3$ | N | |
| L-13 | Q-10 | 0 | H | H | $CH_3$ | Cl | Cl | - | - | - | $CH_3$ | $OCH_3$ | CH | |
| L-13 | Q-10 | 0 | H | H | $CH_3$ | H | $CH_3$ | - | - | - | $CH_3$ | $OCH_3$ | CH | |
| L-13 | Q-10 | 0 | H | H | $CH_3$ | $CH_3$ | $CH_3$ | - | - | - | $OCH_3$ | $OCH_3$ | CH | |
| L-13 | Q-10 | 0 | H | H | $CH_3$ | H | $CO_2CH_3$ | - | - | - | $CH_3$ | $CH_3$ | CH | |
| L-13 | Q-10 | 0 | H | H | $CH_3$ | H | F | - | - | - | $OCH_3$ | $OCH_3$ | CH | |
| L-13 | Q-10 | 0 | H | $OCH_3$ | $CH_3$ | H | Cl | - | - | - | $OCH_3$ | $CH_3$ | N | |
| L-13 | Q-10 | 0 | CH | Cl | $CH_3$ | F | $CH_3$ | - | - | - | $CH_3$ | $OCH_3$ | CH | |
| L-13 | Q-10 | 0 | $CH_3$ | Cl | $CH_3$ | F | $CH_3$ | - | - | - | $CH_3$ | $OCH_3$ | CH | |
| L-13 | Q-10 | 1 | H | H | $CH_3$ | H | Cl | - | - | - | $CH_3$ | $OCH_3$ | CH | |
| L-13 | Q-10 | 1 | H | H | $CH_3$ | Cl | Cl | - | - | - | $OCH_3$ | $OCH_3$ | N | |
| L-13 | Q-10 | 1 | H | H | $CH_3$ | F | $CH_3$ | - | - | - | Cl | $OCH_3$ | CH | |

**Table IIA**

General Structure II (wherein $R_{12}$ is OH and $R_{13}$ is H)

| L | $Q_1$ | n | R | $R_1$ | $R_2$ | $R_7$ | $R_8$ | $R_9$ | $R_{10}$ | $R_{11}$ | X | Y | Z | m.p. (°C) |
|---|---|---|---|---|---|---|---|---|---|---|---|---|---|---|
| L-9 | Q-1 | 1 | H | H | - | H | H | H | H | - | $CH_3$ | $CH_3$ | CH | |
| L-9 | Q-1 | 1 | H | H | - | H | Cl | H | H | - | $CH_3$ | $OCH_3$ | CH | |
| L-9 | Q-1 | 1 | H | H | - | Cl | Cl | H | H | - | $OCH_3$ | $OCH_3$ | CH | |
| L-9 | Q-1 | 1 | H | H | - | F | F | H | H | - | $CH_3$ | $CH_3$ | N | |
| L-9 | Q-1 | 1 | H | H | - | H | Br | H | H | - | $CH_3$ | $OCH_3$ | N | |
| L-9 | Q-1 | 1 | H | H | - | $CH_3$ | H | H | H | - | $OCH_3$ | $OCH_3$ | N | |
| L-9 | Q-1 | 1 | H | H | - | H | $OCH_3$ | H | H | - | Cl | $OCH_3$ | CH | |
| L-9 | Q-1 | 1 | H | H | - | H | F | H | H | - | $CH_3$ | $OCH_3$ | CH | |
| L-9 | Q-1 | 1 | H | H | - | Cl | Br | H | H | - | $OCH_3$ | $OCH_3$ | CH | |
| L-9 | Q-1 | 1 | H | H | - | Cl | $CH_3$ | H | H | - | $CH_3$ | $OCH_3$ | N | |
| L-9 | Q-1 | 1 | H | H | - | F | $CH_3$ | H | H | - | $OCH_3$ | $OCH_3$ | CH | |
| L-9 | Q-1 | 1 | H | H | - | H | Cl | Cl | H | - | $CH_3$ | $CH_3$ | N | |
| L-9 | Q-1 | 1 | H | H | - | H | $CH_3$ | F | F | - | $OCH_3$ | $OCH_3$ | CH | |
| L-9 | Q-1 | 1 | H | $CH_3$ | - | H | H | H | H | - | Cl | $OCH_3$ | CH | |
| L-9 | Q-1 | 1 | $CH_3$ | H | - | H | H | H | H | - | $CH_3$ | $OCH_3$ | N | |
| L-9 | Q-1 | 1 | H | H | - | F | F | H | H | - | $OCH_3$ | $OCH_3$ | CH | |
| L-9 | Q-1 | 1 | H | H | - | H | H | H | H | - | $OCH_3$ | $OCH_3$ | CH | |
| L-9 | Q-2 | 1 | H | H | - | H | H | - | - | H | $CH_3$ | $CH_3$ | CH | |
| L-9 | Q-2 | 1 | H | H | - | H | H | - | - | H | $OCH_3$ | $CH_3$ | CH | |
| L-9 | Q-2 | 1 | H | H | - | H | H | - | - | H | $OCH_3$ | $OCH_3$ | CH | |
| L-9 | Q-2 | 1 | H | H | - | Cl | H | - | - | H | $CH_3$ | $OCH_3$ | N | |
| L-9 | Q-2 | 1 | H | H | - | F | H | - | - | H | Cl | $OCH_3$ | CH | |
| L-9 | Q-2 | 1 | H | H | - | H | Br | - | - | H | $OCH_3$ | $OCH_3$ | CH | |
| L-9 | Q-2 | 1 | H | H | - | F | F | - | - | H | $OCH_3$ | $OCH_3$ | N | |
| L-9 | Q-2 | 1 | H | H | - | Cl | Cl | - | - | H | $CH_3$ | $OCH_3$ | CH | |
| L-9 | Q-2 | 1 | H | H | - | H | $CH_3$ | - | - | H | $CH_3$ | $OCH_3$ | CH | |
| L-9 | Q-2 | 1 | H | H | - | $CH_3$ | $CH_3$ | - | - | H | $OCH_3$ | $OCH_3$ | CH | |
| L-9 | Q-2 | 1 | H | H | - | H | $CO_2CH_3$ | - | - | H | $CH_3$ | $CH_3$ | CH | |
| L-9 | Q-2 | 1 | H | H | - | H | F | - | - | $CH_3$ | $OCH_3$ | $OCH_3$ | CH | |
| L-9 | Q-2 | 1 | H | $OCH_3$ | - | H | Cl | - | - | H | $OCH_3$ | $CH_3$ | N | |
| L-9 | Q-2 | 1 | $CH_3$ | Cl | - | F | $CH_3$ | - | - | $CH_3$ | $CH_3$ | $OCH_3$ | CH | |

Table IIB

General Structure II (wherein $R_{12}$ is $OCH_3$ and $R_{13}$ is H)

| L | $Q_1$ | n | R | $R_1$ | $R_2$ | $R_7$ | $R_8$ | $R_9$ | $R_{10}$ | $R_{11}$ | X | Y | Z | m.p. (°C) |
|---|---|---|---|---|---|---|---|---|---|---|---|---|---|---|
| L-9 | Q-1 | 1 | H | H | - | H | H | H | H | - | $CH_3$ | $CH_3$ | CH | |
| L-9 | Q-1 | 1 | H | H | - | H | Cl | H | H | - | $CH_3$ | $OCH_3$ | CH | |
| L-9 | Q-1 | 1 | H | H | - | Cl | Cl | H | H | - | $OCH_3$ | $OCH_3$ | CH | |
| L-9 | Q-1 | 1 | H | H | - | F | F | H | H | - | $CH_3$ | $CH_3$ | N | |
| L-9 | Q-1 | 1 | H | H | - | H | Br | H | H | - | $CH_3$ | $OCH_3$ | N | |
| L-9 | Q-1 | 1 | H | H | - | $CH_3$ | H | H | H | - | $OCH_3$ | $OCH_3$ | N | |
| L-9 | Q-1 | 1 | H | H | - | H | $OCH_3$ | H | H | - | Cl | $OCH_3$ | CH | |
| L-9 | Q-1 | 1 | H | H | - | H | F | H | H | - | $CH_3$ | $OCH_3$ | CH | |
| L-9 | Q-1 | 1 | H | H | - | Cl | Br | H | H | - | $OCH_3$ | $OCH_3$ | CH | |
| L-9 | Q-1 | 1 | H | H | - | Cl | $CH_3$ | H | H | - | $CH_3$ | $OCH_3$ | N | |
| L-9 | Q-1 | 1 | H | H | - | F | $CH_3$ | H | H | - | $OCH_3$ | $OCH_3$ | CH | |
| L-9 | Q-1 | 1 | H | H | - | H | Cl | Cl | H | - | $CH_3$ | $CH_3$ | N | |
| L-9 | Q-1 | 1 | H | H | - | H | $CH_3$ | F | F | - | $OCH_3$ | $OCH_3$ | CH | |
| L-9 | Q-1 | 1 | H | $CH_3$ | - | H | H | H | H | - | Cl | $OCH_3$ | CH | |
| L-9 | Q-1 | 1 | $CH_3$ | H | - | H | H | H | H | - | $CH_3$ | $OCH_3$ | N | |
| L-9 | Q-1 | 1 | H | H | - | F | F | H | H | - | $OCH_3$ | $OCH_3$ | CH | |
| L-9 | Q-1 | 1 | H | H | - | H | H | H | H | - | $OCH_3$ | $OCH_3$ | CH | |
| L-9 | Q-2 | 1 | H | H | - | H | H | - | - | H | $CH_3$ | $CH_3$ | CH | |
| L-9 | Q-2 | 1 | H | H | - | H | H | - | - | H | $OCH_3$ | $CH_3$ | CH | |
| L-9 | Q-2 | 1 | H | H | - | H | H | - | - | H | $OCH_3$ | $OCH_3$ | CH | |
| L-9 | Q-2 | 1 | H | H | - | Cl | H | - | - | H | $CH_3$ | $OCH_3$ | N | |
| L-9 | Q-2 | 1 | H | H | - | F | H | - | - | H | Cl | $OCH_3$ | CH | |
| L-9 | Q-2 | 1 | H | H | - | H | Br | - | - | H | $OCH_3$ | $OCH_3$ | CH | |
| L-9 | Q-2 | 1 | H | H | - | F | F | - | - | H | $OCH_3$ | $OCH_3$ | N | |
| L-9 | Q-2 | 1 | H | H | - | Cl | Cl | - | - | H | $CH_3$ | $OCH_3$ | CH | |
| L-9 | Q-2 | 1 | H | H | - | H | $CH_3$ | - | - | H | $CH_3$ | $OCH_3$ | CH | |
| L-9 | Q-2 | 1 | H | H | - | $CH_3$ | $CH_3$ | - | - | H | $OCH_3$ | $OCH_3$ | CH | |
| L-9 | Q-2 | 1 | H | H | - | H | $CO_2CH_3$ | - | - | H | $CH_3$ | $CH_3$ | CH | |
| L-9 | Q-2 | 1 | H | H | - | H | F | - | - | $CH_3$ | $OCH_3$ | $OCH_3$ | CH | |
| L-9 | Q-2 | 1 | H | $OCH_3$ | - | H | Cl | - | - | H | $OCH_3$ | $CH_3$ | N | |
| L-9 | Q-2 | 1 | $CH_3$ | Cl | - | F | $CH_3$ | - | - | $CH_3$ | $CH_3$ | $OCH_3$ | CH | |

Table IIC

General Structure II (wherein $R_{12}$ is OH and $R_{13}$ is H)

| L | $Q_1$ | n | R | $R_1$ | $R_2$ | $R_7$ | $R_8$ | $R_9$ | $R_{10}$ | $R_{11}$ | X | Y | Z | m.p. (°C) |
|---|---|---|---|---|---|---|---|---|---|---|---|---|---|---|
| L-9 | Q-4 | 1 | H | H | - | H | H | - | - | - | $CH_3$ | $CH_3$ | CH | |
| L-9 | Q-4 | 1 | H | H | - | H | Cl | - | - | - | $CH_3$ | $OCH_3$ | CH | |
| L-9 | Q-4 | 1 | H | H | - | Cl | Cl | - | - | - | $OCH_3$ | $OCH_3$ | CH | |
| L-9 | Q-4 | 1 | H | H | - | F | F | - | - | - | $CH_3$ | $CH_3$ | N | |
| L-9 | Q-4 | 1 | H | H | - | H | Br | - | - | - | $CH_3$ | $OCH_3$ | N | |
| L-9 | Q-4 | 1 | H | H | - | $CH_3$ | H | - | - | - | $OCH_3$ | $OCH_3$ | N | |
| L-9 | Q-4 | 1 | H | H | - | H | $OCH_3$ | - | - | - | Cl | $OCH_3$ | CH | |
| L-9 | Q-4 | 1 | H | H | - | H | F | - | - | - | $CH_3$ | $OCH_3$ | CH | |
| L-9 | Q-4 | 1 | H | H | - | Cl | Br | - | - | - | $OCH_3$ | $OCH_3$ | CH | |
| L-9 | Q-4 | 1 | H | H | - | Cl | $CH_3$ | - | - | - | $CH_3$ | $OCH_3$ | N | |
| L-9 | Q-4 | 1 | H | H | - | F | $CH_3$ | - | - | - | $OCH_3$ | $OCH_3$ | CH | |
| L-9 | Q-4 | 1 | H | H | - | H | Cl | - | - | - | $CH_3$ | $CH_3$ | N | |
| L-9 | Q-4 | 1 | H | H | - | H | $CH_3$ | - | - | - | $OCH_3$ | $OCH_3$ | CH | |
| L-9 | Q-4 | 1 | H | $CH_3$ | - | H | H | - | - | - | Cl | $OCH_3$ | CH | |
| L-9 | Q-4 | 1 | $CH_3$ | H | - | H | H | - | - | - | $CH_3$ | $OCH_3$ | N | |
| L-9 | Q-4 | 1 | H | H | - | F | F | - | - | - | $OCH_3$ | $OCH_3$ | CH | |
| L-9 | Q-4 | 1 | H | H | - | H | H | - | - | - | $OCH_3$ | $OCH_3$ | CH | |
| L-9 | Q-4 | 1 | H | H | - | H | H | - | - | H | $CH_3$ | $CH_3$ | CH | |
| L-9 | Q-4 | 1 | H | H | - | H | H | - | - | H | $OCH_3$ | $CH_3$ | CH | |
| L-9 | Q-4 | 1 | H | H | - | H | H | - | - | H | $OCH_3$ | $OCH_3$ | CH | |
| L-9 | Q-4 | 1 | H | H | - | Cl | H | - | - | H | $CH_3$ | $OCH_3$ | N | |
| L-9 | Q-4 | 1 | H | H | - | F | H | - | - | H | Cl | $OCH_3$ | CH | |
| L-9 | Q-4 | 1 | H | H | - | H | Br | - | - | H | $OCH_3$ | $OCH_3$ | CH | |
| L-9 | Q-4 | 1 | H | H | - | F | F | - | - | H | $OCH_3$ | $OCH_3$ | N | |
| L-9 | Q-4 | 1 | H | H | - | Cl | Cl | - | - | H | $CH_3$ | $OCH_3$ | CH | |
| L-9 | Q-4 | 1 | H | H | - | H | $CH_3$ | - | - | H | $CH_3$ | $OCH_3$ | CH | |
| L-9 | Q-4 | 1 | H | H | - | $CH_3$ | $CH_3$ | - | - | H | $OCH_3$ | $OCH_3$ | CH | |
| L-9 | Q-4 | 1 | H | H | - | H | $CO_2CH_3$ | - | - | H | $CH_3$ | $CH_3$ | CH | |
| L-9 | Q-4 | 1 | H | H | - | H | F | - | - | $CH_3$ | $OCH_3$ | $OCH_3$ | CH | |
| L-9 | Q-4 | 1 | H | $OCH_3$ | - | H | Cl | - | - | H | $OCH_3$ | $CH_3$ | N | |
| L-9 | Q-4 | 1 | $CH_3$ | Cl | - | F | $CH_3$ | - | - | $CH_3$ | $CH_3$ | $OCH_3$ | CH | |

Table IIC (Continued)

| L | $Q_1$ | n | R | $R_1$ | $R_3$ | $R_7$ | $R_8$ | $R_9$ | $R_{10}$ | $R_{11}$ | X | Y | Z | m.p. (°C) |
|---|---|---|---|---|---|---|---|---|---|---|---|---|---|---|
| L-11 | Q-1 | 1 | $CH_3$ | H | - | H | H | H | H | - | $CH_3$ | $OCH_3$ | N | |
| L-11 | Q-1 | 1 | H | H | - | F | F | H | H | - | $OCH_3$ | $OCH_3$ | CH | |
| L-11 | Q-1 | 1 | H | H | - | H | H | H | H | - | $OCH_3$ | $OCH_3$ | CH | |
| L-11 | Q-2 | 1 | H | H | - | H | H | - | - | H | $CH_3$ | $CH_3$ | CH | |
| L-11 | Q-2 | 1 | H | H | - | H | H | - | - | H | $OCH_3$ | $CH_3$ | CH | |
| L-11 | Q-2 | 1 | H | H | - | H | H | - | - | H | $OCH_3$ | $OCH_3$ | CH | |
| L-11 | Q-2 | 1 | H | H | - | Cl | H | - | - | H | $CH_3$ | $OCH_3$ | N | |
| L-11 | Q-2 | 1 | H | H | - | F | H | - | - | H | Cl | $OCH_3$ | CH | |
| L-11 | Q-2 | 1 | H | H | - | H | Br | - | - | H | $OCH_3$ | $OCH_3$ | CH | |
| L-11 | Q-2 | 1 | H | H | - | F | F | - | - | H | $OCH_3$ | $OCH_3$ | N | |
| L-11 | Q-2 | 1 | H | H | - | Cl | Cl | - | - | H | $CH_3$ | $OCH_3$ | CH | |
| L-11 | Q-2 | 1 | H | H | - | H | $CH_3$ | - | - | H | $CH_3$ | $OCH_3$ | CH | |
| L-11 | Q-2 | 1 | H | H | - | $CH_3$ | $CH_3$ | - | - | H | $OCH_3$ | $OCH_3$ | CH | |
| L-11 | Q-2 | 1 | H | H | - | H | $CO_2CH_3$ | - | - | H | $CH_3$ | $CH_3$ | CH | |
| L-11 | Q-2 | 1 | H | H | - | H | F | - | - | $CH_3$ | $OCH_3$ | $OCH_3$ | CH | |
| L-11 | Q-2 | 1 | H | $OCH_3$ | - | H | Cl | - | - | H | $OCH_3$ | $CH_3$ | N | |
| L-11 | Q-2 | 1 | $CH_3$ | Cl | - | F | $CH_3$ | - | - | $CH_3$ | $CH_3$ | $OCH_3$ | CH | |
| L-11 | Q-2 | 1 | H | H | - | H | Cl | - | - | H | $CH_3$ | $OCH_3$ | CH | |
| L-11 | Q-2 | 1 | H | H | - | Cl | Cl | - | - | H | $OCH_3$ | $OCH_3$ | N | |
| L-11 | Q-2 | 1 | H | H | - | F | $CH_3$ | - | - | H | Cl | $OCH_3$ | CH | |
| L-11 | Q-3 | 1 | H | H | $CH_3$ | H | H | - | - | H | $CH_3$ | $CH_3$ | CH | |
| L-11 | Q-3 | 1 | H | H | $CH_3$ | H | Cl | - | - | H | $CH_3$ | $OCH_3$ | CH | |
| L-11 | Q-3 | 1 | H | H | $CH_3$ | Cl | Cl | - | - | H | $OCH_3$ | $OCH_3$ | CH | |
| L-11 | Q-3 | 1 | H | H | $CH_3$ | F | F | - | - | H | $CH_3$ | $CH_3$ | N | |
| L-11 | Q-3 | 1 | H | H | $CH_3$ | H | Br | - | - | H | $CH_3$ | $OCH_3$ | N | |
| L-11 | Q-3 | 1 | H | H | $CH_3$ | $CH_3$ | H | - | - | H | $OCH_3$ | $OCH_3$ | N | |
| L-11 | Q-3 | 1 | H | H | $CH_3$ | H | $OCH_3$ | - | - | H | Cl | $OCH_3$ | CH | |
| L-11 | Q-3 | 1 | H | H | $CH_3$ | H | F | - | - | H | $CH_3$ | $OCH_3$ | CH | |
| L-11 | Q-3 | 1 | H | H | $CH_3$ | Cl | Br | - | - | H | $OCH_3$ | $OCH_3$ | CH | |
| L-11 | Q-3 | 1 | H | H | $CH_3$ | Cl | $CH_3$ | - | - | H | $CH_3$ | $OCH_3$ | N | |
| L-11 | Q-3 | 1 | H | H | $CH_3$ | F | $CH_3$ | - | - | H | $OCH_3$ | $OCH_3$ | CH | |
| L-11 | Q-3 | 1 | H | H | $CH_3$ | Cl | Cl | - | - | H | $CH_3$ | $CH_3$ | N | |

Table IIC (Continued)

| L | $Q_1$ | n | R | $R_1$ | $R_3$ | $R_7$ | $R_8$ | $R_9$ | $R_{10}$ | $R_{11}$ | X | Y | Z | m.p. (°C) |
|---|---|---|---|---|---|---|---|---|---|---|---|---|---|---|
| L-12 | Q-10 | 1 | H | H | $CH_3$ | F | H | - | - | - | Cl | $OCH_3$ | CH | |
| L-12 | Q-10 | 1 | H | H | $CH_3$ | H | Br | - | - | - | $OCH_3$ | $OCH_3$ | CH | |
| L-12 | Q-10 | 1 | H | H | $CH_3$ | F | F | - | - | - | $OCH_3$ | $OCH_3$ | N | |
| L-12 | Q-10 | 1 | H | H | $CH_2CH_3$ | Cl | Cl | - | - | - | $CH_3$ | $OCH_3$ | CH | |
| L-12 | Q-10 | 1 | H | H | $CH_3$ | H | $CH_3$ | - | - | - | $CH_3$ | $OCH_3$ | CH | |
| L-12 | Q-10 | 1 | H | H | $CH_3$ | $CH_3$ | $CH_3$ | - | - | - | $OCH_3$ | $OCH_3$ | CH | |
| L-12 | Q-10 | 1 | H | H | $CH_3$ | H | $CO_2CH_3$ | - | - | - | $CH_3$ | $CH_3$ | CH | |
| L-12 | Q-10 | 1 | H | H | $CH_3$ | H | F | - | - | - | $OCH_3$ | $OCH_3$ | CH | |
| L-12 | Q-10 | 1 | H | $OCH_3$ | $CH_3$ | H | Cl | - | - | - | $OCH_3$ | $CH_3$ | N | |
| L-12 | Q-10 | 1 | $CH_3$ | Cl | $CH_3$ | F | $CH_3$ | - | - | - | $CH_3$ | $OCH_3$ | CH | |
| L-12 | Q-10 | 1 | H | H | $CH_3$ | H | Cl | - | - | - | $CH_3$ | $OCH_3$ | CH | |
| L-12 | Q-10 | 1 | H | H | $CH_3$ | Cl | Cl | - | - | - | $OCH_3$ | $OCH_3$ | N | |
| L-12 | Q-10 | 1 | H | H | $CH_3$ | F | $CH_3$ | - | - | - | Cl | $OCH_3$ | CH | |
| L-13 | Q-1 | 1 | H | H | - | H | H | H | H | - | $CH_3$ | $CH_3$ | CH | |
| L-13 | Q-1 | 1 | H | H | - | H | Cl | H | H | - | $CH_3$ | $OCH_3$ | CH | |
| L-13 | Q-1 | 1 | H | H | - | Cl | Cl | H | H | - | $OCH_3$ | $OCH_3$ | CH | |
| L-13 | Q-1 | 1 | H | H | - | F | F | H | H | - | $CH_3$ | $CH_3$ | N | |
| L-13 | Q-1 | 1 | H | H | - | H | Br | H | H | - | $CH_3$ | $OCH_3$ | N | |
| L-13 | Q-1 | 1 | H | H | - | $CH_3$ | H | H | H | - | $OCH_3$ | $OCH_3$ | N | |
| L-13 | Q-1 | 1 | H | H | - | H | $OCH_3$ | H | H | - | Cl | $OCH_3$ | CH | |
| L-13 | Q-1 | 1 | H | H | - | H | F | H | H | - | $CH_3$ | Cl | CH | |
| L-13 | Q-1 | 1 | H | H | - | Cl | Br | H | H | - | $OCH_3$ | $OCH_3$ | CH | |
| L-13 | Q-1 | 1 | H | H | - | Cl | $CH_3$ | H | H | - | $CH_3$ | $OCH_3$ | N | |
| L-13 | Q-1 | 1 | H | H | - | F | $CH_3$ | H | H | - | $OCH_3$ | $OCH_3$ | CH | |
| L-13 | Q-1 | 1 | H | H | - | H | Cl | Cl | H | - | $CH_3$ | $CH_3$ | N | |
| L-13 | Q-1 | 1 | H | H | - | H | $CH_3$ | F | F | - | $OCH_3$ | $OCH_3$ | CH | |
| L-13 | Q-1 | 1 | H | $CH_3$ | - | H | H | H | H | - | Cl | $OCH_3$ | CH | |
| L-13 | Q-1 | 1 | $CH_3$ | H | - | H | H | H | H | - | $CH_3$ | $OCH_3$ | N | |
| L-13 | Q-1 | 1 | H | H | - | F | F | H | H | - | $OCH_3$ | $OCH_3$ | CH | |
| L-13 | Q-1 | 1 | H | H | - | H | H | H | H | - | $OCH_3$ | $OCH_3$ | CH | |
| L-13 | Q-2 | 1 | H | H | - | H | H | - | - | H | $CH_3$ | $CH_3$ | CH | |
| L-13 | Q-2 | 1 | H | H | - | H | H | - | - | H | $OCH_3$ | $CH_3$ | CH | |

Table IID

General Structure IIa (where $R_{12}$ and $R_{13}$ are H)

| L | $Q_1$ | n | R | $R_1$ | $R_2$ | $R_7$ | $R_8$ | $R_9$ | $R_{10}$ | $R_{11}$ | X | Y | Z | m.p. (°C) |
|---|---|---|---|---|---|---|---|---|---|---|---|---|---|---|
| L-9 | Q-1 | 0 | H | H | - | H | H | H | H | - | $CH_3$ | $CH_3$ | CH | |
| L-9 | Q-1 | 0 | H | H | - | H | Cl | H | H | - | $CH_3$ | $OCH_3$ | CH | |
| L-9 | Q-1 | 0 | H | H | - | Cl | Cl | H | H | - | $OCH_3$ | $OCH_3$ | CH | |
| L-9 | Q-1 | 0 | H | H | - | F | F | H | H | - | $CH_3$ | $CH_3$ | N | |
| L-9 | Q-1 | 0 | H | H | - | H | Br | H | H | - | $CH_3$ | $OCH_3$ | N | |
| L-9 | Q-1 | 0 | H | H | - | $CH_3$ | H | H | H | - | $OCH_3$ | $OCH_3$ | N | |
| L-9 | Q-1 | 0 | H | H | - | H | $OCH_3$ | H | H | - | Cl | $OCH_3$ | CH | |
| L-9 | Q-1 | 0 | H | H | - | H | F | H | H | - | $CH_3$ | $OCH_3$ | CH | |
| L-9 | Q-1 | 0 | H | H | - | Cl | Br | H | H | - | $OCH_3$ | $OCH_3$ | CH | |
| L-9 | Q-1 | 0 | H | H | - | Cl | $CH_3$ | H | H | - | $CH_3$ | $OCH_3$ | N | |
| L-9 | Q-1 | 0 | H | H | - | F | $CH_3$ | H | H | - | $OCH_3$ | $OCH_3$ | CH | |
| L-9 | Q-1 | 0 | H | H | - | H | Cl | Cl | H | - | $CH_3$ | $CH_3$ | N | |
| L-9 | Q-1 | 0 | H | H | - | H | $CH_3$ | F | F | - | $OCH_3$ | $OCH_3$ | CH | |
| L-9 | Q-1 | 0 | H | $CH_3$ | - | H | H | H | H | - | Cl | $OCH_3$ | CH | |
| L-9 | Q-1 | 0 | $CH_3$ | H | - | H | H | H | H | - | $CH_3$ | $OCH_3$ | N | |
| L-9 | Q-1 | 1 | H | H | - | F | F | H | H | - | $OCH_3$ | $OCH_3$ | CH | |
| L-9 | Q-1 | 1 | H | H | - | H | H | H | H | - | $OCH_3$ | $OCH_3$ | CH | |
| L-9 | Q-2 | 0 | H | H | - | H | H | - | - | H | $CH_3$ | $CH_3$ | CH | |
| L-9 | Q-2 | 0 | H | H | - | H | H | - | - | H | $OCH_3$ | $CH_3$ | CH | |
| L-9 | Q-2 | 0 | H | H | - | H | H | - | - | H | $OCH_3$ | $OCH_3$ | CH | |
| L-9 | Q-2 | 0 | H | H | - | Cl | H | - | - | H | $CH_3$ | $OCH_3$ | N | |
| L-9 | Q-2 | 0 | H | H | - | F | H | - | - | H | Cl | $OCH_3$ | CH | |
| L-9 | Q-2 | 0 | H | H | - | H | Br | - | - | H | $OCH_3$ | $OCH_3$ | CH | |
| L-9 | Q-2 | 0 | H | H | - | F | F | - | - | H | $OCH_3$ | $OCH_3$ | N | |
| L-9 | Q-2 | 0 | H | H | - | Cl | Cl | - | - | H | $CH_3$ | $OCH_3$ | CH | |
| L-9 | Q-2 | 0 | H | H | - | H | $CH_3$ | - | - | H | $CH_3$ | $OCH_3$ | CH | |
| L-9 | Q-2 | 0 | H | H | - | $CH_3$ | $CH_3$ | - | - | H | $OCH_3$ | $OCH_3$ | CH | |
| L-9 | Q-2 | 0 | H | H | - | H | $CO_2CH_3$ | - | - | H | $CH_3$ | $CH_3$ | CH | |
| L-9 | Q-2 | 0 | H | H | - | H | F | - | - | $CH_3$ | $OCH_3$ | $OCH_3$ | CH | |
| L-9 | Q-2 | 0 | H | $OCH_3$ | - | H | Cl | - | - | H | $OCH_3$ | $CH_3$ | N | |
| L-9 | Q-2 | 0 | $CH_3$ | Cl | - | F | $CH_3$ | - | - | $CH_3$ | $CH_3$ | $OCH_3$ | CH | |

Table IID (continued)

| L | $Q_1$ | n | R | $R_1$ | $R_3$ | $R_7$ | $R_8$ | $R_9$ | $R_{10}$ | $R_{11}$ | X | Y | Z | m.p. (°C) |
|---|---|---|---|---|---|---|---|---|---|---|---|---|---|---|
| L-9 | Q-2 | 1 | H | H | - | H | Cl | - | - | H | $CH_3$ | $OCH_3$ | CH | |
| L-9 | Q-2 | 1 | H | H | - | Cl | Cl | - | - | H | $OCH_3$ | $OCH_3$ | N | |
| L-9 | Q-2 | 1 | H | H | - | F | $CH_3$ | - | - | H | Cl | $OCH_3$ | CH | |
| L-9 | Q-3 | 0 | H | H | $CH_3$ | H | H | - | - | H | $CH_3$ | $CH_3$ | CH | |
| L-9 | Q-3 | 0 | H | H | $CH_3$ | H | Cl | - | - | H | $CH_3$ | $OCH_3$ | CH | |
| L-9 | Q-3 | 0 | H | H | $CH_3$ | Cl | Cl | - | - | H | $OCH_3$ | $OCH_3$ | CH | |
| L-9 | Q-3 | 0 | H | H | $CH_3$ | F | F | - | - | H | $CH_3$ | $CH_3$ | N | |
| L-9 | Q-3 | 0 | H | H | $CH_3$ | H | Br | - | - | H | $CH_3$ | $OCH_3$ | N | |
| L-9 | Q-3 | 0 | H | H | $CH_3$ | $CH_3$ | H | - | - | H | $OCH_3$ | $OCH_3$ | N | |
| L-9 | Q-3 | 0 | H | H | $CH_3$ | H | $OCH_3$ | - | - | H | Cl | $OCH_3$ | CH | |
| L-9 | Q-3 | 0 | H | H | $CH_3$ | H | F | - | - | H | $CH_3$ | $OCH_3$ | CH | |
| L-9 | Q-3 | 0 | H | H | $CH_3$ | Cl | Br | - | - | H | $OCH_3$ | $OCH_3$ | CH | |
| L-9 | Q-3 | 0 | H | H | $CH_3$ | Cl | $CH_3$ | - | - | H | $CH_3$ | $OCH_3$ | N | |
| L-9 | Q-3 | 0 | H | H | $CH_3$ | F | $CH_3$ | - | - | $CH_3$ | $OCH_3$ | $OCH_3$ | CH | |
| L-9 | Q-3 | 0 | H | H | $CH_3$ | H | Cl | - | - | H | $CH_3$ | $CH_3$ | N | |
| L-9 | Q-3 | 0 | H | H | $CH_3$ | H | $CH_3$ | - | - | H | $OCH_3$ | $OCH_3$ | CH | |
| L-9 | Q-3 | 0 | H | $CH_3$ | $CH_3$ | H | H | - | - | H | Cl | $OCH_3$ | CH | |
| L-9 | Q-3 | 0 | $CH_3$ | H | $CH_3$ | H | H | - | - | H | $CH_3$ | $OCH_3$ | N | |
| L-9 | Q-3 | 1 | H | H | $CH_3$ | F | F | - | - | H | $OCH_3$ | $OCH_3$ | CH | |
| L-9 | Q-3 | 1 | H | H | $CH_3$ | H | H | - | - | H | $OCH_3$ | $OCH_3$ | CH | |
| L-9 | Q-4 | 0 | H | H | - | H | H | - | - | - | $CH_3$ | $CH_3$ | CH | |
| L-9 | Q-4 | 0 | H | H | - | H | H | - | - | - | $OCH_3$ | $CH_3$ | CH | |
| L-9 | Q-4 | 0 | H | H | - | H | H | - | - | - | $OCH_3$ | $OCH_3$ | CH | |
| L-9 | Q-4 | 0 | H | H | - | Cl | H | - | - | - | $CH_3$ | $OCH_3$ | N | |
| L-9 | Q-4 | 0 | H | H | - | F | H | - | - | - | Cl | $OCH_3$ | CH | |
| L-9 | Q-4 | 0 | H | H | - | H | Br | - | - | - | $OCH_3$ | $OCH_3$ | CH | |
| L-9 | Q-4 | 0 | H | H | - | F | F | - | - | - | $OCH_3$ | $OCH_3$ | N | |
| L-9 | Q-4 | 0 | H | H | - | Cl | Cl | - | - | - | $CH_3$ | $OCH_3$ | CH | |
| L-9 | Q-4 | 0 | H | H | - | H | $CH_3$ | - | - | - | $CH_3$ | $OCH_3$ | CH | |
| L-9 | Q-4 | 0 | H | H | - | $CH_3$ | $CH_3$ | - | - | - | $OCH_3$ | $OCH_3$ | CH | |
| L-9 | Q-4 | 0 | H | H | - | H | $CO_2CH_3$ | - | - | - | $CH_3$ | $CH_3$ | CH | |
| L-9 | Q-4 | 0 | H | H | - | H | F | - | - | - | $OCH_3$ | $OCH_3$ | CH | |
| L-9 | Q-4 | 0 | H | $OCH_3$ | - | H | Cl | - | - | - | $OCH_3$ | $CH_3$ | N | |

Table IID (continued)

| L | $Q_1$ | n | R | $R_1$ | $R_3$ | $R_7$ | $R_8$ | $R_9$ | $R_{10}$ | $R_{11}$ | X | Y | Z | m.p. (°C) |
|---|---|---|---|---|---|---|---|---|---|---|---|---|---|---|
| L-9 | Q-8 | 0 | H | H | - | H | $CO_2CH_3$ | - | - | - | $CH_3$ | $CH_3$ | CH | |
| L-9 | Q-8 | 0 | H | H | - | H | F | - | - | - | $OCH_3$ | $OCH_3$ | CH | |
| L-9 | Q-8 | 0 | H | $OCH_3$ | - | H | Cl | - | - | - | $OCH_3$ | $CH_3$ | N | |
| L-9 | Q-8 | 0 | $CH_3$ | Cl | - | F | $CH_3$ | - | - | - | $CH_3$ | $OCH_3$ | CH | |
| L-9 | Q-8 | 1 | H | H | - | H | Cl | - | - | - | $CH_3$ | $OCH_3$ | CH | |
| L-9 | Q-8 | 1 | H | H | - | Cl | Cl | - | - | - | $OCH_3$ | $OCH_3$ | N | |
| L-9 | Q-8 | 1 | H | H | - | F | $CH_3$ | - | - | - | Cl | $OCH_3$ | CH | |
| L-9 | Q-9 | 0 | H | H | $CH_3$ | H | H | - | - | - | $CH_3$ | $CH_3$ | CH | |
| L-9 | Q-9 | 0 | H | H | $CH_3$ | H | Cl | - | - | - | $CH_3$ | $OCH_3$ | CH | |
| L-9 | Q-9 | 0 | H | H | $CH_3$ | Cl | Cl | - | - | - | $OCH_3$ | $OCH_3$ | CH | |
| L-9 | Q-9 | 0 | H | H | $CH_3$ | F | F | - | - | - | $CH_3$ | $CH_3$ | N | |
| L-9 | Q-9 | 0 | H | H | $CH_3$ | H | Br | - | - | - | $CH_3$ | $OCH_3$ | N | |
| L-9 | Q-9 | 0 | H | H | $CH_3$ | $CH_3$ | H | - | - | - | $OCH_3$ | $OCH_3$ | N | |
| L-9 | Q-9 | 0 | H | H | $CH_3$ | H | $OCH_3$ | - | - | - | Cl | $OCH_3$ | CH | |
| L-9 | Q-9 | 0 | H | H | $CH_3$ | H | F | - | - | - | $CH_3$ | $OCH_3$ | CH | |
| L-9 | Q-9 | 0 | H | H | $CH_3$ | Cl | Br | - | - | - | $OCH_3$ | $OCH_3$ | CH | |
| L-9 | Q-9 | 0 | H | H | $CH_3$ | Cl | $CH_3$ | - | - | - | $CH_3$ | $OCH_3$ | N | |
| L-9 | Q-9 | 0 | H | H | $CH_3$ | F | $CH_3$ | - | - | - | $OCH_3$ | $OCH_3$ | CH | |
| L-9 | Q-9 | 0 | H | H | $CH_3$ | H | Cl | - | - | - | $CH_3$ | $CH_3$ | N | |
| L-9 | Q-9 | 0 | H | H | $CH_3$ | H | $CH_3$ | - | - | - | $OCH_3$ | $OCH_3$ | CH | |
| L-9 | Q-9 | 0 | H | $CH_3$ | $CH_3$ | H | H | - | - | - | Cl | $OCH_3$ | CH | |
| L-9 | Q-9 | 0 | $CH_3$ | H | $CH_3$ | H | H | - | - | - | $CH_3$ | $OCH_3$ | N | |
| L-9 | Q-9 | 1 | H | H | $CH_3$ | F | F | - | - | - | $OCH_3$ | $OCH_3$ | CH | |
| L-9 | Q-9 | 1 | H | H | $CH_3$ | H | H | - | - | - | $OCH_3$ | $OCH_3$ | CH | |
| L-9 | Q-10 | 0 | H | H | $CH_3$ | H | H | - | - | - | $CH_3$ | $CH_3$ | CH | |
| L-9 | Q-10 | 0 | H | H | $CH_3$ | H | H | - | - | - | $OCH_3$ | $CH_3$ | CH | |
| L-9 | Q-10 | 0 | H | H | $CH_3$ | H | H | - | - | - | $OCH_3$ | $OCH_3$ | CH | |
| L-9 | Q-10 | 0 | H | H | $CH_3$ | Cl | H | - | - | - | $CH_3$ | $OCH_3$ | N | |
| L-9 | Q-10 | 0 | H | H | $CH_3$ | F | H | - | - | - | Cl | $OCH_3$ | CH | |
| L-9 | Q-10 | 0 | H | H | $CH_3$ | H | Br | - | - | - | $OCH_3$ | $OCH_3$ | CH | |
| L-9 | Q-10 | 0 | H | H | $CH_3$ | F | F | - | - | - | $OCH_3$ | $OCH_3$ | N | |
| L-9 | Q-10 | 0 | H | H | $CH_3$ | Cl | Cl | - | - | - | $CH_3$ | $OCH_3$ | CH | |

Table IID (continued)

| L | $Q_1$ | n | R | $R_1$ | $R_2$ | $R_7$ | $R_8$ | $R_9$ | $R_{10}$ | $R_{11}$ | X | Y | Z | m.p. (°C) |
|---|---|---|---|---|---|---|---|---|---|---|---|---|---|---|
| L-10 | Q-1 | 0 | H | H | $CH_3$ | H | H | H | H | - | $CH_3$ | $CH_3$ | CH | |
| L-10 | Q-1 | 0 | H | H | $CH_3$ | H | Cl | H | H | - | $CH_3$ | $OCH_3$ | CH | |
| L-10 | Q-1 | 0 | H | H | $CH_3$ | Cl | Cl | H | H | - | $OCH_3$ | $OCH_3$ | CH | |
| L-10 | Q-1 | 0 | H | H | $CH_3$ | F | F | H | H | - | $CH_3$ | $CH_3$ | N | |
| L-10 | Q-1 | 0 | H | H | $CH_3$ | H | Br | H | H | - | $CH_3$ | $OCH_3$ | N | |
| L-10 | Q-1 | 0 | H | H | $CH_3$ | $CH_3$ | H | H | H | - | $OCH_3$ | $OCH_3$ | N | |
| L-10 | Q-1 | 0 | H | H | $CH_3$ | H | $OCH_3$ | H | H | - | Cl | $OCH_3$ | CH | |
| L-10 | Q-1 | 0 | H | H | $CH_3$ | H | F | H | H | - | $CH_3$ | $OCH_3$ | CH | |
| L-10 | Q-1 | 0 | H | H | $CH_3$ | Cl | Br | H | H | - | $OCH_3$ | $OCH_3$ | CH | |
| L-10 | Q-1 | 0 | H | H | $CH_3$ | Cl | $CH_3$ | H | H | - | $CH_3$ | $CH_3$ | N | |
| L-10 | Q-1 | 0 | H | H | $CH_3$ | F | $CH_3$ | H | H | - | $OCH_3$ | $OCH_3$ | CH | |
| L-10 | Q-1 | 0 | H | H | $CH_3$ | H | Cl | Cl | H | - | $CH_3$ | $CH_3$ | N | |
| L-10 | Q-1 | 0 | H | H | $CH_3$ | H | $CH_3$ | F | F | - | $OCH_3$ | $OCH_3$ | CH | |
| L-10 | Q-1 | 0 | H | $CH_3$ | $CH_3$ | H | H | H | H | - | Cl | $OCH_3$ | CH | |
| L-10 | Q-1 | 0 | $CH_3$ | H | $CH_3$ | H | H | H | H | - | $CH_3$ | $OCH_3$ | N | |
| L-10 | Q-1 | 1 | H | H | $CH_3$ | F | F | H | H | - | $OCH_3$ | $OCH_3$ | CH | |
| L-10 | Q-1 | 1 | H | H | $CH_3$ | H | H | H | H | - | $OCH_3$ | $OCH_3$ | CH | |
| L-10 | Q-2 | 0 | H | H | $CH_3$ | H | H | - | - | H | $CH_3$ | $CH_3$ | CH | |
| L-10 | Q-2 | 0 | H | H | $CH_3$ | H | H | - | - | H | $OCH_3$ | $CH_3$ | CH | |
| L-10 | Q-2 | 0 | H | H | $CH_3$ | H | H | - | - | H | $OCH_3$ | $OCH_3$ | CH | |
| L-10 | Q-2 | 0 | H | H | $CH_3$ | Cl | H | - | - | H | $CH_3$ | $OCH_3$ | N | |
| L-10 | Q-2 | 0 | H | H | $CH_3$ | F | H | - | - | H | Cl | $OCH_3$ | CH | |
| L-10 | Q-2 | 0 | H | H | $CH_3$ | H | Br | - | - | H | $OCH_3$ | $OCH_3$ | CH | |

Table IID (continued)

| L | $Q_1$ | n | R | $R_1$ | $R_2$ | $R_7$ | $R_8$ | $R_3$ | $R_{10}$ | $R_{11}$ | X | Y | Z | m.p. (°C) |
|---|---|---|---|---|---|---|---|---|---|---|---|---|---|---|
| L-10 | Q-2 | 0 | H | H | $CH_3$ | F | F | - | - | H | $OCH_3$ | $OCH_3$ | N | |
| L-10 | Q-2 | 0 | H | H | $CH_3$ | Cl | Cl | - | - | H | $CH_3$ | $OCH_3$ | CH | |
| L-10 | Q-2 | 0 | H | H | $CH_3$ | H | $CH_3$ | - | - | H | $CH_3$ | $OCH_3$ | CH | |
| L-10 | Q-2 | 0 | H | H | $CH_3$ | $CH_3$ | $CH_3$ | - | - | H | $OCH_3$ | $OCH_3$ | CH | |
| L-10 | Q-2 | 0 | H | H | $CH_3$ | H | $CO_2CH_3$ | - | - | H | $CH_3$ | $CH_3$ | CH | |
| L-10 | Q-2 | 0 | H | H | $CH_3$ | H | F | - | - | $CH_3$ | $OCH_3$ | $OCH_3$ | CH | |
| L-10 | Q-2 | 0 | H | $OCH_3$ | $CH_3$ | H | Cl | - | - | H | $OCH_3$ | $CH_3$ | N | |
| L-10 | Q-2 | 0 | $CH_3$ | Cl | $CH_3$ | F | $CH_3$ | - | - | $CH_3$ | $CH_3$ | $OCH_3$ | CH | |
| L-10 | Q-2 | 1 | H | H | $CH_3$ | H | Cl | - | - | H | $CH_3$ | $OCH_3$ | CH | |
| L-10 | Q-2 | 1 | H | H | $CH_3$ | Cl | Cl | - | - | H | $OCH_3$ | $OCH_3$ | N | |
| L-10 | Q-2 | 1 | H | H | $CH_3$ | F | $CH_3$ | - | - | H | Cl | $OCH_3$ | CH | |
| L-10 | Q-3 | 0 | H | H | $CH_3$ | H | H | $CH_3$ | - | H | $CH_3$ | $CH_3$ | CH | |
| L-10 | Q-3 | 0 | H | H | $CH_3$ | H | Cl | $CH_3$ | - | H | $CH_3$ | $OCH_3$ | CH | |
| L-10 | Q-3 | 0 | H | H | $CH_3$ | Cl | Cl | $CH_3$ | - | H | $OCH_3$ | $OCH_3$ | CH | |
| L-10 | Q-3 | 0 | H | H | $CH_3$ | F | F | $CH_3$ | - | H | $CH_3$ | $CH_3$ | N | |
| L-10 | Q-3 | 0 | H | H | $CH_3$ | H | Br | $CH_3$ | - | H | $CH_3$ | $OCH_3$ | N | |
| L-10 | Q-3 | 0 | H | H | $CH_3$ | $CH_3$ | H | $CH_3$ | - | H | $OCH_3$ | $OCH_3$ | N | |
| L-10 | Q-3 | 0 | H | H | $CH_3$ | H | $OCH_3$ | $CH_3$ | - | H | Cl | $OCH_3$ | CH | |
| L-10 | Q-3 | 0 | H | H | $CH_3$ | H | F | $CH_3$ | - | H | $CH_3$ | $OCH_3$ | CH | |
| L-10 | Q-3 | 0 | H | H | $CH_3$ | Cl | Br | $CH_3$ | - | H | $OCH_3$ | $OCH_3$ | CH | |
| L-10 | Q-3 | 0 | H | H | $CH_3$ | Cl | $CH_3$ | $CH_3$ | - | H | $CH_3$ | $OCH_3$ | N | |
| L-10 | Q-3 | 0 | H | H | $CH_3$ | F | $CH_3$ | $CH_3$ | - | $CH_3$ | $OCH_3$ | $OCH_3$ | CH | |
| L-10 | Q-3 | 0 | H | H | $CH_3$ | H | Cl | $CH_2CH_3$ | - | H | $CH_3$ | $CH_3$ | N | |
| L-10 | Q-3 | 0 | H | H | $CH_3$ | H | $CH_3$ | $CH_3$ | - | H | $OCH_3$ | $OCH_3$ | CH | |
| L-10 | Q-3 | 0 | H | $CH_3$ | $CH_3$ | H | H | $CH_3$ | - | H | Cl | $OCH_3$ | CH | |
| L-10 | Q-3 | 0 | $CH_3$ | H | $CH_3$ | H | H | $CH_3$ | - | H | $CH_3$ | $OCH_3$ | N | |
| L-10 | Q-3 | 1 | H | H | $CH_3$ | F | F | $CH_3$ | - | H | $OCH_3$ | $OCH_3$ | CH | |
| L-10 | Q-3 | 1 | H | H | $CH_3$ | H | H | $CH_3$ | - | H | $OCH_3$ | $OCH_3$ | CH | |
| L-10 | Q-4 | 0 | H | H | $CH_3$ | H | H | - | - | - | $CH_3$ | $CH_3$ | CH | |
| L-10 | Q-4 | 0 | H | H | $CH_3$ | H | H | - | - | - | $OCH_3$ | $CH_3$ | CH | |
| L-10 | Q-4 | 0 | H | H | $CH_3$ | H | H | - | - | - | $OCH_3$ | $OCH_3$ | CH | |
| L-10 | Q-4 | 0 | H | H | $CH_3$ | Cl | H | - | - | - | $CH_3$ | $OCH_3$ | N | |

Table IID (continued)

| L | $Q_1$ | n | R | $R_1$ | $R_2$ | $R_7$ | $R_8$ | $R_9$ | $R_3$ | $R_{11}$ | X | Y | Z | m.p. (°C) |
|---|---|---|---|---|---|---|---|---|---|---|---|---|---|---|
| L-10 | Q-8 | 0 | H | H | $CH_3$ | H | H | - | - | - | $CH_3$ | $CH_3$ | CH | |
| L-10 | Q-8 | 0 | H | H | $CH_3$ | H | H | - | - | - | $OCH_3$ | $CH_3$ | CH | |
| L-10 | Q-8 | 0 | H | H | $CH_3$ | H | H | - | - | - | $OCH_3$ | $OCH_3$ | CH | |
| L-10 | Q-8 | 0 | H | H | $CH_3$ | Cl | H | - | - | - | $CH_3$ | $OCH_3$ | N | |
| L-10 | Q-8 | 0 | H | H | $CH_3$ | F | H | - | - | - | Cl | $OCH_3$ | CH | |
| L-10 | Q-8 | 0 | H | H | $CH_3$ | H | Br | - | - | - | $OCH_3$ | $OCH_3$ | CH | |
| L-10 | Q-8 | 0 | H | H | $CH_3$ | F | F | - | - | - | $OCH_3$ | $OCH_3$ | N | |
| L-10 | Q-8 | 0 | H | H | $CH_3$ | Cl | Cl | - | - | - | $CH_3$ | $OCH_3$ | CH | |
| L-10 | Q-8 | 0 | H | H | $CH_3$ | H | $CH_3$ | - | - | - | $CH_3$ | $OCH_3$ | CH | |
| L-10 | Q-8 | 0 | H | H | $CH_3$ | $CH_3$ | $CH_3$ | - | - | - | $OCH_3$ | $OCH_3$ | CH | |
| L-10 | Q-8 | 0 | H | H | $CH_3$ | $CH_3$ | $CO_2CH_3$ | - | - | - | $CH_3$ | $CH_3$ | CH | |
| L-10 | Q-8 | 0 | H | H | $CH_3$ | H | F | - | - | - | $OCH_3$ | $OCH_3$ | CH | |
| L-10 | Q-8 | 0 | H | $OCH_3$ | $CH_3$ | H | Cl | - | - | - | $OCH_3$ | $CH_3$ | N | |
| L-10 | Q-8 | 0 | $CH_3$ | Cl | $CH_3$ | F | $CH_3$ | - | - | - | $CH_3$ | $OCH_3$ | CH | |
| L-10 | Q-8 | 1 | H | H | $CH_3$ | H | Cl | - | - | - | $CH_3$ | $OCH_3$ | CH | |
| L-10 | Q-8 | 1 | H | H | $CH_3$ | Cl | Cl | - | - | - | $OCH_3$ | $OCH_3$ | N | |
| L-10 | Q-8 | 1 | H | H | $CH_3$ | F | $CH_3$ | - | - | - | Cl | $OCH_3$ | CH | |
| L-10 | Q-9 | 0 | H | H | $CH_3$ | H | H | - | $CH_3$ | - | $CH_3$ | $CH_3$ | CH | |
| L-10 | Q-9 | 0 | H | H | $CH_3$ | H | Cl | - | $CH_3$ | - | $CH_3$ | $OCH_3$ | CH | |
| L-10 | Q-9 | 0 | H | H | $CH_3$ | Cl | Cl | - | $CH_3$ | - | $OCH_3$ | $OCH_3$ | CH | |
| L-10 | Q-9 | 0 | H | H | $CH_3$ | F | F | - | $CH_3$ | - | $CH_3$ | $CH_3$ | N | |
| L-10 | Q-9 | 0 | H | H | $CH_3$ | H | Br | - | $CH_3$ | - | $CH_3$ | $OCH_3$ | N | |
| L-10 | Q-9 | 0 | H | H | $CH_3$ | $CH_3$ | H | - | $CH_3$ | - | $OCH_3$ | $OCH_3$ | N | |
| L-10 | Q-9 | 0 | H | H | $CH_3$ | H | $OCH_3$ | - | $CH_3$ | - | Cl | $OCH_3$ | CH | |
| L-10 | Q-9 | 0 | H | H | $CH_3$ | H | F | - | $CH_3$ | - | $CH_3$ | $OCH_3$ | CH | |
| L-10 | Q-9 | 0 | H | H | $CH_3$ | Cl | Br | - | $CH_3$ | - | $OCH_3$ | $OCH_3$ | CH | |
| L-10 | Q-9 | 0 | H | H | $CH_3$ | Cl | $CH_3$ | - | $CH_3$ | - | $CH_3$ | $OCH_3$ | N | |
| L-10 | Q-9 | 0 | H | H | $CH_3$ | F | $CH_3$ | - | $CH_3$ | - | $OCH_3$ | $OCH_3$ | CH | |
| L-10 | Q-9 | 0 | H | H | $CH_3$ | H | Cl | - | $CH_3$ | - | $CH_3$ | $CH_3$ | N | |
| L-10 | Q-9 | 0 | H | H | $CH_3$ | H | $CH_3$ | - | $CH_2CH_3$ | - | $OCH_3$ | $OCH_3$ | CH | |
| L-10 | Q-9 | 0 | H | $CH_3$ | $CH_3$ | H | H | - | $CH_2CH_3$ | - | Cl | $OCH_3$ | CH | |
| L-10 | Q-9 | 0 | $CH_3$ | H | $CH_3$ | H | H | - | $CH_2CH_2CH_3$ | - | $CH_3$ | $OCH_3$ | N | |

**Table IIE**

General Structure IIb (wherein $R_{12}$ and $R_{13}$ are H)

| L | $Q_1$ | n | R | $R_1$ | $R_2$ | $R_7$ | $R_8$ | $R_9$ | $R_{10}$ | $R_{11}$ | X | Y | Z | m.p. (°C) |
|---|---|---|---|---|---|---|---|---|---|---|---|---|---|---|
| L-9 | Q-1 | 0 | H | H | - | H | H | H | H | - | $CH_3$ | $CH_3$ | CH | |
| L-9 | Q-1 | 0 | H | H | - | H | Cl | H | H | - | $CH_3$ | $OCH_3$ | CH | |
| L-9 | Q-1 | 0 | H | H | - | Cl | Cl | H | H | - | $OCH_3$ | $OCH_3$ | CH | |
| L-9 | Q-1 | 0 | H | H | - | F | F | H | H | - | $CH_3$ | $CH_3$ | N | |
| L-9 | Q-1 | 0 | H | H | - | H | Br | H | H | - | $CH_3$ | $OCH_3$ | N | |
| L-9 | Q-1 | 0 | H | H | - | $CH_3$ | H | H | H | - | $OCH_3$ | $OCH_3$ | N | |
| L-9 | Q-1 | 0 | H | H | - | H | $OCH_3$ | H | H | - | Cl | $OCH_3$ | CH | |
| L-9 | Q-1 | 0 | H | H | - | H | F | H | H | - | $CH_3$ | $OCH_3$ | CH | |
| L-9 | Q-1 | 0 | H | H | - | Cl | Br | H | H | - | $OCH_3$ | $OCH_3$ | CH | |
| L-9 | Q-1 | 0 | H | H | - | Cl | $CH_3$ | H | H | - | $CH_3$ | $OCH_3$ | N | |
| L-9 | Q-1 | 0 | H | H | - | F | $CH_3$ | H | H | - | $OCH_3$ | $OCH_3$ | CH | |
| L-9 | Q-1 | 0 | H | H | - | H | Cl | Cl | H | - | $CH_3$ | $CH_3$ | N | |
| L-9 | Q-1 | 0 | H | H | - | H | $CH_3$ | F | F | - | $OCH_3$ | $OCH_3$ | CH | |
| L-9 | Q-1 | 0 | H | $CH_3$ | - | H | H | H | H | - | Cl | $OCH_3$ | CH | |
| L-9 | Q-1 | 0 | $CH_3$ | H | - | H | H | H | H | - | $CH_3$ | $OCH_3$ | N | |
| L-9 | Q-1 | 1 | H | H | - | F | F | H | H | - | $OCH_3$ | $OCH_3$ | CH | |
| L-9 | Q-1 | 1 | H | H | - | H | H | H | H | - | $OCH_3$ | $OCH_3$ | CH | |
| L-9 | Q-2 | 0 | H | H | - | H | H | - | - | H | $CH_3$ | $CH_3$ | CH | |
| L-9 | Q-2 | 0 | H | H | - | H | H | - | - | H | $OCH_3$ | $CH_3$ | CH | |
| L-9 | Q-2 | 0 | H | H | - | H | H | - | - | H | $OCH_3$ | $OCH_3$ | CH | |
| L-9 | Q-2 | 0 | H | H | - | Cl | H | - | - | H | $CH_3$ | $OCH_3$ | N | |
| L-9 | Q-2 | 0 | H | H | - | F | H | - | - | H | Cl | $OCH_3$ | CH | |
| L-9 | Q-2 | 0 | H | H | - | H | Br | - | - | H | $OCH_3$ | $OCH_3$ | CH | |
| L-9 | Q-2 | 0 | H | H | - | F | F | - | - | H | $OCH_3$ | $OCH_3$ | N | |
| L-9 | Q-2 | 0 | H | H | - | Cl | Cl | - | - | H | $CH_3$ | $OCH_3$ | CH | |
| L-9 | Q-2 | 0 | H | H | - | H | $CH_3$ | - | - | H | $CH_3$ | $OCH_3$ | CH | |
| L-9 | Q-2 | 0 | H | H | - | $CH_3$ | $CH_3$ | - | - | H | $OCH_3$ | $OCH_3$ | CH | |
| L-9 | Q-2 | 0 | H | H | - | H | $CO_2CH_3$ | - | - | H | $CH_3$ | $CH_3$ | CH | |
| L-9 | Q-2 | 0 | H | H | - | H | F | - | - | $CH_3$ | $OCH_3$ | $OCH_3$ | CH | |
| L-9 | Q-2 | 0 | H | $OCH_3$ | - | H | Cl | - | - | H | $OCH_3$ | $CH_3$ | N | |
| L-9 | Q-2 | 0 | $CH_3$ | Cl | - | F | $CH_3$ | - | - | $CH_3$ | $CH_3$ | $OCH_3$ | CH | |
| L-9 | Q-1 | 0 | H | H | - | H | OH | - | H | - | $CH_3$ | $CH_3$ | CH | |

Table IIE (continued)

| L | $Q_1$ | n | R | $R_1$ | $R_3$ | $R_7$ | $R_8$ | $R_9$ | $R_{10}$ | $R_{11}$ | X | Y | Z | m.p. (°C) |
|---|---|---|---|---|---|---|---|---|---|---|---|---|---|---|
| L-9 | Q-1 | 0 | H | H | - | H | OH | - | H | - | $CH_3$ | $OCH_3$ | CH | |
| L-9 | Q-1 | 0 | H | H | - | H | OH | - | H | - | $OCH_3$ | $OCH_3$ | CH | |
| L-9 | Q-1 | 0 | H | H | - | H | OH | - | H | - | $CH_3$ | $OCH_3$ | N | |
| L-9 | Q-1 | 0 | H | H | - | H | OH | - | H | - | $OCH_3$ | $OCH_3$ | N | |
| L-9 | Q-1 | 0 | H | H | - | H | OH | - | H | - | Cl | $OCH_3$ | CH | |
| L-9 | Q-2 | 1 | H | H | - | H | Cl | - | - | H | $CH_3$ | $OCH_3$ | CH | |
| L-9 | Q-2 | 1 | H | H | - | Cl | Cl | - | - | H | $OCH_3$ | $OCH_3$ | N | |
| L-9 | Q-2 | 1 | H | H | - | F | $CH_3$ | - | - | H | Cl | $OCH_3$ | CH | |
| L-9 | Q-3 | 0 | H | H | $CH_3$ | H | H | - | - | H | $CH_3$ | $CH_3$ | CH | |
| L-9 | Q-3 | 0 | H | H | $CH_3$ | H | Cl | - | - | H | $CH_3$ | $OCH_3$ | CH | |
| L-9 | Q-3 | 0 | H | H | $CH_3$ | Cl | Cl | - | - | H | $OCH_3$ | $OCH_3$ | CH | |
| L-9 | Q-3 | 0 | H | H | $CH_3$ | F | F | - | - | H | $CH_3$ | $CH_3$ | N | |
| L-9 | Q-3 | 0 | H | H | $CH_3$ | H | Br | - | - | H | $CH_3$ | $OCH_3$ | N | |
| L-9 | Q-3 | 0 | H | H | $CH_3$ | $CH_3$ | H | - | - | H | $OCH_3$ | $OCH_3$ | N | |
| L-9 | Q-3 | 0 | H | H | $CH_3$ | H | $OCH_3$ | - | - | H | Cl | $OCH_3$ | CH | |
| L-9 | Q-3 | 0 | H | H | $CH_3$ | H | F | - | - | H | $CH_3$ | $OCH_3$ | CH | |
| L-9 | Q-3 | 0 | H | H | $CH_3$ | Cl | Br | - | - | H | $OCH_3$ | $OCH_3$ | CH | |
| L-9 | Q-3 | 0 | H | H | $CH_3$ | Cl | $CH_3$ | - | - | H | $CH_3$ | $OCH_3$ | N | |
| L-9 | Q-3 | 0 | H | H | $CH_3$ | F | $CH_3$ | - | - | $CH_3$ | $OCH_3$ | $OCH_3$ | CH | |
| L-9 | Q-3 | 0 | H | H | $CH_3$ | H | Cl | - | - | H | $CH_3$ | $CH_3$ | N | |
| L-9 | Q-3 | 0 | H | H | $CH_3$ | H | $CH_3$ | - | - | H | $OCH_3$ | $OCH_3$ | CH | |
| L-9 | Q-3 | 0 | H | $CH_3$ | $CH_3$ | H | H | - | - | H | Cl | $OCH_3$ | CH | |
| L-9 | Q-3 | 0 | $CH_3$ | H | $CH_3$ | H | H | - | - | H | $CH_3$ | $OCH_3$ | N | |
| L-9 | Q-3 | 1 | H | H | $CH_3$ | F | F | - | - | H | $OCH_3$ | $OCH_3$ | CH | |
| L-9 | Q-3 | 1 | H | H | $CH_3$ | H | H | - | - | H | $OCH_3$ | $OCH_3$ | CH | |
| L-9 | Q-4 | 0 | H | H | - | H | H | - | - | - | $CH_3$ | $CH_3$ | CH | |
| L-9 | Q-4 | 0 | H | H | - | H | H | - | - | - | $OCH_3$ | $CH_3$ | CH | |
| L-9 | Q-4 | 0 | H | H | - | H | H | - | - | - | $OCH_3$ | $OCH_3$ | CH | |
| L-9 | Q-4 | 0 | H | H | - | Cl | H | - | - | - | $CH_3$ | $OCH_3$ | N | |
| L-9 | Q-4 | 0 | H | H | - | F | H | - | - | - | Cl | $OCH_3$ | CH | |
| L-9 | Q-4 | 0 | H | H | - | H | Br | - | - | - | $OCH_3$ | $OCH_3$ | CH | |
| L-9 | Q-4 | 0 | H | H | - | F | F | - | - | - | $OCH_3$ | $OCH_3$ | N | |
| L-9 | Q-4 | 0 | H | H | - | Cl | Cl | - | - | - | $CH_3$ | $OCH_3$ | CH | |

Table IIE (continued)

| L | $Q_1$ | n | R | $R_1$ | $R_2$ | $R_7$ | $R_8$ | $R_9$ | $R_{10}$ | $R_{11}$ | X | Y | Z | m.p. (°C) |
|---|---|---|---|---|---|---|---|---|---|---|---|---|---|---|
| L-9 | Q-4 | 0 | H | H | - | H | $CH_3$ | - | - | - | $CH_3$ | $OCH_3$ | CH | |
| L-9 | Q-4 | 0 | H | H | - | $CH_3$ | $CH_3$ | - | - | - | $OCH_3$ | $OCH_3$ | CH | |
| L-9 | Q-4 | 0 | H | H | - | H | $CO_2CH_3$ | - | - | - | $CH_3$ | $CH_3$ | CH | |
| L-9 | Q-4 | 0 | H | H | - | H | F | - | - | - | $OCH_3$ | $OCH_3$ | CH | |
| L-9 | Q-4 | 0 | H | $OCH_3$ | - | H | Cl | - | - | - | $OCH_3$ | $CH_3$ | N | |
| L-9 | Q-4 | 0 | $CH_3$ | Cl | - | F | $CH_3$ | - | - | - | $CH_3$ | $OCH_3$ | CH | |
| L-9 | Q-4 | 1 | H | H | - | H | Cl | - | - | - | $CH_3$ | $OCH_3$ | CH | |
| L-9 | Q-4 | 1 | H | H | - | Cl | Cl | - | - | - | $OCH_3$ | $OCH_3$ | N | |
| L-9 | Q-4 | 1 | H | H | - | F | $CH_3$ | - | - | - | Cl | $OCH_3$ | CH | |
| L-9 | Q-5 | 0 | H | H | - | H | H | - | - | - | $CH_3$ | $CH_3$ | CH | |
| L-9 | Q-5 | 0 | H | H | - | H | Cl | - | - | - | $CH_3$ | $OCH_3$ | CH | |
| L-9 | Q-5 | 0 | H | H | - | Cl | Cl | - | - | - | $OCH_3$ | $OCH_3$ | CH | |
| L-9 | Q-5 | 0 | H | H | - | F | F | - | - | - | $CH_3$ | $CH_3$ | N | |
| L-9 | Q-5 | 0 | H | H | - | H | Br | - | - | - | $CH_3$ | $OCH_3$ | N | |
| L-9 | Q-5 | 0 | H | H | - | $CH_3$ | H | - | - | - | $OCH_3$ | $OCH_3$ | N | |
| L-9 | Q-5 | 0 | H | H | - | H | $OCH_3$ | - | - | - | Cl | $OCH_3$ | CH | |
| L-9 | Q-5 | 0 | H | H | - | H | F | - | - | - | $CH_3$ | $OCH_3$ | CH | |
| L-9 | Q-5 | 0 | H | H | - | Cl | Br | - | - | - | $OCH_3$ | $OCH_3$ | CH | |
| L-9 | Q-5 | 0 | H | H | - | Cl | $CH_3$ | - | - | - | $CH_3$ | $OCH_3$ | N | |
| L-9 | Q-5 | 0 | H | H | - | F | $CH_3$ | - | - | - | $OCH_3$ | $OCH_3$ | CH | |
| L-9 | Q-5 | 0 | H | H | - | H | Cl | - | - | - | $CH_3$ | $CH_3$ | N | |
| L-9 | Q-5 | 0 | H | H | - | H | $CH_3$ | - | - | - | $OCH_3$ | $OCH_3$ | CH | |
| L-9 | Q-5 | 0 | H | $CH_3$ | - | H | H | - | - | - | Cl | $OCH_3$ | CH | |
| L-9 | Q-5 | 0 | $CH_3$ | H | - | H | H | - | - | - | $CH_3$ | $OCH_3$ | N | |
| L-9 | Q-5 | 1 | H | H | - | F | F | - | - | - | $OCH_3$ | $OCH_3$ | CH | |
| L-9 | Q-5 | 1 | H | H | - | H | H | - | - | - | $OCH_3$ | $OCH_3$ | CH | |
| L-9 | Q-6 | 0 | H | H | - | H | H | - | - | - | $CH_3$ | $CH_3$ | CH | |
| L-9 | Q-6 | 0 | H | H | - | H | H | - | - | - | $OCH_3$ | $CH_3$ | CH | |
| L-9 | Q-6 | 0 | H | H | - | H | H | - | - | - | $OCH_3$ | $OCH_3$ | CH | |
| L-9 | Q-6 | 0 | H | H | - | Cl | H | - | - | - | $CH_3$ | $OCH_3$ | N | |
| L-9 | Q-6 | 0 | H | H | - | F | H | - | - | - | Cl | $OCH_3$ | CH | |
| L-9 | Q-6 | 0 | H | H | - | H | Br | - | - | - | $OCH_3$ | $OCH_3$ | CH | |
| L-9 | Q-6 | 0 | H | H | - | F | F | - | - | - | $OCH_3$ | $OCH_3$ | N | |

Table IIE (continued)

| L | $Q_1$ | n | R | $R_1$ | $R_2$ | $R_7$ | $R_8$ | $R_9$ | $R_{10}$ | $R_{11}$ | X | Y | Z | m.p. (°C) |
|---|---|---|---|---|---|---|---|---|---|---|---|---|---|---|
| L-9 | Q-6 | 0 | H | H | - | Cl | Cl | - | - | - | $CH_3$ | $OCH_3$ | CH | |
| L-9 | Q-6 | 0 | H | H | - | H | $CH_3$ | - | - | - | $CH_3$ | $OCH_3$ | CH | |
| L-9 | Q-6 | 0 | H | H | - | $CH_3$ | $CH_3$ | - | - | - | $OCH_3$ | $OCH_3$ | CH | |
| L-9 | Q-6 | 0 | H | H | - | H | $CO_2CH_3$ | - | - | - | Cl | $CH_3$ | CH | |
| L-9 | Q-6 | 0 | H | H | - | H | F | - | - | - | $OCH_3$ | $OCH_3$ | CH | |
| L-10 | Q-1 | 0 | H | H | $CH_3$ | H | H | H | H | - | $CH_3$ | $CH_3$ | CH | |
| L-10 | Q-1 | 0 | H | H | $CH_3$ | H | Cl | H | H | - | $CH_3$ | $OCH_3$ | CH | |
| L-10 | Q-1 | 0 | H | H | $CH_3$ | Cl | Cl | H | H | - | $OCH_3$ | $OCH_3$ | CH | |
| L-10 | Q-1 | 0 | H | H | $CH_3$ | F | F | H | H | - | $CH_3$ | $CH_3$ | N | |
| L-10 | Q-1 | 0 | H | H | $CH_3$ | H | Br | H | H | - | $CH_3$ | $OCH_3$ | N | |
| L-10 | Q-1 | 0 | H | H | $CH_3$ | $CH_3$ | H | H | H | - | $OCH_3$ | $OCH_3$ | N | |
| L-10 | Q-1 | 0 | H | H | $CH_3$ | H | $OCH_3$ | H | H | - | Cl | $OCH_3$ | CH | |
| L-10 | Q-1 | 0 | H | H | $CH_3$ | H | F | H | H | - | $CH_3$ | $OCH_3$ | CH | |
| L-10 | Q-1 | 0 | H | H | $CH_3$ | Cl | Br | H | H | - | $OCH_3$ | $OCH_3$ | CH | |
| L-10 | Q-1 | 0 | H | H | $CH_3$ | Cl | $CH_3$ | H | H | - | $CH_3$ | $CH_3$ | N | |
| L-10 | Q-1 | 0 | H | H | $CH_3$ | F | $CH_3$ | H | H | - | $OCH_3$ | $OCH_3$ | CH | |
| L-10 | Q-1 | 0 | H | H | $CH_3$ | H | Cl | Cl | H | - | $CH_3$ | $CH_3$ | N | |
| L-10 | Q-1 | 0 | H | H | $CH_3$ | H | $CH_3$ | F | F | - | $OCH_3$ | $OCH_3$ | CH | |
| L-10 | Q-1 | 0 | H | $CH_3$ | $CH_3$ | H | H | H | H | - | Cl | $OCH_3$ | CH | |
| L-10 | Q-1 | 0 | $CH_3$ | H | $CH_3$ | H | H | H | H | - | $CH_3$ | $OCH_3$ | N | |
| L-10 | Q-1 | 1 | H | H | $CH_3$ | F | F | H | H | - | $OCH_3$ | $OCH_3$ | CH | |
| L-10 | Q-1 | 1 | H | H | $CH_3$ | H | H | H | H | - | $OCH_3$ | $OCH_3$ | CH | |
| L-10 | Q-2 | 0 | H | H | $CH_3$ | H | H | - | - | H | $CH_3$ | $CH_3$ | CH | |
| L-10 | Q-2 | 0 | H | H | $CH_3$ | H | H | - | - | H | $OCH_3$ | $CH_3$ | CH | |
| L-10 | Q-2 | 0 | H | H | $CH_3$ | H | H | - | - | H | $OCH_3$ | $OCH_3$ | CH | |
| L-10 | Q-2 | 0 | H | H | $CH_3$ | Cl | H | - | - | H | $CH_3$ | $OCH_3$ | N | |
| L-10 | Q-2 | 0 | H | H | $CH_3$ | F | H | - | - | H | Cl | $OCH_3$ | CH | |
| L-10 | Q-2 | 0 | H | H | $CH_3$ | H | Br | - | - | H | $OCH_3$ | $OCH_3$ | CH | |

Table IIE (continued)

| L | $Q_1$ | n | R | $R_1$ | $R_2$ | $R_7$ | $R_8$ | $R_9$ | $R_{10}$ | $R_{11}$ | X | Y | Z | m.p. (°C) |
|---|---|---|---|---|---|---|---|---|---|---|---|---|---|---|
| L-10 | Q-4 | 0 | H | H | $CH_3$ | F | H | - | - | - | Cl | $OCH_3$ | CH | |
| L-10 | Q-4 | 0 | H | H | $CH_3$ | H | Br | - | - | - | $OCH_3$ | $OCH_3$ | CH | |
| L-10 | Q-4 | 0 | H | H | $CH_3$ | F | F | - | - | - | $OCH_3$ | $OCH_3$ | N | |
| L-10 | Q-4 | 0 | H | H | $CH_3$ | Cl | Cl | - | - | - | $CH_3$ | $OCH_3$ | CH | |
| L-10 | Q-4 | 0 | H | H | $CH_3$ | H | $CH_3$ | - | - | - | $CH_3$ | $OCH_3$ | CH | |
| L-10 | Q-4 | 0 | H | H | $CH_3$ | $CH_3$ | $CH_3$ | - | - | - | $OCH_3$ | $OCH_3$ | CH | |
| L-10 | Q-4 | 0 | H | H | $CH_3$ | H | $CO_2CH_3$ | - | - | - | $CH_3$ | $CH_3$ | CH | |
| L-10 | Q-4 | 0 | H | H | $CH_3$ | H | F | - | - | - | $OCH_3$ | $OCH_3$ | CH | |
| L-10 | Q-4 | 0 | H | $OCH_3$ | $CH_3$ | H | Cl | - | - | - | $OCH_3$ | $CH_3$ | N | |
| L-10 | Q-4 | 0 | $CH_3$ | Cl | $CH_3$ | F | $CH_3$ | - | - | - | $CH_3$ | $OCH_3$ | CH | |
| L-10 | Q-4 | 1 | H | H | $CH_3$ | H | Cl | - | - | - | $CH_3$ | $OCH_3$ | CH | |
| L-10 | Q-4 | 1 | H | H | $CH_3$ | Cl | Cl | - | - | - | $OCH_3$ | $OCH_3$ | N | |
| L-10 | Q-4 | 1 | H | H | $CH_3$ | F | $CH_3$ | - | - | - | Cl | $OCH_3$ | CH | |
| L-10 | Q-5 | 0 | H | H | $CH_3$ | H | H | - | - | - | $CH_3$ | $CH_3$ | CH | |
| L-10 | Q-5 | 0 | H | H | $CH_3$ | H | Cl | - | - | - | $CH_3$ | $OCH_3$ | CH | |
| L-10 | Q-5 | 0 | H | H | $CH_3$ | Cl | Cl | - | - | - | $OCH_3$ | $OCH_3$ | CH | |
| L-10 | Q-5 | 0 | H | H | $CH_3$ | F | F | - | - | - | $CH_3$ | $CH_3$ | N | |
| L-10 | Q-5 | 0 | H | H | $CH_3$ | H | Br | - | - | - | $CH_3$ | $OCH_3$ | N | |
| L-10 | Q-5 | 0 | H | H | $CH_3$ | $CH_3$ | H | - | - | - | $OCH_3$ | $OCH_3$ | N | |
| L-10 | Q-5 | 0 | H | H | $CH_3$ | H | $OCH_3$ | - | - | - | Cl | $OCH_3$ | CH | |
| L-10 | Q-5 | 0 | H | H | $CH_3$ | H | F | - | - | - | $CH_3$ | $OCH_3$ | CH | |
| L-10 | Q-5 | 0 | H | H | $CH_3$ | Cl | Br | - | - | - | $OCH_3$ | $OCH_3$ | CH | |
| L-10 | Q-5 | 0 | H | H | $CH_3$ | Cl | $CH_3$ | - | - | - | $CH_3$ | $OCH_3$ | N | |
| L-10 | Q-5 | 0 | H | H | $CH_3$ | F | $CH_3$ | - | - | - | $OCH_3$ | $OCH_3$ | CH | |
| L-10 | Q-5 | 0 | H | H | $CH_3$ | H | Cl | - | - | - | $CH_3$ | $CH_3$ | N | |
| L-10 | Q-5 | 0 | H | H | $CH_3$ | H | $CH_3$ | - | - | - | $OCH_3$ | $OCH_3$ | CH | |
| L-10 | Q-5 | 0 | H | $CH_3$ | $CH_3$ | H | H | - | - | - | Cl | $OCH_3$ | CH | |
| L-10 | Q-5 | 0 | $CH_3$ | H | $CH_3$ | H | H | - | - | - | $CH_3$ | $OCH_3$ | N | |
| L-10 | Q-5 | 1 | H | H | $CH_3$ | F | F | - | - | - | $OCH_3$ | $OCH_3$ | CH | |
| L-10 | Q-5 | 1 | H | H | $CH_3$ | H | H | - | - | - | $OCH_3$ | $OCH_3$ | CH | |
| L-10 | Q-6 | 0 | H | H | $CH_3$ | H | H | - | - | - | $CH_3$ | $CH_3$ | CH | |
| L-10 | Q-6 | 0 | H | H | $CH_3$ | H | H | - | - | - | $OCH_3$ | $CH_3$ | CH | |

Table IIE (continued)

| L | $Q_1$ | n | R | $R_1$ | $R_2$ | $R_7$ | $R_8$ | $R_9$ | $R_3$ | $R_{10}$ | X | Y | Z | m.p. (°C) |
|---|---|---|---|---|---|---|---|---|---|---|---|---|---|---|
| L-10 | Q-9 | 1 | H | H | $CH_3$ | F | F | - | $CH_3$ | - | $OCH_3$ | $OCH_3$ | CH | |
| L-10 | Q-9 | 1 | H | H | $CH_3$ | H | H | - | $CH_2CH_3$ | - | $OCH_3$ | $OCH_3$ | CH | |
| L-10 | Q-10 | 0 | H | H | $CH_3$ | H | H | - | $CH_3$ | - | $CH_3$ | $CH_3$ | CH | |
| L-10 | Q-10 | 0 | H | H | $CH_3$ | H | H | - | $CH_3$ | - | $OCH_3$ | $CH_3$ | CH | |
| L-10 | Q-10 | 0 | H | H | $CH_3$ | H | H | - | $CH_3$ | - | $OCH_3$ | $OCH_3$ | CH | |
| L-10 | Q-10 | 0 | H | H | $CH_3$ | Cl | H | - | $CH_3$ | - | $CH_3$ | $OCH_3$ | N | |
| L-10 | Q-10 | 0 | H | H | $CH_3$ | F | H | - | $CH_3$ | - | Cl | $OCH_3$ | CH | |
| L-10 | Q-10 | 0 | H | H | $CH_3$ | H | Br | - | $CH_3$ | - | $OCH_3$ | $OCH_3$ | CH | |
| L-10 | Q-10 | 0 | H | H | $CH_3$ | F | F | - | $CH_3$ | - | $OCH_3$ | $OCH_3$ | N | |
| L-10 | Q-10 | 0 | H | H | $CH_3$ | Cl | Cl | - | $CH_3$ | - | $CH_3$ | $OCH_3$ | CH | |
| L-10 | Q-10 | 0 | H | H | $CH_3$ | H | $CH_3$ | - | $CH_3$ | - | $CH_3$ | $OCH_3$ | CH | |
| L-10 | Q-10 | 0 | H | H | $CH_3$ | $CH_3$ | $CH_3$ | - | $CH_3$ | - | $OCH_3$ | $OCH_3$ | CH | |
| L-10 | Q-10 | 0 | H | H | $CH_3$ | H | $CO_2CH_3$ | - | $CH_3$ | - | $CH_3$ | $CH_3$ | CH | |
| L-10 | Q-10 | 0 | H | H | $CH_3$ | H | F | - | $CH_3$ | - | $OCH_3$ | $OCH_3$ | CH | |
| L-10 | Q-10 | 0 | H | $OCH_3$ | $CH_3$ | H | Cl | - | $CH_3$ | - | $OCH_3$ | $CH_3$ | N | |
| L-10 | Q-10 | 0 | $CH_3$ | Cl | $CH_3$ | F | $CH_3$ | - | $CH_3$ | - | $CH_3$ | $OCH_3$ | CH | |
| L-10 | Q-10 | 1 | H | H | $CH_3$ | H | Cl | - | $CH_2CH_3$ | - | $CH_3$ | $OCH_3$ | CH | |
| L-10 | Q-10 | 1 | H | H | $CH_3$ | Cl | Cl | - | $CH_3$ | - | $OCH_3$ | $OCH_3$ | N | |
| L-10 | Q-10 | 1 | H | H | $CH_3$ | F | $CH_3$ | - | $CH_2CH_3$ | - | Cl | $OCH_3$ | CH | |

Table III

General Structure III (wherein $R_{12}$ and $R_{13}$ are H)

| A | Q | n | R | $R_1$ | $R_3$ | $R_7$ | $R_8$ | $R_9$ | $R_{10}$ | $R_{11}$ | $X_1$ | $Y_1$ | $Y_2$ | $X_2$ | $Y_3$ | $X_3$ | m.p. (°C) |
|---|---|---|---|---|---|---|---|---|---|---|---|---|---|---|---|---|---|
| A-2 | Q-1 | 0 | H | H | - | H | H | H | H | - | $CH_3$ | O | - | - | - | - | |
| A-2 | Q-1 | 0 | H | H | - | H | H | H | H | - | $CH_3$ | $CH_2$ | - | - | - | - | |
| A-2 | Q-1 | 0 | H | H | - | H | H | H | H | - | $OCH_3$ | O | - | - | - | - | |
| A-2 | Q-5 | 0 | H | H | - | H | H | - | - | - | $CH_3$ | $CH_2$ | - | - | - | - | |
| A-2 | Q-2 | 0 | H | H | - | H | Cl | - | - | H | $OCF_2H$ | O | - | - | - | - | |
| A-3 | Q-1 | 0 | H | H | - | H | H | H | H | - | $CH_3$ | - | - | - | - | - | |
| A-3 | Q-1 | 0 | H | H | - | H | H | H | H | - | $OCH_3$ | - | - | - | - | - | |
| A-3 | Q-1 | 0 | H | H | - | H | F | H | Cl | - | $OCF_2H$ | - | - | - | - | - | |
| A-3 | Q-1 | 1 | H | H | - | Cl | Cl | H | H | - | $CH_3$ | - | - | - | - | - | |
| A-3 | Q-1 | 0 | H | H | - | $CH_3$ | H | H | H | - | $OCH_3$ | - | - | - | - | - | |
| A-3 | Q-4 | 0 | H | H | - | H | H | - | - | - | $OCH_3$ | - | - | - | - | - | |
| A-3 | Q-3 | 0 | H | H | $CH_3$ | H | $CH_3$ | - | - | H | $CH_3$ | - | - | - | - | - | |
| A-4 | Q-1 | 0 | H | H | - | H | H | H | H | - | $CH_3$ | - | H | - | - | - | |
| A-4 | Q-1 | 0 | H | H | - | H | Cl | H | H | - | $OCH_3$ | - | H | - | - | - | |
| A-4 | Q-1 | 1 | H | H | - | F | F | Cl | $CH_3$ | - | $CH_3$ | - | $CH_3$ | - | - | - | |
| A-4 | Q-6 | 0 | H | H | - | H | H | - | - | - | $OCH_3$ | - | H | - | - | - | |
| A-4 | Q-7 | 0 | H | H | - | $CH_3$ | F | - | - | - | $CH_3$ | - | $CH_3$ | - | - | - | |
| A-5 | Q-1 | 0 | H | H | - | H | H | H | H | - | - | - | - | $CH_3$ | $CH_3$ | - | |
| A-5 | Q-1 | 0 | H | H | - | Cl | H | H | Cl | - | - | - | - | $OCH_3$ | $CH_3$ | - | |
| A-5 | Q-10 | 0 | H | H | $CH_3$ | H | H | - | - | - | - | - | - | $CH_3$ | $CH_2CF_3$ | - | |
| A-6 | Q-1 | 0 | H | H | - | H | H | H | H | - | - | - | - | - | - | $CH_3$ | |

| A | Q | n | R | $R_1$ | $R_7$ | $R_8$ | $R_9$ | $R_{10}$ | $R_{11}$ | $X_4$ | $Y_4$ | Z | m.p. (°C) |
|---|---|---|---|---|---|---|---|---|---|---|---|---|---|
| A-7 | Q-1 | 0 | H | H | H | H | H | H | - | $CH_3$ | $CH_3$ | CH | |
| A-7 | Q-2 | 0 | H | H | H | H | - | - | H | $CH_3$ | $OCH_3$ | CH | |
| A-7 | Q-4 | 0 | H | H | H | H | - | - | - | $OCH_3$ | $OCH_3$ | CH | |

Table IV

General Structure IV

| L | Q | A | $R_1$ | $R_2$ | $R_3$ | $R_7$ | $R_8$ | $R_9$ | $R_{10}$ | $R_{11}$ | $X_1$ | $Y_1$ | $Y_2$ | $X_2$ | $Y_3$ | $X_3$ | m.p. (°C) |
|---|---|---|---|---|---|---|---|---|---|---|---|---|---|---|---|---|---|
| L-9 | Q-1 | A-2 | H | - | - | H | H | H | H | - | $CH_3$ | O | - | - | - | - | |
| L-9 | Q-6 | A-4 | H | - | - | H | Cl | - | - | - | $OCH_3$ | - | H | - | - | - | |
| L-9 | Q-10 | A-5 | H | - | $CH_3$ | H | H | - | - | - | - | - | - | $OCH_3$ | $CH_3$ | - | |
| L-10 | Q-1 | A-3 | H | $CH_3$ | - | H | H | H | H | - | $OCH_3$ | - | - | - | - | - | |
| L-10 | Q-1 | A-6 | H | $CH_3$ | - | Cl | H | H | H | - | - | - | - | - | - | $OCH_3$ | |
| L-10 | Q-3 | A-2 | H | $CH_3$ | $CH_3$ | H | H | - | - | H | $OCH_3$ | $CH_2$ | - | - | - | - | |
| L-11 | Q-1 | A-3 | H | - | - | H | H | H | H | - | $OCH_3$ | - | - | - | - | - | |
| L-11 | Q-1 | A-4 | H | - | - | H | F | H | H | - | $OCH_3$ | - | $CH_3$ | - | - | - | |
| L-11 | Q-2 | A-2 | H | - | - | H | $CH_3$ | - | - | H | $OCF_2H$ | O | - | - | - | - | |
| L-11 | Q-3 | A-4 | H | - | $CH_3$ | H | H | - | - | H | $CH_3$ | - | $CH_3$ | - | - | - | |
| L-12 | Q-1 | A-2 | H | - | - | H | H | H | H | - | $OCH_2CH_3$ | $CH_2$ | - | - | - | - | |
| L-12 | Q-1 | A-6 | H | - | - | H | Cl | H | H | - | - | - | - | - | - | $CH_3$ | |
| L-12 | Q-4 | A-2 | H | - | - | H | H | - | - | - | $OCH_3$ | O | - | - | - | - | |
| L-12 | Q-7 | A-5 | H | - | - | H | H | - | - | - | - | - | - | $SCH_3$ | $CH_3$ | - | |
| L-13 | Q-1 | A-2 | H | - | - | H | H | H | H | - | $CH_3$ | O | - | - | - | - | |
| L-13 | Q-5 | A-2 | H | - | - | H | $CH_3$ | - | - | - | $CH_3$ | $CH_2$ | - | - | - | - | |
| L-13 | Q-8 | A-6 | H | - | - | H | H | - | - | - | - | - | - | - | - | $OCH_3$ | |
| L-13 | Q-9 | A-2 | H | - | $CH_3$ | H | H | - | - | - | $OCH_3$ | O | - | - | - | - | |
| L-9 | Q-1 | A-2 | H | - | - | H | OH | - | - | - | $OCH_3$ | O | - | - | - | - | |

Table V

General Structure V (where $R_9$ and $R_{10}$ are a carbonyl group)

| L | Q | R | $R_1$ | $R_2$ | $R_7$ | $R_8$ | X | Y | Z | m.p.(°C) |
|---|---|---|---|---|---|---|---|---|---|---|
| L-8 | Q-1 | H | H | - | H | H | $CH_3$ | $CH_3$ | CH | |
| L-8 | Q-1 | H | H | - | H | H | $OCH_3$ | $CH_3$ | CH | |
| L-8 | Q-1 | H | H | - | H | H | $OCH_3$ | $OCH_3$ | CH | |
| L-8 | Q-1 | H | H | - | H | H | $CH_3$ | $CH_3$ | N | |
| L-8 | Q-1 | H | H | - | H | H | $OCH_3$ | $CH_3$ | N | |
| L-8 | Q-1 | H | H | - | H | H | $OCH_3$ | $OCH_3$ | N | |
| L-8 | Q-1 | H | H | - | H | Cl | $CH_3$ | $OCH_3$ | CH | |
| L-8 | Q-1 | H | H | - | H | F | $CH_3$ | $OCH_3$ | N | |
| L-8 | Q-1 | H | H | - | H | Br | $OCH_3$ | $OCH_3$ | CH | |
| L-8 | Q-1 | H | H | - | H | CN | $OCH_3$ | $OCH_3$ | CH | |
| L-8 | Q-1 | H | H | - | H | $CH_3$ | $CH_3$ | $OCH_3$ | N | |
| L-8 | Q-1 | H | H | - | H | $OCH_3$ | $CH_3$ | $CH_3$ | CH | |
| L-8 | Q-1 | H | H | - | H | $CO_2CH_3$ | $OCH_3$ | $OCH_3$ | N | |
| L-8 | Q-1 | H | H | - | H | $SO_2N(CH_3)_2$ | $CH_3$ | $OCH_3$ | CH | |
| L-8 | Q-1 | H | H | - | H | $SCH_3$ | $CH_3$ | $OCH_3$ | CH | |
| L-8 | Q-1 | H | H | - | H | $SO_2CH_3$ | $CH_3$ | $CH_3$ | N | |
| L-8 | Q-1 | $CH_3$ | H | - | H | H | $OCH_3$ | $OCH_3$ | CH | |
| L-9 | Q-1 | H | H | - | H | H | $CH_3$ | $OCH_3$ | CH | |
| L-9 | Q-1 | H | H | - | H | H | $OCH_3$ | $OCH_3$ | N | |
| L-10 | Q-1 | H | H | $CH_3$ | H | H | $OCH_3$ | $CH_3$ | CH | |
| L-10 | Q-1 | H | H | $CH_3$ | H | H | $OCH_3$ | $OCH_3$ | CH | |
| L-11 | Q-1 | H | H | - | H | H | $CH_3$ | $OCH_3$ | N | |
| L-11 | Q-1 | H | H | - | H | $CH_3$ | $CH_3$ | $OCH_3$ | CH | |
| L-12 | Q-1 | H | H | - | H | H | $OCH_3$ | $OCH_3$ | CH | |
| L-12 | Q-1 | H | H | - | H | Cl | $CH_3$ | $CH_3$ | CH | |
| L-13 | Q-1 | H | H | - | H | H | $OCH_3$ | $OCH_3$ | N | |
| L-13 | Q-1 | H | H | - | H | H | $CH_3$ | $CH_3$ | N | |
| L-13 | Q-1 | H | H | - | Cl | Cl | $OCH_3$ | $OCH_3$ | CH | |

Table VI

General Structure VI

| R | $R_1$ | $R_{17}$ | E | X | Y | Z | m.p.(°C) |
|---|---|---|---|---|---|---|---|
| H | H | H | S | $CH_3$ | $CH_3$ | CH | 142-145 |
| H | H | H | S | $CH_3$ | $OCH_3$ | CH | 141-143 |
| H | H | H | S | $OCH_3$ | $OCH_3$ | CH | 131-134 |
| H | H | H | S | $CH_3$ | $CH_3$ | N | 165-169 |
| H | H | H | S | $CH_3$ | $OCH_3$ | N | 152-156 |
| H | H | H | S | $OCH_3$ | $OCH_3$ | N | 140-142 |
| H | H | H | S | Cl | $OCH_3$ | CH | 186-191 |
| H | H | H | SO | $CH_3$ | $CH_3$ | CH | |
| H | H | H | SO | $CH_3$ | $OCH_3$ | CH | |
| H | H | H | SO | $OCH_3$ | $OCH_3$ | CH | |
| H | H | H | SO | $CH_3$ | $CH_3$ | N | |
| H | H | H | SO | $CH_3$ | $OCH_3$ | N | |
| H | H | H | SO | $OCH_3$ | $OCH_3$ | N | |
| H | H | H | SO | Cl | $OCH_3$ | CH | |
| H | H | H | $SO_2$ | $CH_3$ | $CH_3$ | CH | 210-212 |
| H | H | H | $SO_2$ | $CH_3$ | $OCH_3$ | CH | |
| H | H | H | $SO_2$ | $OCH_3$ | $OCH_3$ | CH | 226-229 |
| H | H | H | $SO_2$ | $CH_3$ | $CH_3$ | N | 185-188 |
| H | H | H | $SO_2$ | $CH_3$ | $OCH_3$ | N | |
| H | H | H | $SO_2$ | $OCH_3$ | $OCH_3$ | N | 165-179 |
| H | H | H | $SO_2$ | Cl | $OCH_3$ | CH | 207-209 |
| H | H | H | Se | $CH_3$ | $CH_3$ | CH | 187-190 |
| H | H | H | Se | $CH_3$ | $OCH_3$ | CH | 165-167 |
| H | H | H | Se | $OCH_3$ | $OCH_3$ | CH | 184-186 |
| H | H | H | Se | $CH_3$ | $CH_3$ | N | 177-181 |
| H | H | H | Se | $CH_3$ | $OCH_3$ | N | 163-166 |
| H | H | H | Se | $OCH_3$ | $OCH_3$ | N | 147-151 |
| H | H | H | Se | Cl | $OCH_3$ | CH | |
| $CH_3$ | H | H | Se | $OCH_3$ | $OCH_3$ | CH | |
| $CH_3$ | H | $CH_3$ | Se | $CH_3$ | $OCH_3$ | CH | |
| H | $CH_3$ | H | Se | $OCH_3$ | $OCH_3$ | CH | |

Table VI (Continued)

| R | $R_1$ | $R_{17}$ | E | X | Y | Z | m.p.(°C) |
|---|---|---|---|---|---|---|---|
| H | H | $CH_3$ | S | $CH_3$ | $CH_3$ | CH | 143-147 |
| H | H | $CH_3$ | S | $CH_3$ | $OCH_3$ | CH | 136-139 |
| H | H | $CH_3$ | S | $OCH_3$ | $OCH_3$ | CH | 148-150 |
| H | H | $CH_3$ | S | $CH_3$ | $CH_3$ | N | 151-153 |
| H | H | $CH_3$ | S | $CH_3$ | $OCH_3$ | N | 164-169 |
| H | H | $CH_3$ | S | $OCH_3$ | $OCH_3$ | N | 154-160 |
| H | H | $CH_3$ | S | Cl | $OCH_3$ | CH | 141-143 |
| H | H | $CH_3$ | Se | $CH_3$ | $CH_3$ | CH | 155-158 |
| H | H | $CH_3$ | Se | $OCH_3$ | $CH_3$ | CH | 159-161 |
| H | H | $CH_3$ | Se | $OCH_3$ | $OCH_3$ | CH | 145-148.5 |
| H | H | $CH_3$ | Se | $CH_3$ | $CH_3$ | N | 173-176 |
| H | H | $CH_3$ | Se | $OCH_3$ | $CH_3$ | N | 185-186.5 |
| H | H | $CH_3$ | Se | $OCH_3$ | $OCH_3$ | N | 182.5-184 |
| H | H | $CH_3$ | Se | Cl | $OCH_3$ | CH | |
| H | H | $CH_3$ | SO | $CH_3$ | $CH_3$ | CH | |
| H | H | $CH_3$ | SO | $CH_3$ | $OCH_3$ | CH | |
| H | H | $CH_3$ | SO | $OCH_3$ | $OCH_3$ | CH | |
| H | H | $CH_3$ | SO | $CH_3$ | $CH_3$ | N | |
| H | H | $CH_3$ | SO | $CH_3$ | $OCH_3$ | N | |
| H | H | $CH_3$ | SO | $OCH_3$ | $OCH_3$ | N | |
| H | H | $CH_3$ | SO | Cl | $OCH_3$ | CH | |
| H | H | $CH_3$ | $SO_2$ | $CH_3$ | $CH_3$ | CH | |
| H | H | $CH_3$ | $SO_2$ | $CH_3$ | $OCH_3$ | CH | |
| H | H | $CH_3$ | $SO_2$ | $OCH_3$ | $OCH_3$ | CH | |
| H | H | $CH_3$ | $SO_2$ | $CH_3$ | $CH_3$ | N | |
| H | H | $CH_3$ | $SO_2$ | $CH_3$ | $OCH_3$ | N | |
| H | H | $CH_3$ | $SO_2$ | $OCH_3$ | $OCH_3$ | N | |
| H | H | $CH_3$ | $SO_2$ | Cl | $OCH_3$ | CH | |

Table VII

General Structure VII

| W | R | $R_1$ | X | Y | Z | m.p.(°C) |
|---|---|---|---|---|---|---|
| $C\equiv CHCH_2CH_3$ | H | H | $CH_3$ | $CH_3$ | CH | |
| $C\equiv CHCH_2CH_3$ | H | H | $CH_3$ | $OCH_3$ | CH | |
| $C\equiv CHCH_2CH_3$ | H | H | $OCH_3$ | $OCH_3$ | CH | |
| $C\equiv CHCH_2CH_3$ | H | H | $CH_3$ | $CH_3$ | N | |
| $C\equiv CHCH_2CH_3$ | H | H | $CH_3$ | $OCH_3$ | N | |
| $C\equiv CHCH_2CH_3$ | H | H | $OCH_3$ | $OCH_3$ | N | |
| $C\equiv CHCH_2CH_3$ | H | H | Cl | $OCH_3$ | CH | |
| $C\equiv C-CH_2Ph$ | H | H | $CH_3$ | $CH_3$ | CH | |
| $C\equiv C-CH_2Ph$ | H | H | $CH_3$ | $OCH_3$ | CH | |
| $C\equiv C-CH_2Ph$ | H | H | $OCH_3$ | $OCH_3$ | CH | |
| $C\equiv C-CH_2Ph$ | H | H | $CH_3$ | $CH_3$ | N | |
| $C\equiv C-CH_2Ph$ | H | H | $CH_3$ | $OCH_3$ | N | |
| $C\equiv C-CH_2Ph$ | H | H | $OCH_3$ | $OCH_3$ | N | |
| $C\equiv C-CH_2Ph$ | H | H | Cl | $CH_3$ | CH | |
| $C\equiv C-CH_2Br$ | H | H | $CH_3$ | $CH_3$ | CH | |
| $C\equiv C-CH_2Br$ | H | H | $CH_3$ | $OCH_3$ | CH | |
| $C\equiv C-CH_2Br$ | H | H | $OCH_3$ | $OCH_3$ | CH | |
| $C\equiv C-CH_2Br$ | H | H | $CH_3$ | $CH_3$ | N | |
| $C\equiv C-CH_2Br$ | H | H | $CH_3$ | $OCH_3$ | N | |
| $C\equiv C-CH_2Br$ | H | H | $OCH_3$ | $OCH_3$ | N | |
| $C\equiv C-CH_2Br$ | H | H | Cl | $OCH_3$ | CH | |
| $C\equiv C-Ph$ | H | H | $CH_3$ | $CH_3$ | CH | |
| $C\equiv C-Ph$ | H | H | $CH_3$ | $OCH_3$ | CH | |
| $C\equiv C-Ph$ | H | H | $OCH_3$ | $OCH_3$ | CH | |
| $C\equiv C-Ph$ | H | H | $CH_3$ | $CH_3$ | N | |
| $C\equiv C-Ph$ | H | H | $CH_3$ | $OCH_3$ | N | |
| $C\equiv C-Ph$ | H | H | $OCH_3$ | $OCH_3$ | N | |
| $C\equiv C-Ph$ | H | H | Cl | $OCH_3$ | CH | |
| $C\equiv CCH_2OCH_3$ | $CH_3$ | H | $CH_3$ | $OCH_3$ | CH | |

Table VII (Continued)

| W | R | $R_1$ | X | Y | Z | m.p.(°C) |
|---|---|---|---|---|---|---|
| $C{\equiv}CCH_2OCH_3$ | $CH_3$ | H | $OCH_3$ | $OCH_3$ | CH | |
| $C{\equiv}CCH_2OCH_3$ | $CH_3$ | H | $CH_3$ | $CH_3$ | N | |
| $C{\equiv}CCH_2OCH_3$ | $CH_3$ | H | $CH_3$ | $OCH_3$ | N | |
| $C{\equiv}CCH_2OCH_3$ | $CH_3$ | H | $OCH_3$ | $OCH_3$ | N | |
| $C{\equiv}CCH_2OCH_3$ | $CH_3$ | H | Cl | $OCH_3$ | CH | |
| $C{\equiv}CCH_2OCH_3$ | H | $CH_3$ | $OCH_3$ | $OCH_3$ | CH | |
| $C{\equiv}CCH_2Cl$ | H | $CH_3$ | $OCH_3$ | $OCH_3$ | N | |
| $C{\equiv}CCH_2Cl$ | H | $CH_3$ | $OCH_3$ | $CH_3$ | CH | |
| $C{\equiv}C-CH_2CH_3$ | H | $SCH_3$ | $OCH_3$ | $CH_3$ | CH | |
| $C{\equiv}C-CH_2CH_3$ | H | $SCH_3$ | $OCH_3$ | $OCH_3$ | N | |
| $C{\equiv}C-CH_2CH_3$ | H | Cl | $OCH_3$ | $OCH_3$ | N | |
| $C{\equiv}C-CH_2CH_3$ | H | $OCH_3$ | $OCH_3$ | $CH_3$ | N | |
| $C{\equiv}C-CH_2CH_3$ | H | $OCH_3$ | $OCH_3$ | $OCH_3$ | N | |
| $C{\equiv}C-CH_2CH_3$ | H | $OCH_3$ | $CH_3$ | $OCH_3$ | CH | |
| $C{\equiv}C-CH_2CH_3$ | H | $OCH_3$ | Cl | $OCH_3$ | CH | |

Table VIII

General Structure VIII

| L | n | W | R | $R_1$ | $R_2$ | X | Y | Z | m.p (°C) |
|---|---|---|---|---|---|---|---|---|---|
| L-2 | 0 | $CH=CH_2$ | H | H | - | $CH_3$ | $CH_3$ | CH | |
| L-2 | 0 | $CH=CH_2$ | H | H | - | $CH_3$ | $OCH_3$ | CH | |
| L-2 | 0 | $CH=CH_2$ | H | H | - | $OCH_3$ | $OCH_3$ | CH | |
| L-2 | 0 | $CH=CH_2$ | H | H | - | $CH_3$ | $CH_3$ | N | |
| L-2 | 0 | $CH=CH_2$ | H | H | - | $CH_3$ | $OCH_3$ | N | |
| L-2 | 0 | $CH=CH_2$ | H | H | - | $OCH_3$ | $OCH_3$ | N | |
| L-2 | 0 | $CH=CH_2$ | H | H | - | Cl | $OCH_3$ | CH | |
| L-2 | 0 | $C(CH_3)=CHCH_3$ | H | H | - | $CH_3$ | $CH_3$ | CH | |
| L-2 | 0 | $C(CH_3)=CHCH_3$ | H | H | - | $CH_3$ | $OCH_3$ | CH | |
| L-2 | 0 | $C(CH_3)=CHCH_3$ | H | H | - | $OCH_3$ | $OCH_3$ | CH | |
| L-2 | 0 | $C(CH_3)=CHCH_3$ | H | H | - | $CH_3$ | $CH_3$ | N | |
| L-2 | 0 | $C(CH_3)=CHCH_3$ | H | H | - | $CH_3$ | $OCH_3$ | N | |
| L-2 | 0 | $C(CH_3)=CHCH_3$ | H | H | - | $OCH_3$ | $OCH_3$ | N | |
| L-2 | 0 | $C(CH_3)=CHCH_3$ | H | H | - | Cl | $OCH_3$ | CH | |
| L-2 | 0 | $CH=CHCH_2CH_3$ | H | H | - | $CH_3$ | $CH_3$ | CH | |
| L-2 | 0 | $CH=CHCH_2CH_3$ | H | H | - | $CH_3$ | $OCH_3$ | CH | |
| L-2 | 0 | $CH=CHCH_2CH_3$ | H | H | - | $OCH_3$ | $OCH_3$ | CH | |
| L-2 | 0 | $CH=CHCH_2CH_3$ | H | H | - | $CH_3$ | $CH_3$ | N | |
| L-2 | 0 | $CH=CHCH_2CH_3$ | H | H | - | $CH_3$ | $OCH_3$ | N | |
| L-2 | 0 | $CH=CHCH_2CH_3$ | H | H | - | $OCH_3$ | $OCH_3$ | N | |
| L-2 | 0 | $CH=CHCH_2CH_3$ | H | H | - | Cl | $OCH_3$ | CH | |
| L-2 | 0 | $C(CH_3)=CHCH_2CH_3$ | H | H | - | $CH_3$ | $CH_3$ | CH | |
| L-2 | 0 | $C(CH_3)=CHCH_2CH_3$ | H | H | - | $CH_3$ | $OCH_3$ | CH | |
| L-2 | 0 | $C(CH_3)=CHCH_2CH_3$ | H | H | - | $OCH_3$ | $OCH_3$ | CH | |
| L-2 | 0 | $C(CH_3)=CHCH_2CH_3$ | H | H | - | $CH_3$ | $CH_3$ | N | |
| L-2 | 0 | $C(CH_3)=CHCH_2CH_3$ | H | H | - | $CH_3$ | $OCH_3$ | N | |
| L-2 | 0 | $C(CH_3)=CHCH_2CH_3$ | H | H | - | $OCH_3$ | $OCH_3$ | N | |
| L-2 | 0 | $C(CH_3)=CHCH_2CH_3$ | H | H | - | Cl | $OCH_3$ | CH | |
| L-2 | 0 | $CH=CHCH_2CH_2CH_3$ | H | H | - | $CH_3$ | $CH_3$ | CH | |
| L-2 | 0 | $CH=CHCH_2CH_2CH_3$ | H | H | - | $CH_3$ | $OCH_3$ | CH | |

Table VIII (Continued)

| L | n | W | R | $R_1$ | $R_2$ | X | Y | Z | m.p. (°C) |
|---|---|---|---|---|---|---|---|---|---|
| L-2 | 0 | $CH=CHCH_2CH_2CH_3$ | H | H | - | $OCH_3$ | $OCH_3$ | CH | |
| L-2 | 0 | $CH=CHCH_2CH_2CH_3$ | H | H | - | $CH_3$ | $CH_3$ | N | |
| L-2 | 0 | $CH=CHCH_2CH_2CH_3$ | H | H | - | $CH_3$ | $OCH_3$ | N | |
| L-2 | 0 | $CH=CHCH_2CH_2CH_3$ | H | H | - | $OCH_3$ | $OCH_3$ | N | |
| L-2 | 0 | $CH=CHCH_2CH_2CH_3$ | H | H | - | Cl | $OCH_3$ | CH | |
| L-2 | 0 | CH=CH-CN | H | H | - | $CH_3$ | $CH_3$ | CH | |
| L-2 | 0 | CH=CH-CN | H | H | - | $CH_3$ | $OCH_3$ | CH | |
| L-2 | 0 | CH=CH-CN | H | H | - | $OCH_3$ | $OCH_3$ | CH | |
| L-2 | 0 | CH=CH-CN | H | H | - | $CH_3$ | $CH_3$ | N | |
| L-2 | 0 | CH=CH-CN | H | H | - | $CH_3$ | $OCH_3$ | N | |
| L-2 | 0 | CH=CH-CN | H | H | - | $OCH_3$ | $OCH_3$ | N | |
| L-2 | 0 | CH=CH-CN | H | H | - | Cl | $OCH_3$ | CH | |
| L-2 | 0 | $CH=CH-OCH_3$ | H | H | - | $CH_3$ | $CH_3$ | CH | |
| L-2 | 0 | $CH=CH-OCH_3$ | H | H | - | $CH_3$ | $OCH_3$ | CH | |
| L-2 | 0 | $CH=CH-OCH_3$ | H | H | - | $OCH_3$ | $OCH_3$ | CH | |
| L-2 | 0 | $CH=CH-OCH_3$ | H | H | - | $CH_3$ | $CH_3$ | N | |
| L-2 | 0 | $CH=CH-OCH_3$ | H | H | - | $CH_3$ | $OCH_3$ | N | |
| L-2 | 0 | $CH=CH-OCH_3$ | H | H | - | $OCH_3$ | $OCH_3$ | N | |
| L-2 | 0 | $CH=CH-OCH_3$ | H | H | - | Cl | $OCH_3$ | CH | |
| L-2 | 0 | CH=CH-Br | H | H | - | $CH_3$ | $CH_3$ | CH | |
| L-2 | 0 | CH=CH-Br | H | H | - | $CH_3$ | $OCH_3$ | CH | |
| L-2 | 0 | CH=CH-Br | H | H | - | $OCH_3$ | $OCH_3$ | CH | |
| L-2 | 0 | CH=CH-Br | H | H | - | $CH_3$ | $CH_3$ | N | |
| L-2 | 0 | CH=CH-Br | H | H | - | $CH_3$ | $OCH_3$ | N | |
| L-2 | 0 | CH=CH-Br | H | H | - | $OCH_3$ | $OCH_3$ | N | |
| L-2 | 0 | CH=CH-Br | H | H | - | Cl | $OCH_3$ | CH | |
| L-2 | 0 | $CH=CH-CF_3$ | H | H | - | $CH_3$ | $CH_3$ | CH | |
| L-2 | 0 | $CH=CH-CF_3$ | H | H | - | $CH_3$ | $OCH_3$ | CH | |
| L-2 | 0 | $CH=CH-CF_3$ | H | H | - | $OCH_3$ | $OCH_3$ | CH | |
| L-2 | 0 | $CH=CH-CF_3$ | H | H | - | $CH_3$ | $CH_3$ | N | |
| L-2 | 0 | $CH=CH-CF_3$ | H | H | - | $CH_3$ | $OCH_3$ | N | |

Table VIII (Continued)

| L | n | W | R | $R_1$ | $R_2$ | X | Y | Z | m.p. (°C) |
|---|---|---|---|---|---|---|---|---|---|
| L-2 | 0 | $CH=CH-CF_3$ | H | H | - | $OCH_3$ | $OCH_3$ | N | |
| L-2 | 0 | $CH=CH-CF_3$ | H | H | - | Cl | $OCH_3$ | CH | |
| L-2 | 0 | $C(CH_3)=CH-OCH_3$ | H | H | - | $CH_3$ | $CH_3$ | CH | |
| L-2 | 0 | $C(CH_3)=CH-OCH_3$ | H | H | - | $CH_3$ | $OCH_3$ | CH | |
| L-2 | 0 | $C(CH_3)=CH-OCH_3$ | H | H | - | $OCH_3$ | $OCH_3$ | CH | |
| L-2 | 0 | $C(CH_3)=CH-OCH_3$ | H | H | - | $CH_3$ | $CH_3$ | N | |
| L-2 | 0 | $C(CH_3)=CH-OCH_3$ | H | H | - | $CH_3$ | $OCH_3$ | N | |
| L-2 | 0 | $C(CH_3)=CH-OCH_3$ | H | H | - | $OCH_3$ | $OCH_3$ | N | |
| L-2 | 0 | $C(CH_3)=CH-OCH_3$ | H | H | - | Cl | $OCH_3$ | CH | |
| L-2 | 0 | CH=CHPh | H | H | - | $CH_3$ | $CH_3$ | CH | |
| L-2 | 0 | CH=CHPh | H | H | - | $CH_3$ | $OCH_3$ | CH | |
| L-2 | 0 | CH=CHPh | H | H | - | $OCH_3$ | $OCH_3$ | CH | |
| L-2 | 0 | CH=CHPh | H | H | - | $CH_3$ | $CH_3$ | N | |
| L-2 | 0 | CH=CHPh | H | H | - | $CH_3$ | $OCH_3$ | N | |
| L-2 | 0 | CH=CHPh | H | H | - | $OCH_3$ | $OCH_3$ | N | |
| L-2 | 0 | CH=CHPh | H | H | - | Cl | $OCH_3$ | CH | |
| L-2 | 0 | $C(CH_3)=CH-Ph$ | H | H | - | $CH_3$ | $CH_3$ | CH | |
| L-2 | 0 | $C(CH_3)=CH-Ph$ | H | H | - | $CH_3$ | $OCH_3$ | CH | |
| L-2 | 0 | $C(CH_3)=CH-Ph$ | H | H | - | $OCH_3$ | $OCH_3$ | CH | |
| L-2 | 0 | $C(CH_3)=CH-Ph$ | H | H | - | $CH_3$ | $CH_3$ | N | |
| L-2 | 0 | $C(CH_3)=CH-Ph$ | H | H | - | $CH_3$ | $OCH_3$ | N | |
| L-2 | 0 | $C(CH_3)=CH-Ph$ | H | H | - | $OCH_3$ | $OCH_3$ | N | |
| L-2 | 0 | $C(CH_3)=CH-Ph$ | H | H | - | Cl | $OCH_3$ | CH | |
| L-2 | 0 | $C=CH-(CH_2)_3$ | H | H | - | $CH_3$ | $CH_3$ | CH | 186-190 |
| L-2 | 0 | $C=CH-(CH_2)_3$ | H | H | - | $CH_3$ | $OCH_3$ | CH | 182-185 |
| L-2 | 0 | $C=CH-(CH_2)_3$ | H | H | - | $OCH_3$ | $OCH_3$ | CH | 178-180 |
| L-2 | 0 | $C=CH-(CH_2)_3$ | H | H | - | $CH_3$ | $CH_3$ | N | |
| L-2 | 0 | $C=CH-(CH_2)_3$ | H | H | - | $CH_3$ | $OCH_3$ | N | 152-156 |
| L-2 | 0 | $C=CH-(CH_2)_3$ | H | H | - | $OCH_3$ | $OCH_3$ | N | 145-148 |
| L-2 | 0 | $C=CH-(CH_2)_3$ | H | H | - | Cl | $OCH_3$ | CH | 192-195 |

Table VIII (Continued)

| L | n | W | R | $R_1$ | $R_2$ | X | Y | Z | m.p. (°C) |
|---|---|---|---|---|---|---|---|---|---|
| L-2 | 0 | $C=CH-(CH_2)_4$ | H | H | - | $CH_3$ | $CH_3$ | CH | 208-211 |
| L-2 | 0 | $C=CH-(CH_2)_4$ | H | H | - | $CH_3$ | $OCH_3$ | CH | 190-192 |
| L-2 | 0 | $C=CH-(CH_2)_4$ | H | H | - | $OCH_3$ | $OCH_3$ | CH | 161-164 |
| L-2 | 0 | $C=CH-(CH_2)_4$ | H | H | - | $NHCH_3$ | $OCH_2CH_3$ | N | 180-185 |
| L-2 | 0 | $C=CH-(CH_2)_4$ | H | H | - | $CH_3$ | $OCH_3$ | N | 169-171 |
| L-2 | 0 | $C=CH-(CH_2)_4$ | H | H | - | $OCH_3$ | $OCH_3$ | N | 154-161 |
| L-2 | 0 | $C=CH-(CH_2)_4$ | H | H | - | Cl | $OCH_3$ | CH | 167-169 |
| L-2 | 0 | $C=CH-(CH_2)_5$ | H | H | - | $CH_3$ | $CH_3$ | CH | |
| L-2 | 0 | $C=CH-(CH_2)_5$ | H | H | - | $CH_3$ | $OCH_3$ | CH | |
| L-2 | 0 | $C=CH-(CH_2)_5$ | H | H | - | $OCH_3$ | $OCH_3$ | CH | |
| L-2 | 0 | $C=CH-(CH_2)_5$ | H | H | - | $CH_3$ | $CH_3$ | N | |
| L-2 | 0 | $C=CH-(CH_2)_5$ | H | H | - | $CH_3$ | $OCH_3$ | N | |
| L-2 | 0 | $C=CH-(CH_2)_5$ | H | H | - | $OCH_3$ | $OCH_3$ | N | |
| L-2 | 0 | $C=CH-(CH_2)_5$ | H | H | - | Cl | $OCH_3$ | CH | |
| L-2 | 0 | C(CN)=CHCN | H | H | - | $CH_3$ | $CH_3$ | CH | |
| L-2 | 0 | C(CN)=CHCN | H | H | - | $CH_3$ | $OCH_3$ | CH | |
| L-2 | 0 | C(CN)=CHCN | H | H | - | $OCH_3$ | $OCH_3$ | CH | |
| L-2 | 0 | C(CN)=CHCN | H | H | - | $CH_3$ | $CH_3$ | N | |
| L-2 | 0 | C(CN)=CHCN | H | H | - | $CH_3$ | $OCH_3$ | N | |
| L-2 | 0 | C(CN)=CHCN | H | H | - | $OCH_3$ | $OCH_3$ | N | |
| L-2 | 0 | C(CN)=CHCN | H | H | - | Cl | $OCH_3$ | CH | |
| L-2 | 0 | $CH=CH-SO_2N(CH_3)_2$ | H | H | - | $CH_3$ | $CH_3$ | CH | |
| L-2 | 0 | $CH=CH-SO_2N(CH_3)_2$ | H | H | - | $CH_3$ | $OCH_3$ | CH | |
| L-2 | 0 | $CH=CH-SO_2N(CH_3)_2$ | H | H | - | $OCH_3$ | $OCH_3$ | CH | |
| L-2 | 0 | $CH=CH-SO_2N(CH_3)_2$ | H | H | - | $CH_3$ | $CH_3$ | N | |
| L-2 | 0 | $CH=CH-SO_2N(CH_3)_2$ | H | H | - | $CH_3$ | $OCH_3$ | N | |
| L-2 | 0 | $CH=CH-SO_2N(CH_3)_2$ | H | H | - | $OCH_3$ | $OCH_3$ | N | |
| L-2 | 0 | $CH=CH-SO_2N(CH_3)_2$ | H | H | - | Cl | $OCH_3$ | CH | |

Table VIII (Continued)

| L | n | W | R | $R_1$ | $R_2$ | X | Y | Z | m.p. (°C) |
|---|---|---|---|---|---|---|---|---|---|
| L-2 | 0 | $\overline{C=CH-(CH_2)_4}$ | $CH_3$ | H | - | $OCH_3$ | $OCH_3$ | CH | |
| L-2 | 0 | $\overline{C=CH-(CH_2)_4}$ | H | $CH_3$ | - | $OCH_3$ | $OCH_3$ | CH | |
| L-2 | 0 | $C(=CH_2)-CH_2CH_2CH(NO_2)CH_3$ | H | H | - | $OCH_3$ | $OCH_3$ | CH | |
| L-2 | 0 | $CH=CHCH_2OSO_2Ph$ | H | H | - | $OCH_3$ | $OCH_3$ | CH | |
| L-2 | 0 | $CH=CHCH_2OSO_2CH_3$ | H | H | - | $OCH_3$ | $OCH_3$ | CH | |
| L-2 | 0 | $CH=CHSCH_3$ | H | H | - | $OCH_3$ | $OCH_3$ | CH | |
| L-2 | 0 | $C(CH_3)=CH-CH_2OH$ | H | H | - | $CH_3$ | $CH_3$ | CH | 185-192 |
| L-2 | 0 | $C(CH_3)=CH-CH_2OH$ | H | H | - | $CH_3$ | $OCH_3$ | CH | 189-194 |
| L-2 | 0 | $C(CH_3)=CH-CH_2OH$ | H | H | - | $OCH_3$ | $OCH_3$ | CH | 142-149 |
| L-2 | 0 | $C(CH_3)=CH-CH_2OH$ | H | H | - | $CH_3$ | $OCH_3$ | N | 145-150 |
| L-2 | 0 | $C(CH_3)=CH-CH_2OH$ | H | H | - | $OCH_3$ | $OCH_3$ | N | 138-144 |
| L-2 | 0 | $C(CH_3)=CH-CH_2OH$ | H | H | - | Cl | $OCH_3$ | CH | 123-130 |
| L-2 | 0 | $CH=CH-NO_2$ | H | H | - | $CH_3$ | $CH_3$ | CH | |
| L-2 | 0 | $CH=CH-NO_2$ | H | H | - | $CH_3$ | $OCH_3$ | CH | |
| L-2 | 0 | $CH=CH-NO_2$ | H | H | - | $OCH_3$ | $OCH_3$ | CH | 86-91 |
| L-2 | 0 | $CH=CH-NO_2$ | H | H | - | $CH_3$ | $OCH_3$ | N | 182-188 |
| L-2 | 0 | $CH=CH-NO_2$ | H | H | - | $OCH_3$ | $OCH_3$ | N | |
| L-2 | 0 | $CH=CH-NO_2$ | H | H | - | Cl | $OCH_3$ | CH | 88-93 |
| L-2 | 0 | $CH=C(CN)_2$ | H | H | - | $CH_3$ | $CH_3$ | CH | |
| L-2 | 0 | $CH=C(CN)_2$ | H | H | - | $CH_3$ | $OCH_3$ | CH | |
| L-2 | 0 | $CH=C(CN)_2$ | H | H | - | $OCH_3$ | $OCH_3$ | CH | |
| L-2 | 0 | $CH=C(CN)_2$ | H | H | - | $CH_3$ | $OCH_3$ | N | |
| L-2 | 0 | $CH=C(CN)_2$ | H | H | - | $OCH_3$ | $OCH_3$ | N | |
| L-2 | 0 | $CH=C(CN)_2$ | H | H | - | Cl | $OCH_3$ | CH | |
| L-2 | 0 | $C\equiv CH$ | H | H | - | $CH_3$ | $CH_3$ | CH | |
| L-2 | 0 | $C\equiv CH$ | H | H | - | $CH_3$ | $OCH_3$ | CH | |

Table VIII (Continued)

| L | n | W | R | $R_1$ | $R_2$ | X | Y | Z | m.p. (°C) |
|---|---|---|---|---|---|---|---|---|---|
| L-2 | 0 | C≡CH | H | H | - | $OCH_3$ | $OCH_3$ | CH | |
| L-2 | 0 | C≡CH | H | H | - | $CH_3$ | $CH_3$ | N | |
| L-2 | 0 | C≡CH | H | H | - | $CH_3$ | $OCH_3$ | N | |
| L-2 | 0 | C≡CH | H | H | - | $OCH_3$ | $OCH_3$ | N | |
| L-2 | 0 | C≡CH | H | H | - | Cl | $OCH_3$ | CH | |
| L-2 | 0 | C≡C-Ph | H | H | - | $CH_3$ | $CH_3$ | CH | |
| L-2 | 0 | C≡C-Ph | H | H | - | $CH_3$ | $OCH_3$ | CH | |
| L-2 | 0 | C≡C-Ph | H | H | - | $OCH_3$ | $OCH_3$ | CH | |
| L-2 | 0 | C≡C-Ph | H | H | - | $CH_3$ | $CH_3$ | N | |
| L-2 | 0 | C≡C-Ph | H | H | - | $CH_3$ | $OCH_3$ | N | |
| L-2 | 0 | C≡C-Ph | H | H | - | $OCH_3$ | $OCH_3$ | N | |
| L-2 | 0 | C≡C-Ph | H | H | - | Cl | $OCH_3$ | CH | |
| L-2 | 0 | $C{\equiv}C-CH_3$ | H | H | - | $CH_3$ | $CH_3$ | CH | |
| L-2 | 0 | $C{\equiv}C-CH_3$ | H | H | - | $CH_3$ | $OCH_3$ | CH | |
| L-2 | 0 | $C{\equiv}C-CH_3$ | H | H | - | $OCH_3$ | $OCH_3$ | CH | |
| L-2 | 0 | $C{\equiv}C-CH_3$ | H | H | - | $CH_3$ | $CH_3$ | N | |
| L-2 | 0 | $C{\equiv}C-CH_3$ | H | H | - | $CH_3$ | $OCH_3$ | N | |
| L-2 | 0 | $C{\equiv}C-CH_3$ | H | H | - | $OCH_3$ | $OCH_3$ | N | |
| L-2 | 0 | $C{\equiv}C-CH_3$ | H | H | - | Cl | $OCH_3$ | CH | |
| L-2 | 0 | $C{\equiv}CCH_2OC(O)CH_3$ | H | H | - | $CH_3$ | $CH_3$ | CH | |
| L-2 | 0 | $C{\equiv}CCH_2OC(O)CH_3$ | H | H | - | $CH_3$ | $OCH_3$ | CH | |
| L-2 | 0 | $C{\equiv}CCH_2OC(O)CH_3$ | H | H | - | $OCH_3$ | $OCH_3$ | CH | |
| L-2 | 0 | $C{\equiv}CCH_2OC(O)CH_3$ | H | H | - | $CH_3$ | $CH_3$ | N | |
| L-2 | 0 | $C{\equiv}CCH_2OC(O)CH_3$ | H | H | - | $CH_3$ | $OCH_3$ | N | |
| L-2 | 0 | $C{\equiv}CCH_2OC(O)CH_3$ | H | H | - | $OCH_3$ | $OCH_3$ | N | |
| L-2 | 0 | $C{\equiv}CCH_2OC(O)CH_3$ | H | H | - | Cl | $OCH_3$ | CH | |
| L-2 | 0 | $C{\equiv}CCH_2OH$ | H | H | - | $CH_3$ | $CH_3$ | CH | |
| L-2 | 0 | $C{\equiv}CCH_2OH$ | H | H | - | $CH_3$ | $OCH_3$ | CH | |
| L-2 | 0 | $C{\equiv}CCH_2OH$ | H | H | - | $OCH_3$ | $OCH_3$ | CH | |
| L-2 | 0 | $C{\equiv}CCH_2OH$ | H | H | - | $CH_3$ | $CH_3$ | N | |
| L-2 | 0 | $C{\equiv}CCH_2OH$ | H | H | - | $CH_3$ | $OCH_3$ | N | |
| L-2 | 0 | $C{\equiv}CCH_2OH$ | H | H | - | $OCH_3$ | $OCH_3$ | N | |
| L-2 | 0 | $C{\equiv}CCH_2OH$ | H | H | - | Cl | $OCH_3$ | CH | |

Table VIII (Continued)

| L | n | W | R | $R_1$ | $R_2$ | X | Y | Z | m.p. (°C) |
|---|---|---|---|---|---|---|---|---|---|
| L-2 | 0 | $C\equiv CCH_2Cl$ | H | H | - | $CH_3$ | $CH_3$ | CH | |
| L-2 | 0 | $C\equiv CCH_2Cl$ | H | H | - | $CH_3$ | $OCH_3$ | CH | |
| L-2 | 0 | $C\equiv CCH_2Cl$ | H | H | - | $OCH_3$ | $OCH_3$ | CH | |
| L-2 | 0 | $C\equiv CCH_2Cl$ | H | H | - | $CH_3$ | $CH_3$ | N | |
| L-2 | 0 | $C\equiv CCH_2Cl$ | H | H | - | $CH_3$ | $OCH_3$ | N | |
| L-2 | 0 | $C\equiv CCH_2Cl$ | H | H | - | $OCH_3$ | $OCH_3$ | N | |
| L-2 | 0 | $C\equiv CCH_2Cl$ | H | H | - | Cl | $OCH_3$ | CH | |
| L-2 | 0 | $C\equiv C-CH_2Br$ | H | H | - | $CH_3$ | $CH_3$ | CH | |
| L-2 | 0 | $C\equiv C-CH_2Br$ | H | H | - | $CH_3$ | $OCH_3$ | CH | |
| L-2 | 0 | $C\equiv C-CH_2Br$ | H | H | - | $OCH_3$ | $OCH_3$ | CH | |
| L-2 | 0 | $C\equiv C-CH_2Br$ | H | H | - | $CH_3$ | $CH_3$ | N | |
| L-2 | 0 | $C\equiv C-CH_2Br$ | H | H | - | $CH_3$ | $OCH_3$ | N | |
| L-2 | 0 | $C\equiv C-CH_2Br$ | H | H | - | $OCH_3$ | $OCH_3$ | N | |
| L-2 | 0 | $C\equiv C-CH_2Br$ | H | H | - | Cl | $OCH_3$ | CH | |
| L-2 | 0 | $C\equiv C-CH_2CN$ | H | H | - | $CH_3$ | $CH_3$ | CH | |
| L-2 | 0 | $C\equiv C-CH_2CN$ | H | H | - | $CH_3$ | $OCH_3$ | CH | |
| L-2 | 0 | $C\equiv C-CH_2CN$ | H | H | - | $OCH_3$ | $OCH_3$ | CH | |
| L-2 | 0 | $C\equiv C-CH_2CN$ | H | H | - | $CH_3$ | $CH_3$ | N | |
| L-2 | 0 | $C\equiv C-CH_2CN$ | H | H | - | $CH_3$ | $OCH_3$ | N | |
| L-2 | 0 | $C\equiv C-CH_2CN$ | H | H | - | $OCH_3$ | $OCH_3$ | N | |
| L-2 | 0 | $C\equiv C-CH_2CN$ | H | H | - | Cl | $OCH_3$ | CH | |
| L-2 | 0 | $C\equiv C-CH_2OCH_3$ | H | H | - | $CH_3$ | $CH_3$ | CH | |
| L-2 | 0 | $C\equiv C-CH_2OCH_3$ | H | H | - | $CH_3$ | $OCH_3$ | CH | |
| L-2 | 0 | $C\equiv C-CH_2OCH_3$ | H | H | - | $OCH_3$ | $OCH_3$ | CH | |
| L-2 | 0 | $C\equiv C-CH_2OCH_3$ | H | H | - | $CH_3$ | $CH_3$ | N | |
| L-2 | 0 | $C\equiv C-CH_2OCH_3$ | H | H | - | $CH_3$ | $OCH_3$ | N | |
| L-2 | 0 | $C\equiv C-CH_2OCH_3$ | H | H | - | $OCH_3$ | $OCH_3$ | N | |
| L-2 | 0 | $C\equiv C-CH_2OCH_3$ | H | H | - | Cl | $OCH_3$ | CH | |
| L-2 | 0 | $C\equiv CCH_2OSO_2CH_3$ | H | H | - | $CH_3$ | $CH_3$ | CH | |
| L-2 | 0 | $C\equiv CCH_2OSO_2CH_3$ | H | H | - | $CH_3$ | $OCH_3$ | CH | |
| L-2 | 0 | $C\equiv CCH_2OSO_2CH_3$ | H | H | - | $OCH_3$ | $OCH_3$ | CH | |
| L-2 | 0 | $C\equiv CCH_2OSO_2CH_3$ | H | H | - | $CH_3$ | $CH_3$ | N | |

Table VIII (Continued)

| L | n | W | R | $R_1$ | $R_2$ | X | Y | Z | m.p. (°C) |
|---|---|---|---|---|---|---|---|---|---|
| L-2 | 0 | $C\equiv CCH_2OSO_2CH_3$ | H | H | - | $CH_3$ | $OCH_3$ | N | |
| L-2 | 0 | $C\equiv CCH_2OSO_2CH_3$ | H | H | - | $OCH_3$ | $OCH_3$ | N | |
| L-2 | 0 | $C\equiv CCH_2OSO_2CH_3$ | H | H | - | Cl | $OCH_3$ | CH | |
| L-2 | 0. | $C\equiv CCH_2SCH_3$ | H | H | - | $CH_3$ | $CH_3$ | CH | |
| L-2 | 0 | $C\equiv CCH_2SCH_3$ | H | H | - | $CH_3$ | $OCH_3$ | CH | |
| L-2 | 0 | $C\equiv CCH_2SCH_3$ | H | H | - | $OCH_3$ | $OCH_3$ | CH | |
| L-2 | 0 | $C\equiv CCH_2SCH_3$ | H | H | - | $CH_3$ | $CH_3$ | N | |
| L-2 | 0 | $C\equiv CCH_2SCH_3$ | H | H | - | $CH_3$ | $OCH_3$ | N | |
| L-2 | 0 | $C\equiv CCH_2SCH_3$ | H | H | - | $OCH_3$ | $OCH_3$ | N | |
| L-2 | 0 | $C\equiv CCH_2SCH_3$ | H | H | - | Cl | $OCH_3$ | CH | |
| L-2 | 1 | $HC=CH_2$ | H | H | - | $CH_3$ | $CH_3$ | CH | |
| L-2 | 1 | $HC=CH_2$ | H | H | - | $CH_3$ | $OCH_3$ | CH | |
| L-2 | 1 | $HC=CH_2$ | H | H | - | $OCH_3$ | $OCH_3$ | CH | |
| L-2 | 1 | $HC=CH_2$ | H | H | - | $CH_3$ | $CH_3$ | N | |
| L-2 | 1 | $HC=CH_2$ | H | H | - | $CH_3$ | $OCH_3$ | N | |
| L-2 | 1 | $HC=CH_2$ | H | H | - | $OCH_3$ | $OCH_3$ | N | |
| L-2 | 1 | $HC=CH_2$ | H | H | - | Cl | $OCH_3$ | CH | |
| L-2 | 1 | $BrC=CH_2$ | H | H | - | $CH_3$ | $CH_3$ | CH | |
| L-2 | 1 | $BrC=CH_2$ | H | H | - | $CH_3$ | $OCH_3$ | CH | |
| L-2 | 1 | $BrC=CH_2$ | H | H | - | $OCH_3$ | $OCH_3$ | CH | |
| L-2 | 1 | $BrC=CH_2$ | H | H | - | $CH_3$ | $CH_3$ | N | |
| L-2 | 1 | $BrC=CH_2$ | H | H | - | $CH_3$ | $OCH_3$ | N | |
| L-2 | 1 | $BrC=CH_2$ | H | H | - | $OCH_3$ | $OCH_3$ | N | |
| L-2 | 1 | $BrC=CH_2$ | H | H | - | Cl | $OCH_3$ | CH | |
| L-2 | 1 | $HC=C(CH_3)_2$ | H | H | - | $CH_3$ | $CH_3$ | CH | |
| L-2 | 1 | $HC=C(CH_3)_2$ | H | H | - | $CH_3$ | $OCH_3$ | CH | |
| L-2 | 1 | $HC=C(CH_3)_2$ | H | H | - | $OCH_3$ | $OCH_3$ | CH | |
| L-2 | 1 | $HC=C(CH_3)_2$ | H | H | - | $CH_3$ | $CH_3$ | N | |
| L-2 | 1 | $HC=C(CH_3)_2$ | H | H | - | $CH_3$ | $OCH_3$ | N | |
| L-2 | 1 | $HC=C(CH_3)_2$ | H | H | - | $OCH_3$ | $OCH_3$ | N | |
| L-2 | 1 | $HC=C(CH_3)_2$ | H | H | - | Cl | $OCH_3$ | CH | |

Table VIII (Continued)

| L | n | W | R | $R_1$ | $R_2$ | X | Y | Z | m.p. (°C) |
|---|---|---|---|---|---|---|---|---|---|
| L-2 | 1 | $HC=C(CH_3)CH_2CH_2CH=C(CH_3)_2$ | H | H | - | $CH_3$ | $CH_3$ | CH | |
| L-2 | 1 | $HC=C(CH_3)CH_2CH_2CH=C(CH_3)_2$ | H | H | - | $CH_3$ | $OCH_3$ | CH | |
| L-2 | 1 | $HC=C(CH_3)CH_2CH_2CH=C(CH_3)_2$ | H | H | - | $OCH_3$ | $OCH_3$ | CH | |
| L-2 | 1 | $HC=C(CH_3)CH_2CH_2CH=C(CH_3)_2$ | H | H | - | $CH_3$ | $CH_3$ | N | |
| L-2 | 1 | $HC=C(CH_3)CH_2CH_2CH=C(CH_3)_2$ | H | H | - | $CH_3$ | $OCH_3$ | N | |
| L-2 | 1 | $HC=C(CH_3)CH_2CH_2CH=C(CH_3)_2$ | H | H | - | $OCH_3$ | $OCH_3$ | N | |
| L-2 | 1 | $HC=C(CH_3)CH_2CH_2CH=C(CH_3)_2$ | H | H | - | Cl | $OCH_3$ | CH | |
| L-2 | 1 | HC=CH-Ph | H | H | - | $CH_3$ | $CH_3$ | CH | |
| L-2 | 1 | HC=CH-Ph | H | H | - | $CH_3$ | $OCH_3$ | CH | |
| L-2 | 1 | HC=CH-Ph | H | H | - | $OCH_3$ | $OCH_3$ | CH | |
| L-2 | 1 | HC=CH-Ph | H | H | - | $CH_3$ | $CH_3$ | N | |
| L-2 | 1 | HC=CH-Ph | H | H | - | $CH_3$ | $OCH_3$ | N | |
| L-2 | 1 | HC=CH-Ph | H | H | - | $OCH_3$ | $OCH_3$ | N | |
| L-2 | 1 | HC=CH-Ph | H | H | - | Cl | $OCH_3$ | CH | |
| L-2 | 1 | $HC=CHCH_2Br$ | H | H | - | $CH_3$ | $CH_3$ | CH | |
| L-2 | 1 | $HC=CHCH_2Br$ | H | H | - | $CH_3$ | $OCH_3$ | CH | |
| L-2 | 1 | $HC=CHCH_2Br$ | H | H | - | $OCH_3$ | $OCH_3$ | CH | |
| L-2 | 1 | $HC=CHCH_2Br$ | H | H | - | $CH_3$ | $CH_3$ | N | |
| L-2 | 1 | $HC=CHCH_2Br$ | H | H | - | $CH_3$ | $OCH_3$ | N | |
| L-2 | 1 | $HC=CHCH_2Br$ | H | H | - | $OCH_3$ | $OCH_3$ | N | |
| L-2 | 1 | $HC=CHCH_2Br$ | H | H | - | Cl | $OCH_3$ | CH | |
| L-2 | 1 | C≡CH | H | H | - | $CH_3$ | $CH_3$ | CH | |
| L-2 | 1 | C≡CH | H | H | - | $CH_3$ | $OCH_3$ | CH | |
| L-2 | 1 | C≡CH | H | H | - | $OCH_3$ | $OCH_3$ | CH | |
| L-2 | 1 | C≡CH | H | H | - | $CH_3$ | $CH_3$ | N | |
| L-2 | 1 | C≡CH | H | H | - | $CH_3$ | $OCH_3$ | N | |
| L-2 | 1 | C≡CH | H | H | - | $OCH_3$ | $OCH_3$ | N | |
| L-2 | 1 | C≡CH | H | H | - | Cl | $OCH_3$ | CH | |
| L-2 | 1 | $C≡CCH_3$ | H | H | - | $CH_3$ | $CH_3$ | CH | |
| L-2 | 1 | $C≡CCH_3$ | H | H | - | $CH_3$ | $OCH_3$ | CH | |
| L-2 | 1 | $C≡CCH_3$ | H | H | - | $OCH_3$ | $OCH_3$ | CH | |
| L-2 | 1 | $C≡CCH_3$ | H | H | - | $CH_3$ | $CH_3$ | N | |

Table VIII (Continued)

| L | n | W | R | $R_1$ | $R_2$ | X | Y | Z | m.p. (°C) |
|---|---|---|---|---|---|---|---|---|---|
| L-2 | 1 | $C\equiv CCH_3$ | H | H | - | $CH_3$ | $OCH_3$ | N | |
| L-2 | 1 | $C\equiv CCH_3$ | H | H | - | $OCH_3$ | $OCH_3$ | N | |
| L-2 | 1 | $C\equiv CCH_3$ | H | H | - | Cl | $OCH_3$ | CH | |
| L-2 | 1 | $C\equiv C-CH_2Br$ | H | H | - | $CH_3$ | $CH_3$ | CH | |
| L-2 | 1 | $C\equiv C-CH_2Br$ | H | H | - | $CH_3$ | $OCH_3$ | CH | |
| L-2 | 1 | $C\equiv C-CH_2Br$ | H | H | - | $OCH_3$ | $OCH_3$ | CH | |
| L-2 | 1 | $C\equiv C-CH_2Br$ | H | H | - | $CH_3$ | $CH_3$ | N | |
| L-2 | 1 | $C\equiv C-CH_2Br$ | H | H | - | $CH_3$ | $OCH_3$ | N | |
| L-2 | 1 | $C\equiv C-CH_2Br$ | H | H | - | $OCH_3$ | $OCH_3$ | N | |
| L-2 | 1 | $C\equiv C-CH_2Br$ | H | H | - | Cl | $OCH_3$ | CH | |
| L-2 | 1 | $C\equiv C-Ph$ | H | H | - | $CH_3$ | $CH_3$ | CH | |
| L-2 | 1 | $C\equiv C-Ph$ | H | H | - | $CH_3$ | $OCH_3$ | CH | |
| L-2 | 1 | $C\equiv C-Ph$ | H | H | - | $OCH_3$ | $OCH_3$ | CH | |
| L-2 | 1 | $C\equiv C-Ph$ | H | H | - | $CH_3$ | $CH_3$ | N | |
| L-2 | 1 | $C\equiv C-Ph$ | H | H | - | $CH_3$ | $OCH_3$ | N | |
| L-2 | 1 | $C\equiv C-Ph$ | H | H | - | $OCH_3$ | $OCH_3$ | N | |
| L-2 | 1 | $C\equiv C-Ph$ | H | H | - | Cl | $OCH_3$ | CH | |
| L-2 | 1 | $CH=CH_2$ | $CH_3$ | H | - | $CH_3$ | $CH_3$ | CH | |
| L-2 | 1 | $CH=CH_2$ | $CH_3$ | H | - | $CH_3$ | $OCH_3$ | CH | |
| L-2 | 1 | $CH=CH_2$ | $CH_3$ | H | - | $OCH_3$ | $OCH_3$ | CH | |
| L-2 | 1 | $CH=CH_2$ | $CH_3$ | H | - | $CH_3$ | $CH_3$ | N | |
| L-2 | 1 | $CH=CH_2$ | $CH_3$ | H | - | $CH_3$ | $OCH_3$ | N | |
| L-2 | 1 | $CH=CH_2$ | $CH_3$ | H | - | $OCH_3$ | $OCH_3$ | N | |
| L-2 | 1 | $CH=CH_2$ | $CH_3$ | H | - | Cl | $OCH_3$ | CH | |
| L-2 | 1 | $C\equiv CH$ | $CH_3$ | H | - | $CH_3$ | $CH_3$ | CH | |
| L-2 | 1 | $C\equiv CH$ | $CH_3$ | H | - | $CH_3$ | $OCH_3$ | CH | |
| L-2 | 1 | $C\equiv CH$ | $CH_3$ | H | - | $OCH_3$ | $OCH_3$ | CH | |
| L-2 | 1 | $C\equiv CH$ | $CH_3$ | H | - | $CH_3$ | $CH_3$ | N | |
| L-2 | 1 | $C\equiv CH$ | $CH_3$ | H | - | $CH_3$ | $OCH_3$ | N | |
| L-2 | 1 | $C\equiv CH$ | $CH_3$ | H | - | $OCH_3$ | $OCH_3$ | N | |
| L-2 | 1 | $C\equiv CH$ | $CH_3$ | H | - | Cl | $OCH_3$ | CH | |
| L-2 | 1 | $CH=CH_2$ | H | $CH_3$ | - | $CH_3$ | $CH_3$ | CH | |

Table VIII (Continued)

| L | n | W | R | $R_1$ | $R_2$ | X | Y | Z | m.p. (°C) |
|---|---|---|---|---|---|---|---|---|---|
| L-2 | 1 | $CH=CH_2$ | H | $CH_3$ | - | $CH_3$ | $OCH_3$ | CH | |
| L-2 | 1 | $CH=CH_2$ | H | $CH_3$ | - | $OCH_3$ | $OCH_3$ | CH | |
| L-2 | 1 | $CH=CH_2$ | H | $CH_3$ | - | $CH_3$ | $CH_3$ | N | |
| L-2 | 1 | $CH=CH_2$ | H | $CH_3$ | - | $CH_3$ | $OCH_3$ | N | |
| L-2 | 1 | $CH=CH_2$ | H | $CH_3$ | - | $OCH_3$ | $OCH_3$ | N | |
| L-2 | 1 | $CH=CH_2$ | H | $CH_3$ | - | Cl | $OCH_3$ | CH | |
| L-2 | 1 | $C\equiv CH$ | H | $CH_3$ | - | $OCH_3$ | $OCH_3$ | CH | |
| L-2 | 1 | $C\equiv CH$ | H | $CH_3$ | - | $OCH_3$ | $OCH_3$ | N | |
| L-2 | 1 | $C\equiv CH$ | H | $CH_3$ | - | $OCH_3$ | $CH_3$ | CH | |
| L-3 | 0 | $CH=CH_2$ | H | H | $CH_3$ | $CH_3$ | $CH_3$ | CH | |
| L-3 | 0 | $CH=CH_2$ | H | H | $CH_3$ | $CH_3$ | $OCH_3$ | CH | |
| L-3 | 0 | $CH=CH_2$ | H | H | $CH_3$ | $OCH_3$ | $OCH_3$ | CH | |
| L-3 | 0 | $CH=CH_2$ | H | H | $CH_3$ | $CH_3$ | $CH_3$ | N | |
| L-3 | 0 | $CH=CH_2$ | H | H | $CH_3$ | $CH_3$ | $OCH_3$ | N | |
| L-3 | 0 | $CH=CH_2$ | H | H | $CH_3$ | $OCH_3$ | $OCH_3$ | N | |
| L-3 | 0 | $CH=CH_2$ | H | H | $CH_3$ | Cl | $OCH_3$ | CH | |
| L-3 | 0 | $C(CH_3)=CHCH_3$ | H | H | $CH_3$ | $CH_3$ | $CH_3$ | CH | |
| L-3 | 0 | $C(CH_3)=CHCH_3$ | H | H | $CH_3$ | $CH_3$ | $OCH_3$ | CH | |
| L-3 | 0 | $C(CH_3)=CHCH_3$ | H | H | $CH_3$ | $OCH_3$ | $OCH_3$ | CH | |
| L-3 | 0 | $C(CH_3)=CHCH_3$ | H | H | $CH_3$ | $CH_3$ | $CH_3$ | N | |
| L-3 | 0 | $C(CH_3)=CHCH_3$ | H | H | $CH_3$ | $CH_3$ | $OCH_3$ | N | |
| L-3 | 0 | $C(CH_3)=CHCH_3$ | H | H | $CH_3$ | $OCH_3$ | $OCH_3$ | N | |
| L-3 | 0 | $C(CH_3)=CHCH_3$ | H | H | $CH_3$ | Cl | $OCH_3$ | CH | |
| L-3 | 0 | $CH=CHCH_2CH_3$ | H | H | $CH_3$ | $CH_3$ | $CH_3$ | CH | |
| L-3 | 0 | $CH=CHCH_2CH_3$ | H | H | $CH_3$ | $CH_3$ | $OCH_3$ | CH | |
| L-3 | 0 | $CH=CHCH_2CH_3$ | H | H | $CH_3$ | $OCH_3$ | $OCH_3$ | CH | |
| L-3 | 0 | $CH=CHCH_2CH_3$ | H | H | $CH_3$ | $CH_3$ | $CH_3$ | N | |
| L-3 | 0 | $CH=CHCH_2CH_3$ | H | H | $CH_3$ | $CH_3$ | $OCH_3$ | N | |
| L-3 | 0 | $CH=CHCH_2CH_3$ | H | H | $CH_3$ | $OCH_3$ | $OCH_3$ | N | |
| L-3 | 0 | $CH=CHCH_2CH_3$ | H | H | $CH_3$ | Cl | $OCH_3$ | CH | |
| L-3 | 0 | $C(CH_3)=CHCH_2CH_3$ | H | H | $CH_3$ | $CH_3$ | $CH_3$ | CH | |
| L-3 | 0 | $C(CH_3)=CHCH_2CH_3$ | H | H | $CH_3$ | $CH_3$ | $OCH_3$ | CH | |

Table VIII (Continued)

| L | n | W | R | $R_1$ | $R_2$ | X | Y | Z | m.p. (°C) |
|---|---|---|---|---|---|---|---|---|---|
| L-3 | 0 | $C(CH_3)=CHCH_2CH_3$ | H | H | $CH_3$ | $OCH_3$ | $OCH_3$ | CH | |
| L-3 | 0 | $C(CH_3)=CHCH_2CH_3$ | H | H | $CH_3$ | $CH_3$ | $CH_3$ | N | |
| L-3 | 0 | $C(CH_3)=CHCH_2CH_3$ | H | H | $CH_3$ | $CH_3$ | $OCH_3$ | N | |
| L-3 | 0 | $C(CH_3)=CHCH_2CH_3$ | H | H | $CH_3$ | $OCH_3$ | $OCH_3$ | N | |
| L-3 | 0 | $C(CH_3)=CHCH_2CH_3$ | H | H | $CH_3$ | Cl | $OCH_3$ | CH | |
| L-3 | 0 | $CH=CHCH_2CH_2CH_3$ | H | H | $CH_3$ | $CH_3$ | $CH_3$ | CH | |
| L-3 | 0 | $CH=CHCH_2CH_2CH_3$ | H | H | $CH_3$ | $CH_3$ | $OCH_3$ | CH | |
| L-3 | 0 | $CH=CHCH_2CH_2CH_3$ | H | H | $CH_3$ | $OCH_3$ | $OCH_3$ | CH | |
| L-3 | 0 | $CH=CHCH_2CH_2CH_3$ | H | H | $CH_3$ | $CH_3$ | $CH_3$ | N | |
| L-3 | 0 | $CH=CHCH_2CH_2CH_3$ | H | H | $CH_3$ | $CH_3$ | $OCH_3$ | N | |
| L-3 | 0 | $CH=CHCH_2CH_2CH_3$ | H | H | $CH_3$ | $OCH_3$ | $OCH_3$ | N | |
| L-3 | 0 | $CH=CHCH_2CH_2CH_3$ | H | H | $CH_3$ | Cl | $OCH_3$ | CH | |
| L-3 | 0 | CH=CH-CN | H | H | $CH_3$ | $CH_3$ | $CH_3$ | CH | |
| L-3 | 0 | CH=CH-CN | H | H | $CH_3$ | $CH_3$ | $OCH_3$ | CH | |
| L-3 | 0 | CH=CH-CN | H | H | $CH_3$ | $OCH_3$ | $OCH_3$ | CH | |
| L-3 | 0 | CH=CH-CN | H | H | $CH_3$ | $CH_3$ | $CH_3$ | N | |
| L-3 | 0 | CH=CH-CN | H | H | $CH_3$ | $CH_3$ | $OCH_3$ | N | |
| L-3 | 0 | CH=CH-CN | H | H | $CH_3$ | $OCH_3$ | $OCH_3$ | N | |
| L-3 | 0 | CH=CH-CN | H | H | $CH_3$ | Cl | $OCH_3$ | CH | |
| L-3 | 0 | $CH=CH-OCH_3$ | H | H | $CH_3$ | $CH_3$ | $CH_3$ | CH | |
| L-3 | 0 | $CH=CH-OCH_3$ | H | H | $CH_3$ | $CH_3$ | $OCH_3$ | CH | |
| L-3 | 0 | $CH=CH-OCH_3$ | H | H | $CH_3$ | $OCH_3$ | $OCH_3$ | CH | |
| L-3 | 0 | $CH=CH-OCH_3$ | H | H | $CH_3$ | $CH_3$ | $CH_3$ | N | |
| L-3 | 0 | $CH=CH-OCH_3$ | H | H | $CH_3$ | $CH_3$ | $OCH_3$ | N | |
| L-3 | 0 | $CH=CH-OCH_3$ | H | H | $CH_3$ | $OCH_3$ | $OCH_3$ | N | |
| L-3 | 0 | $CH=CH-OCH_3$ | H | H | $CH_3$ | Cl | $OCH_3$ | CH | |
| L-3 | 0 | CH=CH-Br | H | H | $CH_3$ | $CH_3$ | $CH_3$ | CH | |
| L-3 | 0 | CH=CH-Br | H | H | $CH_3$ | $CH_3$ | $OCH_3$ | CH | |
| L-3 | 0 | CH=CH-Br | H | H | $CH_3$ | $OCH_3$ | $OCH_3$ | CH | |
| L-3 | 0 | CH=CH-Br | H | H | $CH_3$ | $CH_3$ | $CH_3$ | N | |
| L-3 | 0 | CH=CH-Br | H | H | $CH_3$ | $CH_3$ | $OCH_3$ | N | |
| L-3 | 0 | CH=CH-Br | H | H | $CH_3$ | $OCH_3$ | $OCH_3$ | N | |

Table VIII (Continued)

| L | n | W | R | $R_1$ | $R_2$ | X | Y | Z | m.p. (°C) |
|---|---|---|---|---|---|---|---|---|---|
| L-3 | 0 | CH=CH-Br | H | H | $CH_3$ | Cl | $OCH_3$ | CH | |
| L-3 | 0 | $CH=CH-CF_3$ | H | H | $CH_3$ | $CH_3$ | $CH_3$ | CH | |
| L-3 | 0 | $CH=CH-CF_3$ | H | H | $CH_3$ | $CH_3$ | $OCH_3$ | CH | |
| L-3 | 0 | $CH=CH-CF_3$ | H | H | $CH_3$ | $OCH_3$ | $OCH_3$ | CH | |
| L-3 | 0 | $CH=CH-CF_3$ | H | H | $CH_3$ | $CH_3$ | $CH_3$ | N | |
| L-3 | 0 | $CH=CH-CF_3$ | H | H | $CH_3$ | $CH_3$ | $OCH_3$ | N | |
| L-3 | 0 | $CH=CH-CF_3$ | H | H | $CH_3$ | $OCH_3$ | $OCH_3$ | N | |
| L-3 | 0 | $CH=CH-CF_3$ | H | H | $CH_3$ | Cl | $OCH_3$ | CH | |
| L-3 | 0 | $C(CH_3)=CH-OCH_3$ | H | H | $CH_3$ | $CH_3$ | $CH_3$ | CH | |
| L-3 | 0 | $C(CH_3)=CH-OCH_3$ | H | H | $CH_3$ | $CH_3$ | $OCH_3$ | CH | |
| L-3 | 0 | $C(CH_3)=CH-OCH_3$ | H | H | $CH_3$ | $OCH_3$ | $OCH_3$ | CH | |
| L-3 | 0 | $C(CH_3)=CH-OCH_3$ | H | H | $CH_3$ | $CH_3$ | $CH_3$ | N | |
| L-3 | 0 | $C(CH_3)=CH-OCH_3$ | H | H | $CH_3$ | $CH_3$ | $OCH_3$ | N | |
| L-3 | 0 | $C(CH_3)=CH-OCH_3$ | H | H | $CH_3$ | $OCH_3$ | $OCH_3$ | N | |
| L-3 | 0 | $C(CH_3)=CH-OCH_3$ | H | H | $CH_3$ | Cl | $OCH_3$ | CH | |
| L-3 | 0 | CH=CHPh | H | H | $CH_3$ | $CH_3$ | $CH_3$ | CH | |
| L-3 | 0 | CH=CHPh | H | H | $CH_3$ | $CH_3$ | $OCH_3$ | CH | |
| L-3 | 0 | CH=CHPh | H | H | $CH_3$ | $OCH_3$ | $OCH_3$ | CH | |
| L-3 | 0 | CH=CHPh | H | H | $CH_3$ | $CH_3$ | $CH_3$ | N | |
| L-3 | 0 | CH=CHPh | H | H | $CH_3$ | $CH_3$ | $OCH_3$ | N | |
| L-3 | 0 | CH=CHPh | H | H | $CH_3$ | $OCH_3$ | $OCH_3$ | N | |
| L-3 | 0 | CH=CHPh | H | H | $CH_3$ | Cl | $OCH_3$ | CH | |
| L-3 | 0 | $C(CH_3)=CH-Ph$ | H | H | $CH_3$ | $CH_3$ | $CH_3$ | CH | |
| L-3 | 0 | $C(CH_3)=CH-Ph$ | H | H | $CH_3$ | $CH_3$ | $OCH_3$ | CH | |
| L-3 | 0 | $C(CH_3)=CH-Ph$ | H | H | $CH_3$ | $OCH_3$ | $OCH_3$ | CH | |
| L-3 | 0 | $C(CH_3)=CH-Ph$ | H | H | $CH_3$ | $CH_3$ | $CH_3$ | N | |
| L-3 | 0 | $C(CH_3)=CH-Ph$ | H | H | $CH_3$ | $CH_3$ | $OCH_3$ | N | |
| L-3 | 0 | $C(CH_3)=CH-Ph$ | H | H | $CH_3$ | $OCH_3$ | $OCH_3$ | N | |
| L-3 | 0 | $C(CH_3)=CH-Ph$ | H | H | $CH_3$ | Cl | $OCH_3$ | CH | |
| L-3 | 0 | $\overline{C=CH-(CH_2)_3}$ | H | H | $CH_3$ | $CH_3$ | $CH_3$ | CH | |
| L-3 | 0 | $\overline{C=CH-(CH_2)_3}$ | H | H | $CH_3$ | $CH_3$ | $OCH_3$ | CH | |

Table VIII (Continued)

| L | n | W | R | $R_1$ | $R_2$ | X | Y | Z | m.p. (°C) |
|---|---|---|---|---|---|---|---|---|---|
| L-3 | 0 | $\overline{C=CH-(CH_2)_3}$ | H | H | $CH_3$ | $OCH_3$ | $OCH_3$ | CH | |
| L-3 | 0 | $\overline{C=CH-(CH_2)_3}$ | H | H | $CH_3$ | $CH_3$ | $CH_3$ | N | |
| L-3 | 0 | $\overline{C=CH-(CH_2)_3}$ | H | H | $CH_3$ | $CH_3$ | $OCH_3$ | N | |
| L-3 | 0 | $\overline{C=CH-(CH_2)_3}$ | H | H | $CH_3$ | $OCH_3$ | $OCH_3$ | N | |
| L-3 | 0 | $\overline{C=CH-(CH_2)_3}$ | H | H | $CH_3$ | Cl | $OCH_3$ | CH | |
| L-3 | 0 | $\overline{C=CH-(CH_2)_4}$ | H | H | $CH_3$ | $CH_3$ | $CH_3$ | CH | |
| L-3 | 0 | $\overline{C=CH-(CH_2)_4}$ | H | H | $CH_3$ | $CH_3$ | $OCH_3$ | CH | |
| L-3 | 0 | $\overline{C=CH-(CH_2)_4}$ | H | H | $CH_3$ | $OCH_3$ | $OCH_3$ | CH | |
| L-3 | 0 | $\overline{C=CH-(CH_2)_4}$ | H | H | $CH_3$ | $CH_3$ | $CH_3$ | N | |
| L-3 | 0 | $\overline{C=CH-(CH_2)_4}$ | H | H | $CH_3$ | $CH_3$ | $OCH_3$ | N | |
| L-3 | 0 | $\overline{C=CH-(CH_2)_4}$ | H | H | $CH_3$ | $OCH_3$ | $OCH_3$ | N | |
| L-3 | 0 | $\overline{C=CH-(CH_2)_4}$ | H | H | $CH_3$ | Cl | $OCH_3$ | CH | |
| L-3 | 0 | $\overline{C=CH-(CH_2)_5}$ | H | H | $CH_3$ | $CH_3$ | $CH_3$ | CH | |
| L-3 | 0 | $\overline{C=CH-(CH_2)_5}$ | H | H | $CH_3$ | $CH_3$ | $OCH_3$ | CH | |
| L-3 | 0 | $\overline{C=CH-(CH_2)_5}$ | H | H | $CH_3$ | $OCH_3$ | $OCH_3$ | CH | |
| L-3 | 0 | $\overline{C=CH-(CH_2)_5}$ | H | H | $CH_3$ | $CH_3$ | $CH_3$ | N | |
| L-3 | 0 | $\overline{C=CH-(CH_2)_5}$ | H | H | $CH_3$ | $CH_3$ | $OCH_3$ | N | |
| L-3 | 0 | $\overline{C=CH-(CH_2)_5}$ | H | H | $CH_3$ | $OCH_3$ | $OCH_3$ | N | |
| L-3 | 0 | $\overline{C=CH-(CH_2)_5}$ | H | H | $CH_3$ | Cl | $OCH_3$ | CH | |
| L-3 | 0 | C(CN)=CHCN | H | H | $CH_3$ | $CH_3$ | $CH_3$ | CH | |
| L-3 | 0 | C(CN)=CHCN | H | H | $CH_3$ | $CH_3$ | $OCH_3$ | CH | |
| L-3 | 0 | C(CN)=CHCN | H | H | $CH_3$ | $OCH_3$ | $OCH_3$ | CH | |
| L-3 | 0 | C(CN)=CHCN | H | H | $CH_3$ | $CH_3$ | $CH_3$ | N | |
| L-3 | 0 | C(CN)=CHCN | H | H | $CH_3$ | $CH_3$ | $OCH_3$ | N | |
| L-3 | 0 | C(CN)=CHCN | H | H | $CH_3$ | $OCH_3$ | $OCH_3$ | N | |
| L-3 | 0 | C(CN)=CHCN | H | H | $CH_3$ | Cl | $OCH_3$ | CH | |

Table VIII (Continued)

| L | n | W | R | $R_1$ | $R_2$ | X | Y | Z | m.p. (°C) |
|---|---|---|---|---|---|---|---|---|---|
| L-3 | 0 | $CH{=}CH{-}SO_2N(CH_3)_2$ | H | H | $CH_3$ | $CH_3$ | $CH_3$ | CH | |
| L-3 | 0 | $CH{=}CH{-}SO_2N(CH_3)_2$ | H | H | $CH_3$ | $CH_3$ | $OCH_3$ | CH | |
| L-3 | 0 | $CH{=}CH{-}SO_2N(CH_3)_2$ | H | H | $CH_3$ | $OCH_3$ | $OCH_3$ | CH | |
| L-3 | 0 | $CH{=}CH{-}SO_2N(CH_3)_2$ | H | H | $CH_3$ | $CH_3$ | $CH_3$ | N | |
| L-3 | 0 | $CH{=}CH{-}SO_2N(CH_3)_2$ | H | H | $CH_3$ | $CH_3$ | $OCH_3$ | N | |
| L-3 | 0 | $CH{=}CH{-}SO_2N(CH_3)_2$ | H | H | $CH_3$ | $OCH_3$ | $OCH_3$ | N | |
| L-3 | 0 | $CH{=}CH{-}SO_2N(CH_3)_2$ | H | H | $CH_3$ | Cl | $OCH_3$ | CH | |
| L-3 | 0 | $\overline{CHCH{-}(CH_2)_4}$ | $CH_3$ | H | $CH_3$ | $OCH_3$ | $OCH_3$ | CH | |
| L-3 | 0 | $\overline{CHCH{-}(CH_2)_4}$ | H | $CH_3$ | $CH_3$ | $OCH_3$ | $OCH_3$ | CH | |
| L-3 | 0 | $C(=CH_2){-}CH_2CH_2CH(NO_2)CH_3$ | H | H | $CH_3$ | $OCH_3$ | $OCH_3$ | CH | |
| L-3 | 0 | $CH{=}CHCH_2OSO_2Ph$ | H | H | $CH_3$ | $OCH_3$ | $OCH_3$ | CH | |
| L-3 | 0 | $CH{=}CHCH_2OSO_2CH_3$ | H | H | $CH_3$ | $OCH_3$ | $OCH_3$ | CH | |
| L-3 | 0 | $CH{=}CHSCH_3$ | H | H | $CH_3$ | $OCH_3$ | $OCH_3$ | CH | |
| L-3 | 0 | $C{\equiv}CH$ | H | H | $CH_3$ | $CH_3$ | $CH_3$ | CH | |
| L-3 | 0 | $C{\equiv}CH_3$ | H | H | $CH_3$ | $CH_3$ | $OCH_3$ | CH | |
| L-3 | 0 | $C{\equiv}CH$ | H | H | $CH_3$ | $OCH_3$ | $OCH_3$ | CH | |
| L-3 | 0 | $C{\equiv}CH$ | H | H | $CH_3$ | $CH_3$ | $CH_3$ | N | |
| L-3 | 0 | $C{\equiv}CH$ | H | H | $CH_3$ | $CH_3$ | $OCH_3$ | N | |
| L-3 | 0 | $C{\equiv}CH$ | H | H | $CH_3$ | $OCH_3$ | $OCH_3$ | N | |
| L-3 | 0 | $C{\equiv}CH$ | H | H | $CH_3$ | Cl | $OCH_3$ | CH | |
| L-3 | 0 | $C{\equiv}C{-}Ph$ | H | H | $CH_3$ | $CH_3$ | $CH_3$ | CH | |
| L-3 | 0 | $C{\equiv}C{-}Ph$ | H | H | $CH_3$ | $CH_3$ | $OCH_3$ | CH | |
| L-3 | 0 | $C{\equiv}C{-}Ph$ | H | H | $CH_3$ | $OCH_3$ | $OCH_3$ | CH | |
| L-3 | 0 | $C{\equiv}C{-}Ph$ | H | H | $CH_3$ | $CH_3$ | $CH_3$ | N | |

Table VIII (Continued)

| L | n | W | R | $R_1$ | $R_2$ | X | Y | Z | m.p. (°C) |
|---|---|---|---|---|---|---|---|---|---|
| L-3 | 0 | C≡C-Ph | H | H | $CH_3$ | $CH_3$ | $OCH_3$ | N | |
| L-3 | 0 | C≡C-Ph | H | H | $CH_3$ | $OCH_3$ | $OCH_3$ | N | |
| L-3 | 0 | C≡C-Ph | H | H | $CH_3$ | Cl | $OCH_3$ | CH | |
| L-3 | 0 | $C≡C-CH_3$ | H | H | $CH_3$ | $CH_3$ | $CH_3$ | CH | |
| L-3 | 0 | $C≡C-CH_3$ | H | H | $CH_3$ | $CH_3$ | $OCH_3$ | CH | |
| L-3 | 0 | $C≡C-CH_3$ | H | H | $CH_3$ | $OCH_3$ | $OCH_3$ | CH | |
| L-3 | 0 | $C≡C-CH_3$ | H | H | $CH_3$ | $CH_3$ | $CH_3$ | N | |
| L-3 | 0 | $C≡C-CH_3$ | H | H | $CH_3$ | $CH_3$ | $OCH_3$ | N | |
| L-3 | 0 | $C≡C-CH_3$ | H | H | $CH_3$ | $OCH_3$ | $OCH_3$ | N | |
| L-3 | 0 | $C≡C-CH_3$ | H | H | $CH_3$ | Cl | $OCH_3$ | CH | |
| L-3 | 0 | $C≡CCH_2OC(O)CH_3$ | H | H | $CH_3$ | $CH_3$ | $CH_3$ | CH | |
| L-3 | 0 | $C≡CCH_2OC(O)CH_3$ | H | H | $CH_3$ | $CH_3$ | $OCH_3$ | CH | |
| L-3 | 0 | $C≡CCH_2OC(O)CH_3$ | H | H | $CH_3$ | $OCH_3$ | $OCH_3$ | CH | |
| L-3 | 0 | $C≡CCH_2OC(O)CH_3$ | H | H | $CH_3$ | $CH_3$ | $CH_3$ | N | |
| L-3 | 0 | $C≡CCH_2OC(O)CH_3$ | H | H | $CH_3$ | $CH_3$ | $OCH_3$ | N | |
| L-3 | 0 | $C≡CCH_2OC(O)CH_3$ | H | H | $CH_3$ | $OCH_3$ | $OCH_3$ | N | |
| L-3 | 0 | $C≡CCH_2OC(O)CH_3$ | H | H | $CH_3$ | Cl | $OCH_3$ | CH | |
| L-3 | 0 | $C≡CCH_2OH$ | H | H | $CH_3$ | $CH_3$ | $CH_3$ | CH | |
| L-3 | 0 | $C≡CCH_2OH$ | H | H | $CH_3$ | $CH_3$ | $OCH_3$ | CH | |
| L-3 | 0 | $C≡CCH_2OH$ | H | H | $CH_3$ | $OCH_3$ | $OCH_3$ | CH | |
| L-3 | 0 | $C≡CCH_2OH$ | H | H | $CH_3$ | $CH_3$ | $CH_3$ | N | |
| L-3 | 0 | $C≡CCH_2OH$ | H | H | $CH_3$ | $CH_3$ | $OCH_3$ | N | |
| L-3 | 0 | $C≡CCH_2OH$ | H | H | $CH_3$ | $OCH_3$ | $OCH_3$ | N | |
| L-3 | 0 | $C≡CCH_2OH$ | H | H | $CH_3$ | Cl | $OCH_3$ | CH | |
| L-3 | 0 | $C≡CCH_2Cl$ | H | H | $CH_3$ | $CH_3$ | $CH_3$ | CH | |
| L-3 | 0 | $C≡CCH_2Cl$ | H | H | $CH_3$ | $CH_3$ | $OCH_3$ | CH | |
| L-3 | 0 | $C≡CCH_2Cl$ | H | H | $CH_3$ | $OCH_3$ | $OCH_3$ | CH | |
| L-3 | 0 | $C≡CCH_2Cl$ | H | H | $CH_3$ | $CH_3$ | $CH_3$ | N | |
| L-3 | 0 | $C≡CCH_2Cl$ | H | H | $CH_3$ | $CH_3$ | $OCH_3$ | N | |
| L-3 | 0 | $C≡CCH_2Cl$ | H | H | $CH_3$ | $OCH_3$ | $OCH_3$ | N | |
| L-3 | 0 | $C≡CCH_2Cl$ | H | H | $CH_3$ | Cl | $OCH_3$ | CH | |

Table VIII (Continued)

| L | n | W | R | $R_1$ | $R_2$ | X | Y | Z | m.p. (°C) |
|---|---|---|---|---|---|---|---|---|---|
| L-3 | 0 | $C{\equiv}C-CH_2Br$ | H | H | $CH_3$ | $CH_3$ | $CH_3$ | CH | |
| L-3 | 0 | $C{\equiv}C-CH_2Br$ | H | H | $CH_3$ | $CH_3$ | $OCH_3$ | CH | |
| L-3 | 0 | $C{\equiv}C-CH_2Br$ | H | H | $CH_3$ | $OCH_3$ | $OCH_3$ | CH | |
| L-3 | 0 | $C{\equiv}C-CH_2Br$ | H | H | $CH_3$ | $CH_3$ | $CH_3$ | N | |
| L-3 | 0 | $C{\equiv}C-CH_2Br$ | H | H | $CH_3$ | $CH_3$ | $OCH_3$ | N | |
| L-3 | 0 | $C{\equiv}C-CH_2Br$ | H | H | $CH_3$ | $OCH_3$ | $OCH_3$ | N | |
| L-3 | 0 | $C{\equiv}C-CH_2Br$ | H | H | $CH_3$ | Cl | $OCH_3$ | CH | |
| L-3 | 0 | $C{\equiv}C-CH_2CN$ | H | H | $CH_3$ | $CH_3$ | $CH_3$ | CH | |
| L-3 | 0 | $C{\equiv}C-CH_2CN$ | H | H | $CH_3$ | $CH_3$ | $OCH_3$ | CH | |
| L-3 | 0 | $C{\equiv}C-CH_2CN$ | H | H | $CH_3$ | $OCH_3$ | $OCH_3$ | CH | |
| L-3 | 0 | $C{\equiv}C-CH_2CN$ | H | H | $CH_3$ | $CH_3$ | $CH_3$ | N | |
| L-3 | 0 | $C{\equiv}C-CH_2CN$ | H | H | $CH_3$ | $CH_3$ | $OCH_3$ | N | |
| L-3 | 0 | $C{\equiv}C-CH_2CN$ | H | H | $CH_3$ | $OCH_3$ | $OCH_3$ | N | |
| L-3 | 0 | $C{\equiv}C-CH_2CN$ | H | H | $CH_3$ | Cl | $OCH_3$ | CH | |
| L-3 | 0 | $C{\equiv}C-CH_2OCH_3$ | H | H | $CH_3$ | $CH_3$ | $CH_3$ | CH | |
| L-3 | 0 | $C{\equiv}C-CH_2OCH_3$ | H | H | $CH_3$ | $CH_3$ | $OCH_3$ | CH | |
| L-3 | 0 | $C{\equiv}C-CH_2OCH_3$ | H | H | $CH_3$ | $OCH_3$ | $OCH_3$ | CH | |
| L-3 | 0 | $C{\equiv}C-CH_2OCH_3$ | H | H | $CH_3$ | $CH_3$ | $CH_3$ | N | |
| L-3 | 0 | $C{\equiv}C-CH_2OCH_3$ | H | H | $CH_3$ | $CH_3$ | $OCH_3$ | N | |
| L-3 | 0 | $C{\equiv}C-CH_2OCH_3$ | H | H | $CH_3$ | $OCH_3$ | $OCH_3$ | N | |
| L-3 | 0 | $C{\equiv}C-CH_2OCH_3$ | H | H | $CH_3$ | Cl | $OCH_3$ | CH | |
| L-3 | 0 | $C{\equiv}CCH_2OSO_2CH_3$ | H | H | $CH_3$ | $CH_3$ | $CH_3$ | CH | |
| L-3 | 0 | $C{\equiv}CCH_2OSO_2CH_3$ | H | H | $CH_3$ | $CH_3$ | $OCH_3$ | CH | |
| L-3 | 0 | $C{\equiv}CCH_2OSO_2CH_3$ | H | H | $CH_3$ | $OCH_3$ | $OCH_3$ | CH | |
| L-3 | 0 | $C{\equiv}CCH_2OSO_2CH_3$ | H | H | $CH_3$ | $CH_3$ | $CH_3$ | N | |
| L-3 | 0 | $C{\equiv}CCH_2OSO_2CH_3$ | H | H | $CH_3$ | $CH_3$ | $OCH_3$ | N | |
| L-3 | 0 | $C{\equiv}CCH_2OSO_2CH_3$ | H | H | $CH_3$ | $OCH_3$ | $OCH_3$ | N | |
| L-3 | 0 | $C{\equiv}CCH_2OSO_2CH_3$ | H | H | $CH_3$ | Cl | $OCH_3$ | CH | |
| L-3 | 0 | $C{\equiv}CCH_2SCH_3$ | H | H | $CH_3$ | $CH_3$ | $CH_3$ | CH | |
| L-3 | 0 | $C{\equiv}CCH_2SCH_3$ | H | H | $CH_3$ | $CH_3$ | $OCH_3$ | CH | |
| L-3 | 0 | $C{\equiv}CCH_2SCH_3$ | H | H | $CH_3$ | $OCH_3$ | $OCH_3$ | CH | |
| L-3 | 0 | $C{\equiv}CCH_2SCH_3$ | H | H | $CH_3$ | $CH_3$ | $CH_3$ | N | |

Table VIII (Continued)

| L | n | W | R | $R_1$ | $R_2$ | X | Y | Z | m.p. (°C) |
|---|---|---|---|---|---|---|---|---|---|
| L-3 | 0 | $C{\equiv}CCH_2SCH_3$ | H | H | $CH_3$ | $CH_3$ | $OCH_3$ | N | |
| L-3 | 0 | $C{\equiv}CCH_2SCH_3$ | H | H | $CH_3$ | $OCH_3$ | $OCH_3$ | N | |
| L-3 | 0 | $C{\equiv}CCH_2SCH_3$ | H | H | $CH_3$ | Cl | $OCH_3$ | CH | |
| L-3 | 1 | $HC{=}CH_2$ | H | H | $CH_3$ | $CH_3$ | $CH_3$ | CH | |
| L-3 | 1 | $HC{=}CH_2$ | H | H | $CH_3$ | $CH_3$ | $OCH_3$ | CH | |
| L-3 | 1 | $HC{=}CH_2$ | H | H | $CH_3$ | $OCH_3$ | $OCH_3$ | CH | |
| L-3 | 1 | $HC{=}CH_2$ | H | H | $CH_3$ | $CH_3$ | $CH_3$ | N | |
| L-3 | 1 | $HC{=}CH_2$ | H | H | $CH_3$ | $CH_3$ | $OCH_3$ | N | |
| L-3 | 1 | $HC{=}CH_2$ | H | H | $CH_3$ | $OCH_3$ | $OCH_3$ | N | |
| L-3 | 1 | $HC{=}CH_2$ | H | H | $CH_3$ | Cl | $OCH_3$ | CH | |
| L-3 | 1 | $BrC{=}CH_2$ | H | H | $CH_3$ | $CH_3$ | $CH_3$ | CH | |
| L-3 | 1 | $BrC{=}CH_2$ | H | H | $CH_3$ | $CH_3$ | $OCH_3$ | CH | |
| L-3 | 1 | $BrC{=}CH_2$ | H | H | $CH_3$ | $OCH_3$ | $OCH_3$ | CH | |
| L-3 | 1 | $BrC{=}CH_2$ | H | H | $CH_3$ | $CH_3$ | $CH_3$ | N | |
| L-3 | 1 | $BrC{=}CH_2$ | H | H | $CH_3$ | $CH_3$ | $OCH_3$ | N | |
| L-3 | 1 | $BrC{=}CH_2$ | H | H | $CH_3$ | $OCH_3$ | $OCH_3$ | N | |
| L-3 | 1 | $BrC{=}CH_2$ | H | H | $CH_3$ | Cl | $OCH_3$ | CH | |
| L-3 | 1 | $HC{=}C(CH_3)_2$ | H | H | $CH_3$ | $CH_3$ | $CH_3$ | CH | |
| L-3 | 1 | $HC{=}C(CH_3)_2$ | H | H | $CH_3$ | $CH_3$ | $OCH_3$ | CH | |
| L-3 | 1 | $HC{=}C(CH_3)_2$ | H | H | $CH_3$ | $OCH_3$ | $OCH_3$ | CH | |
| L-3 | 1 | $HC{=}C(CH_3)_2$ | H | H | $CH_3$ | $CH_3$ | $CH_3$ | N | |
| L-3 | 1 | $HC{=}C(CH_3)_2$ | H | H | $CH_3$ | $CH_3$ | $OCH_3$ | N | |
| L-3 | 1 | $HC{=}C(CH_3)_2$ | H | H | $CH_3$ | $OCH_3$ | $OCH_3$ | N | |
| L-3 | 1 | $HC{=}C(CH_3)_2$ | H | H | $CH_3$ | Cl | $OCH_3$ | CH | |
| L-3 | 1 | $HC{=}C(CH_3)CH_2CH_2CH{=}C(CH_3)_2$ | H | H | $CH_3$ | $CH_3$ | $CH_3$ | CH | |
| L-3 | 1 | $HC{=}C(CH_3)CH_2CH_2CH{=}C(CH_3)_2$ | H | H | $CH_3$ | $CH_3$ | $OCH_3$ | CH | |
| L-3 | 1 | $HC{=}C(CH_3)CH_2CH_2CH{=}C(CH_3)_2$ | H | H | $CH_3$ | $OCH_3$ | $OCH_3$ | CH | |
| L-3 | 1 | $HC{=}C(CH_3)CH_2CH_2CH{=}C(CH_3)_2$ | H | H | $CH_3$ | $CH_3$ | $CH_3$ | N | |
| L-3 | 1 | $HC{=}C(CH_3)CH_2CH_2CH{=}C(CH_3)_2$ | H | H | $CH_3$ | $CH_3$ | $OCH_3$ | N | |
| L-3 | 1 | $HC{=}C(CH_3)CH_2CH_2CH{=}C(CH_3)_2$ | H | H | $CH_3$ | $OCH_3$ | $OCH_3$ | N | |
| L-3 | 1 | $HC{=}C(CH_3)CH_2CH_2CH{=}C(CH_3)_2$ | H | H | $CH_3$ | Cl | $OCH_3$ | CH | |

Table VIII (Continued)

| L | n | W | R | $R_1$ | $R_2$ | X | Y | Z | m.p. (°C) |
|---|---|---|---|---|---|---|---|---|---|
| L-3 | 1 | HC=CH-Ph | H | H | $CH_3$ | $CH_3$ | $CH_3$ | CH | |
| L-3 | 1 | HC=CH-Ph | H | H | $CH_3$ | $CH_3$ | $OCH_3$ | CH | |
| L-3 | 1 | HC=CH-Ph | H | H | $CH_3$ | $OCH_3$ | $OCH_3$ | CH | |
| L-3 | 1 | HC=CH-Ph | H | H | $CH_3$ | $CH_3$ | $CH_3$ | N | |
| L-3 | 1 | HC=CH-Ph | H | H | $CH_3$ | $CH_3$ | $OCH_3$ | N | |
| L-3 | 1 | HC=CH-Ph | H | H | $CH_3$ | $OCH_3$ | $OCH_3$ | N | |
| L-3 | 1 | HC=CH-Ph | H | H | $CH_3$ | Cl | $OCH_3$ | CH | |
| L-3 | 1 | $HC=CHCH_3$ | H | H | $CH_3$ | $CH_3$ | $CH_3$ | CH | |
| L-3 | 1 | $HC=CHCH_3$ | H | H | $CH_3$ | $CH_3$ | $OCH_3$ | CH | |
| L-3 | 1 | $HC=CHCH_3$ | H | H | $CH_3$ | $OCH_3$ | $OCH_3$ | CH | |
| L-3 | 1 | $HC=CHCH_3$ | H | H | $CH_3$ | $CH_3$ | $CH_3$ | N | |
| L-3 | 1 | $HC=CHCH_3$ | H | H | $CH_3$ | $CH_3$ | $OCH_3$ | N | |
| L-3 | 1 | $HC=CHCH_3$ | H | H | $CH_3$ | $OCH_3$ | $OCH_3$ | N | |
| L-3 | 1 | $HC=CHCH_3$ | H | H | $CH_3$ | Cl | $OCH_3$ | CH | |
| L-3 | 1 | $HC=CHCH_2Br$ | H | H | $CH_3$ | $CH_3$ | $CH_3$ | CH | |
| L-3 | 1 | $HC=CHCH_2Br$ | H | H | $CH_3$ | $CH_3$ | $OCH_3$ | CH | |
| L-3 | 1 | $HC=CHCH_2Br$ | H | H | $CH_3$ | $OCH_3$ | $OCH_3$ | CH | |
| L-3 | 1 | $HC=CHCH_2Br$ | H | H | $CH_3$ | $CH_3$ | $CH_3$ | N | |
| L-3 | 1 | $HC=CHCH_2Br$ | H | H | $CH_3$ | $CH_3$ | $OCH_3$ | N | |
| L-3 | 1 | $HC=CHCH_2Br$ | H | H | $CH_3$ | $OCH_3$ | $OCH_3$ | N | |
| L-3 | 1 | $HC=CHCH_2Br$ | H | H | $CH_3$ | Cl | $OCH_3$ | CH | |
| L-3 | 1 | C≡CH | H | H | $CH_3$ | $CH_3$ | $CH_3$ | CH | |
| L-3 | 1 | C≡CH | H | H | $CH_3$ | $CH_3$ | $OCH_3$ | CH | |
| L-3 | 1 | C≡CH | H | H | $CH_3$ | $OCH_3$ | $OCH_3$ | CH | |
| L-3 | 1 | C≡CH | H | H | $CH_3$ | $CH_3$ | $CH_3$ | N | |
| L-3 | 1 | C≡CH | H | H | $CH_3$ | $CH_3$ | $OCH_3$ | N | |
| L-3 | 1 | C≡CH | H | H | $CH_3$ | $OCH_3$ | $OCH_3$ | N | |
| L-3 | 1 | C≡CH | H | H | $CH_3$ | Cl | $OCH_3$ | CH | |
| L-3 | 1 | $C≡CCH_3$ | H | H | $CH_3$ | $CH_3$ | $CH_3$ | CH | |
| L-3 | 1 | $C≡CCH_3$ | H | H | $CH_3$ | $CH_3$ | $OCH_3$ | CH | |
| L-3 | 1 | $C≡CCH_3$ | H | H | $CH_3$ | $OCH_3$ | $OCH_3$ | CH | |
| L-3 | 1 | $C≡CCH_3$ | H | H | $CH_3$ | $CH_3$ | $CH_3$ | N | |

Table VIII (Continued)

| L | n | W | R | $R_1$ | $R_2$ | X | Y | Z | m.p. (°C) |
|---|---|---|---|---|---|---|---|---|---|
| L-3 | 1 | $C\equiv CCH_3$ | H | H | $CH_3$ | $CH_3$ | $OCH_3$ | N | |
| L-3 | 1 | $C\equiv CCH_3$ | H | H | $CH_3$ | $OCH_3$ | $OCH_3$ | N | |
| L-3 | 1 | $C\equiv CCH_3$ | H | H | $CH_3$ | Cl | $OCH_3$ | CH | |
| L-3 | 1 | $C\equiv C-CH_2Br$ | H | H | $CH_3$ | $CH_3$ | $CH_3$ | CH | |
| L-3 | 1 | $C\equiv C-CH_2Br$ | H | H | $CH_3$ | $CH_3$ | $OCH_3$ | CH | |
| L-3 | 1 | $C\equiv C-CH_2Br$ | H | H | $CH_3$ | $OCH_3$ | $OCH_3$ | CH | |
| L-3 | 1 | $C\equiv C-CH_2Br$ | H | H | $CH_3$ | $CH_3$ | $CH_3$ | N | |
| L-3 | 1 | $C\equiv C-CH_2Br$ | H | H | $CH_3$ | $CH_3$ | $OCH_3$ | N | |
| L-3 | 1 | $C\equiv C-CH_2Br$ | H | H | $CH_3$ | $OCH_3$ | $OCH_3$ | N | |
| L-3 | 1 | $C\equiv C-CH_2Br$ | H | H | $CH_3$ | Cl | $OCH_3$ | CH | |
| L-3 | 1 | $C\equiv C-Ph$ | H | H | $CH_3$ | $CH_3$ | $CH_3$ | CH | |
| L-3 | 1 | $C\equiv C-Ph$ | H | H | $CH_3$ | $CH_3$ | $OCH_3$ | CH | |
| L-3 | 1 | $C\equiv C-Ph$ | H | H | $CH_3$ | $OCH_3$ | $OCH_3$ | CH | |
| L-3 | 1 | $C\equiv C-Ph$ | H | H | $CH_3$ | $CH_3$ | $CH_3$ | N | |
| L-3 | 1 | $C\equiv C-Ph$ | H | H | $CH_3$ | $CH_3$ | $OCH_3$ | N | |
| L-3 | 1 | $C\equiv C-Ph$ | H | H | $CH_3$ | $OCH_3$ | $OCH_3$ | N | |
| L-3 | 1 | $C\equiv C-Ph$ | H | H | $CH_3$ | Cl | $OCH_3$ | CH | |
| L-3 | 1 | $CH=CH_2$ | $CH_3$ | H | $CH_3$ | $CH_3$ | $CH_3$ | CH | |
| L-3 | 1 | $CH=CH_2$ | $CH_3$ | H | $CH_3$ | $CH_3$ | $OCH_3$ | CH | |
| L-3 | 1 | $CH=CH_2$ | $CH_3$ | H | $CH_3$ | $OCH_3$ | $OCH_3$ | CH | |
| L-3 | 1 | $CH=CH_2$ | $CH_3$ | H | $CH_3$ | $CH_3$ | $CH_3$ | N | |
| L-3 | 1 | $CH=CH_2$ | $CH_3$ | H | $CH_3$ | $CH_3$ | $OCH_3$ | N | |
| L-3 | 1 | $CH=CH_2$ | $CH_3$ | H | $CH_3$ | $OCH_3$ | $OCH_3$ | N | |
| L-3 | 1 | $CH=CH_2$ | $CH_3$ | H | $CH_3$ | Cl | $OCH_3$ | CH | |
| L-3 | 1 | $C\equiv CH$ | $CH_3$ | H | $CH_3$ | $CH_3$ | $CH_3$ | CH | |
| L-3 | 1 | $C\equiv CH$ | $CH_3$ | H | $CH_3$ | $CH_3$ | $OCH_3$ | CH | |
| L-3 | 1 | $C\equiv CH$ | $CH_3$ | H | $CH_3$ | $OCH_3$ | $OCH_3$ | CH | |
| L-3 | 1 | $C\equiv CH$ | $CH_3$ | H | $CH_3$ | $CH_3$ | $CH_3$ | N | |
| L-3 | 1 | $C\equiv CH$ | $CH_3$ | H | $CH_3$ | $CH_3$ | $OCH_3$ | N | |
| L-3 | 1 | $C\equiv CH$ | $CH_3$ | H | $CH_3$ | $OCH_3$ | $OCH_3$ | N | |
| L-3 | 1 | $C\equiv CH$ | $CH_3$ | H | $CH_3$ | Cl | $OCH_3$ | CH | |

Table VIII (Continued)

| L | n | W | R | $R_1$ | $R_2$ | X | Y | Z | m.p. (°C) |
|---|---|---|---|---|---|---|---|---|---|
| L-3 | 1 | $CH=CH_2$ | H | $CH_3$ | $CH_3$ | $CH_3$ | $CH_3$ | CH | |
| L-3 | 1 | $CH=CH_2$ | H | $CH_3$ | $CH_3$ | $CH_3$ | $OCH_3$ | CH | |
| L-3 | 1 | $CH=CH_2$ | H | $CH_3$ | $CH_3$ | $OCH_3$ | $OCH_3$ | CH | |
| L-3 | 1 | $CH=CH_2$ | H | $CH_3$ | $CH_3$ | $CH_3$ | $CH_3$ | N | |
| L-3 | 1 | $CH=CH_2$ | H | $CH_3$ | $CH_3$ | $CH_3$ | $OCH_3$ | N | |
| L-3 | 1 | $CH=CH_2$ | H | $CH_3$ | $CH_3$ | $OCH_3$ | $OCH_3$ | N | |
| L-3 | 1 | $CH=CH_2$ | H | $CH_3$ | $CH_3$ | Cl | $OCH_3$ | CH | |
| L-3 | 1 | C≡CH | H | $CH_3$ | $CH_3$ | $OCH_3$ | $OCH_3$ | CH | |
| L-3 | 1 | C≡CH | H | $CH_3$ | $CH_3$ | $OCH_3$ | $OCH_3$ | N | |
| L-3 | 1 | C≡CH | H | $CH_3$ | $CH_3$ | $OCH_3$ | $CH_3$ | CH | |
| L-3 | 0 | $CH=CH-CH_3$ | H | H | $CH_3$ | $OCH_3$ | $OCH_3$ | N | 159-162 |
| L-3 | 0 | $CH=CH-CH_3$ | H | H | $CH_3$ | $CH_3$ | $OCH_3$ | CH | 155-158 |
| L-3 | 0 | $CH=CH-CH_3$ | H | H | $CH_3$ | $OCH_3$ | $OCH_3$ | CH | 154-156 |
| L-4 | 0 | $CH=CH_2$ | H | H | - | $CH_3$ | $CH_3$ | CH | |
| L-4 | 0 | $CH=CH_2$ | H | H | - | $CH_3$ | $OCH_3$ | CH | |
| L-4 | 0 | $CH=CH_2$ | H | H | - | $OCH_3$ | $OCH_3$ | CH | |
| L-4 | 0 | $CH=CH_2$ | H | H | - | $CH_3$ | $CH_3$ | N | |
| L-4 | 0 | $CH=CH_2$ | H | H | - | $CH_3$ | $OCH_3$ | N | |
| L-4 | 0 | $CH=CH_2$ | H | H | - | $OCH_3$ | $OCH_3$ | N | |
| L-4 | 0 | $CH=CH_2$ | H | H | - | Cl | $OCH_3$ | CH | |
| L-4 | 0 | $C(CH_3)=CHCH_3$ | H | H | - | $CH_3$ | $CH_3$ | CH | |
| L-4 | 0 | $C(CH_3)=CHCH_3$ | H | H | - | $CH_3$ | $OCH_3$ | CH | |
| L-4 | 0 | $C(CH_3)=CHCH_3$ | H | H | - | $OCH_3$ | $OCH_3$ | CH | |
| L-4 | 0 | $C(CH_3)=CHCH_3$ | H | H | - | $CH_3$ | $CH_3$ | N | |
| L-4 | 0 | $C(CH_3)=CHCH_3$ | H | H | - | $CH_3$ | $OCH_3$ | N | |
| L-4 | 0 | $C(CH_3)=CHCH_3$ | H | H | - | $OCH_3$ | $OCH_3$ | N | |
| L-4 | 0 | $C(CH_3)=CHCH_3$ | H | H | - | Cl | $OCH_3$ | CH | |
| L-4 | 0 | $CH=CHCH_2CH_3$ | H | H | - | $CH_3$ | $CH_3$ | CH | |
| L-4 | 0 | $CH=CHCH_2CH_3$ | H | H | - | $CH_3$ | $OCH_3$ | CH | |
| L-4 | 0 | $CH=CHCH_2CH_3$ | H | H | - | $OCH_3$ | $OCH_3$ | CH | |
| L-4 | 0 | $CH=CHCH_2CH_3$ | H | H | - | $CH_3$ | $CH_3$ | N | |
| L-4 | 0 | $CH=CHCH_2CH_3$ | H | H | - | $CH_3$ | $OCH_3$ | N | |

Table VIII (Continued)

| L | n | W | R | $R_1$ | $R_2$ | X | Y | Z | m.p. (°C) |
|---|---|---|---|---|---|---|---|---|---|
| L-4 | 0 | $CH=CHCH_2CH_3$ | H | H | - | $OCH_3$ | $OCH_3$ | N | |
| L-4 | 0 | $CH=CHCH_2CH_3$ | H | H | - | Cl | $OCH_3$ | CH | |
| L-4 | 0 | $C(CH_3)=CHCH_2CH_3$ | H | H | - | $CH_3$ | $CH_3$ | CH | |
| L-4 | 0 | $C(CH_3)=CHCH_2CH_3$ | H | H | - | $CH_3$ | $OCH_3$ | CH | |
| L-4 | 0 | $C(CH_3)=CHCH_2CH_3$ | H | H | - | $OCH_3$ | $OCH_3$ | CH | |
| L-4 | 0 | $C(CH_3)=CHCH_2CH_3$ | H | H | - | $CH_3$ | $CH_3$ | N | |
| L-4 | 0 | $C(CH_3)=CHCH_2CH_3$ | H | H | - | $CH_3$ | $OCH_3$ | N | |
| L-4 | 0 | $C(CH_3)=CHCH_2CH_3$ | H | H | - | $OCH_3$ | $OCH_3$ | N | |
| L-4 | 0 | $C(CH_3)=CHCH_2CH_3$ | H | H | - | Cl | $OCH_3$ | CH | |
| L-4 | 0 | $CH=CHCH_2CH_2CH_3$ | H | H | - | $CH_3$ | $CH_3$ | CH | |
| L-4 | 0 | $CH=CHCH_2CH_2CH_3$ | H | H | - | $CH_3$ | $OCH_3$ | CH | |
| L-4 | 0 | $CH=CHCH_2CH_2CH_3$ | H | H | - | $OCH_3$ | $OCH_3$ | CH | |
| L-4 | 0 | $CH=CHCH_2CH_2CH_3$ | H | H | - | $CH_3$ | $CH_3$ | N | |
| L-4 | 0 | $CH=CHCH_2CH_2CH_3$ | H | H | - | $CH_3$ | $OCH_3$ | N | |
| L-4 | 0 | $CH=CHCH_2CH_2CH_3$ | H | H | - | $OCH_3$ | $OCH_3$ | N | |
| L-4 | 0 | $CH=CHCH_2CH_2CH_3$ | H | H | - | Cl | $OCH_3$ | CH | |
| L-4 | 0 | CH=CH-CN | H | H | - | $CH_3$ | $CH_3$ | CH | |
| L-4 | 0 | CH=CH-CN | H | H | - | $CH_3$ | $OCH_3$ | CH | |
| L-4 | 0 | CH=CH-CN | H | H | - | $OCH_3$ | $OCH_3$ | CH | |
| L-4 | 0 | CH=CH-CN | H | H | - | $CH_3$ | $CH_3$ | N | |
| L-4 | 0 | CH=CH-CN | H | H | - | $CH_3$ | $OCH_3$ | N | |
| L-4 | 0 | CH=CH-CN | H | H | - | $OCH_3$ | $OCH_3$ | N | |
| L-4 | 0 | CH=CH-CN | H | H | - | Cl | $OCH_3$ | CH | |
| L-4 | 0 | $CH=CH-OCH_3$ | H | H | - | $CH_3$ | $CH_3$ | CH | |
| L-4 | 0 | $CH=CH-OCH_3$ | H | H | - | $CH_3$ | $OCH_3$ | CH | |
| L-4 | 0 | $CH=CH-OCH_3$ | H | H | - | $OCH_3$ | $OCH_3$ | CH | |
| L-4 | 0 | $CH=CH-OCH_3$ | H | H | - | $CH_3$ | $CH_3$ | N | |
| L-4 | 0 | $CH=CH-OCH_3$ | H | H | - | $CH_3$ | $OCH_3$ | N | |
| L-4 | 0 | $CH=CH-OCH_3$ | H | H | - | $OCH_3$ | $OCH_3$ | N | |
| L-4 | 0 | $CH=CH-OCH_3$ | H | H | - | Cl | $OCH_3$ | CH | |
| L-4 | 0 | CH=CH-Br | H | H | - | $CH_3$ | $CH_3$ | CH | |
| L-4 | 0 | CH=CH-Br | H | H | - | $CH_3$ | $OCH_3$ | CH | |

Table VIII (Continued)

| L | n | W | R | $R_1$ | $R_2$ | X | Y | Z | m.p. (°C) |
|---|---|---|---|---|---|---|---|---|---|
| L-4 | 0 | CH=CH-Br | H | H | - | $OCH_3$ | $OCH_3$ | CH | |
| L-4 | 0 | CH=CH-Br | H | H | - | $CH_3$ | $CH_3$ | N | |
| L-4 | 0 | CH=CH-Br | H | H | - | $CH_3$ | $OCH_3$ | N | |
| L-4 | 0 | CH=CH-Br | H | H | - | $OCH_3$ | $OCH_3$ | N | |
| L-4 | 0 | CH=CH-Br | H | H | - | Cl | $OCH_3$ | CH | |
| L-4 | 0 | $CH=CH-CF_3$ | H | H | - | $CH_3$ | $CH_3$ | CH | |
| L-4 | 0 | $CH=CH-CF_3$ | H | H | - | $CH_3$ | $OCH_3$ | CH | |
| L-4 | 0 | $CH=CH-CF_3$ | H | H | - | $OCH_3$ | $OCH_3$ | CH | |
| L-4 | 0 | $CH=CH-CF_3$ | H | H | - | $CH_3$ | $CH_3$ | N | |
| L-4 | 0 | $CH=CH-CF_3$ | H | H | - | $CH_3$ | $OCH_3$ | N | |
| L-4 | 0 | $CH=CH-CF_3$ | H | H | - | $OCH_3$ | $OCH_3$ | N | |
| L-4 | 0 | $CH=CH-CF_3$ | H | H | - | Cl | $OCH_3$ | CH | |
| L-4 | 0 | $C(CH_3)=CH-OCH_3$ | H | H | - | $CH_3$ | $CH_3$ | CH | |
| L-4 | 0 | $C(CH_3)=CH-OCH_3$ | H | H | - | $CH_3$ | $OCH_3$ | CH | |
| L-4 | 0 | $C(CH_3)=CH-OCH_3$ | H | H | - | $OCH_3$ | $OCH_3$ | CH | |
| L-4 | 0 | $C(CH_3)=CH-OCH_3$ | H | H | - | $CH_3$ | $CH_3$ | N | |
| L-4 | 0 | $C(CH_3)=CH-OCH_3$ | H | H | - | $CH_3$ | $OCH_3$ | N | |
| L-4 | 0 | $C(CH_3)=CH-OCH_3$ | H | H | - | $OCH_3$ | $OCH_3$ | N | |
| L-4 | 0 | $C(CH_3)=CH-OCH_3$ | H | H | - | Cl | $OCH_3$ | CH | |
| L-4 | 0 | CH=CHPh | H | H | - | $CH_3$ | $CH_3$ | CH | |
| L-4 | 0 | CH=CHPh | H | H | - | $CH_3$ | $OCH_3$ | CH | |
| L-4 | 0 | CH=CHPh | H | H | - | $OCH_3$ | $OCH_3$ | CH | |
| L-4 | 0 | CH=CHPh | H | H | - | $CH_3$ | $CH_3$ | N | |
| L-4 | 0 | CH=CHPh | H | H | - | $CH_3$ | $OCH_3$ | N | |
| L-4 | 0 | CH=CHPh | H | H | - | $OCH_3$ | $OCH_3$ | N | |
| L-4 | 0 | CH=CHPh | H | H | - | Cl | $OCH_3$ | CH | |
| L-4 | 0 | $C(CH_3)=CH-Ph$ | H | H | - | $CH_3$ | $CH_3$ | CH | |
| L-4 | 0 | $C(CH_3)=CH-Ph$ | H | H | - | $CH_3$ | $OCH_3$ | CH | |
| L-4 | 0 | $C(CH_3)=CH-Ph$ | H | H | - | $OCH_3$ | $OCH_3$ | CH | |
| L-4 | 0 | $C(CH_3)=CH-Ph$ | H | H | - | $CH_3$ | $CH_3$ | N | |
| L-4 | 0 | $C(CH_3)=CH-Ph$ | H | H | - | $CH_3$ | $OCH_3$ | N | |
| L-4 | 0 | $C(CH_3)=CH-Ph$ | H | H | - | $OCH_3$ | $OCH_3$ | N | |
| L-4 | 0 | $C(CH_3)=CH-Ph$ | H | H | - | Cl | $OCH_3$ | CH | |

Table VIII (Continued)

| L | n | W | R | $R_1$ | $R_2$ | X | Y | Z | m.p. (°C) |
|---|---|---|---|---|---|---|---|---|---|
| L-4 | 0 | $C{=}CH{-}(CH_2)_3$ | H | H | - | $CH_3$ | $CH_3$ | CH | |
| L-4 | 0 | $C{=}CH{-}(CH_2)_3$ | H | H | - | $CH_3$ | $OCH_3$ | CH | |
| L-4 | 0 | $C{=}CH{-}(CH_2)_3$ | H | H | - | $OCH_3$ | $OCH_3$ | CH | |
| L-4 | 0 | $C{=}CH{-}(CH_2)_3$ | H | H | - | $CH_3$ | $CH_3$ | N | |
| L-4 | 0 | $C{=}CH{-}(CH_2)_3$ | H | H | - | $CH_3$ | $OCH_3$ | N | |
| L-4 | 0 | $C{=}CH{-}(CH_2)_3$ | H | H | - | $OCH_3$ | $OCH_3$ | N | |
| L-4 | 0 | $C{=}CH{-}(CH_2)_3$ | H | H | - | Cl | $OCH_3$ | CH | |
| L-4 | 0 | $C{=}CH{-}(CH_2)_4$ | H | H | - | $CH_3$ | $CH_3$ | CH | |
| L-4 | 0 | $C{=}CH{-}(CH_2)_4$ | H | H | - | $CH_3$ | $OCH_3$ | CH | |
| L-4 | 0 | $C{=}CH{-}(CH_2)_4$ | H | H | - | $OCH_3$ | $OCH_3$ | CH | |
| L-4 | 0 | $C{=}CH{-}(CH_2)_4$ | H | H | - | $CH_3$ | $CH_3$ | N | |
| L-4 | 0 | $C{=}CH{-}(CH_2)_4$ | H | H | - | $CH_3$ | $OCH_3$ | N | |
| L-4 | 0 | $C{=}CH{-}(CH_2)_4$ | H | H | - | $OCH_3$ | $OCH_3$ | N | |
| L-4 | 0 | $C{=}CH{-}(CH_2)_4$ | H | H | - | Cl | $OCH_3$ | CH | |
| L-4 | 0 | $C{=}CH{-}(CH_2)_5$ | H | H | - | $CH_3$ | $CH_3$ | CH | |
| L-4 | 0 | $C{=}CH{-}(CH_2)_5$ | H | H | - | $CH_3$ | $OCH_3$ | CH | |
| L-4 | 0 | $C{=}CH{-}(CH_2)_5$ | H | H | - | $OCH_3$ | $OCH_3$ | CH | |
| L-4 | 0 | $C{=}CH{-}(CH_2)_5$ | H | H | - | $CH_3$ | $CH_3$ | N | |
| L-4 | 0 | $C{=}CH{-}(CH_2)_5$ | H | H | - | $CH_3$ | $OCH_3$ | N | |
| L-4 | 0 | $C{=}CH{-}(CH_2)_5$ | H | H | - | $OCH_3$ | $OCH_3$ | N | |
| L-4 | 0 | $C{=}CH{-}(CH_2)_5$ | H | H | - | Cl | $OCH_3$ | CH | |
| L-4 | 0 | C(CN)=CHCN | H | H | - | $CH_3$ | $CH_3$ | CH | |
| L-4 | 0 | C(CN)=CHCN | H | H | - | $CH_3$ | $OCH_3$ | CH | |
| L-4 | 0 | C(CN)=CHCN | H | H | - | $OCH_3$ | $OCH_3$ | CH | |
| L-4 | 0 | C(CN)=CHCN | H | H | - | $CH_3$ | $CH_3$ | N | |

Table VIII (Continued)

| L | n | W | R | $R_1$ | $R_2$ | X | Y | Z | m.p. (°C) |
|---|---|---|---|---|---|---|---|---|---|
| L-4 | 0 | C(CN)=CHCN | H | H | - | $CH_3$ | $OCH_3$ | N | |
| L-4 | 0 | C(CN)=CHCN | H | H | - | $OCH_3$ | $OCH_3$ | N | |
| L-4 | 0 | C(CN)=CHCN | H | H | - | Cl | $OCH_3$ | CH | |
| L-4 | 0 | $CH=CH-SO_2N(CH_3)_2$ | H | H | - | $CH_3$ | $CH_3$ | CH | |
| L-4 | 0 | $CH=CH-SO_2N(CH_3)_2$ | H | H | - | $CH_3$ | $OCH_3$ | CH | |
| L-4 | 0 | $CH=CH-SO_2N(CH_3)_2$ | H | H | - | $OCH_3$ | $OCH_3$ | CH | |
| L-4 | 0 | $CH=CH-SO_2N(CH_3)_2$ | H | H | - | $CH_3$ | $CH_3$ | N | |
| L-4 | 0 | $CH=CH-SO_2N(CH_3)_2$ | H | H | - | $CH_3$ | $OCH_3$ | N | |
| L-4 | 0 | $CH=CH-SO_2N(CH_3)_2$ | H | H | - | $OCH_3$ | $OCH_3$ | N | |
| L-4 | 0 | $CH=CH-SO_2N(CH_3)_2$ | H | H | - | Cl | $OCH_3$ | CH | |
| L-4 | 0 | $\overline{C=CH-(CH_2)_4}$ | $CH_3$ | H | - | $OCH_3$ | $OCH_3$ | CH | |
| L-4 | 0 | $\overline{C=CH-(CH_2)_4}$ | H | $CH_3$ | - | $OCH_3$ | $OCH_3$ | CH | |
| L-4 | 0 | $C(=CH_2)-CH_2CH_2CH(NO_2)CH_3$ | H | H | - | $OCH_3$ | $OCH_3$ | CH | |
| L-4 | 0 | $CH=CHCH_2OSO_2Ph$ | H | H | - | $OCH_3$ | $OCH_3$ | CH | |
| L-4 | 0 | $CH=CHCH_2OSO_2CH_3$ | H | H | - | $OCH_3$ | $OCH_3$ | CH | |
| L-4 | 0 | $CH=CHSCH_3$ | H | H | - | $OCH_3$ | $OCH_3$ | CH | |
| L-4 | 0 | C≡CH | H | H | - | $CH_3$ | $CH_3$ | CH | |
| L-4 | 0 | C≡CH | H | H | - | $CH_3$ | $OCH_3$ | CH | |
| L-4 | 0 | C≡CH | H | H | - | $OCH_3$ | $OCH_3$ | CH | |
| L-4 | 0 | C≡CH | H | H | - | $CH_3$ | $CH_3$ | N | |
| L-4 | 0 | C≡CH | H | H | - | $CH_3$ | $OCH_3$ | N | |
| L-4 | 0 | C≡CH | H | H | - | $OCH_3$ | $OCH_3$ | N | |
| L-4 | 0 | C≡CH | H | H | - | Cl | $OCH_3$ | CH | |
| L-4 | 0 | C≡C-Ph | H | H | - | $CH_3$ | $CH_3$ | CH | |

Table VIII (Continued)

| L | n | W | R | $R_1$ | $R_2$ | X | Y | Z | m.p. (°C) |
|---|---|---|---|---|---|---|---|---|---|
| L-4 | 0 | C≡C-Ph | H | H | - | $CH_3$ | $OCH_3$ | CH | |
| L-4 | 0 | C≡C-Ph | H | H | - | $OCH_3$ | $OCH_3$ | CH | |
| L-4 | 0 | C≡C-Ph | H | H | - | $CH_3$ | $CH_3$ | N | |
| L-4 | 0 | C≡C-Ph | H | H | - | $CH_3$ | $OCH_3$ | N | |
| L-4 | 0 | C≡C-Ph | H | H | - | $OCH_3$ | $OCH_3$ | N | |
| L-4 | 0 | C≡C-Ph | H | H | - | Cl | $OCH_3$ | CH | |
| L-4 | 0 | C≡C-$CH_3$ | H | H | - | $CH_3$ | $CH_3$ | CH | |
| L-4 | 0 | C≡C-$CH_3$ | H | H | - | $CH_3$ | $OCH_3$ | CH | |
| L-4 | 0 | C≡C-$CH_3$ | H | H | - | $OCH_3$ | $OCH_3$ | CH | |
| L-4 | 0 | C≡C-$CH_3$ | H | H | - | $CH_3$ | $CH_3$ | N | |
| L-4 | 0 | C≡C-$CH_3$ | H | H | - | $CH_3$ | $OCH_3$ | N | |
| L-4 | 0 | C≡C-$CH_3$ | H | H | - | $OCH_3$ | $OCH_3$ | N | |
| L-4 | 0 | C≡C-$CH_3$ | H | H | - | Cl | $OCH_3$ | CH | |
| L-4 | 0 | C≡$CCH_2OC(O)CH_3$ | H | H | - | $CH_3$ | $CH_3$ | CH | |
| L-4 | 0 | C≡$CCH_2OC(O)CH_3$ | H | H | - | $CH_3$ | $OCH_3$ | CH | |
| L-4 | 0 | C≡$CCH_2OC(O)CH_3$ | H | H | - | $OCH_3$ | $OCH_3$ | CH | |
| L-4 | 0 | C≡$CCH_2OC(O)CH_3$ | H | H | - | $CH_3$ | $CH_3$ | N | |
| L-4 | 0 | C≡$CCH_2OC(O)CH_3$ | H | H | - | $CH_3$ | $OCH_3$ | N | |
| L-4 | 0 | C≡$CCH_2OC(O)CH_3$ | H | H | - | $OCH_3$ | $OCH_3$ | N | |
| L-4 | 0 | C≡$CCH_2OC(O)CH_3$ | H | H | - | Cl | $OCH_3$ | CH | |
| L-4 | 0 | C≡$CCH_2OH$ | H | H | - | $CH_3$ | $CH_3$ | CH | |
| L-4 | 0 | C≡$CCH_2OH$ | H | H | - | $CH_3$ | $OCH_3$ | CH | |
| L-4 | 0 | C≡$CCH_2OH$ | H | H | - | $OCH_3$ | $OCH_3$ | CH | |
| L-4 | 0 | C≡$CCH_2OH$ | H | H | - | $CH_3$ | $CH_3$ | N | |
| L-4 | 0 | C≡$CCH_2OH$ | H | H | - | $CH_3$ | $OCH_3$ | N | |
| L-4 | 0 | C≡$CCH_2OH$ | H | H | - | $OCH_3$ | $OCH_3$ | N | |
| L-4 | 0 | C≡$CCH_2OH$ | H | H | - | Cl | $OCH_3$ | CH | |
| L-4 | 0 | C≡$CCH_2Cl$ | H | H | - | $CH_3$ | $CH_3$ | CH | |
| L-4 | 0 | C≡$CCH_2Cl$ | H | H | - | $CH_3$ | $OCH_3$ | CH | |
| L-4 | 0 | C≡$CCH_2Cl$ | H | H | - | $OCH_3$ | $OCH_3$ | CH | |
| L-4 | 0 | C≡$CCH_2Cl$ | H | H | - | $CH_3$ | $CH_3$ | N | |
| L-4 | 0 | C≡$CCH_2Cl$ | H | H | - | $CH_3$ | $OCH_3$ | N | |

Table VIII (Continued)

| L | n | W | R | $R_1$ | $R_2$ | X | Y | Z | m.p. (°C) |
|---|---|---|---|---|---|---|---|---|---|
| L-4 | 0 | $C\equiv CCH_2Cl$ | H | H | - | $OCH_3$ | $OCH_3$ | N | |
| L-4 | 0 | $C\equiv CCH_2Cl$ | H | H | - | Cl | $OCH_3$ | CH | |
| L-4 | 0 | $C\equiv C-CH_2Br$ | H | H | - | $CH_3$ | $CH_3$ | CH | |
| L-4 | 0 | $C\equiv C-CH_2Br$ | H | H | - | $CH_3$ | $OCH_3$ | CH | |
| L-4 | 0 | $C\equiv C-CH_2Br$ | H | H | - | $OCH_3$ | $OCH_3$ | CH | |
| L-4 | 0 | $C\equiv C-CH_2Br$ | H | H | - | $CH_3$ | $CH_3$ | N | |
| L-4 | 0 | $C\equiv C-CH_2Br$ | H | H | - | $CH_3$ | $OCH_3$ | N | |
| L-4 | 0 | $C\equiv C-CH_2Br$ | H | H | - | $OCH_3$ | $OCH_3$ | N | |
| L-4 | 0 | $C\equiv C-CH_2Br$ | H | H | - | Cl | $OCH_3$ | CH | |
| L-4 | 0 | $C\equiv C-CH_2CN$ | H | H | - | $CH_3$ | $CH_3$ | CH | |
| L-4 | 0 | $C\equiv C-CH_2CN$ | H | H | - | $CH_3$ | $OCH_3$ | CH | |
| L-4 | 0 | $C\equiv C-CH_2CN$ | H | H | - | $OCH_3$ | $OCH_3$ | CH | |
| L-4 | 0 | $C\equiv C-CH_2CN$ | H | H | - | $CH_3$ | $CH_3$ | N | |
| L-4 | 0 | $C\equiv C-CH_2CN$ | H | H | - | $CH_3$ | $OCH_3$ | N | |
| L-4 | 0 | $C\equiv C-CH_2CN$ | H | H | - | $OCH_3$ | $OCH_3$ | N | |
| L-4 | 0 | $C\equiv C-CH_2CN$ | H | H | - | Cl | $OCH_3$ | CH | |
| L-4 | 0 | $C\equiv C-CH_2OCH_3$ | H | H | - | $CH_3$ | $CH_3$ | CH | |
| L-4 | 0 | $C\equiv C-CH_2OCH_3$ | H | H | - | $CH_3$ | $OCH_3$ | CH | |
| L-4 | 0 | $C\equiv C-CH_2OCH_3$ | H | H | - | $OCH_3$ | $OCH_3$ | CH | |
| L-4 | 0 | $C\equiv C-CH_2OCH_3$ | H | H | - | $CH_3$ | $CH_3$ | N | |
| L-4 | 0 | $C\equiv C-CH_2OCH_3$ | H | H | - | $CH_3$ | $OCH_3$ | N | |
| L-4 | 0 | $C\equiv C-CH_2OCH_3$ | H | H | - | $OCH_3$ | $OCH_3$ | N | |
| L-4 | 0 | $C\equiv C-CH_2OCH_3$ | H | H | - | Cl | $OCH_3$ | CH | |
| L-4 | 0 | $C\equiv CCH_2OSO_2CH_3$ | H | H | - | $CH_3$ | $CH_3$ | CH | |
| L-4 | 0 | $C\equiv CCH_2OSO_2CH_3$ | H | H | - | $CH_3$ | $OCH_3$ | CH | |
| L-4 | 0 | $C\equiv CCH_2OSO_2CH_3$ | H | H | - | $OCH_3$ | $OCH_3$ | CH | |
| L-4 | 0 | $C\equiv CCH_2OSO_2CH_3$ | H | H | - | $CH_3$ | $CH_3$ | N | |
| L-4 | 0 | $C\equiv CCH_2OSO_2CH_3$ | H | H | - | $CH_3$ | $OCH_3$ | N | |
| L-4 | 0 | $C\equiv CCH_2OSO_2CH_3$ | H | H | - | $OCH_3$ | $OCH_3$ | N | |
| L-4 | 0 | $C\equiv CCH_2OSO_2CH_3$ | H | H | - | Cl | $OCH_3$ | CH | |
| L-4 | 0 | $C\equiv CCH_2SCH_3$ | H | H | - | $CH_3$ | $CH_3$ | CH | |

Table VIII (Continued)

| L | n | W | R | $R_1$ | $R_2$ | X | Y | Z | m.p. (°C) |
|---|---|---|---|---|---|---|---|---|---|
| L-4 | 0 | $C\equiv CCH_2SCH_3$ | H | H | - | $CH_3$ | $OCH_3$ | CH | |
| L-4 | 0 | $C\equiv CCH_2SCH_3$ | H | H | - | $OCH_3$ | $OCH_3$ | CH | |
| L-4 | 0 | $C\equiv CCH_2SCH_3$ | H | H | - | $CH_3$ | $CH_3$ | N | |
| L-4 | 0 | $C\equiv CCH_2SCH_3$ | H | H | - | $CH_3$ | $OCH_3$ | N | |
| L-4 | 0 | $C\equiv CCH_2SCH_3$ | H | H | - | $OCH_3$ | $OCH_3$ | N | |
| L-4 | 0 | $C\equiv CCH_2SCH_3$ | H | H | - | Cl | $OCH_3$ | CH | |
| L-4 | 1 | $HC=CH_2$ | H | H | - | $CH_3$ | $CH_3$ | CH | |
| L-4 | 1 | $HC=CH_2$ | H | H | - | $CH_3$ | $OCH_3$ | CH | |
| L-4 | 1 | $HC=CH_2$ | H | H | - | $OCH_3$ | $OCH_3$ | CH | |
| L-4 | 1 | $HC=CH_2$ | H | H | - | $CH_3$ | $CH_3$ | N | |
| L-4 | 1 | $HC=CH_2$ | H | H | - | $CH_3$ | $OCH_3$ | N | |
| L-4 | 1 | $HC=CH_2$ | H | H | - | $OCH_3$ | $OCH_3$ | N | |
| L-4 | 1 | $HC=CH_2$ | H | H | - | Cl | $OCH_3$ | CH | |
| L-4 | 1 | $BrC=CH_2$ | H | H | - | $CH_3$ | $CH_3$ | CH | |
| L-4 | 1 | $BrC=CH_2$ | H | H | - | $CH_3$ | $OCH_3$ | CH | |
| L-4 | 1 | $BrC=CH_2$ | H | H | - | $OCH_3$ | $OCH_3$ | CH | |
| L-4 | 1 | $BrC=CH_2$ | H | H | - | $CH_3$ | $CH_3$ | N | |
| L-4 | 1 | $BrC=CH_2$ | H | H | - | $CH_3$ | $OCH_3$ | N | |
| L-4 | 1 | $BrC=CH_2$ | H | H | - | $OCH_3$ | $OCH_3$ | N | |
| L-4 | 1 | $BrC=CH_2$ | H | H | - | Cl | $OCH_3$ | CH | |
| L-4 | 1 | $HC=C(CH_3)_2$ | H | H | - | $CH_3$ | $CH_3$ | CH | |
| L-4 | 1 | $HC=C(CH_3)_2$ | H | H | - | $CH_3$ | $OCH_3$ | CH | |
| L-4 | 1 | $HC=C(CH_3)_2$ | H | H | - | $OCH_3$ | $OCH_3$ | CH | |
| L-4 | 1 | $HC=C(CH_3)_2$ | H | H | - | $CH_3$ | $CH_3$ | N | |
| L-4 | 1 | $HC=C(CH_3)_2$ | H | H | - | $CH_3$ | $OCH_3$ | N | |
| L-4 | 1 | $HC=C(CH_3)_2$ | H | H | - | $OCH_3$ | $OCH_3$ | N | |
| L-4 | 1 | $HC=C(CH_3)_2$ | H | H | - | Cl | $OCH_3$ | CH | |
| L-4 | 1 | $HC=C(CH_3)CH_2CH_2CH=C(CH_3)_2$ | H | H | - | $CH_3$ | $CH_3$ | CH | |
| L-4 | 1 | $HC=C(CH_3)CH_2CH_2CH=C(CH_3)_2$ | H | H | - | $CH_3$ | $OCH_3$ | CH | |
| L-4 | 1 | $HC=C(CH_3)CH_2CH_2CH=C(CH_3)_2$ | H | H | - | $OCH_3$ | $OCH_3$ | CH | |
| L-4 | 1 | $HC=C(CH_3)CH_2CH_2CH=C(CH_3)_2$ | H | H | - | $CH_3$ | $CH_3$ | N | |

Table VIII (Continued)

| L | n | W | R | $R_1$ | $R_2$ | X | Y | Z | m.p. (°C) |
|---|---|---|---|---|---|---|---|---|---|
| L-4 | 1 | $HC=C(CH_3)CH_2CH_2CH=C(CH_3)_2$ | H | H | - | $CH_3$ | $OCH_3$ | N | |
| L-4 | 1 | $HC=C(CH_3)CH_2CH_2CH=C(CH_3)_2$ | H | H | - | $OCH_3$ | $OCH_3$ | N | |
| L-4 | 1 | $HC=C(CH_3)CH_2CH_2CH=C(CH_3)_2$ | H | H | - | Cl | $OCH_3$ | CH | |
| L-4 | 1 | HC=CH-Ph | H | H | - | $CH_3$ | $CH_3$ | CH | |
| L-4 | 1 | HC=CH-Ph | H | H | - | $CH_3$ | $OCH_3$ | CH | |
| L-4 | 1 | HC=CH-Ph | H | H | - | $OCH_3$ | $OCH_3$ | CH | |
| L-4 | 1 | HC=CH-Ph | H | H | - | $CH_3$ | $CH_3$ | N | |
| L-4 | 1 | HC=CH-Ph | H | H | - | $CH_3$ | $OCH_3$ | N | |
| L-4 | 1 | HC=CH-Ph | H | H | - | $OCH_3$ | $OCH_3$ | N | |
| L-4 | 1 | HC=CH-Ph | H | H | - | Cl | $OCH_3$ | CH | |
| L-4 | 1 | $HC=CHCH_3$ | H | H | - | $CH_3$ | $CH_3$ | CH | |
| L-4 | 1 | $HC=CHCH_3$ | H | H | - | $CH_3$ | $OCH_3$ | CH | |
| L-4 | 1 | $HC=CHCH_3$ | H | H | - | $OCH_3$ | $OCH_3$ | CH | |
| L-4 | 1 | $HC=CHCH_3$ | H | H | - | $CH_3$ | $CH_3$ | N | |
| L-4 | 1 | $HC=CHCH_3$ | H | H | - | $CH_3$ | $OCH_3$ | N | |
| L-4 | 1 | $HC=CHCH_3$ | H | H | - | $OCH_3$ | $OCH_3$ | N | |
| L-4 | 1 | $HC=CHCH_3$ | H | H | - | Cl | $OCH_3$ | CH | |
| L-4 | 1 | $HC=CHCH_2Br$ | H | H | - | $CH_3$ | $CH_3$ | CH | |
| L-4 | 1 | $HC=CHCH_2Br$ | H | H | - | $CH_3$ | $OCH_3$ | CH | |
| L-4 | 1 | $HC=CHCH_2Br$ | H | H | - | $OCH_3$ | $OCH_3$ | CH | |
| L-4 | 1 | $HC=CHCH_2Br$ | H | H | - | $CH_3$ | $CH_3$ | N | |
| L-4 | 1 | $HC=CHCH_2Br$ | H | H | - | $CH_3$ | $OCH_3$ | N | |
| L-4 | 1 | $HC=CHCH_2Br$ | H | H | - | $OCH_3$ | $OCH_3$ | N | |
| L-4 | 1 | $HC=CHCH_2Br$ | H | H | - | Cl | $OCH_3$ | CH | |
| L-4 | 1 | C≡CH | H | H | - | $CH_3$ | $CH_3$ | CH | |
| L-4 | 1 | C≡CH | H | H | - | $CH_3$ | $OCH_3$ | CH | |
| L-4 | 1 | C≡CH | H | H | - | $OCH_3$ | $OCH_3$ | CH | |
| L-4 | 1 | C≡CH | H | H | - | $CH_3$ | $CH_3$ | N | |
| L-4 | 1 | C≡CH | H | H | - | $CH_3$ | $OCH_3$ | N | |
| L-4 | 1 | C≡CH | H | H | - | $OCH_3$ | $OCH_3$ | N | |
| L-4 | 1 | C≡CH | H | H | - | Cl | $OCH_3$ | CH | |

Table VIII (Continued)

| L | n | W | R | $R_1$ | $R_2$ | X | Y | Z | m.p. (°C) |
|---|---|---|---|---|---|---|---|---|---|
| L-4 | 1 | C≡CCH$_3$ | H | H | - | CH$_3$ | CH$_3$ | CH | |
| L-4 | 1 | C≡CCH$_3$ | H | H | - | CH$_3$ | OCH$_3$ | CH | |
| L-4 | 1 | C≡CCH$_3$ | H | H | - | OCH$_3$ | OCH$_3$ | CH | |
| L-4 | 1 | C≡CCH$_3$ | H | H | - | CH$_3$ | CH$_3$ | N | |
| L-4 | 1 | C≡CCH$_3$ | H | H | - | CH$_3$ | OCH$_3$ | N | |
| L-4 | 1 | C≡CCH$_3$ | H | H | - | OCH$_3$ | OCH$_3$ | N | |
| L-4 | 1 | C≡CCH$_3$ | H | H | - | Cl | OCH$_3$ | CH | |
| L-4 | 1 | C≡C-CH$_2$Br | H | H | - | CH$_3$ | CH$_3$ | CH | |
| L-4 | 1 | C≡C-CH$_2$Br | H | H | - | CH$_3$ | OCH$_3$ | CH | |
| L-4 | 1 | C≡C-CH$_2$Br | H | H | - | OCH$_3$ | OCH$_3$ | CH | |
| L-4 | 1 | C≡C-CH$_2$Br | H | H | - | CH$_3$ | CH$_3$ | N | |
| L-4 | 1 | C≡C-CH$_2$Br | H | H | - | CH$_3$ | OCH$_3$ | N | |
| L-4 | 1 | C≡C-CH$_2$Br | H | H | - | OCH$_3$ | OCH$_3$ | N | |
| L-4 | 1 | C≡C-CH$_2$Br | H | H | - | Cl | OCH$_3$ | CH | |
| L-4 | 1 | C≡C-Ph | H | H | - | CH$_3$ | CH$_3$ | CH | |
| L-4 | 1 | C≡C-Ph | H | H | - | CH$_3$ | OCH$_3$ | CH | |
| L-4 | 1 | C≡C-Ph | H | H | - | OCH$_3$ | OCH$_3$ | CH | |
| L-4 | 1 | C≡C-Ph | H | H | - | CH$_3$ | CH$_3$ | N | |
| L-4 | 1 | C≡C-Ph | H | H | - | CH$_3$ | OCH$_3$ | N | |
| L-4 | 1 | C≡C-Ph | H | H | - | OCH$_3$ | OCH$_3$ | N | |
| L-4 | 1 | C≡C-Ph | H | H | - | Cl | OCH$_3$ | CH | |
| L-4 | 1 | CH=CH$_2$ | CH$_3$ | H | - | CH$_3$ | CH$_3$ | CH | |
| L-4 | 1 | CH=CH$_2$ | CH$_3$ | H | - | CH$_3$ | OCH$_3$ | CH | |
| L-4 | 1 | CH=CH$_2$ | CH$_3$ | H | - | OCH$_3$ | OCH$_3$ | CH | |
| L-4 | 1 | CH=CH$_2$ | CH$_3$ | H | - | CH$_3$ | CH$_3$ | N | |
| L-4 | 1 | CH=CH$_2$ | CH$_3$ | H | - | CH$_3$ | OCH$_3$ | N | |
| L-4 | 1 | CH=CH$_2$ | CH$_3$ | H | - | OCH$_3$ | OCH$_3$ | N | |
| L-4 | 1 | CH=CH$_2$ | CH$_3$ | H | - | Cl | OCH$_3$ | CH | |
| L-4 | 1 | C≡CH | CH$_3$ | H | - | CH$_3$ | CH$_3$ | CH | |
| L-4 | 1 | C≡CH | CH$_3$ | H | - | CH$_3$ | OCH$_3$ | CH | |
| L-4 | 1 | C≡CH | CH$_3$ | H | - | OCH$_3$ | OCH$_3$ | CH | |

Table VIII (Continued)

| L | n | W | R | $R_1$ | $R_2$ | X | Y | Z | m.p. (°C) |
|---|---|---|---|---|---|---|---|---|---|
| L-4 | 1 | C≡CH | $CH_3$ | H | - | $CH_3$ | $CH_3$ | N | |
| L-4 | 1 | C≡CH | $CH_3$ | H | - | $CH_3$ | $OCH_3$ | N | |
| L-4 | 1 | C≡CH | $CH_3$ | H | - | $OCH_3$ | $OCH_3$ | N | |
| L-4 | 1 | C≡CH | $CH_3$ | H | - | Cl | $OCH_3$ | CH | |
| L-4 | 1 | $CH=CH_2$ | H | $CH_3$ | - | $CH_3$ | $CH_3$ | CH | |
| L-4 | 1 | $CH=CH_2$ | H | $CH_3$ | - | $CH_3$ | $OCH_3$ | CH | |
| L-4 | 1 | $CH=CH_2$ | H | $CH_3$ | - | $OCH_3$ | $OCH_3$ | CH | |
| L-4 | 1 | $CH=CH_2$ | H | $CH_3$ | - | $CH_3$ | $CH_3$ | N | |
| L-4 | 1 | $CH=CH_2$ | H | $CH_3$ | - | $CH_3$ | $OCH_3$ | N | |
| L-4 | 1 | $CH=CH_2$ | H | $CH_3$ | - | $OCH_3$ | $OCH_3$ | N | |
| L-4 | 1 | $CH=CH_2$ | H | $CH_3$ | - | Cl | $OCH_3$ | CH | |
| L-4 | 1 | C≡CH | H | $CH_3$ | - | $OCH_3$ | $OCH_3$ | CH | |
| L-4 | 1 | C≡CH | H | $CH_3$ | - | $OCH_3$ | $OCH_3$ | N | |
| L-4 | 1 | C≡CH | H | $CH_3$ | - | $OCH_3$ | $CH_3$ | CH | |
| L-5 | 0 | $CH=CH_2$ | H | H | - | $CH_3$ | $CH_3$ | CH | |
| L-5 | 0 | $CH=CH_2$ | H | H | - | $CH_3$ | $OCH_3$ | CH | |
| L-5 | 0 | $CH=CH_2$ | H | H | - | $OCH_3$ | $OCH_3$ | CH | |
| L-5 | 0 | $CH=CH_2$ | H | H | - | $CH_3$ | $CH_3$ | N | |
| L-5 | 0 | $CH=CH_2$ | H | H | - | $CH_3$ | $OCH_3$ | N | |
| L-5 | 0 | $CH=CH_2$ | H | H | - | $OCH_3$ | $OCH_3$ | N | |
| L-5 | 0 | $CH=CH_2$ | H | H | - | Cl | $OCH_3$ | CH | |
| L-5 | 0 | $C(CH_3)=CHCH_3$ | H | H | - | $CH_3$ | $CH_3$ | CH | |
| L-5 | 0 | $C(CH_3)=CHCH_3$ | H | H | - | $CH_3$ | $OCH_3$ | CH | |
| L-5 | 0 | $C(CH_3)=CHCH_3$ | H | H | - | $OCH_3$ | $OCH_3$ | CH | |
| L-5 | 0 | $C(CH_3)=CHCH_3$ | H | H | - | $CH_3$ | $CH_3$ | N | |
| L-5 | 0 | $C(CH_3)=CHCH_3$ | H | H | - | $CH_3$ | $OCH_3$ | N | |
| L-5 | 0 | $C(CH_3)=CHCH_3$ | H | H | - | $OCH_3$ | $OCH_3$ | N | |
| L-5 | 0 | $C(CH_3)=CHCH_3$ | H | H | - | Cl | $OCH_3$ | CH | |
| L-5 | 0 | $CH=CHCH_2CH_3$ | H | H | - | $CH_3$ | $CH_3$ | CH | |
| L-5 | 0 | $CH=CHCH_2CH_3$ | H | H | - | $CH_3$ | $OCH_3$ | CH | |
| L-5 | 0 | $CH=CHCH_2CH_3$ | H | H | - | $OCH_3$ | $OCH_3$ | CH | |

Table VIII (Continued)

| L | n | W | R | $R_1$ | $R_2$ | X | Y | Z | m.p. (°C) |
|---|---|---|---|---|---|---|---|---|---|
| L-5 | 0 | $CH=CHCH_2CH_3$ | H | H | - | $CH_3$ | $CH_3$ | N | |
| L-5 | 0 | $CH=CHCH_2CH_3$ | H | H | - | $CH_3$ | $OCH_3$ | N | |
| L-5 | 0 | $CH=CHCH_2CH_3$ | H | H | - | $OCH_3$ | $OCH_3$ | N | |
| L-5 | 0 | $CH=CHCH_2CH_3$ | H | H | - | Cl | $OCH_3$ | CH | |
| L-5 | 0 | $C(CH_3)=CHCH_2CH_3$ | H | H | - | $CH_3$ | $CH_3$ | CH | |
| L-5 | 0 | $C(CH_3)=CHCH_2CH_3$ | H | H | - | $CH_3$ | $OCH_3$ | CH | |
| L-5 | 0 | $C(CH_3)=CHCH_2CH_3$ | H | H | - | $OCH_3$ | $OCH_3$ | CH | |
| L-5 | 0 | $C(CH_3)=CHCH_2CH_3$ | H | H | - | $CH_3$ | $CH_3$ | N | |
| L-5 | 0 | $C(CH_3)=CHCH_2CH_3$ | H | H | - | $CH_3$ | $OCH_3$ | N | |
| L-5 | 0 | $C(CH_3)=CHCH_2CH_3$ | H | H | - | $OCH_3$ | $OCH_3$ | N | |
| L-5 | 0 | $C(CH_3)=CHCH_2CH_3$ | H | H | - | Cl | $OCH_3$ | CH | |
| L-5 | 0 | $CH=CHCH_2CH_2CH_3$ | H | H | - | $CH_3$ | $CH_3$ | CH | |
| L-5 | 0 | $CH=CHCH_2CH_2CH_3$ | H | H | - | $CH_3$ | $OCH_3$ | CH | |
| L-5 | 0 | $CH=CHCH_2CH_2CH_3$ | H | H | - | $OCH_3$ | $OCH_3$ | CH | |
| L-5 | 0 | $CH=CHCH_2CH_2CH_3$ | H | H | - | $CH_3$ | $CH_3$ | N | |
| L-5 | 0 | $CH=CHCH_2CH_2CH_3$ | H | H | - | $CH_3$ | $OCH_3$ | N | |
| L-5 | 0 | $CH=CHCH_2CH_2CH_3$ | H | H | - | $OCH_3$ | $OCH_3$ | N | |
| L-5 | 0 | $CH=CHCH_2CH_2CH_3$ | H | H | - | Cl | $OCH_3$ | CH | |
| L-5 | 0 | CH=CH-CN | H | H | - | $CH_3$ | $CH_3$ | CH | |
| L-5 | 0 | CH=CH-CN | H | H | - | $CH_3$ | $OCH_3$ | CH | |
| L-5 | 0 | CH=CH-CN | H | H | - | $OCH_3$ | $OCH_3$ | CH | |
| L-5 | 0 | CH=CH-CN | H | H | - | $CH_3$ | $CH_3$ | N | |
| L-5 | 0 | CH=CH-CN | H | H | - | $CH_3$ | $OCH_3$ | N | |
| L-5 | 0 | CH=CH-CN | H | H | - | $OCH_3$ | $OCH_3$ | N | |
| L-5 | 0 | CH=CH-CN | H | H | - | Cl | $OCH_3$ | CH | |
| L-5 | 0 | $CH=CH-OCH_3$ | H | H | - | $CH_3$ | $CH_3$ | CH | |
| L-5 | 0 | $CH=CH-OCH_3$ | H | H | - | $CH_3$ | $OCH_3$ | CH | |
| L-5 | 0 | $CH=CH-OCH_3$ | H | H | - | $OCH_3$ | $OCH_3$ | CH | |
| L-5 | 0 | $CH=CH-OCH_3$ | H | H | - | $CH_3$ | $CH_3$ | N | |
| L-5 | 0 | $CH=CH-OCH_3$ | H | H | - | $CH_3$ | $OCH_3$ | N | |
| L-5 | 0 | $CH=CH-OCH_3$ | H | H | - | $OCH_3$ | $OCH_3$ | N | |

Table VIII (Continued)

| L | n | W | R | $R_1$ | $R_2$ | X | Y | Z | m.p. (°C) |
|---|---|---|---|---|---|---|---|---|---|
| L-5 | 0 | $CH=CH-OCH_3$ | H | H | - | Cl | $OCH_3$ | CH | |
| L-5 | 0 | CH=CH-Br | H | H | - | $CH_3$ | $CH_3$ | CH | |
| L-5 | 0 | CH=CH-Br | H | H | - | $CH_3$ | $OCH_3$ | CH | |
| L-5 | 0 | CH=CH-Br | H | H | - | $OCH_3$ | $OCH_3$ | CH | |
| L-5 | 0 | CH=CH-Br | H | H | - | $CH_3$ | $CH_3$ | N | |
| L-5 | 0 | CH=CH-Br | H | H | - | $CH_3$ | $OCH_3$ | N | |
| L-5 | 0 | CH=CH-Br | H | H | - | $OCH_3$ | $OCH_3$ | N | |
| L-5 | 0 | CH=CH-Br | H | H | - | Cl | $OCH_3$ | CH | |
| L-5 | 0 | $CH=CH-CF_3$ | H | H | - | $CH_3$ | $CH_3$ | CH | |
| L-5 | 0 | $CH=CH-CF_3$ | H | H | - | $CH_3$ | $OCH_3$ | CH | |
| L-5 | 0 | $CH=CH-CF_3$ | H | H | - | $OCH_3$ | $OCH_3$ | CH | |
| L-5 | 0 | $CH=CH-CF_3$ | H | H | - | $CH_3$ | $CH_3$ | N | |
| L-5 | 0 | $CH=CH-CF_3$ | H | H | - | $CH_3$ | $OCH_3$ | N | |
| L-5 | 0 | $CH=CH-CF_3$ | H | H | - | $OCH_3$ | $OCH_3$ | N | |
| L-5 | 0 | $CH=CH-CF_3$ | H | H | - | Cl | $OCH_3$ | CH | |
| L-5 | 0 | $C(CH_3)=CH-OCH_3$ | H | H | - | $CH_3$ | $CH_3$ | CH | |
| L-5 | 0 | $C(CH_3)=CH-OCH_3$ | H | H | - | $CH_3$ | $OCH_3$ | CH | |
| L-5 | 0 | $C(CH_3)=CH-OCH_3$ | H | H | - | $OCH_3$ | $OCH_3$ | CH | |
| L-5 | 0 | $C(CH_3)=CH-OCH_3$ | H | H | - | $CH_3$ | $CH_3$ | N | |
| L-5 | 0 | $C(CH_3)=CH-OCH_3$ | H | H | - | $CH_3$ | $OCH_3$ | N | |
| L-5 | 0 | $C(CH_3)=CH-OCH_3$ | H | H | - | $OCH_3$ | $OCH_3$ | N | |
| L-5 | 0 | $C(CH_3)=CH-OCH_3$ | H | H | - | Cl | $OCH_3$ | CH | |
| L-5 | 0 | CH=CHPh | H | H | - | $CH_3$ | $CH_3$ | CH | |
| L-5 | 0 | CH=CHPh | H | H | - | $CH_3$ | $OCH_3$ | CH | |
| L-5 | 0 | CH=CHPh | H | H | - | $OCH_3$ | $OCH_3$ | CH | |
| L-5 | 0 | CH=CHPh | H | H | - | $CH_3$ | $CH_3$ | N | |
| L-5 | 0 | CH=CHPh | H | H | - | $CH_3$ | $OCH_3$ | N | |
| L-5 | 0 | CH=CHPh | H | H | - | $OCH_3$ | $OCH_3$ | N | |
| L-5 | 0 | CH=CHPh | H | H | - | Cl | $OCH_3$ | CH | |
| L-5 | 0 | $C(CH_3)=CH-Ph$ | H | H | - | $CH_3$ | $CH_3$ | CH | |
| L-5 | 0 | $C(CH_3)=CH-Ph$ | H | H | - | $CH_3$ | $OCH_3$ | CH | |
| L-5 | 0 | $C(CH_3)=CH-Ph$ | H | H | - | $OCH_3$ | $OCH_3$ | CH | |

Table VIII (Continued)

| L | n | W | R | $R_1$ | $R_2$ | X | Y | Z | m.p. (°C) |
|---|---|---|---|---|---|---|---|---|---|
| L-5 | 0 | C($CH_3$)=CH-Ph | H | H | – | $CH_3$ | $CH_3$ | N | |
| L-5 | 0 | C($CH_3$)=CH-Ph | H | H | – | $CH_3$ | $OCH_3$ | N | |
| L-5 | 0 | C($CH_3$)=CH-Ph | H | H | – | $OCH_3$ | $OCH_3$ | N | |
| L-5 | 0 | C($CH_3$)=CH-Ph | H | H | – | Cl | $OCH_3$ | CH | |
| L-5 | 0 | C=CH-$(CH_2)_3$ | H | H | – | $CH_3$ | $CH_3$ | CH | |
| L-5 | 0 | C=CH-$(CH_2)_3$ | H | H | – | $CH_3$ | $OCH_3$ | CH | |
| L-5 | 0 | C=CH-$(CH_2)_3$ | H | H | – | $OCH_3$ | $OCH_3$ | CH | |
| L-5 | 0 | C=CH-$(CH_2)_3$ | H | H | – | $CH_3$ | $CH_3$ | N | |
| L-5 | 0 | C=CH-$(CH_2)_3$ | H | H | – | $CH_3$ | $OCH_3$ | N | |
| L-5 | 0 | C=CH-$(CH_2)_3$ | H | H | – | $OCH_3$ | $OCH_3$ | N | |
| L-5 | 0 | C=CH-$(CH_2)_3$ | H | H | – | Cl | $OCH_3$ | CH | |
| L-5 | 0 | C=CH-$(CH_2)_4$ | H | H | – | $CH_3$ | $CH_3$ | CH | |
| L-5 | 0 | C=CH-$(CH_2)_4$ | H | H | – | $CH_3$ | $OCH_3$ | CH | |
| L-5 | 0 | C=CH-$(CH_2)_4$ | H | H | – | $OCH_3$ | $OCH_3$ | CH | |
| L-5 | 0 | C=CH-$(CH_2)_4$ | H | H | – | $CH_3$ | $CH_3$ | N | |
| L-5 | 0 | C=CH-$(CH_2)_4$ | H | H | – | $CH_3$ | $OCH_3$ | N | |
| L-5 | 0 | C=CH-$(CH_2)_4$ | H | H | – | $OCH_3$ | $OCH_3$ | N | |
| L-5 | 0 | C=CH-$(CH_2)_4$ | H | H | – | Cl | $OCH_3$ | CH | |
| L-5 | 0 | C=CH-$(CH_2)_5$ | H | H | – | $CH_3$ | $CH_3$ | CH | |
| L-5 | 0 | C=CH-$(CH_2)_5$ | H | H | – | $CH_3$ | $OCH_3$ | CH | |
| L-5 | 0 | C=CH-$(CH_2)_5$ | H | H | – | $OCH_3$ | $OCH_3$ | CH | |
| L-5 | 0 | C=CH-$(CH_2)_5$ | H | H | – | $CH_3$ | $CH_3$ | N | |
| L-5 | 0 | C=CH-$(CH_2)_5$ | H | H | – | $CH_3$ | $OCH_3$ | N | |
| L-5 | 0 | C=CH-$(CH_2)_5$ | H | H | – | $OCH_3$ | $OCH_3$ | N | |
| L-5 | 0 | C=CH-$(CH_2)_5$ | H | H | – | Cl | $OCH_3$ | CH | |
| L-5 | 0 | C(CN)=CHCN | H | H | – | $CH_3$ | $CH_3$ | CH | |

Table VIII (Continued)

| L | n | W | R | $R_1$ | $R_2$ | X | Y | Z | m.p. (°C) |
|---|---|---|---|---|---|---|---|---|---|
| L-5 | 0 | C(CN)=CHCN | H | H | - | $CH_3$ | $OCH_3$ | CH | |
| L-5 | 0 | C(CN)=CHCN | H | H | - | $OCH_3$ | $OCH_3$ | CH | |
| L-5 | 0 | C(CN)=CHCN | H | H | - | $CH_3$ | $CH_3$ | N | |
| L-5 | 0 | C(CN)=CHCN | H | H | - | $CH_3$ | $OCH_3$ | N | |
| L-5 | 0 | C(CN)=CHCN | H | H | - | $OCH_3$ | $OCH_3$ | N | |
| L-5 | 0 | C(CN)=CHCN | H | H | - | Cl | $OCH_3$ | CH | |
| L-5 | 0 | $CH=CH-SO_2N(CH_3)_2$ | H | H | - | $CH_3$ | $CH_3$ | CH | |
| L-5 | 0 | $CH=CH-SO_2N(CH_3)_2$ | H | H | - | $CH_3$ | $OCH_3$ | CH | |
| L-5 | 0 | $CH=CH-SO_2N(CH_3)_2$ | H | H | - | $OCH_3$ | $OCH_3$ | CH | |
| L-5 | 0 | $CH=CH-SO_2N(CH_3)_2$ | H | H | - | $CH_3$ | $CH_3$ | N | |
| L-5 | 0 | $CH=CH-SO_2N(CH_3)_2$ | H | H | - | $CH_3$ | $OCH_3$ | N | |
| L-5 | 0 | $CH=CH-SO_2N(CH_3)_2$ | H | H | - | $OCH_3$ | $OCH_3$ | N | |
| L-5 | 0 | $CH=CH-SO_2N(CH_3)_2$ | H | H | - | Cl | $OCH_3$ | CH | |
| L-5 | 0 | $\overline{C=CH-(CH_2)_4}$ | $CH_3$ | H | - | $OCH_3$ | $OCH_3$ | CH | |
| L-5 | 0 | $\overline{C=CH-(CH_2)_4}$ | H | $CH_3$ | - | $OCH_3$ | $OCH_3$ | CH | |
| L-5 | 0 | $C(=CH_2)-CH_2CH_2CH(NO_2)CH_3$ | H | H | - | $OCH_3$ | $OCH_3$ | CH | |
| L-5 | 0 | $CH=CHCH_2OSO_2Ph$ | H | H | - | $OCH_3$ | $OCH_3$ | CH | |
| L-5 | 0 | $CH=CHCH_2OSO_2CH_3$ | H | H | - | $OCH_3$ | $OCH_3$ | CH | |
| L-5 | 0 | $CH=CHSCH_3$ | H | H | - | $OCH_3$ | $OCH_3$ | CH | |
| L-5 | 0 | C≡CH | H | H | - | $CH_3$ | $CH_3$ | CH | |
| L-5 | 0 | C≡CH | H | H | - | $CH_3$ | $OCH_3$ | CH | |
| L-5 | 0 | C≡CH | H | H | - | $OCH_3$ | $OCH_3$ | CH | |
| L-5 | 0 | C≡CH | H | H | - | $CH_3$ | $CH_3$ | N | |
| L-5 | 0 | C≡CH | H | H | - | $CH_3$ | $OCH_3$ | N | |
| L-5 | 0 | C≡CH | H | H | - | $OCH_3$ | $OCH_3$ | N | |

Table VIII (Continued)

| L | n | W | R | $R_1$ | $R_2$ | X | Y | Z | m.p. (°C) |
|---|---|---|---|---|---|---|---|---|---|
| L-5 | 0 | C≡CH | H | H | - | Cl | $OCH_3$ | CH | |
| L-5 | 0 | C≡C-Ph | H | H | - | $CH_3$ | $CH_3$ | CH | |
| L-5 | 0 | C≡C-Ph | H | H | - | $CH_3$ | $OCH_3$ | CH | |
| L-5 | 0 | C≡C-Ph | H | H | - | $OCH_3$ | $OCH_3$ | CH | |
| L-5 | 0 | C≡C-Ph | H | H | - | $CH_3$ | $CH_3$ | N | |
| L-5 | 0 | C≡C-Ph | H | H | - | $CH_3$ | $OCH_3$ | N | |
| L-5 | 0 | C≡C-Ph | H | H | - | $OCH_3$ | $OCH_3$ | N | |
| L-5 | 0 | C≡C-Ph | H | H | - | Cl | $OCH_3$ | CH | |
| L-5 | 0 | C≡C-$CH_3$ | H | H | - | $CH_3$ | $CH_3$ | CH | |
| L-5 | 0 | C≡C-$CH_3$ | H | H | - | $CH_3$ | $OCH_3$ | CH | |
| L-5 | 0 | C≡C-$CH_3$ | H | H | - | $OCH_3$ | $OCH_3$ | CH | |
| L-5 | 0 | C≡C-$CH_3$ | H | H | - | $CH_3$ | $CH_3$ | N | |
| L-5 | 0 | C≡C-$CH_3$ | H | H | - | $CH_3$ | $OCH_3$ | N | |
| L-5 | 0 | C≡C-$CH_3$ | H | H | - | $OCH_3$ | $OCH_3$ | N | |
| L-5 | 0 | C≡C-$CH_3$ | H | H | - | Cl | $OCH_3$ | CH | |
| L-5 | 0 | C≡C$CH_2$OC(O)$CH_3$ | H | H | - | $CH_3$ | $CH_3$ | CH | |
| L-5 | 0 | C≡C$CH_2$OC(O)$CH_3$ | H | H | - | $CH_3$ | $OCH_3$ | CH | |
| L-5 | 0 | C≡C$CH_2$OC(O)$CH_3$ | H | H | - | $OCH_3$ | $OCH_3$ | CH | |
| L-5 | 0 | C≡C$CH_2$OC(O)$CH_3$ | H | H | - | $CH_3$ | $CH_3$ | N | |
| L-5 | 0 | C≡C$CH_2$OC(O)$CH_3$ | H | H | - | $CH_3$ | $OCH_3$ | N | |
| L-5 | 0 | C≡C$CH_2$OC(O)$CH_3$ | H | H | - | $OCH_3$ | $OCH_3$ | N | |
| L-5 | 0 | C≡C$CH_2$OC(O)$CH_3$ | H | H | - | Cl | $OCH_3$ | CH | |
| L-5 | 0 | C≡C$CH_2$OH | H | H | - | $CH_3$ | $CH_3$ | CH | |
| L-5 | 0 | C≡C$CH_2$OH | H | H | - | $CH_3$ | $OCH_3$ | CH | |
| L-5 | 0 | C≡C$CH_2$OH | H | H | - | $OCH_3$ | $OCH_3$ | CH | |
| L-5 | 0 | C≡C$CH_2$OH | H | H | - | $CH_3$ | $CH_3$ | N | |
| L-5 | 0 | C≡C$CH_2$OH | H | H | - | $CH_3$ | $OCH_3$ | N | |
| L-5 | 0 | C≡C$CH_2$OH | H | H | - | $OCH_3$ | $OCH_3$ | N | |
| L-5 | 0 | C≡C$CH_2$OH | H | H | - | Cl | $OCH_3$ | CH | |
| L-5 | 0 | C≡C$CH_2$Cl | H | H | - | $CH_3$ | $CH_3$ | CH | |
| L-5 | 0 | C≡C$CH_2$Cl | H | H | - | $CH_3$ | $OCH_3$ | CH | |
| L-5 | 0 | C≡C$CH_2$Cl | H | H | - | $OCH_3$ | $OCH_3$ | CH | |
| L-5 | 0 | C≡C$CH_2$Cl | H | H | - | $CH_3$ | $CH_3$ | N | |

Table VIII (Continued)

| L | n | W | R | $R_1$ | $R_2$ | X | Y | Z | m.p. (°C) |
|---|---|---|---|---|---|---|---|---|---|
| L-5 | 0 | $C\equiv CCH_2Cl$ | H | H | - | $CH_3$ | $OCH_3$ | N | |
| L-5 | 0 | $C\equiv CCH_2Cl$ | H | H | - | $OCH_3$ | $OCH_3$ | N | |
| L-5 | 0 | $C\equiv CCH_2Cl$ | H | H | - | Cl | $OCH_3$ | CH | |
| L-5 | 0 | $C\equiv C-CH_2Br$ | H | H | - | $CH_3$ | $CH_3$ | CH | |
| L-5 | 0 | $C\equiv C-CH_2Br$ | H | H | - | $CH_3$ | $OCH_3$ | CH | |
| L-5 | 0 | $C\equiv C-CH_2Br$ | H | H | - | $OCH_3$ | $OCH_3$ | CH | |
| L-5 | 0 | $C\equiv C-CH_2Br$ | H | H | - | $CH_3$ | $CH_3$ | N | |
| L-5 | 0 | $C\equiv C-CH_2Br$ | H | H | - | $CH_3$ | $OCH_3$ | N | |
| L-5 | 0 | $C\equiv C-CH_2Br$ | H | H | - | $OCH_3$ | $OCH_3$ | N | |
| L-5 | 0 | $C\equiv C-CH_2Br$ | H | H | - | Cl | $OCH_3$ | CH | |
| L-5 | 0 | $C\equiv C-CH_2CN$ | H | H | - | $CH_3$ | $CH_3$ | CH | |
| L-5 | 0 | $C\equiv C-CH_2CN$ | H | H | - | $CH_3$ | $OCH_3$ | CH | |
| L-5 | 0 | $C\equiv C-CH_2CN$ | H | H | - | $OCH_3$ | $OCH_3$ | CH | |
| L-5 | 0 | $C\equiv C-CH_2CN$ | H | H | - | $CH_3$ | $CH_3$ | N | |
| L-5 | 0 | $C\equiv C-CH_2CN$ | H | H | - | $CH_3$ | $OCH_3$ | N | |
| L-5 | 0 | $C\equiv C-CH_2CN$ | H | H | - | $OCH_3$ | $OCH_3$ | N | |
| L-5 | 0 | $C\equiv C-CH_2CN$ | H | H | - | Cl | $OCH_3$ | CH | |
| L-5 | 0 | $C\equiv C-CH_2OCH_3$ | H | H | - | $CH_3$ | $CH_3$ | CH | |
| L-5 | 0 | $C\equiv C-CH_2OCH_3$ | H | H | - | $CH_3$ | $OCH_3$ | CH | |
| L-5 | 0 | $C\equiv C-CH_2OCH_3$ | H | H | - | $OCH_3$ | $OCH_3$ | CH | |
| L-5 | 0 | $C\equiv C-CH_2OCH_3$ | H | H | - | $CH_3$ | $CH_3$ | N | |
| L-5 | 0 | $C\equiv C-CH_2OCH_3$ | H | H | - | $CH_3$ | $OCH_3$ | N | |
| L-5 | 0 | $C\equiv C-CH_2OCH_3$ | H | H | - | $OCH_3$ | $OCH_3$ | N | |
| L-5 | 0 | $C\equiv C-CH_2OCH_3$ | H | H | - | Cl | $OCH_3$ | CH | |
| L-5 | 0 | $C\equiv CCH_2OSO_2CH_3$ | H | H | - | $CH_3$ | $CH_3$ | CH | |
| L-5 | 0 | $C\equiv CCH_2OSO_2CH_3$ | H | H | - | $CH_3$ | $OCH_3$ | CH | |
| L-5 | 0 | $C\equiv CCH_2OSO_2CH_3$ | H | H | - | $OCH_3$ | $OCH_3$ | CH | |
| L-5 | 0 | $C\equiv CCH_2OSO_2CH_3$ | H | H | - | $CH_3$ | $CH_3$ | N | |
| L-5 | 0 | $C\equiv CCH_2OSO_2CH_3$ | H | H | - | $CH_3$ | $OCH_3$ | N | |
| L-5 | 0 | $C\equiv CCH_2OSO_2CH_3$ | H | H | - | $OCH_3$ | $OCH_3$ | N | |
| L-5 | 0 | $C\equiv CCH_2OSO_2CH_3$ | H | H | - | Cl | $OCH_3$ | CH | |

Table VIII (Continued)

| L | n | W | R | $R_1$ | $R_2$ | X | Y | Z | m.p. (°C) |
|---|---|---|---|---|---|---|---|---|---|
| L-5 | 0 | $C{\equiv}CCH_2SCH_3$ | H | H | - | $CH_3$ | $CH_3$ | CH | |
| L-5 | 0 | $C{\equiv}CCH_2SCH_3$ | H | H | - | $CH_3$ | $OCH_3$ | CH | |
| L-5 | 0 | $C{\equiv}CCH_2SCH_3$ | H | H | - | $OCH_3$ | $OCH_3$ | CH | |
| L-5 | 0 | $C{\equiv}CCH_2SCH_3$ | H | H | - | $CH_3$ | $CH_3$ | N | |
| L-5 | 0 | $C{\equiv}CCH_2SCH_3$ | H | H | - | $CH_3$ | $OCH_3$ | N | |
| L-5 | 0 | $C{\equiv}CCH_2SCH_3$ | H | H | - | $OCH_3$ | $OCH_3$ | N | |
| L-5 | 0 | $C{\equiv}CCH_2SCH_3$ | H | H | - | Cl | $OCH_3$ | CH | |
| L-5 | 1 | $HC{=}CH_2$ | H | H | - | $CH_3$ | $CH_3$ | CH | |
| L-5 | 1 | $HC{=}CH_2$ | H | H | - | $CH_3$ | $OCH_3$ | CH | |
| L-5 | 1 | $HC{=}CH_2$ | H | H | - | $OCH_3$ | $OCH_3$ | CH | |
| L-5 | 1 | $HC{=}CH_2$ | H | H | - | $CH_3$ | $CH_3$ | N | |
| L-5 | 1 | $HC{=}CH_2$ | H | H | - | $CH_3$ | $OCH_3$ | N | |
| L-5 | 1 | $HC{=}CH_2$ | H | H | - | $OCH_3$ | $OCH_3$ | N | |
| L-5 | 1 | $HC{=}CH_2$ | H | H | - | Cl | $OCH_3$ | CH | |
| L-5 | 1 | $BrC{=}CH_2$ | H | H | - | $CH_3$ | $CH_3$ | CH | |
| L-5 | 1 | $BrC{=}CH_2$ | H | H | - | $CH_3$ | $OCH_3$ | CH | |
| L-5 | 1 | $BrC{=}CH_2$ | H | H | - | $OCH_3$ | $OCH_3$ | CH | |
| L-5 | 1 | $BrC{=}CH_2$ | H | H | - | $CH_3$ | $CH_3$ | N | |
| L-5 | 1 | $BrC{=}CH_2$ | H | H | - | $CH_3$ | $OCH_3$ | N | |
| L-5 | 1 | $BrC{=}CH_2$ | H | H | - | $OCH_3$ | $OCH_3$ | N | |
| L-5 | 1 | $BrC{=}CH_2$ | H | H | - | Cl | $OCH_3$ | CH | |
| L-5 | 1 | $HC{=}C(CH_3)_2$ | H | H | - | $CH_3$ | $CH_3$ | CH | |
| L-5 | 1 | $HC{=}C(CH_3)_2$ | H | H | - | $CH_3$ | $OCH_3$ | CH | |
| L-5 | 1 | $HC{=}C(CH_3)_2$ | H | H | - | $OCH_3$ | $OCH_3$ | CH | |
| L-5 | 1 | $HC{=}C(CH_3)_2$ | H | H | - | $CH_3$ | $CH_3$ | N | |
| L-5 | 1 | $HC{=}C(CH_3)_2$ | H | H | - | $CH_3$ | $OCH_3$ | N | |
| L-5 | 1 | $HC{=}C(CH_3)_2$ | H | H | - | $OCH_3$ | $OCH_3$ | N | |
| L-5 | 1 | $HC{=}C(CH_3)_2$ | H | H | - | Cl | $OCH_3$ | CH | |
| L-5 | 1 | $HC{=}C(CH_3)CH_2CH_2CH{=}C(CH_3)_2$ | H | H | - | $CH_3$ | $CH_3$ | CH | |
| L-5 | 1 | $HC{=}C(CH_3)CH_2CH_2CH{=}C(CH_3)_2$ | H | H | - | $CH_3$ | $OCH_3$ | CH | |
| L-5 | 1 | $HC{=}C(CH_3)CH_2CH_2CH{=}C(CH_3)_2$ | H | H | - | $OCH_3$ | $OCH_3$ | CH | |

Table VIII (Continued)

| L | n | W | R | $R_1$ | $R_2$ | X | Y | Z | m.p. (°C) |
|---|---|---|---|---|---|---|---|---|---|
| L-5 | 1 | $HC=C(CH_3)CH_2CH_2CH=C(CH_3)_2$ | H | H | - | $CH_3$ | $CH_3$ | N | |
| L-5 | 1 | $HC=C(CH_3)CH_2CH_2CH=C(CH_3)_2$ | H | H | - | $CH_3$ | $OCH_3$ | N | |
| L-5 | 1 | $HC=C(CH_3)CH_2CH_2CH=C(CH_3)_2$ | H | H | - | $OCH_3$ | $OCH_3$ | N | |
| L-5 | 1 | $HC=C(CH_3)CH_2CH_2CH=C(CH_3)_2$ | H | H | - | Cl | $OCH_3$ | CH | |
| L-5 | 1 | HC=CH-Ph | H | H | - | $CH_3$ | $CH_3$ | CH | |
| L-5 | 1 | HC=CH-Ph | H | H | - | $CH_3$ | $OCH_3$ | CH | |
| L-5 | 1 | HC=CH-Ph | H | H | - | $OCH_3$ | $OCH_3$ | CH | |
| L-5 | 1 | HC=CH-Ph | H | H | - | $CH_3$ | $CH_3$ | N | |
| L-5 | 1 | HC=CH-Ph | H | H | - | $CH_3$ | $OCH_3$ | N | |
| L-5 | 1 | HC=CH-Ph | H | H | - | $OCH_3$ | $OCH_3$ | N | |
| L-5 | 1 | HC=CH-Ph | H | H | - | Cl | $OCH_3$ | CH | |
| L-5 | 1 | $HC=CHCH_3$ | H | H | - | $CH_3$ | $CH_3$ | CH | |
| L-5 | 1 | $HC=CHCH_3$ | H | H | - | $CH_3$ | $OCH_3$ | CH | |
| L-5 | 1 | $HC=CHCH_3$ | H | H | - | $OCH_3$ | $OCH_3$ | CH | |
| L-5 | 1 | $HC=CHCH_3$ | H | H | - | $CH_3$ | $CH_3$ | N | |
| L-5 | 1 | $HC=CHCH_3$ | H | H | - | $CH_3$ | $OCH_3$ | N | |
| L-5 | 1 | $HC=CHCH_3$ | H | H | - | $OCH_3$ | $OCH_3$ | N | |
| L-5 | 1 | $HC=CHCH_3$ | H | H | - | Cl | $OCH_3$ | CH | |
| L-5 | 1 | $HC=CHCH_2Br$ | H | H | - | $CH_3$ | $CH_3$ | CH | |
| L-5 | 1 | $HC=CHCH_2Br$ | H | H | - | $CH_3$ | $OCH_3$ | CH | |
| L-5 | 1 | $HC=CHCH_2Br$ | H | H | - | $OCH_3$ | $OCH_3$ | CH | |
| L-5 | 1 | $HC=CHCH_2Br$ | H | H | - | $CH_3$ | $CH_3$ | N | |
| L-5 | 1 | $HC=CHCH_2Br$ | H | H | - | $CH_3$ | $OCH_3$ | N | |
| L-5 | 1 | $HC=CHCH_2Br$ | H | H | - | $OCH_3$ | $OCH_3$ | N | |
| L-5 | 1 | $HC=CHCH_2Br$ | H | H | - | Cl | $OCH_3$ | CH | |
| L-5 | 1 | C≡CH | H | H | - | $CH_3$ | $CH_3$ | CH | |
| L-5 | 1 | C≡CH | H | H | - | $CH_3$ | $OCH_3$ | CH | |
| L-5 | 1 | C≡CH | H | H | - | $OCH_3$ | $OCH_3$ | CH | |
| L-5 | 1 | C≡CH | H | H | - | $CH_3$ | $CH_3$ | N | |
| L-5 | 1 | C≡CH | H | H | - | $CH_3$ | $OCH_3$ | N | |
| L-5 | 1 | C≡CH | H | H | - | $OCH_3$ | $OCH_3$ | N | |

Table VIII (Continued)

| L | n | W | R | $R_1$ | $R_2$ | X | Y | Z | m.p. (°C) |
|---|---|---|---|---|---|---|---|---|---|
| L-5 | 1 | C≡CH | H | H | - | Cl | $OCH_3$ | CH | |
| L-5 | 1 | $C{\equiv}CCH_3$ | H | H | - | $CH_3$ | $CH_3$ | CH | |
| L-5 | 1 | $C{\equiv}CCH_3$ | H | H | - | $CH_3$ | $OCH_3$ | CH | |
| L-5 | 1 | $C{\equiv}CCH_3$ | H | H | - | $OCH_3$ | $OCH_3$ | CH | |
| L-5 | 1 | $C{\equiv}CCH_3$ | H | H | - | $CH_3$ | $CH_3$ | N | |
| L-5 | 1 | $C{\equiv}CCH_3$ | H | H | - | $CH_3$ | $OCH_3$ | N | |
| L-5 | 1 | $C{\equiv}CCH_3$ | H | H | - | $OCH_3$ | $OCH_3$ | N | |
| L-5 | 1 | $C{\equiv}CCH_3$ | H | H | - | Cl | $OCH_3$ | CH | |
| L-5 | 1 | $C{\equiv}C-CH_2Br$ | H | H | - | $CH_3$ | $CH_3$ | CH | |
| L-5 | 1 | $C{\equiv}C-CH_2Br$ | H | H | - | $CH_3$ | $OCH_3$ | CH | |
| L-5 | 1 | $C{\equiv}C-CH_2Br$ | H | H | - | $OCH_3$ | $OCH_3$ | CH | |
| L-5 | 1 | $C{\equiv}C-CH_2Br$ | H | H | - | $CH_3$ | $CH_3$ | N | |
| L-5 | 1 | $C{\equiv}C-CH_2Br$ | H | H | - | $CH_3$ | $OCH_3$ | N | |
| L-5 | 1 | $C{\equiv}C-CH_2Br$ | H | H | - | $OCH_3$ | $OCH_3$ | N | |
| L-5 | 1 | $C{\equiv}C-CH_2Br$ | H | H | - | Cl | $OCH_3$ | CH | |
| L-5 | 1 | C≡C-Ph | H | H | - | $CH_3$ | $CH_3$ | CH | |
| L-5 | 1 | C≡C-Ph | H | H | - | $CH_3$ | $OCH_3$ | CH | |
| L-5 | 1 | C≡C-Ph | H | H | - | $OCH_3$ | $OCH_3$ | CH | |
| L-5 | 1 | C≡C-Ph | H | H | - | $CH_3$ | $CH_3$ | N | |
| L-5 | 1 | C≡C-Ph | H | H | - | $CH_3$ | $OCH_3$ | N | |
| L-5 | 1 | C≡C-Ph | H | H | - | $OCH_3$ | $OCH_3$ | N | |
| L-5 | 1 | C≡C-Ph | H | H | - | Cl | $OCH_3$ | CH | |
| L-5 | 1 | $CH=CH_2$ | $CH_3$ | H | - | $CH_3$ | $CH_3$ | CH | |
| L-5 | 1 | $CH=CH_2$ | $CH_3$ | H | - | $CH_3$ | $OCH_3$ | CH | |
| L-5 | 1 | $CH=CH_2$ | $CH_3$ | H | - | $OCH_3$ | $OCH_3$ | CH | |
| L-5 | 1 | $CH=CH_2$ | $CH_3$ | H | - | $CH_3$ | $CH_3$ | N | |
| L-5 | 1 | $CH=CH_2$ | $CH_3$ | H | - | $CH_3$ | $OCH_3$ | N | |
| L-5 | 1 | $CH=CH_2$ | $CH_3$ | H | - | $OCH_3$ | $OCH_3$ | N | |
| L-5 | 1 | $CH=CH_2$ | $CH_3$ | H | - | Cl | $OCH_3$ | CH | |
| L-5 | 1 | C≡CH | $CH_3$ | H | - | $CH_3$ | $CH_3$ | CH | |
| L-5 | 1 | C≡CH | $CH_3$ | H | - | $CH_3$ | $OCH_3$ | CH | |

Table VIII (Continued)

| L | n | W | R | $R_1$ | $R_2$ | X | Y | Z | m.p. (°C) |
|---|---|---|---|---|---|---|---|---|---|
| L-5 | 1 | C≡CH | $CH_3$ | H | - | $OCH_3$ | $OCH_3$ | CH | |
| L-5 | 1 | C≡CH | $CH_3$ | H | - | $CH_3$ | $CH_3$ | N | |
| L-5 | 1 | C≡CH | $CH_3$ | H | - | $CH_3$ | $OCH_3$ | N | |
| L-5 | 1 | C≡CH | $CH_3$ | H | - | $OCH_3$ | $OCH_3$ | N | |
| L-5 | 1 | C≡CH | $CH_3$ | H | - | Cl | $OCH_3$ | CH | |
| L-5 | 1 | $CH=CH_2$ | H | $CH_3$ | - | $CH_3$ | $CH_3$ | CH | |
| L-5 | 1 | $CH=CH_2$ | H | $CH_3$ | - | $CH_3$ | $OCH_3$ | CH | |
| L-5 | 1 | $CH=CH_2$ | H | $CH_3$ | - | $OCH_3$ | $OCH_3$ | CH | |
| L-5 | 1 | $CH=CH_2$ | H | $CH_3$ | - | $CH_3$ | $CH_3$ | N | |
| L-5 | 1 | $CH=CH_2$ | H | $CH_3$ | - | $CH_3$ | $OCH_3$ | N | |
| L-5 | 1 | $CH=CH_2$ | H | $CH_3$ | - | $OCH_3$ | $OCH_3$ | N | |
| L-5 | 1 | $CH=CH_2$ | H | $CH_3$ | - | Cl | $OCH_3$ | CH | |
| L-5 | 1 | C≡CH | H | $CH_3$ | - | $OCH_3$ | $OCH_3$ | CH | |
| L-5 | 1 | C≡CH | H | $CH_3$ | - | $OCH_3$ | $OCH_3$ | N | |
| L-5 | 1 | C≡CH | H | $CH_3$ | - | $OCH_3$ | $CH_3$ | CH | |
| L-6 | 0 | $CH=CH_2$ | H | H | - | $CH_3$ | $CH_3$ | CH | |
| L-6 | 0 | $CH=CH_2$ | H | H | - | $CH_3$ | $OCH_3$ | CH | |
| L-6 | 0 | $CH=CH_2$ | H | H | - | $OCH_3$ | $OCH_3$ | CH | |
| L-6 | 0 | $CH=CH_2$ | H | H | - | $CH_3$ | $CH_3$ | N | |
| L-6 | 0 | $CH=CH_2$ | H | H | - | $CH_3$ | $OCH_3$ | N | |
| L-6 | 0 | $CH=CH_2$ | H | H | - | $OCH_3$ | $OCH_3$ | N | |
| L-6 | 0 | $CH=CH_2$ | H | H | - | Cl | $OCH_3$ | CH | |
| L-6 | 0 | $C(CH_3)=CHCH_3$ | H | H | - | $CH_3$ | $CH_3$ | CH | |
| L-6 | 0 | $C(CH_3)=CHCH_3$ | H | H | - | $CH_3$ | $OCH_3$ | CH | |
| L-6 | 0 | $C(CH_3)=CHCH_3$ | H | H | - | $OCH_3$ | $OCH_3$ | CH | |
| L-6 | 0 | $C(CH_3)=CHCH_3$ | H | H | - | $CH_3$ | $CH_3$ | N | |
| L-6 | 0 | $C(CH_3)=CHCH_3$ | H | H | - | $CH_3$ | $OCH_3$ | N | |
| L-6 | 0 | $C(CH_3)=CHCH_3$ | H | H | - | $OCH_3$ | $OCH_3$ | N | |
| L-6 | 0 | $C(CH_3)=CHCH_3$ | H | H | - | Cl | $OCH_3$ | CH | |
| L-6 | 0 | $CH=CHCH_2CH_3$ | H | H | - | $CH_3$ | $CH_3$ | CH | |
| L-6 | 0 | $CH=CHCH_2CH_3$ | H | H | - | $CH_3$ | $OCH_3$ | CH | |

Table VIII (Continued)

| L | n | W | R | $R_1$ | $R_2$ | X | Y | Z | m.p. (°C) |
|---|---|---|---|---|---|---|---|---|---|
| L-6 | 0 | $CH=CHCH_2CH_3$ | H | H | - | $OCH_3$ | $OCH_3$ | CH | |
| L-6 | 0 | $CH=CHCH_2CH_3$ | H | H | - | $CH_3$ | $CH_3$ | N | |
| L-6 | 0 | $CH=CHCH_2CH_3$ | H | H | - | $CH_3$ | $OCH_3$ | N | |
| L-6 | 0 | $CH=CHCH_2CH_3$ | H | H | - | $OCH_3$ | $OCH_3$ | N | |
| L-6 | 0 | $CH=CHCH_2CH_3$ | H | H | - | Cl | $OCH_3$ | CH | |
| L-6 | 0 | $C(CH_3)=CHCH_2CH_3$ | H | H | - | $CH_3$ | $CH_3$ | CH | |
| L-6 | 0 | $C(CH_3)=CHCH_2CH_3$ | H | H | - | $CH_3$ | $OCH_3$ | CH | |
| L-6 | 0 | $C(CH_3)=CHCH_2CH_3$ | H | H | - | $OCH_3$ | $OCH_3$ | CH | |
| L-6 | 0 | $C(CH_3)=CHCH_2CH_3$ | H | H | - | $CH_3$ | $CH_3$ | N | |
| L-6 | 0 | $C(CH_3)=CHCH_2CH_3$ | H | H | - | $CH_3$ | $OCH_3$ | N | |
| L-6 | 0 | $C(CH_3)=CHCH_2CH_3$ | H | H | - | $OCH_3$ | $OCH_3$ | N | |
| L-6 | 0 | $C(CH_3)=CHCH_2CH_3$ | H | H | - | Cl | $OCH_3$ | CH | |
| L-6 | 0 | $CH=CHCH_2CH_2CH_3$ | H | H | - | $CH_3$ | $CH_3$ | CH | |
| L-6 | 0 | $CH=CHCH_2CH_2CH_3$ | H | H | - | $CH_3$ | $OCH_3$ | CH | |
| L-6 | 0 | $CH=CHCH_2CH_2CH_3$ | H | H | - | $OCH_3$ | $OCH_3$ | CH | |
| L-6 | 0 | $CH=CHCH_2CH_2CH_3$ | H | H | - | $CH_3$ | $CH_3$ | N | |
| L-6 | 0 | $CH=CHCH_2CH_2CH_3$ | H | H | - | $CH_3$ | $OCH_3$ | N | |
| L-6 | 0 | $CH=CHCH_2CH_2CH_3$ | H | H | - | $OCH_3$ | $OCH_3$ | N | |
| L-6 | 0 | $CH=CHCH_2CH_2CH_3$ | H | H | - | Cl | $OCH_3$ | CH | |
| L-6 | 0 | CH=CH-CN | H | H | - | $CH_3$ | $CH_3$ | CH | |
| L-6 | 0 | CH=CH-CN | H | H | - | $CH_3$ | $OCH_3$ | CH | |
| L-6 | 0 | CH=CH-CN | H | H | - | $OCH_3$ | $OCH_3$ | CH | |
| L-6 | 0 | CH=CH-CN | H | H | - | $CH_3$ | $CH_3$ | N | |
| L-6 | 0 | CH=CH-CN | H | H | - | $CH_3$ | $OCH_3$ | N | |
| L-6 | 0 | CH=CH-CN | H | H | - | $OCH_3$ | $OCH_3$ | N | |
| L-6 | 0 | CH=CH-CN | H | H | - | Cl | $OCH_3$ | CH | |
| L-6 | 0 | $CH=CH-OCH_3$ | H | H | - | $CH_3$ | $CH_3$ | CH | |
| L-6 | 0 | $CH=CH-OCH_3$ | H | H | - | $CH_3$ | $OCH_3$ | CH | |
| L-6 | 0 | $CH=CH-OCH_3$ | H | H | - | $OCH_3$ | $OCH_3$ | CH | |
| L-6 | 0 | $CH=CH-OCH_3$ | H | H | - | $CH_3$ | $CH_3$ | N | |
| L-6 | 0 | $CH=CH-OCH_3$ | H | H | - | $CH_3$ | $OCH_3$ | N | |

Table VIII (Continued)

| L | n | W | R | $R_1$ | $R_2$ | X | Y | Z | m.p. (°C) |
|---|---|---|---|---|---|---|---|---|---|
| L-6 | 0 | $CH{=}CH{-}OCH_3$ | H | H | - | $OCH_3$ | $OCH_3$ | N | |
| L-6 | 0 | $CH{=}CH{-}OCH_3$ | H | H | - | Cl | $OCH_3$ | CH | |
| L-6 | 0 | CH=CH-Br | H | H | - | $CH_3$ | $CH_3$ | CH | |
| L-6 | 0 | CH=CH-Br | H | H | - | $CH_3$ | $OCH_3$ | CH | |
| L-6 | 0 | CH=CH-Br | H | H | - | $OCH_3$ | $OCH_3$ | CH | |
| L-6 | 0 | CH=CH-Br | H | H | - | $CH_3$ | $CH_3$ | N | |
| L-6 | 0 | CH=CH-Br | H | H | - | $CH_3$ | $OCH_3$ | N | |
| L-6 | 0 | CH=CH-Br | H | H | - | $OCH_3$ | $OCH_3$ | N | |
| L-6 | 0 | CH=CH-Br | H | H | - | Cl | $OCH_3$ | CH | |
| L-6 | 0 | $CH{=}CH{-}CF_3$ | H | H | - | $CH_3$ | $CH_3$ | CH | |
| L-6 | 0 | $CH{=}CH{-}CF_3$ | H | H | - | $CH_3$ | $OCH_3$ | CH | |
| L-6 | 0 | $CH{=}CH{-}CF_3$ | H | H | - | $OCH_3$ | $OCH_3$ | CH | |
| L-6 | 0 | $CH{=}CH{-}CF_3$ | H | H | - | $CH_3$ | $CH_3$ | N | |
| L-6 | 0 | $CH{=}CH{-}CF_3$ | H | H | - | $CH_3$ | $OCH_3$ | N | |
| L-6 | 0 | $CH{=}CH{-}CF_3$ | H | H | - | $OCH_3$ | $OCH_3$ | N | |
| L-6 | 0 | $CH{=}CH{-}CF_3$ | H | H | - | Cl | $OCH_3$ | CH | |
| L-6 | 0 | $C(CH_3){=}CH{-}OCH_3$ | H | H | - | $CH_3$ | $CH_3$ | CH | |
| L-6 | 0 | $C(CH_3){=}CH{-}OCH_3$ | H | H | - | $CH_3$ | $OCH_3$ | CH | |
| L-6 | 0 | $C(CH_3){=}CH{-}OCH_3$ | H | H | - | $OCH_3$ | $OCH_3$ | CH | |
| L-6 | 0 | $C(CH_3){=}CH{-}OCH_3$ | H | H | - | $CH_3$ | $CH_3$ | N | |
| L-6 | 0 | $C(CH_3){=}CH{-}OCH_3$ | H | H | - | $CH_3$ | $OCH_3$ | N | |
| L-6 | 0 | $C(CH_3){=}CH{-}OCH_3$ | H | H | - | $OCH_3$ | $OCH_3$ | N | |
| L-6 | 0 | $C(CH_3){=}CH{-}OCH_3$ | H | H | - | Cl | $OCH_3$ | CH | |
| L-6 | 0 | CH=CHPh | H | H | - | $CH_3$ | $CH_3$ | CH | |
| L-6 | 0 | CH=CHPh | H | H | - | $CH_3$ | $OCH_3$ | CH | |
| L-6 | 0 | CH=CHPh | H | H | - | $OCH_3$ | $OCH_3$ | CH | |
| L-6 | 0 | CH=CHPh | H | H | - | $CH_3$ | $CH_3$ | N | |
| L-6 | 0 | CH=CHPh | H | H | - | $CH_3$ | $OCH_3$ | N | |
| L-6 | 0 | CH=CHPh | H | H | - | $OCH_3$ | $OCH_3$ | N | |
| L-6 | 0 | CH=CHPh | H | H | - | Cl | $OCH_3$ | CH | |
| L-6 | 0 | $C(CH_3){=}CH{-}Ph$ | H | H | - | $CH_3$ | $CH_3$ | CH | |
| L-6 | 0 | $C(CH_3){=}CH{-}Ph$ | H | H | - | $CH_3$ | $OCH_3$ | CH | |
| L-6 | 0 | $C(CH_3){=}CH{-}Ph$ | H | H | - | $OCH_3$ | $OCH_3$ | CH | |

Table VIII (Continued)

| L | n | W | R | $R_1$ | $R_2$ | X | Y | Z | m.p. (°C) |
|---|---|---|---|---|---|---|---|---|---|
| L-6 | 0 | C($CH_3$)=CH-Ph | H | H | - | $CH_3$ | $CH_3$ | N | |
| L-6 | 0 | C($CH_3$)=CH-Ph | H | H | - | $CH_3$ | $OCH_3$ | N | |
| L-6 | 0 | C($CH_3$)=CH-Ph | H | H | - | $OCH_3$ | $OCH_3$ | N | |
| L-6 | 0 | C($CH_3$)=CH-Ph | H | H | - | Cl | $OCH_3$ | CH | |
| L-6 | 0 | C=CH-$(CH_2)_3$ | H | H | - | $CH_3$ | $CH_3$ | CH | |
| L-6 | 0 | C=CH-$(CH_2)_3$ | H | H | - | $CH_3$ | $OCH_3$ | CH | |
| L-6 | 0 | C=CH-$(CH_2)_3$ | H | H | - | $OCH_3$ | $OCH_3$ | CH | |
| L-6 | 0 | C=CH-$(CH_2)_3$ | H | H | - | $CH_3$ | $CH_3$ | N | |
| L-6 | 0 | C=CH-$(CH_2)_3$ | H | H | - | $CH_3$ | $OCH_3$ | N | |
| L-6 | 0 | C=CH-$(CH_2)_3$ | H | H | - | $OCH_3$ | $OCH_3$ | N | |
| L-6 | 0 | C=CH-$(CH_2)_3$ | H | H | - | Cl | $OCH_3$ | CH | |
| L-6 | 0 | C=CH-$(CH_2)_4$ | H | H | - | $CH_3$ | $CH_3$ | CH | |
| L-6 | 0 | C=CH-$(CH_2)_4$ | H | H | - | $CH_3$ | $OCH_3$ | CH | |
| L-6 | 0 | C=CH-$(CH_2)_4$ | H | H | - | $OCH_3$ | $OCH_3$ | CH | |
| L-6 | 0 | C=CH-$(CH_2)_4$ | H | H | - | $CH_3$ | $CH_3$ | N | |
| L-6 | 0 | C=CH-$(CH_2)_4$ | H | H | - | $CH_3$ | $OCH_3$ | N | |
| L-6 | 0 | C=CH-$(CH_2)_4$ | H | H | - | $OCH_3$ | $OCH_3$ | N | |
| L-6 | 0 | C=CH-$(CH_2)_4$ | H | H | - | Cl | $OCH_3$ | CH | |
| L-6 | 0 | C=CH-$(CH_2)_5$ | H | H | - | $CH_3$ | $CH_3$ | CH | |
| L-6 | 0 | C=CH-$(CH_2)_5$ | H | H | - | $CH_3$ | $OCH_3$ | CH | |
| L-6 | 0 | C=CH-$(CH_2)_5$ | H | H | - | $OCH_3$ | $OCH_3$ | CH | |
| L-6 | 0 | C=CH-$(CH_2)_5$ | H | H | - | $CH_3$ | $CH_3$ | N | |
| L-6 | 0 | C=CH-$(CH_2)_5$ | H | H | - | $CH_3$ | $OCH_3$ | N | |
| L-6 | 0 | C=CH-$(CH_2)_5$ | H | H | - | $OCH_3$ | $OCH_3$ | N | |
| L-6 | 0 | C=CH-$(CH_2)_5$ | H | H | - | Cl | $OCH_3$ | CH | |
| L-6 | 0 | C(CN)=CHCN | H | H | - | $CH_3$ | $CH_3$ | CH | |

Table VIII (Continued)

| L | n | W | R | $R_1$ | $R_2$ | X | Y | Z | m.p. (°C) |
|---|---|---|---|---|---|---|---|---|---|
| L-6 | 0 | C(CN)=CHCN | H | H | - | $CH_3$ | $OCH_3$ | CH | |
| L-6 | 0 | C(CN)=CHCN | H | H | - | $OCH_3$ | $OCH_3$ | CH | |
| L-6 | 0 | C(CN)=CHCN | H | H | - | $CH_3$ | $CH_3$ | N | |
| L-6 | 0 | C(CN)=CHCN | H | H | - | $CH_3$ | $OCH_3$ | N | |
| L-6 | 0 | C(CN)=CHCN | H | H | - | $OCH_3$ | $OCH_3$ | N | |
| L-6 | 0 | C(CN)=CHCN | H | H | - | Cl | $OCH_3$ | CH | |
| L-6 | 0 | $CH=CH-SO_2N(CH_3)_2$ | H | H | - | $CH_3$ | $CH_3$ | CH | |
| L-6 | 0 | $CH=CH-SO_2N(CH_3)_2$ | H | H | - | $CH_3$ | $OCH_3$ | CH | |
| L-6 | 0 | $CH=CH-SO_2N(CH_3)_2$ | H | H | - | $OCH_3$ | $OCH_3$ | CH | |
| L-6 | 0 | $CH=CH-SO_2N(CH_3)_2$ | H | H | - | $CH_3$ | $CH_3$ | N | |
| L-6 | 0 | $CH=CH-SO_2N(CH_3)_2$ | H | H | - | $CH_3$ | $OCH_3$ | N | |
| L-6 | 0 | $CH=CH-SO_2N(CH_3)_2$ | H | H | - | $OCH_3$ | $OCH_3$ | N | |
| L-6 | 0 | $CH=CH-SO_2N(CH_3)_2$ | H | H | - | Cl | $OCH_3$ | CH | |
| L-6 | 0 | $C(CH_3)=CH-CO_2CH_3$ | H | H | - | Cl | $OCH_3$ | CH | |
| L-6 | 0 | $\overline{C=CH-(CH_2)_4}$ | $CH_3$ | H | - | $OCH_3$ | $OCH_3$ | CH | |
| L-6 | 0 | $\overline{C=CH-(CH_2)_4}$ | H | $CH_3$ | - | $OCH_3$ | $OCH_3$ | CH | |
| L-6 | 0 | $C(=CH_2)-CH_2CH_2CH(NO_2)CH_3$ | H | H | - | $OCH_3$ | $OCH_3$ | CH | |
| L-6 | 0 | $CH=CHCH_2OSO_2Ph$ | H | H | - | $OCH_3$ | $OCH_3$ | CH | |
| L-6 | 0 | $CH=CHCH_2OSO_2CH_3$ | H | H | - | $OCH_3$ | $OCH_3$ | CH | |
| L-6 | 0 | $CH=CHSCH_3$ | H | H | - | $OCH_3$ | $OCH_3$ | CH | |
| L-6 | 0 | C≡CH | H | H | - | $CH_3$ | $CH_3$ | CH | |
| L-6 | 0 | C≡CH | H | H | - | $CH_3$ | $OCH_3$ | CH | |
| L-6 | 0 | C≡CH | H | H | - | $OCH_3$ | $OCH_3$ | CH | |
| L-6 | 0 | C≡CH | H | H | - | $CH_3$ | $CH_3$ | N | |
| L-6 | 0 | C≡CH | H | H | - | $CH_3$ | $OCH_3$ | N | |
| L-6 | 0 | C≡CH | H | H | - | $OCH_3$ | $OCH_3$ | N | |
| L-6 | 0 | C≡CH | H | H | - | Cl | $OCH_3$ | CH | |

Table VIII (Continued)

| L | n | W | R | $R_1$ | $R_2$ | X | Y | Z | m.p. (°C) |
|---|---|---|---|---|---|---|---|---|---|
| L-6 | 0 | C≡C-Ph | H | H | - | $CH_3$ | $CH_3$ | CH | |
| L-6 | 0 | C≡C-Ph | H | H | - | $CH_3$ | $OCH_3$ | CH | |
| L-6 | 0 | C≡C-Ph | H | H | - | $OCH_3$ | $OCH_3$ | CH | |
| L-6 | 0 | C≡C-Ph | H | H | - | $CH_3$ | $CH_3$ | N | |
| L-6 | 0 | C≡C-Ph | H | H | - | $CH_3$ | $OCH_3$ | N | |
| L-6 | 0 | C≡C-Ph | H | H | - | $OCH_3$ | $OCH_3$ | N | |
| L-6 | 0 | C≡C-Ph | H | H | - | Cl | $OCH_3$ | CH | |
| L-6 | 0 | $C≡C-CH_3$ | H | H | - | $CH_3$ | $CH_3$ | CH | |
| L-6 | 0 | $C≡C-CH_3$ | H | H | - | $CH_3$ | $OCH_3$ | CH | |
| L-6 | 0 | $C≡C-CH_3$ | H | H | - | $OCH_3$ | $OCH_3$ | CH | |
| L-6 | 0 | $C≡C-CH_3$ | H | H | - | $CH_3$ | $CH_3$ | N | |
| L-6 | 0 | $C≡C-CH_3$ | H | H | - | $CH_3$ | $OCH_3$ | N | |
| L-6 | 0 | $C≡C-CH_3$ | H | H | - | $OCH_3$ | $OCH_3$ | N | |
| L-6 | 0 | $C≡C-CH_3$ | H | H | - | Cl | $OCH_3$ | CH | |
| L-6 | 0 | $C≡CCH_2OC(O)CH_3$ | H | H | - | $CH_3$ | $CH_3$ | CH | |
| L-6 | 0 | $C≡CCH_2OC(O)CH_3$ | H | H | - | $CH_3$ | $OCH_3$ | CH | |
| L-6 | 0 | $C≡CCH_2OC(O)CH_3$ | H | H | - | $OCH_3$ | $OCH_3$ | CH | |
| L-6 | 0 | $C≡CCH_2OC(O)CH_3$ | H | H | - | $CH_3$ | $CH_3$ | N | |
| L-6 | 0 | $C≡CCH_2OC(O)CH_3$ | H | H | - | $CH_3$ | $OCH_3$ | N | |
| L-6 | 0 | $C≡CCH_2OC(O)CH_3$ | H | H | - | $OCH_3$ | $OCH_3$ | N | |
| L-6 | 0 | $C≡CCH_2OC(O)CH_3$ | H | H | - | Cl | $OCH_3$ | CH | |
| L-6 | 0 | $C≡CCH_2OH$ | H | H | - | $CH_3$ | $CH_3$ | CH | |
| L-6 | 0 | $C≡CCH_2OH$ | H | H | - | $CH_3$ | $OCH_3$ | CH | |
| L-6 | 0 | $C≡CCH_2OH$ | H | H | - | $OCH_3$ | $OCH_3$ | CH | |
| L-6 | 0 | $C≡CCH_2OH$ | H | H | - | $CH_3$ | $CH_3$ | N | |
| L-6 | 0 | $C≡CCH_2OH$ | H | H | - | $CH_3$ | $OCH_3$ | N | |
| L-6 | 0 | $C≡CCH_2OH$ | H | H | - | $OCH_3$ | $OCH_3$ | N | |
| L-6 | 0 | $C≡CCH_2OH$ | H | H | - | Cl | $OCH_3$ | CH | |
| L-6 | 0 | $C≡CCH_2Cl$ | H | H | - | $CH_3$ | $CH_3$ | CH | |
| L-6 | 0 | $C≡CCH_2Cl$ | H | H | - | $CH_3$ | $OCH_3$ | CH | |
| L-6 | 0 | $C≡CCH_2Cl$ | H | H | - | $OCH_3$ | $OCH_3$ | CH | |

Table VIII (Continued)

| L | n | W | R | $R_1$ | $R_2$ | X | Y | Z | m.p. (°C) |
|---|---|---|---|---|---|---|---|---|---|
| L-6 | 0 | $C\equiv CCH_2Cl$ | H | H | - | $CH_3$ | $CH_3$ | N | |
| L-6 | 0 | $C\equiv CCH_2Cl$ | H | H | - | $CH_3$ | $OCH_3$ | N | |
| L-6 | 0 | $C\equiv CCH_2Cl$ | H | H | - | $OCH_3$ | $OCH_3$ | N | |
| L-6 | 0 | $C\equiv CCH_2Cl$ | H | H | - | Cl | $OCH_3$ | CH | |
| L-6 | 0 | $C\equiv C-CH_2Br$ | H | H | - | $CH_3$ | $CH_3$ | CH | |
| L-6 | 0 | $C\equiv C-CH_2Br$ | H | H | - | $CH_3$ | $OCH_3$ | CH | |
| L-6 | 0 | $C\equiv C-CH_2Br$ | H | H | - | $OCH_3$ | $OCH_3$ | CH | |
| L-6 | 0 | $C\equiv C-CH_2Br$ | H | H | - | $CH_3$ | $CH_3$ | N | |
| L-6 | 0 | $C\equiv C-CH_2Br$ | H | H | - | $CH_3$ | $OCH_3$ | N | |
| L-6 | 0 | $C\equiv C-CH_2Br$ | H | H | - | $OCH_3$ | $OCH_3$ | N | |
| L-6 | 0 | $C\equiv C-CH_2Br$ | H | H | - | Cl | $OCH_3$ | CH | |
| L-6 | 0 | $C\equiv C-CH_2CN$ | H | H | - | $CH_3$ | $CH_3$ | CH | |
| L-6 | 0 | $C\equiv C-CH_2CN$ | H | H | - | $CH_3$ | $OCH_3$ | CH | |
| L-6 | 0 | $C\equiv C-CH_2CN$ | H | H | - | $OCH_3$ | $OCH_3$ | CH | |
| L-6 | 0 | $C\equiv C-CH_2CN$ | H | H | - | $CH_3$ | $CH_3$ | N | |
| L-6 | 0 | $C\equiv C-CH_2CN$ | H | H | - | $CH_3$ | $OCH_3$ | N | |
| L-6 | 0 | $C\equiv C-CH_2CN$ | H | H | - | $OCH_3$ | $OCH_3$ | N | |
| L-6 | 0 | $C\equiv C-CH_2CN$ | H | H | - | Cl | $OCH_3$ | CH | |
| L-6 | 0 | $C\equiv C-CH_2OCH_3$ | H | H | - | $CH_3$ | $CH_3$ | CH | |
| L-6 | 0 | $C\equiv C-CH_2OCH_3$ | H | H | - | $CH_3$ | $OCH_3$ | CH | |
| L-6 | 0 | $C\equiv C-CH_2OCH_3$ | H | H | - | $OCH_3$ | $OCH_3$ | CH | |
| L-6 | 0 | $C\equiv C-CH_2OCH_3$ | H | H | - | $CH_3$ | $CH_3$ | N | |
| L-6 | 0 | $C\equiv C-CH_2OCH_3$ | H | H | - | $CH_3$ | $OCH_3$ | N | |
| L-6 | 0 | $C\equiv C-CH_2OCH_3$ | H | H | - | $OCH_3$ | $OCH_3$ | N | |
| L-6 | 0 | $C\equiv C-CH_2OCH_3$ | H | H | - | Cl | $OCH_3$ | CH | |
| L-6 | 0 | $C\equiv CCH_2OSO_2CH_3$ | H | H | - | $CH_3$ | $CH_3$ | CH | |
| L-6 | 0 | $C\equiv CCH_2OSO_2CH_3$ | H | H | - | $CH_3$ | $OCH_3$ | CH | |
| L-6 | 0 | $C\equiv CCH_2OSO_2CH_3$ | H | H | - | $OCH_3$ | $OCH_3$ | CH | |
| L-6 | 0 | $C\equiv CCH_2OSO_2CH_3$ | H | H | - | $CH_3$ | $CH_3$ | N | |
| L-6 | 0 | $C\equiv CCH_2OSO_2CH_3$ | H | H | - | $CH_3$ | $OCH_3$ | N | |
| L-6 | 0 | $C\equiv CCH_2OSO_2CH_3$ | H | H | - | $OCH_3$ | $OCH_3$ | N | |

Table VIII (Continued)

| L | n | W | R | $R_1$ | $R_2$ | X | Y | Z | m.p. (°C) |
|---|---|---|---|---|---|---|---|---|---|
| L-6 | 0 | $C{\equiv}CCH_2OSO_2CH_3$ | H | H | - | Cl | $OCH_3$ | CH | |
| L-6 | 0 | $C{\equiv}CCH_2SCH_3$ | H | H | - | $CH_3$ | $CH_3$ | CH | |
| L-6 | 0 | $C{\equiv}CCH_2SCH_3$ | H | H | - | $CH_3$ | $OCH_3$ | CH | |
| L-6 | 0 | $C{\equiv}CCH_2SCH_3$ | H | H | - | $OCH_3$ | $OCH_3$ | CH | |
| L-6 | 0 | $C{\equiv}CCH_2SCH_3$ | H | H | - | $CH_3$ | $CH_3$ | N | |
| L-6 | 0 | $C{\equiv}CCH_2SCH_3$ | H | H | - | $CH_3$ | $OCH_3$ | N | |
| L-6 | 0 | $C{\equiv}CCH_2SCH_3$ | H | H | - | $OCH_3$ | $OCH_3$ | N | |
| L-6 | 0 | $C{\equiv}CCH_2SCH_3$ | H | H | - | Cl | $OCH_3$ | CH | |
| L-6 | 1 | $HC{=}CH_2$ | H | H | - | $CH_3$ | $CH_3$ | CH | |
| L-6 | 1 | $HC{=}CH_2$ | H | H | - | $CH_3$ | $OCH_3$ | CH | |
| L-6 | 1 | $HC{=}CH_2$ | H | H | - | $OCH_3$ | $OCH_3$ | CH | |
| L-6 | 1 | $HC{=}CH_2$ | H | H | - | $CH_3$ | $CH_3$ | N | |
| L-6 | 1 | $HC{=}CH_2$ | H | H | - | $CH_3$ | $OCH_3$ | N | |
| L-6 | 1 | $HC{=}CH_2$ | H | H | - | $OCH_3$ | $OCH_3$ | N | |
| L-6 | 1 | $HC{=}CH_2$ | H | H | - | Cl | $OCH_3$ | CH | |
| L-6 | 1 | $BrC{=}CH_2$ | H | H | - | $CH_3$ | $CH_3$ | CH | |
| L-6 | 1 | $BrC{=}CH_2$ | H | H | - | $CH_3$ | $OCH_3$ | CH | |
| L-6 | 1 | $BrC{=}CH_2$ | H | H | - | $OCH_3$ | $OCH_3$ | CH | |
| L-6 | 1 | $BrC{=}CH_2$ | H | H | - | $CH_3$ | $CH_3$ | N | |
| L-6 | 1 | $BrC{=}CH_2$ | H | H | - | $CH_3$ | $OCH_3$ | N | |
| L-6 | 1 | $BrC{=}CH_2$ | H | H | - | $OCH_3$ | $OCH_3$ | N | |
| L-6 | 1 | $BrC{=}CH_2$ | H | H | - | Cl | $OCH_3$ | CH | |
| L-6 | 1 | $HC{=}C(CH_3)_2$ | H | H | - | $CH_3$ | $CH_3$ | CH | |
| L-6 | 1 | $HC{=}C(CH_3)_2$ | H | H | - | $CH_3$ | $OCH_3$ | CH | |
| L-6 | 1 | $HC{=}C(CH_3)_2$ | H | H | - | $OCH_3$ | $OCH_3$ | CH | |
| L-6 | 1 | $HC{=}C(CH_3)_2$ | H | H | - | $CH_3$ | $CH_3$ | N | |
| L-6 | 1 | $HC{=}C(CH_3)_2$ | H | H | - | $CH_3$ | $OCH_3$ | N | |
| L-6 | 1 | $HC{=}C(CH_3)_2$ | H | H | - | $OCH_3$ | $OCH_3$ | N | |
| L-6 | 1 | $HC{=}C(CH_3)_2$ | H | H | - | Cl | $OCH_3$ | CH | |

Table VIII (Continued)

| L | n | W | R | $R_1$ | $R_2$ | X | Y | Z | m.p. (°C) |
|---|---|---|---|---|---|---|---|---|---|
| L-6 | 1 | $HC=C(CH_3)CH_2CH_2CH=C(CH_3)_2$ | H | H | - | $CH_3$ | $CH_3$ | CH | |
| L-6 | 1 | $HC=C(CH_3)CH_2CH_2CH=C(CH_3)_2$ | H | H | - | $CH_3$ | $OCH_3$ | CH | |
| L-6 | 1 | $HC=C(CH_3)CH_2CH_2CH=C(CH_3)_2$ | H | H | - | $OCH_3$ | $OCH_3$ | CH | |
| L-6 | 1 | $HC=C(CH_3)CH_2CH_2CH=C(CH_3)_2$ | H | H | - | $CH_3$ | $CH_3$ | N | |
| L-6 | 1 | $HC=C(CH_3)CH_2CH_2CH=C(CH_3)_2$ | H | H | - | $CH_3$ | $OCH_3$ | N | |
| L-6 | 1 | $HC=C(CH_3)CH_2CH_2CH=C(CH_3)_2$ | H | H | - | $OCH_3$ | $OCH_3$ | N | |
| L-6 | 1 | $HC=C(CH_3)CH_2CH_2CH=C(CH_3)_2$ | H | H | - | Cl | $OCH_3$ | CH | |
| L-6 | 1 | HC=CH-Ph | H | H | - | $CH_3$ | $CH_3$ | CH | |
| L-6 | 1 | HC=CH-Ph | H | H | - | $CH_3$ | $OCH_3$ | CH | |
| L-6 | 1 | HC=CH-Ph | H | H | - | $OCH_3$ | $OCH_3$ | CH | |
| L-6 | 1 | HC=CH-Ph | H | H | - | $CH_3$ | $CH_3$ | N | |
| L-6 | 1 | HC=CH-Ph | H | H | - | $CH_3$ | $OCH_3$ | N | |
| L-6 | 1 | HC=CH-Ph | H | H | - | $OCH_3$ | $OCH_3$ | N | |
| L-6 | 1 | HC=CH-Ph | H | H | - | Cl | $OCH_3$ | CH | |
| L-6 | 1 | $HC=CHCH_3$ | H | H | - | $CH_3$ | $CH_3$ | CH | |
| L-6 | 1 | $HC=CHCH_3$ | H | H | - | $CH_3$ | $OCH_3$ | CH | |
| L-6 | 1 | $HC=CHCH_3$ | H | H | - | $OCH_3$ | $OCH_3$ | CH | |
| L-6 | 1 | $HC=CHCH_3$ | H | H | - | $CH_3$ | $CH_3$ | N | |
| L-6 | 1 | $HC=CHCH_3$ | H | H | - | $CH_3$ | $OCH_3$ | N | |
| L-6 | 1 | $HC=CHCH_3$ | H | H | - | $OCH_3$ | $OCH_3$ | N | |
| L-6 | 1 | $HC=CHCH_3$ | H | H | - | Cl | $OCH_3$ | CH | |
| L-6 | 1 | $HC=CHCH_2Br$ | H | H | - | $CH_3$ | $CH_3$ | CH | |
| L-6 | 1 | $HC=CHCH_2Br$ | H | H | - | $CH_3$ | $OCH_3$ | CH | |
| L-6 | 1 | $HC=CHCH_2Br$ | H | H | - | $OCH_3$ | $OCH_3$ | CH | |
| L-6 | 1 | $HC=CHCH_2Br$ | H | H | - | $CH_3$ | $CH_3$ | N | |
| L-6 | 1 | $HC=CHCH_2Br$ | H | H | - | $CH_3$ | $OCH_3$ | N | |
| L-6 | 1 | $HC=CHCH_2Br$ | H | H | - | $OCH_3$ | $OCH_3$ | N | |
| L-6 | 1 | $HC=CHCH_2Br$ | H | H | - | Cl | $OCH_3$ | CH | |
| L-6 | 1 | C≡CH | H | H | - | $CH_3$ | $CH_3$ | CH | |
| L-6 | 1 | C≡CH | H | H | - | $CH_3$ | $OCH_3$ | CH | |
| L-6 | 1 | C≡CH | H | H | - | $OCH_3$ | $OCH_3$ | CH | |

Table VIII (Continued)

| L | n | W | R | $R_1$ | $R_2$ | X | Y | Z | m.p. (°C) |
|---|---|---|---|---|---|---|---|---|---|
| L-6 | 1 | C≡CH | H | H | - | $CH_3$ | $CH_3$ | N | |
| L-6 | 1 | C≡CH | H | H | - | $CH_3$ | $OCH_3$ | N | |
| L-6 | 1 | C≡CH | H | H | - | $OCH_3$ | $OCH_3$ | N | |
| L-6 | 1 | C≡CH | H | H | - | Cl | $OCH_3$ | CH | |
| L-6 | 1 | $C≡CCH_3$ | H | H | - | $CH_3$ | $CH_3$ | CH | |
| L-6 | 1 | $C≡CCH_3$ | H | H | - | $CH_3$ | $OCH_3$ | CH | |
| L-6 | 1 | $C≡CCH_3$ | H | H | - | $OCH_3$ | $OCH_3$ | CH | |
| L-6 | 1 | $C≡CCH_3$ | H | H | - | $CH_3$ | $CH_3$ | N | |
| L-6 | 1 | $C≡CCH_3$ | H | H | - | $CH_3$ | $OCH_3$ | N | |
| L-6 | 1 | $C≡CCH_3$ | H | H | - | $OCH_3$ | $OCH_3$ | N | |
| L-6 | 1 | $C≡CCH_3$ | H | H | - | Cl | $OCH_3$ | CH | |
| L-6 | 1 | $C≡C-CH_2Br$ | H | H | - | $CH_3$ | $CH_3$ | CH | |
| L-6 | 1 | $C≡C-CH_2Br$ | H | H | - | $CH_3$ | $OCH_3$ | CH | |
| L-6 | 1 | $C≡C-CH_2Br$ | H | H | - | $OCH_3$ | $OCH_3$ | CH | |
| L-6 | 1 | $C≡C-CH_2Br$ | H | H | - | $CH_3$ | $CH_3$ | N | |
| L-6 | 1 | $C≡C-CH_2Br$ | H | H | - | $CH_3$ | $OCH_3$ | N | |
| L-6 | 1 | $C≡C-CH_2Br$ | H | H | - | $OCH_3$ | $OCH_3$ | N | |
| L-6 | 1 | $C≡C-CH_2Br$ | H | H | - | Cl | $OCH_3$ | CH | |
| L-6 | 1 | C≡C-Ph | H | H | - | $CH_3$ | $CH_3$ | CH | |
| L-6 | 1 | C≡C-Ph | H | H | - | $CH_3$ | $OCH_3$ | CH | |
| L-6 | 1 | C≡C-Ph | H | H | - | $OCH_3$ | $OCH_3$ | CH | |
| L-6 | 1 | C≡C-Ph | H | H | - | $CH_3$ | $CH_3$ | N | |
| L-6 | 1 | C≡C-Ph | H | H | - | $CH_3$ | $OCH_3$ | N | |
| L-6 | 1 | C≡C-Ph | H | H | - | $OCH_3$ | $OCH_3$ | N | |
| L-6 | 1 | C≡C-Ph | H | H | - | Cl | $OCH_3$ | CH | |
| L-6 | 1 | $CH=CH_2$ | $CH_3$ | H | - | $CH_3$ | $CH_3$ | CH | |
| L-6 | 1 | $CH=CH_2$ | $CH_3$ | H | - | $CH_3$ | $OCH_3$ | CH | |
| L-6 | 1 | $CH=CH_2$ | $CH_3$ | H | - | $OCH_3$ | $OCH_3$ | CH | |
| L-6 | 1 | $CH=CH_2$ | $CH_3$ | H | - | $CH_3$ | $CH_3$ | N | |
| L-6 | 1 | $CH=CH_2$ | $CH_3$ | H | - | $CH_3$ | $OCH_3$ | N | |
| L-6 | 1 | $CH=CH_2$ | $CH_3$ | H | - | $OCH_3$ | $OCH_3$ | N | |

Table VIII (Continued)

| L | n | W | R | $R_1$ | $R_2$ | X | Y | Z | m.p.(°C) |
|---|---|---|---|---|---|---|---|---|---|
| L-6 | 1 | $CH=CH_2$ | $CH_3$ | H | - | Cl | $OCH_3$ | CH | |
| L-6 | 1 | C≡CH | $CH_3$ | H | - | $CH_3$ | $CH_3$ | CH | |
| L-6 | 1 | C≡CH | $CH_3$ | H | - | $CH_3$ | $OCH_3$ | CH | |
| L-6 | 1 | C≡CH | $CH_3$ | H | - | $OCH_3$ | $OCH_3$ | CH | |
| L-6 | 1 | C≡CH | $CH_3$ | H | - | $CH_3$ | $CH_3$ | N | |
| L-6 | 1 | C≡CH | $CH_3$ | H | - | $CH_3$ | $OCH_3$ | N | |
| L-6 | 1 | C≡CH | $CH_3$ | H | - | $OCH_3$ | $OCH_3$ | N | |
| L-6 | 1 | C≡CH | $CH_3$ | H | - | Cl | $OCH_3$ | CH | |
| L-6 | 1 | $CH=CH_2$ | H | $CH_3$ | - | $CH_3$ | $CH_3$ | CH | |
| L-6 | 1 | $CH=CH_2$ | H | $CH_3$ | - | $CH_3$ | $OCH_3$ | CH | |
| L-6 | 1 | $CH=CH_2$ | H | $CH_3$ | - | $OCH_3$ | $OCH_3$ | CH | |
| L-6 | 1 | $CH=CH_2$ | H | $CH_3$ | - | $CH_3$ | $CH_3$ | N | |
| L-6 | 1 | $CH=CH_2$ | H | $CH_3$ | - | $CH_3$ | $OCH_3$ | N | |
| L-6 | 1 | $CH=CH_2$ | H | $CH_3$ | - | $OCH_3$ | $OCH_3$ | N | |
| L-6 | 1 | $CH=CH_2$ | H | $CH_3$ | - | Cl | $OCH_3$ | CH | |
| L-6 | 1 | C≡CH | H | $CH_3$ | - | $OCH_3$ | $OCH_3$ | CH | |
| L-6 | 1 | C≡CH | H | $CH_3$ | - | $OCH_3$ | $OCH_3$ | N | |
| L-6 | 1 | C≡CH | H | $CH_3$ | - | $OCH_3$ | $CH_3$ | CH | |
| L-2 | 0 | $CH=C(CN)_2$ | H | H | - | $CH_3$ | $CH_3$ | CH | |
| L-2 | 0 | $CH=C(CN)_2$ | H | H | - | $CH_3$ | $OCH_3$ | CH | |
| L-2 | 0 | $CH=C(CN)_2$ | H | H | - | $OCH_3$ | $OCH_3$ | CH | |
| L-2 | 0 | $CH=C(CN)_2$ | H | H | - | $CH_3$ | $OCH_3$ | N | |
| L-2 | 0 | $CH=C(CN)_2$ | H | H | - | $OCH_3$ | $OCH_3$ | N | |
| L-2 | 0 | $CH=C(CN)_2$ | H | H | - | Cl | $OCH_3$ | CH | |

Table VIIIa

General Structure VIIIa

| L | n | W | R | $R_1$ | $R_2$ | X | Y | Z | m.p. (°C) |
|---|---|---|---|---|---|---|---|---|---|
| L-2 | 0 | $CH=CH_2$ | H | H | - | $CH_3$ | $CH_3$ | CH | |
| L-2 | 0 | $CH=CH_2$ | H | H | - | $CH_3$ | $OCH_3$ | CH | |
| L-2 | 0 | $CH=CH_2$ | H | H | - | $OCH_3$ | $OCH_3$ | CH | |
| L-2 | 0 | $CH=CH_2$ | H | H | - | $CH_3$ | $CH_3$ | N | |
| L-2 | 0 | $CH=CH_2$ | H | H | - | $CH_3$ | $OCH_3$ | N | |
| L-2 | 0 | $CH=CH_2$ | H | H | - | $OCH_3$ | $OCH_3$ | N | |
| L-2 | 0 | $CH=CH_2$ | H | H | - | Cl | $OCH_3$ | CH | |
| L-2 | 0 | $C(CH_3)=CHCH_3$ | H | H | - | $CH_3$ | $CH_3$ | CH | |
| L-2 | 0 | $C(CH_3)=CHCH_3$ | H | H | - | $CH_3$ | $OCH_3$ | CH | |
| L-2 | 0 | $C(CH_3)=CHCH_3$ | H | H | - | $OCH_3$ | $OCH_3$ | CH | |
| L-2 | 0 | $C(CH_3)=CHCH_3$ | H | H | - | $CH_3$ | $CH_3$ | N | |
| L-2 | 0 | $C(CH_3)=CHCH_3$ | H | H | - | $CH_3$ | $OCH_3$ | N | |
| L-2 | 0 | $C(CH_3)=CHCH_3$ | H | H | - | $OCH_3$ | $OCH_3$ | N | |
| L-2 | 0 | $C(CH_3)=CHCH_3$ | H | H | - | Cl | $OCH_3$ | CH | |
| L-2 | 0 | $CH=CHCH_2CH_3$ | H | H | - | $CH_3$ | $CH_3$ | CH | |
| L-2 | 0 | $CH=CHCH_2CH_3$ | H | H | - | $CH_3$ | $OCH_3$ | CH | |
| L-2 | 0 | $CH=CHCH_2CH_3$ | H | H | - | $OCH_3$ | $OCH_3$ | CH | |
| L-2 | 0 | $CH=CHCH_2CH_3$ | H | H | - | $CH_3$ | $CH_3$ | N | |
| L-2 | 0 | $CH=CHCH_2CH_3$ | H | H | - | $CH_3$ | $OCH_3$ | N | |
| L-2 | 0 | $CH=CHCH_2CH_3$ | H | H | - | $OCH_3$ | $OCH_3$ | N | |
| L-2 | 0 | $CH=CHCH_2CH_3$ | H | H | - | Cl | $OCH_3$ | CH | |
| L-2 | 0 | $C(CH_3)=CHCH_2CH_3$ | H | H | - | $CH_3$ | $CH_3$ | CH | |
| L-2 | 0 | $C(CH_3)=CHCH_2CH_3$ | H | H | - | $CH_3$ | $OCH_3$ | CH | |
| L-2 | 0 | $C(CH_3)=CHCH_2CH_3$ | H | H | - | $OCH_3$ | $OCH_3$ | CH | |
| L-2 | 0 | $C(CH_3)=CHCH_2CH_3$ | H | H | - | $CH_3$ | $CH_3$ | N | |
| L-2 | 0 | $C(CH_3)=CHCH_2CH_3$ | H | H | - | $CH_3$ | $OCH_3$ | N | |
| L-2 | 0 | $C(CH_3)=CHCH_2CH_3$ | H | H | - | $OCH_3$ | $OCH_3$ | N | |
| L-2 | 0 | $C(CH_3)=CHCH_2CH_3$ | H | H | - | Cl | $OCH_3$ | CH | |
| L-2 | 0 | $CH=CHCH_2CH_2CH_3$ | H | H | - | $CH_3$ | $CH_3$ | CH | |
| L-2 | 0 | $CH=CHCH_2CH_2CH_3$ | H | H | - | $CH_3$ | $OCH_3$ | CH | |

Table VIIIa (Continued)

| L | n | W | R | $R_1$ | $R_2$ | X | Y | Z | m.p. (°C) |
|---|---|---|---|---|---|---|---|---|---|
| L-2 | 0 | $CH=CHCH_2CH_2CH_3$ | H | H | - | $OCH_3$ | $OCH_3$ | CH | |
| L-2 | 0 | $CH=CHCH_2CH_2CH_3$ | H | H | - | $CH_3$ | $CH_3$ | N | |
| L-2 | 0 | $CH=CHCH_2CH_2CH_3$ | H | H | - | $CH_3$ | $OCH_3$ | N | |
| L-2 | 0 | $CH=CHCH_2CH_2CH_3$ | H | H | - | $OCH_3$ | $OCH_3$ | N | |
| L-2 | 0 | $CH=CHCH_2CH_2CH_3$ | H | H | - | Cl | $OCH_3$ | CH | |
| L-2 | 0 | CH=CH-CN | H | H | - | $CH_3$ | $CH_3$ | CH | |
| L-2 | 0 | CH=CH-CN | H | H | - | $CH_3$ | $OCH_3$ | CH | |
| L-2 | 0 | CH=CH-CN | H | H | - | $OCH_3$ | $OCH_3$ | CH | |
| L-2 | 0 | CH=CH-CN | H | H | - | $CH_3$ | $CH_3$ | N | |
| L-2 | 0 | CH=CH-CN | H | H | - | $CH_3$ | $OCH_3$ | N | |
| L-2 | 0 | CH=CH-CN | H | H | - | $OCH_3$ | $OCH_3$ | N | |
| L-2 | 0 | CH=CH-CN | H | H | - | Cl | $OCH_3$ | CH | |
| L-2 | 0 | $CH=CH-OCH_3$ | H | H | - | $CH_3$ | $CH_3$ | CH | |
| L-2 | 0 | $CH=CH-OCH_3$ | H | H | - | $CH_3$ | $OCH_3$ | CH | |
| L-2 | 0 | $CH=CH-OCH_3$ | H | H | - | $OCH_3$ | $OCH_3$ | CH | |
| L-2 | 0 | $CH=CH-OCH_3$ | H | H | - | $CH_3$ | $CH_3$ | N | |
| L-2 | 0 | $CH=CH-OCH_3$ | H | H | - | $CH_3$ | $OCH_3$ | N | |
| L-2 | 0 | $CH=CH-OCH_3$ | H | H | - | $OCH_3$ | $OCH_3$ | N | |
| L-2 | 0 | $CH=CH-OCH_3$ | H | H | - | Cl | $OCH_3$ | CH | |
| L-2 | 0 | CH=CH-Br | H | H | - | $CH_3$ | $CH_3$ | CH | |
| L-2 | 0 | CH=CH-Br | H | H | - | $CH_3$ | $OCH_3$ | CH | |
| L-2 | 0 | CH=CH-Br | H | H | - | $OCH_3$ | $OCH_3$ | CH | |
| L-2 | 0 | CH=CH-Br | H | H | - | $CH_3$ | $CH_3$ | N | |
| L-2 | 0 | CH=CH-Br | H | H | - | $CH_3$ | $OCH_3$ | N | |
| L-2 | 0 | CH=CH-Br | H | H | - | $OCH_3$ | $OCH_3$ | N | |
| L-2 | 0 | CH=CH-Br | H | H | - | Cl | $OCH_3$ | CH | |
| L-2 | 0 | $CH=CH-CF_3$ | H | H | - | $CH_3$ | $CH_3$ | CH | |
| L-2 | 0 | $CH=CH-CF_3$ | H | H | - | $CH_3$ | $OCH_3$ | CH | |
| L-2 | 0 | $CH=CH-CF_3$ | H | H | - | $OCH_3$ | $OCH_3$ | CH | |
| L-2 | 0 | $CH=CH-CF_3$ | H | H | - | $CH_3$ | $CH_3$ | N | |
| L-2 | 0 | $CH=CH-CF_3$ | H | H | - | $CH_3$ | $OCH_3$ | N | |

Table VIIIa (Continued)

| L | n | W | R | $R_1$ | $R_2$ | X | Y | Z | m.p. (°C) |
|---|---|---|---|---|---|---|---|---|---|
| L-2 | 0 | $CH=CH-CF_3$ | H | H | - | $OCH_3$ | $OCH_3$ | N | |
| L-2 | 0 | $CH=CH-CF_3$ | H | H | - | Cl | $OCH_3$ | CH | |
| L-2 | 0 | $C(CH_3)=CH-OCH_3$ | H | H | - | $CH_3$ | $CH_3$ | CH | |
| L-2 | 0 | $C(CH_3)=CH-OCH_3$ | H | H | - | $CH_3$ | $OCH_3$ | CH | |
| L-2 | 0 | $C(CH_3)=CH-OCH_3$ | H | H | - | $OCH_3$ | $OCH_3$ | CH | |
| L-2 | 0 | $C(CH_3)=CH-OCH_3$ | H | H | - | $CH_3$ | $CH_3$ | N | |
| L-2 | 0 | $C(CH_3)=CH-OCH_3$ | H | H | - | $CH_3$ | $OCH_3$ | N | |
| L-2 | 0 | $C(CH_3)=CH-OCH_3$ | H | H | - | $OCH_3$ | $OCH_3$ | N | |
| L-2 | 0 | $C(CH_3)=CH-OCH_3$ | H | H | - | Cl | $OCH_3$ | CH | |
| L-2 | 0 | CH=CHPh | H | H | - | $CH_3$ | $CH_3$ | CH | |
| L-2 | 0 | CH=CHPh | H | H | - | $CH_3$ | $OCH_3$ | CH | |
| L-2 | 0 | CH=CHPh | H | H | - | $OCH_3$ | $OCH_3$ | CH | |
| L-2 | 0 | CH=CHPh | H | H | - | $CH_3$ | $CH_3$ | N | |
| L-2 | 0 | CH=CHPh | H | H | - | $CH_3$ | $OCH_3$ | N | |
| L-2 | 0 | CH=CHPh | H | H | - | $OCH_3$ | $OCH_3$ | N | |
| L-2 | 0 | CH=CHPh | H | H | - | Cl | $OCH_3$ | CH | |
| L-2 | 0 | $C(CH_3)=CH-Ph$ | H | H | - | $CH_3$ | $CH_3$ | CH | |
| L-2 | 0 | $C(CH_3)=CH-Ph$ | H | H | - | $CH_3$ | $OCH_3$ | CH | |
| L-2 | 0 | $C(CH_3)=CH-Ph$ | H | H | - | $OCH_3$ | $OCH_3$ | CH | |
| L-2 | 0 | $C(CH_3)=CH-Ph$ | H | H | - | $CH_3$ | $CH_3$ | N | |
| L-2 | 0 | $C(CH_3)=CH-Ph$ | H | H | - | $CH_3$ | $OCH_3$ | N | |
| L-2 | 0 | $C(CH_3)=CH-Ph$ | H | H | - | $OCH_3$ | $OCH_3$ | N | |
| L-2 | 0 | $C(CH_3)=CH-Ph$ | H | H | - | Cl | $OCH_3$ | CH | |
| L-2 | 0 | $\overline{C=CH-(CH_2)_3}$ | H | H | - | $CH_3$ | $CH_3$ | CH | |
| L-2 | 0 | $\overline{C=CH-(CH_2)_3}$ | H | H | - | $CH_3$ | $OCH_3$ | CH | |
| L-2 | 0 | $\overline{C=CH-(CH_2)_3}$ | H | H | - | $OCH_3$ | $OCH_3$ | CH | |
| L-2 | 0 | $\overline{C=CH-(CH_2)_3}$ | H | H | - | $CH_3$ | $CH_3$ | N | |
| L-2 | 0 | $\overline{C=CH-(CH_2)_3}$ | H | H | - | $CH_3$ | $OCH_3$ | N | |
| L-2 | 0 | $\overline{C=CH-(CH_2)_3}$ | H | H | - | $OCH_3$ | $OCH_3$ | N | |
| L-2 | 0 | $\overline{C=CH-(CH_2)_3}$ | H | H | - | Cl | $OCH_3$ | CH | |

Table VIIIa (Continued)

| L | n | W | R | $R_1$ | $R_2$ | X | Y | Z | m.p. (°C) |
|---|---|---|---|---|---|---|---|---|---|
| L-2 | 0 | $C=CH-(CH_2)_4$ | H | H | - | $CH_3$ | $CH_3$ | CH | |
| L-2 | 0 | $C=CH-(CH_2)_4$ | H | H | - | $CH_3$ | $OCH_3$ | CH | |
| L-2 | 0 | $C=CH-(CH_2)_4$ | H | H | - | $OCH_3$ | $OCH_3$ | CH | |
| L-2 | 0 | $C=CH-(CH_2)_4$ | H | H | - | $CH_3$ | $CH_3$ | N | |
| L-2 | 0 | $C=CH-(CH_2)_4$ | H | H | - | $CH_3$ | $OCH_3$ | N | |
| L-2 | 0 | $C=CH-(CH_2)_4$ | H | H | - | $OCH_3$ | $OCH_3$ | N | |
| L-2 | 0 | $C=CH-(CH_2)_4$ | H | H | - | Cl | $OCH_3$ | CH | |
| L-2 | 0 | $C=CH-(CH_2)_5$ | H | H | - | $CH_3$ | $CH_3$ | CH | |
| L-2 | 0 | $C=CH-(CH_2)_5$ | H | H | - | $CH_3$ | $OCH_3$ | CH | |
| L-2 | 0 | $C=CH-(CH_2)_5$ | H | H | - | $OCH_3$ | $OCH_3$ | CH | |
| L-2 | 0 | $C=CH-(CH_2)_5$ | H | H | - | $CH_3$ | $CH_3$ | N | |
| L-2 | 0 | $C=CH-(CH_2)_5$ | H | H | - | $CH_3$ | $OCH_3$ | N | |
| L-2 | 0 | $C=CH-(CH_2)_5$ | H | H | - | $OCH_3$ | $OCH_3$ | N | |
| L-2 | 0 | $C=CH-(CH_2)_5$ | H | H | - | Cl | $OCH_3$ | CH | |
| L-2 | 0 | C(CN)=CHCN | H | H | - | $CH_3$ | $CH_3$ | CH | |
| L-2 | 0 | C(CN)=CHCN | H | H | - | $CH_3$ | $OCH_3$ | CH | |
| L-2 | 0 | C(CN)=CHCN | H | H | - | $OCH_3$ | $OCH_3$ | CH | |
| L-2 | 0 | C(CN)=CHCN | H | H | - | $CH_3$ | $CH_3$ | N | |
| L-2 | 0 | C(CN)=CHCN | H | H | - | $CH_3$ | $OCH_3$ | N | |
| L-2 | 0 | C(CN)=CHCN | H | H | - | $OCH_3$ | $OCH_3$ | N | |
| L-2 | 0 | C(CN)=CHCN | H | H | - | Cl | $OCH_3$ | CH | |
| L-2 | 0 | $CH=CH-SO_2N(CH_3)_2$ | H | H | - | $CH_3$ | $CH_3$ | CH | |
| L-2 | 0 | $CH=CH-SO_2N(CH_3)_2$ | H | H | - | $CH_3$ | $OCH_3$ | CH | |
| L-2 | 0 | $CH=CH-SO_2N(CH_3)_2$ | H | H | - | $OCH_3$ | $OCH_3$ | CH | |
| L-2 | 0 | $CH=CH-SO_2N(CH_3)_2$ | H | H | - | $CH_3$ | $CH_3$ | N | |
| L-2 | 0 | $CH=CH-SO_2N(CH_3)_2$ | H | H | - | $CH_3$ | $OCH_3$ | N | |
| L-2 | 0 | $CH=CH-SO_2N(CH_3)_2$ | H | H | - | $OCH_3$ | $OCH_3$ | N | |
| L-2 | 0 | $CH=CH-SO_2N(CH_3)_2$ | H | H | - | Cl | $OCH_3$ | CH | |

Table VIIIa (Continued)

| L | n | W | R | $R_1$ | $R_2$ | X | Y | Z | m.p. (°C) |
|---|---|---|---|---|---|---|---|---|---|
| L-2 | 0 | $\overline{C=CH-(CH_2)_4}$ | $CH_3$ | H | - | $OCH_3$ | $OCH_3$ | CH | |
| L-2 | 0 | $\overline{C=CH-(CH_2)_4}$ | H | $CH_3$ | - | $OCH_3$ | $OCH_3$ | CH | |
| L-2 | 0 | $C(=CH_2)-CH_2CH_2CH(NO_2)CH_3$ | H | H | - | $OCH_3$ | $OCH_3$ | CH | |
| L-2 | 0 | $CH=CHCH_2OSO_2Ph$ | H | H | - | $OCH_3$ | $OCH_3$ | CH | |
| L-2 | 0 | $CH=CHCH_2OSO_2CH_3$ | H | H | - | $OCH_3$ | $OCH_3$ | CH | |
| L-2 | 0 | $CH=CHSCH_3$ | H | H | - | $OCH_3$ | $OCH_3$ | CH | |
| L-2 | 0 | C≡CH | H | H | - | $CH_3$ | $CH_3$ | CH | |
| L-2 | 0 | C≡CH | H | H | - | $CH_3$ | $OCH_3$ | CH | |
| L-2 | 0 | C≡CH | H | H | - | $OCH_3$ | $OCH_3$ | CH | |
| L-2 | 0 | C≡CH | H | H | - | $CH_3$ | $CH_3$ | N | |
| L-2 | 0 | C≡CH | H | H | - | $CH_3$ | $OCH_3$ | N | |
| L-2 | 0 | C≡CH | H | H | - | $OCH_3$ | $OCH_3$ | N | |
| L-2 | 0 | C≡CH | H | H | - | Cl | $OCH_3$ | CH | |
| L-2 | 0 | C≡C-Ph | H | H | - | $CH_3$ | $CH_3$ | CH | |
| L-2 | 0 | C≡C-Ph | H | H | - | $CH_3$ | $OCH_3$ | CH | |
| L-2 | 0 | C≡C-Ph | H | H | - | $OCH_3$ | $OCH_3$ | CH | |
| L-2 | 0 | C≡C-Ph | H | H | - | $CH_3$ | $CH_3$ | N | |
| L-2 | 0 | C≡C-Ph | H | H | - | $CH_3$ | $OCH_3$ | N | |
| L-2 | 0 | C≡C-Ph | H | H | - | $OCH_3$ | $OCH_3$ | N | |
| L-2 | 0 | C≡C-Ph | H | H | - | Cl | $OCH_3$ | CH | |
| L-2 | 0 | $C≡C-CH_3$ | H | H | - | $CH_3$ | $CH_3$ | CH | |
| L-2 | 0 | $C≡C-CH_3$ | H | H | - | $CH_3$ | $OCH_3$ | CH | |
| L-2 | 0 | $C≡C-CH_3$ | H | H | - | $OCH_3$ | $OCH_3$ | CH | |
| L-2 | 0 | $C≡C-CH_3$ | H | H | - | $CH_3$ | $CH_3$ | N | |
| L-2 | 0 | $C≡C-CH_3$ | H | H | - | $CH_3$ | $OCH_3$ | N | |
| L-2 | 0 | $C≡C-CH_3$ | H | H | - | $OCH_3$ | $OCH_3$ | N | |

Table VIIIa (Continued)

| L | n | W | R | $R_1$ | $R_2$ | X | Y | Z | m.p. (°C) |
|---|---|---|---|---|---|---|---|---|---|
| L-2 | 0 | $C{\equiv}C-CH_3$ | H | H | - | Cl | $OCH_3$ | CH | |
| L-2 | 0 | $C{\equiv}CCH_2OC(O)CH_3$ | H | H | - | $CH_3$ | $CH_3$ | CH | |
| L-2 | 0 | $C{\equiv}CCH_2OC(O)CH_3$ | H | H | - | $CH_3$ | $OCH_3$ | CH | |
| L-2 | 0 | $C{\equiv}CCH_2OC(O)CH_3$ | H | H | - | $OCH_3$ | $OCH_3$ | CH | |
| L-2 | 0 | $C{\equiv}CCH_2OC(O)CH_3$ | H | H | - | $CH_3$ | $CH_3$ | N | |
| L-2 | 0 | $C{\equiv}CCH_2OC(O)CH_3$ | H | H | - | $CH_3$ | $OCH_3$ | N | |
| L-2 | 0 | $C{\equiv}CCH_2OC(O)CH_3$ | H | H | - | $OCH_3$ | $OCH_3$ | N | |
| L-2 | 0 | $C{\equiv}CCH_2OC(O)CH_3$ | H | H | - | Cl | $OCH_3$ | CH | |
| L-2 | 0 | $C{\equiv}CCH_2OH$ | H | H | - | $CH_3$ | $CH_3$ | CH | |
| L-2 | 0 | $C{\equiv}CCH_2OH$ | H | H | - | $CH_3$ | $OCH_3$ | CH | |
| L-2 | 0 | $C{\equiv}CCH_2OH$ | H | H | - | $OCH_3$ | $OCH_3$ | CH | |
| L-2 | 0 | $C{\equiv}CCH_2OH$ | H | H | - | $CH_3$ | $CH_3$ | N | |
| L-2 | 0 | $C{\equiv}CCH_2OH$ | H | H | - | $CH_3$ | $OCH_3$ | N | |
| L-2 | 0 | $C{\equiv}CCH_2OH$ | H | H | - | $OCH_3$ | $OCH_3$ | N | |
| L-2 | 0 | $C{\equiv}CCH_2OH$ | H | H | - | Cl | $OCH_3$ | CH | |
| L-2 | 0 | $C{\equiv}CCH_2Cl$ | H | H | - | $CH_3$ | $CH_3$ | CH | |
| L-2 | 0 | $C{\equiv}CCH_2Cl$ | H | H | - | $CH_3$ | $OCH_3$ | CH | |
| L-2 | 0 | $C{\equiv}CCH_2Cl$ | H | H | - | $OCH_3$ | $OCH_3$ | CH | |
| L-2 | 0 | $C{\equiv}CCH_2Cl$ | H | H | - | $CH_3$ | $CH_3$ | N | |
| L-2 | 0 | $C{\equiv}CCH_2Cl$ | H | H | - | $CH_3$ | $OCH_3$ | N | |
| L-2 | 0 | $C{\equiv}CCH_2Cl$ | H | H | - | $OCH_3$ | $OCH_3$ | N | |
| L-2 | 0 | $C{\equiv}CCH_2Cl$ | H | H | - | Cl | $OCH_3$ | CH | |
| L-2 | 0 | $C{\equiv}C-CH_2Br$ | H | H | - | $CH_3$ | $CH_3$ | CH | |
| L-2 | 0 | $C{\equiv}C-CH_2Br$ | H | H | - | $CH_3$ | $OCH_3$ | CH | |
| L-2 | 0 | $C{\equiv}C-CH_2Br$ | H | H | - | $OCH_3$ | $OCH_3$ | CH | |
| L-2 | 0 | $C{\equiv}C-CH_2Br$ | H | H | - | $CH_3$ | $CH_3$ | N | |
| L-2 | 0 | $C{\equiv}C-CH_2Br$ | H | H | - | $CH_3$ | $OCH_3$ | N | |
| L-2 | 0 | $C{\equiv}C-CH_2Br$ | H | H | - | $OCH_3$ | $OCH_3$ | N | |
| L-2 | 0 | $C{\equiv}C-CH_2Br$ | H | H | - | Cl | $OCH_3$ | CH | |
| L-2 | 0 | $C{\equiv}C-CH_2CN$ | H | H | - | $CH_3$ | $CH_3$ | CH | |
| L-2 | 0 | $C{\equiv}C-CH_2CN$ | H | H | - | $CH_3$ | $OCH_3$ | CH | |

Table VIIIa (Continued)

| L | n | W | R | $R_1$ | $R_2$ | X | Y | Z | m.p. (°C) |
|---|---|---|---|---|---|---|---|---|---|
| L-2 | 0 | $C\equiv C-CH_2CN$ | H | H | - | $OCH_3$ | $OCH_3$ | CH | |
| L-2 | 0 | $C\equiv C-CH_2CN$ | H | H | - | $CH_3$ | $CH_3$ | N | |
| L-2 | 0 | $C\equiv C-CH_2CN$ | H | H | - | $CH_3$ | $OCH_3$ | N | |
| L-2 | 0 | $C\equiv C-CH_2CN$ | H | H | - | $OCH_3$ | $OCH_3$ | N | |
| L-2 | 0 | $C\equiv C-CH_2CN$ | H | H | - | Cl | $OCH_3$ | CH | |
| L-2 | 0 | $C\equiv C-CH_2OCH_3$ | H | H | - | $CH_3$ | $CH_3$ | CH | |
| L-2 | 0 | $C\equiv C-CH_2OCH_3$ | H | H | - | $CH_3$ | $OCH_3$ | CH | |
| L-2 | 0 | $C\equiv C-CH_2OCH_3$ | H | H | - | $OCH_3$ | $OCH_3$ | CH | |
| L-2 | 0 | $C\equiv C-CH_2OCH_3$ | H | H | - | $CH_3$ | $CH_3$ | N | |
| L-2 | 0 | $C\equiv C-CH_2OCH_3$ | H | H | - | $CH_3$ | $OCH_3$ | N | |
| L-2 | 0 | $C\equiv C-CH_2OCH_3$ | H | H | - | $OCH_3$ | $OCH_3$ | N | |
| L-2 | 0 | $C\equiv C-CH_2OCH_3$ | H | H | - | Cl | $OCH_3$ | CH | |
| L-2 | 0 | $C\equiv CCH_2OSO_2CH_3$ | H | H | - | $CH_3$ | $CH_3$ | CH | |
| L-2 | 0 | $C\equiv CCH_2OSO_2CH_3$ | H | H | - | $CH_3$ | $OCH_3$ | CH | |
| L-2 | 0 | $C\equiv CCH_2OSO_2CH_3$ | H | H | - | $OCH_3$ | $OCH_3$ | CH | |
| L-2 | 0 | $C\equiv CCH_2OSO_2CH_3$ | H | H | - | $CH_3$ | $CH_3$ | N | |
| L-2 | 0 | $C\equiv CCH_2OSO_2CH_3$ | H | H | - | $CH_3$ | $OCH_3$ | N | |
| L-2 | 0 | $C\equiv CCH_2OSO_2CH_3$ | H | H | - | $OCH_3$ | $OCH_3$ | N | |
| L-2 | 0 | $C\equiv CCH_2OSO_2CH_3$ | H | H | - | Cl | $OCH_3$ | CH | |
| L-2 | 0 | $C\equiv CCH_2SCH_3$ | H | H | - | $CH_3$ | $CH_3$ | CH | |
| L-2 | 0 | $C\equiv CCH_2SCH_3$ | H | H | - | $CH_3$ | $OCH_3$ | CH | |
| L-2 | 0 | $C\equiv CCH_2SCH_3$ | H | H | - | $OCH_3$ | $OCH_3$ | CH | |
| L-2 | 0 | $C\equiv CCH_2SCH_3$ | H | H | - | $CH_3$ | $CH_3$ | N | |
| L-2 | 0 | $C\equiv CCH_2SCH_3$ | H | H | - | $CH_3$ | $OCH_3$ | N | |
| L-2 | 0 | $C\equiv CCH_2SCH_3$ | H | H | - | $OCH_3$ | $OCH_3$ | N | |
| L-2 | 0 | $C\equiv CCH_2SCH_3$ | H | H | - | Cl | $OCH_3$ | CH | |
| L-2 | 1 | $HC=CH_2$ | H | H | - | $CH_3$ | $CH_3$ | CH | |
| L-2 | 1 | $HC=CH_2$ | H | H | - | $CH_3$ | $OCH_3$ | CH | |
| L-2 | 1 | $HC=CH_2$ | H | H | - | $OCH_3$ | $OCH_3$ | CH | |
| L-2 | 1 | $HC=CH_2$ | H | H | - | $CH_3$ | $CH_3$ | N | |
| L-2 | 1 | $HC=CH_2$ | H | H | - | $CH_3$ | $OCH_3$ | N | |

Table VIIIa (Continued)

| L | n | W | R | $R_1$ | $R_2$ | X | Y | Z | m.p. (°C) |
|---|---|---|---|---|---|---|---|---|---|
| L-2 | 1 | $HC=CH_2$ | H | H | - | $OCH_3$ | $OCH_3$ | N | |
| L-2 | 1 | $HC=CH_2$ | H | H | - | Cl | $OCH_3$ | CH | |
| L-2 | 1 | $BrC=CH_2$ | H | H | - | $CH_3$ | $CH_3$ | CH | |
| L-2 | 1 | $BrC=CH_2$ | H | H | - | $CH_3$ | $OCH_3$ | CH | |
| L-2 | 1 | $BrC=CH_2$ | H | H | - | $OCH_3$ | $OCH_3$ | CH | |
| L-2 | 1 | $BrC=CH_2$ | H | H | - | $CH_3$ | $CH_3$ | N | |
| L-2 | 1 | $BrC=CH_2$ | H | H | - | $CH_3$ | $OCH_3$ | N | |
| L-2 | 1 | $BrC=CH_2$ | H | H | - | $OCH_3$ | $OCH_3$ | N | |
| L-2 | 1 | $BrC=CH_2$ | H | H | - | Cl | $OCH_3$ | CH | |
| L-2 | 1 | $HC=C(CH_3)_2$ | H | H | - | $CH_3$ | $CH_3$ | CH | |
| L-2 | 1 | $HC=C(CH_3)_2$ | H | H | - | $CH_3$ | $OCH_3$ | CH | |
| L-2 | 1 | $HC=C(CH_3)_2$ | H | H | - | $OCH_3$ | $OCH_3$ | CH | |
| L-2 | 1 | $HC=C(CH_3)_2$ | H | H | - | $CH_3$ | $CH_3$ | N | |
| L-2 | 1 | $HC=C(CH_3)_2$ | H | H | - | $CH_3$ | $OCH_3$ | N | |
| L-2 | 1 | $HC=C(CH_3)_2$ | H | H | - | $OCH_3$ | $OCH_3$ | N | |
| L-2 | 1 | $HC=C(CH_3)_2$ | H | H | - | Cl | $OCH_3$ | CH | |
| L-2 | 1 | $HC=C(CH_3)CH_2CH=C(CH_3)_2$ | H | H | - | $CH_3$ | $CH_3$ | CH | |
| L-2 | 1 | $HC=C(CH_3)CH_2CH=C(CH_3)_2$ | H | H | - | $CH_3$ | $OCH_3$ | CH | |
| L-2 | 1 | $HC=C(CH_3)CH_2CH=C(CH_3)_2$ | H | H | - | $OCH_3$ | $OCH_3$ | CH | |
| L-2 | 1 | $HC=C(CH_3)CH_2CH=C(CH_3)_2$ | H | H | - | $CH_3$ | $CH_3$ | N | |
| L-2 | 1 | $HC=C(CH_3)CH_2CH=C(CH_3)_2$ | H | H | - | $CH_3$ | $OCH_3$ | N | |
| L-2 | 1 | $HC=C(CH_3)CH_2CH=C(CH_3)_2$ | H | H | - | $OCH_3$ | $OCH_3$ | N | |
| L-2 | 1 | $HC=C(CH_3)CH_2CH=C(CH_3)_2$ | H | H | - | Cl | $OCH_3$ | CH | |
| L-2 | 1 | HC=CH-Ph | H | H | - | $CH_3$ | $CH_3$ | CH | |
| L-2 | 1 | HC=CH-Ph | H | H | - | $CH_3$ | $OCH_3$ | CH | |
| L-2 | 1 | HC=CH-Ph | H | H | - | $OCH_3$ | $OCH_3$ | CH | |
| L-2 | 1 | HC=CH-Ph | H | H | - | $CH_3$ | $CH_3$ | N | |
| L-2 | 1 | HC=CH-Ph | H | H | - | $CH_3$ | $OCH_3$ | N | |
| L-2 | 1 | HC=CH-Ph | H | H | - | $OCH_3$ | $OCH_3$ | N | |
| L-2 | 1 | HC=CH-Ph | H | H | - | Cl | $OCH_3$ | CH | |

Table VIIIa (Continued)

| L | n | W | R | $R_1$ | $R_2$ | X | Y | Z | m.p. (°C) |
|---|---|---|---|---|---|---|---|---|---|
| L-2 | 1 | $HC=CHCH_3$ | H | H | - | $CH_3$ | $CH_3$ | CH | |
| L-2 | 1 | $HC=CHCH_3$ | H | H | - | $CH_3$ | $OCH_3$ | CH | |
| L-2 | 1 | $HC=CHCH_3$ | H | H | - | $OCH_3$ | $OCH_3$ | CH | |
| L-2 | 1 | $HC=CHCH_3$ | H | H | - | $CH_3$ | $CH_3$ | N | |
| L-2 | 1 | $HC=CHCH_3$ | H | H | - | $CH_3$ | $OCH_3$ | N | |
| L-2 | 1 | $HC=CHCH_3$ | H | H | - | $OCH_3$ | $OCH_3$ | N | |
| L-2 | 1 | $HC=CHCH_3$ | H | H | - | Cl | $OCH_3$ | CH | |
| L-2 | 1 | $HC=CHCH_2Br$ | H | H | - | $CH_3$ | $CH_3$ | CH | |
| L-2 | 1 | $HC=CHCH_2Br$ | H | H | - | $CH_3$ | $OCH_3$ | CH | |
| L-2 | 1 | $HC=CHCH_2Br$ | H | H | - | $OCH_3$ | $OCH_3$ | CH | |
| L-2 | 1 | $HC=CHCH_2Br$ | H | H | - | $CH_3$ | $CH_3$ | N | |
| L-2 | 1 | $HC=CHCH_2Br$ | H | H | - | $CH_3$ | $OCH_3$ | N | |
| L-2 | 1 | $HC=CHCH_2Br$ | H | H | - | $OCH_3$ | $OCH_3$ | N | |
| L-2 | 1 | $HC=CHCH_2Br$ | H | H | - | Cl | $OCH_3$ | CH | |
| L-2 | 1 | $C\equiv CH$ | H | H | - | $CH_3$ | $CH_3$ | CH | |
| L-2 | 1 | $C\equiv CH$ | H | H | - | $CH_3$ | $OCH_3$ | CH | |
| L-2 | 1 | $C\equiv CH$ | H | H | - | $OCH_3$ | $OCH_3$ | CH | |
| L-2 | 1 | $C\equiv CH$ | H | H | - | $CH_3$ | $CH_3$ | N | |
| L-2 | 1 | $C\equiv CH$ | H | H | - | $CH_3$ | $OCH_3$ | N | |
| L-2 | 1 | $C\equiv CH$ | H | H | - | $OCH_3$ | $OCH_3$ | N | |
| L-2 | 1 | $C\equiv CH$ | H | H | - | Cl | $OCH_3$ | CH | |
| L-2 | 1 | $C\equiv CCH_3$ | H | H | - | $CH_3$ | $CH_3$ | CH | |
| L-2 | 1 | $C\equiv CCH_3$ | H | H | - | $CH_3$ | $OCH_3$ | CH | |
| L-2 | 1 | $C\equiv CCH_3$ | H | H | - | $OCH_3$ | $OCH_3$ | CH | |
| L-2 | 1 | $C\equiv CCH_3$ | H | H | - | $CH_3$ | $CH_3$ | N | |
| L-2 | 1 | $C\equiv CCH_3$ | H | H | - | $CH_3$ | $OCH_3$ | N | |
| L-2 | 1 | $C\equiv CCH_3$ | H | H | - | $OCH_3$ | $OCH_3$ | N | |
| L-2 | 1 | $C\equiv CCH_3$ | H | H | - | Cl | $OCH_3$ | CH | |
| L-2 | 1 | $C\equiv C-CH_2Br$ | H | H | - | $CH_3$ | $CH_3$ | CH | |
| L-2 | 1 | $C\equiv C-CH_2Br$ | H | H | - | $CH_3$ | $OCH_3$ | CH | |

Table VIIIa (Continued)

| L | n | W | R | $R_1$ | $R_2$ | X | Y | Z | m.p. (°C) |
|---|---|---|---|---|---|---|---|---|---|
| L-2 | 1 | $C≡C-CH_2Br$ | H | H | - | $OCH_3$ | $OCH_3$ | CH | |
| L-2 | 1 | $C≡C-CH_2Br$ | H | H | - | $CH_3$ | $CH_3$ | N | |
| L-2 | 1 | $C≡C-CH_2Br$ | H | H | - | $CH_3$ | $OCH_3$ | N | |
| L-2 | 1 | $C≡C-CH_2Br$ | H | H | - | $OCH_3$ | $OCH_3$ | N | |
| L-2 | 1 | $C≡C-CH_2Br$ | H | H | - | Cl | $OCH_3$ | CH | |
| L-2 | 1 | C≡C-Ph | H | H | - | $CH_3$ | $CH_3$ | CH | |
| L-2 | 1 | C≡C-Ph | H | H | - | $CH_3$ | $OCH_3$ | CH | |
| L-2 | 1 | C≡C-Ph | H | H | - | $OCH_3$ | $OCH_3$ | CH | |
| L-2 | 1 | C≡C-Ph | H | H | - | $CH_3$ | $CH_3$ | N | |
| L-2 | 1 | C≡C-Ph | H | H | - | $CH_3$ | $OCH_3$ | N | |
| L-2 | 1 | C≡C-Ph | H | H | - | $OCH_3$ | $OCH_3$ | N | |
| L-2 | 1 | C≡C-Ph | H | H | - | Cl | $OCH_3$ | CH | |
| L-2 | 1 | $CH=CH_2$ | $CH_3$ | H | - | $CH_3$ | $CH_3$ | CH | |
| L-2 | 1 | $CH=CH_2$ | $CH_3$ | H | - | $CH_3$ | $OCH_3$ | CH | |
| L-2 | 1 | $CH=CH_2$ | $CH_3$ | H | - | $OCH_3$ | $OCH_3$ | CH | |
| L-2 | 1 | $CH=CH_2$ | $CH_3$ | H | - | $CH_3$ | $CH_3$ | N | |
| L-2 | 1 | $CH=CH_2$ | $CH_3$ | H | - | $CH_3$ | $OCH_3$ | N | |
| L-2 | 1 | $CH=CH_2$ | $CH_3$ | H | - | $OCH_3$ | $OCH_3$ | N | |
| L-2 | 1 | $CH=CH_2$ | $CH_3$ | H | - | Cl | $OCH_3$ | CH | |
| L-2 | 1 | C≡CH | $CH_3$ | H | - | $CH_3$ | $CH_3$ | CH | |
| L-2 | 1 | C≡CH | $CH_3$ | H | - | $CH_3$ | $OCH_3$ | CH | |
| L-2 | 1 | C≡CH | $CH_3$ | H | - | $OCH_3$ | $OCH_3$ | CH | |
| L-2 | 1 | C≡CH | $CH_3$ | H | - | $CH_3$ | $CH_3$ | N | |
| L-2 | 1 | C≡CH | $CH_3$ | H | - | $CH_3$ | $OCH_3$ | N | |
| L-2 | 1 | C≡CH | $CH_3$ | H | - | $OCH_3$ | $OCH_3$ | N | |
| L-2 | 1 | C≡CH | $CH_3$ | H | - | Cl | $OCH_3$ | CH | |
| L-2 | 1 | $CH=CH_2$ | H | $CH_3$ | - | $CH_3$ | $CH_3$ | CH | |
| L-2 | 1 | $CH=CH_2$ | H | $CH_3$ | - | $CH_3$ | $OCH_3$ | CH | |
| L-2 | 1 | $CH=CH_2$ | H | $CH_3$ | - | $OCH_3$ | $OCH_3$ | CH | |
| L-2 | 1 | $CH=CH_2$ | H | $CH_3$ | - | $CH_3$ | $CH_3$ | N | |
| L-2 | 1 | $CH=CH_2$ | H | $CH_3$ | - | $CH_3$ | $OCH_3$ | N | |

Table VIIIa (Continued)

| L | n | W | R | $R_1$ | $R_2$ | X | Y | Z | m.p. (°C) |
|---|---|---|---|---|---|---|---|---|---|
| L-2 | 1 | $CH=CH_2$ | H | $CH_3$ | - | $OCH_3$ | $OCH_3$ | N | |
| L-2 | 1 | $CH=CH_2$ | H | $CH_3$ | - | Cl | $OCH_3$ | CH | |
| L-2 | 1 | C≡CH | H | $CH_3$ | - | $OCH_3$ | $OCH_3$ | CH | |
| L-2 | 1 | C≡CH | H | $CH_3$ | - | $OCH_3$ | $OCH_3$ | N | |
| L-2 | 1 | C≡CH | H | $CH_3$ | - | $OCH_3$ | $CH_3$ | CH | |
| L-2 | 0 | $CH=C(CN)_2$ | H | H | - | $CH_3$ | $CH_3$ | CH | |
| L-2 | 0 | $CH=C(CN)_2$ | H | H | - | $CH_3$ | $OCH_3$ | CH | |
| L-2 | 0 | $CH=C(CN)_2$ | H | H | - | $OCH_3$ | $OCH_3$ | CH | |
| L-2 | 0 | $CH=C(CN)_2$ | H | H | - | $CH_3$ | $OCH_3$ | N | |
| L-2 | 0 | $CH=C(CN)_2$ | H | H | - | $OCH_3$ | $OCH_3$ | N | |
| L-2 | 0 | $CH=C(CN)_2$ | H | H | - | Cl | $OCH_3$ | CH | |
| L-2 | 0 | $CH=CH_2$ | H | H | - | $OCH_3$ | $\overline{CHCH_2CH_2}$ | N | |
| L-2 | 0 | $CH=CH_2$ | H | H | - | $OCH_3$ | $NHCH_3$ | N | |
| L-2 | 0 | $CH=CH_2$ | H | H | - | $OCH_2CH_3$ | $NHCH_3$ | N | |
| L-2 | 0 | $CH=CH_2$ | H | H | - | $OCF_2H$ | $OCH_3$ | CH | |
| L-2 | 0 | $CH=CH_2$ | H | H | - | $OCH_2CH_3$ | $CH_3$ | CH | |
| L-2 | 0 | $CH=CH_2$ | H | H | - | $OCH_3$ | $OCH_2CF_3$ | N | |

Table VIIIb

General Structure VIIIb

| L | n | W | R | $R_1$ | $R_2$ | X | Y | Z | m.p. (°C) |
|---|---|---|---|---|---|---|---|---|---|
| L-2 | 0 | $CH=CH_2$ | H | H | - | $CH_3$ | $CH_3$ | CH | |
| L-2 | 0 | $CH=CH_2$ | H | H | - | $CH_3$ | $OCH_3$ | CH | |
| L-2 | 0 | $CH=CH_2$ | H | H | - | $OCH_3$ | $OCH_3$ | CH | |
| L-2 | 0 | $CH=CH_2$ | H | H | - | $CH_3$ | $CH_3$ | N | |
| L-2 | 0 | $CH=CH_2$ | H | H | - | $CH_3$ | $OCH_3$ | N | |
| L-2 | 0 | $CH=CH_2$ | H | H | - | $OCH_3$ | $OCH_3$ | N | |
| L-2 | 0 | $CH=CH_2$ | H | H | - | Cl | $OCH_3$ | CH | |
| L-2 | 0 | $C(CH_3)=CHCH_3$ | H | H | - | $CH_3$ | $CH_3$ | CH | |
| L-2 | 0 | $C(CH_3)=CHCH_3$ | H | H | - | $CH_3$ | $OCH_3$ | CH | |
| L-2 | 0 | $C(CH_3)=CHCH_3$ | H | H | - | $OCH_3$ | $OCH_3$ | CH | |
| L-2 | 0 | $C(CH_3)=CHCH_3$ | H | H | - | $CH_3$ | $CH_3$ | N | |
| L-2 | 0 | $C(CH_3)=CHCH_3$ | H | H | - | $CH_3$ | $OCH_3$ | N | |
| L-2 | 0 | $C(CH_3)=CHCH_3$ | H | H | - | $OCH_3$ | $OCH_3$ | N | |
| L-2 | 0 | $C(CH_3)=CHCH_3$ | H | H | - | Cl | $OCH_3$ | CH | |
| L-2 | 0 | $CH=CHCH_2CH_3$ | H | H | - | $CH_3$ | $CH_3$ | CH | |
| L-2 | 0 | $CH=CHCH_2CH_3$ | H | H | - | $CH_3$ | $OCH_3$ | CH | |
| L-2 | 0 | $CH=CHCH_2CH_3$ | H | H | - | $OCH_3$ | $OCH_3$ | CH | |
| L-2 | 0 | $CH=CHCH_2CH_3$ | H | H | - | $CH_3$ | $CH_3$ | N | |
| L-2 | 0 | $CH=CHCH_2CH_3$ | H | H | - | $CH_3$ | $OCH_3$ | N | |
| L-2 | 0 | $CH=CHCH_2CH_3$ | H | H | - | $OCH_3$ | $OCH_3$ | N | |
| L-2 | 0 | $CH=CHCH_2CH_3$ | H | H | - | Cl | $OCH_3$ | CH | |
| L-2 | 0 | $C(CH_3)=CHCH_2CH_3$ | H | H | - | $CH_3$ | $CH_3$ | CH | |
| L-2 | 0 | $C(CH_3)=CHCH_2CH_3$ | H | H | - | $CH_3$ | $OCH_3$ | CH | |
| L-2 | 0 | $C(CH_3)=CHCH_2CH_3$ | H | H | - | $OCH_3$ | $OCH_3$ | CH | |
| L-2 | 0 | $C(CH_3)=CHCH_2CH_3$ | H | H | - | $CH_3$ | $CH_3$ | N | |
| L-2 | 0 | $C(CH_3)=CHCH_2CH_3$ | H | H | - | $CH_3$ | $OCH_3$ | N | |
| L-2 | 0 | $C(CH_3)=CHCH_2CH_3$ | H | H | - | $OCH_3$ | $OCH_3$ | N | |
| L-2 | 0 | $C(CH_3)=CHCH_2CH_3$ | H | H | - | Cl | $OCH_3$ | CH | |
| L-2 | 0 | $CH=CHCH_2CH_2CH_3$ | H | H | - | $CH_3$ | $CH_3$ | CH | |
| L-2 | 0 | $CH=CHCH_2CH_2CH_3$ | H | H | - | $CH_3$ | $OCH_3$ | CH | |

Table VIIIb (Continued)

| L | n | W | R | $R_1$ | $R_2$ | X | Y | Z | m.p. (°C) |
|---|---|---|---|---|---|---|---|---|---|
| L-2 | 0 | $CH=CHCH_2CH_2CH_3$ | H | H | - | $OCH_3$ | $OCH_3$ | CH | |
| L-2 | 0 | $CH=CHCH_2CH_2CH_3$ | H | H | - | $CH_3$ | $CH_3$ | N | |
| L-2 | 0 | $CH=CHCH_2CH_2CH_3$ | H | H | - | $CH_3$ | $OCH_3$ | N | |
| L-2 | 0 | $CH=CHCH_2CH_2CH_3$ | H | H | - | $OCH_3$ | $OCH_3$ | N | |
| L-2 | 0 | $CH=CHCH_2CH_2CH_3$ | H | H | - | Cl | $OCH_3$ | CH | |
| L-2 | 0 | CH=CH-CN | H | H | - | $CH_3$ | $CH_3$ | CH | |
| L-2 | 0 | CH=CH-CN | H | H | - | $CH_3$ | $OCH_3$ | CH | |
| L-2 | 0 | CH=CH-CN | H | H | - | $OCH_3$ | $OCH_3$ | CH | |
| L-2 | 0 | CH=CH-CN | H | H | - | $CH_3$ | $CH_3$ | N | |
| L-2 | 0 | CH=CH-CN | H | H | - | $CH_3$ | $OCH_3$ | N | |
| L-2 | 0 | CH=CH-CN | H | H | - | $OCH_3$ | $OCH_3$ | N | |
| L-2 | 0 | CH=CH-CN | H | H | - | Cl | $OCH_3$ | CH | |
| L-2 | 0 | $CH=CH-OCH_3$ | H | H | - | $CH_3$ | $CH_3$ | CH | |
| L-2 | 0 | $CH=CH-OCH_3$ | H | H | - | $CH_3$ | $OCH_3$ | CH | |
| L-2 | 0 | $CH=CH-OCH_3$ | H | H | - | $OCH_3$ | $OCH_3$ | CH | |
| L-2 | 0 | $CH=CH-OCH_3$ | H | H | - | $CH_3$ | $CH_3$ | N | |
| L-2 | 0 | $CH=CH-OCH_3$ | H | H | - | $CH_3$ | $OCH_3$ | N | |
| L-2 | 0 | $CH=CH-OCH_3$ | H | H | - | $OCH_3$ | $OCH_3$ | N | |
| L-2 | 0 | $CH=CH-OCH_3$ | H | H | - | Cl | $OCH_3$ | CH | |
| L-2 | 0 | CH=CH-Br | H | H | - | $CH_3$ | $CH_3$ | CH | |
| L-2 | 0 | CH=CH-Br | H | H | - | $CH_3$ | $OCH_3$ | CH | |
| L-2 | 0 | CH=CH-Br | H | H | - | $OCH_3$ | $OCH_3$ | CH | |
| L-2 | 0 | CH=CH-Br | H | H | - | $CH_3$ | $CH_3$ | N | |
| L-2 | 0 | CH=CH-Br | H | H | - | $CH_3$ | $OCH_3$ | N | |
| L-2 | 0 | CH=CH-Br | H | H | - | $OCH_3$ | $OCH_3$ | N | |
| L-2 | 0 | CH=CH-Br | H | H | - | Cl | $OCH_3$ | CH | |
| L-2 | 0 | $CH=CH-CF_3$ | H | H | - | $CH_3$ | $CH_3$ | CH | |
| L-2 | 0 | $CH=CH-CF_3$ | H | H | - | $CH_3$ | $OCH_3$ | CH | |
| L-2 | 0 | $CH=CH-CF_3$ | H | H | - | $OCH_3$ | $OCH_3$ | CH | |
| L-2 | 0 | $CH=CH-CF_3$ | H | H | - | $CH_3$ | $CH_3$ | N | |
| L-2 | 0 | $CH=CH-CF_3$ | H | H | - | $CH_3$ | $OCH_3$ | N | |

Table VIIIb (Continued)

| L | n | W | R | $R_1$ | $R_2$ | X | Y | Z | m.p. (°C) |
|---|---|---|---|---|---|---|---|---|---|
| L-2 | 0 | $CH=CH-CF_3$ | H | H | - | $OCH_3$ | $OCH_3$ | N | |
| L-2 | 0 | $CH=CH-CF_3$ | H | H | - | Cl | $OCH_3$ | CH | |
| L-2 | 0 | $C(CH_3)=CH-OCH_3$ | H | H | - | $CH_3$ | $CH_3$ | CH | |
| L-2 | 0 | $C(CH_3)=CH-OCH_3$ | H | H | - | $CH_3$ | $OCH_3$ | CH | |
| L-2 | 0 | $C(CH_3)=CH-OCH_3$ | H | H | - | $OCH_3$ | $OCH_3$ | CH | |
| L-2 | 0 | $C(CH_3)=CH-OCH_3$ | H | H | - | $CH_3$ | $CH_3$ | N | |
| L-2 | 0 | $C(CH_3)=CH-OCH_3$ | H | H | - | $CH_3$ | $OCH_3$ | N | |
| L-2 | 0 | $C(CH_3)=CH-OCH_3$ | H | H | - | $OCH_3$ | $OCH_3$ | N | |
| L-2 | 0 | $C(CH_3)=CH-OCH_3$ | H | H | - | Cl | $OCH_3$ | CH | |
| L-2 | 0 | CH=CHPh | H | H | - | $CH_3$ | $CH_3$ | CH | |
| L-2 | 0 | CH=CHPh | H | H | - | $CH_3$ | $OCH_3$ | CH | |
| L-2 | 0 | CH=CHPh | H | H | - | $OCH_3$ | $OCH_3$ | CH | |
| L-2 | 0 | CH=CHPh | H | H | - | $CH_3$ | $CH_3$ | N | |
| L-2 | 0 | CH=CHPh | H | H | - | $CH_3$ | $OCH_3$ | N | |
| L-2 | 0 | CH=CHPh | H | H | - | $OCH_3$ | $OCH_3$ | N | |
| L-2 | 0 | CH=CHPh | H | H | - | Cl | $OCH_3$ | CH | |
| L-2 | 0 | $C(CH_3)=CH-Ph$ | H | H | - | $CH_3$ | $CH_3$ | CH | |
| L-2 | 0 | $C(CH_3)=CH-Ph$ | H | H | - | $CH_3$ | $OCH_3$ | CH | |
| L-2 | 0 | $C(CH_3)=CH-Ph$ | H | H | - | $OCH_3$ | $OCH_3$ | CH | |
| L-2 | 0 | $C(CH_3)=CH-Ph$ | H | H | - | $CH_3$ | $CH_3$ | N | |
| L-2 | 0 | $C(CH_3)=CH-Ph$ | H | H | - | $CH_3$ | $OCH_3$ | N | |
| L-2 | 0 | $C(CH_3)=CH-Ph$ | H | H | - | $OCH_3$ | $OCH_3$ | N | |
| L-2 | 0 | $C(CH_3)=CH-Ph$ | H | H | - | Cl | $OCH_3$ | CH | |
| L-2 | 0 | $C=CH-(CH_2)_3$ | H | H | - | $CH_3$ | $CH_3$ | CH | |
| L-2 | 0 | $C=CH-(CH_2)_3$ | H | H | - | $CH_3$ | $OCH_3$ | CH | |
| L-2 | 0 | $C=CH-(CH_2)_3$ | H | H | - | $OCH_3$ | $OCH_3$ | CH | |
| L-2 | 0 | $C=CH-(CH_2)_3$ | H | H | - | $CH_3$ | $CH_3$ | N | |
| L-2 | 0 | $C=CH-(CH_2)_3$ | H | H | - | $CH_3$ | $OCH_3$ | N | |
| L-2 | 0 | $C=CH-(CH_2)_3$ | H | H | - | $OCH_3$ | $OCH_3$ | N | |
| L-2 | 0 | $C=CH-(CH_2)_3$ | H | H | - | Cl | $OCH_3$ | CH | |

Table VIIIb (Continued)

| L | n | W | R | $R_1$ | $R_2$ | X | Y | Z | m.p. (°C) |
|---|---|---|---|---|---|---|---|---|---|
| L-2 | 0 | $\overline{C=CH-(CH_2)}_4$ | H | H | - | $CH_3$ | $CH_3$ | CH | |
| L-2 | 0 | $\overline{C=CH-(CH_2)}_4$ | H | H | - | $CH_3$ | $OCH_3$ | CH | |
| L-2 | 0 | $\overline{C=CH-(CH_2)}_4$ | H | H | - | $OCH_3$ | $OCH_3$ | CH | |
| L-2 | 0 | $\overline{C=CH-(CH_2)}_4$ | H | H | - | $CH_3$ | $CH_3$ | N | |
| L-2 | 0 | $\overline{C=CH-(CH_2)}_4$ | H | H | - | $CH_3$ | $OCH_3$ | N | |
| L-2 | 0 | $\overline{C=CH-(CH_2)}_4$ | H | H | - | $OCH_3$ | $OCH_3$ | N | |
| L-2 | 0 | $\overline{C=CH-(CH_2)}_4$ | H | H | - | Cl | $OCH_3$ | CH | |
| L-2 | 0 | $\overline{C=CH-(CH_2)}_5$ | H | H | - | $CH_3$ | $CH_3$ | CH | |
| L-2 | 0 | $\overline{C=CH-(CH_2)}_5$ | H | H | - | $CH_3$ | $OCH_3$ | CH | |
| L-2 | 0 | $\overline{C=CH-(CH_2)}_5$ | H | H | - | $OCH_3$ | $OCH_3$ | CH | |
| L-2 | 0 | $\overline{C=CH-(CH_2)}_5$ | H | H | - | $CH_3$ | $CH_3$ | N | |
| L-2 | 0 | $\overline{C=CH-(CH_2)}_5$ | H | H | - | $CH_3$ | $OCH_3$ | N | |
| L-2 | 0 | $\overline{C=CH-(CH_2)}_5$ | H | H | - | $OCH_3$ | $OCH_3$ | N | |
| L-2 | 0 | $\overline{C=CH-(CH_2)}_5$ | H | H | - | Cl | $OCH_3$ | CH | |
| L-2 | 0 | C(CN)=CHCN | H | H | - | $CH_3$ | $CH_3$ | CH | |
| L-2 | 0 | C(CN)=CHCN | H | H | - | $CH_3$ | $OCH_3$ | CH | |
| L-2 | 0 | C(CN)=CHCN | H | H | - | $OCH_3$ | $OCH_3$ | CH | |
| L-2 | 0 | C(CN)=CHCN | H | H | - | $CH_3$ | $CH_3$ | N | |
| L-2 | 0 | C(CN)=CHCN | H | H | - | $CH_3$ | $OCH_3$ | N | |
| L-2 | 0 | C(CN)=CHCN | H | H | - | $OCH_3$ | $OCH_3$ | N | |
| L-2 | 0 | C(CN)=CHCN | H | H | - | Cl | $OCH_3$ | CH | |
| L-2 | 0 | $CH=CH-SO_2N(CH_3)_2$ | H | H | - | $CH_3$ | $CH_3$ | CH | |
| L-2 | 0 | $CH=CH-SO_2N(CH_3)_2$ | H | H | - | $CH_3$ | $OCH_3$ | CH | |
| L-2 | 0 | $CH=CH-SO_2N(CH_3)_2$ | H | H | - | $OCH_3$ | $OCH_3$ | CH | |
| L-2 | 0 | $CH=CH-SO_2N(CH_3)_2$ | H | H | - | $CH_3$ | $CH_3$ | N | |
| L-2 | 0 | $CH=CH-SO_2N(CH_3)_2$ | H | H | - | $CH_3$ | $OCH_3$ | N | |
| L-2 | 0 | $CH=CH-SO_2N(CH_3)_2$ | H | H | - | $OCH_3$ | $OCH_3$ | N | |
| L-2 | 0 | $CH=CH-SO_2N(CH_3)_2$ | H | H | - | Cl | $OCH_3$ | CH | |

Table VIIIb (Continued)

| L | n | W | R | $R_1$ | $R_2$ | X | Y | Z | m.p. (°C) |
|---|---|---|---|---|---|---|---|---|---|
| L-2 | 0 | $CH=CH-(CH_2)_4$ | $CH_3$ | H | - | $OCH_3$ | $OCH_3$ | CH | |
| L-2 | 0 | $CH=CH-(CH_2)_4$ | H | $CH_3$ | - | $OCH_3$ | $OCH_3$ | CH | |
| L-2 | 0 | $C(=CH_2)-CH_2CH_2CH(NO_2)CH_3$ | H | H | - | $OCH_3$ | $OCH_3$ | CH | |
| L-2 | 0 | $CH=CHCH_2OSO_2Ph$ | H | H | - | $OCH_3$ | $OCH_3$ | CH | |
| L-2 | 0 | $CH=CHCH_2OSO_2CH_3$ | H | H | - | $OCH_3$ | $OCH_3$ | CH | |
| L-2 | 0 | $CH=CHSCH_3$ | H | H | - | $OCH_3$ | $OCH_3$ | CH | |
| L-2 | 0 | C≡CH | H | H | - | $CH_3$ | $CH_3$ | CH | |
| L-2 | 0 | C≡CH | H | H | - | $CH_3$ | $OCH_3$ | CH | |
| L-2 | 0 | C≡CH | H | H | - | $OCH_3$ | $OCH_3$ | CH | |
| L-2 | 0 | C≡CH | H | H | - | $CH_3$ | $CH_3$ | N | |
| L-2 | 0 | C≡CH | H | H | - | $CH_3$ | $OCH_3$ | N | |
| L-2 | 0 | C≡CH | H | H | - | $OCH_3$ | $OCH_3$ | N | |
| L-2 | 0 | C≡CH | H | H | - | Cl | $OCH_3$ | CH | |
| L-2 | 0 | C≡C-Ph | H | H | - | $CH_3$ | $CH_3$ | CH | |
| L-2 | 0 | C≡C-Ph | H | H | - | $CH_3$ | $OCH_3$ | CH | |
| L-2 | 0 | C≡C-Ph | H | H | - | $OCH_3$ | $OCH_3$ | CH | |
| L-2 | 0 | C≡C-Ph | H | H | - | $CH_3$ | $CH_3$ | N | |
| L-2 | 0 | C≡C-Ph | H | H | - | $CH_3$ | $OCH_3$ | N | |
| L-2 | 0 | C≡C-Ph | H | H | - | $OCH_3$ | $OCH_3$ | N | |
| L-2 | 0 | C≡C-Ph | H | H | - | Cl | $OCH_3$ | CH | |
| L-2 | 0 | $C≡C-CH_3$ | H | H | - | $CH_3$ | $CH_3$ | CH | |
| L-2 | 0 | $C≡C-CH_3$ | H | H | - | $CH_3$ | $OCH_3$ | CH | |
| L-2 | 0 | $C≡C-CH_3$ | H | H | - | $OCH_3$ | $OCH_3$ | CH | |
| L-2 | 0 | $C≡C-CH_3$ | H | H | - | $CH_3$ | $CH_3$ | N | |
| L-2 | 0 | $C≡C-CH_3$ | H | H | - | $CH_3$ | $OCH_3$ | N | |

Table VIIIb (Continued)

| L | n | W | R | $R_1$ | $R_2$ | X | Y | Z | m.p. (°C) |
|---|---|---|---|---|---|---|---|---|---|
| L-2 | 0 | $C{\equiv}C-CH_3$ | H | H | - | $OCH_3$ | $OCH_3$ | N | |
| L-2 | 0 | $C{\equiv}C-CH_3$ | H | H | - | Cl | $OCH_3$ | CH | |
| L-2 | 0 | $C{\equiv}CCH_2OC(O)CH_3$ | H | H | - | $CH_3$ | $CH_3$ | CH | |
| L-2 | 0 | $C{\equiv}CCH_2OC(O)CH_3$ | H | H | - | $CH_3$ | $OCH_3$ | CH | |
| L-2 | 0 | $C{\equiv}CCH_2OC(O)CH_3$ | H | H | - | $OCH_3$ | $OCH_3$ | CH | |
| L-2 | 0 | $C{\equiv}CCH_2OC(O)CH_3$ | H | H | - | $CH_3$ | $CH_3$ | N | |
| L-2 | 0 | $C{\equiv}CCH_2OC(O)CH_3$ | H | H | - | $CH_3$ | $OCH_3$ | N | |
| L-2 | 0 | $C{\equiv}CCH_2OC(O)CH_3$ | H | H | - | $OCH_3$ | $OCH_3$ | N | |
| L-2 | 0 | $C{\equiv}CCH_2OC(O)CH_3$ | H | H | - | Cl | $OCH_3$ | CH | |
| L-2 | 0 | $C{\equiv}CCH_2OH$ | H | H | - | $CH_3$ | $CH_3$ | CH | |
| L-2 | 0 | $C{\equiv}CCH_2OH$ | H | H | - | $CH_3$ | $OCH_3$ | CH | |
| L-2 | 0 | $C{\equiv}CCH_2OH$ | H | H | - | $OCH_3$ | $OCH_3$ | CH | |
| L-2 | 0 | $C{\equiv}CCH_2OH$ | H | H | - | $CH_3$ | $CH_3$ | N | |
| L-2 | 0 | $C{\equiv}CCH_2OH$ | H | H | - | $CH_3$ | $OCH_3$ | N | |
| L-2 | 0 | $C{\equiv}CCH_2OH$ | H | H | - | $OCH_3$ | $OCH_3$ | N | |
| L-2 | 0 | $C{\equiv}CCH_2OH$ | H | H | - | Cl | $OCH_3$ | CH | |
| L-2 | 0 | $C{\equiv}CCH_2Cl$ | H | H | - | $CH_3$ | $CH_3$ | CH | |
| L-2 | 0 | $C{\equiv}CCH_2Cl$ | H | H | - | $CH_3$ | $OCH_3$ | CH | |
| L-2 | 0 | $C{\equiv}CCH_2Cl$ | H | H | - | $OCH_3$ | $OCH_3$ | CH | |
| L-2 | 0 | $C{\equiv}CCH_2Cl$ | H | H | - | $CH_3$ | $CH_3$ | N | |
| L-2 | 0 | $C{\equiv}CCH_2Cl$ | H | H | - | $CH_3$ | $OCH_3$ | N | |
| L-2 | 0 | $C{\equiv}CCH_2Cl$ | H | H | - | $OCH_3$ | $OCH_3$ | N | |
| L-2 | 0 | $C{\equiv}CCH_2Cl$ | H | H | - | Cl | $OCH_3$ | CH | |
| L-2 | 0 | $C{\equiv}C-CH_2Br$ | H | H | - | $CH_3$ | $CH_3$ | CH | |
| L-2 | 0 | $C{\equiv}C-CH_2Br$ | H | H | - | $CH_3$ | $OCH_3$ | CH | |
| L-2 | 0 | $C{\equiv}C-CH_2Br$ | H | H | - | $OCH_3$ | $OCH_3$ | CH | |
| L-2 | 0 | $C{\equiv}C-CH_2Br$ | H | H | - | $CH_3$ | $CH_3$ | N | |
| L-2 | 0 | $C{\equiv}C-CH_2Br$ | H | H | - | $CH_3$ | $OCH_3$ | N | |
| L-2 | 0 | $C{\equiv}C-CH_2Br$ | H | H | - | $OCH_3$ | $OCH_3$ | N | |
| L-2 | 0 | $C{\equiv}C-CH_2Br$ | H | H | - | Cl | $OCH_3$ | CH | |
| L-2 | 0 | $C{\equiv}C-CH_2CN$ | H | H | - | $CH_3$ | $CH_3$ | CH | |
| L-2 | 0 | $C{\equiv}C-CH_2CN$ | H | H | - | $CH_3$ | $OCH_3$ | CH | |

Table VIIIb (Continued)

| L | n | W | R | $R_1$ | $R_2$ | X | Y | Z | m.p. (°C) |
|---|---|---|---|---|---|---|---|---|---|
| L-2 | 0 | $C{\equiv}C-CH_2CN$ | H | H | - | $OCH_3$ | $OCH_3$ | CH | |
| L-2 | 0 | $C{\equiv}C-CH_2CN$ | H | H | - | $CH_3$ | $CH_3$ | N | |
| L-2 | 0 | $C{\equiv}C-CH_2CN$ | H | H | - | $CH_3$ | $OCH_3$ | N | |
| L-2 | 0 | $C{\equiv}C-CH_2CN$ | H | H | - | $OCH_3$ | $OCH_3$ | N | |
| L-2 | 0 | $C{\equiv}C-CH_2CN$ | H | H | - | Cl | $OCH_3$ | CH | |
| L-2 | 0 | $C{\equiv}C-CH_2OCH_3$ | H | H | - | $CH_3$ | $CH_3$ | CH | |
| L-2 | 0 | $C{\equiv}C-CH_2OCH_3$ | H | H | - | $CH_3$ | $OCH_3$ | CH | |
| L-2 | 0 | $C{\equiv}C-CH_2OCH_3$ | H | H | - | $OCH_3$ | $OCH_3$ | CH | |
| L-2 | 0 | $C{\equiv}C-CH_2OCH_3$ | H | H | - | $CH_3$ | $CH_3$ | N | |
| L-2 | 0 | $C{\equiv}C-CH_2OCH_3$ | H | H | - | $CH_3$ | $OCH_3$ | N | |
| L-2 | 0 | $C{\equiv}C-CH_2OCH_3$ | H | H | - | $OCH_3$ | $OCH_3$ | N | |
| L-2 | 0 | $C{\equiv}C-CH_2OCH_3$ | H | H | - | Cl | $OCH_3$ | CH | |
| L-2 | 0 | $C{\equiv}CCH_2OSO_2CH_3$ | H | H | - | $CH_3$ | $CH_3$ | CH | |
| L-2 | 0 | $C{\equiv}CCH_2OSO_2CH_3$ | H | H | - | $CH_3$ | $OCH_3$ | CH | |
| L-2 | 0 | $C{\equiv}CCH_2OSO_2CH_3$ | H | H | - | $OCH_3$ | $OCH_3$ | CH | |
| L-2 | 0 | $C{\equiv}CCH_2OSO_2CH_3$ | H | H | - | $CH_3$ | $CH_3$ | N | |
| L-2 | 0 | $C{\equiv}CCH_2OSO_2CH_3$ | H | H | - | $CH_3$ | $OCH_3$ | N | |
| L-2 | 0 | $C{\equiv}CCH_2OSO_2CH_3$ | H | H | - | $OCH_3$ | $OCH_3$ | N | |
| L-2 | 0 | $C{\equiv}CCH_2OSO_2CH_3$ | H | H | - | Cl | $OCH_3$ | CH | |
| L-2 | 0 | $C{\equiv}CCH_2SCH_3$ | H | H | - | $CH_3$ | $CH_3$ | CH | |
| L-2 | 0 | $C{\equiv}CCH_2SCH_3$ | H | H | - | $CH_3$ | $OCH_3$ | CH | |
| L-2 | 0 | $C{\equiv}CCH_2SCH_3$ | H | H | - | $OCH_3$ | $OCH_3$ | CH | |
| L-2 | 0 | $C{\equiv}CCH_2SCH_3$ | H | H | - | $CH_3$ | $CH_3$ | N | |
| L-2 | 0 | $C{\equiv}CCH_2SCH_3$ | H | H | - | $CH_3$ | $OCH_3$ | N | |
| L-2 | 0 | $C{\equiv}CCH_2SCH_3$ | H | H | - | $OCH_3$ | $OCH_3$ | N | |
| L-2 | 0 | $C{\equiv}CCH_2SCH_3$ | H | H | - | Cl | $OCH_3$ | CH | |
| L-2 | 1 | $HC=CH_2$ | H | H | - | $CH_3$ | $CH_3$ | CH | |
| L-2 | 1 | $HC=CH_2$ | H | H | - | $CH_3$ | $OCH_3$ | CH | |
| L-2 | 1 | $HC=CH_2$ | H | H | - | $OCH_3$ | $OCH_3$ | CH | |
| L-2 | 1 | $HC=CH_2$ | H | H | - | $CH_3$ | $CH_3$ | N | |
| L-2 | 1 | $HC=CH_2$ | H | H | - | $CH_3$ | $OCH_3$ | N | |

Table VIIIb (Continued)

| L | n | W | R | $R_1$ | $R_2$ | X | Y | Z | m.p. (°C) |
|---|---|---|---|---|---|---|---|---|---|
| L-2 | 1 | $HC=CH_2$ | H | H | - | $OCH_3$ | $OCH_3$ | N | |
| L-2 | 1 | $HC=CH_2$ | H | H | - | Cl | $OCH_3$ | CH | |
| L-2 | 1 | $BrC=CH_2$ | H | H | - | $CH_3$ | $CH_3$ | CH | |
| L-2 | 1 | $BrC=CH_2$ | H | H | | $CH_3$ | $OCH_3$ | CH | |
| L-2 | 1 | $BrC=CH_2$ | H | H | - | $OCH_3$ | $OCH_3$ | CH | |
| L-2 | 1 | $BrC=CH_2$ | H | H | - | $CH_3$ | $CH_3$ | N | |
| L-2 | 1 | $BrC=CH_2$ | H | H | - | $CH_3$ | $OCH_3$ | N | |
| L-2 | 1 | $BrC=CH_2$ | H | H | - | $OCH_3$ | $OCH_3$ | N | |
| L-2 | 1 | $BrC=CH_2$ | H | H | - | Cl | $OCH_3$ | CH | |
| L-2 | 1 | $HC=C(CH_3)_2$ | H | H | - | $CH_3$ | $CH_3$ | CH | |
| L-2 | 1 | $HC=C(CH_3)_2$ | H | H | - | $CH_3$ | $OCH_3$ | CH | |
| L-2 | 1 | $HC=C(CH_3)_2$ | H | H | - | $OCH_3$ | $OCH_3$ | CH | |
| L-2 | 1 | $HC=C(CH_3)_2$ | H | H | - | $CH_3$ | $CH_3$ | N | |
| L-2 | 1 | $HC=C(CH_3)_2$ | H | H | - | $CH_3$ | $OCH_3$ | N | |
| L-2 | 1 | $HC=C(CH_3)_2$ | H | H | - | $OCH_3$ | $OCH_3$ | N | |
| L-2 | 1 | $HC=C(CH_3)_2$ | H | H | - | Cl | $OCH_3$ | CH | |
| L-2 | 1 | $HC=C(CH_3)CH_2CH_2CH=C(CH_3)_2$ | H | H | - | $CH_3$ | $CH_3$ | CH | |
| L-2 | 1 | $HC=C(CH_3)CH_2CH_2CH=C(CH_3)_2$ | H | H | - | $CH_3$ | $OCH_3$ | CH | |
| L-2 | 1 | $HC=C(CH_3)CH_2CH_2CH=C(CH_3)_2$ | H | H | - | $OCH_3$ | $OCH_3$ | CH | |
| L-2 | 1 | $HC=C(CH_3)CH_2CH_2CH=C(CH_3)_2$ | H | H | - | $CH_3$ | $CH_3$ | N | |
| L-2 | 1 | $HC=C(CH_3)CH_2CH_2CH=C(CH_3)_2$ | H | H | - | $CH_3$ | $OCH_3$ | N | |
| L-2 | 1 | $HC=C(CH_3)CH_2CH_2CH=C(CH_3)_2$ | H | H | - | $OCH_3$ | $OCH_3$ | N | |
| L-2 | 1 | $HC=C(CH_3)CH_2CH_2CH=C(CH_3)_2$ | H | H | - | Cl | $OCH_3$ | CH | |
| L-2 | 1 | HC=CH-Ph | H | H | - | $CH_3$ | $CH_3$ | CH | |
| L-2 | 1 | HC=CH-Ph | H | H | - | $CH_3$ | $OCH_3$ | CH | |
| L-2 | 1 | HC=CH-Ph | H | H | - | $OCH_3$ | $OCH_3$ | CH | |
| L-2 | 1 | HC=CH-Ph | H | H | - | $CH_3$ | $CH_3$ | N | |
| L-2 | 1 | HC=CH-Ph | H | H | - | $CH_3$ | $OCH_3$ | N | |
| L-2 | 1 | HC=CH-Ph | H | H | - | $OCH_3$ | $OCH_3$ | N | |
| L-2 | 1 | HC=CH-Ph | H | H | - | Cl | $OCH_3$ | CH | |

Table VIIIb (Continued)

| L | n | W | R | $R_1$ | $R_2$ | X | Y | Z | m.p. (°C) |
|---|---|---|---|---|---|---|---|---|---|
| L-2 | 1 | $HC=CHCH_3$ | H | H | - | $CH_3$ | $CH_3$ | CH | |
| L-2 | 1 | $HC=CHCH_3$ | H | H | - | $CH_3$ | $OCH_3$ | CH | |
| L-2 | 1 | $HC=CHCH_3$ | H | H | - | $OCH_3$ | $OCH_3$ | CH | |
| L-2 | 1 | $HC=CHCH_3$ | H | H | - | $CH_3$ | $CH_3$ | N | |
| L-2 | 1 | $HC=CHCH_3$ | H | H | - | $CH_3$ | $OCH_3$ | N | |
| L-2 | 1 | $HC=CHCH_3$ | H | H | - | $OCH_3$ | $OCH_3$ | N | |
| L-2 | 1 | $HC=CHCH_3$ | H | H | - | Cl | $OCH_3$ | CH | |
| L-2 | 1 | $HC=CHCH_2Br$ | H | H | - | $CH_3$ | $CH_3$ | CH | |
| L-2 | 1 | $HC=CHCH_2Br$ | H | H | - | $CH_3$ | $OCH_3$ | CH | |
| L-2 | 1 | $HC=CHCH_2Br$ | H | H | - | $OCH_3$ | $OCH_3$ | CH | |
| L-2 | 1 | $HC=CHCH_2Br$ | H | H | - | $CH_3$ | $CH_3$ | N | |
| L-2 | 1 | $HC=CHCH_2Br$ | H | H | - | $CH_3$ | $OCH_3$ | N | |
| L-2 | 1 | $HC=CHCH_2Br$ | H | H | - | $OCH_3$ | $OCH_3$ | N | |
| L-2 | 1 | $HC=CHCH_2Br$ | H | H | - | Cl | $OCH_3$ | CH | |
| L-2 | 1 | C≡CH | H | H | - | $CH_3$ | $CH_3$ | CH | |
| L-2 | 1 | C≡CH | H | H | - | $CH_3$ | $OCH_3$ | CH | |
| L-2 | 1 | C≡CH | H | H | - | $OCH_3$ | $OCH_3$ | CH | |
| L-2 | 1 | C≡CH | H | H | - | $CH_3$ | $CH_3$ | N | |
| L-2 | 1 | C≡CH | H | H | - | $CH_3$ | $OCH_3$ | N | |
| L-2 | 1 | C≡CH | H | H | - | $OCH_3$ | $OCH_3$ | N | |
| L-2 | 1 | C≡CH | H | H | - | Cl | $OCH_3$ | CH | |
| L-2 | 1 | $C≡CCH_3$ | H | H | - | $CH_3$ | $CH_3$ | CH | |
| L-2 | 1 | $C≡CCH_3$ | H | H | - | $CH_3$ | $OCH_3$ | CH | |
| L-2 | 1 | $C≡CCH_3$ | H | H | - | $OCH_3$ | $OCH_3$ | CH | |
| L-2 | 1 | $C≡CCH_3$ | H | H | - | $CH_3$ | $CH_3$ | N | |
| L-2 | 1 | $C≡CCH_3$ | H | H | - | $CH_3$ | $OCH_3$ | N | |
| L-2 | 1 | $C≡CCH_3$ | H | H | - | $OCH_3$ | $OCH_3$ | N | |
| L-2 | 1 | $C≡CCH_3$ | H | H | - | Cl | $OCH_3$ | CH | |
| L-2 | 1 | $C≡C-CH_2Br$ | H | H | - | $CH_3$ | $CH_3$ | CH | |
| L-2 | 1 | $C≡C-CH_2Br$ | H | H | - | $CH_3$ | $OCH_3$ | CH | |

Table VIIIb (Continued)

| L | n | W | R | $R_1$ | $R_2$ | X | Y | Z | m.p. (°C) |
|---|---|---|---|---|---|---|---|---|---|
| L-2 | 1 | C≡C-$CH_2$Br | H | H | - | $OCH_3$ | $OCH_3$ | CH | |
| L-2 | 1 | C≡C-$CH_2$Br | H | H | - | $CH_3$ | $CH_3$ | N | |
| L-2 | 1 | C≡C-$CH_2$Br | H | H | - | $CH_3$ | $OCH_3$ | N | |
| L-2 | 1 | C≡C-$CH_2$Br | H | H | - | $OCH_3$ | $OCH_3$ | N | |
| L-2 | 1 | C≡C-$CH_2$Br | H | H | - | Cl | $OCH_3$ | CH | |
| L-2 | 1 | C≡C-Ph | H | H | - | $CH_3$ | $CH_3$ | CH | |
| L-2 | 1 | C≡C-Ph | H | H | - | $CH_3$ | $OCH_3$ | CH | |
| L-2 | 1 | C≡C-Ph | H | H | - | $OCH_3$ | $OCH_3$ | CH | |
| L-2 | 1 | C≡C-Ph | H | H | - | $CH_3$ | $CH_3$ | N | |
| L-2 | 1 | C≡C-Ph | H | H | - | $CH_3$ | $OCH_3$ | N | |
| L-2 | 1 | C≡C-Ph | H | H | - | $OCH_3$ | $OCH_3$ | N | |
| L-2 | 1 | C≡C-Ph | H | H | - | Cl | $OCH_3$ | CH | |
| L-2 | 1 | CH=$CH_2$ | $CH_3$ | H | - | $CH_3$ | $CH_3$ | CH | |
| L-2 | 1 | CH=$CH_2$ | $CH_3$ | H | - | $CH_3$ | $OCH_3$ | CH | |
| L-2 | 1 | CH=$CH_2$ | $CH_3$ | H | - | $OCH_3$ | $OCH_3$ | CH | |
| L-2 | 1 | CH=$CH_2$ | $CH_3$ | H | - | $CH_3$ | $CH_3$ | N | |
| L-2 | 1 | CH=$CH_2$ | $CH_3$ | H | - | $CH_3$ | $OCH_3$ | N | |
| L-2 | 1 | CH=$CH_2$ | $CH_3$ | H | - | $OCH_3$ | $OCH_3$ | N | |
| L-2 | 1 | CH=$CH_2$ | $CH_3$ | H | - | Cl | $OCH_3$ | CH | |
| L-2 | 1 | C≡CH | H | H | - | $CH_3$ | $CH_3$ | CH | |
| L-2 | 1 | C≡CH | H | H | - | $CH_3$ | $OCH_3$ | CH | |
| L-2 | 1 | C≡CH | H | H | - | $OCH_3$ | $OCH_3$ | CH | |
| L-2 | 1 | C≡CH | H | H | - | $CH_3$ | $CH_3$ | N | |
| L-2 | 1 | C≡CH | H | H | - | $CH_3$ | $OCH_3$ | N | |
| L-2 | 1 | C≡CH | H | H | - | $OCH_3$ | $OCH_3$ | N | |
| L-2 | 1 | C≡CH | H | H | - | Cl | $OCH_3$ | CH | |
| L-2 | 1 | CH=$CH_2$ | H | $CH_3$ | - | $CH_3$ | $CH_3$ | CH | |
| L-2 | 1 | CH=$CH_2$ | H | $CH_3$ | - | $CH_3$ | $OCH_3$ | CH | |
| L-2 | 1 | CH=$CH_2$ | H | $CH_3$ | - | $OCH_3$ | $OCH_3$ | CH | |
| L-2 | 1 | CH=$CH_2$ | H | $CH_3$ | - | $CH_3$ | $CH_3$ | N | |
| L-2 | 1 | CH=$CH_2$ | H | $CH_3$ | - | $CH_3$ | $OCH_3$ | N | |

Table VIIIb (Continued)

| L | n | W | R | $R_1$ | $R_2$ | X | Y | Z | m.p. (°C) |
|---|---|---|---|---|---|---|---|---|---|
| L-2 | 1 | $CH=CH_2$ | H | $CH_3$ | - | $OCH_3$ | $OCH_3$ | N | |
| L-2 | 1 | $CH=CH_2$ | H | $CH_3$ | - | Cl | $OCH_3$ | CH | |
| L-2 | 1 | C≡CH | H | $CH_3$ | - | $OCH_3$ | $OCH_3$ | CH | |
| L-2 | 1 | C≡CH | H | $CH_3$ | - | $OCH_3$ | $OCH_3$ | N | |
| L-2 | 1 | C≡CH | H | $CH_3$ | - | $OCH_3$ | $CH_3$ | CH | |
| L-3 | 0 | $CH=CH_2$ | H | H | $CH_3$ | $CH_3$ | $CH_3$ | CH | |
| L-3 | 0 | $CH=CH_2$ | H | H | $CH_3$ | $CH_3$ | $OCH_3$ | CH | |
| L-3 | 0 | $CH=CH_2$ | H | H | $CH_3$ | $OCH_3$ | $OCH_3$ | CH | |
| L-3 | 0 | $CH=CH_2$ | H | H | $CH_3$ | $CH_3$ | $CH_3$ | N | |
| L-3 | 0 | $CH=CH_2$ | H | H | $CH_3$ | $CH_3$ | $OCH_3$ | N | |
| L-3 | 0 | $CH=CH_2$ | H | H | $CH_3$ | $OCH_3$ | $OCH_3$ | N | |
| L-3 | 0 | $CH=CH_2$ | H | H | $CH_3$ | Cl | $OCH_3$ | CH | |
| L-3 | 0 | $C(CH_3)=CHCH_3$ | H | H | $CH_3$ | $CH_3$ | $CH_3$ | CH | |
| L-3 | 0 | $C(CH_3)=CHCH_3$ | H | H | $CH_3$ | $CH_3$ | $OCH_3$ | CH | |
| L-3 | 0 | $C(CH_3)=CHCH_3$ | H | H | $CH_3$ | $OCH_3$ | $OCH_3$ | CH | |
| L-3 | 0 | $C(CH_3)=CHCH_3$ | H | H | $CH_3$ | $CH_3$ | $CH_3$ | N | |
| L-3 | 0 | $C(CH_3)=CHCH_3$ | H | H | $CH_3$ | $CH_3$ | $OCH_3$ | N | |
| L-3 | 0 | $C(CH_3)=CHCH_3$ | H | H | $CH_3$ | $OCH_3$ | $OCH_3$ | N | |
| L-3 | 0 | $C(CH_3)=CHCH_3$ | H | H | $CH_3$ | Cl | $OCH_3$ | CH | |
| L-3 | 0 | $CH=CHCH_2CH_3$ | H | H | $CH_3$ | $CH_3$ | $CH_3$ | CH | |
| L-3 | 0 | $CH=CHCH_2CH_3$ | H | H | $CH_3$ | $CH_3$ | $OCH_3$ | CH | |
| L-3 | 0 | $CH=CHCH_2CH_3$ | H | H | $CH_3$ | $OCH_3$ | $OCH_3$ | CH | |
| L-3 | 0 | $CH=CHCH_2CH_3$ | H | H | $CH_3$ | $CH_3$ | $CH_3$ | N | |
| L-3 | 0 | $CH=CHCH_2CH_3$ | H | H | $CH_3$ | $CH_3$ | $OCH_3$ | N | |
| L-3 | 0 | $CH=CHCH_2CH_3$ | H | H | $CH_3$ | $OCH_3$ | $OCH_3$ | N | |
| L-3 | 0 | $CH=CHCH_2CH_3$ | H | H | $CH_3$ | Cl | $OCH_3$ | CH | |
| L-3 | 0 | $C(CH_3)=CHCH_2CH_3$ | H | H | $CH_3$ | $CH_3$ | $CH_3$ | CH | |
| L-3 | 0 | $C(CH_3)=CHCH_2CH_3$ | H | H | $CH_3$ | $CH_3$ | $OCH_3$ | CH | |
| L-3 | 0 | $C(CH_3)=CHCH_2CH_3$ | H | H | $CH_3$ | $OCH_3$ | $OCH_3$ | CH | |
| L-3 | 0 | $C(CH_3)=CHCH_2CH_3$ | H | H | $CH_3$ | $CH_3$ | $CH_3$ | N | |
| L-3 | 0 | $C(CH_3)=CHCH_2CH_3$ | H | H | $CH_3$ | $CH_3$ | $OCH_3$ | N | |

Table VIIIb (Continued)

| L | n | W | R | $R_1$ | $R_2$ | X | Y | Z | m.p. (°C) |
|---|---|---|---|---|---|---|---|---|---|
| L-3 | 0 | $C(CH_3)=CHCH_2CH_3$ | H | H | $CH_3$ | $OCH_3$ | $OCH_3$ | N | |
| L-3 | 0 | $C(CH_3)=CHCH_2CH_3$ | H | H | $CH_3$ | Cl | $OCH_3$ | CH | |
| L-3 | 0 | $CH=CHCH_2CH_2CH_3$ | H | H | $CH_3$ | $CH_3$ | $CH_3$ | CH | |
| L-3 | 0 | $CH=CHCH_2CH_2CH_3$ | H | H | $CH_3$ | $CH_3$ | $OCH_3$ | CH | |
| L-3 | 0 | $CH=CHCH_2CH_2CH_3$ | H | H | $CH_3$ | $OCH_3$ | $OCH_3$ | CH | |
| L-3 | 0 | $CH=CHCH_2CH_2CH_3$ | H | H | $CH_3$ | $CH_3$ | $CH_3$ | N | |
| L-3 | 0 | $CH=CHCH_2CH_2CH_3$ | H | H | $CH_3$ | $CH_3$ | $OCH_3$ | N | |
| L-3 | 0 | $CH=CHCH_2CH_2CH_3$ | H | H | $CH_3$ | $OCH_3$ | $OCH_3$ | N | |
| L-3 | 0 | $CH=CHCH_2CH_2CH_3$ | H | H | $CH_3$ | Cl | $OCH_3$ | CH | |
| L-3 | 0 | CH=CH-CN | H | H | $CH_3$ | $CH_3$ | $CH_3$ | CH | |
| L-3 | 0 | CH=CH-CN | H | H | $CH_3$ | $CH_3$ | $OCH_3$ | CH | |
| L-3 | 0 | CH=CH-CN | H | H | $CH_3$ | $OCH_3$ | $OCH_3$ | CH | |
| L-3 | 0 | CH=CH-CN | H | H | $CH_3$ | $CH_3$ | $CH_3$ | N | |
| L-3 | 0 | CH=CH-CN | H | H | $CH_3$ | $CH_3$ | $OCH_3$ | N | |
| L-3 | 0 | CH=CH-CN | H | H | $CH_3$ | $OCH_3$ | $OCH_3$ | N | |
| L-3 | 0 | CH=CH-CN | H | H | $CH_3$ | Cl | $OCH_3$ | CH | |
| L-3 | 0 | $CH=CH-OCH_3$ | H | H | $CH_3$ | $CH_3$ | $CH_3$ | CH | |
| L-3 | 0 | $CH=CH-OCH_3$ | H | H | $CH_3$ | $CH_3$ | $OCH_3$ | CH | |
| L-3 | 0 | $CH=CH-OCH_3$ | H | H | $CH_3$ | $OCH_3$ | $OCH_3$ | CH | |
| L-3 | 0 | $CH=CH-OCH_3$ | H | H | $CH_3$ | $CH_3$ | $CH_3$ | N | |
| L-3 | 0 | $CH=CH-OCH_3$ | H | H | $CH_3$ | $CH_3$ | $OCH_3$ | N | |
| L-3 | 0 | $CH=CH-OCH_3$ | H | H | $CH_3$ | $OCH_3$ | $OCH_3$ | N | |
| L-3 | 0 | $CH=CH-OCH_3$ | H | H | $CH_3$ | Cl | $OCH_3$ | CH | |
| L-3 | 0 | CH=CH-Br | H | H | $CH_3$ | $CH_3$ | $CH_3$ | CH | |
| L-3 | 0 | CH=CH-Br | H | H | $CH_3$ | $CH_3$ | $OCH_3$ | CH | |
| L-3 | 0 | CH=CH-Br | H | H | $CH_3$ | $OCH_3$ | $OCH_3$ | CH | |
| L-3 | 0 | CH=CH-Br | H | H | $CH_3$ | $CH_3$ | $CH_3$ | N | |
| L-3 | 0 | CH=CH-Br | H | H | $CH_3$ | $CH_3$ | $OCH_3$ | N | |
| L-3 | 0 | CH=CH-Br | H | H | $CH_3$ | $OCH_3$ | $OCH_3$ | N | |
| L-3 | 0 | CH=CH-Br | H | H | $CH_3$ | Cl | $OCH_3$ | CH | |
| L-3 | 0 | $CH=CH-CF_3$ | H | H | $CH_3$ | $CH_3$ | $CH_3$ | CH | |

Table VIIIb (Continued)

| L | n | W | R | $R_1$ | $R_2$ | X | Y | Z | m.p. (°C) |
|---|---|---|---|---|---|---|---|---|---|
| L-3 | 0 | $CH=CH-CF_3$ | H | H | $CH_3$ | $CH_3$ | $OCH_3$ | CH | |
| L-3 | 0 | $CH=CH-CF_3$ | H | H | $CH_3$ | $OCH_3$ | $OCH_3$ | CH | |
| L-3 | 0 | $CH=CH-CF_3$ | H | H | $CH_3$ | $CH_3$ | $CH_3$ | N | |
| L-3 | 0 | $CH=CH-CF_3$ | H | H | $CH_3$ | $CH_3$ | $OCH_3$ | N | |
| L-3 | 0 | $CH=CH-CF_3$ | H | H | $CH_3$ | $OCH_3$ | $OCH_3$ | N | |
| L-3 | 0 | $CH=CH-CF_3$ | H | H | $CH_3$ | Cl | $OCH_3$ | CH | |
| L-3 | 0 | $C(CH_3)=CH-OCH_3$ | H | H | $CH_3$ | $CH_3$ | $CH_3$ | CH | |
| L-3 | 0 | $C(CH_3)=CH-OCH_3$ | H | H | $CH_3$ | $CH_3$ | $OCH_3$ | CH | |
| L-3 | 0 | $C(CH_3)=CH-OCH_3$ | H | H | $CH_3$ | $OCH_3$ | $OCH_3$ | CH | |
| L-3 | 0 | $C(CH_3)=CH-OCH_3$ | H | H | $CH_3$ | $CH_3$ | $CH_3$ | N | |
| L-3 | 0 | $C(CH_3)=CH-OCH_3$ | H | H | $CH_3$ | $CH_3$ | $OCH_3$ | N | |
| L-3 | 0 | $C(CH_3)=CH-OCH_3$ | H | H | $CH_3$ | $OCH_3$ | $OCH_3$ | N | |
| L-3 | 0 | $C(CH_3)=CH-OCH_3$ | H | H | $CH_3$ | Cl | $OCH_3$ | CH | |
| L-3 | 0 | CH=CHPh | H | H | $CH_3$ | $CH_3$ | $CH_3$ | CH | |
| L-3 | 0 | CH=CHPh | H | H | $CH_3$ | $CH_3$ | $OCH_3$ | CH | |
| L-3 | 0 | CH=CHPh | H | H | $CH_3$ | $OCH_3$ | $OCH_3$ | CH | |
| L-3 | 0 | CH=CHPh | H | H | $CH_3$ | $CH_3$ | $CH_3$ | N | |
| L-3 | 0 | CH=CHPh | H | H | $CH_3$ | $CH_3$ | $OCH_3$ | N | |
| L-3 | 0 | CH=CHPh | H | H | $CH_3$ | $OCH_3$ | $OCH_3$ | N | |
| L-3 | 0 | CH=CHPh | H | H | $CH_3$ | Cl | $OCH_3$ | CH | |
| L-3 | 0 | $C(CH_3)=CH-Ph$ | H | H | $CH_3$ | $CH_3$ | $CH_3$ | CH | |
| L-3 | 0 | $C(CH_3)=CH-Ph$ | H | H | $CH_3$ | $CH_3$ | $OCH_3$ | CH | |
| L-3 | 0 | $C(CH_3)=CH-Ph$ | H | H | $CH_3$ | $OCH_3$ | $OCH_3$ | CH | |
| L-3 | 0 | $C(CH_3)=CH-Ph$ | H | H | $CH_3$ | $CH_3$ | $CH_3$ | N | |
| L-3 | 0 | $C(CH_3)=CH-Ph$ | H | H | $CH_3$ | $CH_3$ | $OCH_3$ | N | |
| L-3 | 0 | $C(CH_3)=CH-Ph$ | H | H | $CH_3$ | $OCH_3$ | $OCH_3$ | N | |
| L-3 | 0 | $C(CH_3)=CH-Ph$ | H | H | $CH_3$ | Cl | $OCH_3$ | CH | |
| L-3 | 0 | $\overline{C=CH-(CH_2)_3}$ | H | H | $CH_3$ | $CH_3$ | $CH_3$ | CH | |
| L-3 | 0 | $\overline{C=CH-(CH_2)_3}$ | H | H | $CH_3$ | $CH_3$ | $OCH_3$ | CH | |
| L-3 | 0 | $\overline{C=CH-(CH_2)_3}$ | H | H | $CH_3$ | $OCH_3$ | $OCH_3$ | CH | |
| L-3 | 0 | $\overline{C=CH-(CH_2)_3}$ | H | H | $CH_3$ | $CH_3$ | $CH_3$ | N | |

Table VIIIb (Continued)

| L | n | W | R | $R_1$ | $R_2$ | X | Y | Z | m.p. (°C) |
|---|---|---|---|---|---|---|---|---|---|
| L-3 | 0 | $\overline{C=CH-(CH_2)}_3$ | H | H | $CH_3$ | $CH_3$ | $OCH_3$ | N | |
| L-3 | 0 | $\overline{C=CH-(CH_2)}_3$ | H | H | $CH_3$ | $OCH_3$ | $OCH_3$ | N | |
| L-3 | 0 | $\overline{C=CH-(CH_2)}_3$ | H | H | $CH_3$ | Cl | $OCH_3$ | CH | |
| L-3 | 0 | $\overline{C=CH-(CH_2)}_4$ | H | H | $CH_3$ | $CH_3$ | $CH_3$ | CH | |
| L-3 | 0 | $\overline{C=CH-(CH_2)}_4$ | H | H | $CH_3$ | $CH_3$ | $OCH_3$ | CH | |
| L-3 | 0 | $\overline{C=CH-(CH_2)}_4$ | H | H | $CH_3$ | $OCH_3$ | $OCH_3$ | CH | |
| L-3 | 0 | $\overline{C=CH-(CH_2)}_4$ | H | H | $CH_3$ | $CH_3$ | $CH_3$ | N | |
| L-3 | 0 | $\overline{C=CH-(CH_2)}_4$ | H | H | $CH_3$ | $CH_3$ | $OCH_3$ | N | |
| L-3 | 0 | $\overline{C=CH-(CH_2)}_4$ | H | H | $CH_3$ | $OCH_3$ | $OCH_3$ | N | |
| L-3 | 0 | $\overline{C=CH-(CH_2)}_4$ | H | H | $CH_3$ | Cl | $OCH_3$ | CH | |
| L-3 | 0 | $\overline{C=CH-(CH_2)}_5$ | H | H | $CH_3$ | $CH_3$ | $CH_3$ | CH | |
| L-3 | 0 | $\overline{C=CH-(CH_2)}_5$ | H | H | $CH_3$ | $CH_3$ | $OCH_3$ | CH | |
| L-3 | 0 | $\overline{C=CH-(CH_2)}_5$ | H | H | $CH_3$ | $OCH_3$ | $OCH_3$ | CH | |
| L-3 | 0 | $\overline{C=CH-(CH_2)}_5$ | H | H | $CH_3$ | $CH_3$ | $CH_3$ | N | |
| L-3 | 0 | $\overline{C=CH-(CH_2)}_5$ | H | H | $CH_3$ | $CH_3$ | $OCH_3$ | N | |
| L-3 | 0 | $\overline{C=CH-(CH_2)}_5$ | H | H | $CH_3$ | $OCH_3$ | $OCH_3$ | N | |
| L-3 | 0 | $\overline{C=CH-(CH_2)}_5$ | H | H | $CH_3$ | Cl | $OCH_3$ | CH | |
| L-3 | 0 | C(CN)=CHCN | H | H | $CH_3$ | $CH_3$ | $CH_3$ | CH | |
| L-3 | 0 | C(CN)=CHCN | H | H | $CH_3$ | $CH_3$ | $OCH_3$ | CH | |
| L-3 | 0 | C(CN)=CHCN | H | H | $CH_3$ | $OCH_3$ | $OCH_3$ | CH | |
| L-3 | 0 | C(CN)=CHCN | H | H | $CH_3$ | $CH_3$ | $CH_3$ | N | |
| L-3 | 0 | C(CN)=CHCN | H | H | $CH_3$ | $CH_3$ | $OCH_3$ | N | |
| L-3 | 0 | C(CN)=CHCN | H | H | $CH_3$ | $OCH_3$ | $OCH_3$ | N | |
| L-3 | 0 | C(CN)=CHCN | H | H | $CH_3$ | Cl | $OCH_3$ | CH | |
| L-3 | 0 | $CH=CH-SO_2N(CH_3)_2$ | H | H | $CH_3$ | $CH_3$ | $CH_3$ | CH | |
| L-3 | 0 | $CH=CH-SO_2N(CH_3)_2$ | H | H | $CH_3$ | $CH_3$ | $OCH_3$ | CH | |
| L-3 | 0 | $CH=CH-SO_2N(CH_3)_2$ | H | H | $CH_3$ | $OCH_3$ | $OCH_3$ | CH | |

Table VIIIb (Continued)

| L | n | W | R | $R_1$ | $R_2$ | X | Y | Z | m.p. (°C) |
|---|---|---|---|---|---|---|---|---|---|
| L-3 | 0 | $CH=CH-SO_2N(CH_3)_2$ | H | H | $CH_3$ | $CH_3$ | $CH_3$ | N | |
| L-3 | 0 | $CH=CH-SO_2N(CH_3)_2$ | H | H | $CH_3$ | $CH_3$ | $OCH_3$ | N | |
| L-3 | 0 | $CH=CH-SO_2N(CH_3)_2$ | H | H | $CH_3$ | $OCH_3$ | $OCH_3$ | N | |
| L-3 | 0 | $CH=CH-SO_2N(CH_3)_2$ | H | H | $CH_3$ | Cl | $OCH_3$ | CH | |
| L-3 | 0 | $\overline{CH=CH-(CH_2)_4}$ | $CH_3$ | H | $CH_3$ | $OCH_3$ | $OCH_3$ | CH | |
| L-3 | 0 | $\overline{CH=CH-(CH_2)_4}$ | H | $CH_3$ | $CH_3$ | $OCH_3$ | $OCH_3$ | CH | |
| L-3 | 0 | $\overline{CH=CH-(CH_3)}$ | H | $CH_3$ | $CH_3$ | $OCH_3$ | $OCH_3$ | CH | |
| L-3 | 0 | $C(=CH_2)-CH_2CH_2CH(NO_2)CH_3$ | H | H | $CH_3$ | $OCH_3$ | $OCH_3$ | CH | |
| L-3 | 0 | $CH=CHCH_2OSO_2Ph$ | H | H | $CH_3$ | $OCH_3$ | $OCH_3$ | CH | |
| L-3 | 0 | $CH=CHCH_2OSO_2CH_3$ | H | H | $CH_3$ | $OCH_3$ | $OCH_3$ | CH | |
| L-3 | 0 | $CH=CHSCH_3$ | H | H | $CH_3$ | $OCH_3$ | $OCH_3$ | CH | |
| L-3 | 0 | C≡CH | H | H | $CH_3$ | $CH_3$ | $CH_3$ | CH | |
| L-3 | 0 | C≡CH | H | H | $CH_3$ | $CH_3$ | $OCH_3$ | CH | |
| L-3 | 0 | C≡CH | H | H | $CH_3$ | $OCH_3$ | $OCH_3$ | CH | |
| L-3 | 0 | C≡CH | H | H | $CH_3$ | $CH_3$ | $CH_3$ | N | |
| L-3 | 0 | C≡CH | H | H | $CH_3$ | $CH_3$ | $OCH_3$ | N | |
| L-3 | 0 | C≡CH | H | H | $CH_3$ | $OCH_3$ | $OCH_3$ | N | |
| L-3 | 0 | C≡CH | H | H | $CH_3$ | Cl | $OCH_3$ | CH | |
| L-3 | 0 | C≡C-Ph | H | H | $CH_3$ | $CH_3$ | $CH_3$ | CH | |
| L-3 | 0 | C≡C-Ph | H | H | $CH_3$ | $CH_3$ | $OCH_3$ | CH | |
| L-3 | 0 | C≡C-Ph | H | H | $CH_3$ | $OCH_3$ | $OCH_3$ | CH | |
| L-3 | 0 | C≡C-Ph | H | H | $CH_3$ | $CH_3$ | $CH_3$ | N | |
| L-3 | 0 | C≡C-Ph | H | H | $CH_3$ | $CH_3$ | $OCH_3$ | N | |
| L-3 | 0 | C≡C-Ph | H | H | $CH_3$ | $OCH_3$ | $OCH_3$ | N | |
| L-3 | 0 | C≡C-Ph | H | H | $CH_3$ | Cl | $OCH_3$ | CH | |
| L-3 | 0 | $C≡C-CH_3$ | H | H | $CH_3$ | $CH_3$ | $CH_3$ | CH | |

Table VIIIb (Continued)

| L | n | W | R | $R_1$ | $R_2$ | X | Y | Z | m.p. (°C) |
|---|---|---|---|---|---|---|---|---|---|
| L-3 | 0 | $C{\equiv}C-CH_3$ | H | H | $CH_3$ | $CH_3$ | $OCH_3$ | CH | |
| L-3 | 0 | $C{\equiv}C-CH_3$ | H | H | $CH_3$ | $OCH_3$ | $OCH_3$ | CH | |
| L-3 | 0 | $C{\equiv}C-CH_3$ | H | H | $CH_3$ | $CH_3$ | $CH_3$ | N | |
| L-3 | 0 | $C{\equiv}C-CH_3$ | H | H | $CH_3$ | $CH_3$ | $OCH_3$ | N | |
| L-3 | 0 | $C{\equiv}C-CH_3$ | H | H | $CH_3$ | $OCH_3$ | $OCH_3$ | N | |
| L-3 | 0 | $C{\equiv}C-CH_3$ | H | H | $CH_3$ | Cl | $OCH_3$ | CH | |
| L-3 | 0 | $C{\equiv}CCH_2OC(O)CH_3$ | H | H | $CH_3$ | $CH_3$ | $CH_3$ | CH | |
| L-3 | 0 | $C{\equiv}CCH_2OC(O)CH_3$ | H | H | $CH_3$ | $CH_3$ | $OCH_3$ | CH | |
| L-3 | 0 | $C{\equiv}CCH_2OC(O)CH_3$ | H | H | $CH_3$ | $OCH_3$ | $OCH_3$ | CH | |
| L-3 | 0 | $C{\equiv}CCH_2OC(O)CH_3$ | H | H | $CH_3$ | $CH_3$ | $CH_3$ | N | |
| L-3 | 0 | $C{\equiv}CCH_2OC(O)CH_3$ | H | H | $CH_3$ | $CH_3$ | $OCH_3$ | N | |
| L-3 | 0 | $C{\equiv}CCH_2OC(O)CH_3$ | H | H | $CH_3$ | $OCH_3$ | $OCH_3$ | N | |
| L-3 | 0 | $C{\equiv}CCH_2OC(O)CH_3$ | H | H | $CH_3$ | Cl | $OCH_3$ | CH | |
| L-3 | 0 | $C{\equiv}CCH_2OH$ | H | H | $CH_3$ | $CH_3$ | $CH_3$ | CH | |
| L-3 | 0 | $C{\equiv}CCH_2OH$ | H | H | $CH_3$ | $CH_3$ | $OCH_3$ | CH | |
| L-3 | 0 | $C{\equiv}CCH_2OH$ | H | H | $CH_3$ | $OCH_3$ | $OCH_3$ | CH | |
| L-3 | 0 | $C{\equiv}CCH_2OH$ | H | H | $CH_3$ | $CH_3$ | $CH_3$ | N | |
| L-3 | 0 | $C{\equiv}CCH_2OH$ | H | H | $CH_3$ | $CH_3$ | $OCH_3$ | N | |
| L-3 | 0 | $C{\equiv}CCH_2OH$ | H | H | $CH_3$ | $OCH_3$ | $OCH_3$ | N | |
| L-3 | 0 | $C{\equiv}CCH_2OH$ | H | H | $CH_3$ | Cl | $OCH_3$ | CH | |
| L-3 | 0 | $C{\equiv}CCH_2Cl$ | H | H | $CH_3$ | $CH_3$ | $CH_3$ | CH | |
| L-3 | 0 | $C{\equiv}CCH_2Cl$ | H | H | $CH_3$ | $CH_3$ | $OCH_3$ | CH | |
| L-3 | 0 | $C{\equiv}CCH_2Cl$ | H | H | $CH_3$ | $OCH_3$ | $OCH_3$ | CH | |
| L-3 | 0 | $C{\equiv}CCH_2Cl$ | H | H | $CH_3$ | $CH_3$ | $CH_3$ | N | |
| L-3 | 0 | $C{\equiv}CCH_2Cl$ | H | H | $CH_3$ | $CH_3$ | $OCH_3$ | N | |
| L-3 | 0 | $C{\equiv}CCH_2Cl$ | H | H | $CH_3$ | $OCH_3$ | $OCH_3$ | N | |
| L-3 | 0 | $C{\equiv}CCH_2Cl$ | H | H | $CH_3$ | Cl | $OCH_3$ | CH | |
| L-3 | 0 | $C{\equiv}C-CH_2Br$ | H | H | $CH_3$ | $CH_3$ | $CH_3$ | CH | |
| L-3 | 0 | $C{\equiv}C-CH_2Br$ | H | H | $CH_3$ | $CH_3$ | $OCH_3$ | CH | |
| L-3 | 0 | $C{\equiv}C-CH_2Br$ | H | H | $CH_3$ | $OCH_3$ | $OCH_3$ | CH | |
| L-3 | 0 | $C{\equiv}C-CH_2Br$ | H | H | $CH_3$ | $CH_3$ | $CH_3$ | N | |
| L-3 | 0 | $C{\equiv}C-CH_2Br$ | H | H | $CH_3$ | $CH_3$ | $OCH_3$ | N | |

Table VIIIb (Continued)

| L | n | W | R | $R_1$ | $R_2$ | X | Y | Z | m.p. (°C) |
|---|---|---|---|---|---|---|---|---|---|
| L-3 | 0 | $C\equiv C-CH_2Br$ | H | H | $CH_3$ | $OCH_3$ | $OCH_3$ | N | |
| L-3 | 0 | $C\equiv C-CH_2Br$ | H | H | $CH_3$ | Cl | $OCH_3$ | CH | |
| L-3 | 0 | $C\equiv C-CH_2CN$ | H | H | $CH_3$ | $CH_3$ | $CH_3$ | CH | |
| L-3 | 0 | $C\equiv C-CH_2CN$ | H | H | $CH_3$ | $CH_3$ | $OCH_3$ | CH | |
| L-3 | 0 | $C\equiv C-CH_2CN$ | H | H | $CH_3$ | $OCH_3$ | $OCH_3$ | CH | |
| L-3 | 0 | $C\equiv C-CH_2CN$ | H | H | $CH_3$ | $CH_3$ | $CH_3$ | N | |
| L-3 | 0 | $C\equiv C-CH_2CN$ | H | H | $CH_3$ | $CH_3$ | $OCH_3$ | N | |
| L-3 | 0 | $C\equiv C-CH_2CN$ | H | H | $CH_3$ | $OCH_3$ | $OCH_3$ | N | |
| L-3 | 0 | $C\equiv C-CH_2CN$ | H | H | $CH_3$ | Cl | $OCH_3$ | CH | |
| L-3 | 0 | $C\equiv C-CH_2OCH_3$ | H | H | $CH_3$ | $CH_3$ | $CH_3$ | CH | |
| L-3 | 0 | $C\equiv C-CH_2OCH_3$ | H | H | $CH_3$ | $CH_3$ | $OCH_3$ | CH | |
| L-3 | 0 | $C\equiv C-CH_2OCH_3$ | H | H | $CH_3$ | $OCH_3$ | $OCH_3$ | CH | |
| L-3 | 0 | $C\equiv C-CH_2OCH_3$ | H | H | $CH_3$ | $CH_3$ | $CH_3$ | N | |
| L-3 | 0 | $C\equiv C-CH_2OCH_3$ | H | H | $CH_3$ | $CH_3$ | $OCH_3$ | N | |
| L-3 | 0 | $C\equiv C-CH_2OCH_3$ | H | H | $CH_3$ | $OCH_3$ | $OCH_3$ | N | |
| L-3 | 0 | $C\equiv C-CH_2OCH_3$ | H | H | $CH_3$ | Cl | $OCH_3$ | CH | |
| L-3 | 0 | $C\equiv CCH_2OSO_2CH_3$ | H | H | $CH_3$ | $CH_3$ | $CH_3$ | CH | |
| L-3 | 0 | $C\equiv CCH_2OSO_2CH_3$ | H | H | $CH_3$ | $CH_3$ | $OCH_3$ | CH | |
| L-3 | 0 | $C\equiv CCH_2OSO_2CH_3$ | H | H | $CH_3$ | $OCH_3$ | $OCH_3$ | CH | |
| L-3 | 0 | $C\equiv CCH_2OSO_2CH_3$ | H | H | $CH_3$ | $CH_3$ | $CH_3$ | N | |
| L-3 | 0 | $C\equiv CCH_2OSO_2CH_3$ | H | H | $CH_3$ | $CH_3$ | $OCH_3$ | N | |
| L-3 | 0 | $C\equiv CCH_2OSO_2CH_3$ | H | H | $CH_3$ | $OCH_3$ | $OCH_3$ | N | |
| L-3 | 0 | $C\equiv CCH_2OSO_2CH_3$ | H | H | $CH_3$ | Cl | $OCH_3$ | CH | |
| L-3 | 0 | $C\equiv CCH_2SCH_3$ | H | H | $CH_3$ | $CH_3$ | $CH_3$ | CH | |
| L-3 | 0 | $C\equiv CCH_2SCH_3$ | H | H | $CH_3$ | $CH_3$ | $OCH_3$ | CH | |
| L-3 | 0 | $C\equiv CCH_2SCH_3$ | H | H | $CH_3$ | $OCH_3$ | $OCH_3$ | CH | |
| L-3 | 0 | $C\equiv CCH_2SCH_3$ | H | H | $CH_3$ | $CH_3$ | $CH_3$ | N | |
| L-3 | 0 | $C\equiv CCH_2SCH_3$ | H | H | $CH_3$ | $CH_3$ | $OCH_3$ | N | |
| L-3 | 0 | $C\equiv CCH_2SCH_3$ | H | H | $CH_3$ | $OCH_3$ | $OCH_3$ | N | |
| L-3 | 0 | $C\equiv CCH_2SCH_3$ | H | H | $CH_3$ | Cl | $OCH_3$ | CH | |
| L-3 | 1 | $HC=CH_2$ | H | H | $CH_3$ | $CH_3$ | $CH_3$ | CH | |
| L-3 | 1 | $HC=CH_2$ | H | H | $CH_3$ | $CH_3$ | $OCH_3$ | CH | |
| L-3 | 1 | $HC=CH_2$ | H | H | $CH_3$ | $OCH_3$ | $OCH_3$ | CH | |

Table VIIIb (Continued)

| L | n | W | R | $R_1$ | $R_2$ | X | Y | Z | m.p. (°C) |
|---|---|---|---|---|---|---|---|---|---|
| L-3 | 1 | $HC=CH_2$ | H | H | $CH_3$ | $CH_3$ | $CH_3$ | N | |
| L-3 | 1 | $HC=CH_2$ | H | H | $CH_3$ | $CH_3$ | $OCH_3$ | N | |
| L-3 | 1 | $HC=CH_2$ | H | H | $CH_3$ | $OCH_3$ | $OCH_3$ | N | |
| L-3 | 1 | $HC=CH_2$ | H | H | $CH_3$ | Cl | $OCH_3$ | CH | |
| L-3 | 1 | $BrC=CH_2$ | H | H | $CH_3$ | $CH_3$ | $CH_3$ | CH | |
| L-3 | 1 | $BrC=CH_2$ | H | H | $CH_3$ | $CH_3$ | $OCH_3$ | CH | |
| L-3 | 1 | $BrC=CH_2$ | H | H | $CH_3$ | $OCH_3$ | $OCH_3$ | CH | |
| L-3 | 1 | $BrC=CH_2$ | H | H | $CH_3$ | $CH_3$ | $CH_3$ | N | |
| L-3 | 1 | $BrC=CH_2$ | H | H | $CH_3$ | $CH_3$ | $OCH_3$ | N | |
| L-3 | 1 | $BrC=CH_2$ | H | H | $CH_3$ | $OCH_3$ | $OCH_3$ | N | |
| L-3 | 1 | $BrC=CH_2$ | H | H | $CH_3$ | Cl | $OCH_3$ | CH | |
| L-3 | 1 | $HC=C(CH_3)_2$ | H | H | $CH_3$ | $CH_3$ | $CH_3$ | CH | |
| L-3 | 1 | $HC=C(CH_3)_2$ | H | H | $CH_3$ | $CH_3$ | $OCH_3$ | CH | |
| L-3 | 1 | $HC=C(CH_3)_2$ | H | H | $CH_3$ | $OCH_3$ | $OCH_3$ | CH | |
| L-3 | 1 | $HC=C(CH_3)_2$ | H | H | $CH_3$ | $CH_3$ | $CH_3$ | N | |
| L-3 | 1 | $HC=C(CH_3)_2$ | H | H | $CH_3$ | $CH_3$ | $OCH_3$ | N | |
| L-3 | 1 | $HC=C(CH_3)_2$ | H | H | $CH_3$ | $OCH_3$ | $OCH_3$ | N | |
| L-3 | 1 | $HC=C(CH_3)_2$ | H | H | $CH_3$ | Cl | $OCH_3$ | CH | |
| L-3 | 1 | $HC=C(CH_3)CH_2CH=C(CH_3)_2$ | H | H | $CH_3$ | $CH_3$ | $CH_3$ | CH | |
| L-3 | 1 | $HC=C(CH_3)CH_2CH=C(CH_3)_2$ | H | H | $CH_3$ | $CH_3$ | $OCH_3$ | CH | |
| L-3 | 1 | $HC=C(CH_3)CH_2CH=C(CH_3)_2$ | H | H | $CH_3$ | $OCH_3$ | $OCH_3$ | CH | |
| L-3 | 1 | $HC=C(CH_3)CH_2CH=C(CH_3)_2$ | H | H | $CH_3$ | $CH_3$ | $CH_3$ | N | |
| L-3 | 1 | $HC=C(CH_3)CH_2CH=C(CH_3)_2$ | H | H | $CH_3$ | $CH_3$ | $OCH_3$ | N | |
| L-3 | 1 | $HC=C(CH_3)CH_2CH=C(CH_3)_2$ | H | H | $CH_3$ | $OCH_3$ | $OCH_3$ | N | |
| L-3 | 1 | $HC=C(CH_3)CH_2CH=C(CH_3)_2$ | H | H | $CH_3$ | Cl | $OCH_3$ | CH | |
| L-3 | 1 | HC=CH-Ph | H | H | $CH_3$ | $CH_3$ | $CH_3$ | CH | |
| L-3 | 1 | HC=CH-Ph | H | H | $CH_3$ | $CH_3$ | $OCH_3$ | CH | |
| L-3 | 1 | HC=CH-Ph | H | H | $CH_3$ | $OCH_3$ | $OCH_3$ | CH | |
| L-3 | 1 | HC=CH-Ph | H | H | $CH_3$ | $CH_3$ | $CH_3$ | N | |
| L-3 | 1 | HC=CH-Ph | H | H | $CH_3$ | $CH_3$ | $OCH_3$ | N | |
| L-3 | 1 | HC=CH-Ph | H | H | $CH_3$ | $OCH_3$ | $OCH_3$ | N | |

Table VIIIb (Continued)

| L | n | W | R | $R_1$ | $R_2$ | X | Y | Z | m.p. (°C) |
|---|---|---|---|---|---|---|---|---|---|
| L-3 | 1 | HC=CH-Ph | H | H | $CH_3$ | Cl | $OCH_3$ | CH | |
| L-3 | 1 | $HC=CHCH_3$ | H | H | $CH_3$ | $CH_3$ | $CH_3$ | CH | |
| L-3 | 1 | $HC=CHCH_3$ | H | H | $CH_3$ | $CH_3$ | $OCH_3$ | CH | |
| L-3 | 1 | $HC=CHCH_3$ | H | H | $CH_3$ | $OCH_3$ | $OCH_3$ | CH | |
| L-3 | 1 | $HC=CHCH_3$ | H | H | $CH_3$ | $CH_3$ | $CH_3$ | N | |
| L-3 | 1 | $HC=CHCH_3$ | H | H | $CH_3$ | $CH_3$ | $OCH_3$ | N | |
| L-3 | 1 | $HC=CHCH_3$ | H | H | $CH_3$ | $OCH_3$ | $OCH_3$ | N | |
| L-3 | 1 | $HC=CHCH_3$ | H | H | $CH_3$ | Cl | $OCH_3$ | CH | |
| L-3 | 1 | $HC=CHCH_2Br$ | H | H | $CH_3$ | $CH_3$ | $CH_3$ | CH | |
| L-3 | 1 | $HC=CHCH_2Br$ | H | H | $CH_3$ | $CH_3$ | $OCH_3$ | CH | |
| L-3 | 1 | $HC=CHCH_2Br$ | H | H | $CH_3$ | $OCH_3$ | $OCH_3$ | CH | |
| L-3 | 1 | $HC=CHCH_2Br$ | H | H | $CH_3$ | $CH_3$ | $CH_3$ | N | |
| L-3 | 1 | $HC=CHCH_2Br$ | H | H | $CH_3$ | $CH_3$ | $OCH_3$ | N | |
| L-3 | 1 | $HC=CHCH_2Br$ | H | H | $CH_3$ | $OCH_3$ | $OCH_3$ | N | |
| L-3 | 1 | $HC=CHCH_2Br$ | H | H | $CH_3$ | Cl | $OCH_3$ | CH | |
| L-3 | 1 | C≡CH | H | H | $CH_3$ | $CH_3$ | $CH_3$ | CH | |
| L-3 | 1 | C≡CH | H | H | $CH_3$ | $CH_3$ | $OCH_3$ | CH | |
| L-3 | 1 | C≡CH | H | H | $CH_3$ | $OCH_3$ | $OCH_3$ | CH | |
| L-3 | 1 | C≡CH | H | H | $CH_3$ | $CH_3$ | $CH_3$ | N | |
| L-3 | 1 | C≡CH | H | H | $CH_3$ | $CH_3$ | $OCH_3$ | N | |
| L-3 | 1 | C≡CH | H | H | $CH_3$ | $OCH_3$ | $OCH_3$ | N | |
| L-3 | 1 | C≡CH | H | H | $CH_3$ | Cl | $OCH_3$ | CH | |
| L-3 | 1 | $C≡CCH_3$ | H | H | $CH_3$ | $CH_3$ | $CH_3$ | CH | |
| L-3 | 1 | $C≡CCH_3$ | H | H | $CH_3$ | $CH_3$ | $OCH_3$ | CH | |
| L-3 | 1 | $C≡CCH_3$ | H | H | $CH_3$ | $OCH_3$ | $OCH_3$ | CH | |
| L-3 | 1 | $C≡CCH_3$ | H | H | $CH_3$ | $CH_3$ | $CH_3$ | N | |
| L-3 | 1 | $C≡CCH_3$ | H | H | $CH_3$ | $CH_3$ | $OCH_3$ | N | |
| L-3 | 1 | $C≡CCH_3$ | H | H | $CH_3$ | $OCH_3$ | $OCH_3$ | N | |
| L-3 | 1 | $C≡CCH_3$ | H | H | $CH_3$ | Cl | $OCH_3$ | CH | |
| L-3 | 1 | $C≡C-CH_2Br$ | H | H | $CH_3$ | $CH_3$ | $CH_3$ | CH | |
| L-3 | 1 | $C≡C-CH_2Br$ | H | H | $CH_3$ | $CH_3$ | $OCH_3$ | CH | |

Table VIIIb (Continued)

| L | n | W | R | $R_1$ | $R_2$ | X | Y | Z | m.p. (°C) |
|---|---|---|---|---|---|---|---|---|---|
| L-3 | 1 | C≡C-$CH_2$Br | H | H | $CH_3$ | $OCH_3$ | $OCH_3$ | CH | |
| L-3 | 1 | C≡C-$CH_2$Br | H | H | $CH_3$ | $CH_3$ | $CH_3$ | N | |
| L-3 | 1 | C≡C-$CH_2$Br | H | H | $CH_3$ | $CH_3$ | $OCH_3$ | N | |
| L-3 | 1 | C≡C-$CH_2$Br | H | H | $CH_3$ | $OCH_3$ | $OCH_3$ | N | |
| L-3 | 1 | C≡C-$CH_2$Br | H | H | $CH_3$ | Cl | $OCH_3$ | CH | |
| L-3 | 1 | C≡C-Ph | H | H | $CH_3$ | $CH_3$ | $CH_3$ | CH | |
| L-3 | 1 | C≡C-Ph | H | H | $CH_3$ | $CH_3$ | $OCH_3$ | CH | |
| L-3 | 1 | C≡C-Ph | H | H | $CH_3$ | $OCH_3$ | $OCH_3$ | CH | |
| L-3 | 1 | C≡C-Ph | H | H | $CH_3$ | $CH_3$ | $CH_3$ | N | |
| L-3 | 1 | C≡C-Ph | H | H | $CH_3$ | $CH_3$ | $OCH_3$ | N | |
| L-3 | 1 | C≡C-Ph | H | H | $CH_3$ | $OCH_3$ | $OCH_3$ | N | |
| L-3 | 1 | C≡C-Ph | H | H | $CH_3$ | Cl | $OCH_3$ | CH | |
| L-3 | 1 | $CH=CH_2$ | $CH_3$ | H | $CH_3$ | $CH_3$ | $CH_3$ | CH | |
| L-3 | 1 | $CH=CH_2$ | $CH_3$ | H | $CH_3$ | $CH_3$ | $OCH_3$ | CH | |
| L-3 | 1 | $CH=CH_2$ | $CH_3$ | H | $CH_3$ | $OCH_3$ | $OCH_3$ | CH | |
| L-3 | 1 | $CH=CH_2$ | $CH_3$ | H | $CH_3$ | $CH_3$ | $CH_3$ | N | |
| L-3 | 1 | $CH=CH_2$ | $CH_3$ | H | $CH_3$ | $CH_3$ | $OCH_3$ | N | |
| L-3 | 1 | $CH=CH_2$ | $CH_3$ | H | $CH_3$ | $OCH_3$ | $OCH_3$ | N | |
| L-3 | 1 | $CH=CH_2$ | $CH_3$ | H | $CH_3$ | Cl | $OCH_3$ | CH | |
| L-3 | 1 | C≡CH | H | H | $CH_3$ | $CH_3$ | $CH_3$ | CH | |
| L-3 | 1 | C≡CH | H | H | $CH_3$ | $CH_3$ | $OCH_3$ | CH | |
| L-3 | 1 | C≡CH | H | H | $CH_3$ | $OCH_3$ | $OCH_3$ | CH | |
| L-3 | 1 | C≡CH | H | H | $CH_3$ | $CH_3$ | $CH_3$ | N | |
| L-3 | 1 | C≡CH | H | H | $CH_3$ | $CH_3$ | $OCH_3$ | N | |
| L-3 | 1 | C≡CH | H | H | $CH_3$ | $OCH_3$ | $OCH_3$ | N | |
| L-3 | 1 | C≡CH | H | H | $CH_3$ | Cl | $OCH_3$ | CH | |
| L-3 | 1 | $CH=CH_2$ | H | $CH_3$ | $CH_3$ | $CH_3$ | $CH_3$ | CH | |
| L-3 | 1 | $CH=CH_2$ | H | $CH_3$ | $CH_3$ | $CH_3$ | $OCH_3$ | CH | |
| L-3 | 1 | $CH=CH_2$ | H | $CH_3$ | $CH_3$ | $OCH_3$ | $OCH_3$ | CH | |
| L-3 | 1 | $CH=CH_2$ | H | $CH_3$ | $CH_3$ | $CH_3$ | $CH_3$ | N | |
| L-3 | 1 | $CH=CH_2$ | H | $CH_3$ | $CH_3$ | $CH_3$ | $OCH_3$ | N | |

Table VIIIb (Continued)

| L | n | W | R | $R_1$ | $R_2$ | X | Y | Z | m.p. (°C) |
|---|---|---|---|---|---|---|---|---|---|
| L-3 | 1 | $CH=CH_2$ | H | $CH_3$ | $CH_3$ | $OCH_3$ | $OCH_3$ | N | |
| L-3 | 1 | $CH=CH_2$ | H | $CH_3$ | $CH_3$ | Cl | $OCH_3$ | CH | |
| L-3 | 1 | C≡CH | H | $CH_3$ | $CH_3$ | $OCH_3$ | $OCH_3$ | CH | |
| L-3 | 1 | C≡CH | H | $CH_3$ | $CH_3$ | $OCH_3$ | $OCH_3$ | N | |
| L-3 | 1 | C≡CH | H | $CH_3$ | $CH_3$ | $OCH_3$ | $CH_3$ | CH | |
| L-2 | 0 | $CH=C(CN)_2$ | H | H | - | $CH_3$ | $CH_3$ | CH | |
| L-2 | 0 | $CH=C(CN)_2$ | H | H | - | $CH_3$ | $OCH_3$ | CH | |
| L-2 | 0 | $CH=C(CN)_2$ | H | H | - | $OCH_3$ | $OCH_3$ | CH | |
| L-2 | 0 | $CH=C(CN)_2$ | H | H | - | $CH_3$ | $OCH_3$ | N | |
| L-2 | 0 | $CH=C(CN)_2$ | H | H | - | $OCH_3$ | $OCH_3$ | N | |
| L-2 | 0 | $CH=C(CN)_2$ | H | H | - | Cl | $OCH_3$ | CH | |
| L-2 | 0 | $CH=CH_2$ | H | H | - | $OCH_2CH_3$ | $CH_3$ | CH | |
| L-2 | 0 | $CH=CH_2$ | H | H | - | $OCH_2CH_3$ | $NHCH_3$ | N | |
| L-2 | 0 | $CH=CH_2$ | H | H | - | $OCH_3$ | $N(CH_3)_2$ | N | |
| L-2 | 0 | $CH=CH_2$ | H | H | - | $OCH_3$ | $CH(CH_3)_2$ | N | |
| L-2 | 0 | $CH=CH_2$ | H | H | - | $OCH_3$ | $CH_2OCH_3$ | CH | |
| L-2 | 0 | $CH=CH_2$ | H | H | - | $CH_3$ | $OCH_2CH_2F$ | N | |
| L-2 | 0 | $CH=CH_2$ | H | H | - | $OCH_3$ | $CH(OCH_3)_2$ | CH | |
| L-2 | 0 | $CH=CH_2$ | H | H | - | Br | $OCH_3$ | CH | |
| L-2 | 0 | $CH=CH_2$ | H | H | - | $OCH_3$ | $OCH_2CF_3$ | N | |
| L-2 | 0 | $CH=CH_2$ | H | H | - | $OCH_3$ | H | CH | |

Table IX

General Structure IX

| A | W | R | $R_1$ | $X_1$ | $Y_1$ | $Y_2$ | $X_2$ | $Y_3$ | $X_3$ | m.p. (°C) |
|---|---|---|---|---|---|---|---|---|---|---|
| A-2 | C≡C-$CH_2$F | H | H | $CH_3$ | O | - | - | - | - | |
| A-2 | C≡C-$CH_2$F | H | H | $CH_3$ | $CH_2$ | - | - | - | - | |
| A-2 | C≡C-$CH_2$F | H | H | $OCH_3$ | O | - | - | - | - | |
| A-2 | C≡C-$CH_2$F | H | H | $OCF_2H$ | O | - | - | - | - | |
| A-3 | C≡C-$CH_2$F | H | H | $CH_3$ | - | - | - | - | - | |
| A-3 | C≡C-$CH_2$F | H | H | $OCH_3$ | - | - | - | - | - | |
| A-3 | C≡C-$CH_2$F | H | H | $OCF_2H$ | - | - | - | - | - | |
| A-4 | C≡C-$CH_2$F | H | H | $CH_3$ | - | H | - | - | - | |
| A-4 | C≡C-$CH_2$F | H | H | $OCH_3$ | - | H | - | - | - | |
| A-4 | C≡C-$CH_2$F | H | H | $CH_3$ | - | $CH_3$ | - | - | - | |
| A-4 | C≡C-$CH_2$F | H | H | $OCH_3$ | - | $CH_3$ | - | - | - | |
| A-5 | C≡C-$CH_2$F | H | H | - | - | - | $CH_3$ | $CH_3$ | - | |
| A-5 | C≡C-$CH_2$F | H | H | - | - | - | $OCH_3$ | $CH_3$ | - | |
| A-5 | C≡C-$CH_2$F | H | H | - | - | - | $CH_3$ | $CH_2CF_3$ | - | |
| A-6 | C≡C-$CH_2$F | H | H | - | - | - | - | - | $CH_3$ | |

Table X

General Structure X

| L | W | A | n | $X_1$ | $Y_1$ | $Y_2$ | $X_2$ | $Y_3$ | $X_3$ | $X_4$ | $Y_4$ | m.p. (°C) |
|---|---|---|---|---|---|---|---|---|---|---|---|---|
| L-2 | $\overline{C=CH(CH_2)_4}$ | A-2 | 0 | $CH_3$ | O | - | - | - | - | - | - | |
| L-2 | $\overline{C=CH(CH_2)_4}$ | A-2 | 0 | $OCH_3$ | O | - | - | - | - | - | - | |
| L-2 | $\overline{C=CH(CH_2)_4}$ | A-2 | 0 | $CH_3$ | CH | - | - | - | - | - | - | |
| L-2 | $\overline{C=CH(CH_2)_4}$ | A-3 | 0 | $OCH_3$ | - | - | - | - | - | - | - | |
| L-2 | $\overline{C=CH(CH_2)_4}$ | A-4 | 0 | $OCH_3$ | - | $CH_3$ | - | - | - | - | - | |
| L-2 | $\overline{C=CH(CH_2)_4}$ | A-5 | 0 | - | - | - | $OCH_3$ | $CH_3$ | - | - | - | |
| L-2 | $\overline{C=CH(CH_2)_4}$ | A-6 | 0 | - | - | - | - | - | $CH_3$ | - | - | |
| L-3 | $\overline{C=CH(CH_2)_4}$ | A-2 | 0 | $CH_3$ | O | - | - | - | - | - | - | |
| L-3 | $\overline{C=CH(CH_2)_4}$ | A-3 | 0 | $OCH_3$ | - | - | - | - | - | - | - | |
| L-3 | $\overline{C=CH(CH_2)_4}$ | A-4 | 0 | $OCH_3$ | - | $CH_3$ | - | - | - | - | - | |
| L-3 | $\overline{C=CH(CH_2)_4}$ | A-5 | 0 | - | - | - | $OCH_3$ | $CH_3$ | - | - | - | |
| L-3 | $\overline{C=CH(CH_2)_4}$ | A-6 | 0 | - | - | - | - | - | $CH_3$ | - | - | |
| L-4 | $\overline{C=CH(CH_2)_4}$ | A-2 | 0 | $CH_3$ | O | - | - | - | - | - | - | |
| L-4 | $\overline{C=CH(CH_2)_4}$ | A-3 | 0 | $OCH_3$ | - | - | - | - | - | - | - | |
| L-4 | $\overline{C=CH(CH_2)_4}$ | A-4 | 0 | $OCH_3$ | - | $CH_3$ | - | - | - | - | - | |
| L-4 | $\overline{C=CH(CH_2)_4}$ | A-5 | 0 | - | - | - | $OCH_3$ | $CH_3$ | - | - | - | |
| L-4 | $\overline{C=CH(CH_2)_4}$ | A-6 | 0 | - | - | - | - | - | $CH_3$ | - | - | |
| L-5 | $\overline{C=CH(CH_2)_4}$ | A-2 | 0 | $CH_3$ | O | - | - | - | - | - | - | |
| L-5 | $\overline{C=CH(CH_2)_4}$ | A-3 | 0 | $OCH_3$ | - | - | - | - | - | - | - | |
| L-5 | $\overline{C=CH(CH_2)_4}$ | A-4 | 0 | $OCH_3$ | - | $CH_3$ | - | - | - | - | - | |
| L-5 | $\overline{C=CH(CH_2)_4}$ | A-5 | 0 | - | - | - | $OCH_3$ | $CH_3$ | - | - | - | |
| L-5 | $\overline{C=CH(CH_2)_4}$ | A-6 | 0 | - | - | - | - | - | $CH_3$ | - | - | |
| L-6 | $\overline{C=CH(CH_2)_4}$ | A-2 | 0 | $CH_3$ | O | - | - | - | - | - | - | |
| L-6 | $\overline{C=CH(CH_2)_4}$ | A-3 | 0 | $OCH_3$ | - | - | - | - | - | - | - | |

Table X (Continued)

| L | W | A | n | $X_1$ | $Y_1$ | $Y_2$ | $X_2$ | $Y_3$ | $X_3$ | $X_4$ | $Y_4$ | m.p. (°C) |
|---|---|---|---|---|---|---|---|---|---|---|---|---|
| L-6 | $C{=}CH(CH_2)_4$ | A-4 | 0 | $OCH_3$ | - | $CH_3$ | - | - | - | - | - | |
| L-6 | $C{=}CH(CH_2)_4$ | A-5 | 0 | - | - | - | $OCH_3$ | $CH_3$ | - | - | - | |
| L-6 | $C{=}CH(CH_2)_4$ | A-6 | 0 | - | - | - | - | - | $CH_3$ | - | - | |
| L-2 | $CH{=}CH_2$ | A-2 | 0 | $CH_3$ | O | - | - | - | - | - | - | |
| L-2 | $CH{=}CH_2$ | A-2 | 0 | $OCH_3$ | O | - | - | - | - | - | - | |
| L-2 | $CH{=}CH_2$ | A-2 | 0 | $CH_3$ | CH | - | - | - | - | - | - | |
| L-2 | $CH{=}CH_2$ | A-3 | 0 | $OCH_3$ | - | - | - | - | - | - | - | |
| L-2 | $CH{=}CH_2$ | A-4 | 0 | $OCH_3$ | - | $CH_3$ | - | - | - | - | - | |
| L-2 | $CH{=}CH_2$ | A-5 | 0 | - | - | - | $OCH_3$ | $CH_3$ | - | - | - | |
| L-2 | $CH{=}CH_2$ | A-6 | 0 | - | - | - | - | - | $CH_3$ | - | - | |
| L-3 | $CH{=}CH_2$ | A-2 | 0 | $CH_3$ | O | - | - | - | - | - | - | |
| L-3 | $CH{=}CH_2$ | A-3 | 0 | $OCH_3$ | - | - | - | - | - | - | - | |
| L-3 | $CH{=}CH_2$ | A-4 | 0 | $OCH_3$ | - | $CH_3$ | - | - | - | - | - | |
| L-3 | $CH{=}CH_2$ | A-5 | 0 | - | - | - | $OCH_3$ | $CH_3$ | - | - | - | |
| L-3 | $CH{=}CH_2$ | A-6 | 0 | - | - | - | - | - | $CH_3$ | - | - | |
| L-4 | $CH{=}CH_2$ | A-2 | 0 | $CH_3$ | O | - | - | - | - | - | - | |
| L-4 | $CH{=}CH_2$ | A-3 | 0 | $OCH_3$ | - | - | - | - | - | - | - | |
| L-4 | $CH{=}CH_2$ | A-4 | 0 | $OCH_3$ | - | $CH_3$ | - | - | - | - | - | |
| L-4 | $CH{=}CH_2$ | A-5 | 0 | - | - | - | $OCH_3$ | $CH_3$ | - | - | - | |
| L-4 | $CH{=}CH_2$ | A-6 | 0 | - | - | - | - | - | $CH_3$ | - | - | |
| L-5 | $CH{=}CH_2$ | A-2 | 0 | $CH_3$ | O | - | - | - | - | - | - | |
| L-5 | $CH{=}CH_2$ | A-3 | 0 | $OCH_3$ | - | - | - | - | - | - | - | |
| L-5 | $CH{=}CH_2$ | A-4 | 0 | $OCH_3$ | - | $CH_3$ | - | - | - | - | - | |
| L-5 | $CH{=}CH_2$ | A-5 | 0 | - | - | - | $OCH_3$ | $CH_3$ | - | - | - | |
| L-5 | $CH{=}CH_2$ | A-6 | 0 | - | - | - | - | - | $CH_3$ | - | - | |
| L-6 | $CH{=}CH_2$ | A-2 | 0 | $CH_3$ | O | - | - | - | - | - | - | |
| L-6 | $CH{=}CH_2$ | A-3 | 0 | $OCH_3$ | - | - | - | - | - | - | - | |
| L-6 | $CH{=}CH_2$ | A-4 | 0 | $OCH_3$ | - | $CH_3$ | - | - | - | - | - | |
| L-6 | $CH{=}CH_2$ | A-5 | 0 | - | - | - | $OCH_3$ | $CH_3$ | - | - | - | |
| L-6 | $CH{=}CH_2$ | A-6 | 0 | - | - | - | - | - | $CH_3$ | - | - | |

Table X (Continued)

| L | W | A | n | $X_1$ | $Y_1$ | $Y_2$ | $X_2$ | $Y_3$ | $X_3$ | $X_4$ | $Y_4$ | Z |
|---|---|---|---|---|---|---|---|---|---|---|---|---|
| L-2 | $CH=CH_2$ | A-7 | 0 | - | - | - | - | - | - | $CH_3$ | $OCH_3$ | CH |
| L-2 | $CH=CH_2$ | A-7 | 0 | - | - | - | - | - | - | $OCH_3$ | $OCH_3$ | CH |
| L-2 | $CH=CH_2$ | A-7 | 0 | - | - | - | - | - | - | Cl | $OCH_3$ | CH |
| L-2 | $CH=CH_2$ | A-7 | 0 | - | - | - | - | - | - | $OCH_3$ | $OCH_2CH_3$ | CH |

Table Xa

General Structure Xa

| L | W | A | n | $X_1$ | $Y_1$ | $Y_2$ | $X_2$ | $Y_3$ | $X_3$ | $X_4$ | $Y_4$ | m.p. (°C) |
|---|---|---|---|---|---|---|---|---|---|---|---|---|
| L-2 | $C=CH(CH_2)_4$ | A-2 | O | $CH_3$ | O | - | - | - | - | - | - | |
| L-2 | $C=CH(CH_2)_4$ | A-2 | O | $OCH_3$ | O | - | - | - | - | - | - | |
| L-2 | $C=CH(CH_2)_4$ | A-2 | O | $CH_3$ | CH | - | - | - | - | - | - | |
| L-2 | $C=CH(CH_2)_4$ | A-3 | O | $OCH_3$ | - | - | - | - | - | - | - | |
| L-2 | $C=CH(CH_2)_4$ | A-4 | O | $OCH_3$ | - | $CH_3$ | - | - | - | - | - | |
| L-2 | $C=CH(CH_2)_4$ | A-5 | O | - | - | - | $OCH_3$ | $CH_3$ | - | - | - | |
| L-2 | $C=CH(CH_2)_4$ | A-6 | O | - | - | - | - | - | $CH_3$ | - | - | |
| L-2 | $CH=CH_2$ | A-2 | O | $CH_3$ | O | - | - | - | - | - | - | |
| L-2 | $CH=CH_2$ | A-2 | O | $OCH_3$ | O | - | - | - | - | - | - | |
| L-2 | $CH=CH_2$ | A-2 | O | $CH_3$ | CH | - | - | - | - | - | - | |
| L-2 | $CH=CH_2$ | A-3 | O | $OCH_3$ | - | - | - | - | - | - | - | |
| L-2 | $CH=CH_2$ | A-4 | O | $OCH_3$ | - | $CH_3$ | - | - | - | - | - | |
| L-2 | $CH=CH_2$ | A-5 | O | - | - | - | $OCH_3$ | $CH_3$ | - | - | - | |
| L-2 | $CH=CH_2$ | A-6 | O | - | - | - | - | - | $CH_3$ | - | - | |
| L-2 | $CH=CH_2$ | A-7 | - | - | - | - | - | - | - | $CH_3$ | $OCH_3$ | |
| L-2 | $CH=CH_2$ | A-7 | - | - | - | - | - | - | - | $OCH_3$ | $OCH_3$ | |
| L-2 | $CH=CH_2$ | A-7 | - | - | - | - | - | - | - | Cl | $OCH_3$ | |

Formulations

Useful formulations of the compounds of Formula I can be prepared in conventional ways. They include dusts, granules, pellets, solutions, suspensions, emulsions, wettable powders, emulsifiable concentrates and the like. Many of these may be applied directly. Sprayable formulations can be extended in suitable media and used at spray volumes of from a few liters to several hundred liters per hectare. High strength compositions are primarily used as intermediates for further formulation. The formulations, broadly, contain about 0.1% to 99% by weight of active ingredient(s) and at least one of (a) about 0.1% to

20% surfactant(s) and (b) about 1% to 99.9% solid or liquid inert diluent(s). More specifically, they will contain the ingredients in the following approximate proportions:

| | Weight Percent* | | |
|---|---|---|---|
| | Active Ingredient | Diluent(s) | Surfactant(s) |
| Wettable Powders | 20-90 | 0-74 | 1-10 |
| Oil Suspensions, Emulsions, Solutions, (including Emulsifiable Concentrates) | 3-50 | 40-95 | 0-15 |
| Aqueous Suspension | 10-50 | 40-84 | 1-20 |
| Dusts | 1-25 | 70-99 | 0-5 |
| Granules and Pellets | 0.1-95 | 5-99.9 | 0-15 |
| High Strength Compositions | 90-99 | 0-10 | 0-2 |

* Active ingredient plus at least one of a Surfactant or a Diluent equals 100 weight percent.

Lower or higher levels of active ingredient can, of course, be present depending on the intended use and the physical properties of the compound. Higher ratios of surfactant to active ingredient are sometimes desirable, and are achieved by incorporation into the formulation or by tank mixing.

Typical solid diluents are described in Watkins, et al., "Handbook of Insecticide Dust Diluents and Carriers". 2nd Ed., Dorland Books, Caldwell, New Jersey, but other solids, either mined or manufactured, may be used. The more absorptive diluents are preferred for wettable powders and the denser ones for dusts. Typical liquid diluents and solvents are described in Marsden, "Solvents Guide", 2nd Ed., Interscience, New York, 1950. Solubility under 0.1% is preferred for suspension concentrates; solution concentrates are preferably stable against phase separation at 0°C. "McCutcheon's Detergents and Emulsifiers Annual", MC Publishing Corp., Ridgewood, New Jersey, as well as Sisely and Wood, "Encyclopedia of Surface Active Agents", Chemical Publishing Co., Inc., New York, 1964, list surfactants and recommended uses. All formulations can contain minor amounts of additives to reduce foaming, caking, corrosion, microbiological growth, etc.

The methods of making such compositions are well known. Solutions are prepared by simply mixing the ingredients. Fine solid compositions are made by blending and, usually, grinding as in a hammer or fluid energy mill. Suspensions are prepared by wet milling (see, for example, Littler, U.S. Patent 3,060,084). Granules and pellets may be made by spraying the active material upon preformed granular carries or by agglomeration techniques. See J. E. Browning, "Agglomeration", *Chemical Engineering*, December 4, 1967, pp. 147ff. and "Perry's Chemical Engineer's Handbook", 5th Ed., McGraw-Hill, New York, 1973, pp. 8—57ff.

For further information regarding the art of formulation, see for example:

H. M. Loux, U.S. Patent 3,235,361, February 15, 1966, Col. 6, line 16 through Col. 7, line 19 and Examples 10 through 41;

R. W. Luckenbaugh, U.S. Patent 3,309,192, March 14, 1967, Col. 5, line 43 through Col. 7, line 62 and Examples 8, 12, 15, 39, 41, 52, 53, 58, 132, 138—140, 162—164, 166, 167 and 169—182;

H. Gysin and E. Knusli, U.S. Patent 2,891,855, June 23, 1959, Col. 3, line 66 through Col. 5, line 17 and Examples 1—4;

G. C. Klingman, "Weed Control as a Science", John Wiley and Sons, Inc., New York, 1961, pp. 81—96; and

J. D. Fryer and S. A. Evans, "Weed Control Handbook", 5th Ed., Blackwell Scientific Publications, Oxford, 1968, pp. 101—103.

In the following examples, all parts are by weight unless otherwise indicated.

Example 13

Wettable Powder

| | |
|---|---|
| N-[(4,6-dimethoxypyrimidin-2-yl)aminocarbonyl]-2-(2-oxiranylmethyl)benzenesulfonamide | 50% |
| sodium alkylnaphthalenesulfonate | 2% |
| low viscosity methyl cellulose | 2% |
| diatomaceous earth | 46% |

The ingredients are blended, coarsely hammer-milled and then air-milled to produce particles essentially all below 10 microns in diameter. The product is reblended before packaging.

Example 14

Granule

| | |
|---|---|
| Wettable Powder of Example 13 | 5% |
| attapulgite granules (U.S.S. 20—40 mesh; 0.84—0.42 mm) | 95% |

A slurry of wettable powder containing 25% solids is sprayed on the surface of attapulgite granules in a double-cone blender. The granules are dried and packaged.

Example 15

Extruded Pellet

| | |
|---|---|
| N-[(4,6-dimethoxypyrimidin-2-yl)aminocarbonyl]-2-(2-oxiranylmethyl)benzenesulfonamide | 25% |
| anhydrous sodium sulfate | 10% |
| crude calcium ligninsulfonate | 5% |
| sodium alkylnaphthalenesulfonate | 1% |
| calcium/magnesium bentonite | 59% |

The ingredients are blended, hammer-milled and then moistened with about 12% water. The mixture is extruded as cylinders about 3 mm diameter which are cut to produce pellets about 3 mm long. These may be used directly after drying, or the dried pellets may be crushed to pass a U.S.S. No. 20 sieve (0.84 mm openings). The granules held on a U.S.S. No. 40 sieve (0.42 mm openings) may be packaged for use and the fines recycled.

Example 16

Low Strength Granule

| | |
|---|---|
| 2-(2,2-dichlorocyclopropyl)-N-[(4-methoxy-6-methyl)-aminocarbonyl]benzenesulfonamide | 1% |
| N,N-dimethylformamide | 9% |
| attapulgite granules (U.S.S. 20—40 sieve) | 90% |

The active ingredient is dissolved in the solvent and the solution is sprayed upon dedusted granules in a double cone blender. After spraying of the solution has been completed, the blender is allowed to run for a short period and then the granules are packaged.

Example 17

Aqueous Suspension

| | |
|---|---|
| 2-(2,2-dichlorocyclopropyl)-N-[(4-methoxy-6-methyl)-aminocarbonyl]benzenesulfonamide | 40% |
| polyacrylic acid thickener | 0.3% |
| dodecylphenol polyethylene glycol ether | 0.5% |
| disodium phosphate | 1% |
| monosodium phosphate | 0.5% |
| polyvinyl alcohol | 1.0% |
| water | 56.7% |

The ingredients are blended and ground together in a sand mill to produce particles essentially all under 5 microns in size.

Example 18

Oil Suspension

| | |
|---|---|
| N-[(4,6-dimethoxypyrimidin-2-yl)aminocarbonyl]-2-(2-oxiranylmethyl)benzenesulfonamide | 35% |
| blend of polyalcohol carboxylic esters and oil soluble petroleum sulfonates | 6% |
| xylene | 59% |

The ingredients are combined and ground together in a sand mill to produce particles essentially all below 5 microns. the product can be used directly, extended with oils, or emulsified in water.

Example 19

Granule

| | |
|---|---|
| 2-(2,2-dichlorocyclopropyl)-N-[(4,6-dimethoxypyrimidin-2-yl)aminocarbonyl]benzenesulfonamide | 80% |
| wetting agent | 1% |
| crude ligninsulfonate (salt (containing 5—20% of the natural sugars) | 10% |
| attapulgite clay | 9% |

The ingredients are blended and milled to pass through a 100 mesh screen. This material is then added to a fluid bed granulator, the air flow is adjusted to gently fluidize the material, and a fine spray of water is sprayed onto the fluidized material. The fluidization and spraying are continued until granules of the desired size range are made. The spraying is stopped, but fluidization is continued, optionally with heat, until the water content is reduced to the desired level, generally less than 1%. The material is then discharged, screened to the desired size range, generally 14—100 mesh (1410—149 microns), and packaged for use.

Example 20

High Strength Concentrate

| | |
|---|---|
| N-[(4,6-dimethoxypyrimidin-2-yl)aminocarbonyl]-2-(2-oxiranylmethyl)benzenesulfonamide | 99% |
| silica aerogel | 0.5% |
| synthetic amorphous silica | 0.5% |

The ingredients are blended and ground in a hammer-mill to produce a material essentially all passing a U.S.S. No. 50 screen (0.3 mm opening). The concentrate may be formulated further if necessary.

Example 21

Wettable Powder

| | |
|---|---|
| 2-(2,2-dichlorocyclopropyl)-N-[(4-methoxy-6-methyl)aminocarbonyl]benzenesulfonamide | 90% |
| dioctyl sodium sulfosuccinate | 0.1% |
| synthetic fine silica | 9.9% |

The ingredients are blended and ground in a hammer-mill to produce particles essentially all below 100 microns. The material is sifted through a U.S.S. No. 50 screen and then packaged.

Example 22

Wettable Powder

| | |
|---|---|
| 2-(2,2-dichlorocyclopropyl)-N-[(4,6-dimethoxypyrimidin-2-yl)aminocarbonyl]benzenesulfonamide | 40% |
| sodium ligninsulfonate | 20% |
| montmorillonite clay | 40% |

The ingredients are thoroughly blended, coarsely hammer-milled and then air-milled to produce particles essentially all below 10 microns in size. The material is reblended and then packaged.

Example 23

Dust

| | |
|---|---|
| N-[(4,6-dimethoxypyrimdin-2-yl)aminocarbonyl]-2-(2-oxiranylmethyl)benzenesulfonamide | 10% |
| attapulgite | 10% |
| Pyrophyllite | 80% |

The active ingredient is blended with attapulgite and then passed through a hammer-mill to produce particles substantially all below 200 microns. The ground concentrate is then blended with powdered pyrophyllite until homogeneous.

Example 24

Wettable Powder

| | |
|---|---|
| 2-(1-cyclohexen-2-yl)-N-[4-methoxy-6-methylpyrimidin-2-yl)aminocarbonyl]-3-thiophenesulfonamide | 80% |
| sodium alkylnaphthalenesulfonate | 2% |
| sodium ligninsulfonate | 2% |
| synthetic amorphous silica | 3% |
| kaolinite | 13% |

The ingredients are blended, hammer-milled until all the solids are essentially under 50 microns, reblended, and packaged.

Example 25

Wettable Powder

| | |
|---|---|
| 2-(1-cyclohexen-2-yl)-N-[4-methoxy-65-methyl-1,3,5-triazin-2-yl)aminocrbonyl]-3-thiophenesulfonamide | 50% |
| sodium alkylnaphthalenesulfonate | 2% |
| low viscosity methyl cellulose | 2% |
| diatomaceous earth | 46% |

The ingredients are blended, coarsely hammer-milled and then air-milled to produce particles essentially all below 10 microns in diameter. The product is reblended before packaging.

Example 26

Granule

| | |
|---|---|
| Wettable Powder of Example 25 | 5% |
| attapulgite granules (U.S.S. 20—40 mesh; 0.84—0.42 mm) | 95% |

A slurry of wettable powder containing 25% solids is sprayed on the surface of attapulgite granules in a double-cone blender. The granules are dried and packaged.

Example 27

Extruded Pellet

| | |
|---|---|
| 2-(1-cyclohexen-2-yl)-N-[4-methoxy-6-methylpyrimidin-2-yl)aminocarbonyl]-3-thiophenesulfonamide | 25% |
| anhydrous sodium sulfate | 10% |
| crude calcium ligninsulfonate | 5% |
| sodium alkylnaphthalenesulfonate | 1% |
| calcium/magensium bentonite | 59% |

The ingredients are blended, hammer-milled and then moistened with about 12% water. The mixture is extruded as cylinders about 3 mm diameter which are cut to produce pellets about 3 mm long. These may be used directly after drying, or the dried pellets may be crushed to pass a U.S.S. No. 20 sieve (0.84 mm openings). The granules held in a U.S.S. No. 40 sieve (0.42 mm openings) may be packaged for use and the fines recycled.

Example 28

Oil Suspension

| | |
|---|---|
| 2-(1-cyclohexen-2-yl)-N-[4-methoxy-6-methyl-1,3,5-triazin-2-yl)aminocarbonyl]-3-thiophenesulfonamide | 25% |
| polyoxyethylene sorbitol hexaoleate | 5% |
| highly aliphatic hydrocarbon oil | 70% |

The ingredients are ground together in a sand mill until the solid particles have been reduced to under about 5 microns. The resulting thick suspension may be applied directly, but preferably after being extended with oils or emulsified in water.

Example 29

Wettable Powder

| | |
|---|---|
| 2-(1-cyclohexen-2-yl)-N-[4-methoxy-6-methylpyrimidin-2-yl)aminocarbonyl]-3-thiophenesulfonamide | 20% |
| sodium alkylnaphthalenesulfonate | 4% |
| sodium ligninsulfonate | 4% |
| low viscosity methyl cellulose | 3% |
| attapulgite | 69% |

The ingredients are thoroughly blended. After grinding in a hammer-mill to produce particles essentially all below 100 microns. The material is reblended and sifted through a U.S.S. No. 50 sieve (0.3 mm opening) and packaged.

Example 30

Low Strength Granule

| | |
|---|---|
| 2-(1-cyclohexen-2-yl)-N-[4-methoxy-6-methyl-1,3,5-triazin-2-yl)aminocarbonyl]-3-thiophenesulfonamide | 1% |
| N,N-dimethylformamide | 9% |
| attapulgite granules (U.S.S. 20—40 sieve) | 90% |

The active ingredient is dissolved in the solvent and the solution is sprayed upon dedusted granules in a double cone blender. After spraying of the solution has been completed, the blender is allowed to run for a short period and then the granules are packaged.

Example 31

Aqueous Suspension

| | |
|---|---|
| 2-(1-cyclohexen-2-yl)-N-[4-methoxy-6-methyl-1,3,5-triazin-2-yl)aminocarbonyl]-3-thiophenesulfonamide | 40% |
| polyacrylic acid thickener | 0.3% |
| dodecylphenol polyethylene glycol ether | 0.5% |
| disodium phosphate | 1% |
| monosodium phosphate | 0.5% |
| polyvinyl alcohol | 1.0% |
| water | 56.7% |

The ingredients are blended and ground together in a sand mill to produce particles essentially all under 5 microns in size.

Example 32

Solution

| | |
|---|---|
| 2-(1-cyclohexen-2-yl)-N-[4-methoxy-6-methylpyrimidin-2-yl)aminocarbonyl]-3-thiophenesulfonamide, ammonium salt | 5% |
| water | 95% |

The salt is added directly to the water with stirring to produce the solution, which may then be packaged for use.

Example 33

Low Strength Granule

| | |
|---|---|
| 2-(1-cyclohexen-2-yl)-N-[4-methoxy-6-methylpyrimidin-2-yl)aminocarbonyl]-3-thiophenesulfonamide | 0.1% |
| attapulgite granules (U.S.S. 20—40 mesh) | 99.9% |

The active ingredient is dissolved in a solvent and the solution is sprayed upon dedusted granules in a double-cone blender. After spraying of the solution has been completed, the material is warmed to evaporate the solvent. The material is allowed to cool and then packaged.

Example 34

Granule

| | |
|---|---|
| 2-(1-cyclohexen-2-yl)-N-[(4-methoxy-6-methyl-1,3,5-triazin-2-yl)aminocarbonyl]-3-thiophenesulfonamide | 80% |
| wetting agent | 1% |
| crude ligninsulfonate salt (containing 5—20% of the natural sugars) | 10% |
| attapulgite clay | 9% |

The ingredients are blended and milled to pass through a 100 mesh screen. This material is then added to a fluid bed granulator, the air flow is adjusted to gently fluidize the material, and a fine spray of water is sprayed onto the fluidized material. The fluidization and spraying are continued until granules of the desired size range are made. The spraying is stopped, but fluidization is continued, optionally with heat, until the water content is reduced to the desired level, generally less than 1%. The material is then discharged, screened to the desired size range, generally 14—100 mesh (1410—149 microns), and packaged for use.

Example 35

High Strength Concentrate

| | |
|---|---|
| 2-(1-cyclohexen-2-yl)-N-[4-methoxy-6-methylpyrimidin-2-yl)aminocarbonyl]-3-thiophenesulfonamide | 99% |
| silica aerogel | 0.5% |
| synthetic amorphous silica | 0.5% |

The ingredients are blended and ground in a hammer-mill to produce a material essentially all passing a U.S.S. No. 50 screen (0.3 mm opening). The concentrate may be formulated further if necessary.

Example 36

Wettable Powder

| | |
|---|---|
| N-[(4-methoxy-6-methylpyrimidin-2-yl)aminocarbonyl]-1-methyl-4-(1-propenyl)-1H-pyrazole-5-sulfonamide | 90% |
| dioctyl sodium sulfosuccinate | 0.1% |
| synthetic fine silica | 9.9% |

The ingredients are blended and ground in a hammer-mill to produce particles essentially all below 100 microns. The material is sifted through a U.S.S. No. 50 screen and then packaged.

Example 37

Wettable Powder

| | |
|---|---|
| N-[(4-methoxy-6-methylpyrimidin-2-yl)aminocarbonyl]-1-methyl-4-(1-propenyl)-1H-pyrazole-5-sulfonamide | 40% |
| sodium ligninsulfonate | 20% |
| montmorillonite clay | 40% |

The ingredients are thoroughly blended, coarsely hammer-milled and then air-milled to produce particles essentially all below 10 microns in size. The material is reblended and then packaged.

Example 38

Oil Suspension

| | |
|---|---|
| N-[(4-methoxy-6-methylpyrimidin-2-yl)aminocarbonyl]-1-methyl-4-(1-propenyl)-1H-pyrazole-5-sulfonamide | 35% |
| blend of polyalcohol carboxylic esters and oil soluble petroleum sulfonates | 6% |
| xylene | 59% |

The ingredients are combined and ground together in a sand mill to produce particles essentially all below 5 microns. The product can be used directly, extended with oils, or emulsified in water.

Example 39

Dust

| | |
|---|---|
| N-[(4-methoxy-6-methylpyrimidin-2-yl)aminocarbonyl]-1-methyl-4-(1-propenyl)-1H-pyrazole-5-sulfonamide | 10% |
| attapulgite | 10% |
| Pyrophyllite | 80% |

The active ingredient is blended with attapulgite and then passed through a hammer-mill to produce particles substantially all below 200 microns. The ground concentrate is then blended with powdered pyrophyllite until homogeneous.

Example 40

Emulsifiable Concentrate

| | |
|---|---|
| N-[(4-methoxy-6-methylpyrimidin-2-yl)aminocarbonyl]-1-methyl-4-(1-propenyl)-1H-pyrazole-5-sulfonamide | 10% |
| chlorobenzene | 84% |
| sorbitan monostearate and polyoxyethylene condensates thereof | 6% |

The ingredients are combined and stirred to produce a solution which can be emulsified in water for application.

Utility

Test results indicate that the compounds of the present invention are highly active preemergent or postemergent herbicides or plant growth regulants. Many of them have utility for broad-spectrum pre- and or post-emergence weed control in areas where complete control of all vegetation is desired, such as around fuel storage tanks, ammunition depots, industrial storage areas, parking lots, drive-in theaters, around billboards, highway and railroad structures. Some of the compounds have utility for selective weed control in crops such as rice and wheat. Alternatively, the subject compounds are useful to modify plant growth.

The rates of application for the compunds of the invention are determined by a number of factors, including their use as plant growth modifiers or as herbicides, the crop species involved, the types of weeds to be controlled, weather and climate, formulations selected, mode of application, amount of foliage present, etc. In general terms, the subject compounds should be applied at levels of around 0.05 to 10 kg/ha, the lower rates being suggested for use on lighter soils and/or those having a low organic matter content, for plant growth modification or for situations where only short-term persistence is required.

The compounds of the invention may be used in combination with any other commercial herbicide, examples of which are those of the triazine, triazole, uracil, urea, amide, diphenylether, carbamate and bipyridylium types.

The herbicidal properties of the subject compounds were discovered in a number of greenhouse tests. The test procedures and results follow.

Test A

Seeds of crabgrass (*Digitaria* sp.), barnyardgrass (*Echinochloa crusgalli*), wild oats (*Avena fatua*), cheatgrass (*Bromus secalinus*), velvetleaf (*Abutilon theophrasti*), sicklepod (*Cassia obtusifolia*),

morningglory (*Ipomoea* sp.), cocklebur (*Xanthium* sp.), sorghum, corn, soybean, rice, wheat and nutsedge tubers (*Cyperus rotundus*) were planted in a growth medium and treated pre-emergence with the chemicals dissolved in a non-phytotoxic solvent. At the same time, cotton having five leaves (including cotyledonary ones), bush beans with the third trifoliate leaf expanding, crabgrass, barnyardgrass and wild oats with two leavers, sicklepod with three leaves (including cotyledonary ones), morningglory and cocklebur with four leaves (including the cotyledonary ones), sorghum and corn with four leaves, soybean with two cotyledonary leaves, rice with three leaves, wheat with one leaf, and nutsedge with three to five leaves were sprayed. Treated plants and controls were maintained in a greenhouse for sixteen days, whereupon all species were compared to controls and visually rated for response to treatment. The ratings are based on a numerical scale extending from 0 = no injury, to 10 = complete kill. The accompanying descriptive symbols have the following meanings:

C = chlorosis or necrosis;
B = burn;
D = defoliation;
E = emergence inhibition;
G = growth retardation;
H = formative effects;
U = unusual pigmentation;
X = axillary stimulation;
S = albinism; and
6Y = abscised buds or flowers.

Compounds

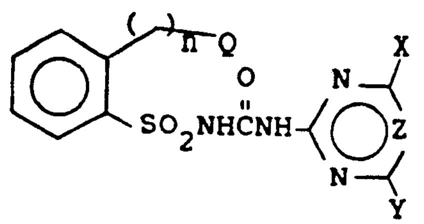

| Compound | n | Q | X | Y | Z |
|---|---|---|---|---|---|
| 1 | 0 | 2,2-dichloro-cyclopropyl | $CH_3$ | $CH_3$ | CH |
| 2 | 0 | 2,2-dichloro-cyclopropyl | $CH_3$ | $OCH_3$ | CH |
| 3 | 0 | 2,2-dichloro-cyclopropyl | $OCH_3$ | $OCH_3$ | CH |
| 4 | 0 | 2,2-dichloro-cyclopropyl | $CH_3$ | $CH_3$ | N |
| 5 | 0 | 2,2-dichloro-cyclopropyl | $CH_3$ | $OCH_3$ | N |
| 6 | 0 | 2,2-dichloro-cyclopropyl | $OCH_3$ | $OCH_3$ | N |
| 7 | 1 | 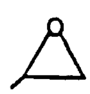 | $OCH_3$ | $OCH_3$ | CH |
| 8 | 0 | 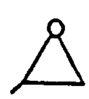 | $OCH_3$ | $OCH_3$ | CH |
| 9 | 0 | 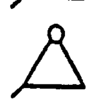 | $CH_3$ | $CH_3$ | CH |

Compounds (continued)

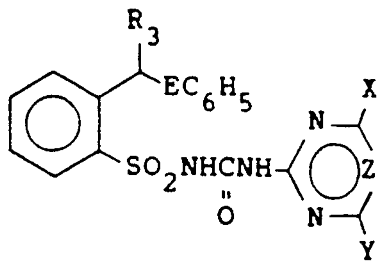

| Compound | $R_3$ | E | X | Y | Z |
|---|---|---|---|---|---|
| 10 | H | Se | $CH_3$ | $CH_3$ | CH |
| 11 | H | Se | $CH_3$ | $OCH_3$ | CH |
| 12 | H | Se | $OCH_3$ | $OCH_3$ | CH |
| 13 | H | Se | $CH_3$ | $OCH_3$ | N |
| 14 | H | Se | $OCH_3$ | $OCH_3$ | N |
| 15 | $CH_3$ | Se | $CH_3$ | $CH_3$ | CH |
| 16 | $CH_3$ | Se | $CH_3$ | $OCH_3$ | CH |
| 17 | $CH_3$ | Se | $OCH_3$ | $OCH_3$ | CH |
| 18 | $CH_3$ | Se | $CH_3$ | $CH_3$ | N |
| 19 | $CH_3$ | Se | $CH_3$ | $OCH_3$ | N |
| 20 | $CH_3$ | Se | $OCH_3$ | $OCH_3$ | N |
| 21 | H | S | $CH_3$ | $CH_3$ | CH |
| 22 | H | S | $OCH_3$ | $CH_3$ | CH |
| 23 | H | S | $OCH_3$ | $OCH_3$ | CH |
| 24 | H | S | $CH_3$ | $CH_3$ | N |
| 25 | H | S | $OCH_3$ | $OCH_3$ | N |

Compounds (continued)

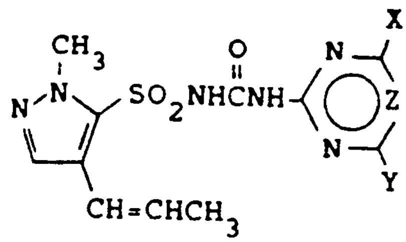

| Compound | X | Y | Z |
|---|---|---|---|
| 26 | $OCH_3$ | $CH_3$ | CH |
| 27 | $OCH_3$ | $OCH_3$ | CH |
| 28 | $OCH_3$ | $OCH_3$ | N |

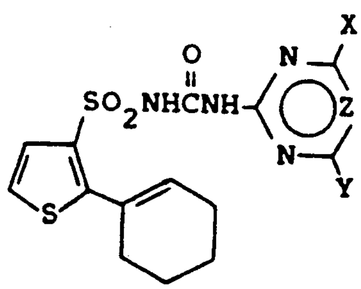

| Compound | X | Y | Z |
|---|---|---|---|
| 29 | $CH_3$ | $CH_3$ | CH |
| 30 | $OCH_3$ | $CH_3$ | CH |
| 31 | $OCH_3$ | $OCH_3$ | CH |
| 32 | $OCH_3$ | $CH_3$ | N |
| 33 | $OCH_3$ | $OCH_3$ | N |
| 34 | Cl | $OCH_3$ | CH |

Table A

| | Compound 1 | Compound 2 | Compound 3 |
|---|---|---|---|
| Rate (kg/ha) | 0.05 | 0.05 | 0.05 |
| POSTEMERGENCE | | | |
| Morningglory | 0 | 2C,5G | 5C,8G |
| Cocklebur | 5G | 9C | 9C |
| Velvetleaf | 2C,8G | 9C | 10C |
| Nutsedge | 0 | 10C | 9C |
| Crabgrass | 0 | 2G | 4G |
| Barnyardgrass | 0 | 8H | 3H |
| Cheatgrass | 0 | 2C,6G | 2C,6G |
| Wild Oats | 0 | 2G | 0 |
| Wheat | 0 | 0 | 0 |
| Corn | 0 | 2C,8H | 1C,4G |
| Soybean | 2C,9G | 4C,9G | 5C,9G |
| Rice | 5G | 2C,7G | 2C,6G |
| Sorghum | 2H | 2G | 2G |
| Sugar Beets | 0 | 5G | 4C,8G |
| Cotton | 4C,8H | 5C,9G | 5C,9G |
| PREEMERGENCE | | | |
| Morningglory | 4G | 8G | 7G |
| Cocklebur | - | 8H | 9G |
| Velvetleaf | 2G | 9G | 9G |
| Nutsedge | 0 | 9G | 10E |
| Crabgrass | 0 | 2G | 2C,8G |
| Barnyardgrass | 0 | 2C,9H | 2C,7H |
| Cheatgrass | 2G | 5C,9H | 5C,9H |
| Wild Oats | 0 | 3C,8G | 3G |
| Wheat | 0 | 6G | 2G |
| Corn | 2G | 2C,9H | 2C,8G |
| Soybean | 0 | 4C,8H | 7G |
| Rice | 3G | 4C,8G | 2C,8H |
| Sorghum | 5G | 4C,8H | 2C,7H |
| Sugar Beets | 5G | 8G | 9G |
| Cotton | 5G | 9G | 8G |

Table A (continued)

| | Compound 4 | Compound 5 | Compound 6 |
|---|---|---|---|
| Rate (kg/ha) | 0.05 | 0.05 | 0.05 |
| POSTEMERGENCE | | | |
| Morningglory | 4C,8G | 10C | 10C |
| Cocklebur | 3C,8G | 9C | 10C |
| Velvetleaf | 4C,8H | 10C | 10C |
| Nutsedge | 0 | 9C | 9C |
| Crabgrass | 0 | 2G | 0 |
| Barnyardgrass | 0 | 2H | 0 |
| Cheatgrass | 0 | 2C | 1C |
| Wild Oats | 0 | 0 | 0 |
| Wheat | 0 | 3G | 0 |
| Corn | 0 | 3C,8H | 3C,8H |
| Soybean | 2C,9G | 3C,9G | 4C,9G |
| Rice | 3G | 5G | 3G |
| Sorghum | 0 | 2C,5H | 2C,5H |
| Sugar Beets | 5C,9G | 9C | 10C |
| Cotton | 4C,8H | 5C,9G | 9C |
| PREEMERGENCE | | | |
| Morningglory | 7H | 9G | 8G |
| Cocklebur | 2C | 9H | 9C |
| Velvetleaf | 0 | 9G | 5C,9G |
| Nutsedge | 0 | 4G | 7G |
| Crabgrass | 0 | 0 | 0 |
| Barnyardgrass | 0 | 2C,3H | 2C |
| Cheatgrass | 0 | 5G | 0 |
| Wild Oats | 0 | 2G | 0 |
| Wheat | 0 | 0 | 0 |
| Corn | 2G | 4G | 2C,6G |
| Soybean | 3C,2H | 4C,8H | 3C,8H |
| Rice | 0 | 4G | 3G |
| Sorghum | 0 | 2C,5G | 2G |
| Sugar Beets | 3C,5G | 10E | 5C,9G |
| Cotton | 5G | 9G | 9G |

Table A (continued)

| | Compound 7 | Compound 8 | Compound 9 |
|---|---|---|---|
| Rate (kg/ha) | 0.05 | 0.05 | 0.05 |
| POSTEMERGENCE | | | |
| Morningglory | 2C,8G | 1C | 0 |
| Cocklebur | 8H | 2C,7G | 2G |
| Velvetleaf | - | 9C | 2G |
| Nutsedge | 10C | 4C,9G | 1C |
| Crabgrass | 1C,5G | 1C | 0 |
| Barnyardgrass | 5C,9H | 4C,9H | 2C,4H |
| Cheatgrass | - | 3G | 0 |
| Wild Oats | 2G | 0 | 0 |
| Wheat | 2G | 0 | 0 |
| Corn | 2C,8H | 2C,7H | 2C,5G |
| Soybean | 4C,8H | 3C,9G,7X | 2C,2H |
| Rice | 4C,9G | 6G | 3G |
| Sorghum | 2C,9H | 2C,7H | 2C,5G |
| Sugar Beets | 9C | 9C | 2C,5G |
| Cotton | 4C,4H | 9C | 1H |
| Bushbean | 5C,9G | - | - |
| Cassia | 2C,9G | - | - |
| PREEMERGENCE | | | |
| Morningglory | 7G | 5G | 0 |
| Cocklebur | 9H | 0 | 0 |
| Velvetleaf | - | 0 | 0 |
| Nutsedge | 5G | 0 | 0 |
| Crabgrass | 2C | 0 | 0 |
| Barnyardgrass | 4C,8H | 0 | 0 |
| Cheatgrass | - | 0 | 0 |
| Wild Oats | 3C,6G | 0 | 0 |
| Wheat | 1C,3G | 0 | 0 |
| Corn | 2C,8H | 0 | 1H |
| Soybean | 2C,8H | 2C | 1H |
| Rice | 3C,8G | 0 | 4G |
| Sorghum | 3C,9G | 1C | 2G |
| Sugar Beets | 9G | 6G | 2H |
| Cotton | - | 2G | 0 |
| Bushbean | - | - | - |
| Cassia | 5C,9H | - | - |

Table A (continued)

| | Compound 10 | Compound 11 |
|---|---|---|
| Rate (kg/ha) | 0.40 | 0.05 |
| POSTEMERGENCE | | |
| Morningglory | 0 | 2C,6H |
| Cocklebur | 0 | 2G |
| Velvetleaf | 2G | 3C,8G |
| Nutsedge | 0 | 2C,5G |
| Crabgrass | 0 | 2G |
| Barnyardgrass | 4H | 2C,7H |
| Cheatgrass | 0 | 5G |
| Wild Oats | 0 | 2C,4G |
| Wheat | 0 | 3G |
| Corn | 2C,7H | 3C,8G |
| Soybean | 2H | 3H,9G |
| Rice | 6G | 7G |
| Sorghum | 2C,5H | 3C,8G |
| Sugar Beets | 3C,6G | 2C,8G |
| Cotton | 3C,7H | 3C,7H |
| PREEMERGENCE | | |
| Morningglory | 3G | 8G |
| Cocklebur | 1H | 7H |
| Velvetleaf | 2G | 8G |
| Nutsedge | 5G | 0 |
| Crabgrass | 0 | 2G |
| Barnyardgrass | 0 | 2C,7G |
| Cheatgrass | 5G | 0 |
| Wild Oats | 5G | 2C,7G |
| Wheat | 4G | 4G |
| Corn | 2C,6H | 2C,8H |
| Soybean | 2C,2H | 3C,7G |
| Rice | 7H | 7G |
| Sorghum | 7H | 3C,9H |
| Sugar Beets | 3C,8G | 2C,8G |
| Cotton | 5G | 7G |

Table A (continued)

| | Compound 12 | Compound 13 |
|---|---|---|
| Rate (kg/ha) | 0.05 | 0.05 |
| POSTEMERGENCE | | |
| Morningglory | 1C | 2C |
| Cocklebur | 1C,3G | 2C,8H |
| Velvetleaf | 5C,9G | 4C,9G |
| Nutsedge | 5G | 0 |
| Crabgrass | 0 | 0 |
| Barnyardgrass | 3C,8H | 0 |
| Cheatgrass | 0 | 0 |
| Wild Oats | 0 | 0 |
| Wheat | 0 | 0 |
| Corn | 0 | 2H |
| Soybean | 4C,9G | 2C,9G |
| Rice | 8G | 0 |
| Sorghum | 7H | 2G |
| Sugar Beets | 9C | 2C,5H |
| Cotton | 2C,8G | 3C,8H |
| PREEMERGENCE | | |
| Morningglory | 4G | 2C |
| Cocklebur | 7G | 2C |
| Velvetleaf | 7G | 2G |
| Nutsedge | 7G | 0 |
| Crabgrass | 0 | 0 |
| Barnyardgrass | 4G | 0 |
| Cheatgrass | 2G | 0 |
| Wild Oats | 0 | 0 |
| Wheat | 0 | 0 |
| Corn | 2C,8G | 2G |
| Soybean | 3C,6G | 1C |
| Rice | 2C,7G | 0 |
| Sorghum | 2C,7H | 2G |
| Sugar Beets | 8G | 2C,6G |
| Cotton | 5G | 6G |

Table A (continued)

| | Compound 14 | Compound 15 | Compound 16 |
|---|---|---|---|
| Rate (kg/ha) | 0.05 | 0.05 | 0.05 |
| POSTEMERGENCE | | | |
| Morningglory | 1C | 6G | 5C,8G |
| Cocklebur | 2C,4H | 2C,8G | 8G,9G |
| Velvetleaf | 4C,8G | 5C,9G | 9C |
| Nutsedge | 0 | 3C,7G | 3C,8G |
| Crabgrass | 0 | 2G | 3G |
| Barnyardgrass | 0 | 3C,9H | 2C,9H |
| Cheatgrass | 0 | 0 | 4G |
| Wild Oats | 0 | 3C,9G | 2C,7G |
| Wheat | 0 | 6G | 5G |
| Corn | 1H | 2C,8G | 3C,9G |
| Soybean | 7H | 3C,9G | 5C,9G |
| Rice | 0 | 3C,9G | 2C,8G |
| Sorghum | 0 | 2C,9G | 3C,9H |
| Sugar Beets | 3C,5G | 4C,9G | 7G |
| Cotton | 3C,6H | 4C,9G | 4C,9G |
| PREEMERGENCE | | | |
| Morningglory | 0 | 7G | 9G |
| Cocklebur | 0 | 7G | 9G |
| Velvetleaf | 0 | 8G | 10C |
| Nutsedge | 0 | 8G | 9G |
| Crabgrass | 0 | 2G | 5G |
| Barnyardgrass | 0 | 7H | 9H |
| Cheatgrass | 0 | 9G | 3C,9G |
| Wild Oats | 0 | 8G | 4C,9H |
| Wheat | 0 | 8G | 5C,9H |
| Corn | 0 | 2C,9G | 5C,9H |
| Soybean | 0 | 3C,5H | 2C,8G |
| Rice | 0 | 10E | 10H |
| Sorghum | 0 | 5C,9H | 10H |
| Sugar Beets | 0 | 9C | 10C |
| Cotton | 0 | 9G | 9G |

Table A (continued)

| | Compound 17 | Compound 18 | Compound 19 |
|---|---|---|---|
| Rate (kg/ha) | 0.05 | 0.05 | 0.05 |
| POSTEMERGENCE | | | |
| Morningglory | 4C,9G | 2C,7G | 10C |
| Cocklebur | 3C,8G | 6G | 4C,8G |
| Velvetleaf | 10C | 9C | 9C |
| Nutsedge | 9C | 0 | 0 |
| Crabgrass | 0 | 0 | 3G |
| Barnyardgrass | 5C,9H | 3C,5H | 3C,8H |
| Cheatgrass | 4G | 0 | 0 |
| Wild Oats | 0 | 0 | 0 |
| Wheat | 0 | 0 | 0 |
| Corn | 3C,9H | 9G | 3C,8H |
| Soybean | 5C,9G | 4C,9G | 5C,9G |
| Rice | 2C,8G | 3C,9G | 5G |
| Sorghum | 3C,8H | 2C,8H | 4C,9H |
| Sugar Beets | 9C | 9C | 9C |
| Cotton | 5C,9G | 9H | 9C |
| PREEMERGENCE | | | |
| Morningglory | 8G | 2C | 9G |
| Cocklebur | 8H | 5G | - |
| Velvetleaf | 9C | 8G | 5C,9G |
| Nutsedge | 8G | 0 | 0 |
| Crabgrass | 3G | 0 | 0 |
| Barnyardgrass | 3C,9H | 0 | 2C |
| Cheatgrass | 5C,9G | 6G | 0 |
| Wild Oats | 8G | 2C,5G | 2G |
| Wheat | 4G | 0 | 0 |
| Corn | 8H | 2C,7G | 8G |
| Soybean | 7H | 6G | 8H |
| Rice | 10E | 8H | 8G |
| Sorghum | 3C,8H | 6G | 3C,8H |
| Sugar Beets | 9C | 9C | 10C |
| Cotton | 9G | 6H | 9G |

Table A (continued)

| | Compound 20 | Compound 21 | Compound 22 |
|---|---|---|---|
| Rate (kg/ha) | 0.05 | 0.05 | 0.05 |
| POSTEMERGENCE | | | |
| Morningglory | 9C | 1H | 4C,9G |
| Cocklebur | 7G | 7H | 4C,9G |
| Velvetleaf | 9C | 8H | 9C |
| Nutsedge | 0 | 7G | 3C,6G |
| Crabgrass | 0 | 2G | 2C,5G |
| Barnyardgrass | 2C,5H | 3H | 4C,9H |
| Cheatgrass | 0 | 5G | 2C,5G |
| Wild Oats | 0 | 0 | 3C,3H |
| Wheat | 0 | 0 | 2G |
| Corn | 2C,8H | 3H | 3C,9H |
| Soybean | 4C,9G | 0 | 3C,7H |
| Rice | 0 | 7G | 8G |
| Sorghum | 2C,5H | 2C,7G | 3C,9G |
| Sugar Beets | 9C | 4C,9G | 9C |
| Cotton | 9C | 4C,8H | 9C |
| PREEMERGENCE | | | |
| Morningglory | 9G | 5G | 7G |
| Cocklebur | 7H | 0 | 6G |
| Velvetleaf | 5C,9G | 5G | 8G |
| Nutsedge | 0 | 0 | 5G |
| Crabgrass | 0 | 0 | 0 |
| Barnyardgrass | 1C | 1C | 3C,7H |
| Cheatgrass | 0 | 0 | 5G |
| Wild Oats | 0 | 3G | 2C,8G |
| Wheat | 0 | 2G | 5G |
| Corn | 5G | 2C,4G | 2C,8G |
| Soybean | 8H | 2C,2H | 3C,8H |
| Rice | 5G | 2G | 7H |
| Sorghum | 3G | 2C | 3C,8H |
| Sugar Beets | 10C | 6G | 9G |
| Cotton | 9G | 4G | 8G |

Table A (continued)

| | Compound 23 | Compound 24 | Compound 25 |
|---|---|---|---|
| Rate (kg/ha) | 0.05 | 0.05 | 0.05 |
| POSTEMERGENCE | | | |
| Morningglory | 3C,8H | 1H | 2C,7G |
| Cocklebur | 3C,8H | 0 | 2C |
| Velvetleaf | 10C | 2C,7G | 5C,9G |
| Nutsedge | 5C,9G | 0 | 0 |
| Crabgrass | 1C | 0 | 0 |
| Barnyardgrass | 3C,8H | 0 | 0 |
| Cheatgrass | 0 | 0 | 0 |
| Wild Oats | 0 | 0 | 0 |
| Wheat | 0 | 0 | 0 |
| Corn | 2G | 0 | 0 |
| Soybean | 3H,9G | 1C,1H | 2C,9G |
| Rice | 8G | 5G | 0 |
| Sorghum | 2C,6G | 2G | 2C |
| Sugar Beets | 9C | 4C,8H | 9C |
| Cotton | 5C,9G | 3C,4G | 9H |
| PREEMERGENCE | | | |
| Morningglory | 6G | 1H | 5G |
| Cocklebur | 2C,5G | 0 | 3G |
| Velvetleaf | 9G | 0 | 5G |
| Nutsedge | 5G | 0 | 0 |
| Crabgrass | 0 | 0 | 0 |
| Barnyardgrass | 2C,2H | 0 | 2C |
| Cheatgrass | 5G | 0 | 0 |
| Wild Oats | 0 | 0 | 0 |
| Wheat | 0 | 0 | 0 |
| Corn | 3C,8H | 0 | 0 |
| Soybean | 6H | 0 | 5H |
| Rice | 3C,8H | 0 | 3G |
| Sorghum | 3C,5G | 0 | 0 |
| Sugar Beets | 9G | 0 | 4C,9G |
| Cotton | 8G | 0 | 7G |

Table A (continued)

| | Compound 26 | Compound 27 | Compound 28 |
|---|---|---|---|
| Rate (kg/ha) | 0.05 | 0.05 | 0.05 |
| POSTEMERGENCE | | | |
| Morningglory | 3C,8G | 2C,7G | 2H,5G |
| Cocklebur | 9C | 9C | 9H |
| Velvetleaf | 9C | 4C,9G | 8G |
| Nutsedge | 3C,8G | 2C,9G | 0 |
| Crabgrass | 3G | 0 | 0 |
| Barnyardgrass | 3C,9H | 4C,8H | 0 |
| Cheatgrass | 6G | 2G | 0 |
| Wild Oats | 0 | 2C | 0 |
| Wheat | 2G | 0 | 0 |
| Corn | 3C,9H | 2C,9H | 0 |
| Soybean | 4C,9H | 3C,8H | 2C,3H |
| Rice | 2C,6G | 5G | 0 |
| Sorghum | 4C,9H | 3C,5G | 3H |
| Sugar Beets | 4C,9H | 2C,8G | 3C,7G |
| Cotton | 9C | 9C | 2C,8G |
| PREEMERGENCE | | | |
| Morningglory | 7H | 9G | 3C,7H |
| Cocklebur | 7H | 7H | 1C |
| Velvetleaf | 8G | 8G | 0 |
| Nutsedge | 9G | 10E | 0 |
| Crabgrass | 0 | 0 | 0 |
| Barnyardgrass | 6H | 4G | 0 |
| Cheatgrass | 6G | 0 | 0 |
| Wild Oats | 1C | 0 | 0 |
| Wheat | 2G | 5G | 0 |
| Corn | 9G | 8G | 2C,6G |
| Soybean | 8H | 2C,3G | 3C,3H |
| Rice | 5G | 5G | 5G |
| Sorghum | 2C,8G | 2C,6H | 2C,4G |
| Sugar Beets | 9G | 10C | 4G |
| Cotton | 9G | 9G | 7G |

Table A (continued)

| | Cmpd. 29 | Cmpd. 30 | Cmpd. 31 | Cmpd. 32 |
|---|---|---|---|---|
| Rate kg/ha | 0.05 | 0.05 | 0.05 | 0.05 |
| POSTEMERGENCE | | | | |
| Morningglory | 10C | 9C | 4C,8G | 10C |
| Cocklebur | 2C,9H | 9C | 10C | 9C |
| Velvetleaf | 10C | 9C | 9G | 10C |
| Nutsedge | 0 | 5G | 7G | 0 |
| Crabgrass | 0 | 2G | 0 | 0 |
| Barnyardgrass | 6H | 2C,6H | 2H | 1H |
| Cheatgrass | 5G | 3G | 5G | 0 |
| Wild Oats | 2C | 0 | 0 | 0 |
| Wheat | 0 | 0 | 0 | 0 |
| Corn | 3C,8H | 0 | 0 | 0 |
| Soybean | 3C,9G | 4C,9G | 5C,9G | 9C |
| Rice | 3G | 2G | 2G | 1G |
| Sorghum | 3C,8H | 2C,8G | 2C,6G | 2C,6G |
| Sugar beet | 2C,7G | 4C,9G | 9C | 9C |
| Cotton | 9C | 9C | 9C | 10C |
| PREEMERGENCE | | | | |
| Morningglory | 9G | 9G | 7G | 9G |
| Cocklebur | 8G | 8H | 8H | 8G |
| Velvetleaf | 8G | 8G | 7G | 8G |
| Nutsedge | 4G | 8G | 7G | 0 |
| Crabgrass | 0 | 0 | 0 | 0 |
| Barnyardgrass | 3G | 2C,7G | 4G | 0 |
| Cheatgrass | 4G | 8G | 5G | 0 |
| Wild Oats | 0 | 2C | 0 | 0 |
| Wheat | 2G | 0 | 0 | 0 |
| Corn | 5G | 2C,5G | 2C,2G | 3G |
| Soybean | 0 | 5G | 2G | 3G |
| Rice | 7G | 6G | 2G | 0 |
| Sorghum | 4C,9G | 2C,8H | 3C,7H | 3C,8G |
| Sugar beet | 8G | 4C,9G | 2C,8G | 4C,9G |
| Cotton | 5G | 8G | 5G | 9G |

Table A (continued)

| | Cmpd. 33 | Cmpd. 34 |
|---|---|---|
| Rate kg/ha | 0.05 | 0.05 |
| POSTEMERGENCE | | |
| Morningglory | 10C | 4C,9G |
| Cocklebur | 10C | 10C |
| Velvetleaf | 3C,8G | 3C,8G |
| Nutsedge | 3G | 0 |
| Crabgrass | 0 | 0 |
| Barnyardgrass | 0 | 0 |
| Cheatgrass | 0 | 0 |
| Wild Oats | 0 | 0 |
| Wheat | 0 | 0 |
| Corn | 0 | 0 |
| Soybean | 9C | 2C,5G |
| Rice | 0 | 0 |
| Sorghum | 2G | 2C,5G |
| Sugar beet | 9C | 3H |
| Cotton | 10C | 10C |
| PREEMERGENCE | | |
| Morningglory | 9G | 6G |
| Cocklebur | 8H | 3H |
| Velvetleaf | 6G | 3G |
| Nutsedge | 0 | 0 |
| Crabgrass | 0 | 0 |
| Barnyardgrass | 0 | 0 |
| Cheatgrass | 0 | 0 |
| Wild Oats | 0 | 0 |
| Wheat | 0 | 0 |
| Corn | 2G | 0 |
| Soybean | 0 | 0 |
| Rice | 0 | 0 |
| Sorghum | 3C,5G | 2C,5G |
| Sugar beet | 9G | 6G |
| Cotton | 8G | 5G |

**Claims**

1. A compound of the formula:

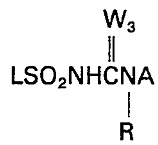

wherein

L is 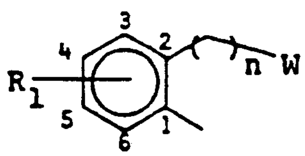 , 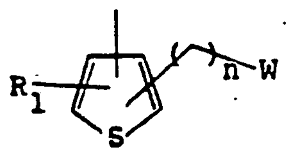 ,

L-1 L-2

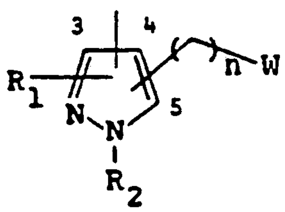 , 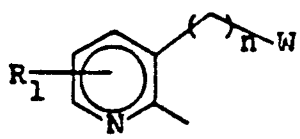 ,

L-3 L-4

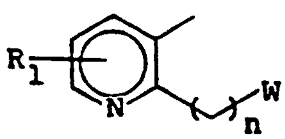 , 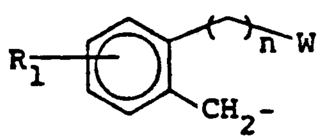 ,

L-5 L-6

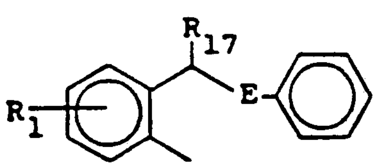 .

L-7

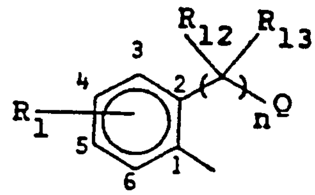,

L-8

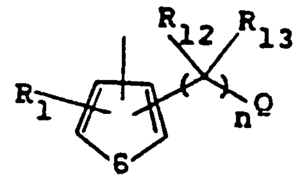,

L-9

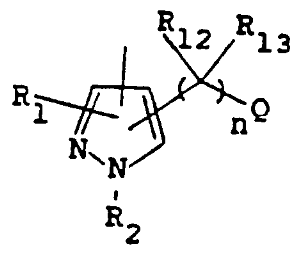,

L-10

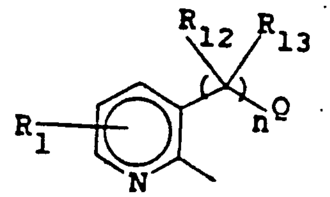,

L-11

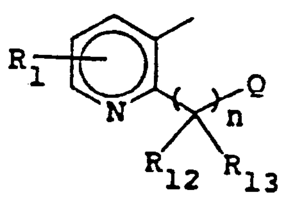

L-12

or

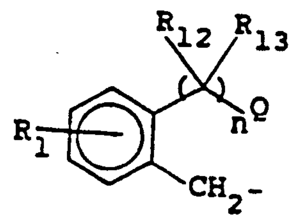;

L-13

R is H or $CH_3$;
$W_3$ is O or S;
n is 0 or 1;
E is $S(O)_p$ or Se;
p is 0, 1 or 2;
$R_1$ is H, $C_1$—$C_3$ alkyl, $C_1$—$C_3$ haloalkyl, halogen, nitro, $CH_2CN$, $CH_2OCH_3$, $CH_2SCH_3$, CN, $C_1$—$C_3$ alkoxy, $SO_2NR^IR^{II}$, $C_1$—$C_3$ alkylathio, $C_1$—$C_3$ alkylsulfinyl, $C_1$—$C_3$ alkylsulfonyl or $CO_2R^{III}$;
$R^I$ is H, $C_1$—$C_4$ alkyl, $C_2$—$C_3$ cyanoalkyl, methoxy or ethoxy;
$R^{II}$ is H, $C_1$—$C_4$ alkyl or $C_3$—$C_4$ alkenyl; or
$R^I$ and $R^{II}$ may be taken together as $—(CH_2)_3—$, $—(CH_2)_4—$, $—(CH_2)_5—$ or $—CH_2CH_2OCH_2CH_2—$;
$R^{III}$ is $C_1$—$C_4$ alkyl, $C_3$—$C_4$ alkenyl, $C_3$—$C_4$ alkynyl, $C_2$—$C_4$ haloalkyl, $C_2$—$C_3$ cyanoalkyl, $C_5$—$C_6$ cycloalkyl, $C_4$—$C_7$ cycloalkylalkyl or $C_2$—$C_4$ alkoxyalkyl;
$R_2$ is $C_1$—$C_3$ alkyl;
W is $W_1$ or $W_2$;
$W_1$ is $C_2$—$C_8$ alkenyl or $C_4$—$C_7$ cycloalkenyl either of which may be optionally substituted with 1—3 atoms of F, Cl or Br,. OH, CN, $NO_2$, $C_1$—$C_3$ alkoxy, $C_1$—$C_3$ alkylthio, $C_1$—$C_3$alkylsulfinyl, $C_1$—$C_3$ alkylsulfonyl, amino, $C_2$—$C_4$ alkenyl, $C_1$—$C_3$ alkylamino, di($C_1$—$C_3$ alkyl)amino, ($C_1$—$C_3$ alkylamino)sulfamoyl, di($C_1$—$C_3$ alkylamino)sulfamoyl, $OC(O)C_1$—$C_3$ alkyl, $OC(O)C_1$—$C_3$ alkoxy, $OSO_2C_1$—$C_3$ alkyl, $OSO_2C_6H_5$, $OSO_2$—4—$CH_3$—$C_6H_4$, phenyl or phenyl substituted with halogen, $CH_3$, $OCH_3$, $SCH_3$ or $NO_2$;
$W_2$ is $C_2$ alkynyl which may be substituted with phenyl or phenyl substituted with halogen, $CH_3$, $OCH_3$, $SCH_3$ or $NO_2$ or $W_2$ is $C_3$—$C_8$ alkynyl which may be optionally substituted with 1—3 atoms of F, Cl or Br, OH, CN, $NO_2$, $C_1$—$C_3$ alkoxy, $C_1$—$C_3$ alkylthio, $C_1$—$C_3$ alkylsulfinyl, $C_1$—$C_3$ alkylsulfonyl, amino, $C_1$—$C_3$ alkylamino, di($C_1$—$C_3$ alkyl)amino, ($C_1$—$C_3$ alkylamino)sulfamoyl, di($C_1$—$C_3$ alkylamino)sulfamoyl, $OC(O)C_1$—$C_3$ alkyl, $OC(O)C_1$—$C_3$ alkoxy, $OSO_2C_1$—$C_3$ alkyl, $OSO_2C_6H_5$, $OSO_2$—4—$CH_3$—$C_6H_4$, phenyl or phenyl substituted with halogen, $CH_3$, $OCH_3$, $SCH_3$ or $NO_2$;
Q is a 3- or 4-membered heterocyclic ring which contains one heteroatom selected from O, S and $Nr_3$, or a 3- or 4-membered carbocyclic ring in which one carbon atom may optionally be in the form of a carbonyl group, or a fully-saturated 5- or 6-membered carbocyclic ring, and Q may be optionally substituted with 1—4 substituents selected from halogen, CN, OH, $C_1$—$C_3$ alkyl, $C_1$—$C_3$ alkoxy, $C_1$—$C_3$

alkylthio, $C_2$—$C_3$ alkoxycarbonyl, $C_1$—$C_4$ alkylsulfonyl, ($C_1$—$C_4$ alkyl)aminosulfamoyl and di($C_1$—$C_4$ alkyl)aminosulfamoyl;

$R_3$ is $C_1$—$C_4$ alkyl;

$R_{12}$ is H or $OR_{14}$;

$R_{13}$ is H or $C_1$—$C_4$ alkyl;

$R_{14}$ is H, $C_1$—$C_4$ alkyl, $C_3$—$C_4$ alkenyl, $C_3$—$C_4$ haloalkenyl, $C_3$—$C_4$ alkynyl, $C_3$—$C_4$ haloalkynyl, $C_2$—$C_4$ alkylcarbonyl, $C(O)NR_{15}R_{16}$, $C_1$—$C_4$ alkylsulfonyl, $C_2$—$C_4$ alkoxyalkyl or $C_2$—$C_4$ alkylathioalkyl;

$R_{15}$ and $R_{16}$ are independently H or $C_1$—$C_2$ alkyl;

$R_{17}$ is H or $C_1$—$C_4$ alkyl;

A is

A is 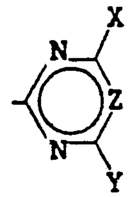 , 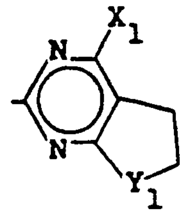 , 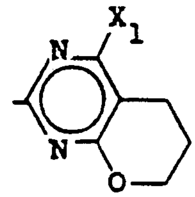 ,

A-1 A-2 A-3

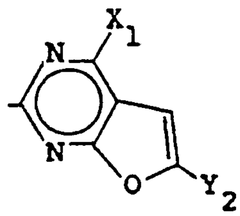 , 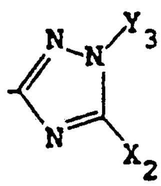 , 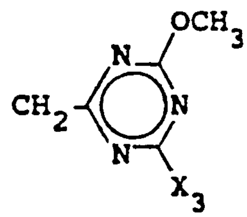 or

A-4 A-5 A-6

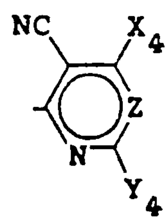 ;

A-7

X is H, $C_1$—$C_4$ alkyl, $C_1$—$C_4$ alkoxy, $C_1$—$C_4$ haloalkoxy, $C_1$—$C_4$ haloalkyl, $C_1$—$C_4$ haloalkylthio, $C_1$—$C_4$ alkylthio, halogen, $C_2$—$C_5$ alkoxyalkyl, $C_2$—$C_5$ alkoxyalkoxy, amino, $C_1$—$C_3$ alkylamino or di($C_1$—$C_3$ alkyl)amino;

Y is H, $C_1$—$C_4$ alkyl, $C_1$—$C_4$ alkoxy, $C_1$—$C_4$ haloalkoxy, $C_1$—$C_4$ haloalkylthio, $C_1$—$C_4$ alkylthio, $C_2$—$C_5$ alkoxyalkyl, $C_2$—$C_5$ alkoxyalkoxy, amino, $C_1$—$C_3$ alkylamino, di($C_1$—$C_3$ alkyl)amino, $C_3$—$C_4$ alkenyloxy, $C_3$—$C_4$ alkynyloxy, $C_2$—$C_5$ alkylthioalkyl, $C_1$—$C_4$ haloalkyl, $C_3$—$C_5$ cycloalkyl,

$C_2$-$C_4$ alkynyl, 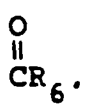 , 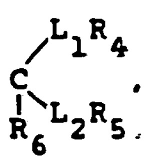 , 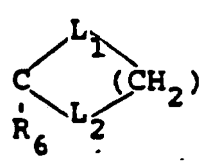 , 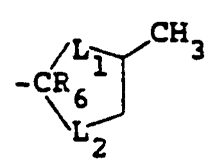

or $N(OCH_3)CH_3$;

m is 2 or 3;

$L_1$ and $L_2$ are independently O or S;

$R_4$ and $R_5$ are independently $C_1$—$C_2$ alkyl;

$R_6$ is H or $CH_3$;

Z is CH or N;

$Y_1$ is O or $CH_2$;

$X_1$ is $CH_3$, $OCH_3$, $OC_2H_5$ or $OCF_2H$;

$Y_2$ is H or $CH_3$;

$X_2$ is $CH_3$, $OCH_3$ or $SCH_3$;
$Y_3$ is $CH_3$, $CH_2CH_3$ or $CH_2CF_3$;
$X_3$ is $CH_3$ or $OCH_3$;
$Y_4$ is $CH_3$, $OCH_3$, $OC_3H_5$ or Cl; and
$X_4$ is $CH_3$, $OCH_3$, $OC_2H_5$ or $CH_2$ $OCH_3$;

and their agriculturally suitable salts;
provided that

a) when X is Cl, F, Br or I, then Z is CH and Y is $OCH_3$, $OC_2H_5$, $N(OCH_3)CH_3$, $NHCH_3$, $N(CH_3)_2$ or $OCF_2H$;
b) when X or Y is $OCF_2H$ then Z is CH;
c) when L is L—2 or L—3, then the W substituent and the sulfonylurea bridge are on adjacent carbon atoms, and when L is L—9 or L—10, then Q and the sulfonylurea bridge are on adjacent carbon atoms;
d) when $W_1$ is unsubstituted $C_3$ alkenyl, then L is L—3, L—4, L—5 or L—6;
e) when L is L—1, then n is 1 and W is $W_2$;
f) when L is L—1 and $W_2$ is $C_2$ or $C_3$ alkynyl, said group must be substituted;
g) when $W_1$ is $C_3$—$C_8$ alkenyl or $C_4$—$C_7$ cycloalkenyl substituted with OH, then the OH substituent is not bonded directly to the olefinic group;
h) when L is L—3, then the sulfonylurea bridge is bonded at the 3- or 5-position;
i) when $W_3$ is S, then R is H, A is A—1, and Y is $CH_3$, $OCH_3$, $OC_2H_5$, $CH_2OCH_3$, $C_2H_5$, $CF_3$, $SCH_3$, $OCH_2CH{=}CH_2$, $OCH_2C{\equiv}CH$, $OCH_2CH_2OCH_3$ or $CH(OCH_3)_2$;
j) when the total number of carbon atoms of X and Y is greater than four, then the number of carbons of $R_1$ must be less than or equal to two and either the number of carbons of W must be less than or equal to four, or the number of carbons of Q must be less than or equal to six; and
k) the total number of carbon atoms of $R_{12}$ and $R_{13}$ must be less than or equal to four.

2. The compounds of Claim 1 where
R is H;
$W_3$ is O; and
Q is

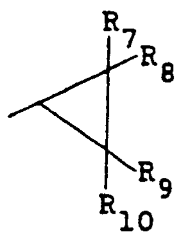
Q-1
,
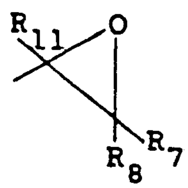
Q-2
,
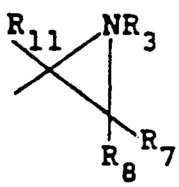
Q-3
,
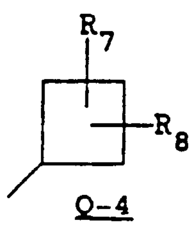
Q-4
,
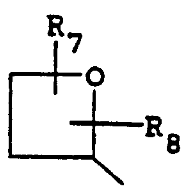
Q-5
,
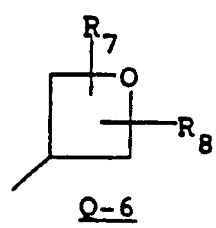
Q-6
,
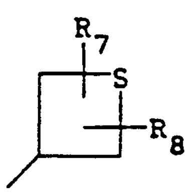
Q-7
,
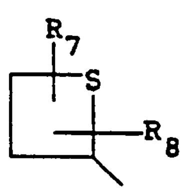
Q-8
,
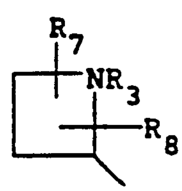
Q-9

or
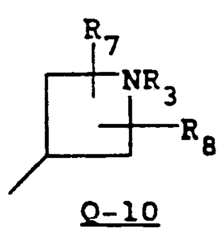
Q-10
;

wherein

$R_3$ is $C_1$—$C_3$ alkyl;

$R_7$ is H, F, Cl, Br, CN, $CH_3$ or $OCH_3$;

$R_8$ is H, F, Cl, Br, I, CN, OH, $C_1$—$C_3$ alkyl, $C_1$—$C_3$ alkoxy, $C_2$—$C_3$ alkoxycarbonyl, di($C_1$—$C_3$ alkyl)aminosulfamoyl, $C_1$—$C_3$ alkylthio or $C_1$—$C_3$ alkylsulfonyl;

$R_9$ and $R_{10}$ are independently H, $CH_3$, F, Cl or may be taken together to form C=O; and

$R_{11}$ is H or $C_1$—$C_3$ alkyl;

provided that

when $R_7$ and $R_8$ are attached to the same carbon atoms as in Q—1, Q—2 or Q—3, or in Q—4 through Q—10, and $R_7$ is $OCH_3$, then $R_8$ is other than F, Cl, Br, I or OH.

3. The compounds of Claim 2 where

$R_1$ is H, F, Cl, $NO_2$, $C_1$—$C_2$ alkyl, $C_1$—$C_2$ haloalkyl, $C_1$—$C_2$ alkoxy, $C_1$—$C_2$ alkylthio, $CH_2OCH_3$ or $CH_2SCH_3$, and when L is L—1, L—4, L—5, L—6, L—7, L—8, L—11, L—12 or L—13, $R_1$ is not *para* to the sulfonylurea bridge.

4. The compounds of Claim 3 where

$R_{13}$ is H or $CH_3$;

$R_{14}$ is H, $C_1$—$C_2$ alkyl, $CH_2CH{=}CH_2$ or $CH_2C{\equiv}CH$;

X is $CH_3$, $OCH_3$, $OC_2H_5$, Cl, F, Br, $OCF_2H$, $CH_2F$, $OCH_2CH_2F$, $OCH_2CHF_2$, $OCH_2CF_3$, $CF_3$, $CH_2Cl$ or $CH_2Br$; and

Y is H, $C_1$—$C_3$ alkyl, $OCH_3$, $OC_2H_5$, $CH_2OCH_3$, $NHCH_3$, $N(OCH_3)CH_3$, $N(CH_3)_2$, $CF_3$, $SCH_3$, $OCH_2CH{=}CH_2$, $OCH_2C{\equiv}CH$, $CH_2OC_2H_5$, $OCH_2CH_2OCH_3$, $CH_2SCH_3$, $OCF_2H$, $SCF_2H$, cyclopropyl, $C{\equiv}CH$ or $C{\equiv}CCH_3$.

5. The compounds of Claim 4 where

$W_1$ is $C_2$—$C_5$ alkenyl which may be optionally substituted by 1—3 atoms of F, Cl or Br, OH, $OCH_3$, $OC(O)CH_3$, $OSO_2CH_3$, CN or $SCH_3$, or $W_1$ is $C_5$—$C_6$ cycloalkenyl; and

$W_2$ is $C{\equiv}CC_6H_5$ or $C_3$—$C_5$ alkynyl which may be optionally substituted by 1—3 atoms of F, Cl or Br, OH, $OCH_3$, $OC(O)CH_3$, CN or $SCH_3$.

6. The compounds of Claim 5 where

A is A—1;

X is $CH_3$, $OCH_3$, $OC_2H_5$, Cl or $OCF_2H$;

Y is $CH_3$, $C_2H_5$, $OCH_3$, $CH_2OCH_3$, $CH(OCH_3)_2$, $OCF_2H$, $NHCH_3$, $N(CH_3)_2$ or cyclopropyl; and

$R_1$ is H, $CH_3$, $OCH_3$, $SCH_3$ or Cl.

7. The compound of Claim 1 which is 2-(1-cyclohexen-2-yl)-N-[4-methoxy-6-methylpyrimidin-2-yl)aminocarbonyl]-3-thiophenesulfonamide.

8. The compound of Claim 1 which is 2-(1-cyclohexen-2-yl)-N-[(4-methoxy-6-methyl-1,3,5-triazin-2-yl)aminocarbonyl]-3-thiophenesulfonamide.

9. The compound of claim 1 which is N-[(4-methoxy-6-methylpyrimidin-2-yl)aminocarbonyl]-1-methyl-4-(1-propenyl)-1H-pyrazole-5-sulfonamide.

10. The compound of claim 1 which is 2-(2,2-dichlorocyclopropyl)-N-[(4,6-dimethoxypyrimidin-2-yl)aminocarbonyl]benzenesulfonamide.

11. The compound of claim 1 which is 2-(2,2-dichlorocyclopropyl)-N-[(4-methoxy-6-methyl-1,3,5-triazin-2-yl)aminocarbonyl]benzenesulfonamide.

12. The compound of claim 1 which is N-[(4,6-dimethoxypyrimidin-2-yl)aminocarbonyl]-2-(2-oxiranylmethyl)benzenesulfonamide.

13. A compound of claim 1 wherein:

$W_3$ is O;

$R_1$ is H, $C_1$—$C_3$ alkyl or haloalkyl, halogen, nitro, $C_1$—$C_3$ alkoxy, $SO_2NR^IR^{II}$, $C_1$—$C_3$ alkylthio, $C_1$—$C_3$ alkylsulfinyl, $C_1$—$C_3$ alkylsulfonyl or $CO_2R^{III}$;

W is $C_2$—$C_5$ alkenyl, $C_2$—$C_5$ alkynyl, $C_5$—$C_6$ cycloalkenyl, any of which may optionally be substituted with 1—4 substituents selected from halogen, cyano, $C_1$—$C_3$ alkyl, $C_1$—$C_3$ alkoxy, $C_1$—$C_3$ alkylthio, $C_1$—$C_3$ alkoxycarbonyl, $C_1$—$C_4$ alkylsulfonyl or ($C_1$—$C_4$ alkyl)aminosulfamoyl or di($C_1$—$C_4$ alkyl)aminosulfamoyl;

Q is a 3- or 4-membered carbocyclic or heterocyclic ring which contains 0—1 heteroatoms selected from O, S or $NR_3$, said ring optionally being substituted as defined for W above;

$R_{12}$ and $R_{13}$ are H; and

A is selected from A—1 to A—6.

14. An agriculturally suitable composition for controlling the growth of undesired vegetation which comprises an effective amount of compound of any of claims 1—13 and at least one of the following: surfactant, solid or liquid diluent.

15. A method for controlling the growth of undesired vegetation which comprises applying to the locus to be protected an effective amount of a compuond of any of claims 1—13.

16. A method for regulating the growth of plants which comprises applying to the locus of such plants an effective but substantially non-phytotoxic amount of a plant growth regulant selected from compounds of any of claims 1 to 13.

17. A process for the preparation of a compound of claim 1 which comprises:
(a) reacting an appropriate sulfonylisocyanate or isothiocyanate of formula

$$LSO_2NCW_3 \qquad (II)$$

with an appopriately substituted aminoheterocycle of formula

$$RNHA \qquad (III)$$

wherein R, $W_3$, A and H are as defined in claim 1; or
(b) reacting a sulfonamide of formula

$$LSO_2NH_2 \qquad (IV)$$

with a carbamate of formula

$$R'O\overset{\overset{\displaystyle O}{\|}}{C}NRA \qquad (V)$$

wherein L, R and A are as defined in claim 1, except that $R_1$ is other than $CO_2R'''$, and R' is $CH_3$ or Ph.

18. Sulfonyl isocyanates and isothiocyanates of formula (II) as defined in claim 17 and sulfonamides of formula (IV) as defined in claim 17.

**Patentansprüche**

1. Verbindung der Formel:

$$LSO_2NH\overset{\overset{\displaystyle W_3}{\|}}{C}\underset{\underset{\displaystyle R}{|}}{N}A$$

worin L /

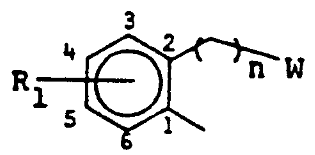
L-1
,
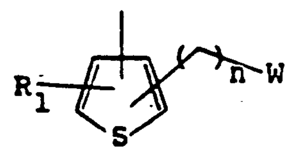
L-2
,
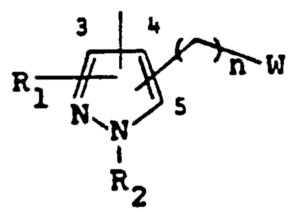
L-3
,
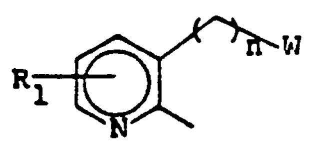
L-4
,
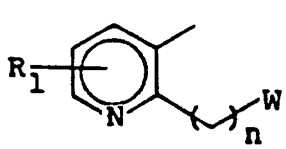
L-5
,
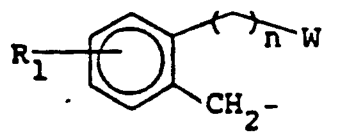
L-6
,
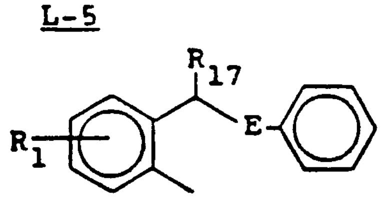
L-7
.

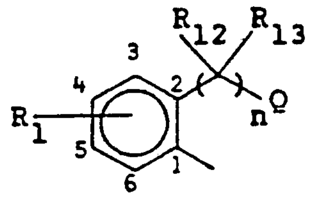 ,

L-8

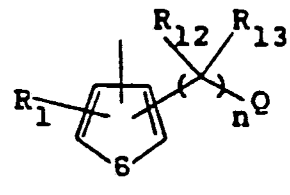 ,

L-9

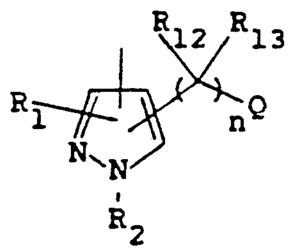 ,

L-10

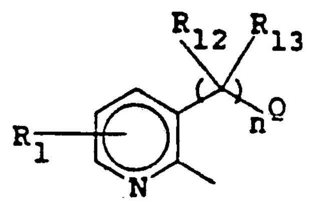 ,

L-11

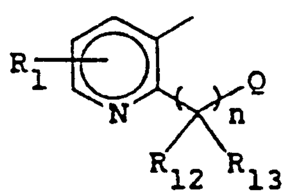

L-12

oder

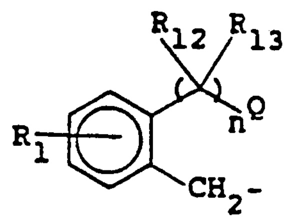 ist;

L-13

R H oder $CH_3$ ist;

$W_3$ O oder S ist;

n 0 oder 1 ist;

E $S(O)_p$ oder Se ist;

p 0, 1 oder 2 ist;

$R_1$ H, $C_1$—$C_3$-Alkyl, $C_1$—$C_3$-Halogenalkyl, Halogen, Nitro, $CH_2Cn$, $CH_2OCH_3$, $CH_2SCH_3$, CN, $C_1$—$C_3$-Alkoxy, $SO_2NR^IR^{II}$, $C_1$—$C_3$-Alkylthio, $C_1$—$C_3$-Alkylsulfinyl, $C_1$—$C_3$-Alkylsulfonyl oder $CO_2R^{III}$ ist;

$R^I$ H, $C_1$—$C_4$-Alkyl, $C_2$—$C_3$-Cyanalkyl, Methoxy oder Ethoxy ist;

$R^{II}$ H, $C_1$—$C_4$-Alkyl oder $C_3$—$C_4$-Alkenyl ist; oder

$R^I$ und $R^{II}$ als —$(CH_2)_3$—, —$(CH_2)_4$—, —$(CH_2)_5$— oder —$(CH_2CH_2OCH_2CH_2$— zusammengenommen werden kann;

$R^{III}$ $C_1$—$C_4$-Alkyl, $C_3$—$C_4$-Alkenyl, $C_3$—$C_4$-Alkinyl, $C_2$—$C_4$-Halogenalkyl, $C_2$—$C_3$-Cyanalkyl, $C_5$—$C_6$-Cycloalkyl, $C_4$—$C_7$-Cycloalkylalkyl oder $C_2$—$C_4$-Alkoxyalkyl ist;

$R_2$ $C_1$—$C_3$-Alkyl ist;

W $W_1$ oder $W_2$ ist;

$W_1$ $C_2$—$C_8$-Alkenyl oder $C_4$—$C_7$-Cycloalkenyl, ist, von denen beide gegebenenfalls mit 1—3 Atomen aus F, Cl oder Br, OH, CN, $NO_2$, $C_1$—$C_3$-Alkoxy, $C_1$—$C_3$-Alkylthio, $C_1$—$C_3$-Alkylsulfinyl, $C_1$—$C_3$-Alkylsulfonyl, Amino, $C_2$—$C_4$-Alkenyl, $C_1$—$C_3$-Alkylamino, Di($C_1$—$C_3$-alkyl)amino, ($C_1$—$C_3$-alkylamino)sulfamoyl, Di($C_1$—$C_3$-alkylamino)sulfamoyl, $OC(O)C_1$—$C_3$-Alkyl, $OC(O)C_1$—$C_3$-Alkoxy, $OSO_2C_1$—$C_3$-Alkyl, $OSO_2C_6H_5$, $OSO_2$—4—$CH_3$—$C_6H_4$, Phenyl oder durch Halogen, $CH_3$, $OCH_3$, $SCH_3$ oder $NO_2$ substituiertes Phenyl substituiert sein kann;

$W_2$ $C_2$-Alkinyl ist, das durch Phenyl oder durch Halogen, $CH_3$, $OCH_3$, $SCH_3$ oder $NO_2$ substituiertes Phenyl substituiert sein kann, oder $W_2$ $C_3$—$C_8$-Alkinyl ist, das gegebenenfalls mit 1—3 Atomen aus F, Cl oder Br, OH, CN, $NO_2$, $C_1$—$C_3$-Alkoxy- $C_1$—$C_3$-Alkylthio, $C_1$—$C_3$-Alkylsulfinyl, $C_1$—$C_3$-Alkylsulfonyl, Amino, $C_1$—$C_3$-Alkylamino, Di($C_1$—$C_3$-alkyl)amino, ($C_1$—$C_3$-Alkylamino)sulfamoyl, Di($C_1$—$C_3$-alkylamino)sulfamoyl, $OC(O)C_1$—$C_3$-Alkyl, $OC(O)C_1$—$C_3$-Alkoxy, $OSO_2C_1$—$C_3$-Alkyl, $OSO_2C_6H_5$, $OSO_2$—4—$CH_3$—$C_6H_4$, Phenyl oder durch Halogen, $CH_3$, $OCH_3$, $SCH_3$ oder $NO_2$ substituiertes Phenyl substituiert sein kann;

Q ein 3- oder 4-gliedriger heterocyclischer Ring ist, der ein aus O, S und $NR_3$ ausgewähltes Heteroatom enthält, oder ein 3- oder 4-gliedriger carbocyclischer Ring, in dem ein Kohlenstoffatom gegebenenfalls in Form einer Carbonylgruppe vorliegt, oder ein vollständig gesättigter 5- oder 6-gliedriger carbocyclischer Ring, und Q gegebenenfalls mit 1 bis 4 aus Halogen, CN, OH, $C_1$—$C_3$-Alkyl, $C_1$—$C_3$-Alkoxy, $C_1$—$C_3$-Alkylthio,

$C_2$—$C_3$-Alkoxycarbonyl, $C_1$—$C_4$-Alkylsulfonyl, ($C_1$—$C_4$-Alkyl)aminosulfamoyl und Di($C_1$—$C_4$-alkyl)aminosulfamoyl substituiert sein kann;

$R_3$ $C_1$—$C_4$-Alkyl ist;

$R_{12}$ H oder $OR_{14}$ ist;

$R_{13}$ H oder $C_1$—$C_4$-Alkyl ist;

$R_{14}$ H, $C_1$—$C_4$-Alkyl, $C_3$—$C_4$-Alkenyl, $C_3$—$C_4$-Halogenalkenyl, $C_3$—$C_4$-Alkinyl, $C_3$—$C_4$-Halogenalkinyl, $C_2$—$C_4$-Alkylcarbonyl, $C(O)NR_{15}R_{16}$, $C_1$—$C_4$-Alkylsulfonyl, $C_2$—$C_4$-Alkoxyalkyl oder $C_2$—$C_4$-Alkylthioalkyl ist;

$R_{15}$ und $R_{16}$ unabhängig H oder $C_1$—$C_2$-Alkyl sind;

$R_{17}$ H oder $C_1$—$C_4$-Alkyl ist;

A is 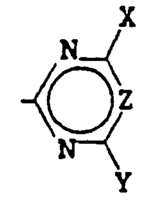 , 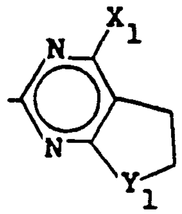 , 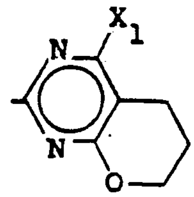 ,

A-1 A-2 A-3

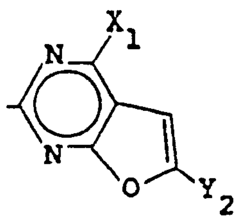 , 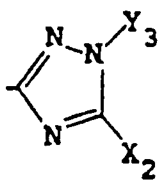 , 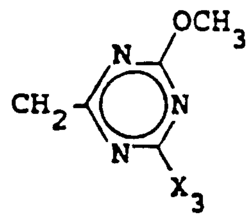 oder

A-4 A-5 A-6

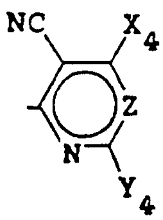 ist;

A-7

X H, $C_1$—$C_4$-Alkyl, $C_1$—$C_4$-Alkoxy, $C_1$—$C_4$-Halogenalkoxy, $C_1$—$C_4$-Halogenalkyl, $C_1$—$C_4$-Halogenalkylthio, $C_1$—$C_4$-Alkylthio, Halogen, $C_2$—$C_5$-Alkoxyalkyl, $C_2$—$C_5$-Alkoxyalkoxy, Amino, $C_1$—$C_3$-Alkylamino oder Di($C_1$—$C_3$-alkyl)amino ist;

Y H, $C_1$—$C_4$-Alkyl, $C_1$—$C_4$-Alkoxy, $C_1$—$C_4$-Halogenalkoxy, $C_1$—$C_4$-Halogenalkylthio, $C_1$—$C_4$-Alkylthio, $C_2$—$C_5$-Alkoxyalkyl, $C_2$—$C_5$-Alkoxyalkoxy, Amino, $C_1$—$C_3$-Alkylamino, Di($C_1$—$C_3$-alkyl)amino, $C_3$—$C_4$-Alkenyloxy, $C_3$—$C_4$-Alkinyloxy, $C_2$—$C_5$-Alkylthioalkyl, $C_1$—$C_4$-Halogenalkyl, $C_3$—$C_5$-Cycloalkyl,

$C_2$-$C_4$ alkinyl, 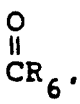 , 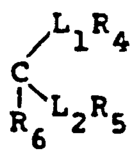 , 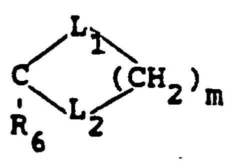 , 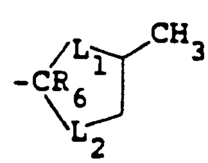

oder $N(OCH_3;CH_3$ ist;

m 2 oder 3 ist;

$L_1$ und $L_2$ unabhängig O oder S sind;

$R_4$ und $R_5$ unabhängig $C_1$—$C_2$-Alkyl sind;

$R_6$ H oder $CH_3$ ist;

Z CH oder N ist;

$Y_1$ O oder $CH_2$ ist;

$X_1$ $CH_3$, $OCH_3$, $OC_2H_5$ oder $OCF_2H$ ist;

$Y_2$ H oder $CH_3$ ist;

$X_2$ $CH_3$, $OCH_3$ oder $SCH_3$ ist;

$Y_3$ $CH_3$, $CH_2CH_3$ oder $CH_2CF_3$ ist;

$X_3$ $CH_3$ oder $OCH_3$ ist;
$Y_4$ $CH_3$, $OCH_3$, $OC_2H_5$ oder Cl ist; und
$X_4$ $CH_3$, $OCH_3$, $OC_2H_5$ oder $CH_2OCH_3$ ist;
sowie ihre landwirtschaftlich geeigneten Salze;
mit der maßgabe, daß
a) wenn X Cl, F, Br oder I ist, dann Z CH ist und Y $OCH_3$, $OC_2H_5$, $N(OCH_3)CH_3$, $NHCH_3$, $N(CH_3)_2$ oder $OCF_2H$ ist;
b) wenn X oder Y $OCF_2H$ ist, dann Z CH ist;
c) wenn L L—2 oder L—3 ist, dann der W-Substituent und die Sulfonylharnstoffbrücke, sich an benachbarten Kohlenstoffatomen befinden, und wenn L L—9 oder L—10 ist, dann Q und die Sulfonyl harnstoffbrücke sich an benachbarten Kohlenstoffatomen befinden;
d) wenn $W_1$ unsubstituiertes $C_3$-Alkenyl ist, dann L L—3, L—4, L—5 oder L—6 ist;
e) wenn L L—1 ist, dann n 1 ist und W $W_2$ ist;
f) wenn L L—1 ist und $W_2$ $C_2$— oder $C_3$-Alkinyl ist; die Gruppe substituiert sein muß;
g) wenn $W_1$ mit OH substituiertes $C_3$—$C_8$-Alkenyl oder $C_4$—$C_7$-Cycloalkenyl ist, dann der OH-Substituent nicht direkt an die olefinische Gruppe gebunden ist;
h) wenn L L—3 ist, dann die Sulfonylharnstoffbrücke in der 3- oder 5-Stellung gebunden ist;
i) wenn $W_3$ S ist, dann R H ist, A A—1 ist und Y $CH_3$, $OCH_3$, $OC_2H_5$, $CH_2OCH_3$, $C_2H_6$, $CF_3$, $SCH_3$, $OCH_2CH=CH_2$, $OCH_2C\equiv CH$, $OCH_2CH_2OCH_3$ oder $CH(OCH_3)_2$ ist;
j) wenn die Gesamtzahl der Kohlenstoffatome von X und Y größer als vier ist, dann die Zahl der Kohlenstoffe von $R_1$ kleiner oder gleich zwei sein muß und entweder die Zahl der Kohlenstoffe von W kleiner oder gleich vier oder die Zahl der Kohlenstoffe von Q kleiner oder gleich sechs sein muß; und
k) die Gesamtzahl der Kohlenstoffatome von $R_{12}$ und $R_{13}$ kleiner oder gleich vier sein muß.
2. Verbindungen nach Anspruch 1, worin
R H ist;
$W_3$ O ist; und
Q

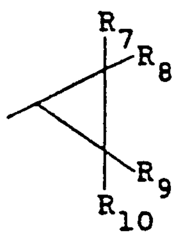
Q-1
,
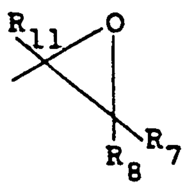
Q-2
,
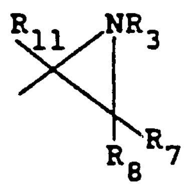
Q-3
,
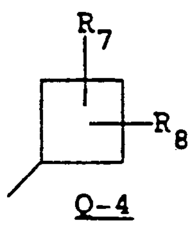
Q-4
,
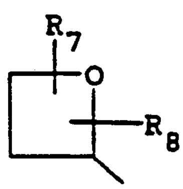
Q-5
,
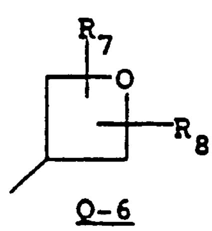
Q-6
,
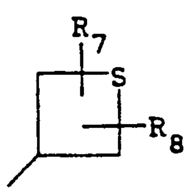
Q-7
,
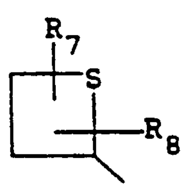
Q-8
,
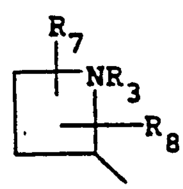
Q-9

oder
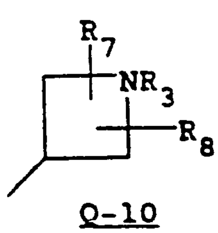
Q-10
ist;

worin

$R_3$ $C_1$—$C_3$-Alkyl ist;

$R_7$ H, F, Cl, Br, CN, $CH_3$ oder $OCH_3$ ist;

$R_8$ H, F, Cl, Br, I, CN, OH, $C_1$—$C_3$-Alkyl, $C_1$—$C_3$-Alkoxy, $C_2$—$C_3$-Alkoxycarbonyl, Di($C_1$—$C_3$-alkyl)aminosulfamoyl, $C_1$—$C_3$-Alkylthio oder $C_1$—$C_3$-Alkylsulfonyl ist;

$R_9$ und $R_{10}$ unabhängig H, $CH_3$, F, Cl sind oder unter Bildung von C=O zusammengenommen werden können; und

$R_{11}$ H oder $C_1$—$C_3$-Alkyl ist;

mit der Maßgabe, daß wenn $R_7$ und $R_8$ an das gleiche Kohlenstoffatom gebunden sind, wie in Q—1, Q—2 oder Q—3 oder in Q—4 bis Q—10, und $R_7$ $OCH_3$ ist, dann $R_8$ von F, Cl, Br, I oder OH verschieden ist.

3. Verbindung nach Anspruch 2, worin

$R_1$ H, F, Cl, $NO_2$, $C_1$—$C_2$-Alkyl, $C_1$—$C_2$-Halkogenalkyl, $C_1$—$C_2$-Alkoxy, $C_1$—$C_2$-Alkylthio, $CH_2OCH_3$ oder $CH_2SCH_3$ ist und, wenn L L—1, L—4, L—5, L—6, L—7, L—8, L—11, L—12 oder L—13 ist, $R_1$ nicht *para* zur Sulfonylharnstoffbrücke angeordnet ist.

4. Verbindung nach Anspruch 3, worin

$R_{13}$ H oder $CH_3$ ist;

$R_{14}$ H, $C_1$—$C_2$-Alkyl, $CH_2CH{=}CH_2$ oder $CH_2C{\equiv}CH$ ist;

X $CH_3$, $OCH_3$, $OC_2H_5$, Cl, F, Br, $OCF_2H$, $CH_2F$, $OCH_2CH_2F$, $OCH_2CHF_2$, $OCH_2CF_3$, $CF_3$, $CH_2Cl$ oder $CH_2Br$ ist; und

Y H, $C_1$—$C_3$-Alkyl, $OCH_3$, $OC_2H_5$, $CH_2OCH_3$, $NHCH_3$, $N(OCH_3)CH_3$, $N(CH_3)_2$, $CF_3$, $SCH_3$, $OCH_2CH{=}CH_2$, $OCH_2C{\equiv}CH$, $CH_2OC_2H_5$, $OCH_2CH_2OCH_3$, $CH_2SCH_3$, $OCF_2H$, $SCF_2H$, Cyclopropyl, $C{\equiv}CH$ oder $C{\equiv}CCH_3$ ist.

5. Verbindung nach Anspruch 4, worin

$W_1$ $C_2$—$C_5$-Alkenyl ist, das gegebenenfalls durch 1 bis 3 Atome aus F, Cl oder Br, OH, $OCH_3$, $OC(O)CH_3$, $OSO_2CH_3$, CN oder $SCH_3$ substituiert sein kann, oder $W_1$ $C_5$—$C_6$-Cycloalkenyl ist; und

$W_2$ $C{\equiv}CC_6H_5$ oder $C_3$—$C_5$-Alkinyl ist, gegebenenfalls substituiert durch 1—3 Atome aus F, Cl oder Br, OH, $OCH_3$, $OC(O)CH_3$, CN oder $SCH_3$.

6. Verbindungen nach Anspruch 5, worin

A A—1 ist;

X $CH_3$, $OCH_3$, $OC_2H_5$, Cl oder $OCF_2H$ ist;

Y $CH_3$, $C_2H_5$, $OCH_3$, $CH_2OCH_3$, $CH(OCH_3)_2$, $OCF_2H$, $NHCH_3$, $N(CH_3)_2$ oder Cyclopropyl ist; und

$R_1$ H, $CH_3$, $OCH_3$, $SCH_3$ oder Cl ist.

7. Verbindung nach Anspruch 1, welche 2-(1-Cyclohexen-2-yl)-N-[4-methoxy-6-methylpyrimidin-2-yl)-aminocarbonyl]-3-thiophensulfonamid ist.

8. Verbindung nach Anspruch 1, welche 2-(1-Cyclohexen-2-yl)-N-[(4-methoxy-6-methyl-1,3,5-triazin-2-yl)aminocarbonyl]-3-thiophensulfonamid ist.

9. Verbindung nach Anspruch 1, welche N-[(4-Methoxy-6-methylpyrimidin-2-yl)aminocarbonyl]-1-methyl-4-(1-propenyl)-1H-pyrazol-5-sulfonamid ist.

10. Verbindung nach Anspruch 1, welche 2-(2,2-Dichlorocyclopropyl)-N-[(4,6-dimethoxypyrimidin-2-yl)aminocarbonyl]benzolsulfonamid ist.

11. Verbindung nach Anspruch 1, welche 2-(2,2-Dichlorcyclopropyl)-N-[(4-methoxy-6-methyl-1,3,5-triazin-2-yl)aminocarbonyl]benzolsulfonamid ist.

12. Verbindung nach Anspruch 1, welche N-[(4,6-Dimethoxypyrimidin-2-yl)aminocarbonyl]-2-(2-oxiranylmethyl)benzolsulfonamid ist.

13. Verbindung nach Anspruch 1, worin

$W_3$ O ist;

$R_1$ H, $C_1$—$C_3$-Alkyl oder Halogenalkyl, Halogen, Nitro, $C_1$—$C_3$--Alkoxy, $SO_2NR^IR^{II}$, $C_1$—$C_3$-Alkylthio, $C_1$—$C_3$-Alkylsulfinyl, $C_1$—$C_3$-Alkylsulfonyl oder $CO_2R^{III}$ ist;

W $C_2$—$C_5$-Alkenyl, $C_2$—$C_5$-Alkinyl, $C_5$—$C_6$-Cycloalkenyl, von denen jedes gegebenenfalls mit 1—4 aus Halogen, Cyan, $C_1$—$C_3$-Alkyl, $C_1$—$C_3$-Alkoxy, $C_1$—$C_3$-Alkylthio, $C_1$—$C_3$-Alkoxycarbonyl, $C_1$—$C_4$-Alkylsulfonyl oder ($C_1$—$C_4$-Alkyl)aminosulfamoyl oder Di($C_1$—$C_4$-alkyl)aminosulfamoyl ausgewählten Substituenten substituiert sein kann;

Q ein 3- oder 4-gliedriger carbocyclischer oder heterocyclischer Ring ist, der 0 bis 1 aus O, S oder $NR_3$ ausgewählte Heteroatome enthält, wobei der Ring gegebenenfalls substituiert ist, wie für W oben definiert;

$R_{12}$ und $R_{13}$ H sind; und

A aus A—1 bis A—6 ausgewählt ist.

14. Landwirtschaftlich geeignete Zusammensetzung zur Bekämpfung des Wachstums unerwünschter Vegetation, welche eine wirksame Menge einer Verbindung nach einem der Ansprüche 1 bis 13 und wenigstens eines der folgenden: oberflächenaktives Mittel, festes oder flüssiges Verdünnungsmittel, umfaßt.

15. Verfahren zur Bekämpfung des Wachstums unerwünschter Vegetation, welches die Anwendung einer wirksamen Menge einer Verbindung nach einem der Ansprüche 1 bis 13 auf den zu schützenden Ort umfaßt.

16. Verfahren zur Pflanzenwachstumsregulierung, welches die Anwendung einer wirksamen, jedoch im wesentlichen nicht phytotoxischen Menge eines Pflanzenwachstumsregulans, ausgewählt aus Verbindungen nach einem der Ansprüche 1 bis 13, auf den Ort solcher Pflanzen, umfaßt.

17. Verfahren zur Herstellung einer Verbindung nach Anspruch 1 durch
(a) Umsetzung eines geeigneten Sulfonylisocyanats oder Isothiocynanats der Formel

$$LSO_2NCW_3 \qquad (II)$$

mit einem geeignet substituierten Aminoheterocyclus der Formel

$$RNHA \qquad (III)$$

worin R, $W_3$, a und H wie in Anspruch 1 definiert sind; oder
(b) Umsetzung eines Sulfonamids der Formel

$$LSO_2NH_2 \qquad (IV)$$

mit einem Carbamat der Formel

O
||
R'OCNRA (V)

worin L, R und A wie in Anspruch 1 definiert sind, außer daß $R_1$ von $CO_2R'''$ verschieden ist, und R' $CH_3$ oder Phenyl ist.

18. Sulfonylisocyanat und Isothiocyanate der Formel (II), wie in Anspruch 17 definiert, und Sulfonamide der Formel (IV), wie in Anspruch 17 definiert.

## Revendications

1. Un composé de la formula:

$W_3$
||
$LSO_2NHCNA$
|
R

dans laquelle

L est 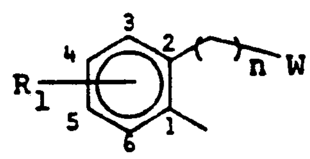 ,

L-1

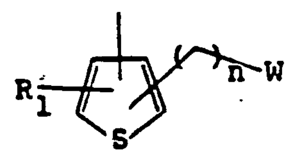 ,

L-2

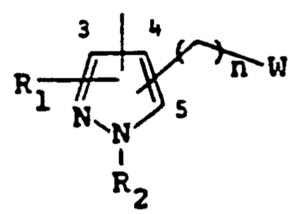 ,

L-3

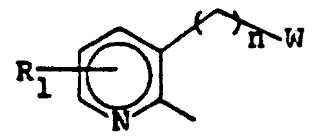 ,

L-4

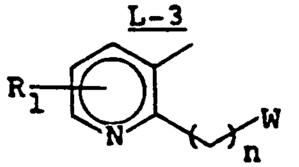 ,

L-5

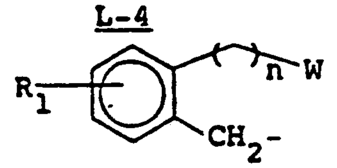 ,

L-6

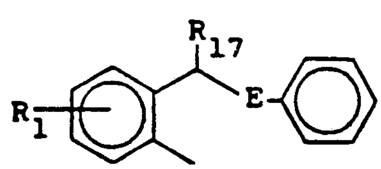 ,

L-7

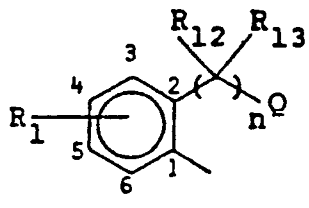

L-8

,

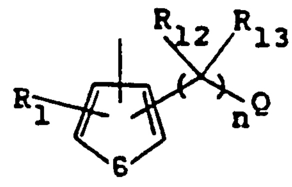

L-9

,

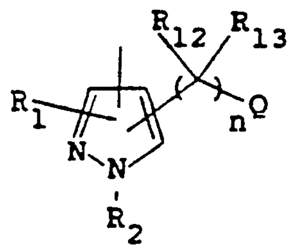

L-10

,

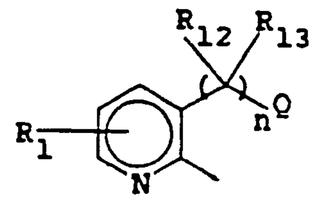

L-11

,

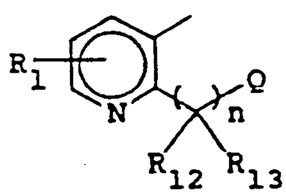

L-12

ou

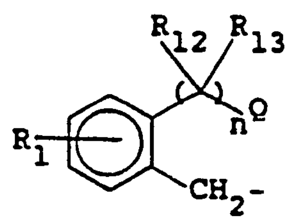

L-13

;

R est H ou $CH_3$;
$W_3$ est O ou S;
n est 0 ur 1;
E est $S(O)_p$ ou Se;
p est 0, 1 ou 2;

$R_1$ est H ou un groupe alkyle en $C_1$—$C_3$, halogénalkyle en $C_1$—$C_3$, halogéno, nitro, $CH_2CN$, $CH_2OCH_3$, $CH_2SCH_3$, CN, alcoxy en $C_1$—$C_3$, $SO_2NR^IR^{II}$, alkylthio en $C_1$—$C_3$, alkylsulfinyle en $C_1$—$C_3$, alkylsulfonyle en $C_1$—$C_3$ ou $CO_2R^{III}$;

$R^I$ est H ou un groupe alkyle en $C_1$—$C_4$, cyanalkyle en $C_2$—$C_3$, méthoxy ou éthoxy;

$R^{II}$ est H ou un groupe alkyle en $C_1$—$C_4$ ou alcényle en $C_3$—$C_4$; ou bien

$R^I$ et $R^{II}$ peuvent être pris ensemble pour former —$(CH_2)_3$—, —$(CH_2)_4$—, —$(CH_2)_5$— ou —$CH_2CH_2OCH_2CH_2$—;

$R^{III}$ est un groupe alkyle en $C_1$—$C_4$, alcényle en $C_3$—$C_4$, alcynyle en $C_3$—$C_4$, halogénalkyle en $C_2$—$C_4$, cyanalkyle en $C_2$—$C_3$, cycloalkyle en $C_5$—$C_6$, cycloalkylalkyle en $C_4$—$C_7$ ou alcoxylalkyle en $C_2$—$C_4$;

$R_2$ est un groupe alkyle en $C_1$—$C_3$;

W est $W_1$ ou $W_2$;

$W_1$ est un groupe alcényle en $C_2$—$C_8$, ou cycloalcényle en $C_4$—$C_7$, dont chacun peut ètre facultativement substitué par 1 à 3 atomes de F, Cl ou Br, OH, CN, $NO_2$, un groupe alcoxy en $C_1$—$C_3$, alkylthio en $C_1$—$C_3$, alkylsulfinyle en $C_1$—$C_3$, alkylsulfonyle en $C_1$—$C_3$, amino, alcényle en $C_2$—$C_4$, alkylamino en $C_1$—$C_3$, di(alkyle en $C_1$—$C_3$)amino, (alkylamino en $C_1$—$C_3$)sulfamyle, di(alkylamino en $C_1$—$C_3$)sulfamyle, OC(O)(alkyle en $C_1$—$C_3$), OC(O)(alcoxy en $C_1$—$C_3$), $OSO_2$(alkyle en $C_1$—$C_3$), $OSO_2C_6H_5$, $OSO_2$—4—$CH_2$—$C_6H_4$, phényle ou phényle substitué par un halogène, $CH_3$, $OCH_3$, $SCH_3$ ou $NO_2$;

$W_2$ est un groupe alcynyle en $C_2$ qui peut être substitué par un groupe phényle ou phényle substitué par un halogène, $CH_3$, $OCH_3$, $SCH_3$ ou $NO_2$, ou bien $W_2$ est un groupe alcynyle en $C_3$—$C_8$ qui peut être facultativement substitué par 1 à 3 atomes de F, Cl ou Br, OH, CN, $NO_2$, un groupe alcoxy en $C_1$—$C_3$, alkylthio en $C_1$—$C_3$, alkylsulfinyle en $C_1$—$C_3$, alkylsulfonyle en $C_1$—$C_3$, amino, alkylamino en $C_1$—$C_3$, di(alkyle en $C_1$—$C_3$)amino, (alkylamino en $C_1$—$C_3$sulfamyle, di(alkylamino en $C_1$—$C_3$)sulfamyle, OC(O)(alkyle en $C_1$—$C_3$), OC(O)(alcoxy en $C_1$—$C_3$), $OSO_2$(alkyle en $C_1$—$C_3$), $OSO_2C_6H_5$, $OSO_2$—4—$CH_3$—$C_6H_4$, phényle ou phényle substitué par un halogène, $CH_3$, $OCH_3$, $SCH_3$ ou $NO_2$;

Q est un hétérocycle à 3 ou ê chaînons qui contient un hétéroatome choisi parmi O, S et $NR_2$, ou un carbocycle à 3 ou 4 chaînons dans lequel l'un des atomes de carbone peut être facultativement sous la forme d'un groupe carbonyle, ou un carbocycle à 5 ou 6 chaînons complètement saturé, et Q peut étre

facultativement substitué par 1 à 4 substituants choisis parmi les halogènes, CN, OH et les groupes alkyles en $C_1$—$C_3$, alcoxy en $C_1$—$C_3$, alkylthio en $C_1$—$C_3$, alcoxycarbonyles en $C_2$—$C_3$, alkylsulfonyles en $C_1$—$C_4$, (alkyle en $C_1$—$C_4$)aminosulfamyles et di(alkyle en $C_1$—$C_4$)aminosulfamyles;

$R_3$ est un groupe alkyle en $C_1$—$C_4$;

$R_{12}$ est H ou $OR_{14}$;

$R_{13}$ est H ou un groupe alkyle en $C_1$—$C_4$;

$R_{14}$ est H ou un groupe alkyle en $C_1$—$C_4$, alcényle en $C_3$—$C_4$, halogénalcényle en $C_3$—$C_4$, alcynyle en $C_3$—$C_4$, halogénalcynyle en $C_3$—$C_4$, alkylcarbonyle en $C_2$—$C_4$, $C(O)NR_{15}R_{16}$, alkylsulfonyle en $C_1$—$C_4$, alcoxyalkyle en $C_2$—$C_4$ ou alkylthioalkyle en $C_2$—$C_4$;

$R_{15}$ et $R_{16}$ sont chacun indépendamment H ou un groupe alkyle en $C_1$—$C_2$;

$R_{17}$ est H ou un groupe alkyle en $C_1$—$C_4$)

A est

A est 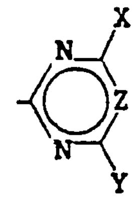 , 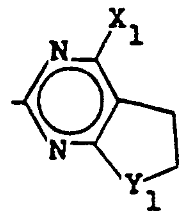 , 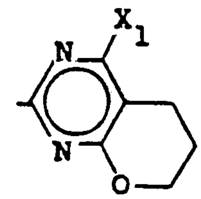 ,

A-1 A-2 A-3

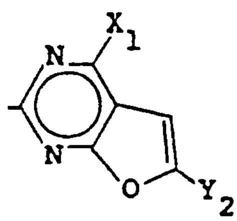 , 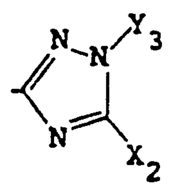 , 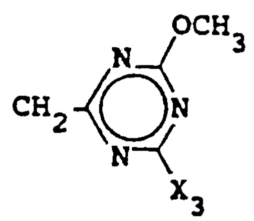 ou

A-4 A-5 A-6

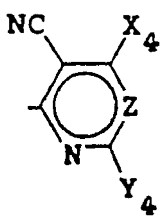 ;

A-7

X est H, un groupe alkyle en $C_1$—$C_4$, alcoxy en $C_1$—$C_4$, halogènalcoxy en $C_1$—$C_4$, halogénalkyle en $C_1$—$C_4$, halogénalkylthio en $C_1$—$C_4$, alkylthio en $C_1$—$C_4$, halogéno, alcoxyalkyle en $C_2$—$C_5$, alcoxyalcoxy en $C_2$—$C_5$, amino, alkylamino en $C_1$—$C_3$ ou di(alkyle en $C_1$—$C_3$)amino;

Y est H ou un groupe alkyle en $C_1$—$C_4$, alcoxy en $C_1$—$C_4$, halogénalcoxy en $C_1$—$C_4$, halogénalkylthio en $C_1$—$C_4$, alkylthio en $C_1$—$C_4$, alcoxyalkyle en $C_2$—$C_5$, alcoxyalcoxy en $C_2$—$C_5$, amino, alkylamino en $C_1$—$C_3$, di(alkyle en $C_1$—$C_3$,)amino, alcényloxy $C_3$—$C_4$, alcynyloxy en $C_3$—$C_4$, alkylthioalkyle en $C_2$—$C_5$, halogénalkyle en $C_1$—$C_4$, cycloalkyle en $C_3$—$C_5$, alcynyle en

$C_2$-$C_4$, 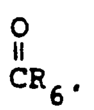 , 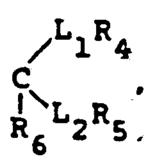 , 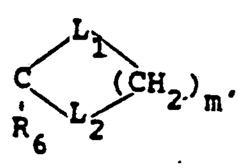 , 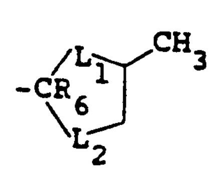

ou $N(OCH_3)CH_3$;

m est 2 ou 3;

$L_1$ et $L_2$ sont indépendamment O ou S;

$R_4$ et $R_5$ sont indépendamment un groupe alkyle en $C_1$—$C_2$;

$R_6$ est H ou $CH_3$;

Z est CH ou N;

$Y_1$ est O ou $CH_2$;

$X_1$ est $CH_3$, $OCH_3$, $OC_2H_5$ ou $OCF_2H$;
$Y_2$ est H ou $CH_3$;
$X_2$ est $CH_3$, $OCH_3$ ou $SCH_3$;
$Y_3$ est $CH_3$, $CH_2CH_3$ ou $CH_2CF_3$;
$X_3$ est $CH_3$ ou $OCH_3$;
$Y_4$ est $CH_3$, $OCH_3$, $OC_2H_5$ ou Cl; et
$X_4$ est $CH_3$, $OCH_3$, $OC_2H_5$ ou $CH_2OCH_3$;
et leurs sels utilisables en agriculture;
avec les conditions suivantes

a) si X est Cl, F, Br ou I, alors Z est CH et Y est $OCH_3$, $OC_2H_5$, $N(OCH_3)CH_3$, $NHCH_3$, $N(CH_3)_2$ ou $OCF_2H$;

b) si X ou Y est $OCF_2H$, alors Z est CH;

c) si L est L—2 ou L—3, alors le substituant W et le pont sulfonylurée se trouvent sur des atomes de carbone adjacents, et si L est L—9 ou L—10, alors Q et le pont sulfonylurée se trouvent sur des atomes de carbone adjacents;

d) si $W_1$ est un groupe alcényle en $C_3$ non substitué, alors L est L—3, L—4, L—5 ou L—6;

e) si L est L—1, alors n est 1 et W est $W_2$;

f) si L est L—1 et $W_2$ est un groupe alcynyle en $C_2$—$C_3$, ledit groupe doit être substitué;

g) si $W_1$ est un groupe alcényle en $C_3$—$C_8$ ou cycloalcényle en $C_4$—$C_7$ substitué par OH, alors le substituant OH n'est pas lié directement au groupe oléfinique;

h) si L est L—3, alors le pont sulfonylurée est lié à la position 3 ou 5;

i) si $W_3$ est S, alors R est H, A est A—1, et Y est $CH_3$, $OCH_3$, $OC_2H_5$, $CH_2OCH_3$, $C_2H_5$, $CF_3$, $SCH_3$, $OCH_2CH{=}CH_2$, $OCH_2C{\equiv}CH$, $OCH_2CH_2OCH_3$ ou $CH(OCH_3)_2$;

j) si le nombre total d'atomes de carbone de X et Y est supérieur à quatre, alors le nombre d'atomes de carbone de $R_1$ doit être inférieur ou égal à deux et, soit le nombre d'atomes de carbone de W doit être inférieur ou égal à quatre, soit le nombre d'atomes de carbone de Q doit être inférieur ou égal à six; et

k) le nombre total d'atomes de carbone de $R_{12}$ et $R_{13}$ doit être inférieur ou égal à quatre.

2. Les composés de la revendication 1, dans lesquels
R est H;
$W_3$ est O; et
Q est

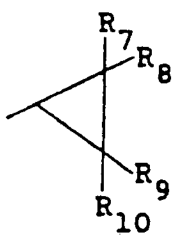
Q-1
,
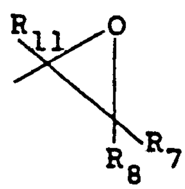
Q-2
,
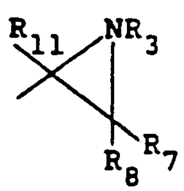
Q-3
,
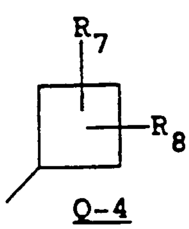
Q-4
,
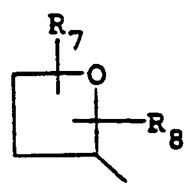
Q-5
,
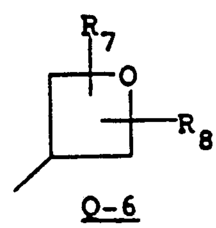
Q-6
,
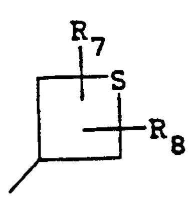
Q-7
,
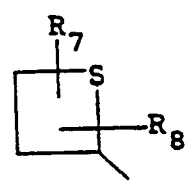
Q-8
,
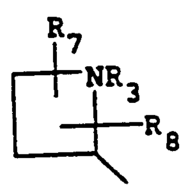
Q-9

ou
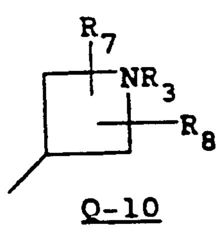
Q-10
;

où

$R_3$ est un groupe alkyle en $C_1$—$C_3$;

$R_7$ est H, F, Cl, Br, CN, $CH_3$ ou $OCH_3$;

$R_8$ est H, F, Cl, Br, I, CN, OH, un groupe alkyle en $C_1$—$C_3$, alcoxy en $C_1$—$C_3$, alcoxycarbonyle en $C_2$—$C_3$, di(alkyle en $C_1$—$C_3$)aminosulfamyle, alkylthio en $C_1$—$C_3$ ou alkylsulfonyle en $C_1$—$C_3$;

$R_9$ et $R_{10}$ sont indépendamment H, $CH_3$, F ou Cl, ou bien peuvent être pris ensemble pour former C=O; et

$R_{11}$ est H ou un groupe alkyle en $C_1$—$C_3$;

avec la condition suivante si $R_7$ et $R_8$ sont fixés au même atome de carbone, comme dans Q—1, Q—2 ou Q—3, ou dans Q—4 à Q—10, et $R_7$ est $OCH_3$, alors $R_8$ est autre chose que F, Cl, Br, I ou OH.

3. Les composés de la revendication 2, dans lesquels

$R_1$ est , Cl, $NO_2$, un groupe alkyle en $C_1$—$C_2$, halogénalkyle en $C_1$—$C_2$, alcoxy en $C_1$—$C_2$, alkylthio en $C_1$—$C_2$, $CH_2OCH_3$ ou $CH_2SCH_3$ et si L est L—1, L—4, L—5, L—6, L—7, L—8, L—11, L—12 ou L—13, $R_1$ n'est pas en *para* par rapport au pont sulfonylurée.

4. Les composés de la revendication 2, dans lesquels

$R_{13}$ est H ou $CH_3$;

$R_{14}$ est H, un groupe alkyle en $C_1$—$C_2$, $CH_2CH=CH_2$ ou $CH_2C{\equiv}CH$;

X est $CH_3$, $OCH_3$, $OC_2H_5$, Cl, F, Br, $OCF_2H$, $CH_2F$, $OCH_2CH_2F$, $OCH_2CHF_2$, $OCH_2CF_3$, $CF_3$, $CH_2Cl$ ou $CH_2Br$; et

Y est H, un groupe alkyle en $C_1$—$C_3$, $OCH_3$, $OC_2H_5$, $CH_2OCH_3$, $NHCH_3$, $N(OCH_3)CH_3$, $N(CH_3)_2$, $CF_3$, $SCH_3$, $OCH_2CH=CH_2$, $OCH_2C{\equiv}CH$, $CH_2OC_2H_5$, $OCH_2CH_2OCH_3$, $CH_2SCH_3$, $OCF_2H$, $SCF_2H$, le groupe cyclopropyle, $C{\equiv}CH$ ou $C{\equiv}CCH_3$.

5. Les composés de la revendication 4, dans lesquels

$W_1$ est une groupe alcényle en $C_2$—$C_5$ qui peut être facultativement substitué par 1 à 3 atomes de F, Cl ou Br, OH, $OCH_3$, $OC(O)CH_3$, $OSO_2CH_3$, CN ou $SCH_3$, ou bien $W_1$ est un groupe cycloalcényle en $C_5$—$C_6$; et

$W_2$ est un groupe $C{\equiv}CC_6H_5$ ou alcynyle en $C_3$—$C_5$ qui peut être facultativement substitué par 1 à 3 atomes de F, Cl ou Br, OH, $OCH_3$, $OC(O)CH_3$, CN ou $SCH_3$.

6. Les composés de la revendication 3, dans lesquels

A est A—1;

X est $CH_3$, $OCH_3$, $OC_2H_5$, Cl ou $OCF_2H$;

Y est $CH_3$, $C_2H_5$, $OCH_3$, $CH_2OCH_3$, $CH(OCH_3)_2$, $OCF_2H$, $NHCH_3$, $N(CH_3)_2$ ou le groupe cyclopropyle; et

$R_1$ est H, $CH_3$, $OCH_3$, $SCH_3$ ou Cl.

7. Le composé de la revendication 1, qui est le 2-(1-cyclohexène-2-yl)-N-[(4-méthoxy-6-méthylpyrimidine-2-yl)aminocarbonyl]-3-thiophène-sulfonamide.

8. Le composé de la revendication 1, qui est le 2-(1-cyclohexène-2-yl)-N-[(4-méthoxy-6-méthyl-1,3,5-triazine-2-yl)aminocarbonyl]-3-thiophène-sulfonamide.

9. Le composé de la revendication 1, qui est le N-[(4-méthoxy-6-méthylpyrimidine-2-yl)aminocarbonyl]-1-méthyl-4-(1-propényl)-1H-pyrazole-5-sulfonamide.

10. Le composé de la revendication 1, qui est le 2-(2,2-dichlorocyclopropyl)-N-[(4,6-diméthoxypyrimidine-2-yl)aminocarbonyl]benzène-sulfonamide.

11. Le composé de la revendication 1, qui est le 2-(2,2-dichlorocyclopropyl)-N-[(4-méthoxy-6-méthyl-1,3,5-triazine-2-yl)aminocarbonyl]benzène-sulfonamide.

12. Le composé de la revendication 1, qui est le N-[(4,6-diméthoxypyrimidine-2-yl)aminocarbonyl]-2-(2-oxirannylméthyl)benzène-sulfonamide.

13. Un composé de la revendication 1, dans lequel

$W_3$ est O;

$R_1$ est H, un groupe alkyle ou halogénalkyle en $C_1$—$C_3$, halogéno, nitro, alcoxy en $C_1$—$C_3$, $SO_2NR^IR^{II}$, alkylthio en $C_1$—$C_3$, alkylsulfinyle en $C_1$—$C_3$, alkylsulfonyle en $C_1$—$C_3$, ou $CO_2R^{III}$;

W est un groupe alcényle en $C_2$—$C_5$, alcynyle en $C_2$—$C_5$, cycloalcényle en $C_5$—$C_6$, dont chacun peut être facultativement substitué par 1 à 4 substituants choisis parmi les halogènes et les groupe cyano, alkyles en $C_1$—$C_3$, alcoxy en $C_1$—$C_3$, alkylthio en $C_1$—$C_3$, (alcoxy en $C_1$—$C_3$)carbonyle, alkylsulfonyle en $C_1$—$C_4$, (alkyle en $C_1$—$C_4$)aminosulfamyle ou di(alkyle en $C_1$—$C_4$)aminosulfamyle;

Q est un hétérocycle ou un carbocycle à 3 ou 4 chaînons qui contient 0 ou 1 hétéroatome choisi parmi O, S ou $NR_3$, ledit cycle pouvant être facultativement substitué comme défini pour W ci -dessus;

$R_{12}$ et $R_{13}$ sont H et;

A est choisi parmi A—1 à A—6.

14. Une composition utilisable en agriculture pour lutter la croissance de végétation indésirable, qui comprend une quantité efficace d'un composé de l'une quelconque des revendications 1 à 13 et au moins l'un des ingrédients suivants: un agent tensio-actif, un diluant solide ou un diluant liquide.

15. Un procédé pour lutter contre la croissance de végétation indésirable, qui consiste à appliquer au lieu à protéger une quantité efficace d'un composé de l'une quelconque des revendications 1 à 13.

16. Un procédé pour réguler la croissance de plantes, qui consiste à appliquer au lieu où se trouvent ces plantes une quantité efficace, mais sensiblement non phytotoxique, d'un régulateur de croissance de plantes choisi parmi les composé de l'une quelconque des revendications 1 à 13.

17. Un procédé pour la préparation d'un composé de la revendication 1, qui consiste à:
(a) faire réagir un isocyanate ou isothiocyanate de sulfonyle approprié de formule

$$LSO_2NCW_3 \qquad (II)$$

avec un aminohétérocycle convenablement substitué de formule

$$RNHA \qquad (III)$$

où R, $W_3$, A et H sont tels que définis dans la revendication 1; ou
(b) faire réagir un sulfonamide de formule

$$LSO_2NH_2 \qquad (IV)$$

avec un carbamate de formule

$$R'O\overset{\overset{\displaystyle O}{\|}}{C}NRA \qquad (V)$$

où L, R et A sont tels que définis dans la revendication 1, excepté que $R_1$ est autre chose que $CO_2R^{III}$, et R' est $CH_3$ ou Ph.

18. Isocyanates et isothiocyanates de sulfonyle de la formule (II) définie dans la revendication 17 et sulfonamides de la formule (IV) définie dans la revendication 17.